# EUROPEAN PATENT APPLICATION

(11) **EP 1 829 863 A1**
(43) Date of publication of application: **05.09.2007**
(21) Application number: 05811684.9
(22) Date of filing: 25.11.2005
(51) Int. Cl.: C07D 213/30, A61K 31/415, A61K 31/422, A61K 31/426, A61K 31/427, A61K 31/44, A61K 31/4402, A61K 31/4709, A61K 31/506, A61P 3/02, A61P 3/06, A61P 3/10, A61P 43/00, C07D 213/50, C07D 213/64, C07D 213/69, C07D 231/20, C07D 231/22, C07D 277/20, C07D 277/56, C07D 401/06

(54) **ARYLALKANOIC ACID DERIVATIVE**

(30) Priority: 26.11.2004 JP 2004342635
(71) Applicant: Takeda Pharmaceutical Company Limited, Osaka-shi, Osaka 541-0045 (JP)
(72) Inventor: MAEKAWA, Tsuyoshi, TAKEDA PHARMACEUTICAL COMP., Osaka-shi, Osaka 5328686 (JP); UJIKAWA, Osamu, TAKEDA PHARMACEUTICAL COMP., Osaka-shi, Osaka 5328686 (JP); ABE, Hidenori, TAKEDA PHARMACEUTICAL COMP., Osaka-shi, Osaka 5328686 (JP); NOMURA, Izumi, TAKEDA PHARMACEUTICAL COMP., Osaka-shi, Osaka 5328686 (JP)
(74) Representative: Rickard, Timothy Mark Adrian
(86) International application number: PCT/JP2005/022132
(87) International publication number: WO 2006/057448

(57) **Abstract**

A compound represented by the formula (I): 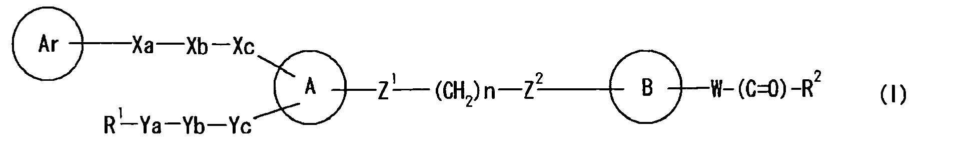 wherein
Ar is an optionally substituted aromatic ring;
Xa, Xc, Ya, Yc, Z¹ and Z² are each a bond, O, S, -CO-, -CS-, - CR³ (OR⁴) -, -NR⁵-, -SO-, -SO₂-, -CONR⁶- or -NR⁶CO- (wherein R³, R⁴, R⁵ and R⁶ are as defined in the specification);
Xb and Yb are each a bond or a divalent hydrocarbon group having 1 to 20 carbon atoms;
R¹ is an optionally substituted hydrocarbon group;
ring A is an optionally further substituted aromatic ring, provided that the ring should not be benzimidazole;
n is an integer of 1 to 8;
ring B is an optionally further substituted aromatic ring, provided that the ring should not be oxazole;
W is a divalent saturated hydrocarbon group having 1 to 20 carbon atoms; and
R² is -OR⁸ or -NR⁹R¹⁰ (wherein R⁸, R⁹ and R¹⁰ are as defined in the specification)
or a salt thereof,
is useful as an agent for the prophylaxis or treatment of diabetes and the like.

## Description

### Technical Field

The present invention relates to a novel arylalkanoic acid derivative having a superior hypoglycemic action and hypolipidemic action, which is useful as an agent for the prophylaxis or treatment of diabetes, hyperlipidemia, impaired glucose tolerance and the like.

### Background Art

As alkane acid derivatives, the compounds described in the following literatures are known.
(1) As a PPAR ligand-receptor binder, WO00/64876 describes a compound represented by the formula: 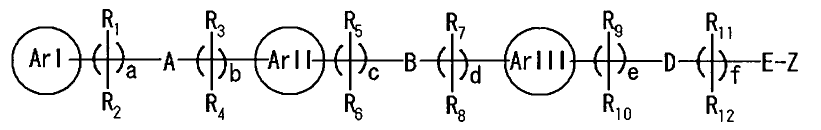 wherein
   ArI, ArII and ArIII are each an aryl, a fused arylcycloalkyl, a fused arylheterocyclyl and the like; A, B and D are each an oxygen atom, a bond, NR and the like; E is a bond or an ethylene group; Z is R₂₁O₂C-, R₂₁OC- (wherein R₂₁ is a hydrogen atom and the like), tetrazolyl, thiazolidinedionyl and the like; a, b, c and e are each 0-4; d is 0-5; f is 0-6 ; and R₁ - R₁₂ are each a hydrogen atom and the like.
(2) As a PPARδ agonist, WO01/00603 describes a compound represented by the formula: 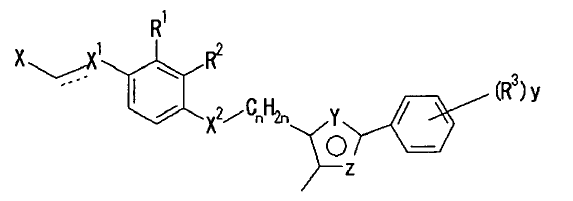 wherein
   X is carboxy and the like; X¹ is an oxygen atom and the like; X² is an oxygen atom and the like; R¹ and R² are each a hydrogen atom and the like; n is 1 or 2; one of Y and Z is a nitrogen atom, and the other is a sulfur atom or an oxygen atom; y is 0-5; and R³ is trifluoromethyl and the like.
(3) WO02/053547 describes a compound represented by the formula: 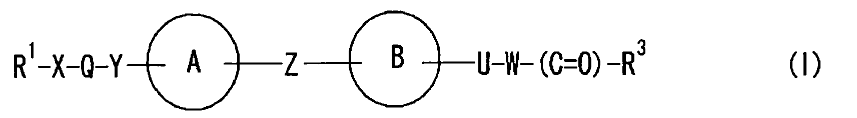 wherein
   R¹ is an optionally substituted 5-membered aromatic heterocyclic group; X is a bond and the like; Q is a divalent C₁₋₂₀ hydrocarbon group; Y is a bond and the like; ring A is an optionally substituted aromatic ring; Z is -(CH₂)n-Z¹- (wherein n is 1-8, and Z¹ is an oxygen atom and the like) and the like; ring B is an optionally substituted benzene and the like; U is a bond and the like; W is a divalent C₁₋₂₀ hydrocarbon group; and R³ is -OH and the like,
   which is useful as an agent for the prophylaxis or treatment of diabetes and the like.
(4) WO02/076959 describes a compound represented by the formula: 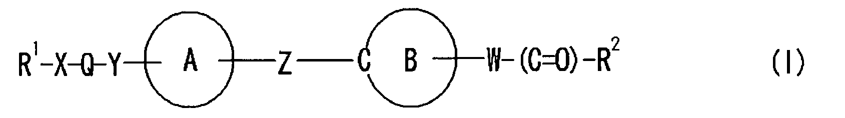 wherein
   R¹ is an optionally substituted 5-membered heterocyclic group; X and Y are each a bond, an oxygen atom, a sulfur atom and the like; Q is a divalent C₁₋₂₀ hydrocarbon group; ring A is an aromatic ring optionally further having 1 to 3 substituents; Z is -(CH₂)n-Z¹- (wherein n is 1-8, and Z¹ is an oxygen atom and the like) and the like; ring B is a 5-membered heterocycle optionally further having 1 to 3 substituents; W is a divalent C₁₋₂₀ saturated hydrocarbon group; and R² is -OH and the like, which is useful as an agent for the prophylaxis or treatment of diabetes and the like.
(5) WO03/024937 describes a compound represented by the formula: 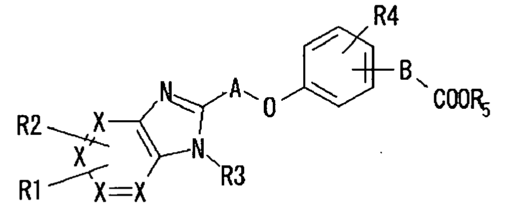 wherein
   X is a carbon atom and the like; R1 - R5 are each a hydrogen atom, an optionally substituted alkyl, an optionally substituted arylalkyl and the like; A is an optionally substituted alkyl; and B is a single bond or an optionally substituted alkyl,
   which is useful for the treatment of hyperglycemia, diabetes and the like.
(6) WO03/099793 describes a compound represented by the formula: 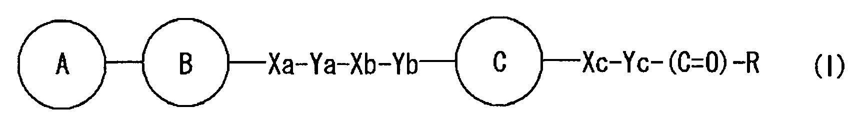 wherein
   ring A is a ring optionally having 1 to 3 substituents; ring B is an 1,2-azole ring optionally having 1 to 3 substituents; Xa, Xb and Xc are each a bond, -O- and the like; Ya is a divalent C₁-₂₀ aliphatic hydrocarbon residue; Yb and Yc are each a bond or a divalent C₁₋₂₀ aliphatic hydrocarbon residue; ring C is a monocyclic aromatic ring optionally having 1 to 3 substituents; and R is -OH and the like,
   which is useful for the prophylaxis or treatment of diabetes and the like.
(7) WO2004/022551 describes a compound represented by the formula: 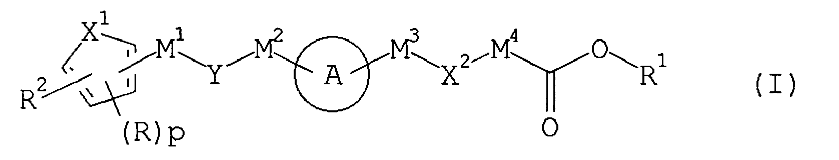 wherein
   R is an optionally substituted hydrocarbon group and the like; p is 0-2; R¹ is a hydrogen atom and the like; R² is an optionally substituted aromatic group; ring A is an optionally substituted monocyclic aromatic ring and the like; X¹ is an oxygen atom and the like; X² is a bond, an oxygen atom and the like; Y is a bond, an oxygen atom and the like; M¹, M² and M³ are each a bond, an optionally substituted divalent aliphatic hydrocarbon group; and M⁴ is an optionally substituted divalent aliphatic hydrocarbon group, which is useful for the prophylaxis or treatment of diabetes and the like.
(8) As an insecticide and the like, WO01/20993 describes a compound represented by the formula: 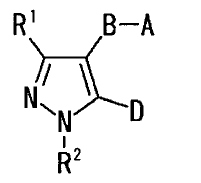 wherein
   A is an optionally substituted aryl and the like; B is the formula: -(G1)ₙ- G²- (G¹)ₘ- [wherein G¹ is an oxygen atom and the like; G² is an alkylene and the like; and n and m are each 0 or 1] and the like; R¹ is a hydrogen atom and the like; R² is a hydrogen atom and the like; and D is the formula: -C(=Y)COX [wherein X is OH and the like; and Y is the formula: CH-(G³)ₙ-G⁴ [wherein G³ is an oxygen atom and the like; G⁴ is an alkyl and the like; and n is 0 or 1] and the like] and the like.
(9) As a PPARγ agonist or antagonist, US 2003/0083329 describes a compound represented by the formula: 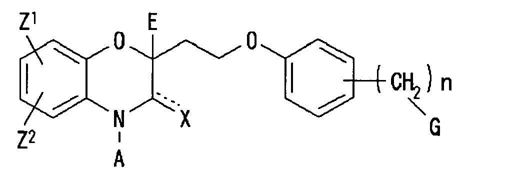 wherein
   A is an optionally substituted alkyl and the like; Z¹ is an alkyl, an aryl and the like; Z² is an alkyl and the like; n is 0-3; G is -COOR¹ (wherein R¹ is H and the like) and the like; E is a hydrogen atom and the like; and X is a hydrogen atom and the like.
(10) As an agricultural fungicide, JP-A-4-217668 describes a compound represented by the formula: 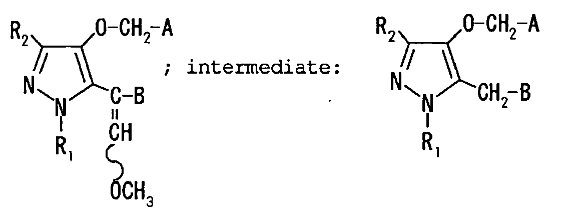
wherein
R₁ and R₂ are each a hydrogen atom, a C₁₋₅ alkyl group and the like; B is methoxycarbonyl and the like; and A is 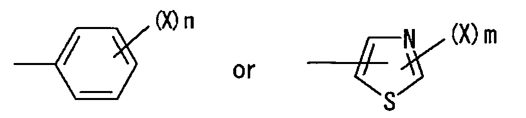 wherein n is 1-5; m is 1-2; and X is a C₂₋₁₃ heteroaryloxy having 5 to 14 atoms in total, and the like.

Peroxisome proliferator-activated receptor gamma (PPARγ), which is one member of the nuclear hormone receptor superfamily represented by steroid hormone receptors and thyroid gland hormone receptors, shows an induced expression at the beginning of differentiation of adipocytes and plays an important role as a master regulator in the differentiation of adipocytes. PPARγ binds to a ligand to form a dimer with retinoid X receptor (RXR), and the dimer binds to a responsive element of a target gene in the nucleus to directly control (activate) the transcription efficiency.

In recent years, a possibility has been suggested that 15-deoxy-Δ12.14 prostaglandin J2, which is a metabolite of prostaglandin D2, is an endogenous ligand of PPARγ, and moreover, it has been clarified that certain insulin sensitizers represented by thiazolidinedione derivatives have a PPARγ ligand activity and the strength of the activity parallels with a hypoglycemic action or adipocyte differentiation promoting action [Cell, 83, 803 (1995); The Journal of Biological Chemistry, 270, 12953(1995); Journal of Medicinal Chemistry, 39, 665 (1996)].

It has also been elucidated that 1) PPARγ is expressed in the cultured cell derived from human liposarcoma and the addition of PPARγ ligand stops its growth [Proceedings of The National Academy of Sciences of The United States of America, 94, 237 (1997)]; 2) nonsteroidal anti-inflammatory drugs represented by indomethacin and phenoprofen have a PPARγ ligand activity [The Journal of Biological Chemistry, 272, 3406 (1997)]; 3) PPARγ is highly expressed in activated macrophage, and the addition of its ligand leads to the inhibition of the transcription of the gene involved in inflammation [Nature, 391, 79 (1998)]; 4) PPARγ ligand inhibits production of inflammatory cytokines by monocyte (TNFα, IL-1β, IL-6) [Nature, 391, 82 (1998)] and the like.

### Disclosure of the Invention

There is a demand for the development of a novel compound which is useful as an agent for the prophylaxis or treatment of diabetes, hyperlipidemia, impaired glucose tolerance and the like, and has superior properties as a pharmaceutical agent, such as a fewer side effects, and the like.

The present inventors have conducted intensive studies in an attempt to find such compound and completed the present invention. Accordingly, the present invention relates to
[1] a compound represented by the formula (I): 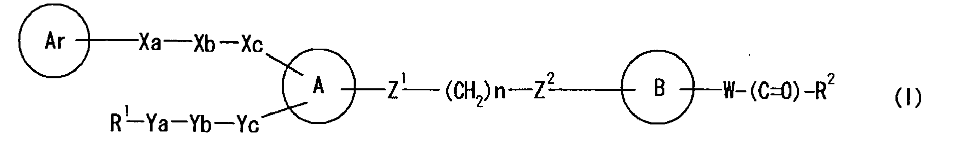 wherein
   Ar is an optionally substituted aromatic ring;
   Xa, Xc, Ya, Yc, Z¹ and Z² are the same or different and each is a bond, an oxygen atom, a sulfur atom, -CO-, -CS-, -CR³(OR⁴)-, - NR⁵-, -SO-, -SO₂-, -CONR⁶- or -NR⁶CO- (wherein R³ is a hydrogen atom or an optionally substituted hydrocarbon group, R⁴ is a hydrogen atom or a hydroxyl-protecting group, R⁵ is a hydrogen atom, an optionally substituted hydrocarbon group or an amino-protecting group, and R⁶ is a hydrogen atom or an optionally substituted hydrocarbon group);
   Xb and Yb are the same or different and each is a bond or a divalent hydrocarbon group having 1 to 20 carbon atoms;
   R¹ is an optionally substituted hydrocarbon group;
   ring A is an optionally further substituted aromatic ring, provided that the ring should not be benzimidazole;
   n is an integer of 1 to 8;
   ring B is an optionally further substituted aromatic ring, provided that the ring should not be oxazole;
   W is a divalent saturated hydrocarbon group having 1 to 20 carbon atoms; and
   R² is -OR⁸ (wherein R⁸ is a hydrogen atom or an optionally substituted hydrocarbon group) or -NR⁹R¹⁰ (wherein R⁹ and R¹⁰ are the same or different and each is a hydrogen atom, an optionally substituted hydrocarbon group, an optionally substituted heterocyclic group or an acyl group, or R⁹ and R¹⁰ are bonded to each other to form, together with the adjacent nitrogen atom, an optionally substituted ring):
   with the proviso that
   a compound wherein Xa, Xb and Xc are each a bond is excluded;
   a compound wherein Z¹ and Z² are each a bond or each an oxygen atom is excluded; and
   when ring A is a benzene ring, then the carbon atom thereon to which Xc is bonded and the carbon atom thereon to which Yc is bonded should not be adjacent to each other, and Z² should not be a sulfur atom,
   or a salt thereof (hereinafter sometimes to be abbreviated as compound (I));
[2] the compound of the above-mentioned [1], wherein the aromatic ring for Ar is a benzene ring, a pyridine ring, an oxazole ring or a quinoline ring;
[3] the compound of the above-mentioned [1], wherein Xa is a bond or an oxygen atom, Xb is a bond or a divalent hydrocarbon group having 1 to 6 carbon atoms, and Xc is a bond, an oxygen atom, -CO- or -NR⁶CO- (wherein R⁶ is a hydrogen atom or an alkyl group having 1 to 4 carbon atoms);
[4] the compound of the above-mentioned [3], wherein Xa is a bond, Xb is a bond or a divalent hydrocarbon group having 1 to 6 carbon atoms, and Xc is an oxygen atom;
[5] the compound of the above-mentioned [3], wherein Xa is a bond, Xb is a divalent hydrocarbon group having 1 to 6 carbon atoms, and Xc is a bond;
[6] the compound of the above-mentioned [1], wherein R¹ is an optionally substituted alkyl group having 1 to 10 carbon atoms;
[7] the compound of the above-mentioned [1], wherein Ya is a bond, an oxygen atom, a sulfur atom, -NR⁵- (wherein R⁵ is an alkyl group having 1 to 4 carbon atoms), -SO₂-, -CONR⁶- or - NR⁶CO- (wherein R⁶ is a hydrogen atom or an alkyl group having 1 to 4 carbon atoms), Yb is a bond or a divalent hydrocarbon group having 1 to 6 carbon atoms, and Yc is a bond or an oxygen atom;
[8] the compound of the above-mentioned [7], wherein Ya and Yb are each a bond;
[9] the compound of the above-mentioned [1], wherein the aromatic ring for ring A is a benzene ring, a pyridine ring, a pyrimidine ring, a pyrazole ring or a thiazole ring;
[10] the compound of the above-mentioned [9], wherein the aromatic ring for ring A is a benzene ring;
[11] the compound of the above-mentioned [1], wherein Z¹ is a bond, n is an integer of 1 to 4, and Z² is an oxygen atom;
[12] the compound of the above-mentioned [1], wherein the aromatic ring for ring B is a benzene ring, a naphthalene ring, a pyridine ring, a pyrazole ring or a thiazole ring;
[13] the compound of the above-mentioned [8], wherein the aromatic ring for ring B is a pyrazole ring;
[14] the compound of the above-mentioned [1], wherein R² is -OR⁸, and R⁸ is a hydrogen atom or an alkyl group having 1 to 4 carbon atoms;
[15] the compound of the above-mentioned [1], which is 3-{3-[3-(2-{[3-chloro-5-(trifluoromethyl)pyridin-2-yl]oxy}-4-isopropoxyphenyl)propoxy]-1-ethyl-1H-pyrazol-5-yl}propanoic acid, 3-(3-{3-[2-{[3-chloro-5-(trifluoromethyl)pyridin-2-yl]oxy}-4-(2-methoxyethoxy)phenyl]propoxy}-1-ethyl-1H-pyrazol-5-yl)propanoic acid, {2-[3-(2-{[3-chloro-5-(trifluoromethyl)pyridin-2-yl]oxy}-4-isopropoxyphenyl)propoxy]-3-methoxyphenyl}acetic acid, (1-benzyl-3-{3-[1-(2,4-dichlorobenzyl)-3-isopropoxy-1H-pyrazol-5-yl]propoxy}-1H-pyrazol-4-yl)acetic acid, (2-{3-[1-(2,4-dichlorobenzyl)-3-isopropoxy-1H-pyrazol-5-yl]propoxy}-3-methoxyphenyl)acetic acid, (4-{3-[1-(2,4-dichlorobenzyl)-3-isopropoxy-1H-pyrazol-5-yl]propoxy}-2-methyl-1,3-thiazol-5-yl)acetic acid, [2-(3-{1-[2-chloro-4-(trifluoromethyl)benzyl]-3-isopropyl-1H-pyrazol-5-yl}propoxy)pyridin-3-yl]acetic acid, (3-{3-[2-(2,4-dichlorophenoxy)-6-isopropoxypyridin-3-yl]propoxy}-1-methyl-1H-pyrazol-4-yl)acetic acid, or (1-{3-[1-(2,4-dichlorobenzyl)-3-isopropoxy-1H-pyrazol-5-yl]propoxy}-2-naphthyl)acetic acid;
[16] a prodrug of compound (I);
[17] a pharmaceutical agent comprising compound (I) or a prodrug thereof;
[18] the pharmaceutical agent of the above-mentioned [17], which is an agent for the prophylaxis or treatment of diabetes;
[19] the pharmaceutical agent of the above-mentioned [17], which is an agent for the prophylaxis or treatment of hyperlipidemia;
[20] the pharmaceutical agent of the above-mentioned [17], which is an agent for the prophylaxis or treatment of impaired glucose tolerance;
[21] a retinoid-related receptor function regulator comprising compound (I) or a prodrug thereof;
[22] the retinoid-related receptor function regulator of the above-mentioned [21], which is a peroxisome proliferator-activated receptor ligand;
[23] the retinoid-related receptor function regulator of the above-mentioned [21], which is a retinoid X receptor ligand;
[24] an insulin sensitizer comprising compound (I) or a prodrug thereof;
[25] a method for the prophylaxis or treatment of diabetes in a mammal, which comprises administering compound (I) or a prodrug thereof to the mammal;
[26] a method for the prophylaxis or treatment of hyperlipidemia in a mammal, which comprises administering compound (I) or a prodrug thereof to the mammal;
[27] a method for the prophylaxis or treatment of impaired glucose tolerance in a mammal, which comprises administering compound (I) or a prodrug thereof to the mammal;
[28] use of compound (I) or a prodrug thereof for the production of an agent for the prophylaxis or treatment of diabetes:
[29] use of compound (I) or a prodrug thereof for the production of an agent for the prophylaxis or treatment of hyperlipidemia:
[30] use of compound (I) or a prodrug thereof for the production of an agent for the prophylaxis or treatment of impaired glucose tolerance:
[31] a production method of a compound represented by the formula (I-1): 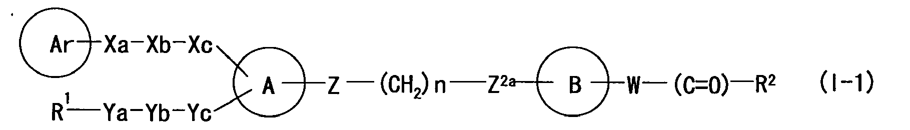
wherein each symbol is as defined in the above-mentioned [1] (hereinafter sometimes to be abbreviated as compound (I-1)), which comprises reacting a compound represented by the formula (II): 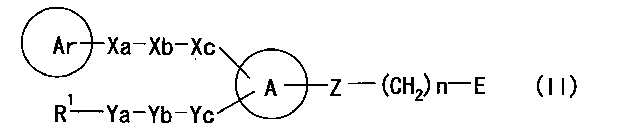 wherein E is a leaving group, and the other symbols are as defined in the above-mentioned [1],
or a salt thereof (hereinafter sometimes to be abbreviated as compound (II)) with a compound represented by the formula (III): 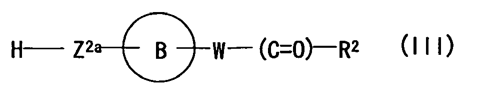 wherein Z^{2a} is an oxygen atom, a sulfur atom or -NR⁵- (wherein R⁵ is as defined in the above-mentioned [1]), and the other symbols are as defined in the above-mentioned [1],
or a salt thereof (hereinafter sometimes to be abbreviated as compound (III));
and the like.

The compound of the present invention has a superior hypoglycemic action and hypolipidemic action, and is useful as an agent for the prophylaxis or treatment of diabetes, hyperlipidemia, impaired glucose tolerance and the like.

### Best Mode for Embodying the Invention

Each symbol in the formulas is described in detail in the following.

Ar is an optionally substituted aromatic ring.

As the "aromatic ring" of the "optionally substituted aromatic ring" for Ar, for example, a benzene ring, a fused aromatic hydrocarbon ring, a 5- or 6-membered aromatic heterocycle, a fused aromatic heterocycle and the like can be mentioned.

As used herein, as the "fused aromatic hydrocarbon ring", for example, a fused aromatic hydrocarbon ring having 9 to 14 carbon atoms and the like can be mentioned, and it may be partially hydrogenated. To be specific, naphthalene, indene, fluorene, anthracene and the like can be mentioned.

As the "5- or 6-membered aromatic heterocycle", for example, a 5- or 6-membered aromatic heterocycle containing, besides carbon atoms, 1 to 3 heteroatoms selected from a nitrogen atom, a sulfur atom and an oxygen atom, and the like can be mentioned. To be specific, thiophene, furan, pyrrole, imidazole, pyrazole, thiazole, isothiazole, oxazole, isoxazole, pyridine, pyrazine, pyrimidine, pyridazine, 1,2,4-oxadiazole, 1,3,4-oxadiazole, 1,2,4-thiadiazole, 1,3,4-thiadiazole, furazan and the like can be mentioned.

As the "fused aromatic heterocycle", for example, a 9- to 14-membered (preferably 9- or 10-membered) fused aromatic heterocycle containing, besides carbon atoms, 1 to 4 heteroatoms selected from a nitrogen atom, a sulfur atom and an oxygen atom, and the like can be mentioned. To be specific, benzofuran, benzothiophene, benzimidazole, benzoxazole, benzothiazole, benzisothiazole, naphto[2,3-b]thiophene, isoquinoline, quinoline, indole, quinoxaline, phenanthridine, phenothiazine, phenoxathiine, phthalazine, naphthyridine, quinazoline, cinnoline, carbazole, β-carboline, acridine, phenazine and the like can be mentioned.

As the "aromatic ring" of the "optionally substituted aromatic ring" for Ar, a benzene ring, a 5- or 6-membered aromatic heterocycle (e.g., pyridine, oxazole and the like), a 9- to 14-membered fused aromatic heterocycle (e.g., quinoline and the like) and the like are preferable, and a benzene ring, a pyridine ring, an oxazole ring, a quinoline ring and the like are more preferable.

The "aromatic ring" of the "optionally substituted aromatic ring" for Ar optionally has 1 to 4, preferably 1 to 3, substituents at substitutable positions. As such substituents, for example, a "halogen atom", a "nitro group", an "optionally substituted aliphatic hydrocarbon group", an "optionally substituted alicyclic hydrocarbon group", an "optionally substituted aromatic hydrocarbon group", an "optionally substituted heterocyclic group", an "optionally substituted acyl group", an "optionally substituted amino group", an "optionally substituted hydroxy group", an "optionally substituted mercapto group" and the like can be mentioned. When the ring has 2 or more substituents, respective substituents may be the same or different.

As the "halogen atom", fluorine, chlorine, bromine and iodine can be mentioned. Of these, fluorine and chlorine are preferable.

As the aliphatic hydrocarbon group of the "optionally substituted aliphatic hydrocarbon group", a linear or branched aliphatic hydrocarbon group having 1 to 15 carbon atoms, for example, an alkyl group, an alkenyl group, an alkynyl group and the like can be mentioned.

As preferable examples of the alkyl group, an alkyl group having 1 to 10 carbon atoms, for example, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl and the like can be mentioned.

As preferable examples of the alkenyl group, an alkenyl group having 2 to 10 carbon atoms, for example, ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl and the like can be mentioned.

As preferable examples of the alkynyl group, an alkynyl group having 2 to 10 carbon atoms, for example, ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl and the like can be mentioned.

As the substituents of the "optionally substituted aliphatic hydrocarbon group", for example,
a cycloalkyl group having 3 to 10 carbon atoms (e.g., cyclohexyl),
an aryl group having 6 to 14 carbon atoms (e.g., phenyl, naphthyl),
an aromatic heterocyclic group (e.g., thienyl, furyl, pyridyl, oxazolyl, thiazolyl),
a non-aromatic heterocyclic group (e.g., tetrahydrofuryl, morpholino, thiomorpholino, piperidino, pyrrolidinyl, piperazinyl),
an amino group,
an amino group mono- or di-substituted by an alkyl group having 1 to 4 carbon atoms or an acyl group having 2 to 8 carbon atoms (e.g., alkylcarbonyl group),
an amidino group,
an acyl group having 2 to 8 carbon atoms (e.g., alkylcarbonyl group),
a carbamoyl group,
a carbamoyl group mono- or di-substituted by an alkyl group having 1 to 4 carbon atoms,
a sulfamoyl group,
a sulfamoyl group mono- or di-substituted by an alkyl group having 1 to 4 carbon atoms,
a carboxyl group,
an alkoxycarbonyl group having 2 to 8 carbon atoms (e.g., methoxycarbonyl, ethoxycarbonyl),
a hydroxy group,
an alkoxy group having 1 to 6 carbon atoms (e.g., methoxy, ethoxy), which is optionally substituted by 1 to 3 halogen atoms (e.g., fluorine, chlorine, bromine, iodine),
an alkenyloxy group having 2 to 5 carbon atoms (e.g., allyloxy), which is optionally substituted by 1 to 3 halogen atoms (e.g., fluorine, chlorine, bromine, iodine),
a cycloalkyloxy group having 3 to 7 carbon atoms (e.g., cyclohexyloxy),
an aralkyloxy group having 7 to 9 carbon atoms (e.g., benzyloxy), an aryloxy group having 6 to 14 carbon atoms (e.g., phenyloxy, naphthyloxy),
a mercapto group,
an alkylthio group having 1 to 6 carbon atoms (e.g., methylthio, ethylthio), which is optionally substituted by 1 to 3 halogen atoms (e.g., fluorine, chlorine, bromine, iodine), an aralkylthio group having 7 to 9 carbon atoms (e.g., benzylthio),
an arylthio group having 6 to 14 carbon atoms (e.g., phenylthio, naphthylthio),
a sulfo group,
a cyano group,
a nitro group,
a nitroso group,
a halogen atom (e.g., fluorine, chlorine, bromine, iodine) and the like can be mentioned. The number of the substituents is, for example, 1 to 3. When the ring has 2 or more substituents, respective substituents may be the same or different.

As the alicyclic hydrocarbon group of the "optionally substituted alicyclic hydrocarbon group", a saturated or unsaturated alicyclic hydrocarbon group having 3 to 12 carbon atoms, for example, a cycloalkyl group, a cycloalkenyl group, a cycloalkadienyl group and the like can be mentioned.

As preferable examples of the cycloalkyl group, a cycloalkyl group having 3 to 10 carbon atoms, for example, cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl and the like can be mentioned.

As preferable examples of the cycloalkenyl group, a cycloalkenyl group having 3 to 10 carbon atoms, for example, 2-cyclopenten-1-yl, 3-cyclopenten-1-yl, 2-cyclohexen-1-yl, 3-cyclohexen-1-yl and the like can be mentioned.

As preferable examples of the cycloalkadienyl group, a cycloalkadienyl group having 4 to 10 carbon atoms, for example, 2,4-cyclopentadien-1-yl, 2,4-cyclohexadien-1-yl, 2,5-cyclohexadien-1-yl and the like can be mentioned.

As the aromatic hydrocarbon group of the "optionally substituted aromatic hydrocarbon group", an aromatic hydrocarbon group having 6 to 14 carbon atoms (e.g., an aryl group), for example, phenyl, naphthyl, anthryl, phenanthryl, acenaphthylenyl, biphenylyl and the like can be mentioned. Of these, phenyl, 1-naphthyl, 2-naphthyl and the like are preferable. The aromatic hydrocarbon group may be partially hydrogenated, and as the partially hydrogenated aromatic hydrocarbon group, for example, tetrahydronaphthalenyl and the like can be mentioned.

As the heterocyclic group of the "optionally substituted heterocyclic group", an aromatic heterocyclic group and a non-aromatic heterocyclic group can be mentioned.

As the aromatic heterocyclic group, for example, a monocyclic, bicyclic or tricyclic aromatic heterocyclic group containing, as a ring-constituting atom besides carbon atoms, 1 to 5 heteroatoms selected from an oxygen atom, a sulfur atom and a nitrogen atom, and the like can be mentioned.

As preferable examples of the monocyclic aromatic heterocyclic group, furyl, thienyl, pyrrolyl, oxazolyl, isoxazolyl, thiazolyl, isothiazolyl, imidazolyl, pyrazolyl, oxadiazolyl (1,2,3-oxadiazolyl, 1,2,4-oxadiazolyl, 1,3,4-oxadiazolyl), furazanyl, thiadiazolyl (1,2,3-thiadiazolyl, 1,2,4-thiadiazolyl, 1,3,4-thiadiazolyl), triazolyl (1,2,3-triazolyl, 1,2,4-triazolyl), tetrazolyl, pyridyl, pyrimidinyl, pyridazinyl, pyrazinyl, triazinyl and the like can be mentioned.

As preferable examples of the bicyclic or tricyclic aromatic heterocyclic group, benzofuranyl, isobenzofuranyl, benzo[b]thienyl, indolyl, isoindolyl, 1H-indazolyl, benzimidazolyl, benzoxazolyl, benzothiazolyl, 1H-benzotriazolyl, quinolyl, isoquinolyl, cinnolinyl, quinazolyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl and the like can be mentioned.

As the non-aromatic heterocyclic group, for example, a non-aromatic heterocyclic group having 2 to 10 carbon atoms and containing, as a ring-constituting atom besides carbon atoms, 1 to 3 heteroatoms selected from an oxygen atom, a sulfur atom and a nitrogen atom, and the like can be mentioned. As preferable examples of the non-aromatic heterocyclic group, oxiranyl, azetidinyl, oxetanyl, thietanyl, pyrrolidinyl, tetrahydrofuryl, tetrahydropyranyl, oxazolinyl, oxazolidinyl, dioxooxazolidinyl, thiazolinyl, thiazolidinyl, dioxothiazolidinyl, morpholinyl, thiomorpholinyl, piperazinyl, pyrrolidinyl, piperidino, morpholino, thiomorpholino and the like can be mentioned.

As the substituents of the aforementioned "optionally substituted alicyclic hydrocarbon group", "optionally substituted aromatic hydrocarbon group" and "optionally substituted heterocyclic group", for example,
an alkyl group having 1 to 6 carbon atoms, which is optionally substituted by 1 to 3 halogen atoms (e.g., fluorine, chlorine, bromine, iodine),
an alkenyl group having 2 to 6 carbon atoms, which is optionally substituted by 1 to 3 halogen atoms (e.g., fluorine, chlorine, bromine, iodine),
a cycloalkyl group having 3 to 10 carbon atoms (e.g., cyclohexyl),
an aryl group having 6 to 14 carbon atoms (e.g., phenyl, naphthyl),
an aromatic heterocyclic group (e.g., thienyl, furyl, pyridyl, oxazolyl, thiazolyl),
a non-aromatic heterocyclic group (e.g., tetrahydrofuryl, morpholino, thiomorpholino, piperidino, pyrrolidinyl, piperazinyl),
an aralkyl group having 7 to 9 carbon atoms (e.g., benzyl),
an amino group,
an amino group mono- or di-substituted by an alkyl group having 1 to 4 carbon atoms or an acyl group having 2 to 8 carbon atoms (e.g., alkylcarbonyl group),
an amidino group,
an acyl group having 2 to 8 carbon atoms (e.g., alkylcarbonyl group),
a carbamoyl group,
a carbamoyl group mono- or di-substituted by an alkyl group having 1 to 4 carbon atoms,
a sulfamoyl group,
a sulfamoyl group mono- or di-substituted by an alkyl group having 1 to 4 carbon atoms,
a carboxyl group,
an alkoxycarbonyl group having 2 to 8 carbon atoms (e.g., methoxycarbonyl, ethoxycarbonyl),
a hydroxy group,
an alkoxy group having 1 to 6 carbon atoms (e.g., methoxy, ethoxy), which is optionally substituted by 1 to 3 halogen atoms (e.g., fluorine, chlorine, bromine, iodine and the like),
an alkenyloxy group having 2 to 5 carbon atoms (e.g., allyloxy), which is optionally substituted by 1 to 3 halogen atoms (e.g., fluorine, chlorine, bromine, iodine),
a cycloalkyloxy group having 3 to 7 carbon atoms (e.g., cyclohexyloxy),
an aralkyloxy group having 7 to 9 carbon atoms (e.g., benzyloxy), an aryloxy group having 6 to 14 carbon atoms (e.g., phenyloxy, naphthyloxy),
a mercapto group,
an alkylthio group having 1 to 6 carbon atoms (e.g., methylthio, ethylthio), which is optionally substituted by 1 to 3 halogen atoms (e.g., fluorine, chlorine, bromine, iodine), an aralkylthio group having 7 to 9 carbon atoms (e.g., benzylthio),
an arylthio group having 6 to 14 carbon atoms (e.g., phenylthio, naphthylthio),
a sulfo group,
a cyano group,
a nitro group,
a nitroso group,
a halogen atom (e.g., fluorine, chlorine, bromine, iodine) and the like can be mentioned. The number of the substituents is, for example, 1 to 3. When the ring has 2 or more substituents, respective substituents may be the same or different.

As the acyl group of the "optionally substituted acyl group", for example, -COR¹¹, -CO-OR¹¹, -SO₂R¹¹, -SOR¹¹, - PO(OR¹¹) (OR¹²), -CO-NR¹³R¹⁴, -CS-NR¹³R¹⁴, -SO₂-NR¹³R¹⁴ [wherein R¹¹ and R¹² are the same or different and each is a hydrogen atom, a hydrocarbon group or a heterocyclic group, an R¹³ and R¹⁴ are the same or different and each is a hydrogen atom, a hydrocarbon group or a heterocyclic group, or R¹³ and R¹⁴ are bonded to each other to form, together with the adjacent nitrogen atom, a nitrogen-containing heterocycle] and the like can be mentioned.

As the "hydrocarbon group" for R¹¹, R¹², R¹³ or R¹⁴, for example, an aliphatic hydrocarbon group, an alicyclic hydrocarbon group, an alicyclic-aliphatic hydrocarbon group, an aromatic-aliphatic hydrocarbon group, an aromatic hydrocarbon group and the like can be mentioned. The carbon atom number of these hydrocarbon groups is preferably 1 to 15.

As used herein, as the aliphatic hydrocarbon group, alicyclic hydrocarbon group and aromatic hydrocarbon group, those exemplified as the substituents which the aforementioned "optionally substituted aromatic ring" for Ar optionally has, can be mentioned.

As the alicyclic-aliphatic hydrocarbon group, for example, a group wherein the aforementioned alicyclic hydrocarbon group is bonded to the aforementioned aliphatic hydrocarbon group (e.g., a cycloalkyl-alkyl group, a cycloalkenyl-alkyl group) can be mentioned. Of these, an alicyclic-aliphatic hydrocarbon group having 4 to 9 carbon atoms is preferable. As preferable examples of the alicyclic-aliphatic hydrocarbon group, cyclopropylmethyl, cyclopropylethyl, cyclobutylmethyl, cyclopentylmethyl, 2-cyclopentenylmethyl, 3-cyclopentenylmethyl, cyclohexylmethyl, 2-cyclohexenylmethyl, 3-cyclohexenylmethyl, cyclohexylethyl, cyclohexylpropyl, cycloheptylmethyl, cycloheptylethyl and the like can be mentioned.

As the aromatic-aliphatic hydrocarbon group, for example, an aromatic-aliphatic hydrocarbon group having 7 to 13 carbon atoms (e.g., an aralkyl group having 7 to 13 carbon atoms, an arylalkenyl group having 8 to 13 carbon atoms) and the like can be mentioned. As preferable examples of the aromatic-aliphatic hydrocarbon group, a phenylalkyl having 7 to 9 carbon atoms (e.g., benzyl, phenethyl, 1-phenylethyl, 1-phenylpropyl, 2-phenylpropyl, 3-phenylpropyl etc.); a naphthylalkyl having 11 to 13 carbon atoms (e.g., α-naphthylmethyl, α-naphthylethyl, β-naphthylmethyl, β-naphthylethyl etc.); a phenylalkenyl having 8 to 10 carbon atoms (e.g., styryl etc.); a naphthylalkenyl having 12 to 13 carbon atoms (e.g., 2-(2-naphthylvinyl) etc.) and the like can be mentioned.

As the hydrocarbon group, an alkyl group having 1 to 10 carbon atoms, an alkenyl group having 2 to 10 carbon atoms, a cycloalkyl group having 3 to 10 carbon atoms, a cycloalkenyl group having 3 to 10 carbon atoms, an aryl group having 6 to 14 carbon atoms and the like are preferable.

As the "heterocyclic group" for R¹¹, R¹², R¹³ or R¹⁴, those exemplified as the substituents which the aforementioned "optionally substituted aromatic ring" for Ar optionally has, can be mentioned.

As the "nitrogen-containing heterocycle", which is formed, together with the adjacent nitrogen atom, by R¹³ and R¹⁴ bonded to each other, for example, a 5 to 7-membered nitrogen-containing heterocycle containing, as a ring-constituting atom besides carbon atoms, at least one nitrogen atom and optionally further containing 1 or 2 heteroatoms selected from an oxygen atom, a sulfur atom and a nitrogen atom can be mentioned. As preferable examples of the nitrogen-containing heterocycle, pyrrolidine, imidazolidine, pyrazolidine, piperidine, piperazine, morpholine, thiomorpholine and the like can be mentioned.

The acyl group optionally has 1 to 3 substituents at substitutable positions. As such substituents, for example,
a C₁₋₆ alkyl group (e.g., methyl, ethyl) optionally substituted by 1 to 3 halogen atoms (e.g., fluorine, chlorine, iodine),
a C₁₋₆ alkoxy group (e.g., methoxy, ethoxy) optionally substituted by 1 to 3 halogen atoms (e.g., fluorine, chlorine, bromine, iodine),
a halogen atom (e.g., fluorine, chlorine, bromine, iodine),
a nitro group,
a hydroxy group,
an amino group
and the like can be mentioned. When the ring has 2 or more substituents, respective substituents may be the same or different.

As preferable examples of the acyl group, for example,
a formyl group,
a carboxyl group,
a carbamoyl group,
a thiocarbamoyl group,
a C₁₋₁₀ alkyl-carbonyl group (e.g., acetyl, propionyl, butyryl, isobutyryl, valeryl, isovaleryl, pivaloyl, hexanoyl, heptanoyl, octanoyl),
a C₂₋₁₀ alkenyl-carbonyl group (e.g., crotonyl),
a C₃₋₁₀ cycloalkyl-carbonyl group (e.g., cyclobutanecarbonyl, cyclopentanecarbonyl, cyclohexanecarbonyl, cycloheptanecarbonyl),
a C₃₋₁₀ cycloalkenyl-carbonyl group (e.g., 2-cyclohexenecarbonyl),
a C₆₋₁₄ aryl-carbonyl group (e.g., benzoyl, 1-naphthoyl, 2-naphthoyl),
a C₇₋₁₃ aralkyl-carbonyl group (e.g., benzylcarbonyl, phenethylcarbonyl),
an aromatic heterocyclylcarbonyl group (e.g., nicotinoyl, isonicotinoyl),
a non-aromatic heterocyclylcarbonyl group (e.g., pyrrolidinylcarbonyl, piperidinocarbonyl),
a C₁₋₆ alkoxy-carbonyl group (e.g., methoxycarbonyl, ethoxycarbonyl, propoxycarbonyl, butoxycarbonyl, tert-butoxycarbonyl),
a C₆₋₁₄ aryloxy-carbonyl group (e.g., phenyloxycarbonyl, naphthyloxycarbonyl),
a C₇₋₁₃ aralkyloxy-carbonyl group (e.g., benzyloxycarbonyl, phenethyloxycarbonyl),
a mono- or di-(a C₁₋₆ alkyl optionally having 1 to 3 substituents selected from a halogen atom and a C₁₋₆ alkoxy-carbonyl)-carbamoyl group (e.g., methylcarbamoyl, ethylcarbamoyl, dimethylcarbamoyl, diethylcarbamoyl, ethylmethylcarbamoyl, propylcarbamoyl, trifluoroethylcarbamoyl),
a mono- or di-(a C₁₋₆ alkyl optionally substituted by 1 to 3 halogens)-thiocarbamoyl group (e.g., methylthiocarbamoyl, ethylthiocarbamoyl),
a C₆₋₁₄ aryl-carbamoyl group (e.g., phenylcarbamoyl),
a C₃₋₁₀ cycloalkyl-carbamoyl group (e.g., cyclopropylcarbamoyl), a C₇₋₁₃ aralkyl-carbamoyl group (e.g., benzylcarbamoyl),
a C₁₋₆ alkoxy-carbamoyl group (e.g., methoxycarbamoyl),
a C₁₋₁₀ alkylsulfinyl group (e.g., methylsulfinyl, ethylsulfinyl), a C₁₋₁₀ alkylsulfonyl group (e.g., methylsulfonyl, ethylsulfonyl), a C₆₋₁₄ aryl sulfonyl group (e.g., phenylsulfonyl),
a mono- or di-C₁₋₁₀ alkylphosphono group (e.g., dimethylphosphono, diethylphosphono, diisopropylphosphono, dibutylphosphono),
a mono- or di-(a C₁₋₆ alkyl optionally substituted by 1 to 3 halogens)-sulfamoyl group (e.g., methylsulfamoyl, ethylsulfamoyl)
and the like can be mentioned.

As the "optionally substituted amino group", for example, an amino group optionally mono- or di-substituted by an alkyl group having 1 to 10 carbon atoms, an alkenyl group having 2 to 10 carbon atoms, a cycloalkyl group having 3 to 10 carbon atoms, a cycloalkenyl group having 3 to 10 carbon atoms, an aryl group having 6 to 14 carbon atoms, an acyl group having 1 to 13 carbon atoms and the like can be mentioned. As used herein, as the "alkyl group having 1 to 10 carbon atoms", "alkenyl group having 2 to 10 carbon atoms", "cycloalkyl group having 3 to 10 carbon atoms", "cycloalkenyl group having 3 to 10 carbon atoms" and "aryl group having 6 to 14 carbon atoms", those exemplified as the substituents which the aforementioned "optionally substituted aromatic ring" for Ar optionally has, can be mentioned. As the "acyl group having 1 to 13 carbon atoms", an acyl group having 1 to 13 carbon atoms, from among the acyl groups exemplified as the substituents which the aforementioned "optionally substituted aromatic ring" for Ar optionally has, can be mentioned, and an alkylcarbonyl group having 2 to 10 carbon atoms, an arylcarbonyl group having 7 to 13 carbon atoms and the like are preferable.

As preferable examples of the substituted amino group, methylamino, dimethylamino, ethylamino, diethylamino, propylamino, dibutylamino, diallylamino, cyclohexylamino, acetylamino, propionylamino, benzoylamino, phenylamino, N-methyl-N-phenylamino and the like can be mentioned.

As the "optionally substituted hydroxy group", for example, a hydroxy group optionally substituted by an alkyl group having 1 to 10 carbon atoms, a cycloalkyl group having 3 to 10 carbon atoms, an alkenyl group having 2 to 10 carbon atoms, a cycloalkenyl group having 3 to 10 carbon atoms, an aralkyl group having 7 to 13 carbon atoms, an acyl group having 1 to 13 carbon atoms, an aryl group having 6 to 14 carbon atoms and the like, each of which is optionally substituted, can be mentioned. As used herein, as the "alkyl group having 1 to 10 carbon atoms", "cycloalkyl group having 3 to 10 carbon atoms", "alkenyl group having 2 to 10 carbon atoms", "cycloalkenyl group having 3 to 10 carbon atoms" and "aryl group having 6 to 14 carbon atoms", those exemplified as the substituents which the aforementioned "optionally substituted aromatic ring" for Ar optionally has, can be mentioned. As the "aralkyl group having 7 to 13 carbon atoms", those exemplified as the aforementioned "hydrocarbon group" for R¹¹ can be mentioned. As the "acyl group having 1 to 13 carbon atoms", an acyl group having 1 to 13 carbon atoms, from among the acyl groups exemplified as the substituents which the aforementioned "optionally substituted aromatic ring" for Ar optionally has, can be mentioned.

As the substituents which the aforementioned alkyl group having 1 to 10 carbon atoms, cycloalkyl group having 3 to 10 carbon atoms, alkenyl group having 2 to 10 carbon atoms, cycloalkenyl group having 3 to 10 carbon atoms, aralkyl group having 7 to 13 carbon atoms, acyl group having 1 to 13 carbon atoms and aryl group having 6 to 14 carbon atoms optionally have, for example,
a halogen atom (e.g., fluorine, chlorine, bromine, iodine),
a C₁₋₆ alkoxy group (e.g., methoxy, ethoxy) optionally substituted by 1 to 3 halogen atoms (e.g., fluorine, chlorine, bromine, iodine),
a hydroxy group,
a nitro group,
an amino group
and the like can be mentioned. The number of the substituents is, for example, 1 or 2. When the ring has 2 or more substituents, respective substituents may be the same or different.

As the substituted hydroxy group, for example, an alkoxy group having 1 to 10 carbon atoms, a cycloalkyloxy group having 3 to 10 carbon atoms, an alkenyloxy group having 2 to 10 carbon atoms, a cycloalkenyloxy group having 3 to 10 carbon atoms, an aralkyloxy group having 7 to 13 carbon atoms, an acyloxy group having 1 to 13 carbon atoms, an aryloxy group having 6 to 14 carbon atoms and the like, each of which is optionally substituted, can be mentioned.

As the alkoxy group having 1 to 10 carbon atoms, for example, methoxy, ethoxy, propoxy, isopropoxy, butoxy, isobutoxy, sec-butoxy, tert-butoxy, pentyloxy, isopentyloxy, neopentyloxy, hexyloxy, heptyloxy, nonyloxy and the like can be mentioned.

As the cycloalkyloxy group having 3 to 10 carbon atoms, for example, cyclobutoxy, cyclopentyloxy, cyclohexyloxy and the like can be mentioned.

As the alkenyloxy group having 2 to 10 carbon atoms, for example, allyl(allyl)oxy, crotyloxy, 2-pentenyloxy, 3-hexenyloxy and the like can be mentioned.

As the cycloalkenyloxy group having 3 to 10 carbon atoms, for example, 2-cyclopentenylmethoxy, 2-cyclohexenylmethoxy and the like can be mentioned.

As the aralkyloxy group having 7 to 13 carbon atoms, an aralkyloxy group having 7 to 10 carbon atoms is preferable, and, for example, a phenyl-C₁₋₄ alkyloxy group (e.g., benzyloxy, phenethyloxy) and the like can be mentioned.

As the acyloxy group having 1 to 13 carbon atoms, an acyloxy group having 2 to 13 carbon atoms is preferable, and an alkylcarbonyloxy group having 2 to 4 carbon atoms (e.g., acetyloxy, propionyloxy, butyryloxy, isobutyryloxy) and the like are particularly preferable.

As the aryloxy group having 6 to 14 carbon atoms, for example, phenoxy, naphthyloxy and the like can be mentioned.

The above-mentioned alkoxy group having 1 to 10 carbon atoms, cycloalkyloxy group having 3 to 10 carbon atoms, alkenyloxy group having 2 to 10 carbon atoms, cycloalkenyloxy group having 3 to 10 carbon atoms, aralkyloxy group having 7 to 13 carbon atoms, acyloxy group having 1 to 13 carbon atoms and aryloxy group having 6 to 14 carbon atoms optionally have 1 or 2 substituents at substitutable positions. When the ring has 2 or more substituents, respective substituents may be the same or different. As such substituents, for example,
a halogen atom (e.g., fluorine, chlorine, bromine, iodine),
a C₁₋₆ alkoxy group (e. g. , methoxy, ethoxy) optionally substituted by 1 to 3 halogen atoms (e.g., fluorine, chlorine, bromine, iodine),
a hydroxy group,
a nitro group,
an amino group
and the like can be mentioned.

As the "optionally substituted mercapto group", for example, a mercapto group optionally substituted by an alkyl group having 1 to 10 carbon atoms, a cycloalkyl group having 3 to 10 carbon atoms, an aralkyl group having 7 to 13 carbon atoms, an acyl group having 1 to 13 carbon atoms, an aryl group having 6 to 14 carbon atoms, a heteroaryl group and the like, each of which is optionally substituted, can be mentioned. As used herein, as the "alkyl group having 1 to 10 carbon atoms", "cycloalkyl group having 3 to 10 carbon atoms" and "aryl group having 6 to 14 carbon atoms", those exemplified as the substituents which the aforementioned "optionally substituted aromatic ring" for Ar optionally has, can be mentioned. As the "aralkyl group having 7 to 13 carbon atoms", those exemplified as the aforementioned "hydrocarbon group" for R¹¹ can be mentioned. As the "acyl group having 1 to 13 carbon atoms", an acyl group having 1 to 13 carbon atoms, from among the acyl groups exemplified as the substituents which the aforementioned "optionally substituted aromatic ring" for Ar optionally has, can be mentioned. As preferable examples of the heteroaryl group, pyridyl (e.g., 2-pyridyl, 3-pyridyl), imidazolyl (e.g., 2-imidazolyl), triazolyl (e.g., 1,2,4-triazol-5-yl) and the like can be mentioned.

As the substituents which the aforementioned alkyl group having 1 to 10 carbon atoms, cycloalkyl group having 3 to 10 carbon atoms, aralkyl group having 7 to 13 carbon atoms, acyl group having 1 to 13 carbon atoms, aryl group having 6 to 14 carbon atoms and heteroaryl group optionally have, for example,
a halogen atom (e.g., fluorine, chlorine, bromine, iodine),
a C₁₋₆ alkoxy group (e.g., methoxy, ethoxy) optionally substituted by 1 to 3 halogen atoms (e.g., fluorine, chlorine, bromine, iodine),
a hydroxy group,
a nitro group,
an amino group
and the like can be mentioned. The number of the substituents is, for example, 1 or 2. When the ring has 2 or more substituents, respective substituents may be the same or different.

As the substituted mercapto group, for example, an alkylthio group having 1 to 10 carbon atoms, a cycloalkylthio group having 3 to 10 carbon atoms, an aralkylthio group having 7 to 13 carbon atoms, an acylthio group having 1 to 13 carbon atoms, an arylthio group having 6 to 14 carbon atoms, a heteroarylthio group and the like can be mentioned.

As the alkylthio group having 1 to 10 carbon atoms, for example, methylthio, ethylthio, propylthio, isopropylthio, butylthio, isobutylthio, sec-butylthio, tert-butylthio, pentylthio, isopentylthio, neopentylthio, hexylthio, heptylthio, nonylthio and the like can be mentioned.

As preferable examples of the cycloalkylthio group having 3 to 10 carbon atoms, for example, cyclobutylthio, cyclopentylthio, cyclohexylthio and the like can be mentioned.

As the aralkylthio group having 7 to 13 carbon atoms, an aralkylthio group having 7 to 10 carbon atoms is preferable, and, for example, a phenyl-C₁₋₄ alkylthio (e.g., benzylthio, phenethylthio) and the like can be mentioned.

As the acylthio group having 1 to 13 carbon atoms, an alkylcarbonylthio group having 2 to 4 carbon atoms (e.g., acetylthio, propionylthio, butyrylthio, isobutyrylthio) and the like are preferable.

As the arylthio group having 6 to 14 carbon atoms, for example, phenylthio, naphthylthio and the like can be mentioned.

As preferable examples of the heteroarylthio group, pyridylthio (e.g., 2-pyridylthio, 3-pyridylthio), imidazolylthio (e.g., 2-imidazolylthio), triazolylthio (e.g., 1,2,4-triazol-5-ylthio) and the like can be mentioned.

As the substituents of Ar,
1) a halogen atom (e.g., fluorine, chlorine, bromine, iodine);
2) an alkyl group having 1 to 10 (preferably 1 to 4) carbon atoms and optionally having 1 to 3 substituents selected from an alkoxy group having 1 to 6 carbon atoms, which is optionally substituted by 1 to 3 halogen atoms (e.g., fluorine, chlorine, bromine, iodine), a halogen atom (e.g., fluorine, chlorine, bromine, iodine), a nitro group, a hydroxy group and an amino group;
3) an alkoxy group having 1 to 10 (preferably 1 to 4) carbon atoms and optionally having 1 to 3 substituents selected from an alkoxy group having 1 to 6 carbon atoms, which is optionally substituted by 1 to 3 halogen atoms (e.g., fluorine, chlorine, bromine, iodine), a halogen atom (e.g., fluorine, chlorine, bromine, iodine), a nitro group, a hydroxy group and an amino group;
4) an aryl group having 6 to 14 carbon atoms (preferably phenyl and the like) and optionally having 1 to 3 substituents selected from an alkyl group having 1 to 6 carbon atoms, which is optionally substituted by 1 to 3 halogen atoms (e.g., fluorine, chlorine, bromine, iodine), an alkoxy group having 1 to 6 carbon atoms, which is optionally substituted by 1 to 3 halogen atoms (e.g., fluorine, chlorine, bromine, iodine), a halogen atom (e.g., fluorine, chlorine, bromine, iodine), a nitro group, a hydroxy group and an amino group;
   and the like are preferable.

As the substituents of Ar,
a halogen atom (preferably fluorine, chlorine),
an alkyl group having 1 to 4 carbon atoms (preferably methyl), which is optionally substituted by 1 to 3 halogen atoms (preferably fluorine),
an alkoxy group having 1 to 4 carbon atoms (preferably methoxy, ethoxy, isopropoxy),
phenyl
and the like are more preferable.

The number of the substituents of Ar is preferably 1 to 3, more preferably 1 or 2.

Ar is preferably a benzene ring, a 5- or 6-membered aromatic heterocycle (preferably pyridine, oxazole and the like) or a 9- to 14-membered fused aromatic heterocycle (preferably quinoline and the like), each of which optionally has 1 to 3 substituents selected from
1) a halogen atom (e.g., fluorine, chlorine, bromine, iodine);
2) an alkyl group having 1 to 10 (preferably 1 to 4) carbon atoms and optionally having 1 to 3 substituents selected from an alkoxy group having 1 to 6 carbon atoms, which is optionally substituted by 1 to 3 halogen atoms (e.g., fluorine, chlorine, bromine, iodine), a halogen atom (e.g., fluorine, chlorine, bromine, iodine), a nitro group, a hydroxy group and an amino group;
3) an alkoxy group having 1 to 10 (preferably 1 to 4) carbon atoms and optionally having 1 to 3 substituents selected from an alkoxy group having 1 to 6 carbon atoms, which is optionally substituted by 1 to 3 halogen atoms (e.g., fluorine, chlorine, bromine, iodine), a halogen atom (e.g., fluorine, chlorine, bromine, iodine), a nitro group, a hydroxy group and an amino group; and
4) an aryl group having 6 to 14 carbon atoms (preferably phenyl and the like) and optionally having 1 to 3 substituents selected from an alkyl group having 1 to 6 carbon atoms, which is optionally substituted by 1 to 3 halogen atoms (e.g., fluorine, chlorine, bromine, iodine), an alkoxy group having 1 to 6 carbon atoms, which is optionally substituted by 1 to 3 halogen atoms (e.g., fluorine, chlorine, bromine, iodine), a halogen atom (e.g., fluorine, chlorine, bromine, iodine), a nitro group, a hydroxy group and an amino group.

Ar is more preferably
a benzene ring, a pyridine ring, an oxazole ring or a quinoline ring, each of which optionally has 1 or 2 substituents selected from
a halogen atom (preferably fluorine, chlorine),
an alkyl group having 1 to 4 carbon atoms (preferably methyl), which is optionally substituted by 1 to 3 halogen atoms (preferably fluorine),
an alkoxy group having 1 to 4 carbon atoms (preferably methoxy, ethoxy, isopropoxy), and
phenyl.

Xa, Xc, Ya, Yc, Z¹ and Z² are the same or different and each is a bond, an oxygen atom, a sulfur atom, -CO-, -CS-, - CR³(OR⁴)-, -NR⁵-, -SO-, -SO₂- -CONR⁶- or -NR⁶CO- (wherein R³ is a hydrogen atom or an optionally substituted hydrocarbon group, R⁴ is a hydrogen atom or a hydroxyl-protecting group, R⁵ is a hydrogen atom, an optionally substituted hydrocarbon group or an amino-protecting group, and R⁶ is a hydrogen atom or an optionally substituted hydrocarbon group).

As used herein, as the "hydrocarbon group" of the "optionally substituted hydrocarbon group" for R³, R⁵ or R⁶, those exemplified as the aforementioned R¹¹ can be mentioned. As the hydrocarbon group, an optionally substituted alkyl group having 1 to 4 carbon atoms, for example, methyl, ethyl, propyl, isopropyl, butyl, isobutyl, sec-butyl, tert-butyl and the like are preferable.

The hydrocarbon group optionally has 1 to 3 substituents at substitutable positions. As such substituents, for example, a halogen atom (e.g., fluorine, chlorine, bromine, iodine),
an alkoxy group having 1 to 4 carbon atoms (e.g., methoxy, ethoxy, propoxy, isopropoxy, butoxy, isobutoxy, sec-butoxy, tert-butoxy),
a hydroxy group,
a nitro group,
an amino group, an acyl group having 1 to 4 carbon atoms (e.g., formyl; an alkylcarbonyl group having 2 to 4 carbon atoms, such as acetyl, propionyl and the like),
an aryl group having 6 to 14 carbon atoms (e.g., phenyl),
a non-aromatic heterocyclic group (e.g., pyrrolidinyl) optionally substituted by 1 to 3 oxo groups
and the like can be mentioned.

As the "hydroxyl-protecting group" for R⁴, for example, a C₁₋₆ alkyl group (e.g., methyl, ethyl, propyl, isopropyl, butyl, tert-butyl), a phenyl group, a trityl group, a C₇₋₁₀ aralkyl group (e.g., benzyl), a formyl group, a C₁₋₆ alkyl-carbonyl group (e.g., acetyl, propionyl), a benzoyl group, a C₇₋₁₀ aralkyl-carbonyl group (e.g., benzylcarbonyl), a 2-tetrahydropyranyl group, a 2-tetrahydrofuranyl group, a silyl group (e.g., trimethylsilyl, triethylsilyl, dimethylphenylsilyl, tert-butyldimethylsilyl, tert-butyldiethylsilyl), a C₂₋₆ alkenyl group (e.g., 1-allyl) and the like can be mentioned. These groups are optionally substituted by 1 to 3 substituents selected from a halogen atom (e.g., fluorine, chlorine, bromine, iodine), a C₁₋₆ alkyl group (e.g., methyl, ethyl, propyl), a C₁₋₆ alkoxy group (e.g., methoxy, ethoxy, propoxy) and a nitro group. When the ring has 2 or more substituents, respective substituents may be the same or different.

As the "amino-protecting group" for R⁵, for example, a formyl group, a C₁₋₆ alkyl-carbonyl group (e.g., acetyl, propionyl), a C₁₋₆ alkoxy-carbonyl group (e.g., methoxycarbonyl, ethoxycarbonyl, tert-butoxycarbonyl), a benzoyl group, a C₇₋₁₀ aralkyl-carbonyl group (e.g., benzylcarbonyl), a C₇₋₁₄ aralkyloxy-carbonyl group (e.g., benzyloxycarbonyl, 9-fluorenylmethoxycarbonyl), a trityl group, a phthaloyl group, a N,N-dimethylaminomethylene group, a silyl group (e.g., trimethylsilyl, triethylsilyl, dimethylphenylsilyl, tert-butyldimethylsilyl, tert-butyldiethylsilyl), a C₂₋₆ alkenyl group (e.g., 1-allyl and the like) and the like can be mentioned. These groups are optionally substituted by 1 to 3 substituents selected from a halogen atom (e.g., fluorine, chlorine, bromine, iodine), a C₁₋₆ alkoxy group (e.g., methoxy, ethoxy, propoxy) and a nitro group. When the ring has 2 or more substituents, respective substituents may be the same or different.

Xa is preferably a bond or an oxygen atom, more preferably a bond.

Xc is preferably a bond, an oxygen atom, -CO- or -NR⁶CO-(wherein R⁶ is a hydrogen atom or an alkyl group having 1 to 4 carbon atoms), more preferably a bond, an oxygen atom, -CO- or - NHCO-, particularly preferably a bond or an oxygen atom.

Ya is preferably a bond, an oxygen atom, a sulfur atom, - NR⁵- (wherein R⁵ is an alkyl group having 1 to 4 carbon atoms), - SO₂-, -CONR⁶- or -NR⁶CO- (wherein R⁶ is a hydrogen atom or an alkyl group having 1 to 4 carbon atoms), more preferably a bond, an oxygen atom, a sulfur atom, -N(CH₃)-, -CON(CH₂CH₃)-, -SO₂- or - N(CH₂CH₃)CO-, particularly preferably a bond.

Yc is preferably a bond or an oxygen atom.

Z¹ is preferably a bond or an oxygen atom, more preferably a bond.

Z² is preferably an oxygen atom.

Xb and Yb are the same or different and each is a bond or a divalent hydrocarbon group having 1 to 20 carbon atoms.

As the "divalent hydrocarbon group having 1 to 20 carbon atoms" for Xb or Yb, for example, a "divalent acyclic hydrocarbon group", a "divalent cyclic hydrocarbon group", and a divalent group wherein one or more "divalent acyclic hydrocarbon groups" and one or more "divalent cyclic hydrocarbon groups" are bonded can be mentioned.

As used herein, as the "divalent acyclic hydrocarbon group", for example, an alkylene group having 1 to 20 carbon atoms, an alkenylene group having 2 to 20 carbon atoms, an alkynylene group having 2 to 20 carbon atoms and the like can be mentioned.

As the "divalent cyclic hydrocarbon group", a divalent group obtained by removing any two hydrogen atoms from a cycloalkane having 3 to 20 carbon atoms, a cycloalkene having 5 to 20 carbon atoms or an aromatic hydrocarbon having 6 to 18 carbon atoms (e.g., benzene, naphthalene, anthracene etc. containing a partially hydrogenated aromatic hydrocarbon such as indene and the like) and the like can be mentioned. As specific examples, 1,2-cyclopropylene, 1,2-cyclobutylene, 1,3-cyclobutylene, 1,2-cyclopentylene, 1,3-cyclopentylene, 1,2-cyclohexylene, 1,3-cyclohexylene, 1,4-cyclohexylene, 1,2-cycloheptylene, 1,3-cycloheptylene, 1,4-cycloheptylene, 3-cyclohexen-1,4-ylene, 3-cyclohexen-1,2-ylene, 2,5-cyclohexadien-1,4-ylene, 1,2-phenylene, 1,3-phenylene, 1,4-phenylene, 1,4-naphthylene, 1,6-naphthylene, 2,6-naphthylene, 2,7-naphthylene, 1,5-indenylene, 2,5-indenylene and the like can be mentioned.

As the "divalent hydrocarbon group having 1 to 20 carbon atoms", a divalent hydrocarbon group having 1 to 6 carbon atoms is preferable. Of these,
(1) a C₁₋₆ alkylene group (e.g., -CH₂-, -(CH₂)₂-, -(CH₂)₃-, -(CH₂)₄-, - (CH₂)₅-, - (CH₂)₆-, -CH (CH₃) -, -C(CH₃)₂-, - (CH (CH₃))₂-, - (CH₂)₂C(CH₃)₂-, -(CH₂)₃C(CH₃)₂- and the like);
(2) a C₂₋₆ alkenylene group (e.g., -CH=CH-, -CH₂-CH=CH-, -C(CH₃)₂-CH=CH-, -CH₂-CH=CH-CH₂-, -CH₂-CH₂-CH=CH-, -CH=CH-CH=CH-, -CH=CH-CH₂-CH₂-CH₂- and the like);
(3) a C₂₋₆ alkynylene group (e.g., -CΞC-, -CH₂-CΞC-, -CH₂-CΞC-CH₂-CH₂- and the like)
   and the like are preferable.

Xb is preferably a bond or a divalent hydrocarbon group having 1 to 6 carbon atoms, more preferably a bond, a C₁₋₆ alkylene group or a C₂₋₆ alkenylene group, further more preferably a bond, -CH₂-, -(CH₂)₂-, -CH(CH₃)-, -(CH₂)₃-, -(CH₂)₄-, -CH=CH- and the like, particularly preferably a bond, -CH₂-, - CH(CH₃)- or the like.

Yb is preferably a bond or a divalent hydrocarbon group having 1 to 6 carbon atoms (preferably a C₁₋₆ alkylene group), more preferably a bond, -CH₂-, -(CH₂)₂- or the like, particularly preferably a bond.

As a preferable combination of Xa, Xb and Xc, a combination of Xa being a bond or an oxygen atom, Xb being a bond or a divalent hydrocarbon group having 1 to 6 carbon atoms (preferably a C₁₋₆ alkylene group), and Xc being a bond, an oxygen atom, -CO- or -NR⁶CO- (wherein R⁶ is a hydrogen atom or an alkyl group having 1 to 4 carbon atoms), can be mentioned.

As a more preferable combination of Xa, Xb and Xc, "a combination of Xa being a bond, Xb being a bond or a divalent hydrocarbon group having 1 to 6 carbon atoms (preferably a C₁₋₆ alkylene group (preferably -CH₂-, -CH(CH₃)-)), and Xc being an oxygen atom",
"a combination of Xa being a bond, Xb being a divalent hydrocarbon group having 1 to 6 carbon atoms (preferably a C₁₋₆ alkylene group (preferably -CH₂-, -CH(CH₃)-)), and Xc being a bond", and
"a combination of Xa being an oxygen atom, Xb being a divalent hydrocarbon group having 1 to 6 carbon atoms (preferably a C₁₋₆ alkylene group (preferably -CH₂-, -CH(CH₃)-)), and Xc being a bond"
can be mentioned.

As a preferable combination of Ya, Yb and Yc, a combination of Ya being a bond, an oxygen atom, a sulfur atom, - NR⁵- (wherein R⁵ is an alkyl group having 1 to 4 carbon atoms), - SO₂-, -CONR⁶- or -NR⁶CO- (wherein R⁶ is a hydrogen atom or an alkyl group having 1 to 4 carbon atoms), Yb being a bond or a divalent hydrocarbon group having 1 to 6 carbon atoms (preferably a C₁₋₆ alkylene group), and Yc being a bond or an oxygen atom, can be mentioned. More preferably, Ya and Yb are each a bond.

As a more preferable combination of Ya, Yb and Yc, a combination of Ya being a bond, an oxygen atom, a sulfur atom, - N(CH₃)-, -CON(CH₂CH₃)-, -SO₂- or -N(CH₂CH₃)CO-, Yb being a bond or a C₁₋₆ alkylene group (preferably -CH₂-, -(CH₂)₂-), and Yc being a bond or an oxygen atom, can be mentioned. More preferably, Ya and Yb are each a bond.

R¹ is an optionally substituted hydrocarbon group.

As the "optionally substituted hydrocarbon group" for R¹, those exemplified as the aforementioned R³ can be mentioned.

R¹ is preferably an optionally substituted alkyl group having 1 to 10 (preferably 1 to 4) carbon atoms, more preferably an alkyl group having 1 to 10 (preferably 1 to 4) carbon atoms (preferably methyl, ethyl, propyl, isopropyl, butyl, isobutyl) and optionally having 1 to 3 substituents selected from a halogen atom (e.g., fluorine, chlorine, bromine, iodine),
an alkoxy group having 1 to 4 carbon atoms (e.g., methoxy, ethoxy, propoxy, isopropoxy, butoxy, isobutoxy, sec-butoxy, tert-butoxy),
a hydroxy group,
a nitro group,
an amino group, an acyl group having 1 to 4 carbon atoms (e.g., formyl; an alkylcarbonyl group having 2 to 4 carbon atoms, such as acetyl, propionyl and the like),
an aryl group having 6 to 14 carbon atoms (e.g., phenyl), and
a non-aromatic heterocyclic group (e.g., pyrrolidinyl) optionally substituted by 1 to 3 oxo groups.

R¹ is more preferably an alkyl group having 1 to 4 carbon atoms and optionally having 1 to 3 substituents selected from an aryl group having 6 to 14 carbon atoms (e.g., phenyl), and a non-aromatic heterocyclic group (e.g., pyrrolidinyl) optionally substituted by 1 to 3 oxo groups, particularly preferably an alkyl group having 1 to 4 carbon atoms.

Ring A is an optionally further substituted aromatic ring, provided that the ring should not be benzimidazole.

As the "aromatic ring" of the "optionally further substituted aromatic ring" for ring A, those (excluding benzimidazole) exemplified as the "aromatic ring" of the aforementioned "optionally substituted aromatic ring" for Ar can be mentioned. Of these, a benzene ring, a 5- or 6-membered aromatic heterocycle (e.g., pyridine, pyrimidine, pyrazole, thiazole) and the like are preferable, a benzene ring, a pyridine ring, a pyrimidine ring, a pyrazole ring, a thiazole ring and the like are more preferable, and a benzene ring is particularly preferable.

Ring A optionally further has, besides Xc, Yc and Z¹, 1 to 3, preferably 1 or 2, substituents at substitutable positions. As such substituents, for example, an "optionally substituted aliphatic hydrocarbon group (preferably an alkyl group)", an "optionally substituted hydroxy group", a "halogen atom", an "optionally substituted acyl group", a "nitro group", an "optionally substituted amino group" and the like can be mentioned. As these substituents, those exemplified as the substituents which the aforementioned "optionally substituted aromatic ring" for Ar optionally has, can be mentioned. When the ring has 2 or more substituents, respective substituents may be the same or different.

As the substituents of ring A,
an alkyl group having 1 to 4 carbon atoms,
a hydroxy group,
an alkoxy group having 1 to 4 carbon atoms (preferably methoxy), an aralkyloxy group having 7 to 10 carbon atoms, and a halogen atom
are preferable.

Ring A is preferably a benzene ring or a 5- or 6-membered aromatic heterocycle (preferably pyridine, pyrimidine, pyrazole, thiazole and the like), each of which optionally further has 1 or 2 substituents selected from an alkyl group having 1 to 4 carbon atoms,
a hydroxy group,
an alkoxy group having 1 to 4 carbon atoms (preferably methoxy), an aralkyloxy group having 7 to 10 carbon atoms, and
a halogen atom.

Ring A is more preferably a benzene ring, a pyridine ring, a pyrimidine ring, a pyrazole ring or a thiazole ring
(preferably a benzene ring).

When ring A is a benzene ring, then Xc and Yc are preferably bonded at meta-positions on the benzene ring.

That is, 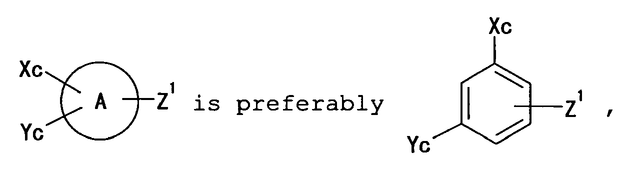 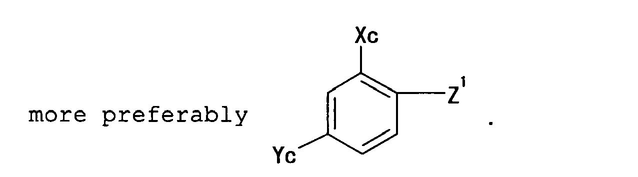 n is an integer of 1 to 8. n is preferably an integer of 1 to 4, more preferably an integer of 1 to 3.

As a preferable combination of Z¹, n and Z², a combination of Z¹ being a bond, n being an integer of 1 to 4, and Z² being an oxygen atom can be mentioned.

Ring B is an optionally further substituted aromatic ring, provided that the ring should not be oxazole.

As the "aromatic ring" of the "optionally further substituted aromatic ring" for ring B, those (excluding oxazole) exemplified as the "aromatic ring" of the aforementioned "optionally substituted aromatic ring" for Ar can be mentioned. Of these, a benzene ring, a fused aromatic hydrocarbon ring having 9 to 14 carbon atoms (e.g., naphthalene), a 5- or 6-membered aromatic heterocycle (excluding oxazole, e.g., pyridine, pyrazole, thiazole) and the like are preferable, a benzene ring, a naphthalene ring, a pyridine ring, a pyrazole ring a thiazole ring, and the like are more preferable, and a pyrazole ring is particularly preferable.

Ring B optionally further has, besides Z² and W, 1 to 3, preferably 1 or 2, substituents at substitutable positions. As such substituents, for example, a "halogen atom", a "nitro group", an "optionally substituted aliphatic hydrocarbon group", an "optionally substituted alicyclic hydrocarbon group", an "optionally substituted aromatic hydrocarbon group", an "optionally substituted heterocyclic group", an "optionally substituted acyl group", an "optionally substituted amino group", an "optionally substituted hydroxy group", an "optionally substituted mercapto group" and the like can be mentioned. As these substituents, those exemplified as the substituents which the aforementioned "optionally substituted aromatic ring" for Ar optionally has, can be mentioned. When the ring has 2 or more substituents, respective substituents may be the same or different.

As the substituents of ring B,
an alkyl group having 1 to 10 (preferably 1 to 4) carbon atoms (e.g., methyl, ethyl, isopropyl, isobutyl),
an aryl group having 6 to 14 carbon atoms-alkyl group having 1 to 10 (preferably 1 to 4) carbon atoms (e.g., benzyl), an alkoxy group having 1 to 10 (preferably 1 to 4) carbon atoms (e.g., methoxy, ethoxy),
an aryl group having 6 to 14 (preferably 6 to 10) carbon atoms (e.g., phenyl),
a cycloalkyl group having 3 to 10 (preferably 3 to 8) carbon atoms (e.g., cyclohexyl)
and the like are preferable.

Ring B is preferably a benzene ring, a fused aromatic hydrocarbon ring having 9 to 14 carbon atoms (preferably naphthalene and the like) or a 5- or 6-membered aromatic heterocycle (excluding oxazole, preferably pyridine, pyrazole, thiazole and the like), each of which optionally further has 1 to 3 substituents selected from
an alkyl group having 1 to 10 (preferably 1 to 4) carbon atoms (preferably methyl, ethyl, isopropyl, isobutyl and the like),
an aryl group having 6 to 14 carbon atoms-alkyl group having 1 to 10 (preferably 1 to 4) carbon atoms (preferably benzyl and the like),
an alkoxy group having 1 to 10 (preferably 1 to 4) carbon atoms (preferably methoxy, ethoxy and the like),
an aryl group having 6 to 14 (preferably 6 to 10) carbon atoms (preferably phenyl and the like), and
a cycloalkyl group having 3 to 10 (preferably 3 to 8) carbon atoms (preferably cyclohexyl and the like).

Ring B is more preferably a benzene ring, a naphthalene ring, a pyridine ring, a pyrazole ring or a thiazole ring (preferably a thiazole ring), each of which optionally further has 1 to 3 substituents selected from an alkyl group having 1 to 4 carbon atoms (preferably methyl, ethyl, isopropyl, isobutyl), an aryl group having 6 to 14 carbon atoms-alkyl group having 1 to 4 carbon atoms (preferably benzyl), an alkoxy group having 1 to 4 carbon atoms (preferably methoxy, ethoxy),
an aryl group having 6 to 10 carbon atoms (preferably phenyl), and
a cycloalkyl group having 3 to 8 carbon atoms (preferably cyclohexyl).

When ring B is a benzene ring, a naphthalene ring, a pyridine ring or a thiazole ring, then the constituting atom thereon to which Z² is bonded and the constituting atom thereon to which W is bonded are preferably adjacent to each other.

W is a divalent saturated hydrocarbon group having 1 to 20 carbon atoms.

As the "divalent saturated hydrocarbon group having 1 to 20 carbon atoms" for W, saturated one, from among the "divalent hydrocarbon groups having 1 to 20 carbon atoms" exemplified as the aforementioned Xb, can be mentioned.

W is preferably a divalent saturated hydrocarbon group having 1 to 6 carbon atoms, more preferably a C₁₋₆ alkylene group, particularly preferably -CH₂-, -(CH₂)₂- or the like.

R² is -OR⁸ (wherein R⁸ is a hydrogen atom or an optionally substituted hydrocarbon group) or -NR⁹R¹⁰ (wherein R⁹ and R¹⁰ are the same or different and each is a hydrogen atom, an optionally substituted hydrocarbon group, an optionally substituted heterocyclic group or an acyl group, or R⁹ and R¹⁰ are bonded to each other to form, together with the adjacent nitrogen atom, an optionally substituted ring).

As the "optionally substituted hydrocarbon group" for R⁸, R⁹ or R¹⁰, those exemplified as the aforementioned R³ can be mentioned.

As the "optionally substituted hydrocarbon group", an "alkyl group having 1 to 4 carbon atoms" and the like are preferable. As used herein, as the "alkyl group having 1 to 4 carbon atoms", for example, methyl, ethyl, propyl, butyl, isobutyl, sec-butyl, tert-butyl and the like can be mentioned. Of these, methyl, ethyl are preferable.

As the "optionally substituted heterocyclic group" and "acyl group" for R⁹ or R¹⁰, those exemplified as the substituents which the aforementioned "optionally substituted aromatic ring" for Ar optionally has, can be mentioned.

As the ring, which is formed, together with the adjacent nitrogen atom, by R⁹ and R¹⁰ bonded to each other, for example, a 5 to 7-membered nitrogen-containing heterocycle can be mentioned. As preferable examples of the 5 to 7-membered nitrogen-containing heterocycle, pyrrolidine, piperidine, hexamethylenimine, morpholine, thiomorpholine, piperazine and the like can be mentioned.

The ring optionally has 1 to 3 substituents at substitutable positions. As such substituents, for example, a halogen atom (e.g., fluorine, chlorine, bromine, iodine), a C₁₋₆ alkyl group (e.g., methyl, ethyl) optionally substituted by 1 to 3 halogen atoms (e.g., fluorine, chlorine, bromine, iodine),
a C₁₋₆ alkoxy group (e.g., methoxy, ethoxy) optionally substituted by 1 to 3 halogen atoms (e.g., fluorine, chlorine, bromine, iodine),
a nitro group,
a hydroxy group,
an amino group
and the like can be mentioned. When the ring has 2 or more substituents, respective substituents may be the same or different.

R² is preferably -OR⁸ (wherein R⁸ is as defined above), and R⁸ is preferably a hydrogen atom or an alkyl group having 1 to 4 carbon atoms (e.g., methyl, ethyl). R² is particularly preferably -OH.

In the formula (I),
a compound wherein Xa, Xb and Xc are each a bond is excluded;
a compound wherein Z¹ and Z² are each a bond or each an oxygen atom is excluded; and
when ring A is a benzene ring, then the carbon atom thereon to which Xc is bonded and the carbon atom thereon to which Yc is bonded should not be adjacent to each other, and Z² should not be a sulfur atom.

As preferable examples of the compound represented by the formula (I), the following compounds can be mentioned.

### [Compound A]

A compound wherein
Ar is a benzene ring, a 5- or 6-membered aromatic heterocycle (preferably pyridine, oxazole) or a 9- to 14-membered fused aromatic heterocycle (preferably quinoline), each of which optionally has 1 to 3 substituents selected from
1) a halogen atom;
2) an alkyl group having 1 to 10 carbon atoms and optionally having 1 to 3 substituents selected from an alkoxy group having 1 to 6 carbon atoms, which is optionally substituted by 1 to 3 halogen atoms, a halogen atom, a nitro group, a hydroxy group and an amino group;
3) an alkoxy group having 1 to 10 carbon atoms and optionally having 1 to 3 substituents selected from an alkoxy group having 1 to 6 carbon atoms, which is optionally substituted by 1 to 3 halogen atoms, a halogen atom, a nitro group, a hydroxy group and an amino group; and
4) an aryl group having 6 to 14 carbon atoms (preferably phenyl and the like) and optionally having 1 to 3 substituents selected from an alkyl group having 1 to 6 carbon atoms, which is optionally substituted by 1 to 3 halogen atoms, an alkoxy group having 1 to 6 carbon atoms, which is optionally substituted by 1 to 3 halogen atoms, a halogen atom, a nitro group, a hydroxy group and an amino group;

Xa is a bond, Xb is a bond or a divalent hydrocarbon group having 1 to 6 carbon atoms (preferably a C₁₋₆ alkylene group), and Xc is a bond, an oxygen atom, -CO- or -NR⁶CO- (wherein R⁶ is a hydrogen atom or an alkyl group having 1 to 4 carbon atoms);
R¹ is an alkyl group having 1 to 10 (preferably 1 to 4) carbon atoms and optionally having 1 to 3 substituents selected from a halogen atom (e.g., fluorine, chlorine, bromine, iodine),
an alkoxy group having 1 to 4 carbon atoms (e.g., methoxy, ethoxy, propoxy, isopropoxy, butoxy, isobutoxy, sec-butoxy, tert-butoxy),
a hydroxy group,
a nitro group,
an amino group, and
an acyl group having 1 to 4 carbon atoms (e.g., formyl, an alkylcarbonyl group having 2 to 4 carbon atoms, such as acetyl, propionyl and the like);
Ya is a bond, an oxygen atom, a sulfur atom, -NR⁵-(wherein R⁵ is an alkyl group having 1 to 4 carbon atoms) or - SO₂-, Yb is a bond or a divalent hydrocarbon group having 1 to 6 carbon atoms (preferably a C₁₋₆ alkylene group), and Yc is a bond or an oxygen atom;
ring A is a benzene ring or a 5- or 6-membered aromatic heterocycle (preferably a pyridine ring, a pyrimidine ring, a pyrazole ring, a thiazole ring and the like), each of which optionally further has 1 or 2 substituents selected from an alkyl group having 1 to 4 carbon atoms,
a hydroxy group,
an alkoxy group having 1 to 4 carbon atoms,
an aralkyloxy group having 7 to 10 carbon atoms, and
a halogen atom;
Z¹ is a bond or an oxygen atom, n is an integer of 1 to 3, and Z² is an oxygen atom;
ring B is a benzene ring, a fused aromatic hydrocarbon ring having 9 to 14 carbon atoms (preferably naphthalene) or a 5- or 6-membered aromatic heterocycle (excluding oxazole, preferably pyridine, pyrazole, thiazole), each of which optionally further has 1 to 3 substituents selected from an alkyl group having 1 to 10 carbon atoms,
an aryl group having 6 to 14 carbon atoms-alkyl group having 1 to 10 carbon atoms, and
an alkoxy group having 1 to 10 carbon atoms;
W is a divalent saturated hydrocarbon group having 1 to 6 carbon atoms; and
R² is -OR⁸, and R⁸ is a hydrogen atom or an alkyl group having 1 to 4 carbon atoms (preferably methyl, ethyl);
with the proviso that
a compound wherein Xa, Xb and Xc are each a bond is excluded; and
when ring A is a benzene ring, then the carbon atom thereon to which Xc is bonded and the carbon atom thereon to which Yc is bonded should not be adjacent to each other.

### [Compound B]

A compound wherein
Ar is a benzene ring, a pyridine ring, an oxazole ring or a quinoline ring, each of which optionally has 1 or 2 substituents selected from a halogen atom (preferably fluorine, chlorine),
an alkyl group having 1 to 4 carbon atoms (preferably methyl), which is optionally substituted by 1 to 3 halogen atoms (preferably fluorine),
an alkoxy group having 1 to 4 carbon atoms (preferably methoxy, ethoxy, isopropoxy), and
phenyl;
Xa is a bond, Xb is a bond or a C₁₋₆ alkylene group (preferably -CH₂-, -CH(CH₃)-), and Xc is a bond, an oxygen atom, -CO- or -NHCO-;
R¹ is an alkyl group having 1 to 4 carbon atoms (preferably methyl, ethyl, propyl, isopropyl, butyl, isobutyl);
Ya is a bond, an oxygen atom, a sulfur atom, -N(CH₃)- or - SO₂-, Yb is a bond or a C₁₋₆ alkylene group (preferably -CH₂-, - (CH₂)₂-), and Yc is a bond or an oxygen atom;
ring A is a benzene ring, a pyridine ring, a pyrimidine ring, a pyrazole ring or a thiazole ring;
Z¹ is a bond or an oxygen atom, n is an integer of 1 to 3, and Z² is an oxygen atom;
ring B is a benzene ring, a naphthalene ring, a pyridine ring, a pyrazole ring or a thiazole ring, each of which optionally further has 1 to 3 substituents selected from an alkyl group having 1 to 4 carbon atoms (preferably methyl, ethyl),
an aryl group having 6 to 14 carbon atoms-alkyl group having 1 to 4 carbon atoms (preferably benzyl), and
an alkoxy group having 1 to 4 carbon atoms (preferably methoxy, ethoxy and the like);
W is a C₁₋₆ alkylene group (preferably -CH₂-); and
R² is -OH;
with the proviso that
a compound wherein Xa, Xb and Xc are each a bond is excluded; and
when ring A is a benzene ring, then the carbon atom thereon to which Xc is bonded and the carbon atom thereon to which Yc is bonded should not be adjacent to each other.

### [Compound C]

A compound wherein
Ar is a benzene ring, a 5- or 6-membered aromatic heterocycle (preferably pyridine, oxazole) or a 9- to 14-membered fused aromatic heterocycle (preferably quinoline), each of which optionally has 1 to 3 substituents selected from
1) a halogen atom;
2) an alkyl group having 1 to 10 carbon atoms and optionally having 1 to 3 substituents selected from an alkoxy group having 1 to 6 carbon atoms, which is optionally substituted by 1 to 3 halogen atoms, a halogen atom, a nitro group, a hydroxy group and an amino group;
3) an alkoxy group having 1 to 10 carbon atoms and optionally having 1 to 3 substituents selected from an alkoxy group having 1 to 6 carbon atoms, which is optionally substituted by 1 to 3 halogen atoms, a halogen atom, a nitro group, a hydroxy group and an amino group; and
4) an aryl group having 6 to 14 carbon atoms (preferably phenyl and the like) and optionally having 1 to 3 substituents selected from an alkyl group having 1 to 6 carbon atoms, which is optionally substituted by 1 to 3 halogen atoms, an alkoxy group having 1 to 6 carbon atoms, which is optionally substituted by 1 to 3 halogen atoms, a halogen atom, a nitro group, a hydroxy group and an amino group;
   Xa is a bond or an oxygen atom, Xb is a bond or a divalent hydrocarbon group having 1 to 6 carbon atoms (preferably a C₁₋₆ alkylene group), an Xc is a bond, an oxygen atom, -CO- or -NR⁶CO-(wherein R⁶ is a hydrogen atom or an alkyl group having 1 to 4 carbon atoms);
   R¹ is an alkyl group having 1 to 10 (preferably 1 to 4) carbon atoms and optionally having 1 to 3 substituents selected from a halogen atom (e.g., fluorine, chlorine, bromine, iodine),
   an alkoxy group having 1 to 4 carbon atoms (e.g., methoxy, ethoxy, propoxy, isopropoxy, butoxy, isobutoxy, sec-butoxy, tert-butoxy),
   a hydroxy group,
   a nitro group,
   an amino group,
   an acyl group having 1 to 4 carbon atoms (preferably formyl; an alkylcarbonyl group having 2 to 4 carbon atoms, such as acetyl, propionyl and the like),
   an aryl group having 6 to 14 carbon atoms (preferably phenyl), and
   a non-aromatic heterocyclic group (preferably pyrrolidinyl) optionally substituted by 1 to 3 oxo groups;
   Ya is a bond, an oxygen atom, a sulfur atom, -NR⁵-(wherein R⁵ is an alkyl group having 1 to 4 carbon atoms), -SO₂--CONR⁶- or -NR⁶CO- (wherein R⁶ is a hydrogen atom or an alkyl group having 1 to 4 carbon atoms), Yb is a bond or a divalent hydrocarbon group having 1 to 6 carbon atoms (preferably a C₁₋₆ alkylene group), and Yc is a bond or an oxygen atom;
   ring A is a benzene ring or a 5- or 6-membered aromatic heterocycle (preferably pyridine, pyrimidine, pyrazole, thiazole), each of which optionally further has 1 or 2 substituents selected from an alkyl group having 1 to 4 carbon atoms,
   a hydroxy group,
   an alkoxy group having 1 to 4 carbon atoms,
   an aralkyloxy group having 7 to 10 carbon atoms, and
   a halogen atom;
   Z¹ is a bond, n is an integer of 1 to 4, and Z² is an oxygen atom;
   ring B is a benzene ring, a fused aromatic hydrocarbon ring having 9 to 14 carbon atoms (preferably naphthalene) or a 5- or 6-membered aromatic heterocycle (excluding oxazole, preferably pyridine, pyrazole, thiazole), each of which optionally further has 1 to 3 substituents selected from an alkyl group having 1 to 10 carbon atoms,
   an aryl group having 6 to 14 carbon atoms-alkyl group having 1 to 10 carbon atoms,
   an alkoxy group having 1 to 10 carbon atoms,
   an aryl group having 6 to 14 carbon atoms, and
   a cycloalkyl group having 3 to 10 carbon atoms;
   W is a divalent saturated hydrocarbon group having 1 to 6 carbon atoms; and
   R² is -OR⁸, and R⁸ is a hydrogen atom or an alkyl group having 1 to 4 carbon atoms (preferably methyl, ethyl);
   with the proviso that
   a compound wherein Xa, Xb and Xc are each a bond is excluded; and
   when ring A is a benzene ring, then the carbon atom thereon to which Xc is bonded and the carbon atom thereon to which Yc is bonded should not be adjacent to each other.

### [Compound D]

Compound C wherein
a combination of Xa, Xb and Xc is
"a combination of Xa being a bond, Xb being a bond or a divalent hydrocarbon group having 1 to 6 carbon atoms (preferably a C₁₋₆ alkylene group), and Xc being an oxygen atom",
"a combination of Xa being a bond, Xb being a divalent hydrocarbon group having 1 to 6 carbon atoms (preferably a C₁₋₆ alkylene group), and Xc being a bond", or
"a combination of Xa being an oxygen atom, Xb being a divalent hydrocarbon group having 1 to 6 carbon atoms (preferably a C₁₋₆ alkylene group), and Xc being a bond";
ring B is a benzene ring, a naphthalene ring, a pyridine ring, a pyrazole ring or a thiazole ring, each of which optionally further has 1 to 3 substituents selected from an alkyl group having 1 to 10 carbon atoms,
an aryl group having 6 to 14 carbon atoms-alkyl group having 1 to 10 carbon atoms,
an alkoxy group having 1 to 10 carbon atoms,
an aryl group having 6 to 14 carbon atoms, and
a cycloalkyl group having 3 to 10 carbon atoms;
with the proviso that
when ring A is a benzene ring, then the carbon atom thereon to which Xc is bonded and the carbon atom thereon to which Yc is bonded should not be adjacent to each other.

### [Compound E]

3-{3-[3-(2-{[3-chloro-5-(trifluoromethyl)pyridin-2-yl]oxy}-4-isopropoxyphenyl)propoxy]-1-ethyl-1H-pyrazol-5-yl}propanoic acid (Example 141),
3-(3-{3-[2-{[3-chloro-5-(trifluoromethyl)pyridin-2-yl]oxy}-4-(2-methoxyethoxy)phenyl]propoxy}-1-ethyl-1H-pyrazol-5-yl)propanoic acid (Example 149),
{2-[3-(2-{[3-chloro-5-(trifluoromethyl)pyridin-2-yl]oxy}-4-isopropoxyphenyl)propoxy]-3-methoxyphenyl}acetic acid (Example 115),
(1-benzyl-3-{3-[1-(2, 4-dichlorobenzyl)-3-isopropoxy-1H-pyrazol-5-yl]propoxy}-1H-pyrazol-4-yl)acetic acid (Example 86),
(2-{3-[1-(2,4-dichlorobenzyl)-3-isopropoxy-1H-pyrazol-5-yl]propoxy}-3-methoxyphenyl)acetic acid (Example 16),
(4-{3-[1-(2,4-dichlorobenzyl)-3-isopropoxy-1H-pyrazol-5-yl]propoxy}-2-methyl-1,3-thiazol-5-yl)acetic acid (Example 36),
[2-(3-{1-[2-chloro-4-(trifluoromethyl)benzyl]-3-isopropyl-1H-pyrazol-5-yl}propoxy)pyridin-3-yl]acetic acid (Example 77),
(3-{3-[2-(2,4-dichlorophenoxy)-6-isopropoxypyridin-3-yl]propoxy}-1-methyl-1H-pyrazol-4-yl)acetic acid (Example 130), or
(1-{3-[1-(2,4-dichlorobenzyl)-3-isopropoxy-1H-pyrazol-5-yl]propoxy}-2-naphthyl)acetic acid (Example 87).

When compound (I) is a salt, the salt is preferably a pharmacologically acceptable salt and, for example, salts with inorganic bases, salts with organic bases, salts with inorganic acids, salts with organic acids, salts with basic or acidic amino acids and the like can be mentioned.

Preferable examples of the salts with inorganic base include alkali metal salts such as sodium salt, potassium salt and the like; alkaline earth metal salts such as calcium salt, magnesium salt and the like; aluminum salt, ammonium salt and the like.

Preferable examples of the salt with organic base include a salt with trimethylamine, triethylamine, pyridine, picoline, ethanolamine, diethanolamine, triethanolamine, tromethamine [tris(hydroxymethyl)methylamine], tert-butylamine, cyclohexylamine, benzylamine, dicyclohexylamine, N,N'-dibenzylethylenediamine and the like.

Preferable examples of the salt with inorganic acid include a salt with hydrochloric acid, hydrobromic acid, nitric acid, sulfuric acid, phosphoric acid and the like.

Preferable examples of the salt with organic acid include a salt with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of the salt with basic amino acid include a salt with arginine, lysine, ornithine and the like.

Preferable examples of the salt with acidic amino acid include a salt with aspartic acid, glutamic acid and the like.

Of those, the salt is preferably sodium salt, potassium salt, the salt with hydrochloric acid or the like.

The prodrug of the compound (I) is a compound which is converted to the compound (I) with a reaction due to an enzyme, gastric acid, etc. under the physiological condition in the living body, that is, a compound which is converted to the compound (I) by enzymatic oxidation, reduction, hydrolysis, etc.; a compound which is converted to the compound (I) by hydrolysis etc. due to gastric acid, and the like. A prodrug of the compound (I) may be a compound obtained by subjecting an amino group in the compound (I) to an acylation, alkylation or phosphorylation (e.g., a compound obtained by subjecting an amino group in the compound (I) to an eicosanoylation, alanylation, pentylaminocarbonylation, (5-methyl-2-oxo-1,3-dioxolen-4-yl)methoxycarbonylation, tetrahydrofuranylation, tetrahydropyranylation, pyrrolidylmethylation, pivaloyloxymethylation or tert-butylation, and the like); a compound obtained by subjecting a hydroxy group in the compound (I) to an acylation, alkylation, phosphorylation or boration (e.g., a compound obtained by subjecting an hydroxy group in the compound (I) to an acetylation, palmitoylation, propanoylation, pivaloylation, succinylation, fumarylation, alanylation, dimethylaminomethylcarbonylation, or tetrahydropyranylation, and the like); a compound obtained by subjecting a carboxyl group in the compound (I) to an esterification or amidation (e.g., a compound obtained by subjecting a carboxyl group in the compound (I) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification or methylamidation, and the like) and the like. Any of these compounds can be produced from the compound (I) by a method known *per se.*

A prodrug of the compound (I) may be a compound that converts to the compound (I) under physiological conditions as described in Development of Pharmaceutical Products, vol. 7, Molecule Design, 163-198, Hirokawa Shoten (1990).

The compound (I) may be in the form of a crystal, and the crystal form of the crystal may be single or plural. The crystal can be produced by a crystallization method known *per se.*

The compound (I) may be a solvate (e.g., hydrate etc.) or a non-solvate, both of which are encompassed in the compound (I).

A compound labeled with an isotope (e.g., ³H, ¹⁴C, ³⁵S, ¹²⁵I) and the like are also encompassed in compound (I).

The compound (I) or a prodrug thereof (hereinafter sometimes to be simply abbreviated as the compound of the present invention) shows low toxicity (e.g., acute toxicity, chronic toxicity, genetic toxicity, reproductive toxicity, cardiotoxicity, drug interaction, carcinogenicity), and can be used as it is or as a pharmaceutical composition in admixture with a commonly known pharmaceutically acceptable carrier etc., as an agent for the prophylaxis or treatment of the below-mentioned various disease, in mammals (e.g., humans, mice, rats, rabbits, dogs, cats, bovines, horses, pigs, monkeys etc.).

Here, as the pharmacologically acceptable carrier, various organic or inorganic carrier substances conventionally used as a preparation material can be used. They are incorporated as excipient, lubricant, binder and disintegrant for solid preparations; solvent, dissolution aids, suspending agent, isotonicity agent, buffer and soothing agent for liquid preparations and the like. Where necessary, preparation additives such as preservatives, antioxidants, coloring agents, sweetening agents and the like can be used.

As preferable examples of the excipient, lactose, sucrose, D-mannitol, D-sorbitol, starch, α-starch, dextrin, crystalline cellulose, low-substituted hydroxypropylcellulose, sodium carboxymethylcellulose, gum arabic, pullulan, light anhydrous silicic acid, synthetic aluminum silicate, magnesium alumino metasilicate and the like can be mentioned.

As preferable examples of the lubricant, magnesium stearate, calcium stearate, talc, colloidal silica and the like can be mentioned.

As preferable examples of the binder, α-starch, saccharose, gelatin, gum arabic, methylcellulose, carboxymethylcellulose, carboxymethylcellulose sodium, crystalline cellulose, sucrose, D-mannitol, trehalose, dextrin, pullulan, hydroxypropylcellulose, hydroxypropylmethylcellulose, polyvinylpyrrolidone and the like can be mentioned.

As preferable examples of the disintegrant, lactose, sucrose, starch, carboxymethylcellulose, carboxymethylcellulose calcium, croscarmellose sodium, carboxymethylstarch sodium, light anhydrous silicic acid, low-substituted hydroxypropylcellulose and the like can be mentioned.

As preferable examples of the solvent, water for injection, physiological brine, Ringer solution, alcohol, propylene glycol, polyethylene glycol, sesame oil, corn oil, olive oil, cottonseed oil and the like can be mentioned.

As preferable examples of the dissolution aids, polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like can be mentioned.

As preferable examples of the suspending agent, surfactants such as stearyltriethanolamine, sodium lauryl sulfate, laurylaminopropionic acid, lecithin, benzalkonium chloride, benzethonium chloride, glycerol monostearate and the like; hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose and the like; polysorbates, polyoxyethylene hydrogenated castor oil, and the like can be mentioned.

As preferable examples of the isotonicity agent, sodium chloride, glycerin, D-mannitol, D-sorbitol, glucose and the like can be mentioned.

As preferable examples of the buffer, buffers such as phosphate, acetate, carbonate, citrate and the like, and the like can be mentioned.

As preferable examples of the soothing agent, benzyl alcohol and the like can be mentioned.

As preferable examples of the preservative, p-oxybenzoates, chlorobutanol, benzyl alcohol, phenethyl alcohol, dehydroacetic acid, sorbic acid and the like can be mentioned.

As preferable examples of the antioxidant, sulfite, ascorbate and the like can be mentioned.

As preferable examples of the coloring agent, water-soluble food tar colors (e.g., food colors such as Food Red Nos. 2 and 3, Food Yellow Nos. 4 and 5, Food Blue Nos. 1 and 2 and the like), water insoluble lake dye (e.g., aluminum salts of the aforementioned water-soluble food tar colors), natural dyes (e.g., β-carotene, chlorophyll, red iron oxide) and the like can be mentioned.

As preferable examples of the sweetening agent, saccharin sodium, dipotassium glycyrrhizinate, aspartame, stevia and the like can be mentioned.

As the dosage form of the aforementioned pharmaceutical composition, for example, oral preparation such as tablets (including sublingual tablet, orally disintegrating tablet), capsules (including soft capsule, microcapsule), granule, powder, troche, syrup, emulsion, suspension and the like; and parenteral preparation such as injections (e.g., subcutaneous injection, intravenous injection, intramuscular injection, intraperitoneal injection, drip infusion), external preparations (e.g., dermal preparation, ointment), suppositories (e.g., rectal suppository, vaginal suppository), pellets, transnasal preparations, pulmonary preparations (inhalant), eye drops and the like can be mentioned. They can be safely administered orally or parenterally.

These preparations may be controlled-release preparations (e.g., sustained-release microcapsule) such as immediate-release preparation, sustained-release preparation and the like.

The pharmaceutical composition can be produced by a method conventionally used in the preparation technical field, such as a method described in the Japanese Pharmacopoeia and the like.

While the content of the compound of the present invention in the pharmaceutical composition varies depending on the dosage form, the dose of the compound of the present invention and the like, it is, for example, about 0.1 to 100 wt%.

The compound of the present invention can be used as an insulin sensitizer, an agent for enhancing insulin sensitivity, a retinoid-related receptor function regulator, a peroxisome proliferator-activated receptor ligand, a retinoid X receptor ligand and the like. The function regulator here means both agonists and antagonists. The function regulator may be a partial agonist or partial antagonist.

The compound of the present invention has a hypoglycemic action, a hypolipidemic action, a blood insulin lowering action, an insulin resistance improving action, an insulin sensitivity enhancing action and a retinoid-related receptor function regulating action.

Here, the retinoid-related receptors are DNA binding transcription factors included in the nuclear receptors and using a signal molecule such as fat-soluble vitamin and the like as a ligand, which may be monomer receptors, homodimer receptors or heterodimer receptors.

Here, as the monomer receptors, for example, retinoid O receptor (hereinafter sometimes to be abbreviated as ROR) α (GenBank Accession No. L14611), RORβ (GenBank Accession No. L14160), RORγ (GenBank Accession No. U16997); Rev-erb α (GenBank Accession No. M24898), Rev-erb β (GenBank Accession No. L31785); ERRα (GenBank Accession No. X51416), ERRβ (GenBank Accession No. X51417); Ftz-FI α (GenBank Accession No. S65876), Ftz-FI β (GenBank Accession No. M81385); TIx (GenBank Accession No. S77482); GCNF (GenBank Accession No. U14666) and the like can be mentioned.

As the homodimer receptors, for example, homodimers formed by retinoid X receptors (hereinafter sometimes to be abbreviated as RXR) α (GenBank Accession No. X52773), RXRβ (GenBank Accession No. M84820), RXRγ (GenBank Accession No. U38480); COUPα (GenBank Accession No. X12795), COUPβ (GenBank Accession No. M64497), COUPγ (GenBank Accession No. X12794); TR2α (GenBank Accession No. M29960), TR2β (GenBank Accession No. L27586); or HNF4α (GenBank Accession No. X76930), HNF4γ (GenBank Accession No. Z49826) and the like can be mentioned.

As the heterodimer receptors, for example, heterodimers formed by the above-mentioned retinoid X receptors (RXRα, RXRβ or RXRγ) and one kind of receptor selected from retinoid A receptor (hereinafter sometimes to be abbreviated as RAR) α (GenBank Accession No. X06614), RARβ (GenBank Accession No. Y00291), RARγ (GenBank Accession No. M24857); thyroid gland hormone receptor (hereinafter sometimes to be abbreviated as TR) α (GenBank Accession No. M24748), TRβ (GenBank Accession No. M26747); vitamin D receptor (VDR) (GenBank Accession No. JO3258); peroxisome proliferator-activated receptor (hereinafter sometimes to be abbreviated as PPAR) α (GenBank Accession No. L02932), PPARβ (PPARδ) (GenBank Accession No. U10375), PPARγ (GenBank Accession No. L40904); LXRα (GenBank Accession No. U22662), LXRβ (GenBank Accession No. U14534); FXR (GenBank Accession No. U18374); MB67 (GenBank Accession No. L29263); ONR (GenBank Accession No. X75163); and NURα (GenBank Accession No. L13740), NURβ (GenBank Accession No. X75918) and NURγ (GenBank Accession No. U12767) can be mentioned.

The compound of the present invention has a superior ligand activity (activating action) for retinoid X receptors (RXRα, RXRβ, RXRγ) and peroxisome proliferator-activated receptors (PPARα, PPARβ (PPARδ), PPARγ), from among the above-mentioned retinoid-related receptors, and is useful as an agonist, a partial agonist, an antagonist or a partial antagonist of these receptors.

Moreover, the compound of the present invention has a superior ligand activity (activating action) for peroxisome proliferator-activated receptor of heterodimer receptors formed by retinoid X receptor and peroxisome proliferator-activated receptor (e.g., heterodimer receptor formed by RXRα and PPARδ, heterodimer receptor formed by RXRα and PPARγ, etc.).

Therefore, the retinoid-related receptor ligand of the present invention is preferably used as a peroxisome proliferator-activated receptor ligand or a retinoid X receptor ligand.

The compound of the present invention particularly has a selective agonistic (including partial agonistic) action on PPARδ and PPARγ and is useful as a hypoglycemic agent free of body weight gain.

The compound of the present invention can be used, for example, as an agent for the prophylaxis or treatment of diabetes (e.g., type-1 diabetes, type-2 diabetes, gestational diabetes); an agent for the prophylaxis or treatment of hyperlipidemia (e.g., hypertriglyceridemia, hypercholesterolemia, hypo-HDL-emia, postprandial hyperlipidemia); insulin sensitizer; an agent for enhancing insulin sensitivity; an agent for the prophylaxis or treatment of impaired glucose tolerance [IGT (Impaired Glucose Tolerance)]; and an agent for preventing progress of impaired glucose tolerance into diabetes.

For diagnostic criteria of diabetes, Japan Diabetes Society reported new diagnostic criteria in 1999.

According to this report, diabetes is a condition showing any of a fasting blood glucose level (glucose concentration of intravenous plasma) of not less than 126 mg/dl, a 75 g oral glucose tolerance test (75 g OGTT) 2 h level (glucose concentration of intravenous plasma) of not less than 200 mg/dl, and a non-fasting blood glucose level (glucose concentration of intravenous plasma) of not less than 200 mg/dl. A condition not falling under the above-mentioned diabetes and different from "a condition showing a fasting blood glucose level (glucose concentration of intravenous plasma) of less than 110 mg/dl or a 75 g oral glucose tolerance test (75 g OGTT) 2 h level (glucose concentration of intravenous plasma) of less than 140 mg/dl" (normal type) is called a "borderline type".

In addition, ADA (American Diabetes Association) in 1997 and WHO in 1998 reported new diagnostic criteria of diabetes.

According to these reports, diabetes is a condition showing a fasting blood glucose level (glucose concentration of intravenous plasma) of not less than 126 mg/dl and a 75 g oral glucose tolerance test 2 h level (glucose concentration of intravenous plasma) of not less than 200 mg/dl.

According to the above-mentioned reports, impaired glucose tolerance is a condition showing a fasting blood glucose level (glucose concentration of intravenous plasma) of less than 126 mg/dl and a 75 g oral glucose tolerance test 2 h level (glucose concentration of intravenous plasma) of not less than 140 mg/dl and less than 200 mg/dl. According to the report of ADA, a condition showing a fasting blood glucose level (glucose concentration of intravenous plasma) of not less than 110 mg/dl and less than 126 mg/dl is called IFG (Impaired Fasting Glucose). On the other hand, according to the report of WHO, of IFG (Impaired Fasting Glucose), a condition showing a 75 g oral glucose tolerance test 2 h level (glucose concentration of intravenous plasma) of less than 140 mg/dl is called IFG (Impaired Fasting Glycemia).

The compound of the present invention can be also used as an agent for the prophylaxis or treatment of diabetes, borderline type, impaired glucose tolerance, IFG (Impaired Fasting Glucose) and IFG (Impaired Fasting Glycaemia), as determined according to the above-mentioned new diagnostic criteria. Moreover, the compound of the present invention can prevent progress of borderline type, impaired glucose tolerance, IFG (Impaired Fasting Glucose) or IFG (Impaired Fasting Glycaemia) into diabetes.

The compound of the present invention can also be used as an agent for the prophylaxis or treatment of, for example, diabetic complications [e.g., neuropathy, nephropathy, retinopathy, cataract, macroangiopathy, osteopenia, hyperosmolar diabetic coma, infectious disease (e.g., respiratory infection, urinary tract infection, gastrointestinal infection, dermal soft tissue infections, inferior limb infection), diabetic gangrene, xerostomia, hypacusis, cerebrovascular disorder, peripheral blood circulation disorder], obesity, osteoporosis, cachexia (e.g., cancerous cachexia, tuberculous cachexia, diabetic cachexia, blood disease cachexia, endocrine disease cachexia, infectious disease cachexia or cachexia due to acquired immunodeficiency syndrome), fatty liver, hypertension, polycystic ovary syndrome, kidney disease (e.g., diabetic nephropathy, glomerular nephritis, glomerulosclerosis, nephrotic syndrome, hypertensive nephrosclerosis, end stage kidney disease), muscular dystrophy, myocardial infarction, angina pectoris, cerebrovascular accident (e.g., cerebral infarction, cerebral apoplexy), insulin resistance syndrome, Syndrome X, metabolic syndrome (e.g., pathology having three or more selected from hypertriglyceridemia (TG), hypoHDL cholesterolemia (HDL-C), hypertension, abdomen overweight and impaired glucose tolerance), hyperinsulinemia, hyperinsulinemia-induced sensory disorder, tumor (e.g., leukemia, breast cancer, prostate cancer, skin cancer), irritable bowel syndrome, acute or chronic diarrhea, inflammatory diseases (e.g., chronic rheumatoid arthritis, spondylitis deformans, osteoarthritis, lumbago, gout, postoperative or traumatic inflammation, swelling, neuralgia, pharyngolaryngitis, cystitis, hepatitis (inclusive of nonalcoholic steatohepatitis), pneumonia, pancreatitis, inflammatory bowel disease, ulcerative colitis), visceral obesity syndrome and the like.

Since the compound of the present invention has a total cholesterol lowering action and increases the plasma antiatherogenic index [(HDL cholesterol/total cholesterol) × 100], it can also be used as an agent for the prophylaxis or treatment of arteriosclerosis (e.g., atherosclerosis).

The compound of the present invention can also be used for ameliorating the condition of bellyache, nausea, vomiting, dysphoria in epigastrium and the like, each of which is accompanied by peptic ulcer, acute or chronic gastritis, biliary dyskinesia, cholecystitis and the like.

The compound of the present invention can also be used as an agent for the prophylaxis or treatment of inflammatory disease involving TNF-α. Here, the inflammatory disease involving TNF-α is an inflammatory disease developed by the presence of TNF-α, which can be treated via a TNF-α inhibitory effect. As such inflammatory disease, for example, diabetic complications (e.g., retinopathy, nephropathy, neuropathy, macroangiopathy), chronic rheumatoid arthritis, spondylitis deformans, osteoarthritis, lumbago, gout, postoperative or traumatic inflammation, swelling, neuralgia, pharyngolaryngitis, cystitis, hepatitis, pneumonia, stomach mucous membrane injury (including stomach mucous membrane injury caused by aspirin) and the like can be mentioned.

The compound of the present invention has an apoptosis inhibitory action and can also be used as an agent for the prophylaxis or treatment of diseases involving promotion of apoptosis. As the disease involving promotion of apoptosis, for example, viral diseases (e.g., AIDS, fulminant hepatitis), neurodegenerative diseases (e.g., Alzheimer's disease, Parkinson's syndrome, amyotrophic lateral sclerosis, pigmentosa, cerebellar degeneration), myelodysplasia (e.g., aplastic anemia), ischemic diseases (e.g., cardiac infarction, cerebral apoplexy), hepatic diseases (e.g., alcoholic hepatitis, hepatitis B, hepatitis C), joint-diseases (e.g., osteoarthritis), atherosclerosis and the like can be mentioned.

The compound of the present invention can also be used for reduction of visceral fat, inhibition of visceral fat accumulation, glycometabolism improvement, lipometabolism improvement, insulin resistance improvement, oxidized LDL production inhibition, lipoprotein metabolism improvement, coronary metabolism improvement, prophylaxis or treatment of cardiovascular complications, prophylaxis or treatment of heart failure complications, decrease of blood remnant, prophylaxis or treatment of anovulation, prophylaxis or treatment of hirsutism, prophylaxis or treatment of hyperandrogenemia and the like.

The compound of the present invention can also be used as secondary prevention and suppression of progression of the above-mentioned various diseases (e.g., cardiovascular event such as cardiac infarction and the like).

While the dose of the compound of the present invention varies depending on the administration subject, administration route, target disease, condition and the like, for example, a single dose is generally about 0.005 to 50 mg/kg body weight, preferably 0.01 to 2 mg/kg body weight, more preferably 0.025 to 0.5 mg/kg body weight, for oral administration to adult diabetic patients, which amount is desirably administered in one to three times a day.

The compound of the present invention can be used in combination with pharmaceutical agents (hereinafter to be abbreviated as combination drug) such as therapeutic agents for diabetes, therapeutic agents for diabetic complications, antilipemic agents, antihypertensive agents, antiobesity agents, diuretics, chemotherapeutic agents, immunotherapeutic agents, antithrombotic agents, therapeutic agents for osteoporosis, antidementia agents, erectile dysfunction ameliorating agents, therapeutic agents for urinary incontinence or pollakiuria, therapeutic agents for dysuria and the like. These combination drugs may be low-molecular-weight compounds, or high-molecular-weight protein, polypeptide, antibody, vaccine and the like.

The administration time of the compound of the present invention and the combination drug is not restricted, and these can be administered to an administration subject simultaneously, or may be administered at staggered times.

As the administration mode of the compound of the present invention and the combination drug, the following methods can be mentioned: (1) The compound of the present invention and the combination drug are simultaneously formulated to give a single preparation which is administered. (2) The compound of the present invention and the combination drug are separately formulated to give two kinds of preparations which are administered simultaneously by the same administration route. (3) The compound of the present invention and the combination drug are separately formulated to give two kinds of preparations which are administered by the same administration route at staggered times. (4) The compound of the present invention and the combination drug are separately formulated to give two kinds of preparations which are administered simultaneously by the different administration routes. (5) The compound of the present invention and the combination drug are separately formulated to give two kinds of preparations which are administered by the different administration routes at staggered times (for example, the compound of the present invention and the combination drug are administered in this order, or in the reverse order), and the like.

The dose of the combination drug can be appropriately determined based on the dose employed clinically. The mixing ratio of the compound of the present invention and a combination drug can be appropriately determined depending on the administration subject, administration route, target disease, symptom, combination and the like. When the administration subject is human, for example, a combination drug can be used in 0.01 to 100 parts by weight relative to 1 part by weight of the compound of the present invention.

As the therapeutic agents for diabetes, insulin preparations (e.g., animal insulin preparations extracted from pancreas of bovine and swine; human insulin preparations genetically synthesized using Escherichia coli, yeast; zinc insulin; protamine zinc insulin; fragment or derivative of insulin (e.g., INS-1 etc.), oral insulin preparation), insulin sensitizers (e.g., pioglitazone or a salt thereof (preferably hydrochloride), rosiglitazone or a salt thereof (preferably maleate), Reglixane (JTT-501), Netoglitazone (MCC-555), Rivoglitazone (CS-011), FK-614, the compound described in WO99/58510 (e.g., (E)-4-[4-(5-methyl-2-phenyl-4-oxazolylmethoxy)benzyloxyimino]-4-phenylbutyric acid), the compound described in WO01/38325, Tesaglitazar (AZ-242), Ragaglitazar (NN-622), Muraglitazar (BMS-298585), ONO-5816, Edaglitazone (BM-13-1258), LM-4156, MBX-102, Naveglitazar (LY-519818), MX-6054, LY-510929, Balaglitazone (NN-2344), T-131 or salts thereof, THR-0921), α-glucosidase inhibitors (e.g., voglibose, acarbose, miglitol, emiglitate etc.), biguanides (e.g., phenformin, metformin, buformin or a salt thereof (e.g., hydrochloride, fumarate, succinate)), insulin secretagogues [sulfonylurea (e.g., tolbutamide, glibenclamide, gliclazide, chlorpropamide, tolazamide, acetohexamide, glyclopyramide, glimepiride, glipizide, glybuzole), repaglinide, nateglinide, mitiglinide or calcium salt hydrate thereof], dipeptidyl peptidase IV inhibitors (e.g., Vidagliptin (LAF237), P32/98, MK-431, P93/01, PT-100, Saxagliptin (BMS-477118), T-6666, TS-021), β3 agonists (e.g., AJ-9677), GPR40 agonists, GLP-1 receptor agonists [e.g., GLP-1, GLP-1MR agent, NN-2211, AC-2993 (exendin-4), BIM-51077, Aib(8,35)hGLP-1(7,37)NH₂, CJC-1131], amylin agonists (e.g., pramlintide), phosphotyrosine phosphatase inhibitors (e.g., sodium vanadate), gluconeogenesis inhibitors (e.g., glycogen phosphorylase inhibitors, glucose-6-phosphatase inhibitors, glucagon antagonists), SGLUT (sodium-glucose cotransporter) inhibitors (e.g., T-1095), 11β-hydroxysteroid dehydrogenase inhibitors (e.g., BVT-3498), adiponectin or agonists thereof, IKK inhibitors (e.g., AS-2868), leptin resistance improving drugs, somatostatin receptor agonists (compounds described in WO01/25228, WO03/42204, WO98/44921, WO98/45285, WO99/22735 etc.), glucokinase activators (e.g., Ro-28-1675) and the like can be mentioned.

Examples of the therapeutic agents for diabetic complications include aldose reductase inhibitors (e.g., Tolrestat, Epalrestat, Zenarestat, Zopolrestat, Minalrestat, Fidarestat, CT-112, ranirestat (AS-3201)), neurotrophic factors and increasing drugs thereof (e.g., NGF, NT-3, BDNF, neurotrophin production-secretion promoters described in WO01/14372 (e.g., 4-(4-chlorophenyl)-2-(2-methyl-1-imidazolyl)-5-[3-(2-methylphenoxy)propyl]oxazole), PKC inhibitors (e.g., ruboxistaurin mesylate)), AGE inhibitors (e.g., ALT946, pimagedine, pyratoxanthine, N-phenacylthiazolium bromide (ALT-766), EXO-226, Pyridorin, Pyridoxamine), active oxygen scavengers (e.g., thioctic acid), cerebral vasodilators (e.g., tiapuride, mexiletine), somatostatin receptor agonist (e.g., BIM23190), apoptosis signal regulating kinase-1 (ASK-1) inhibitors and the like.

Examples of the antilipemic agents include HMG-CoA reductase inhibitors (e.g., pravastatin, simvastatin, lovastatin, atorvastatin, fluvastatin, itavastatin, rosuvastatin, pitavastatin or a salt thereof (e.g., sodium salt, calcium salt)), squalene synthase inhibitors (e.g., compounds described in WO97/10224, such as N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid and the like), fibrate compounds (e.g., bezafibrate, clofibrate, simfibrate, clinofibrate), ACAT inhibitors (e.g., Avasimibe), Eflucimibe)), anion exchange resins (e.g., colestyramine), probucol, nicotinic acid drugs (e.g., nicomol, niceritrol)), ethyl icosapentate, plant sterol (e.g., soysterol), γ-oryzanol) and the like.

Examples of the antihypertensive agents include angiotensin converting enzyme inhibitors (e.g., captopril, enalapril, delapril), angiotensin II antagonists (e.g., candesartan cilexetil, losartan, eprosartan, valsartan, telmisartan, irbesartan, tasosartan, 1-[[2' -(2,5-dihydro-5-oxo-4H-1,2,4-oxadiazol-3-yl)biphenyl-4-yl]methyl]-2-ethoxy-1H-benzimidazole-7-carboxylic acid), calcium antagonists (e.g., manidipine, nifedipine, nicardipine, amlodipine, efonidipine), potassium channel openers (e.g., levcromakalim, L-27152, AL0671, NIP-121), clonidine and the like.

Examples of the antiobesity agents include antiobesity agents acting on the central nervous system (e.g., dexfenfluramine, fenfluramine, phentermine, sibutramine, amfepramone, dexamphetamine, mazindol, phenylpropanolamine, clobenzorex; MCH receptor antagonists (e.g., SB-568849; SNAP-7941; compounds described in WO01/82925 and WO01/87834); neuropeptide Y antagonists (e.g., CP-422935); cannabinoid receptor antagonists (e.g., SR-141716, SR-147778); ghrelin antagonists; 11β-hydroxysteroid dehydrogenase inhibitors (e.g., BVT-3498)), pancreatic lipase inhibitors (e.g., orlistat, ATL-962), β3 agonists (e.g., AJ-9677), peptide anorexiants (e.g., leptin, CNTF (Ciliary Neurotropic Factor)), cholecystokinin agonists (e.g., lintitript, FPL-15849), feeding deterrents (e.g., P-57) and the like.

Examples of the diuretics include xanthine derivatives (e.g., sodium salicylate and theobromine, calcium salicylate and theobromine), thiazide preparations (e.g., ethiazide, cyclopenthiazide, trichloromethiazide, hydrochlorothiazide, hydroflumethiazide, benzylhydrochlorothiazide, penflutizide, polythiazide, methyclothiazide), antialdosterone preparations (e.g., spironolactone, triamterene), carbonate dehydratase inhibitors (e.g., acetazolamide), chlorobenzenesulfonamide preparations (e.g., chlortalidone, mefruside, indapamide), azosemide, isosorbide, etacrynic acid, piretanide, bumetanide, furosemide and the like.

Examples of the chemotherapeutic agents include alkylating agents (e.g., cyclophosphamide, ifosfamide), metabolic antagonists (e.g., methotrexate, 5-fluorouracil and a derivative thereof), antitumor antibiotics (e.g., mitomycin, adriamycin), plant-derived antitumor agent (e.g., vincristine, vindesine, Taxol), cisplatin, carboplatin, etoposide and the like. Of these, Furtulon or NeoFurtulon, which are 5-fluorouracil derivatives, and the like are preferable.

Examples of the immunotherapeutic agents include microorganism or bacteral components (e.g., muramyl dipeptide derivative, Picibanil), polysaccharides having immunity potentiating activity (e.g., lentinan, schizophyllan, krestin), cytokines obtained by genetic engineering techniques (e.g., interferon, interleukin (IL)), colony stimulating factors (e.g., granulocyte colony stimulating factor, erythropoietin) and the like, with preference given to interleukins such as IL-1, IL-2, IL-12 and the like.

Examples of the antithrombotic agents include heparin (e.g., heparin sodium, heparin calcium, dalteparin sodium), warfarin (e.g., warfarin potassium), anti-thrombin drugs (e.g., aragatroban), thrombolytic agents (e.g., urokinase, tisokinase, alteplase, nateplase, monteplase, pamiteplase), platelet aggregation inhibitors (e.g., ticlopidine hydrochloride, cilostazol, ethyl icosapentate, beraprost sodium, sarpogrelate hydrochloride) and the like.

Examples of the therapeutic agents for osteoporosis include alfacalcidol, calcitriol, elcatonin, calcitonin salmon, estriol, ipriflavone, risedronate disodium, pamidronate disodium, alendronate sodium hydrate, incadronate disodium and the like.

Examples of the antidementia agents include tacrine, donepezil, rivastigmine, galanthamine and the like.

Examples of the erectile dysfunction ameliorating agents include apomorphine, sildenafil citrate and the like.

Examples of the therapeutic agents for urinary incontinence or pollakiuria include flavoxate hydrochloride, oxybutynin hydrochloride, propiverine hydrochloride and the like.

Examples of the therapeutic agents for dysuria include acetylcholine esterase inhibitors (e.g., distigmine) and the like.

Examples of the combination drugs include drugs having a cachexia-ameliorating action established in animal models and clinical situations, such as cyclooxygenase inhibitors (e.g., indomethacin), progesterone derivatives (e.g., megestrol acetate), glucosteroids (e.g., dexamethasone), metoclopramide agents, tetrahydrocannabinol agents, fat metabolism improving agents (e.g., eicosapentanoic acid), growth hormones, IGF-1, or antibodies to a cachexia-inducing factor such as TNF-α, LIF, IL-6, oncostatin M and the like.

As the combination drugs, nerve regeneration promoting drugs (e.g., Y-128, VX853, prosaptide), antidepressants (e.g., desipramine, amitriptyline, imipramine), antiepileptics (e.g., lamotrigine), antiarrhythmic agents (e.g., mexiletine), acetylcholine receptor ligands (e.g., ABT-594), endothelin receptor antagonists (e.g., ABT-627), monoamine uptake inhibitors (e.g., tramadol), narcotic analgesics (e.g., morphine), GABA receptor agonists (e.g., gabapentin), α2 receptor agonists (e.g., clonidine), local analgesics (e.g., capsaicin), antianxiety drugs (e.g., benzothiazepines), dopamine receptor agonists (e.g., apomorphine), midazolam, ketoconazole and the like can also be mentioned.

The combination drug is preferably an insulin preparation, an insulin sensitizer, an α-glucosidase inhibitor, a biguanide, an insulin secretagogue (preferably sulfonylurea) and the like.

The above-mentioned combination drugs may be used in a mixture of two or more kinds thereof at an appropriate ratio. Preferable combinations in the case of using two or more combination drugs are, for example, as shown in the following.
1) an insulin sensitizer and an insulin preparation;
2) an insulin sensitizer and an insulin secretagogue;
3) an insulin sensitizer and an α-glucosidase inhibitor;
4) an insulin sensitizer and a biguanide;
5) an insulin sensitizer, an insulin preparation and a biguanide;
6) an insulin sensitizer, an insulin preparation and an insulin secretagogue;
7) an insulin sensitizer, an insulin preparation and an α-glucosidase inhibitor;
8) an insulin sensitizer, an insulin secretagogue and a biguanide;
9) an insulin sensitizer, an insulin secretagogue and an α-glucosidase inhibitor; and
10) an insulin sensitizer, a biguanide and an α-glucosidase inhibitor.

When the compound of the present invention is used in combination with a combination drug, the dose of each agent can be reduced within a safe range in consideration of the side effects thereof. Particularly, the doses of insulin sensitizers, insulin secretagogues and biguanides can be reduced from generally dose levels. Therefore, the side effects possibly caused by these agents can be safely prevented. In addition, the doses of the therapeutic agents for diabetic complications, the antilipemic agents and the antihypertensive agents can be reduced, and as a result, the side effects possibly caused by these agents can be effectively prevented.

The production method of the compound of the present invention is explained in the following.

Compound (I) can be produced by a method known per se, for example, Method A to Method F shown below or a method analogous thereto. In the following respective production methods, the starting material compound may be used as a salt, and as such salt, those exemplified as the salts of the compound represented by the formula (I) can be used.

Compound (I-1) of the formula (I) wherein Z² is Z^{2a} (wherein Z^{2a} is an oxygen atom, a sulfur atom or -NR⁵- (wherein R⁵ is as defined above)) can be produced, for example, by the following Method A. 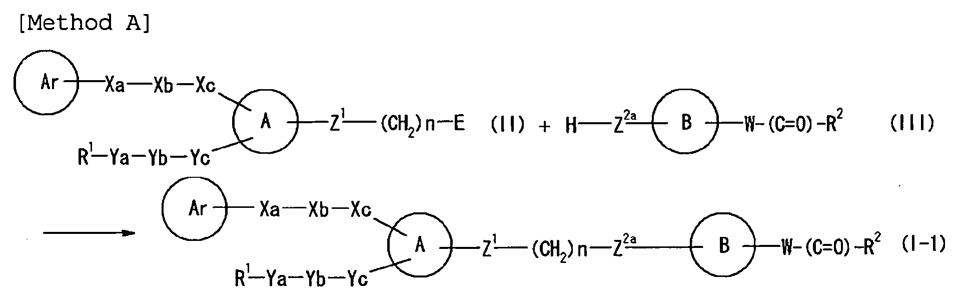 wherein E is a leaving group, and the other symbols are as defined above.

As used herein, as the leaving group for E, for example, a hydroxy group, a halogen atom, -OSO₂R²¹ (wherein R²¹ is an alkyl group having 1 to 4 carbon atoms, or an aryl group having 6 to 10 carbon atoms, which is optionally substituted by an alkyl group having 1 to 4 carbon atoms) and the like can be mentioned.

As the "alkyl group having 1 to 4 carbon atoms" for R²¹ and the alkyl group having 1 to 4 carbon atoms of the "aryl group having 6 to 10 carbon atoms, which is optionally substituted by an alkyl group having 1 to 4 carbon atoms" for R²¹, for example, methyl, ethyl, propyl, isopropyl, butyl, isobutyl, sec-butyl and tert-butyl can be mentioned. Of these, methyl is preferable.

As the aryl group having 6 to 10 carbon atoms of the "aryl group having 6 to 10 carbon atoms, which is optionally substituted by an alkyl group having 1 to 4 carbon atoms" for R²¹, for example, phenyl and naphthyl can be mentioned. Of these, phenyl is preferable.

R²¹ is particularly preferably methyl, tolyl or the like.

In this method, compound (I-1) can be produced by reacting compound (II) with compound (III).

When E is a hydroxy group, this reaction is carried out by a method known per se, for example, the method described in Synthesis, page 1 (1981), or a method analogous thereto. In other words, this reaction is generally carried out in the presence of an organic phosphorus compound and an electrophilic agent in a solvent that does not adversely influence the reaction.

As the organic phosphorus compound, for example, triphenylphosphine, tributylphosphine and the like can be mentioned.

As the electrophilic agent, for example, diethyl azodicarboxylate, diisopropyl azodicarboxylate, 1,1'-azodicarbonyldipiperidine and the like can be mentioned.

The amounts of the organic phosphorus compound and the electrophilic agent to be used are preferably about 1 to about 5 mol, per 1 mol of compound (III).

The amount of compound (II) to be used is preferably about 1 to about 5 mol, per 1 mol of compound (III).

As the solvent that does not adversely influence the reaction, for example, ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethyl sulfoxide and the like; and the like can be mentioned. These solvents may be used as a mixture thereof at an appropriate ratio.

The reaction temperature is generally about -50°C to about 150°C, preferably about -10°C to about 100°C.

The reaction time is generally about 0.5 to about 20 hr.

When E is a halogen atom or -OSO₂R²¹, this reaction is carried out according to a conventional method in the presence of a base in a solvent that does not adversely influence the reaction.

As the base, for example, alkali metal salts such as potassium hydroxide, sodium hydroxide, sodium hydrogencarbonate, potassium carbonate and the like; amines such as pyridine, triethylamine, N,N-dimethylaniline, 1,8-diazabicyclo[5.4.0]undec-7-ene and the like; metal hydrides such as potassium hydride, sodium hydride and the like; alkali metal alkoxides such as sodium methoxide, sodium ethoxide, potassium tert-butoxide and the like can be mentioned.

The amount of the base to be used is preferably about 1 to about 5 mol, per 1 mol of compound (III).

The amount of compound (II) to be used is preferably about 1 to about 5 mol, per 1 mol of compound (III).

As the solvent that does not adversely influence the reaction, for example, aromatic hydrocarbons such as benzene, toluene, xylene and the like; ethers such as tetrahydrofuran, dioxane, diethyl ether and the like; ketones such as acetone, 2-butanone and the like; halogenated hydrocarbons such as chloroform, dichloromethane and the like; amides such as N,N-dimethylformamide and the like; sulfoxides such as dimethyl sulfoxide and the like; and the like can be mentioned. These solvents may be used as a mixture thereof at an appropriate ratio.

The reaction temperature is generally about -50°C to about 150°C, preferably about -10°C to about 100°C.

The reaction time is generally about 0.5 to about 20 hr.

Compound (I-1) thus obtained can be isolated and purified by a known separation and purification means, such as concentration, concentration under reduced pressure, solvent extraction, crystallization, recrystallization, phase transfer, chromatography and the like.

Compound (II) used as a starting material compound in the above-mentioned Method A can be produced, for example, by the below-mentioned Method E. Compound (III) can be produced, for example, by the methods described in WO01/38325, WO03/99793 and the like, or a method analogous thereto.

Compound (I-1a) of the formula (I) wherein Yc is Yc¹ (wherein Yc¹ is an oxygen atom, a sulfur atom or -NR⁵- (wherein R⁵ is an alkyl group having 1 to 4 carbon atoms)) can be produced, for example, by the following Method A2. 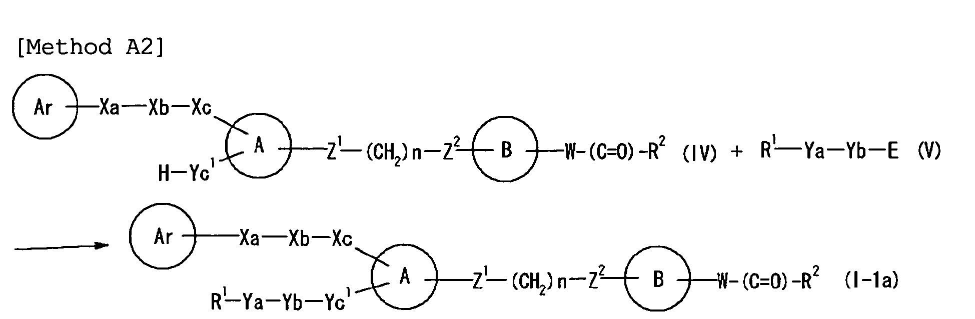 wherein the symbols in the formula are as defined above.

In this method, compound (I-1a) can be produced by reacting compound (IV) with compound (V).

This method is performed in the same manner as in the aforementioned Method A.

Compound (I-1a) thus obtained can be isolated and purified by a known separation and purification means, such as concentration, concentration under reduced pressure, solvent extraction, crystallization, recrystallization, phase transfer, chromatography and the like.

Compound (IV) used as a starting material compound in the above-mentioned Method A2 can be produced, for example, by the below-mentioned Method F. Compound (V) can be produced by a method known per se.

Compound (I-3) of the formula (I) wherein Ya is -S(O)m-(wherein m is 1 or 2) can be produced, for example, by the following Method B. 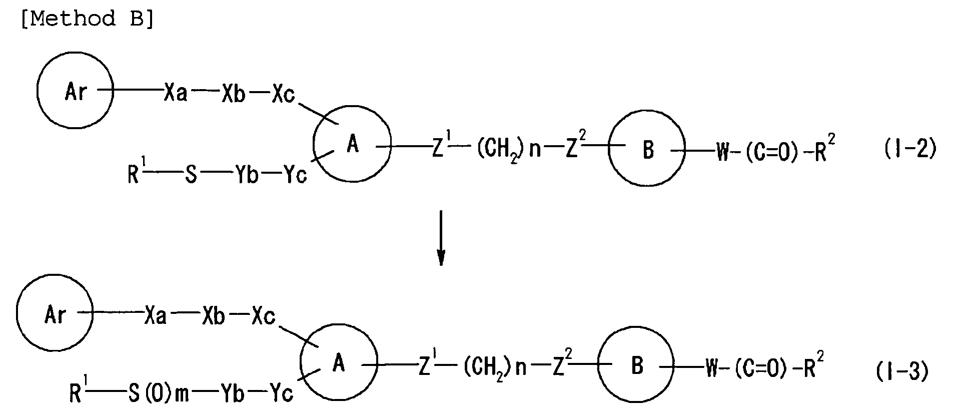 wherein the symbols in the formula are as defined above.

In this method, compound (I-3) can be produced by subjecting compound (I-2) to an oxidation reaction. This reaction is generally carried out in the presence of an oxidant in a solvent that does not adversely influence the reaction.

As the oxidant, for example, 3-chloroperbenzoic acid, sodium periodate, hydrogen peroxide, peracetic acid and the like can be mentioned.

The amount of the oxidant to be used is preferably about 1 to about 10 mol, per 1 mol of compound (I-2).

As the solvent that does not adversely influence the reaction, for example, ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; alcohols such as ethanol, methanol and the like; and the like can be mentioned. These solvents may be used as a mixture thereof at an appropriate ratio.

The reaction temperature is generally about -50°C to about 150°C, preferably about -10°C to about 100°C.

The reaction time is generally about 0.5 to about 20 hr.

Compound (I-3) thus obtained can be isolated and purified by a known separation and purification means, such as concentration, concentration under reduced pressure, solvent extraction, crystallization, recrystallization, phase transfer, chromatography and the like.

Compound (I-2) used as a starting material compound in the above-mentioned Method B can be produced, for example, by the above-mentioned Method A or A2.

Compound (I-5) of the formula (I) wherein R² is -OH can also be produced, for example, by the following Method C. 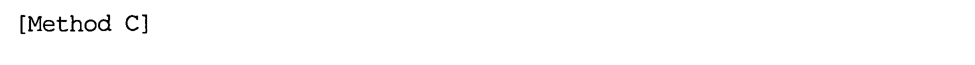 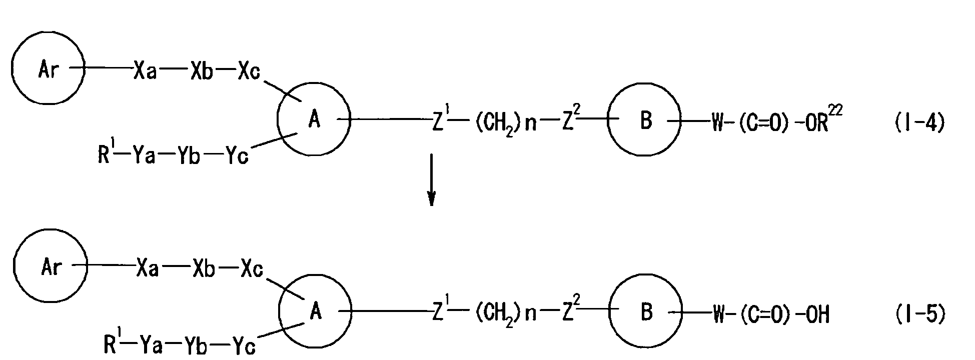 wherein R²² is an optionally substituted hydrocarbon group, and the other symbols are as defined above.

In this method, compound (I-5) can be produced by subjecting compound (I-4) to a hydrolysis reaction.

As used herein, the "optionally substituted hydrocarbon group" for the above-mentioned R²² is as defined for the "optionally substituted hydrocarbon group" for the aforementioned R⁸. R²² is preferably an alkyl group having 1 to 6 carbon atoms, more preferably methyl, ethyl or the like.

This reaction is carried out by a conventional method in the presence of an acid or base in a water-containing solvent.

As the acid, for example, inorganic acids such as hydrochloric acid, sulfuric acid, hydrobromic acid and the like; organic acids such as acetic acid and the like; and the like can be mentioned.

As the base, for example, alkali metal carbonates such as potassium carbonate, sodium carbonate and the like; alkali metal C₁₋₆ alkoxides such as sodium methoxide and the like; alkali metal hydroxides such as potassium hydroxide, sodium hydroxide, lithium hydroxide and the like; and the like can be mentioned.

The amount of the acid or base to be used is generally an excess amount relative to compound (1-4). The amount of the acid to be used is preferably about 2 to about 50 equivalents relative to compound (I-4) and the amount of the base to be used is about 1.2 to about 5 equivalents relative to compound (I-4).

As the water-containing solvent, for example, a mixed solvent of water with one or more kinds of solvents selected from alcohols such as methanol, ethanol and the like; ethers such as tetrahydrofuran, dioxane, diethyl ether and the like; dimethyl sulfoxide, acetone and the like; and the like can be mentioned.

The reaction temperature is generally about -20°C to about 150°C, preferably about -10°C to about 100°C.

The reaction time is generally about 0.1 to about 20 hr.

Compound (I-5) thus obtained can be isolated and purified by a known separation and purification means, such as concentration, concentration under reduced pressure, solvent extraction, crystallization, recrystallization, phase transfer, chromatography and the like.

Compound (I-4) to be used as a starting material compound in the above-mentioned Method C can be produced, for example, by the above-mentioned Method A, Method A2 or Method B.

Compound (1-6) of the formula (I) wherein R² is -NR⁹R¹⁰ (wherein R⁹ and R¹⁰ are as defined above) can also be produced, for example, by the following Method D. 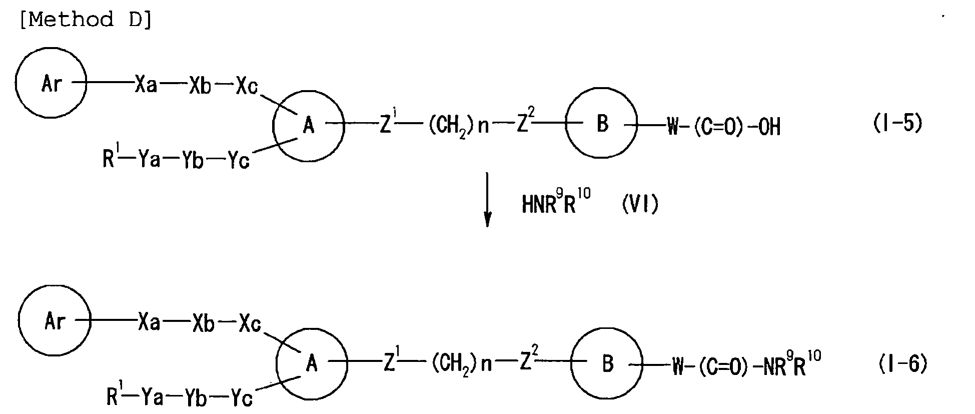 wherein the symbols in the formula are as defined above.

In this method, compound (I-6) can be produced by subjecting compound (I-5) to an amidation reaction. This reaction is carried out by a method known per se, for example, a method including directly condensing compound (I-5) with compound (VI), or a method including reacting a reactive derivative of compound (I-5) with compound (VI) and the like. Here, as the reactive derivative of compound (I-5), for example, an acid anhydride, an acid halide (e.g., an acid chloride, an acid bromide), an imidazolide, a mixed acid anhydride (e.g., an anhydride with methyl carbonate, ethyl carbonate or isobutyl carbonate, and the like) and the like can be mentioned.

The method including directly condensing compound (I-5) with compound (VI) is carried out in the presence of a condensing reagent, in a solvent that does not adversely influence the reaction.

As the condensing reagent, for example, generally known condensing reagents such as carbodiimide condensing reagents (e.g., dicyclohexylcarbodiimide, diisopropylcarbodiimide, 1-ethyl-3-dimethylaminopropylcarbodiimide and hydrochloride thereof); phosphoric acid condensing reagents such as diethyl cyanophosphate, diphenylphosphoryl azide and the like; carbonyldiimidazole, 2-chloro-1,3-dimethylimidazolium tetrafluoroborate, and the like can be mentioned.

The amount of the condensing reagent to be used is generally 0.1 to 10 mol, preferably 0.3 to 3 mol, per 1 mol of compound (I-5).

The amount of compound (VI) to be used is generally 0.1 to 10 mol, preferably 0.3 to 3 mol, per 1 mol of compound (I-5).

As the solvent that does not adversely influence the reaction, for example, amides such as N,N-dimethylformamide, N,N-dimethylacetamide and the like; halogenated hydrocarbons such as chloroform, dichloromethane and the like; aromatic hydrocarbons such as benzene, toluene and the like; ethers such as tetrahydrofuran, dioxane, diethyl ether and the like; ethyl acetate, water and the like can be mentioned. These solvents may be used as a mixture thereof at an appropriate ratio.

When a carbodiimide condensing reagent is used as a condensing reagent, a suitable condensing promoter (e.g., 1-hydroxy-7-azabenzotriazole, 1-hydroxybenzotriazole, N-hydroxysuccinimide, N-hydroxyphthalimide) is used as necessary to improve the reaction efficiency. When a phosphoric acid condensing reagent is used as a condensing reagent, an organic amine base such as triethylamine and the like is generally added to improve the reaction efficiency.

The amount of the above-mentioned condensing promoter and the organic amine base to be used is generally 0.1 to 10 mol, preferably 0.3 to 3 mol, per 1 mol of compound (I-5).

The reaction temperature is generally -30°C to 100°C.

The reaction time is generally 0.5 to 60 hr.

In a method using a reactive derivative of compound (I-5), when, for example, an acid halide is used as the reactive derivative of compound (I-5), the reaction is carried out in the presence of a base in a solvent that does not adversely influence the reaction.

As the base, for example, amines such as triethylamine, N-methylmorpholine, N,N-dimethylaniline and the like; alkali metal salts such as sodium hydrogencarbonate, sodium carbonate, potassium carbonate and the like; and the like can be mentioned.

The amount of the base to be used is generally 0.1 to 10 mol, preferably 0.3 to 3 mol, per 1 mol of compound (I-5).

The amount of compound (VI) to be used is generally 0.1 to 10 mol, preferably 0.3 to 3 mol, per 1 mol of compound (I-5).

As the solvent that does not adversely influence the reaction, for example, halogenated hydrocarbons such as chloroform, dichloromethane and the like; aromatic hydrocarbons such as benzene, toluene and the like; ethers such as tetrahydrofuran, dioxane, diethyl ether and the like, ethyl acetate, water and the like can be mentioned. These solvents may be used as a mixture thereof at an appropriate ratio.

The reaction temperature is generally -30°C to 100°C. The reaction time is generally 0.5 to 20 hr.

When a mixed acid anhydride is used as a reactive derivative of compound (I-5), compound (I-5) is reacted with a chlorocarbonate (e.g., methyl chlorocarbonate, ethyl chlorocarbonate, isobutyl chlorocarbonate) in the presence of a base (e.g., amines such as triethylamine, N-methylmorpholine, N,N-dimethylaniline and the like; alkali metal salts such as sodium hydrogencarbonate, sodium carbonate, potassium carbonate and the like), and then reacted with compound (VI).

The amount of compound (VI) to be used is generally 0.1 to 10 mol, preferably 0.3 to 3 mol, per 1 mol of compound (I-5).

The reaction temperature is generally -30°C to 100°C. The reaction time is generally 0.5 to 20 hr.

Compound (I-6) thus obtained can be isolated and purified by a known separation and purification means, such as concentration, concentration under reduced pressure, solvent extraction, crystallization, recrystallization, phase transfer, chromatography and the like.

Compound (I-5) used as a starting material compound in the above-mentioned Method D can be produced, for example, by the above-mentioned Method A to Method C. Compound (VI) can be produced by a method known per se.

Compound (II-1), which is compound (II) (used as a starting material compound in the above-mentioned Method A) wherein Z1 is a bond, n is 3, and E is a hydroxy group, can be produced, for example, by the following Method E. 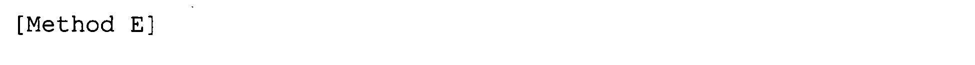 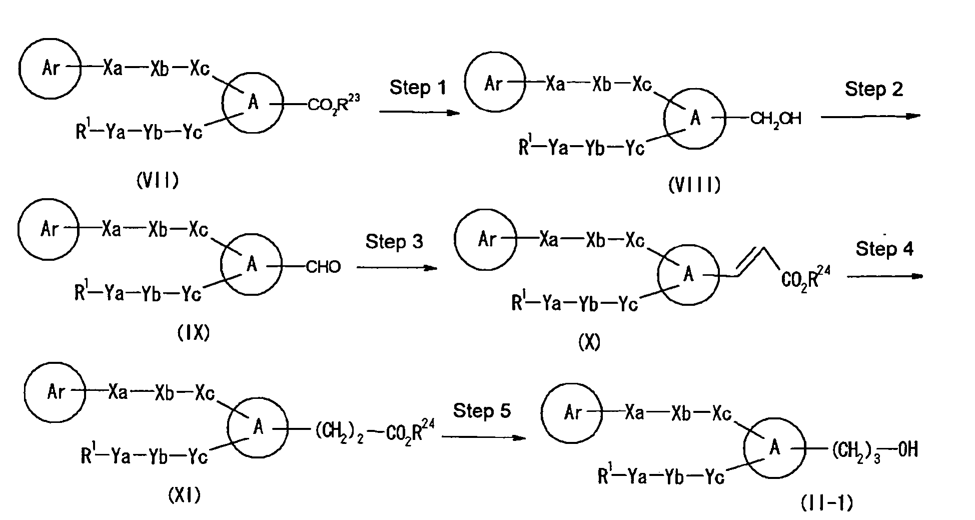 wherein R²³ and R²⁴ are the same or different and each is a alkyl group having 1 to 6 carbon atoms, and the other symbols are as defined above.

As the "alkyl group having 1 to 6 carbon atoms" for R²³ or R²⁴, those exemplified as the aforementioned R²² can be mentioned.

### [Step 1]

In this step, compound (VIII) can be produced by subjecting compound (VII) to a reduction reaction.

This reaction is generally carried out in the presence of a reducing agent in a solvent that does not adversely influence the reaction.

As the reducing agent, for example, metal hydrogen compounds such as sodium bis(2-methoxyethoxy)aluminum hydride, diisobutylaluminum hydride and the like; metal hydrogen complex compounds such as sodium borohydride, sodium cyanoborohydride, lithium aluminum hydride, sodium aluminum hydride and the like; and the like can be mentioned.

The amount of the reducing agent to be used is generally 1 to 20 mol, per 1 mol of compound (VII).

As the solvent that does not adversely influence the reaction, for example, alcohols such as methanol, ethanol, propanol, 2-propanol, butanol, isobutanol, tert-butanol and the like; aromatic hydrocarbons such as benzene, toluene, xylene and the like; aliphatic hydrocarbons such as hexane, heptane and the like; ethers such as diethyl ether, diisopropyl ether, tert-butyl methyl ether, tetrahydrofuran, dioxane, dimethoxyethane and the like; amides such as N,N-dimethylformamide, N,N-dimethylacetamide, N-methylpyrrolidone and the like; halogenated hydrocarbons such as dichloromethane, chloroform, 1,2-dichloroethane, 1,1,2,2-tetrachloroethane and the like; and the like can be mentioned. These solvents may be used as a mixture thereof at an appropriate ratio.

The reaction temperature is generally -70°C to 150°C, preferably -20°C to 100°C.

The reaction time is generally 0.1 to 100 hr, preferably 0.1 to 40 hr.

Compound (VIII) thus obtained can be isolated and purified by a known separation and purification means, such as concentration, concentration under reduced pressure, solvent extraction, crystallization, recrystallization, phase transfer, chromatography and the like.

Compound (VII) can be produced, for example, by methods described in WO01/38325 and the like, or a method analogous thereto.

### [Step 2]

In this step, compound (IX) can be produced by subjecting compound (VIII) to an oxidation reaction. This reaction is generally carried out in the presence of an oxidant in a solvent that does not adversely influence the reaction.

As the oxidant, for example, metal oxidants such as manganese dioxide, pyridinium chlorochlomate, pyridinium dichlorochlomate, ruthenium oxide and the like can be mentioned.

The amount of the metal oxidant to be used is generally 1 to 50 mol, preferably 1 to 10 mol, per 1 mol of compound (VIII).

As the solvent that does not adversely influence the reaction, for example, ethers such as diethyl ether, tetrahydrofuran, dioxane and the like; halogenated hydrocarbons such as chloroform, dichloromethane and the like; aromatic hydrocarbons such as benzene, toluene, xylene and the like; and the like can be mentioned. These solvents may be used as a mixture thereof at an appropriate ratio.

The reaction temperature is generally about -50°C to about 150°C, preferably about -10°C to about 100°C.

The reaction time is generally about 0.5 to about 20 hr.

Compound (IX) can also be produced by dissolving compound (VIII) in dimethyl sulfoxide or a mixed solvent of dimethyl sulfoxide and halogenated hydrocarbon (e.g., chloroform, dichloromethane) at an appropriate ratio, adding a sulfur trioxide pyridine complex or oxalyl chloride, and reacting with an organic base (e.g., triethylamine, N-methylmorpholine).

The amount of the sulfur trioxide pyridine complex or oxalyl chloride to be used is generally 1 to 50 mol, preferably 1 to 10 mol, per of 1 mol of compound (VIII).

The amount of the organic base to be used is generally 1 to 50 mol, preferably 1 to 10 mol, per of 1 mol of compound (VIII).

The reaction temperature is generally about -100°C to about 150°C, preferably about -70°C to about 100°C.

The reaction time is generally about 0.5 to about 20 hr.

Compound (IX) obtained thus can also be isolated and purified by a known separation and purification means, such as concentration, concentration under reduced pressure, solvent extraction, crystallization, recrystallization, phase transfer, chromatography and the like.

### [Step 3]

In this step, compound (X) can be produced by subjecting compound (IX) to a carbon addition reaction. This reaction is generally carried out using an organic phosphorus reagent in a solvent that does not adversely influence the reaction, and in the presence of a base.

As the base, for example, alkali metal salts such as potassium hydroxide, sodium hydroxide, sodium hydrogencarbonate, potassium carbonate and the like; amines such as pyridine, triethylamine, N,N-dimethylaniline, 1,8-diazabicyclo[5.4.0]undec-7-ene and the like; metal hydrides such as potassium hydride, sodium hydride and the like; alkali metal C₁₋₆ alkoxides such as sodium methoxide, sodium ethoxide, potassium tert-butoxide and the like can be mentioned.

The amount of the base to be used is generally 1 to 50 mol, preferably 1 to 10 mol, per of 1 mol of compound (IX).

As the organic phosphorus reagent, for example, trimethyl phosphonoacetate, methyl diethylphosphonoacetate, triethyl phosphonoacetate, tert-butyl diethylphosphonoacetate and the like can be mentioned.

The amount of the organic phosphorus reagent to be used is generally 1 to 50 mol, preferably 1 to 10 mol, per of 1 mol of compound (IX).

As the solvent that does not adversely influence the reaction, for example, aromatic hydrocarbons such as benzene, toluene, xylene and the like; aliphatic hydrocarbons such as hexane, heptane and the like ; ethers such as diethyl ether, diisopropyl ether, tert-butyl methyl ether, tetrahydrofuran, dioxane, dimethoxyethane and the like; halogenated hydrocarbons such as chloroform, dichloromethane and the like; amides such as N,N-dimethylformamide, N,N-dimethylacetamide, N-methylpyrrolidone and the like ; sulfoxides such as dimethyl sulfoxide and the like; and the like can be mentioned. These solvents may be used as a mixture thereof at an appropriate ratio.

The reaction temperature is generally about -100°C to about 150°C, preferably about -10°C to about 100°C.

The reaction time is generally about 0.5 to about 20 hr.

Compound (X) obtained thus can be isolated and purified by a known separation and purification means, such as concentration, concentration under reduced pressure, solvent extraction, crystallization, recrystallization, phase transfer, chromatography and the like.

### [Step 4]

In this step, compound (XI) can be produced by subjecting compound (X) to a hydrogenation reaction.

This reaction can be carried out, for example, in the presence of a metal catalyst such as palladium-carbon, palladium black, palladium chloride, platinum oxide, platinum black, platinum-palladium, Raney-nickel, Raney-cobalt and the like and a hydrogen source, in a solvent that does not adversely influence the reaction.

The amount of the metal catalyst to be used is generally 0.001 to 1000 mol, preferably 0.01 to 100 mol, per of 1 mol of compound (X).

As the hydrogen source, for example, hydrogen gas, formic acid, formic acid amine salt, phosphinate, hydrazine and the like can be mentioned.

As the solvent that does not adversely influence the reaction, those exemplified in the aforementioned Step 1 can be used.

The reaction temperature and the reaction time are the same as in the aforementioned Step 1.

Compound (XI) obtained thus can also be isolated and purified by a known separation and purification means, such as concentration, concentration under reduced pressure, solvent extraction, crystallization, recrystallization, phase transfer, chromatography and the like.

### [Step 5]

This step is performed in the same manner as in the above-mentioned Step 1.

Compound (II-1) thus obtained can be isolated and purified by a known separation and purification means, such as concentration, concentration under reduced pressure, solvent extraction, crystallization, recrystallization, phase transfer, chromatography and the like.

Compound (IV) used as a starting material compound in the above-mentioned Method A2 can be produced, for example, by the below-mentioned Method F. 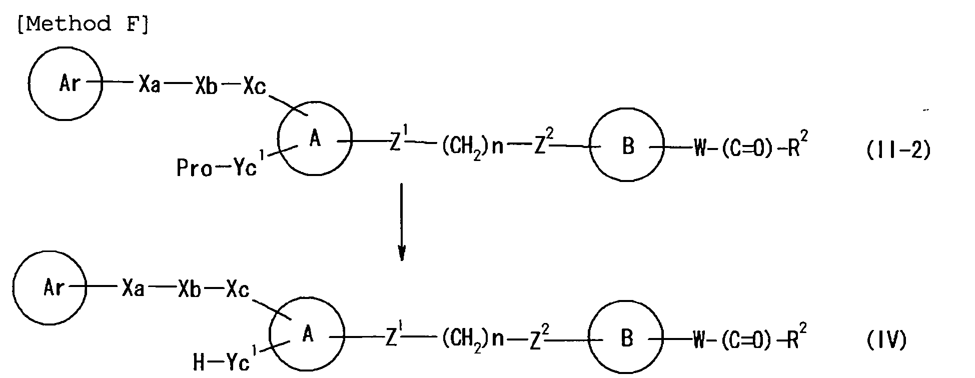 wherein Pro is a protecting group, and the other symbols are as defined above.

As the protecting group for Pro, a hydroxyl-protecting group, a mercapto-protecting group and an amino-protecting group can be mentioned, depending on the kind of Yc¹.

As used herein, as the hydroxyl-protecting group, those exemplified as the aforementioned R⁴ can be mentioned.

As the mercapto-protecting group, for example, a C₁₋₆ alkyl group (e.g., methyl, ethyl, tert-butyl), a phenyl group, a trityl group, a C₇₋₁₀ aralkyl group (e.g., benzyl), a C₁₋₆ alkyl-carbonyl group (e.g., acetyl, propionyl), a benzoyl group, a C₇₋₁₀ aralkyl-carbonyl group (e.g., benzylcarbonyl), a C₁₋₆ alkoxy-carbonyl group (e.g., methoxycarbonyl, ethoxycarbonyl, propoxycarbonyl, butoxycarbonyl, tert-butoxycarbonyl), a C₆₋₁₄ aryloxy-carbonyl group (e.g., phenyloxycarbonyl, naphthyloxycarbonyl), a C₇₋₁₃ aralkyloxy-carbonyl group (e.g., benzyloxycarbonyl, phenethyloxycarbonyl), a 2-tetrahydropyranyl group, a C₁₋₆ alkylamino-carbonyl group (e.g., methylaminocarbonyl, ethylaminocarbonyl), and the like can be mentioned. These groups are optionally substituted by 1 to 3 substituents selected from a halogen atom (e.g., fluorine, chlorine, bromine, iodine), a C₁₋₆ alkyl group (e.g., methyl, ethyl, propyl), a phenyl group, a C₁₋₆ alkoxy group (e.g., methoxy, ethoxy, propoxy, isobutoxy), an acylamino group (e.g., acetylamino, benzoylamino), a cyano group and a nitro group. When the ring has 2 or more substituents, respective substituents may be the same or different.

As the amino-protecting group, those exemplified as the aforementioned R⁵ can be mentioned.

In this method, compound (IV) can be produced by subjecting compound (II-2) to a deprotection reaction.

This reaction is carried out by a method known per se, depending on the kind of the protecting group for Pro.

Compound (IV) thus obtained can be isolated and purified by a known separation and purification means, such as concentration, concentration under reduced pressure, solvent extraction, crystallization, recrystallization, phase transfer, chromatography and the like.

Compound (II-2) can be produced, for example, by the above-mentioned Method A.

In each of the aforementioned reactions, when the starting material compound has an amino group, a carboxyl group, a hydroxy group or a carbonyl group as a substituent, a protecting group generally used in the peptide chemistry and the like may be introduced into these groups, and the object compound can be obtained by eliminating the protecting group as necessary after the reaction.

As the amino-protecting group, those exemplified as the aforementioned R⁵ can be mentioned.

As the carboxyl-protecting group, for example, a C₁₋₆ alkyl group (e.g., methyl, ethyl, propyl, isopropyl, butyl, tert-butyl), a C₇₋₁₁ aralkyl group (e.g., benzyl), a phenyl group, a trityl group, a silyl group (e.g., trimethylsilyl, triethylsilyl, dimethylphenylsilyl, tert-butyldimethylsilyl, tert-butyldiethylsilyl), a C₂₋₆ alkenyl group (e.g., 1-allyl) and the like can be mentioned. These groups are optionally substituted by 1 to 3 substituents selected from a halogen atom (e.g., fluorine, chlorine, bromine, iodine), a C₁₋₆ alkoxy group (e.g., methoxy, ethoxy, propoxy), a nitro group and the like.

As the hydroxyl-protecting group, those exemplified as the aforementioned R⁴ can be mentioned.

As the carbonyl-protecting group, for example, a cyclic acetal (e.g., 1,3-dioxane), a non-cyclic acetal (e.g., a di-C₁₋₆ alkylacetal) and the like can be mentioned.

For elimination of the above-mentioned protecting group, a method known *per se*, for example, a method described in Protective Groups in Organic Synthesis, John Wiley and Sons (1980) and the like can be mentioned. For example, employed is a method using acid, base, UV light, hydrazine, phenyl hydrazine, sodium N-methyldithiocarbamate, tetrabutylammonium fluoride, palladium acetate, trialkylsilyl halide (e.g., trimethylsilyl iodide, trimethylsilyl bromide and the like) and the like, reduction and the like.

When compound (I) contains an optical isomer, a stereoisomer, a positional isomer or a rotational isomer, they are also encompassed in compound (I) and can be obtained as single products by synthesis techniques and separation techniques known *per se.* For example, when compound (I) contains an optical isomer, an optical isomer separated from the compound is also encompassed in compound (I).

### Examples

The present invention is explained in detail in the following by referring to Experimental Example, Reference Examples, Examples and Formulation Examples, which are not to be construed as limitative.

In the following Reference Examples and Examples, % means wt% unless otherwise specified. In addition, room temperature means a temperature of 1-30°C.

### Experimental Example 1

### Blood glucose and blood lipid (triglyceride) lowering action in mice

The test compound was mixed with a powder diet (CE-2, CLEA JAPAN, INC.) at a proportion of 0.005% (0.01% for the compound of Example 105) and freely given to KKA^{y} mice (9 to 12-week-old, 5 mice per group) which are obesity insulin independent diabetes mellitus (type-2 diabetes) models, for 4 days. During this period, water was freely given. The blood was drawn from the orbital venous plexus. Glucose and triglyceride in the plasma separated from the blood were quantitated using L Type Wako Glu2 (Wako Pure Chemical Industries, Ltd.) and L Type Wako TG·H (Wako Pure Chemical Industries, Ltd.), respectively, according to an enzyme method. The results are shown in Table 1.

In the Table, the "hypoglycemic action (%)" shows lowering rate (%) of the blood glucose level of the test compound administration group when the blood glucose level of the test compound non-administration group is 100%. In addition, the "hypolipidemic action (%)" shows a lowering rate (%) of the blood triglyceride of the test compound administration group when the blood triglyceride value of the test compound non-administration group is 100%.

**Table 1**

| test compound | hypoglycemic action | hypolipidemic action |
|---|---|---|
| Example No. | (%) | (%) |
| 16 | 57 | 28 |
| 17 | 57 | 78 |
| 21 | 51 | 78 |
| 22 | 49 | 53 |
| 31 | 44 | 55 |
| 60 | 54 | 76 |
| 61 | 51 | 90 |
| 62 | 48 | 61 |
| 64 | 59 | 71 |
| 76 | 64 | 57 |
| 77 | 63 | 42 |
| 78 | 63 | 55 |
| 86 | 51 | 51 |
| 95 | 65 | 65 |
| 105 | 64 | 64 |
| 111 | 64 | 45 |
| 117 | 49 | 18 |
| 122 | 72 | 33 |
| 136 | 57 | 38 |
| 141 | 44 | 41 |
| 149 | 57 | 51 |
| 150 | 58 | 54 |

As shown above, it is clear that the compound of the present invention has superior hypoglycemic action and hypolipidemic action, and is useful as an agent for the prophylaxis or treatment of diabetes, hyperlipidemia (particularly, hypertriglyceridemia), impaired glucose tolerance and the like.

### Experimental Example 2

### Plasma antiatherogenic index increasing action in mice

The test compound was mixed with a powder diet (CE-2, CLEA JAPAN, INC.) at a proportion of 0.005% (0.01% for the compound of Example 105) and freely given to KKA^{y} mice (9 to 12-week-old, 5 mice per group) which are obesity insulin independent diabetes mellitus (type-2 diabetes) models, for 4 days. During this period, water was freely given. The blood was drawn from fundus and the components were measured using the plasma separated from the blood. The total cholesterol was quantified using L Type Wako cholesterol (Wako Pure Chemical Industries, Ltd.). In addition, an HDL cholesterol precipitation reagent (Wako Pure Chemical Industries, Ltd.) was added to a part of the plasma to allow precipitation of a non-HDL lipoprotein, and the cholesterol (HDL cholesterol) of the supernatant was measured. The plasma antiatherogenic index [(HDL cholesterol/total cholesterol)×100] was calculated from these cholesterol values. The results are shown in Table 2. In the Table, the "plasma antiatherogenic index increasing action (%)" shows the increase rate (%) in the plasma antiatherogenic index of the test compound administration group when the plasma antiatherogenic index of the test compound non-administration group is 100%.

**Table 2**

| test compound | plasma antiatherogenic index increasing action (%) |
|---|---|
| Example No. | |
| 16 | 13 |
| 22 | 12 |
| 51 | 15 |
| 57 | 15 |
| 56 | 12 |
| 62 | 13 |
| 105 | 13 |
| 107 | 13 |
| 108 | 12 |
| 111 | 11 |

As shown above, it is clear that the compound of the present invention has a superior total cholesterol lowering action, and is useful as an agent for the prophylaxis or treatment of hyperlipidemia (particularly, hypercholesterolemia) and the like. It is also clear that the compound of the present invention has a superior plasma antiatherogenic index increasing action, and is useful as an agent for the prophylaxis or treatment of hyperlipidemia (particularly, hypoHDL-emia), arteriosclerosis and the like.

### Experimental Example 3 (PPARγ-RXRα heterodimer ligand activity)

The PPARγ:RXRα:4ERPP/CHO-K1 cells described in WO03/099793 were cultured in a Ham F12 medium [manufactured by Life Technologies, Inc., US] containing 10% calf fetal serum [manufactured by Life Technologies, Inc., US], sown in a 96-well white plate [manufactured by Corning Coster Corporation, US] at 2×10⁴ cells/well, and incubated overnight in a carbon dioxide gas incubator at 37°C.

Then, the medium was removed from the 96 well white plate, 80 µl of Ham F12 medium containing 0.1% fatty acid-free bovine serum albumin (BSA) and a test compound (20 µl) were added, and the cells were incubated for 18 - 24 hr in a carbon dioxide gas incubator at 37°C. The medium was removed, 40 µl of PicaGene 7.5 (manufactured by Wako Pure Chemical Industries, Ltd.) diluted 2-fold with HBSS (HANKS' BALANCED SALT SOLUTION) [manufactured by BIO WHITTAKER, US] was added. After stirring, the luciferase activity was determined using the 1420 ARVO Multilabel Counter [manufactured by PerkinElmer, US].

The induction rate was calculated from the luciferase activity of each test compound based on the luciferase activity of the test compound non-administration group as 1. The test compound concentration and the induction rate were analyzed by PRISM [manufactured by GraphPad Software, Inc., US] to calculate EC₅₀ value (compound concentration showing 50% of the maximum value of induction rate) of the test compound. The results are shown in Table 3.

**Table 3**

| test compound | EC₅₀ |
|---|---|
| Example No. | (nM) |
| 16 | 2.3 |
| 17 | 6.2 |
| 19 | 3.2 |
| 21 | 41 |
| 22 | 15 |
| 31 | 14 |
| 33 | 30 |
| 34 | 1.7 |
| 35 | 2.7 |
| 36 | 20 |
| 37 | 6.4 |
| 51 | 39 |
| 57 | 1.4 |
| 59 | 23 |
| 61 | 25 |
| 62 | 34 |
| 64 | 11 |
| 66 | 1.2 |
| 68 | 6.0 |
| 71 | 39 |
| 73 | 3.0 |
| 75 | 1.9 |
| 76 | 1.5 |
| 77 | 5.4 |
| 78 | 1.3 |
| 80 | 5.4 |
| 82 | 26 |
| 83 | 18 |
| 85 | 2.6 |
| 86 | 2.5 |
| 92 | 5.1 |
| 93 | 42 |
| 95 | 2.4 |
| 98 | 5.1 |
| 111 | 3.8 |
| 112 | 0.57 |
| 116 | 1.6 |
| 117 | 0.78 |
| 118 | 0.75 |
| 120 | 3.3 |
| 121 | 3.3 |
| 122 | 4.1 |
| 132 | 11 |
| 133 | 1.2 |
| 136 | 0.42 |
| 137 | 0.61 |
| 138 | 1.2 |
| 139 | 9.0 |
| 141 | 1.6 |
| 142 | 7.0 |
| 143 | 1.4 |
| 144 | 3.7 |
| 145 | 2.6 |
| 147 | 0.35 |
| 149 | 5.0 |
| 150 | 2.6 |
| 156 | 0.99 |
| 158 | 7.1 |
| 160 | 5.3 |
| 166 | 24 |
| 177 | 1.5 |
| 196 | 16 |

As mentioned above, it is clear that the compound of the present invention has a superior PPARγ-RXRα heterodimer ligand activity.

### Experimental Example 4 (PPARδ-RXRα heterodimer ligand activity)

The transformant described in Reference Example 9a of WO03/099793 was suspended in a DMEM medium [manufactured by Life Technologies, Inc., US] containing 0.1% fatty acid-free bovine serum albumin (BSA) (manufactured by Wako Pure Chemical Industries, Ltd.), and inoculated to each well of a 96 well white plate [manufactured by Corning Coster Corporation, US] by 80 µl to 1×10⁴ cells/well. Then, the test compound (20 µl) was added, and cultured under the conditions of 37°C, 5% CO₂ for 36 - 48 hr. The medium was removed from the 96 well white plate, 40 µl of PicaGene LT 7.5 (manufactured by Wako Pure Chemical Industries, Ltd.) diluted 2-fold with HBSS (HANKS' BALANCED SALT SOLUTION) [manufactured by BIO WHITTAKER, US] was added and, after stirring, the luciferase activity was determined using the 1420 ARVO Multilabel Counter [manufactured by PerkinElmer, US].

The induction rate was calculated from the luciferase activity of each test compound based on the luciferase activity of the test compound non-administration group as 1. The test compound concentration and the induction rate were analyzed by PRISM [manufactured by GraphPad Software, Inc., US] to calculate EC₅₀ value (compound concentration showing 50% of the maximum value of induction rate) of the test compound. The results are shown in Table 4.

**Table 4**

| test compound | EC₅₀ |
|---|---|
| Example No. | (nM) |
| 16 | 14 |
| 57 | 21 |
| 64 | 93 |
| 66 | 2.8 |
| 68 | 74 |
| 70 | 60 |
| 101 | 44 |
| 102 | 54 |
| 111 | 77 |
| 112 | 57 |
| 113 | 44 |
| 114 | 25 |
| 115 | 2.5 |
| 116 | 9.3 |
| 117 | 4.3 |
| 118 | 9.2 |
| 119 | 39 |
| 120 | 23 |
| 121 | 7.6 |
| 122 | 76 |
| 132 | 88 |
| 136 | 2.5 |
| 138 | 4.0 |
| 139 | 17 |
| 141 | 6.9 |
| 142 | 14 |
| 143 | 11 |
| 147 | 4.4 |
| 156 | 3.4 |
| 158 | 17 |
| 160 | 6.0 |
| 166 | 2.2 |
| 196 | 11 |

As shown above, it is clear that the compound of the present invention has superior PPARδ-RXRα heterodimer ligand activity.

### Reference Example 1

To tert-butyl 3-hydroxy-4-propyl-1H-pyrazole-1-carboxylate (8.88 g), (quinolin-2-yl)methanol (6.25 g) and tributylphosphine (20 ml) in tetrahydrofuran (200 ml) was added 1,1'-azodicarbonyldipiperidine (19.8 g), and the mixture was stirred at 60°C for 2 hr. The reaction mixture was concentrated, diisopropyl ether was added, and the precipitated crystals were filtered off. The filtrate was concentrated, and the obtained residue was subjected to silica gel column chromatography and eluted with ethyl acetate-hexane (2:1, v/v). The eluate was concentrated.

To a solution of the obtained oil in ethyl acetate (150 ml) was added 4N hydrogen chloride-ethyl acetate (150 ml), and the mixture was stirred overnight at room temperature. The reaction mixture was poured into saturated aqueous sodium hydrogencarbonate (400 ml), and the organic layer was separated. The organic layer was washed with brine, dried (MgSO₄), filtered and concentrated. The obtained crystals were collected by filtration while washing with hexane and dried to give 2-{[(4-propyl-1H-pyrazol-3-yl)oxy]methyl}quinoline as colorless crystals (5.73 g, yield 55%). melting point 94 - 95°C.

### Reference Example 2

To a solution of 2-{[(4-propyl-1H-pyrazol-3-yl)oxy]methyl}quinoline (3.0 g) in N,N-dimethylformamide (15 ml) was added sodium hydride (0.58 g, 60% in oil), and the mixture was stirred at room temperature for 1 hr. Then, ethyl bromoacetate (1.4 ml) was added, and the mixture was stirred at room temperature for 2 hr. Water (100 ml) was added to the reaction mixture, and the precipitated crystals were collected by filtration, washed with water and dried. The obtained crude crystals were subjected to silica gel column chromatography and eluted with ethyl acetate-hexane (3:2, v/v) and the eluate was concentrated. Recrystallization from hexane-ethyl acetate gave ethyl [4-propyl-3-(quinolin-2-ylmethoxy)-1H-pyrazol-1-yl]acetate as colorless crystals (2.60 g, yield 66%). melting point 82 - 83°C.

### Reference Example 3

To a solution of ethyl [4-propyl-3-(quinolin-2-ylmethoxy)-1H-pyrazol-1-yl]acetate (2.2 g) in tetrahydrofuran (20 ml) was added under ice-cooling a 1.5M solution (14 ml) of diisobutylaluminum hydride in toluene over 20 min. After stirring the mixture at room temperature for 30 min, ethanol (10 ml) and saturated aqueous ammonium chloride (2 ml) were added. The precipitated solid was filtered and washed with acetone. The filtrate was concentrated, and the residue was subjected to silica gel column chromatography and eluted with acetone-hexane (2:3, v/v). The eluate was concentrated to give 2-[4-propyl-3-(quinolin-2-ylmethoxy)-1H-pyrazol-1-yl]ethanol as colorless crystals (1.70 g, yield 88%). melting point 61 - 62°C.

### Reference Example 4

To a solution of 2-{[(4-propyl-1H-pyrazol-3-yl)oxy]methyl}quinoline (2.0 g) in N,N-dimethylformamide (20 ml) was added sodium hydride (0.36 g, 60% in oil), and the mixture was stirred at room temperature for 30 min. Then, 2-(3-bromopropoxy)tetrahydro-2H-pyran (2.0 g) was added, and the mixture was stirred at 60°C for 2 hr. Water (30 ml) was added to the reaction mixture, and the mixture was extracted with ethyl acetate (30 mlx2). The organic layer was washed with brine, dried (MgSO₄), filtered and concentrated. The residue was subjected to silica gel column chromatography and eluted with ethyl acetate-hexane (2:3, v/v) to give a yellow oil (2.26 g).

To a solution of the obtained oil in methanol (20 ml) was added 10% hydrogen chloride-methanol (15 ml), and the mixture was stirred overnight at room temperature. The reaction mixture was neutralized with 4N aqueous sodium hydroxide, and the mixture was extracted with ethyl acetate (40 mlx2). The organic layer was washed with brine, dried (MgSO₄), filtered and concentrated. The residue was subjected to silica gel column chromatography and eluted with ethyl acetate-hexane (3:1, v/v) to give 3-[4-propyl-3-(quinolin-2-ylmethoxy)-1H-pyrazol-1-yl]propan-1-ol as a colorless oil (1.63 g, yield 67%). ¹H-NMR (300 MHz, CDCl₃) δ:0.95 (3 H, t, J= 7.3 Hz), 1.58 (2 H, sextet, J = 7.4 Hz), 1.83 - 2.00 (2 H, m), 2.38 (2 H, t, J = 7.5 Hz), 3.08 (1 H, brs), 3.54 (2 H, t, J = 5.4 Hz), 4.03 (2 H, t, J = 6.2 Hz), 5.50 (2 H, s), 7.00 (1 H, s), 7.46 - 7.86 (4 H, m), 8.03 - 8.23 (2 H, m).

### Reference Example 5

A mixture of methyl 3-hydroxy-1H-pyrazole-5-carboxylate (10.0 g), potassium carbonate (19.5 g), 2-(chloromethyl)quinoline hydrochloride (16.6 g) and N,N-dimethylformamide (150 ml) was stirred overnight at room temperature. Water (300 ml) was added to the reaction mixture, and the precipitated crystals were collected by filtration, washed with water and ethanol, and dried to give methyl 3-(quinolin-2-ylmethoxy)-1H-pyrazole-5-carboxylate as colorless crystals (5.48 g, yield 27%). melting point not less than 175°C (decomposition). ¹H-NMR (300 MHz, DMSO-d₆) δ:3.81 (3 H, s), 5.43 (2 H, s), 6.34 (1 H, s), 7.56 - 7.85 (3 H, m), 7.95 - 8.06 (2 H, m), 8.40 (1 H, d, J = 8.4Hz).

### Reference Example 6

To a suspension of sodium hydride (0.93 g, 60% in oil) in N,N-dimethylformamide (30 ml) was added methyl 3-(quinolin-2-ylmethoxy)-1H-pyrazole-5-carboxylate (5.48 g), and the mixture was stirred at room temperature for 30 min. 1-Iodopropane (2.2 ml) was added, and the mixture was stirred for 2 hr. Water (50 ml) was added to the reaction mixture, and the mixture was extracted with ethyl acetate (30 mlx2). The organic layer was washed with brine, dried (MgSO₄), filtered and concentrated. The residue was subjected to silica gel column chromatography and eluted with ethyl acetate-hexane (1:1, v/v) to give methyl 1-propyl-3-(quinolin-2-ylmethoxy)-1H-pyrazole-5-carboxylate (Reference Example 6a) as a yellow oil (3.08 g, yield 49%). ¹H-NMR (300 MHz, CDCl₃) δ:0.87 (3 H, t, J = 7.4 Hz), 1.73 - 1.88 (2 H, m), 3.84 (3 H, s), 4.38 (2 H, t, J= 7.1 Hz), 5.48 (2 H, s), 6.28 (1 H, s), 7.50 - 7.85 (4 H, m), 8.03 - 8.23 (2 H, m).

Then, methyl 1-propyl-5-(quinolin-2-ylmethoxy)-1H-pyrazole-3-carboxylate (Reference Example 6b) was obtained as yellow crystals (1.09 g, yield 17%). melting point 71 - 72°C.

### Reference Example 7

Methyl 1-propyl-3-(quinolin-2-ylmethoxy)-1H-pyrazole-5-carboxylate (1.09 g) was added under ice-cooling to a suspension of lithium aluminum hydride (0.16 g) in tetrahydrofuran (10 ml), and the mixture was stirred at room temperature for 30 min. Ethanol (2 ml) was slowly added to the reaction mixture, and water (0.5 ml) was added. The precipitated solid was filtered and washed with acetone. The filtrate was concentrated, and the obtained residue was subjected to silica gel column and eluted with ethyl acetone-hexane (2:3, v/v) to give [1-propyl-3-(quinolin-2-ylmethoxy)-1H-pyrazol-5-yl]methanol as colorless crystals (0.78 g, yield 78%). melting point 95 - 96°C.

### Reference Example 8

Methyl 1-propyl-5-(quinolin-2-ylmethoxy)-1H-pyrazole-3-carboxylate (1.50 g) was added under ice-cooling to a suspension of lithium aluminum hydride (0.25 g) in tetrahydrofuran (20 ml), and the mixture was stirred at room temperature for 30 min. Ethanol (2 ml) was slowly added to the reaction mixture, and water (0.7 ml) was added. The precipitated solid was filtered and washed with acetone. The filtrate was concentrated, and the residue was subjected to silica gel column chromatography and eluted with ethyl acetate-hexane (1:1, v/v) to give [1-propyl-5-(quinolin-2-ylmethoxy)-1H-pyrazol-3-yl]methanol as colorless crystals (0.70 g, yield 75%). melting point 98 - 99°C.

### Reference Example 9

To a solution of methyl 3-propoxy-1-(quinolin-2-ylmethyl)-1H-pyrazole-5-carboxylate (8.26 g) in tetrahydrofuran (100 ml) was added under ice-cooling a 1.5M solution (43.0 ml) of diisobutylaluminum hydride in toluene over 30 min. After stirring the mixture at room temperature for 30 min, ethanol (20 ml) and saturated aqueous ammonium chloride (20 ml) were added to the reaction mixture. The precipitated solid was filtered and washed with acetone. The filtrate was concentrated, and the residue was subjected to silica gel column chromatography and eluted with acetone-hexane (2:3, v/v) to give [3-propoxy-1-(quinolin-2-ylmethyl)-1H-pyrazol-5-yl]methanol as yellow crystals (3.60 g, yield 48%). melting point 90 - 91°C.

### Reference Example 10

A mixture of ethyl 3-hydroxy-1H-pyrazole-5-carboxylate (6.00 g), 2-iodopropane (4.20 ml), potassium carbonate (5.30 g) and N,N-dimethylformamide (50 ml) was stirred at room temperature for 5 hr. Water (80 ml) was added to the reaction mixture, and the mixture was extracted with ethyl acetate (50 mlx2). The organic layer was washed with brine, dried (MgSO₄), filtered and concentrated. The residue was subjected to silica gel column chromatography and eluted with ethyl acetate-hexane (1:2, v/v) to give ethyl 3-isopropoxy-1H-pyrazole-5-carboxylate as a yellow oil (5.06 g, yield 66%). ¹H-NMR (300 MHz, CDCl₃) δ:1.32 - 1.43 (9 H, m), 4.35 (2 H, q, J = 7.0 Hz), 4.75 (1 H, septet, J = 6.1 Hz), 6.19 (1 H, s), 10.56 (1 H, brs).

### Reference Example 11

A mixture of ethyl 3-isopropoxy-1H-pyrazole-5-carboxylate (5.06 g), 2-chloromethylquinoline hydrochloride (5.70 g), potassium carbonate (7.00 g) and N,N-dimethylformamide (30 ml) was stirred overnight at room temperature. Water (60 ml) was added to the reaction mixture, and the mixture was extracted with ethyl acetate (50 mlx2). The organic layer was washed with brine, dried (MgSO₄), filtered and concentrated. The residue was subjected to silica gel column chromatography and eluted with ethyl acetate-hexane (1:3 to 1:2, v/v) to give ethyl 3-isopropoxy-1-(quinolin-2-ylmethyl)-1H-pyrazole-5-carboxylate (5.41 g, yield 62%) as a yellow oil. ¹H-NMR (300 MHz, CDCl₃) δ:1.26 (3 H, t, J = 6.9 Hz), 1.35 (6 H, d, J = 6.3 Hz), 4.24 (2 H, q, J = 7.0 Hz), 4.75 (1 H, septet, J = 6.2 Hz), 5.93 (2 H, s), 6.33 (1 H, s), 6.90 (1 H, d, J = 8.7 Hz), 7.47 - 7.55 (1 H, m), 7.66 - 7.78 (2 H, m), 8.06 (1 H, d, J = 8.4 Hz).

### Reference Example 12

To a solution of ethyl 3-isopropoxy-1-(quinolin-2-ylmethyl)-1H-pyrazole-5-carboxylate (5.41 g) in tetrahydrofuran (100 ml) was added under ice-cooling a 1.5M solution (25.5 ml) of diisobutylaluminum hydride in toluene over 30 min. After stirring the mixture at room temperature for 30 min, ethanol (20 ml) and saturated aqueous ammonium chloride (15 ml) were added to the reaction mixture. The precipitated solid was filtered and washed with acetone. The filtrate was concentrated, and the obtained residue was subjected to silica gel column chromatography and eluted with ethyl acetate-hexane (1:2, v/v) to give [3-isopropoxy-1-(quinolin-2-ylmethyl)-1H-pyrazol-5-yl]methanol as a yellow oil (2.94 g, yield 62%). ¹H-NMR (300 MHz, CDCl₃) δ:1.27 (6 H, d, J = 6.2 Hz), 4.58 (1 H, septet, J = 6.2 Hz), 4.72 (2 H, brs), 5.39 (2 H, s), 5.69 (1 H, s), 6.46 (1 H, brs), 7.49 - 8.00 (5 H, m), 8.21 (1 H, d, J = 8.4 Hz).

### Reference Example 13

A mixture of methyl 3-hydroxy-1H-pyrazole-5-carboxylate (12.00 g), benzyl bromide (10.5 ml), potassium carbonate (11.7 g) and N,N-dimethylformamide (70 ml) was stirred at room temperature for 5 hr. Water (100 ml) was added to the reaction mixture, and the mixture was extracted with ethyl acetate (100 mlx2). The organic layer was washed with brine, dried (MgSO₄), filtered and concentrated. Diisopropyl ether was added to the residue, and the precipitated crystals were collected by filtration and dried to give methyl 1-benzyl-3-hydroxy-1H-pyrazole-5-carboxylate (Reference Example 13a) (4.23 g, yield 22%) as colorless crystals. melting point 177 - 178°C.

The filtrate was concentrated, subjected to silica gel column chromatography and eluted with ethyl acetate-hexane (1:2, v/v) to give methyl 3-benzyloxy-1H-pyrazole-5-carboxylate (Reference Example 13b) (11.39 g, yield 58%) as colorless crystals. melting point 82 - 84°C.

### Reference Example 14

A mixture of methyl 3-benzyloxy-1H-pyrazole-5-carboxylate (11.39 g), 2-iodopropane (5.4 ml), potassium carbonate (6.80 g) and N,N-dimethylformamide (50 ml) was stirred overnight at room temperature. Water (100 ml) was added to the reaction mixture, and the mixture was extracted with ethyl acetate (50 mlx2). The organic layer was washed with brine, dried (MgSO₄), filtered and concentrated. The residue was subjected to silica gel column chromatography and eluted with ethyl acetate-hexane (1:4 to 1:2, v/v) to give methyl 3-benzyloxy-1-isopropyl-1H-pyrazole-5-carboxylate (Reference Example 14a) (8.90 g, yield 66%) as a colorless oil. ¹H-NMR (300 MHz, CDCl₃) δ:1.44 (6 H, d, J = 6.6 Hz), 3.84 (3 H, s), 5.18 (2 H, s), 5.41 (1 H, septet, J = 6.6 Hz), 6.18 (1 H, s), 7.20 - 7.50 (5 H, m).

Then, methyl 5-benzyloxy-1-isopropyl-1H-pyrazole-3-carboxylate (Reference Example 14b) (3.70 g, yield 28%) was obtained as colorless crystals. melting point 73 - 74°C.

### Reference Example 15

A mixture of methyl 5-benzyloxy-1-isopropyl-1H-pyrazole-3-carboxylate (3.70 g), 10% palladium-carbon (0.75 g) and ethanol (25 ml) was stirred overnight at 50°C under a hydrogen atmosphere. The catalyst was filtered off, and the filtrate was concentrated to give methyl 5-hydroxy-1-isopropyl-1H-pyrazole-3-carboxylate as colorless crystals (2.35 g, yield 95%). melting point 162 - 163°C.

### Reference Example 16

A mixture of methyl 5-hydroxy-1-isopropyl-1H-pyrazole-3-carboxylate (2.35 g), potassium carbonate (3.90 g), 2-chloromethylquinoline hydrochloride (3.00 g) and N,N-dimethylformamide (25 ml) was stirred overnight at room temperature. Water (30 ml) was added to the reaction mixture, and the mixture was extracted with ethyl acetate (40 mlx2). The organic layer was washed with brine, dried (MgSO₄), filtered and concentrated. The residue was subjected to silica gel column chromatography and eluted with ethyl acetate-hexane (1:1, v/v) to give methyl 1-isopropyl-5-(quinolin-2-ylmethoxy)-1H-pyrazole-3-carboxylate as colorless crystals (3.28 g, yield 79%). melting point 106 - 107°C.

### Reference Example 17

To a solution of methyl 1-isopropyl-5-(quinolin-2-ylmethoxy)-1H-pyrazole-3-carboxylate (3.28 g) in tetrahydrofuran (30 ml) was added under ice-cooling a 1.5M solution (16.0 ml) of diisobutylaluminum hydride in toluene over 15 min. After stirring the mixture at room temperature for 30 min, ethanol (20 ml) and saturated aqueous ammonium chloride (5.0 ml) were added to the reaction mixture. The precipitated solid was filtered and washed with acetone. The filtrate was concentrated, ethyl acetate (30 ml) was added, and the mixture was dried (MgSO₄) and filtrated. The filtrate was concentrated, and the obtained crystals were collected by filtration while washing with diisopropyl ether and dried to give [1-isopropyl-5-(quinolin-2-ylmethoxy)-1H-pyrazol-3-yl]methanol as colorless crystals (2.50 g, yield 83%). melting point 118 - 119°C.

### Reference Example 18

A mixture of ethyl 3-(3-isopropoxy-1H-pyrazol-5-yl)propionate (1.00 g), 2-chloromethylquinoline hydrochloride (1.04 g), potassium carbonate (1.22 g) and N,N-dimethylformamide (15 ml) was stirred overnight at 50°C. Water (30 ml) was added to the reaction mixture, and the mixture was extracted with ethyl acetate (30 mlx2). The organic layer was washed with brine, dried (MgSO₄), filtered and concentrated. The residue was subjected to silica gel column chromatography and eluted with ethyl acetate-hexane (1:1, v/v) to give ethyl 3-[3-isopropoxy-1-(quinolin-2-ylmethyl)-1H-pyrazol-5-yl]propionate as a yellow oil (1.10 g, yield 68%). ¹H-NMR (300 MHz, CDCl₃) δ:1.20 (3 H, t, J = 7.2 Hz), 1.35 (6 H, d, J = 6.0 Hz), 2.54 (2 H, t, J = 7.7 Hz), 2.85 (2 H, t, J = 7.7 Hz), 4.09 (2 H, q, J = 7.2 Hz), 4.73 (1 H, septet, J = 6.1 Hz), 5.45 (2 H, s), 5.53 (1 H, s), 7.04 (1 H, d, J = 8.7 Hz), 7.47 - 7.58 (1 H, m), 7.68 - 7.83 (2 H, m), 8.02 - 8.13 (2 H, m).

### Reference Example 19

To a solution of ethyl 3-[3-isopropoxy-1-(quinolin-2-ylmethyl)-1H-pyrazol-5-yl]propionate (1.10 g) in tetrahydrofuran (20 ml) was added under ice-cooling a 1.5M solution (4.8 ml) of diisobutylaluminum hydride in toluene over 15 min. After stirring the mixture at room temperature for 30 min, ethanol (20 ml) and saturated aqueous ammonium chloride (2.0 ml) were added to the reaction mixture. The precipitated solid was filtered and washed with acetone. The filtrate was concentrated, and the obtained residue was subjected to silica gel column chromatography and eluted with ethyl acetate-hexane (3:1, v/v) to give 3-[3-isopropoxy-1-(quinolin-2-ylmethyl)-1H-pyrazol-5-yl]propan-1-ol as a colorless oil (0.64 g, yield 67%). ¹H-NMR (300 MHz, CDCl₃) δ:1.33 (6 H, d, J = 6.3 Hz), 1.73 - 1.88 (2 H, m), 2.72 (2 H, t, J = 7.4 Hz), 3.14 (1 H, brs), 3.59 (2 H, t, J = 5.7 Hz), 4.68 (1 H, septet, J = 6.2 Hz), 5.40 (2 H, s), 5.52 (1 H, s), 7.29 (1 H, d, J = 8.4 Hz), 7.50 - 7.58 (1 H, m), 7.67 - 7.84 (2 H, m), 8.03 - 8.17 (2 H, m).

### Reference Example 20

To a solution of ethyl 3-[3-propoxy-1-(quinolin-2-ylmethyl)-1H-pyrazol-5-yl]propionate (1.97 g) in tetrahydrofuran (40 ml) was added under ice-cooling a 1.5M solution (10.0 ml) of diisobutylaluminum hydride in toluene over 30 min. After stirring the mixture at room temperature for 30 min, ethanol (20 ml) and saturated aqueous ammonium chloride (3.0 ml) were added to the reaction mixture. The precipitated solid was filtered and washed with acetone. The filtrate was concentrated, and the obtained residue was subjected to silica gel column chromatography and eluted with ethyl acetate-hexane (6:1, v/v) to give 3-[3-propoxy-1-(quinolin-2-ylmethyl)-1H-pyrazol-5-yl]propan-1-ol as a yellow oil (1.21 g, yield 70%). ¹H-NMR (300 MHz, CDCl₃) δ:1.01 (3 H, t, J = 7.4 Hz), 1.68 - 1.90 (4 H, m), 2.73 (2 H, t, J = 7.2 Hz), 3.06 (1 H, brs), 3.59 (2 H, t, J = 6.0 Hz), 4.05 (2 H, t, J = 6.6 Hz), 5.41 (2 H, s), 5.54 (1 H, s), 7.29 (1 H, d, J = 8.4 Hz), 7.50 - 7.58 (1 H, m), 7.66 - 7.84 (2 H, m), 8.03 - 8.16 (2 H, m).

### Reference Example 21

A mixture of methyl 3-(benzyloxy)-1-isopropyl-1H-pyrazole-5-carboxylate (4.00 g), 5% palladium-carbon (0.80 g) and ethanol (25 ml) was stirred overnight at 50°C under a hydrogen atmosphere. The catalyst was filtered off, and the filtrate was concentrated to give crude crystals. A mixture of the obtained crude crystals, potassium carbonate (4.00 g), 2-chloromethylquinoline hydrochloride (3.40 g) and N,N-dimethylformamide (30 ml) was stirred at 70°C for 4 hr. Water (50 ml) was added to the reaction mixture, and the mixture was extracted with ethyl acetate (40 mlx2). The organic layer was washed with brine, dried (MgSO₄), filtered and concentrated. The residue was subjected to silica gel column chromatography and eluted with ethyl acetate-hexane (1:3, v/v) to give methyl 1-isopropyl-3-(quinolin-2-ylmethoxy)-1H-pyrazole-5-carboxylate as colorless crystals (3.53 g, yield 74%). melting point 64 - 65°C.

### Reference Example 22

To a solution of methyl 1-isopropyl-3-(quinolin-2-ylmethoxy)-1H-pyrazole-5-carboxylate (3.53 g) in tetrahydrofuran (50 ml) was added under ice-cooling a 1.5M solution (18.0 ml) of diisobutylaluminum hydride in toluene over 15 min. After stirring the mixture at room temperature for 30 min, ethanol (20 ml) and saturated aqueous ammonium chloride (4.0 ml) were added to the reaction mixture. The precipitated solid was filtered and washed with acetone. The filtrate was concentrated, ethyl acetate (30 ml) was added, and the mixture was dried (MgSO₄) and filtrated. The filtrate was concentrated, and the obtained crystals were collected by filtration while washing with diisopropyl ether and dried to give [1-isopropyl-3-(quinolin-2-ylmethoxy)-1H-pyrazol-5-yl]methanol as colorless crystals (2.90 g, yield 90%). melting point 137 - 138°C.

### Reference Example 23

Methyl 5-benzyloxy-1-isopropyl-1H-pyrazole-3-carboxylate (4.87 g) was added under ice-cooling to a suspension of lithium aluminum hydride (0.84 g) in tetrahydrofuran (25 ml) over 20 min, and the mixture was stirred at room temperature for 30 min. Ethanol (10 ml) was slowly added to the reaction mixture, and water (2.5 ml) was added. The precipitated solid was filtered and washed with acetone. The filtrate was concentrated, and the obtained residue was subjected to silica gel column chromatography and eluted with ethyl acetate-hexane (1:2, v/v) to give (5-benzyloxy-1-isopropyl-1H-pyrazol-3-yl)methanol as a colorless oil (3.19 g, yield 73%). ¹H-NMR (300 MHz, CDCl₃) δ:1.39 (6 H, d, J = 7.0 Hz), 2.44 (1 H, t, J = 5.9 Hz), 4.50 (1 H, septet, J = 6.8 Hz), 4.57 (1 H, d, J = 5.8 Hz), 5.07 (2 H, s), 5.58 (1 H, s), 7.30 - 7.43 (5 H, m).

### Reference Example 24

A mixture of (5-benzyloxy-1-isopropyl-1H-pyrazol-3-yl)methanol (3.19 g), manganese dioxide (15.0 g) and tetrahydrofuran (50 ml) was stirred overnight at room temperature. Manganese dioxide was filtered and thoroughly washed with acetone. The filtrate was concentrated to give 5-benzyloxy-1-isopropyl-1H-pyrazole-3-carbaldehyde as a yellow oil (2.59 g).

A solution of the obtained yellow oil and ethyl diethylphosphonoacetate (2.60 g) in tetrahydrofuran (5 ml) was added to a suspension of sodium hydride (0.50 g, 60% in oil) in N,N-dimethylformamide (20 ml) under ice-cooling over 15 min, and the mixture was stirred at room temperature for 1 hr. Water (50 ml) was added to the reaction mixture, and the mixture was extracted with ethyl acetate (50 mlx2). The organic layer was' washed with brine, dried (MgSO₄), filtered and concentrated. The residue was subjected to silica gel column chromatography and eluted with ethyl acetate-hexane (1:4, v/v) to give ethyl (2E)-3-(5-benzyloxy-1-isopropyl-1H-pyrazol-3-yl)propenoate as a colorless oil (2.75 g, yield 67%). ¹H-NMR (300 MHz, CDCl₃) δ:1.30 (3 H, t, J = 7.1 Hz), 1.43 (6 H, d, J = 6.6 Hz), 4.22 (2 H, q, J = 7.2 Hz), 4.55 (1 H, septet, J = 6.7 Hz), 5.09 (2 H, s), 5.78 (1 H, s), 6.24 (1 H, d, J = 15.8 Hz), 7.34 - 7.46 (5 H, m), 7.59 (1 H, d, J = 15.6 Hz).

### Reference Example 25

A mixture of ethyl (2E)-3-(5-benzyloxy-1-isopropyl-1H-pyrazol-3-yl)propenoate (2.75 g), 10% palladium-carbon (0.55 g) and ethanol (20 ml) was stirred at 50°C for 5 hr under a hydrogen atmosphere. The catalyst was filtered off, and the filtrate was concentrated to give ethyl 3-(4,5-dihydro-1-isopropyl-5-oxo-1H-pyrazol-3-yl)propionate as a yellow oil (1.97 g, yield 99%). ¹H-NMR (300 MHz, CDCl₃) δ:1.23 - 1.50 (9 H, m), 2.62 - 2.76 (4 H, m), 3.21 (2 H, s), 4.16 (2 H, q, J = 7.1 Hz), 4.38 (1 H, septet, J = 6.7 Hz).

### Reference Example 26

A mixture of ethyl 3-(4,5-dihydro-1-isopropyl-5-oxo-1H-pyrazol-3-yl)propionate (1.97 g), potassium carbonate (2.40 g), 2-chloromethylquinoline hydrochloride (2.05 g) and N,N-dimethylformamide (20 ml) was stirred overnight at 70°C. Water (30 ml) was added to the reaction mixture, and the mixture was extracted with ethyl acetate (30 mlx2). The organic layer was washed with brine, dried (MgSO₄), filtered and concentrated. The residue was subjected to silica gel column chromatography and eluted with ethyl acetate-hexane (1:1, v/v) to give ethyl 3-[1-isopropyl-5-(quinolin-2-ylmethoxy)-1H-pyrazol-3-yl]propionate as a yellow oil (1.58 g, yield 49%). ¹H-NMR (300 MHz, CDCl₃) δ:1.21 (3 H, t, J = 7.2 Hz), 1.46 (6 H, d, J = 6.9 Hz), 2.55 - 2.64 (2 H, m), 2.80 - 2.90 (2 H, m), 4.10 (2 H, q, J = 7.2 Hz), 4.57 (1 H, septet, J = 6.8 Hz), 5.35 (2 H, s), 5.38 (1 H, s), 7.54 - 7.63 (2 H, m), 7.72 - 7.88 (2 H, m), 8.08 (1 H, d, J = 8.1 Hz), 8.22 (1 H, d, J = 8.4 Hz).

### Reference Example 27

To a solution of ethyl 3-[1-isopropyl-5-(quinolin-2-ylmethoxy)-1H-pyrazol-3-yl]propionate (1.58 g) in tetrahydrofuran (20 ml) was added under ice-cooling a 1.5M solution (7.2 ml) of diisobutylaluminum hydride in toluene over 15 min. After stirring the mixture at room temperature for 30 min, ethanol (10 ml) and saturated aqueous ammonium chloride (1.8 ml) were added to the reaction mixture. The precipitated solid was filtered and washed with acetone. The filtrate was concentrated, ethyl acetate (30 ml) was added, and the mixture was dried (MgSO₄) and filtrated. The filtrate was concentrated to give 3-[1-isopropyl-5-(quinolin-2-ylmethoxy)-1H-pyrazol-3-yl]propan-1-ol as a yellow oil (1.28 g, yield 91%). ¹H-NMR (300 MHz, CDCl₃) δ:1.45 (6 H, d, J = 6.6 Hz), 1.80 - 1.94 (2 H, m), 2.66 (2 H, t, J = 6.7 Hz), 3.63 - 3.75 (3 H, m), 4.57 (1 H, septet, J = 6.8 Hz), 5.36 (2 H, s), 5.38 (1 H, s), 7.52 - 7.63 (1 H, m), 7.70 - 7.90 (2 H, m), 8.08 (1 H, d, J = 8.4 Hz), 8.22 (1 H, d, J = 8.4 Hz).

### Reference Example 28

A mixture of ethyl 3-(3-hydroxy-1-isopropyl-1H-pyrazol-5-yl)propionate (2.00 g), potassium carbonate (2.70 g), 2-chloromethylquinoline hydrochloride (2.10 g) and N,N-dimethylformamide (15 ml) was stirred overnight at 70°C. Water (30 ml) was added to the reaction mixture, and the mixture was extracted with ethyl acetate (30 mlx2). The organic layer was washed with brine, dried (MgSO₄), filtered and concentrated. The residue was subjected to silica gel column chromatography and eluted with ethyl acetate-hexane (1:2, v/v) to give ethyl 3-[1-isopropyl-3-(quinolin-2-ylmethoxy)-1H-pyrazol-5-yl]propionate as a yellow oil (2.25 g, yield 69%). ¹H-NMR (300 MHz, CDC1₃) δ:1.24 (3 H, t, J = 7.1 Hz), 1.41 (6 H, d, J = 6.6 Hz), 2.54 - 2.68 (2 H, m), 2.80 - 2.94 (2 H, m), 4.13 (2 H, q, J = 7.1 Hz), 4.32 (1 H, septet, J = 6.6 Hz), 5.43 (2 H, s), 5.49 (1 H, s), 7.48 - 7.58 (1 H, m), 7.65 - 7.86 (3 H, m), 8.07 (1 H, d, J = 8.4 Hz), 8.17 (1 H, d, J = 8.4 Hz).

### Reference Example 29

To a solution of ethyl 3-[1-isopropyl-3-(quinolin-2-ylmethoxy)-1H-pyrazol-5-yl]propionate (2.25 g) in tetrahydrofuran (30 ml) was added under ice-cooling a 1.5M solution (10.2 ml) of diisobutylaluminum hydride in toluene over 15 min. After stirring the mixture at room temperature for 30 min, ethanol (20 ml) and saturated aqueous ammonium chloride (2.4 ml) were added to the reaction mixture. The precipitated solid was filtered and washed with acetone. The filtrate was concentrated, and the obtained residue was subjected to silica gel column chromatography and eluted with ethyl acetate-hexane (4:1, v/v) to give 3-[1-isopropyl-3-(quinolin-2-ylmethoxy)-1H-pyrazol-5-yl]propan-1-ol as a colorless oil (1.69 g, yield 85%). ¹H-NMR (300 MHz, CDCl₃) δ:1.40 (6 H, d, J = 6.6 Hz), 1.57 (1 H, brs), 1.80 - 1.92 (2 H, m), 2.65 (2 H, t, J = 7.7 Hz), 3.71 (2 H, brs), 4.30 (1 H, septet, J = 6.5 Hz), 5.43 (2 H, s), 5.49 (1 H, s), 7.48 - 7.56 (1 H, m), 7.66 - 7.84 (3 H, m), 8.05 (1 H, d, J = 8.4 Hz), 8.16 (1 H, d, J = 8.4 Hz).

### Reference Example 30

Methyl 3-benzyloxy-1-isopropyl-1H-pyrazole-5-carboxylate (22.16 g) was added under ice-cooling to a suspension of lithium aluminum hydride (3.90 g) in tetrahydrofuran (80 ml) over 30 min, and the mixture was stirred at room temperature for 30 min. Ethanol (30 ml) was slowly added to the reaction mixture, and water (11 ml) was added. The precipitated solid was filtered and washed with acetone. The filtrate was concentrated, and the obtained residue was subjected to silica gel column chromatography and eluted with ethyl acetate-hexane (1:1, v/v) to give (3-benzyloxy-1-isopropyl-1H-pyrazol-5-yl)methanol as colorless crystals (17.71 g, yield 87%). melting point 81 - 82°C.

### Reference Example 31

A mixture of (3-benzyloxy-1-isopropyl-1H-pyrazol-5-yl)methanol (11.0 g), manganese dioxide (45.0 g) and tetrahydrofuran (100 ml) was stirred overnight at room temperature. Manganese dioxide (45.0 g) was added, and the mixture was stirred at 50°C for 7 hr. Manganese dioxide was filtered and thoroughly washed with acetone. The filtrate was concentrated, and the obtained residue was subjected to silica gel column chromatography and eluted with ethyl acetate-hexane (1:2, v/v) to give 3-benzyloxy-1-isopropyl-1H-pyrazole-5-carbaldehyde as a yellow oil (6.30 g).

A solution of the obtained yellow oil and ethyl diethylphosphonoacetate (6.40 g) in tetrahydrofuran (10 ml) was added to a suspension of sodium hydride (1.24 g, 60% in oil) in N,N-dimethylformamide (50 ml) under ice-cooling over 20 min, and the mixture was stirred at room temperature for 30 min. Water (50 ml) was added to the reaction mixture, and the mixture was extracted with ethyl acetate (50 mlx2). The organic layer was washed with brine, dried (MgSO₄), filtered and concentrated. The residue was subjected to silica gel column chromatography and eluted with ethyl acetate-hexane (1:6, v/v) to give ethyl (2E)-3-(3-benzyloxy-1-propyl-1H-pyrazol-5-yl)propenoate as colorless crystals (6.57 g, yield 47%). melting point 58 - 59°C.

### Reference Example 32

A mixture of ethyl (2E)-3-(3-benzyloxy-1-isopropyl-1H-pyrazol-5-yl)propenoate (6.57 g), 10% palladium-carbon (1.3 g) and ethanol (35 ml) was stirred at room temperature for 5 hr under a hydrogen atmosphere. The catalyst was filtered off, and the filtrate was concentrated to give ethyl 3-(3-hydroxy-1-isopropyl-1H-pyrazol-5-yl)propionate as colorless crystals (4.27 g, yield 90%). melting point 119 - 120°C.

### Reference Example 33

Ethyl 3-(3-hydroxy-1-isopropyl-1H-pyrazol-5-yl)propionate (0.62 g) was added to a suspension of sodium hydride (0.13 g, 60% in oil) in N,N-dimethylformamide (6 ml), and the mixture was stirred at room temperature for 10 min. Then, 2-chloroquinoline (0.50 g) was added, and the mixture was stirred overnight at 110°C. Water (20 ml) was added to the reaction mixture, and the mixture was extracted with ethyl acetate (30 mlx2). The organic layer was washed with brine, dried (MgSO₄), filtered and concentrated. The residue was subjected to silica gel column chromatography and eluted with ethyl acetate-hexane (1:2, v/v) to give ethyl 3-[1-isopropyl-3-(quinolin-2-yloxy)-1H-pyrazol-5-yl]propionate as a yellow oil (0.38 g, yield 41%). ¹H-NMR (300 MHz, CDCl₃) δ:1.27 (3 H, t, J = 7.1 Hz), 1.50 (6 H, d, J = 6.6 Hz), 2.68 - 2.77 (2 H, m), 2.95 - 3.05 (2 H, m), 4.18 (2 H, q, J = 7.1 Hz), 4.45 (1 H, septet, J = 6.6 Hz), 6.21 (1 H, s), 7.15 (1 H, d, J = 8.7 Hz), 7.40 - 7.47 (1 H, m), 7.60 - 7.67 (1 H, m), 7.74 - 7.80 (1 H, m), 7.84 - 7.90 (1 H, m), 8.10 (1 H, d, J = 8.4 Hz).

### Reference Example 34

To a solution of ethyl 3-[1-isopropyl-3-(quinolin-2-yloxy)-1H-pyrazol-5-yl]propionate (0.38 g) in tetrahydrofuran (10 ml) was added under ice-cooling a 1.5M solution (1.9 ml) of diisobutylaluminum hydride in toluene over 5 min. After stirring the mixture at room temperature for 30 min, ethanol (10 ml) and saturated aqueous ammonium chloride (0.5 ml) were added to the reaction mixture. The precipitated solid was filtered and washed with acetone. The filtrate was concentrated, ethyl acetate (30 ml) was added, and the mixture was dried (MgSO₄) and filtrated. The filtrate was concentrated to give 3-[1-isopropyl-3-(quinolin-2-yloxy)-1H-pyrazol-5-yl]propan-1-ol as a colorless oil (0.33 g, yield quant.). ¹H-NMR (300 MHz, CDCl₃) δ:1.49 (6 H, d, J = 6.6 Hz), 1.61 (1 H, brs), 1.88 - 2.05 (2 H, m), 2.78 (2 H, t, J = 7.7 Hz), 3.72 - 3.83 (2 H, m), 4.43 (1 H, septet, J = 6.6 Hz), 6.19 (1 H, s), 7.15 (1 H, d, J = 8.8 Hz), 7.37 - 7.48 (1 H, m), 7.57 - 7.92 (3 H, m), 8.09 (1 H, d, J = 8.8 Hz).

### Reference Example 35

A mixture of methyl 3-isopropoxy-1H-pyrazole-5-carboxylate (11.08 g), 2,4-dichlorobenzyl chloride (13.0 g), potassium carbonate (10.00 g) and N,N-dimethylformamide (80 ml) was stirred overnight at room temperature. Water (100 ml) was added to the reaction mixture, and the mixture was extracted with ethyl acetate (100 mlx2). The organic layer was washed with brine, dried (MgSO₄), filtered and concentrated. The residue was subjected to silica gel column chromatography and eluted with ethyl acetate-hexane (1:8 to 1:2, v/v) to give methyl 1-(2,4-dichlorobenzyl)-3-isopropoxy-1H-pyrazole-5-carboxylate (Reference Example 35a) (13.68 g, yield 66%) as a colorless oil. ¹H-NMR (300 MHz, CDCl₃) δ:1.34 (6 H, d, J = 5.8 Hz), 3.81 (3 H, s), 4.72 (1 H, septet, J = 6.2 Hz), 5.67 (2 H, s), 6.28 (1 H, s), 6.54 (1 H, d, J = 8.4 Hz), 7.12 (1 H, dd, J = 8.4, 1.8 Hz), 7.38 (1 H, d, J = 2.2 Hz).

Then, methyl 1-(2,4-dichlorobenzyl)-5-isopropoxy-1H-pyrazole-3-carboxylate (Reference Example 35b) (6.00 g, yield 29%) was obtained as colorless crystals. melting point 89 - 90°C.

### Reference Example 36

A solution of methyl 1-(2,4-dichlorobenzyl)-3-isopropoxy-1H-pyrazole-5-carboxylate (13.68 g) in tetrahydrofuran (40 ml) was added under ice-cooling to a suspension of lithium aluminum hydride (1.90 g) in tetrahydrofuran (70 ml) over 45 min, and the mixture was stirred for 30 min. To the reaction mixture were added ethanol (20 ml) and then saturated aqueous ammonium chloride (7.0 ml). The precipitated inorganic substance was filtered and washed with acetone. Ethyl acetate (100 ml) was added to the residue, and the mixture was dried (MgSO₄), filtrated and concentrated to give [1-(2,4-dichlorobenzyl)-3-isopropoxy-1H-pyrazol-5-yl]methanol as colorless crystals (12.05 g, yield 96%). melting point 89 - 90°C.

### Reference Example 37

A solution of dimethyl sulfoxide (10.8 ml) in dichloromethane (100 ml) was cooled to -70°C, oxalyl chloride (6.70 ml) was added over 20 min, and the mixture was stirred for 20 min. Then, a solution of [1-(2,4-dichlorobenzyl)-3-isopropoxy-1H-pyrazol-5-yl]methanol (12.05 g) in dichloromethane (15 ml) was added over 20 min, and the mixture was stirred for 30 min. Triethylamine (29 ml) was added to the reaction mixture over 10 min, and the temperature was gradually raised to room temperature. To the reaction mixture was added 1N hydrochloric acid (100 ml), and the dichloromethane layer was separated and concentrated to give a residue. Separately, the aqueous layer was extracted with ethyl acetate (100 ml), and the organic layer was combined with the residue obtained earlier. The organic layer was washed with brine, dried (MgSO₄), filtered and concentrated. The residue was subjected to silica gel column chromatography and eluted with ethyl acetate-hexane (1:6, v/v) to give 1-(2,4-dichlorobenzyl)-3-isopropoxy-1H-pyrazole-5-carbaldehyde as a yellow oil (11.37 g, yield 95%). ¹H-NMR (300 MHz, CDCl₃) δ:1.35 (6 H, d, J = 6.2 Hz), 4.76 (1 H, septet, J = 6.1 Hz), 5.64 (2 H, s), 6.31 (1 H, s), 6.60 (1 H, d, J = 8.4 Hz), 7.13 (1 H, dd, J = 8.4, 2.2 Hz), 7.39 (1 H, d, J = 2.2 Hz), 9.74 (1 H, s).

### Reference Example 38

A solution of 1-(2,4-dichlorobenzyl)-3-isopropoxy-1H-pyrazole-5-carbaldehyde (11.37 g) and ethyl diethylphosphonoacetate (8.95 g) in tetrahydrofuran (40 ml) was added to a suspension of sodium hydride (1.75 g, 60% in oil) in N,N-dimethylformamide (140 ml) under ice-cooling over 40 min, and the mixture was stirred at room temperature for 30 min. Water (200 ml) was added to the reaction mixture, and the mixture was extracted with ethyl acetate (100 mlx2). The organic layer was washed with brine, dried (MgSO₄), filtered and concentrated. The residue was subjected to silica gel column chromatography and eluted with ethyl acetate-hexane (1:6, v/v) to give ethyl (2E)-3-[1-(2,4-dichlorobenzyl)-3-isopropoxy-1H-pyrazol-5-yl]propenoate as a yellow oil (12.78 g, yield 92%). ¹H-NMR (300 MHz, CDCl₃) δ:1.30 (3 H, t, J = 6.9 Hz), 1.34 (6 H, d, J = 6.2 Hz), 4.23 (2 H, q, J = 7.2 Hz), 4.72 (1 H, septet, J = 6.4 Hz), 5.33 (2 H, s), 6.00 (1 H, s), 6.27 (1 H, d, J = 15.9 Hz), 6.66 (1 H, d, J = 8.4 Hz), 7.15 (1 H, dd, J = 8.2, 1.8 Hz), 7.35 - 7.45 (2 H, m).

### Reference Example 39

A mixture of ethyl (2E)-3-[1-(2,4-dichlorobenzyl)-3-isopropoxy-1H-pyrazol-5-yl]propenoate (12.78 g), 5% palladium-carbon (1.0 g) and tetrahydrofuran (80 ml) was stirred at room temperature for 6 hr under a hydrogen atmosphere. The catalyst was filtered off, and the filtrate was concentrated. The residue was subjected to silica gel column chromatography and eluted with ethyl acetate-hexane (1:6, v/v) to give ethyl 3-[1-(2,4-dichlorobenzyl)-3-isopropoxy-1H-pyrazol-5-yl]propionate as a pale-yellow oil (11.37 g, yield 89%). ¹H-NMR (300 MHz, CDCl₃) δ:1.24 (3 H, t, J = 7.2 Hz), 1.33 (6 H, d, J = 6.2 Hz), 2.47 - 2.64 (2 H, m), 2.69 - 2.85 (2 H, m), 4.12 (2 H, q, J = 7.2 Hz), 4. 63 - 4.80 (1 H, m), 5.18 (2 H, s), 5.52 (1 H, s), 6.56 (1 H, d, J = 8.5 Hz), 7.15 (1 H, dd, J = 8.4, 2.2 Hz), 7.38 (1 H, d, J = 2.1 Hz).

### Reference Example 40

A mixture of ethyl 3-(3-isopropyl-1H-pyrazol-5-yl)propionate (1.22 g), 2,4-dichlorobenzyl chloride (0.82 ml), potassium carbonate (0.75 g) and N,N-dimethylformamide (10 ml) was stirred overnight at 80°C. Water (15 ml) was added to the reaction mixture, and the mixture was extracted with ethyl acetate (20 mlx2). The organic layer was washed with brine, dried (MgSO₄), filtered and concentrated. The residue was subjected to silica gel column chromatography and eluted with ethyl acetate-hexane (1:7, v/v) to give ethyl 3-[1-(2,4-dichlorobenzyl)-3-isopropoxy-1H-pyrazol-5-yl]propionate as a yellow oil (1.12 g, yield 54%). ¹H-NMR (300 MHz, CDCl₃) δ:1.24 (3 H, t, J = 7.1 Hz), 1.33 (6 H, d, J = 5.8 Hz), 2.50 - 2.63 (2 H, m), 2.70 - 2.82 (2 H, m), 4.12 (2 H, q, J = 7.2 Hz), 4.70 (1 H, septet, J = 6.2 Hz), 5.18 (2 H, s), 5.52 (1 H, s), 6.56 (1 H, d, J = 8.0 Hz), 7.15 (1 H, dd, J = 8.4, 2.2 Hz), 7.38 (1 H, d, J = 2.2 Hz).

### Reference Example 41

To a solution of ethyl 3-[1-(2,4-dichlorobenzyl)-3-isopropoxy-1H-pyrazol-5-yl]propionate (1.12 g) in tetrahydrofuran (15 ml) was added under ice-cooling a 1.5M solution (5.0 ml) of diisobutylaluminum hydride in toluene over 10 min. After stirring the mixture at room temperature for 30 min, ethanol (10 ml) and saturated aqueous ammonium chloride (1.1 ml) were added to the reaction mixture. The precipitated solid was filtered and washed with acetone. The filtrate was concentrated, ethyl acetate (30 ml) was added, and the mixture was dried (MgSO₄) and filtrated. The filtrate was concentrated to give 3-[1-(2,4-dichlorobenzyl)-3-isopropoxy-1H-pyrazol-5-yl]propan-1-ol as colorless crystals (1.00 g, yield quant.). melting point 51 - 52°C.

### Reference Example 42

To a solution of 2*'*,4*'*-dichloroacetophenone (3.0 g) in methanol (15 ml) was slowly added sodium borohydride (0.33 g) under ice-cooling. After stirring the mixture at room temperature for 30 min, water (30 ml) was added, and the mixture was extracted with ethyl acetate (30 mlx2). The organic layer was washed with brine, dried (MgSO₄), filtered and concentrated. The residue was subjected to silica gel column chromatography and eluted with ethyl acetate-hexane (2:1, v/v) to give 1-(2,4-dichlorophenyl)ethanol as colorless oil (3.00 g, yield 99%). ¹H-NMR (300 MHz, CDCl₃) δ:1.47 (3 H, d, J = 6.2 Hz), 1.94 (1 H, d, J = 3.6 Hz), 5.25 (1 H, qd, J = 6.3, 3.9 Hz), 7.23 - 7.37 (2 H, m), 7.55 (1 H, d, J = 8.0 Hz).

### Reference Example 43

To a solution of 1-(2,4-dichlorophenyl)ethanol (3.00 g) in toluene (10 ml) was added thionyl chloride (2.40 ml), and the mixture was stirred at 80°C for 3 hr. After cooling the mixture to room temperature, ethyl acetate (30 ml) was added. The organic layer was washed successively with brine, aqueous sodium hydrogencarbonate and brine, dried (MgSO₄) and filtrated. The filtrate was concentrated to give 2,4-dichloro-1-(1-chloroethyl)benzene as colorless oil (3.03 g, yield 92%). ¹H-NMR (300 MHz, CDCl₃) δ:1.80 (3 H, d, J = 6.9 Hz), 5.50 (1 H, q, J = 6.8 Hz), 7.25 - 7.39 (2 H, m), 7.57 (1 H, d, J = 8.4 Hz).

### Reference Example 44

A mixture of methyl 3-isopropoxy-1H-pyrazole-5-carboxylate (2.50 g), potassium carbonate (2.00 g), 2,4-dichloro-1-(1-chloroethyl)benzene (3.03 g) and N,N-dimethylformamide (15 ml) was stirred at 100°C for 24 hr. Water (30 ml) was added to the reaction mixture, and the mixture was extracted with ethyl acetate (30 mlx2). The organic layer was washed with brine, dried (MgSO₄) filtered and concentrated. The residue was subjected to silica gel column chromatography and eluted with ethyl acetate-hexane (1:6 to 1:3, v/v) to give methyl 1-[1-(2,4-dichlorophenyl)ethyl]-3-isopropoxy-1H-pyrazole-5-carboxylate (Reference Example 44a) (1.92 g, yield 39%) as a colorless oil. ¹H-NMR (300 MHz, CDCl₃) δ:1.38 (6 H, d, J = 6.0 Hz), 1.80 (3 H, d, J = 6.8 Hz), 3.80 (3 H, s), 4.69 (1 H, septet, J = 6.2 Hz), 6.26 (1 H, s), 6.65 (1 H, q, J = 7.0 Hz), 7.01 (1 H, d, J = 8.4 Hz), 7.13 (1 H, dd, J = 8.4, 2.2 Hz), 7.35 (1 H, d, J = 2.2 Hz).

Then, methyl 1-[1-(2,4-dichlorophenyl)ethyl]-5-isopropoxy-1H-pyrazole-3-carboxylate (Reference Example 44b) (1.14 g, yield 23%) was obtained as a colorless oil. ¹H-NMR (300 MHz, CDCl₃) δ:1.11 (3 H, d, J = 6.0 Hz), 1.33 (3 H, d, J = 6.0 Hz), 1.84 (3 H, d, J = 6.9 Hz), 3.92 (3 H, s), 4.33 (1 H, septet, J = 6.2 Hz), 5.92 (1 H, q, J = 7.1 Hz), 6.06 (1 H, s), 7.11 (1 H, d, J = 8.4 Hz), 7.15 (1 H, dd, J = 8.6, 2.0 Hz), 7.36 (1 H, dd, J = 1.8, 0.6 Hz).

### Reference Example 45

To a solution of methyl 1-[1-(2,4-dichlorophenyl)ethyl]-3-isopropoxy-1H-pyrazole-5-carboxylate (1.92 g) in tetrahydrofuran (20 ml) was added under ice-cooling a 1.5M solution (9.3 ml) of diisobutylaluminum hydride in toluene over 15 min, and the mixture was stirred at room temperature for 30 min. To the reaction solution were added ethanol (20 ml) and then saturated aqueous ammonium chloride (2.0 ml). The precipitated inorganic substance was filtered and washed with acetone. The filtrate was concentrated, ethyl acetate (30 ml) was added, and the mixture was dried (MgSO₄) and filtrated. The filtrate was concentrated to give {1-[1-(2,4-dichlorophenyl)ethyl]-3-isopropoxy-1H-pyrazol-5-yl}methanol as colorless crystals (1.48 g, yield 84%). ¹H-NMR (300 MHz, CDCl₃) δ:1.37 (6 H, d, J = 6.3 Hz), 1.53 (1 H, t, J = 5.7 Hz), 1.80 (3 H, d, J = 6.9 Hz), 4.50 (2 H, d, J = 5.4 Hz), 4.67 (1 H, septet, J = 6.1 Hz), 5.66 (1 H, s), 5.86 (1 H, q, J = 6.9 Hz), 7.17 (1 H, dd, J = 8.6, 2.0 Hz), 7.29 (1 H, d, J = 8.4 Hz), 7.35 (1 H, dd, J = 1.8, 0.3 Hz).

### Reference Example 46

A solution of dimethyl sulfoxide (1.30 ml) in dichloromethane (15 ml) was cooled to -70°C, oxalyl chloride (0.79 ml) was added over 15 min, and the mixture was stirred for 20 min. Then, a solution of {1-[1-(2,4-dichlorophenyl)ethyl]-3-isopropoxy-1H-pyrazol-5-yl}methanol (1.48 g) in dichloromethane (2 ml) was added over 10 min, and the mixture was stirred for 20 min. Triethylamine (3.5 ml) was added to the reaction mixture over 10 min, and the temperature was gradually raised to room temperature. The reaction mixture was concentrated, and the obtained residue was passed through a silica gel short column (ethyl acetate-hexane=2:1, v/v). The obtained residue was subjected to silica gel column chromatography and eluted with ethyl acetate-hexane (1:6, v/v) to give 1-[1-(2,4-dichlorophenyl)ethyl]-3-isopropoxy-1H-pyrazole-5-carbaldehyde as a colorless oil (1.41 g, yield 96%). ¹H-NMR (300 MHz, CDCl₃) δ:1.37 (6 H, d, J = 6.3 Hz), 1.38 (1 H, d, J = 6.0 Hz), 1.81 (3 H, d, J = 6.9 Hz), 4.73 (1 H, septet, J = 6.1 Hz), 6.26 (1 H, s), 6.55 (1 H, q, J = 7.0 Hz), 7.06 (1 H, d, J = 8.4 Hz), 7.14 (1 H, dd, J = 8.3, 2.0 Hz), 7.35 (1 H, d, J = 1.8 Hz), 9.68 (1 H, s).

### Reference Example 47

A solution of 1-[1-(2,4-dichlorophenyl)ethyl]-3-isopropoxy-1H-pyrazole-5-carbaldehyde (1.41 g) and ethyl diethylphosphonoacetate (1.10 g) in tetrahydrofuran (2 ml) was added to a suspension of sodium hydride (0.21 g, 60% in oil) in N,N-dimethylformamide (10 ml) under ice-cooling over 10 min, and the mixture was stirred at room temperature for 30 min. Water (20 ml) was added to the reaction mixture, and the mixture was extracted with ethyl acetate (30 mlx2). The organic layer was washed with brine, dried (MgSO₄), filtered and concentrated. The residue was subjected to silica gel column chromatography and eluted with ethyl acetate-hexane (1:6, v/v) to give ethyl 3-{1-[1-(2,4-dichlorophenyl)ethyl]-3-isopropoxy-1H-pyrazol-5-yl}propenoate (E/Z=77/23 mixture) as a colorless oil (1.58 g, yield 96%). ¹H-NMR (300 MHz, CDCl₃) δ:1.24 - 1.45 (9 H, m), 1.34 (6 H, d, J = 6.2 Hz), 4.22 (2 H, q, J = 7.1 Hz), 4.73 (1 H, septet, J = 6.2 Hz), 5.28 (2 H, s), 5.96 (1 H, s), 6.24 (1 H, d, J = 15.8 Hz), 7.10 - 7.37 (5 H, m), 7.46 (1 H, d, J = 15.8 Hz).

### Reference Example 48

A mixture of ethyl 3-{1-[1-(2,4-dichlorophenyl)ethyl]-3-isopropoxy-1H-pyrazol-5-yl}propenoate (E/Z=77/23 mixture, 1.58 g), platinum oxide (0.03 g) and ethanol (15 ml) was stirred at room temperature for 6 hr under a hydrogen atmosphere. The catalyst was filtered off, and the filtrate was concentrated. The residue was subjected to silica gel column chromatography and eluted with ethyl acetate-hexane (1:8, v/v) to give ethyl 3-{1-[1-(2,4-dichlorophenyl)ethyl]-3-isopropoxy-1H-pyrazol-5-yl}propionate as a colorless oil (1.31 g, yield 80%). ¹H-NMR (300 MHz, CDCl₃) δ:1.23 (3 H, t, J = 7.1 Hz), 1.35 (6 H, d, J = 6.2 Hz), 1.79 (3 H, d, J = 6.6 Hz), 2.40 - 2.96 (4 H, m), 4.11 (2 H, q, J = 7.2 Hz), 4.65 (1 H, septet, J = 6.2 Hz), 5.47 (1 H, s), 5.68 (1 H, q, J = 6.9 Hz), 7.10 - 7.23 (2 H, m), 7.35 (1 H, dd, J = 2.0, 0.8 Hz).

### Reference Example 49

To a solution of ethyl 3-{1-[1-(2,4-dichlorophenyl)ethyl]-3-isopropoxy-1H-pyrazol-5-yl}propionate (1.31 g) in tetrahydrofuran (20 ml) was added under ice-cooling a 1.5M solution (6.0 ml) in toluene over 10 min. After stirring the mixture at room temperature for 30 min, to the reaction solution were added ethanol (25 ml) and then saturated aqueous ammonium chloride (2.0 ml). The precipitated inorganic substance was filtered and washed with acetone. The filtrate was concentrated, ethyl acetate (30 ml) was added, and the mixture was dried (MgSO₄) and filtrated. The filtrate was concentrated to give 3-{1-[1-(2,4-dichlorophenyl)ethyl]-3-isopropoxy-1H-pyrazol-5-yl}propan-1-ol as a yellow oil (1.13 g, yield quant.). ¹H-NMR (300 MHz, CDCl₃) δ:1.36 (6 H, d, J = 6.2 Hz), 1.26 (1 H, brs), 1.64 - 1.84 (5 H, m), 2.34 - 2.72 (2 H, m), 3.55 - 3.68 (2 H, m), 4.66 (1 H, septet, J = 6.2 Hz), 5.49 (1 H, s), 5.66 (1 H, q, J = 6.9 Hz), 7.14 (1 H, dd, J = 8.7, 2.1 Hz), 7.22 (1 H, d, J = 8.4 Hz), 7.34 (1 H, d, J = 1.8 Hz).

### Reference Example 50

A mixture of ethyl 3-(3-isopropoxy-1H-pyrazol-5-yl)propionate (1.97 g), potassium carbonate (3.00 g), 2-(chloromethyl)pyridine hydrochloride (1.71 g) and N,N-dimethylformamide (20 ml) was stirred overnight at 80°C. Potassium carbonate (0.84 g) and 2-(chloromethyl)pyridine hydrochloride (0.50 g) were added, and the mixture was stirred at 100°C for 5 hr. Water (30 ml) was added to the reaction mixture, and the mixture was extracted with ethyl acetate (30 mlx2). The organic layer was washed with brine, dried (MgSO₄), filtered and concentrated. The residue was subjected to silica gel column chromatography and eluted with ethyl acetate-hexane (2:1, v/v) to give ethyl 3-[3-isopropoxy-1-(pyridin-2-ylmethyl)-1H-pyrazol-5-yl]propionate as a yellow oil (1.02 g, yield 37%). ¹H-NMR (300 MHz, CDCl₃) δ:1.20 - 1.40 (9 H, m), 2.50 - 2.67 (2 H, m), 2.77 - 2.91 (2 H, m), 4.12 (2 H, q, J = 7.7 Hz), 4.70 (1 H, septet, J = 6.1 Hz), 5.27 (2 H, s), 5.51 (1 H, s), 6.84 (1 H, d, J = 7.6 Hz), 7.12 - 7.23 (1 H, m), 7.61 (1 H, td, J = 7.7, 1.8 Hz), 8.50 - 8.58 (1 H, m).

### Reference Example 51

To a solution of ethyl 3-[3-isopropoxy-1-(pyridin-2-ylmethyl)-1H-pyrazol-5-yl]propionate (1.02 g) in tetrahydrofuran (15 ml) was added under ice-cooling a 1.5M solution (6.0 ml) of diisobutylaluminum hydride in toluene over 10 min. After stirring the mixture at room temperature for 30 min, to the reaction solution were added ethanol (20 ml) and then saturated aqueous ammonium chloride (1.5 ml). The precipitated inorganic substance was filtered and washed with acetone. The filtrate was concentrated, ethyl acetate (30 ml) was added, and the mixture was dried (MgSO₄) and filtrated. The filtrate was concentrated, and the residue was subjected to silica gel column chromatography and eluted with ethyl acetate-methanol (1:0 to 95:5, v/v) to give 3-[3-isopropoxy-1-(pyridin-2-ylmethyl)-1H-pyrazol-5-yl]propan-1-ol as a yellow oil (0.45 g, yield 50%). ¹H-NMR (300 MHz, CDCl₃) δ:1.31 (6 H, d, J = 6.2 Hz), 1.76 - 1.92 (5 H, m), 2.74 (2 H, t, J = 7.2 Hz), 3.59 (2 H, t, J = 5.9 Hz), 4.64 (1 H, septet, J = 6.2 Hz), 5.21 (2 H, s), 5.50 (1 H, s), 7.10 - 7.25 (2 H, m), 7.66 (1 H, td, J = 7.7, 1.8 Hz), 8.46 - 8.53 (1 H, m).

### Reference Example 52

A mixture of ethyl 3-(3-propoxy-1H-pyrazol-5-yl)propionate (1.50 g), potassium carbonate (1.30 g), 2,4-dichloro-1-(1-chloroethyl)benzene (3.50 g) and N,N-dimethylformamide (20 ml) was stirred at 100°C for 2 days. Water (30 ml) was added to the reaction mixture, and the mixture was extracted with ethyl acetate (30 mlx2). The organic layer was washed with brine, dried (MgSO₄), filtered and concentrated. The residue was subjected to silica gel column chromatography and eluted with ethyl acetate-hexane (1:6, v/v) to give ethyl 3-{1-[1-(2,4-dichlorophenyl)ethyl]-3-propoxy-1H-pyrazol-5-yl}propionate as a yellow oil (0.50 g, yield 20%). ¹H-NMR (300 MHz, CDCl₃) δ:1.03 (3 H, t, J = 7.4 Hz), 1.23 (3 H, t, J = 7.1 Hz), 1.70 - 1.90 (5 H, m), 2.43 - 2.96 (4 H, m), 4.04 (2 H, t, J = 6.7 Hz), 4.11 (2 H, q, J = 7.2 Hz), 5.49 (1 H, s), 5.68 (1 H, q, J = 6.9 Hz), 7.11 - 7.23 (2 H, m), 7.33 - 7.38 (1 H, m).

### Reference Example 53

To a solution of ethyl 3-{1-[1-(2,4-dichlorophenyl)ethyl]-3-propoxy-1H-pyrazol-5-yl}propionate (0.50 g) in tetrahydrofuran (10 ml) was added under ice-cooling a 1.5M solution (2.2 ml) of diisobutylaluminum hydride in toluene over 10 min. After stirring the mixture at room temperature for 30 min, to the reaction solution were added ethanol (10 ml) and then saturated aqueous ammonium chloride (0.6 ml). The precipitated inorganic substance was filtered and washed with acetone. The filtrate was concentrated, ethyl acetate (30 ml) was added, and the mixture was dried (MgSO₄) and filtrated. The filtrate was concentrated to give 3-{1-[1-(2,4-dichlorophenyl)ethyl]-3-propoxy-1H-pyrazol-5-yl}propan-1-ol as a yellow oil (0.44 g, yield quant.). ¹H-NMR (300 MHz, CDCl₃) δ:1.03 (3 H, t, J = 7.5 Hz), 1.46 (1 H, brs), 1.64 - 1.90 (7 H, m), 2.33 - 2.72 (2 H, m), 3.54 - 3.72 (2 H, m), 4.05 (2 H, t, J = 6.6 Hz), 5.52 (1 H, s), 5.66 (1 H, q, J = 6.9 Hz), 7.10 - 7.40 (3 H, m).

### Reference Example 54

A mixture of methyl 3-isopropoxy-1H-pyrazole-5-carboxylate (10.62 g), benzyl bromide (7.54 ml), potassium carbonate (8.0 g) and N,N-dimethylformamide (50 ml) was stirred overnight at room temperature. Water (100 ml) was added to the reaction mixture, and the mixture was extracted with ethyl acetate (80 mlx2). The organic layer was washed with brine, dried (MgSO₄), filtered and concentrated. The residue was subjected to silica gel column chromatography and eluted with ethyl acetate-hexane (1:6 to 1:2, v/v) to give methyl 1-benzyl-3-isopropoxy-1H-pyrazole-5-carboxylate (Reference Example 54a) as a colorless oil (10.94 g, yield 69%). ¹H-NMR (300 MHz, CDCl₃) δ:1.34 (6 H, d, J = 6.2 Hz), 3.82 (3 H, s), 4.71 (1 H, septet, J = 6.2 Hz), 5.60 (2 H, s), 6.21 (1 H, s), 7.20 - 7.40 (5 H, m).

Then, methyl 1-benzyl-5-isopropoxy-1H-pyrazole-3-carboxylate (Reference Example 54b) was obtained as a colorless oil (3.52 g, yield 22%). ¹H-NMR (300 MHz, CDCl₃) δ:1.30 (6 H, d, J = 6.0 Hz), 3.90 (3 H, s), 4.40 (1 H, septet, J = 6.2 Hz), 5.21 (2 H, s), 6.07 (1 H, s), 7.18 - 7.40 (5 H, m).

### Reference Example 55

A solution of methyl 1-benzyl-3-isopropoxy-1H-pyrazole-5-carboxylate (8.56 g) in tetrahydrofuran (5 ml) was added under ice-cooling to a suspension of lithium aluminum hydride (1.50 g) in tetrahydrofuran (60 ml) over 20 min, and the mixture was stirred for 20 min. To the reaction mixture were added ethanol (20 ml) and then saturated aqueous ammonium chloride (4.5 ml). The precipitated inorganic substance was filtered and washed with acetone. Ethyl acetate (50 ml) was added to the residue, and the mixture was dried (MgSO₄), filtrated and concentrated to give (1-benzyl-3-isopropoxy-1H-pyrazol-5-yl)methanol as a colorless oil (7.91 g, yield quant.). ¹H-NMR (300 MHz, CDCl₃) δ:1.34 (6 H, d, J = 5.8 Hz), 1.67 (1 H, brs), 4.48 (2 H, brs), 4.71 (1 H, septet, J = 6.2 Hz), 5.23 (2 H, s), 5.65 (1 H, s), 7.10 - 7.38 (5 H, m).

### Reference Example 56

A solution of dimethyl sulfoxide (9.10 ml) in dichloromethane (100 ml) was cooled to -70°C, oxalyl chloride (5.60 ml) was added over 15 min, and the mixture was stirred for 20 min. Then, a solution of (1-benzyl-3-isopropoxy-1H-pyrazol-5-yl)methanol (7.91 g) in dichloromethane (20 ml) was added over 20 min, and the mixture was stirred for 20 min. Triethylamine (25 ml) was added to the reaction mixture over 10 min, and the temperature was gradually raised to room temperature. To the reaction mixture was added 1N hydrochloric acid (50 ml), the dichloromethane layer was separated, and concentrated to give a residue. Separately, the aqueous layer was extracted with ethyl acetate (100 ml), and the organic layer was combined with the residue obtained earlier. The organic layer was washed with brine, dried (MgSO₄), filtered and concentrated. The residue was subjected to silica gel column chromatography and eluted with ethyl acetate-hexane (1:6, v/v) to give 1-benzyl-3-isopropoxy-1H-pyrazole-5-carbaldehyde as a yellow oil (7.22 g, yield 95%). ¹H-NMR (300 MHz, CDCl₃) δ:1.36 (6 H, d, J = 5.8 Hz), 4.77 (1 H, septet, J = 6.2 Hz), 5.56 (2 H, s), 6.24 (1 H, s), 7.20 - 7.35 (5 H, m), 9.71 (1 H, s).

### Reference Example 57

A solution of 1-benzyl-3-isopropoxy-1H-pyrazole-5-carbaldehyde (7.22 g) and ethyl diethylphosphonoacetate (7.30 g) in tetrahydrofuran (10 ml) was added to a suspension of sodium hydride (1.42 g, 60% in oil) in N,N-dimethylformamide (80 ml) under ice-cooling over 20 min, and the mixture was stirred at room temperature for 30 min. Water (100 ml) was added to the reaction mixture, and the mixture was extracted with ethyl acetate (50 mlx2). The organic layer was washed with brine, dried (MgSO₄), filtered and concentrated. The residue was subjected to silica gel column chromatography and eluted with ethyl acetate-hexane (1:4, v/v) to give ethyl (2E)-3-(1-benzyl-3-isopropoxy-1H-pyrazol-5-yl)propenoate as a yellow oil (7.57 g, yield 81%). ¹H-NMR (300 MHz, CDCl₃) δ:1.30 (3 H, t, J = 7.4 Hz), 1.34 (6 H, d, J = 6.2 Hz), 4.22 (2 H, q, J = 7.1 Hz), 4.73 (1 H, septet, J = 6.2 Hz), 5.28 (2 H, s), 5.96 (1 H, s), 6.24 (1 H, d, J = 15.8 Hz), 7.10 - 7.37 (5 H, m), 7.46 (1 H, d, J = 15.8 Hz).

### Reference Example 58

A mixture of ethyl (2E)-3-(1-benzyl-3-isopropoxy-1H-pyrazol-5-yl)propenoate (7.57 g), 10% palladium-carbon (1.50 g) and ethanol (45 ml) was stirred overnight at 50°C under a hydrogen atmosphere. The catalyst was filtered off, and the filtrate was concentrated to give ethyl 3-(3-isopropoxy-1H-pyrazol-5-yl)propionate as a pale-yellow oil (5.41 g, yield 99%). ¹H-NMR (300 MHz, CDCl₃) δ:1.26 (3 H, t, J = 7.2 Hz), 1.34 (6 H, d, J = 6.2 Hz), 2.58 - 2.70 (2 H, m), 2.83 - 2.95 (2 H, m), 4.16 (2 H, q, J = 7.2 Hz), 4.70 (1 H, septet, J = 6.2 Hz), 5.48 (1 H, s), 8.33 (1 H, brs).

### Reference Example 59

A mixture of ethyl (2E)-3-(1-benzyl-3-isopropoxy-1H-pyrazol-5-yl)propenoate (5.94 g), 5% palladium-carbon (1.0 g) and tetrahydrofuran (50 ml) was stirred at room temperature for 2 hr under a hydrogen atmosphere. The catalyst was filtered off, and the filtrate was concentrated. The residue was subjected to silica gel column chromatography and eluted with ethyl acetate-hexane (1:4 to 4:1, v/v) to give ethyl 3-(1-benzyl-3-isopropoxy-1H-pyrazol-5-yl)propionate as a colorless oil (3.80 g, yield 63%). ¹H-NMR (300 MHz, CDCl₃) δ:1.23 (3 H, t, J = 7.1 Hz), 1.33 (6 H, d, J = 5.8 Hz), 2.45 - 2.58 (2 H, m), 2.70 - 2.83 (2 H, m), 4.11 (2 H, q, J = 7.2 Hz), 4.71 (1 H, septet, J = 6.2 Hz), 5.15 (2 H, s), 5.48 (1 H, s), 7.03 - 7.12 (2 H, m), 7.18 - 7.36 (3 H, m).

### Reference Example 60

To a solution of ethyl 3-(1-benzyl-3-isopropoxy-1H-pyrazol-5-yl)propionate (0.90 g) in tetrahydrofuran (15 ml) was added under ice-cooling a 1.5M solution (5.0 ml) of diisobutylaluminum hydride in toluene over 10 min. After stirring the mixture at room temperature for 30 min, to the reaction solution were added ethanol (10 ml) and then saturated aqueous ammonium chloride (1.4 ml). The precipitated inorganic substance was filtered and washed with acetone. The filtrate was concentrated, ethyl acetate (30 ml) was added, and the mixture was dried (MgSO₄) and filtrated. The filtrate was concentrated to give 3-(1-benzyl-3-isopropoxy-1H-pyrazol-5-yl)propan-1-ol as a yellow oil (0.77 g, yield quant.). ¹H-NMR (300 MHz, CDCl₃) δ:1.34 (6 H, d, J = 6.2 Hz), 1.70 - 1.83 (2 H, m), 2.55 (2 H, t, J = 7.5 Hz), 3.55 - 3.66 (2 H, brm), 4.71 (1 H, septet, J = 6.2 Hz), 5.14 (2 H, s), 5.50 (1 H, s), 7.02 - 7.13 (2 H, m), 7.18 -7.35 (3 H, m).

### Reference Example 61

To a solution of ethyl 3-(3-isopropoxy-1H-pyrazol-5-yl)propionate (1.50 g) in N,N-dimethylformamide (20 ml) was added sodium hydride (0.30 g, 60% in oil), and the mixture was stirred at room temperature for 20 min. 2-Methylbenzyl bromide (1.35 g) was added. After stirring the mixture for 30 min, ice water (30 ml) was added, and the mixture was extracted with ethyl acetate (20 mlx2). The organic layer was washed with brine, dried (MgSO₄), filtered and concentrated. The residue was subjected to silica gel column chromatography and eluted with ethyl acetate-hexane (1:6, v/v) to give ethyl 3-[3-isopropoxy-1-(2-methylbenzyl)-1H-pyrazol-5-yl]propionate as a colorless oil (0.54 g, yield 25%). ¹H-NMR (300 MHz, CDCl₃) δ:1.23 (3 H, t, J = 7.2 Hz), 1.33 (6 H, d, J = 5.8 Hz), 2.33 (3 H, s), 2.46 - 2.60 (2 H, m), 2.67 - 2.80 (2 H, m), 4.11 (2 H, q, J = 7.1 Hz), 4.72 (1 H, septet, J = 6.2 Hz), 5.13 (2 H, s), 5.51 (1 H, s), 6.53 (1 H, d, J = 6.6 Hz), 7.03 - 7.23 (3 H, m).

### Reference Example 62

To a solution of ethyl 3-[3-isopropoxy-1-(2-methylbenzyl)-1H-pyrazol-5-yl]propionate (1.16 g) in tetrahydrofuran (15 ml) was added under ice-cooling a 1.5M solution (5.85 ml) of diisobutylaluminum hydride in toluene over 10 min, and the mixture was stirred at room temperature for 30 min. To the reaction solution were added ethanol (20 ml) and then saturated aqueous ammonium chloride (1.3 ml). The precipitated inorganic substance was filtered and washed with acetone. The filtrate was concentrated, ethyl acetate (30 ml) was added, and the mixture was dried (MgSO₄) and filtrated. The filtrate was concentrated, and the obtained residue was subjected to silica gel column chromatography and eluted with ethyl acetate-hexane (1:1, v/v) to give 3-[3-isopropoxy-1-(2-methylbenzyl)-1H-pyrazol-5-yl]propan-1-ol as a colorless oil (0.88 g, yield 87%). ¹H-NMR (300 MHz, CDCl₃) δ:1.33 (6 H, d, J = 5.8 Hz), 1.70 - 1.86 (2 H, m), 2.32 (3 H, s), 2.52 (2 H, t, J = 7.7 Hz), 3.61 (2 H, q, J = 5.6 Hz), 4.72 (1 H, septet, J = 6.1 Hz), 5.11 (2 H, s), 5.53 (1 H, s), 6.53 (1 H, d, J = 6.6 Hz), 7.02 - 7.18 (3 H, m).

### Reference Example 63

A mixture of 2,4-dichlorobenzyl chloride (27.0 g), methyl carbazate (25.1 g), sodium hydrogencarbonate (11.6 g) and ethanol (200 ml) was stirred overnight heated under reflux. Water (400 ml) was added to the reaction mixture, and the mixture was extracted with diethyl ether (100 ml×3). The organic layer was washed with brine, dried (MgSO₄), filtered and concentrated. The residue was subjected to silica gel column chromatography and eluted with ethyl acetate-hexane (1:2, v/v) to give methyl N'-(2,4-dichlorobenzyl)carbazate as colorless crystals (23.19 g, yield 67%). melting point 82 - 83°C.

### Reference Example 64

A solution of methyl N'-(2,4-dichlorobenzyl)carbazate (23.19 g) and dimethyl acetylenedicarboxylate in methanol (100 ml) was stirred with heating under reflux for 2 hr. 28% Sodium methoxide methanol solution (18.9 g) was slowly added to the reaction mixture under ice-cooling, and the mixture was stirred with heating under reflux for 1 hr. To the reaction mixture were added ice water (100 ml) and concentrated hydrochloric acid (10 ml), and the precipitated crystals were collected by filtration, washed with water and dried to give methyl 1-(2,4-dichlorobenzyl)-3-hydroxy-1H-pyrazole-5-carboxylate as pale-yellow crystals (27.88 g, yield 90%). melting point 222 - 223°C.

### Reference Example 65

To a mixture of methyl 1-(2,4-dichlorobenzyl)-3-hydroxy-1H-pyrazole-5-carboxylate (2.57 g), potassium carbonate (1.77 g) and N,N-dimethylformamide (35 ml) was added a solution of chloromethyl methyl ether (1.00 ml) in tetrahydrofuran (10 ml) over 30 min under ice-cooling, and the mixture was stirred overnight at room temperature. Water (60 ml) was added to the reaction mixture, and the mixture was extracted with ethyl acetate (30 mlx2). The organic layer was washed with brine, dried (MgSO₄), filtered and concentrated. The residue was subjected to silica gel column chromatography and eluted with ethyl acetate-hexane (1:4, v/v) to give methyl 1-(2,4-dichlorobenzyl)-3-(methoxymethoxy)-1H-pyrazole-5-carboxylate as a colorless oil (2.66 g, yield 90%). ¹H-NMR (300 MHz, CDCl₃) δ:3.52 (3 H, s), 3.83 (3 H, s), 5.23 (2 H, s), 5.70 (2 H, s), 6.44 (1 H, s), 6.57 (1 H, d, J = 8.0 Hz), 7.12 (1 H, dd, J = 8.0, 2.0 Hz), 7.39 (1 H, d, J = 1.8 Hz).

### Reference Example 66

To a solution of methyl 1-(2,4-dichlorobenzyl)-3-(methoxymethoxy)-1H-pyrazole-5-carboxylate (6.36 g) in tetrahydrofuran (80 ml) was added under ice-cooling a 1.5M solution (31.0 ml) of diisobutylaluminum hydride in toluene over 30 min. The mixture was stirred at room temperature for 30 min. Since the starting material remained, a 1.5M solution (2.5 ml) of diisobutylaluminum hydride in toluene was added. After stirring the mixture at room temperature for 30 min, to the reaction solution were added ethanol (15 ml) and then saturated aqueous ammonium chloride (6.7 ml). The precipitated inorganic substance was filtered and washed with acetone. The filtrate was concentrated, ethyl acetate (30 ml) was added, and the mixture was dried (MgSO₄) and filtrated. The filtrate was concentrated, and the residue was subjected to silica gel column chromatography and eluted with ethyl acetate-hexane (2:3, v/v) to give [1-(2,4-dichlorobenzyl)-3-(methoxymethoxy)-1H-pyrazol-5-yl]methanol as colorless crystals (5.35 g, yield 92%). melting point 82 - 83°C.

### Reference Example 67

A solution of dimethyl sulfoxide (4.80 ml) in dichloromethane (80 ml) was cooled to -70°C, oxalyl chloride (2.90 ml) was added over 20 min, and the mixture was stirred for 20 min. Then, a solution of [1-(2,4-dichlorobenzyl)-3-(methoxymethoxy)-1H-pyrazol-5-yl]methanol (5.35 g) in dichloromethane (15 ml) was added over 30 min, and the mixture was stirred for 20 min. Triethylamine (13.0 ml) was added to the reaction mixture over 10 min, and the temperature was gradually raised to room temperature. The reaction mixture was concentrated, and the precipitated salt was filtered and washed with diisopropyl ether. The filtrate was concentrated, and the obtained residue was subjected to silica gel column chromatography and eluted with ethyl acetate-hexane (1:3, v/v) to give 1-(2,4-dichlorobenzyl)-3-(methoxymethoxy)-1H-pyrazole-5-carbaldehyde as a yellow oil (5.22 g, yield 98%). ¹H-NMR (300 MHz, CDCl₃) δ:3.52 (3 H, s), 5.24 (2 H, s), 5.67 (2 H, s), 6.48 (1 H, s), 6.65 (1 H, d, J = 8.4 Hz), 7.13 (1 H, dd, J = 8.6, 2.2 Hz), 7.39 (1H, d, J=2.2Hz), 9.75 (1H, s).

### Reference Example 68

A solution of 1-(2,4-dichlorobenzyl)-3-(methoxymethoxy)-1H-pyrazole-5-carbaldehyde (5.22 g) and ethyl diethylphosphonoacetate (4.10 g) in tetrahydrofuran (15 ml) was added to a suspension of sodium hydride (0.80 g, 60% in oil) in N,N-dimethylformamide (35 ml) under ice-cooling over 20 min, and the mixture was stirred at room temperature for 30 min. Water (50 ml) was added to the reaction mixture, and the mixture was extracted with ethyl acetate (40 mlx2). The organic layer was washed with brine, dried (MgSO₄), filtered and concentrated. The residue was subjected to silica gel column chromatography and eluted with ethyl acetate-hexane (1:4, v/v) to give ethyl (2E)-3-[1-(2,4-dichlorobenzyl)-3-(methoxymethoxy)-1H-pyrazol-5-yl]propenoate as colorless crystals (5.93 g, yield 93%). melting point 98 - 99°C.

### Reference Example 69

A mixture of ethyl (2E)-3-[1-(2,4-dichlorobenzyl)-3-(methoxymethoxy)-1H-pyrazol-5-yl]propenoate (5.93 g), 5% palladium-carbon (0.60 g) and tetrahydrofuran (50 ml) was stirred overnight at room temperature under a hydrogen atmosphere. The catalyst was filtered off, and the filtrate was concentrated. The obtained residue was subjected to silica gel column chromatography and eluted with ethyl acetate-hexane (1:3, v/v) to give ethyl 3-[1-(2,4-dichlorobenzyl)-3-(methoxymethoxy)-1H-pyrazol-5-yl]propionate as colorless crystals (5.72 g, yield 96%). melting point 47 - 49°C.

### Reference Example 70

To a solution of ethyl 3-[1-(2,4-dichlorobenzyl)-3-(methoxymethoxy)-1H-pyrazol-5-yl]propionate (5.72 g) in tetrahydrofuran (80 ml) was added under ice-cooling a 1.5M solution (25.0 ml) of diisobutylaluminum hydride in toluene over 30 min. After stirring the mixture at room temperature for 30 min, to the reaction solution were added ethanol (20 ml) and then saturated aqueous ammonium chloride (5.0 ml). The precipitated inorganic substance was filtered and washed with acetone. The filtrate was concentrated, and the obtained residue was subjected to silica gel column chromatography and eluted with ethyl acetate-hexane (3:2, v/v) to give 3-[1-(2,4-dichlorobenzyl)-3-(methoxymethoxy)-1H-pyrazol-5-yl]propan-1-ol as a colorless oil (4.98 g, yield 97%). ¹H-NMR (300 MHz, CDCl₃) δ:1.40 (6 H, t, J = 4.9 Hz), 1.74 - 1.90 (2 H, m), 2.57 (2 H, t, J = 7.7 Hz), 3.52 (3 H, s), 3.66 (2 H, q, J = 5.7 Hz), 5.20 (2 H, s), 5.21 (2 H, s), 5.68 (1 H, s), 6.60 (1 H, d, J = 8.4 Hz), 7.15 (1 H, dd, J = 8.4, 2.2 Hz), 7.38 (1 H, d, J = 2.2 Hz).

### Reference Example 71

To a solution of methyl (2-{3-[1-(2,4-dichlorobenzyl)-3-(methoxymethoxy)-1H-pyrazol-5-yl]propoxy}-3-methoxyphenyl)acetate (4.34 g) in methanol (30 ml) was added 4 drops of concentrated hydrochloric acid, and the mixture was stirred overnight with heating under reflux. The reaction mixture was concentrated, and the obtained crystals were collected by filtration while washing with diisopropyl ether to give methyl (2-{3-[1-(2,4-dichlorobenzyl)-3-hydroxy-1H-pyrazol-5-yl]propoxy}-3-methoxyphenyl)acetate as pale-yellow crystals (3.80 g, yield 96%). melting point 110 - 111°C.

### Reference Example 72

To a mixture of potassium tert-butoxide (55.1 g) in tetrahydrofuran (300 ml) was added a mixture of 3-methylbutan-2-one (30.0 g) and diethyl oxalate (51.0 g) at room temperature over 1 hr, and the mixture was stirred overnight at room temperature. Acetic acid (47.7 ml) and hydrazine monohydrate (19.0 g) were added, and the mixture was stirred with heating under reflux for 2 hr. The reaction mixture was concentrated, water (200 ml) was added, and the mixture was extracted with ethyl acetate (150 mlx2). The organic layer was washed with brine, dried (MgSO₄) filtered and concentrated. The residue was subjected to silica gel column chromatography and eluted with ethyl acetate-hexane (1:1, v/v) to give ethyl 3-isopropyl-1H-pyrazole-5-carboxylate as a brown oil (31.82 g, yield 50%). ¹H-NMR (300 MHz, CDCl₃) δ:1.30 (6 H, d, J = 7.0 Hz), 1.38 (3 H, t, J = 7.2 Hz), 3.04 (1 H, septet, J = 6.8 Hz), 4.37 (2 H, q, J = 6.8 Hz), 4.37 (2 H, q, J = 7.1 Hz), 6.63 (1 H, d, J = 0.6 Hz).

### Reference Example 73

Ethyl 3-isopropyl-1H-pyrazole-5-carboxylate (10.00 g), 2,4-dichlorobenzyl chloride (11.8 g), potassium carbonate (9.0 g) and N,N-dimethylformamide (50 ml) were stirred overnight at room temperature. Water (80 ml) was added to the reaction mixture, and the mixture was extracted with ethyl acetate (50 mlx2). The organic layer was washed with brine, dried (MgSO₄) filtered and concentrated. The residue was subjected to silica gel column chromatography and eluted with ethyl acetate-hexane (1:15 to 1:4, v/v) to give ethyl 1-(2,4-dichlorobenzyl)-3-isopropyl-1H-pyrazole-5-carboxylate (Reference Example 73a) as a brown oil (10.52 g, yield 56%). ¹H-NMR (300 MHz, CDCl₃) δ:1.29 (6 H, d, J = 6.8 Hz), 1.29 (3 H, t, J = 7.1 Hz), 3.02 (1 H, septet, J = 6.9 Hz), 4.26 (2 H, q, J = 7.1 Hz), 5.77 (2 H, s), 6.34 (1 H, d, J = 8.6 Hz), 6.78 (1 H, s), 7.09 (1 H, dd, J = 8.4, 2.2 Hz), 7.38 (1 H, dd, J = 2.2 Hz).

Then, ethyl 1-(2,4-dichlorobenzyl)-5-isopropyl-1H-pyrazole-3-carboxylate (Reference Example 73b) (8.73 g, yield 47%) was obtained. ¹H-NMR (300 MHz, CDCl₃) δ:1.18 (6 H, d, J = 7.0 Hz), 1.40 (3 H, t, J = 7.1 Hz), 2.79 (1 H, septet, J = 6.8 Hz), 4.42 (2 H, q, J = 7.2 Hz), 5.46 (2 H, s), 6.49 (1 H, d, J = 8.0 Hz), 6.70 (1 H, s), 7.13 (1 H, dd, J = 8.4, 2.2 Hz), 7.40 (1 H, dd, J = 1.8 Hz).

### Reference Example 74

To a solution of ethyl 1-(2,4-dichlorobenzyl)-3-isopropyl-1H-pyrazole-5-carboxylate (10.52 g) in tetrahydrofuran (100 ml) was added under ice-cooling a 1.5M solution (51.5 ml) of diisobutylaluminum hydride in toluene over 30 min. After stirring the mixture at room temperature for 30 min, to the reaction solution were added ethanol (30 ml) and then saturated aqueous ammonium chloride (13 ml). The precipitated inorganic substance was filtered and washed with acetone. The filtrate was concentrated, and the obtained residue was subjected to silica gel column chromatography and eluted with ethyl acetate-hexane (1:2, v/v) to give [1-(2,4-dichlorobenzyl)-3-isopropyl-1H-pyrazol-5-yl]methanol as colorless crystals (7.90 g, yield 86%). melting point 89 - 90°C.

### Reference Example 75

A solution of dimethyl sulfoxide (7.50 ml) in dichloromethane (100 ml) was cooled to -70°C, oxalyl chloride (4.60 ml) was added over 15 min, and the mixture was stirred for 15 min. Then, a solution of [1-(2,4-dichlorobenzyl)-3-isopropyl-1H-pyrazol-5-yl]methanol (7.90 g) in dichloromethane (25 ml) was added over 20 min, and the mixture was stirred for 20 min. Triethylamine (20.0 ml) was added to the reaction mixture over 10 min, and the temperature was gradually raised to room temperature. The reaction mixture was concentrated, and the precipitated salt was filtered and washed with diisopropyl ether. The filtrate was concentrated, and the obtained residue was subjected to silica gel column chromatography and eluted with ethyl acetate-hexane (1:15 to 1:6, v/v) to give 1-(2,4-dichlorobenzyl)-3-isopropyl-1H-pyrazole-5-carbaldehyde as a yellow oil (7.08 g, yield 90%). ¹H-NMR (300 MHz, CDCl₃) δ:1.30 (6 H, d, J = 7.0 Hz), 3.05 (1 H, septet, J = 6.9 Hz), 5.75 (2 H, s), 6.42 (1 H, d, J = 8.6 Hz), 6.81 (1 H, s), 7.10 (1 H, dd, J = 8.2, 2.0 Hz), 7.39 (1 H, d, J = 2.2 Hz), 9.79 (1 H, s).

### Reference Example 76

A solution of 1-(2,4-dichlorobenzyl)-3-isopropyl-1H-pyrazole-5-carbaldehyde (7.08 g) and ethyl diethylphosphonoacetate (5.87 g) in tetrahydrofuran (15 ml) was added to a suspension of sodium hydride (1.15 g, 60% in oil) in N,N-dimethylformamide (40 ml) under ice-cooling over 20 min, and the mixture was stirred at room temperature for 1 hr. Water (80 ml) was added to the reaction mixture, and the mixture was extracted with ethyl acetate (40 mlx2). The organic layer was washed with brine, dried (MgSO₄), filtered and concentrated. The residue was subjected to silica gel column chromatography and eluted with ethyl acetate-hexane (1:4, v/v) to give ethyl (2E)-3-[1-(2,4-dichlorobenzyl)-3-isopropyl-1H-pyrazol-5-yl]propenoate as a yellow oil (7.07 g, yield 81%). ¹H-NMR (300 MHz, CDCl₃) δ:1.22 - 1.37 (9 H, m), 2.99 (1 H, septet, J = 6.9 Hz), 4.22 (2 H, q, J = 7.2 Hz), 5.44 (2 H, s), 6.30 (1 H, d, J = 15.8 Hz), 6.44 - 6.53 (2 H, m), 7.13 (1 H, dd, J = 8.2, 2.0 Hz), 7.40 (1 H, d, J = 2.2 Hz), 7.41 (1 H, d, J = 15.8 Hz).

### Reference Example 77

A mixture of ethyl (2E)-3-[1-(2,4-dichlorobenzyl)-3-isopropyl-1H-pyrazol-5-yl]propenoate (7.07 g), 5% palladium-carbon (1.4 g) and tetrahydrofuran (45 ml) was stirred at room temperature for 4 hr under a hydrogen atmosphere. The catalyst was filtered off, and the filtrate was concentrated. The residue was subjected to silica gel column chromatography and eluted with ethyl acetate-hexane (1:6, v/v) to give ethyl 3-[1-(2,4-dichlorobenzyl)-3-isopropyl-1H-pyrazol-5-yl]propionate as a colorless oil (5.36 g, yield 76%) . ¹H-NMR (300 MHz, CDCl₃) δ:1.20 - 1.32 (9 H, m), 2.53 - 2.65 (2 H, m), 2.72 - 2.85 (2 H, m), 2.96 (1 H, septet, J = 7.0 Hz), 4.13 (2 H, q, J = 7.2 Hz), 5.30 (2 H, s), 5.94 (1 H, s), 6.38 (1 H, d, J = 8.4 Hz), 7.13 (1 H, dd, J = 8.4, 2.2 Hz), 7.38 (1 H, d, J = 2.2 Hz).

### Reference Example 78

To a solution of ethyl 3-[1-(2,4-dichlorobenzyl)-3-isopropyl-1H-pyrazol-5-yl]propionate (4.34 g) in tetrahydrofuran (60 ml) was added under ice-cooling a 1.5M solution (20.0 ml) of diisobutylaluminum hydride in toluene over 20 min. After stirring the mixture at room temperature for 30 min, to the reaction solution were added ethanol (20 ml) and then saturated aqueous ammonium chloride (40 ml). The precipitated inorganic substance was filtered and washed with acetone. The filtrate was concentrated, and the obtained residue was subjected to silica gel column chromatography and eluted with ethyl acetate-hexane (3:2, v/v) to give 3-[1-(2,4-dichlorobenzyl)-3-isopropyl-1H-pyrazol-5-yl]propan-1-ol as a colorless oil (3.70 g, yield 96%). ¹H-NMR (300 MHz, CDCl₃) δ:1.27 (6 H, d, J = 6.6 Hz), 1.42 (1 H, brs), 1.75 - 1.92 (2 H, m), 2.55 (2 H, t, J = 7.7 Hz), 2.97 (1 H, septet, J = 7.0 Hz), 3.65 (2 H, q, J = 5.5 Hz), 5.29 (2 H, s), 5.97 (1 H, s), 6.38 (1 H, d, J = 8.4 Hz), 7.12 (1 H, dd, J = 8.4, 2.2 Hz), 7.38 (1 H, d, J = 1.8 Hz).

### Reference Example 79

To a solution of 3-[1-(2,4-dichlorobenzyl)-3-isopropoxy-1H-pyrazol-5-yl]propan-1-ol (1.00 g), ethyl 4-hydroxy-2-methyl-1,3-thiazole-5-carboxylate (0.60 g) and tributylphosphine (1.45 ml) in tetrahydrofuran (60 ml) was added 1,1'-azodicarbonyldipiperidine (1.47 g), and the mixture was stirred at 60°C for 4 hr. The reaction mixture was concentrated, diisopropyl ether was added, and the precipitated crystals were filtered off. The filtrate was concentrated, and the residue was subjected to silica gel column chromatography and eluted with ethyl acetate-hexane (1:4, v/v) to give ethyl 4-{3-[1-(2,4-dichlorobenzyl)-3-isopropoxy-1H-pyrazol-5-yl]propoxy}-2-methyl-1,3-thiazole-5-carboxylate as colorless crystals (1.52 g, yield quant.). melting point 92 - 93°C.

### Reference Example 80

To a solution of ethyl 4-{3-[1-(2,4-dichlorobenzyl)-3-isopropoxy-1H-pyrazol-5-yl]propoxy}-2-methyl-1,3-thiazole-5-carboxylate (1.47 g) in tetrahydrofuran (30 ml) was added under ice-cooling a 1.5M solution (4.80 ml) of diisobutylaluminum hydride in toluene over 10 min. After stirring the mixture at room temperature for 30 min, to the reaction solution were added ethanol (10 ml) and then saturated aqueous ammonium chloride (15 ml). The precipitated inorganic substance was filtered and washed with acetone. The filtrate was concentrated, and the obtained residue was subjected to silica gel column chromatography and eluted with ethyl acetate-hexane (2:3, v/v) to give (4-{3-[1-(2,4-dichlorobenzyl)-3-isopropoxy-1H-pyrazol-5-yl]propoxy}-2-methyl-1,3-thiazol-5-yl)methanol as a colorless oil (0.98 g, yield 73%). ¹H-NMR (300 MHz, CDCl₃) δ:1.33 (6 H, d, J = 6.2 Hz), 1.80 (1 H, t, J = 5.9 Hz), 1.90 - 2.06 (2 H, m), 2.54 - 2.66 (5 H, m), 4.28 (2 H, t, J = 6.2 Hz), 4.58 (2 H, d, J = 5.8 Hz), 4.70 (1 H, septet, J = 6.2 Hz), 5.15 (2 H, s), 5.57 (1 H, s), 6.57 (1 H, d, J = 8.4 Hz), 7.11 (1 H, dd, J = 8.4, 2.2 Hz), 7.36 (1 H, d, J = 1.8 Hz).

### Reference Example 81

To a solution of (4-{3-[1-(2,4-dichlorobenzyl)-3-isopropoxy-1H-pyrazol-5-yl]propoxy}-2-methyl-1,3-thiazol-5-yl)methanol (0.98 g), acetone cyanohydrin (0.40 g) and tributylphosphine (1.05 ml) in tetrahydrofuran (60 ml) was added 1,1'-azodicarbonyldipiperidine (1.06 g), and the mixture was stirred at room temperature for 3 hr. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:4, v/v) to give (4-{3-[1-(2,4-dichlorobenzyl)-3-isopropoxy-1H-pyrazol-5-yl]propoxy}-2-methyl-1,3-thiazol-5-yl)acetonitrile as yellow crystals (0.72 g, yield 71%). melting point 92 - 93°C.

### Reference Example 82

A mixture of ethyl 3-ethoxy-1H-pyrazole-4-carboxylate (13.0 g) and 2,4-dichlorobenzylamine (14.5 ml) was stirred overnight at 180°C. The reaction mixture was cooled to room temperature and the resulting precipitate was collected by filtration to give N-(2,4-dichlorobenzyl)-3-ethoxy-1H-pyrazole-4-carboxamide (15.5 g, yield 70%) as a white solid. Recrystallization from ethyl acetate-hexane gave colorless crystals. melting point 197 - 198°C.

### Reference Example 83

To a solution of N-(2,4-dichlorobenzyl)-3-ethoxy-1H-pyrazole-4-carboxamide (9.86 g) in N,N-dimethylformamide (100 ml) were added potassium carbonate (6.50 g) and ethyl bromoacetate (5.21 ml), and the mixture was stirred overnight at room temperature. Water was added to the reaction mixture, and the mixture was extracted with ethyl acetate. The extract was washed with diluted hydrochloric acid and saturated brine, dried (MgSO₄) and concentrated. The obtained pale-brown solid was recrystallized from ethyl acetate-hexane to give ethyl (4-{[(2,4-dichlorobenzyl)amino]carbonyl}-3-ethoxy-1H-pyrazol-1-yl)acetate (10.6 g, yield 84%) as colorless crystals. melting point 120 - 121°C.

### Reference Example 84

To a mixture of ethyl (4-{[(2,4-dichlorobenzyl)amino]carbonyl}-3-ethoxy-1H-pyrazol-1-yl)acetate (5.00 g), tetrahydrofuran (20 ml) and methanol (80 ml) was added sodium borohydride (2.36 g) at 0°C, and the mixture was stirred at the same temperature for 30 min. Diluted hydrochloric acid was added to the reaction mixture, and the mixture was extracted with ethyl acetate. The extract was washed with diluted hydrochloric acid and saturated brine, dried (MgSO₄) and concentrated. The obtained white solid was recrystallized from ethyl acetate-hexane to give N-(2,4-dichlorobenzyl)-3-ethoxy-1-(2-hydroxyethyl)-1H-pyrazole-4-carboxamide (3.59 g, yield 80%) as colorless crystals. melting point 152 - 153°C.

### Reference Example 85

To a mixture of 1-tert-butyl 4-ethyl 3-hydroxy-1H-pyrazole-1,4-dicarboxylate (15.2 g), isopropanol (5.46 ml), tributylphosphine (29.6 ml) and tetrahydrofuran (250 ml) was added a 40% solution (54.1 ml) of diethyl azodicarboxylate in toluene, and the mixture was stirred overnight at room temperature. The reaction solution was concentrated, and the residue was washed with diisopropyl ether. The washing was concentrated. The residue was subjected to silica gel column chromatography to give a pale-yellow oil from a fraction eluted with ethyl acetate-hexane (1:9 to 1:4, v/v). A mixture of the obtained oil, 40% aqueous methylamine solution (100 ml) and tetrahydrofuran (200 ml) was stirred at room temperature for 1 hr, and the mixture was extracted with ethyl acetate. The extract was washed with saturated brine, dried (MgSO₄) and concentrated. The residue was subjected to silica gel column chromatography to give, from a fraction eluted with ethyl acetate-hexane (3:7, v/v), ethyl 3-isopropoxy-1H-pyrazole-4-carboxylate (5.68 g, yield 66%) was obtained as a white solid. Recrystallization from ethyl acetate-hexane gave colorless crystals. melting point 55 - 56°C.

### Reference Example 86

A mixture of ethyl 3-isopropoxy-1H-pyrazole-4-carboxylate (5.43 g) and 2,4-dichlorobenzylamine (5.53 ml) was stirred overnight at 180°C. The reaction mixture was cooled to room temperature, and the resulting precipitate was collected by filtration to give N-(2,4-dichlorobenzyl)-3-isopropoxy-1H-pyrazole-4-carboxamide (5.97 g, yield 66%) as a white solid. Recrystallization from ethyl acetate-hexane gave colorless crystals. melting point 185 - 186°C.

### Reference Example 87

To a solution of N-(2,4-dichlorobenzyl)-3-isopropoxy-1H-pyrazole-4-carboxamide (3.50 g) in N,N-dimethylformamide (50 ml) were added ethyl bromoacetate (1.79 ml) and potassium carbonate (2.23 g), and the mixture was stirred at room temperature for 3 hr. Water was added to the reaction mixture, and the mixture was extracted with ethyl acetate. The extract was washed with a saturated aqueous ammonium chloride solution and saturated brine, dried (MgSO₄) and concentrated. To a mixture of the obtained white solid, tetrahydrofuran (20 ml) and methanol (60 ml) was added sodium borohydride (2.02 g) at 0°C, and the mixture was stirred at the same temperature for 1 hr. Diluted hydrochloric acid was added to the reaction mixture, and the mixture was extracted with ethyl acetate. The extract was washed with diluted hydrochloric acid and saturated brine, dried (MgSO₄) and concentrated. The obtained white solid was recrystallized from ethyl acetate-hexane to give N-(2,4-dichlorobenzyl)-1-(2-hydroxyethyl)-3-isopropoxy-1H-pyrazole-4-carboxamide (2.98 g, yield 75%) as colorless crystals. melting point 122 - 123°C.

### Reference Example 88

To a solution of ethyl 3-isopropoxy-1H-pyrazole-4-carboxylate (3.00 g) in tetrahydrofuran (80 ml) was added sodium hydride (1.09 g, 60% in oil), and the mixture was stirred at room temperature for 30 min. Then, a solution of 2-(3-bromopropoxy)tetrahydro-2H-pyran (4.40 g) in N,N-dimethylformamide (40 ml) was added, and the mixture was stirred at 50°C for 1 hr. Water was added to the reaction mixture, and the mixture was extracted with ethyl acetate. The extract was washed with a saturated aqueous ammonium chloride solution and saturated brine, dried (MgSO₄) and concentrated. The residue was subjected to silica gel column chromatography to give, from a fraction eluted with ethyl acetate-hexane (1:3, v/v), ethyl 3-isopropoxy-1-[3-(tetrahydro-2H-pyran-2-yloxy)propyl]-1H-pyrazole-4-carboxylate (3.76 g, yield 73%) as a colorless oil. ¹H-NMR (300 MHz, CDCl₃) δ:1.31 (3 H, t, J = 7.2 Hz), 1.39 (6 H, d, J = 6.3 Hz), 1.48 - 1.62 (4 H, m), 1.65 - 1.88 (2 H, m), 2.04 - 2.15 (2 H, m), 3.30 - 3.39 (1 H, m), 3.45 - 3.54 (1 H, m), 3.69 - 3.78 (1 H, m), 3.80 - 3.89 (1 H, m), 4.02 - 4.10 (2 H, m), 4.24 (2 H, q, J = 7.2 Hz), 4.51 - 4.56 (1 H, m), 4.86 - 4.96 (1 H, m), 7.70 (1 H, s).

### Reference Example 89

To a solution of ethyl 3-isopropoxy-1-[3-(tetrahydro-2H-pyran-2-yloxy)propyl]-1H-pyrazole-4-carboxylate (12.0 g) in ethanol (100 ml) was added a 1N aqueous sodium hydroxide solution (100 ml), and the mixture was stirred at 50°C for 5 hr. 1N Hydrochloric acid (100 ml) was added dropwise to the reaction mixture at 0°C, and the mixture was extracted with ethyl acetate. The extract was washed with saturated brine, dried (MgSO₄) and concentrated. A mixture of the obtained colorless oil, 2,4-dichlorobenzylamine (5.67 ml), triethylamine (5.87 ml), 1-ethyl-3-(3-dimethylaminopropyl)carbodiimide hydrochloride (8.07 g), 1-hydroxybenzotriazole monohydrate (6.45 g) and N,N-dimethylformamide (200 ml) was stirred overnight at room temperature. A saturated aqueous ammonium chloride solution was added, and the mixture was extracted with ethyl acetate. The extract was washed with a saturated aqueous ammonium chloride solution and saturated brine, dried (MgSO₄) and concentrated. The residue was subjected to silica gel column chromatography to give, from a fraction eluted with ethyl acetate-hexane (1:1, v/v), N-(2,4-dichlorobenzyl)-3-isopropoxy-1-[3-(tetrahydro-2H-pyran-2-yloxy)propyl]-1H-pyrazole-4-carboxamide (14.9 g, yield 90%) as a colorless oil. ¹H-NMR (300 MHz, CDCl₃) δ:1.38 (6 H, d, J = 6.0 Hz), 1.46 - 1.88 (6 H, m), 2.02 - 2.12 (2 H, m), 3.28 - 3.37 (1 H, m), 3.44 - 3.53 (1 H, m), 3.69 - 3.88 (2 H, m), 4.02 - 4.09 (2 H, m), 4.50 - 4.56 (1 H, m), 4.60 (2 H, d, J = 6.3 Hz), 4.93 - 5.03 (1 H, m), 7.19 - 7.24 (1 H, m), 7.35 - 7.43 (3 H, m), 7.73 (1 H, s).

### Reference Example 90

A mixture of N-(2,4-dichlorobenzyl)-3-isopropoxy-1-[3-(tetrahydro-2H-pyran-2-yloxy)propyl]-1H-pyrazole-4-carboxamide (15.6 g), methanol (150 ml) and 1N hydrochloric acid (50 ml) was stirred overnight at room temperature, and the mixture was extracted with ethyl acetate. The extract was washed with saturated brine, dried (MgSO₄) and concentrated. The residue was subjected to silica gel column chromatography to give, from a fraction eluted with ethyl acetate, N-(2,4-dichlorobenzyl)-1-(3-hydroxypropyl)-3-isopropoxy-1H-pyrazole-4-carboxamide (11.5 g, yield 90%) as a white solid. Recrystallization from ethyl acetate-hexane gave colorless crystals. melting point 91 - 92°C.

### Reference Example 91

To a mixture of ethyl 4-hydroxy-2-methyl-1,3-thiazole-5-carboxylate (7.00 g), isopropanol (3.44 ml), tributylphosphine (18.6 ml) and tetrahydrofuran (200 ml) was added a 40% solution (34.1 ml) of diethyl azodicarboxylate in toluene, and the mixture was stirred overnight at room temperature. The reaction solution was concentrated, and the residue was washed with diisopropyl ether. The washing was concentrated. The residue was subjected to silica gel column chromatography to give, from a fraction eluted with ethyl acetate-hexane (1:19, v/v), ethyl 4-isopropoxy-2-methyl-1,3-thiazole-5-carboxylate (6.71 g, yield 78%) as a pale-yellow oil. ¹H-NMR (300 MHz, CDCl₃) δ:1.32 (3 H, t, J = 7.2 Hz), 1.40 (6 H, d, J = 6.0 Hz), 2.61 (3 H, s), 4.27 (2 H, q, J = 7.2 Hz), 5.13 - 5.26 (1 H, m).

### Reference Example 92

To a mixture of ethyl 4-isopropoxy-2-methyl-1,3-thiazole-5-carboxylate (6.21 g), N-bromosuccinimide (5.30 g) and carbon tetrachloride (150 ml) was added 2,2'-azobis(isobutyronitrile) (445 mg) at room temperature, and the mixture was stirred with heating under reflux for 2 hr. The reaction mixture was cooled to room temperature, the insoluble substance was removed by filtration, and the filtrate was concentrated. The residue was subjected to silica gel column chromatography to give, from a fraction eluted with ethyl acetate-hexane (1:19, v/v), ethyl 2-(bromomethyl)-4-isopropoxy-1,3-thiazole-5-carboxylate (5.43 g, yield 65%) as a pale-yellow solid. Recrystallization from ethyl acetate-hexane gave colorless crystals. melting point 77 - 78°C.

### Reference Example 93

To a solution of ethyl 2-(bromomethyl)-4-isopropoxy-1,3-thiazole-5-carboxylate (5.76 g) in N,N-dimethylformamide (100 ml) was added potassium acetate (3.67 g), and the mixture was stirred at room temperature for 5 hr. To the reaction mixture was added a saturated aqueous ammonium chloride solution, and the mixture was extracted with ethyl acetate. The extract was washed with diluted hydrochloric acid and saturated brine, dried (MgSO₄) and concentrated. The residue was subjected to silica gel column chromatography to give, from a fraction eluted with ethyl acetate-hexane (3:17, v/v), ethyl 2-[(acetyloxy)methyl]-4-isopropoxy-1,3-thiazole-5-carboxylate (4.22 g, yield 79%) as a yellow oil. ¹H-NMR (300 MHz, CDCl₃) δ:1.33 (3 H, t, J = 7.2 Hz), 1.41 (6 H, d, J = 6.0 Hz), 2.19 (3 H, s), 4.29 (2 H, q, J = 7.2 Hz), 5.14 - 5.28 (1 H, m), 5.26 (2 H, s).

### Reference Example 94

To a solution of ethyl 2-[(acetyloxy)methyl]-4-isopropoxy-1,3-thiazole-5-carboxylate (4.60 g) in ethanol (80 ml) was added potassium carbonate (2.65 g), and the mixture was stirred at room temperature for 1 hr. Diluted hydrochloric acid was added to the reaction mixture, and the mixture was extracted with ethyl acetate. The extract was washed with saturated brine, dried (MgSO₄) and concentrated. To a mixture of the obtained yellow solid, 3,4-dihydro-2H-pyran (4.38 ml) and tetrahydrofuran (120 ml) was added (+)-10-camphorsulfonic acid (372 mg) at room temperature, and the mixture was stirred at 50°C for 3 hr. A saturated aqueous sodium hydrogencarbonate solution was added to the reaction mixture, and the mixture was extracted with ethyl acetate. The extract was washed with saturated brine, dried (MgSO₄) and concentrated. The residue was subjected to silica gel column chromatography to give, from a fraction eluted with ethyl acetate-hexane (1:9, v/v), ethyl 4-isopropoxy-2-[(tetrahydro-2H-pyran-2-yloxy)methyl]-1,3-thiazole-5-carboxylate (5.20 g, yield 99%) as a yellow oil. ¹H-NMR (300 MHz, CDCl₃) δ:1.34 (3 H, t, J = 7.4 Hz), 1.40 (6 H, d, J = 6.2 Hz), 1.50 - 2.00 (6 H, m), 3.51 - 3.64 (1 H, m), 3.80 - 3.96 (1 H, m), 4.29 (2 H, q, J = 7.4 Hz), 4.70 (1 H, d, J = 15.4 Hz), 4.78 - 4.84 (1 H, m), 4.90 (1 H, d, J = 15.4 Hz), 5.08 - 5.28 (1 H, m).

### Reference Example 95

To a solution of ethyl 4-isopropoxy-2-[(tetrahydro-2H-pyran-2-yloxy)methyl]-1,3-thiazole-5-carboxylate (5.20 g) in ethanol (60 ml) was added a 1N aqueous sodium hydroxide solution (50 ml), and the mixture was stirred at 50°C for 3 hr. 1N Hydrochloric acid (50 ml) was added dropwise to the reaction mixture at 0°C, and the mixture was extracted with ethyl acetate. The extract was washed with saturated brine, dried (MgSO₄) and concentrated. A mixture of the obtained yellow solid, 2,4-dichlorobenzylamine (2.44 ml), triethylamine (2.52 ml), 1-ethyl-3-(3-dimethylaminopropyl)carbodiimide hydrochloride (3.47 g), 1-hydroxybenzotriazole monohydrate (2.77 g) and N,N-dimethylformamide (100 ml) was stirred overnight at room temperature, and the mixture was extracted with ethyl acetate. The extract was washed with a saturated aqueous ammonium chloride solution and saturated brine, dried (MgSO₄) and concentrated. The residue was subjected to silica gel column chromatography to give, from a fraction eluted with ethyl acetate-hexane (3:17, v/v), N-(2,4-dichlorobenzyl)-4-isopropoxy-2-[(tetrahydro-2H-pyran-2-yloxy)methyl]-1,3-thiazole-5-carboxamide (6.12 g, yield 84%) as a pale-yellow oil. ¹H-NMR (300 MHz, CDCl₃) δ:1.38 (6 H, d, J = 6.0 Hz), 1.50 - 1.96 (6 H, m), 3.52 - 3.60 (1 H, m), 3.82 - 3.92 (1 H, m), 4.63 (2 H, d, J = 6.3 Hz), 4.69 (1 H, d, J = 15.0 Hz), 4.78 - 4.82 (1 H, m), 4.86 (1 H, d, J = 15.0 Hz), 5.16 - 5.30 (1 H, m), 7.22 (1 H, dd, J = 2.1, 8.4 Hz), 7.38 - 7.44 (2 H, m), 7.64 - 7.71 (1 H, brm).

### Reference Example 96

A mixture of N-(2,4-dichlorobenzyl)-4-isopropoxy-2-[(tetrahydro-2H-pyran-2-yloxy)methyl]-1,3-thiazole-5-carboxamide (6.12 g), ethanol (60 ml) and 1N hydrochloric acid (20 ml) was stirred overnight at room temperature, and the mixture was extracted with ethyl acetate. The extract was washed with saturated brine, dried (MgSO₄) and concentrated to give N-(2,4-dichlorobenzyl)-2-(hydroxymethyl)-4-isopropoxy-1,3-thiazole-5-carboxamide (4.97 g, yield quant.) as a white solid. Recrystallization from ethyl acetate-hexane gave colorless crystals. melting point 127 - 128°C.

### Reference Example 97

To a mixture of ethyl 3-(3-isopropoxy-1H-pyrazol-5-yl)propanoate (1.96 g), 4-(trifluoromethyl)benzyl bromide (2.28 g) and N,N-dimethylformamide (30 ml) was added potassium carbonate (1.32 g) at room temperature, and the mixture was stirred overnight at 80°C. To the reaction mixture were added 4-(trifluoromethyl)benzyl bromide (2.28 g) and potassium carbonate (1.32 g), and the mixture was stirred at 120°C for 5 hr. To the reaction mixture was added a saturated aqueous ammonium chloride solution, and the mixture was extracted with ethyl acetate. The extract was washed with a saturated aqueous ammonium chloride solution and saturated brine, dried (MgSO₄) and concentrated. The residue was subjected to silica gel column chromatography to give, from a fraction eluted with ethyl acetate-hexane (3:17, v/v), ethyl 3-{3-isopropoxy-1-[4-(trifluoromethyl)benzyl]-1H-pyrazol-5-yl}propanoate (500 mg, yield 15%) as a yellow oil. ¹H-NMR (300 MHz, CDCl₃) δ:1.23 (3 H, t, J = 7.2 Hz), 1.33 (6 H, d, J = 6.0 Hz), 2.51 - 2.60 (2 H, m), 2.73 - 2.81 (2 H, m), 4.12 (2 H, q, J = 7.2 Hz), 4.64 - 4.77 (1 H, m), 5.21 (2 H, s), 5.51 (1 H, s), 7.18 (2 H, d, J = 8.4 Hz), 7.56 (2 H, d, J = 8.4 Hz).

### Reference Example 98

To a solution of ethyl 3-{3-isopropoxy-1-[4-(trifluoromethyl)benzyl]-1H-pyrazol-5-yl}propanoate (500 mg) in tetrahydrofuran (25 ml) was added dropwise at 0°C a 1.5M solution (2.60 ml) of diisobutylaluminum hydride in toluene, and the mixture was stirred at the same temperature for 30 min. Diluted hydrochloric acid was added to the reaction mixture, and the mixture was extracted with ethyl acetate. The extract was washed with diluted hydrochloric acid and saturated brine, dried (MgSO₄) and concentrated to give 3-{3-isopropoxy-1-[4-(trifluoromethyl)benzyl]-1H-pyrazol-5-yl}propan-1-ol (410 mg, yield 92%) as a yellow oil. ¹H-NMR (300 MHz, CDCl₃) δ:1.34 (6 H, d, J = 6.2 Hz), 1.73 - 1.88 (2 H, m), 2.51 - 2.61 (2 H, m), 3.61 - 3.70 (2 H, m), 4.60 - 4.77 (1 H,m), 5.19 (2 H, s), 5.53 (1 H, s), 7.17 (2 H, d, J = 8.0 Hz), 7.56 (2 H, d, J = 8.0 Hz).

### Reference Example 99

To a mixture of (2,4-dichlorophenyl)acetic acid (10.0 g), oxalyl chloride (4.68 ml) and tetrahydrofuran (100 ml) was added several drops of N,N-dimethylformamide, and the mixture was stirred at room temperature for 1 hr and concentrated to give a brown oil. To a solution of ethyl isocyanoacetate (10.7 ml) in tetrahydrofuran (200 ml) was added dropwise a 1.6M solution (61.0 ml) of n-butyllithium in hexane at -78°C, the mixture was stirred at said temperature for 1 hr, and a solution of the aforementioned oil in tetrahydrofuran (50 ml) was added dropwise. The reaction mixture was stirred at -78°C for 1 hr, and the temperature was gradually raised to room temperature. To the reaction mixture was added a saturated aqueous ammonium chloride solution, and the mixture was extracted with ethyl acetate. The extract was washed with a saturated aqueous ammonium chloride solution and saturated brine, dried (MgSO₄) and concentrated. The residue was subjected to silica gel column chromatography to give, from a fraction eluted with ethyl acetate-hexane (1:6, v/v), ethyl 5-(2,4-dichlorobenzyl)-1,3-oxazole-4-carboxylate (9.74 g, yield 66%) as a yellow oil. ¹H-NMR (300 MHz, CDCl₃) δ:1.40 (3 H, t, J = 7.2 Hz), 4.41 (2 H, q, J = 7.2 Hz), 4.52 (2 H, s), 7.15 (1 H, d, J = 8.4 Hz), 7.19 (1 H, dd, J = 2.4, 8.4 Hz), 7.41 (1 H, d, J = 2.4 Hz), 7.77 (1 H, s).

### Reference Example 100

To a solution of ethyl 5-(2,4-dichlorobenzyl)-1,3-oxazole-4-carboxylate (9.74 g) in ethanol (100 ml) was added concentrated hydrochloric acid (20 ml), and the mixture was stirred overnight at 50°C. The reaction mixture was concentrated, and the residue was washed with diisopropyl ether to give a white solid. To a solution of the obtained solid in N,N-dimethylacetamide (150 ml) was added isobutyryl chloride (3.32 ml), and the mixture was stirred at room temperature for 1 hr. Water (150 ml) was added to the reaction mixture, and the resulting white precipitate was collected by filtration to give ethyl 4-(2,4-dichlorophenyl)-2-(isobutyrylamino)-3-oxobutanoate (9.00 g, yield 77%) as a white solid. Recrystallization from ethyl acetate-hexane gave colorless crystals. melting point 139 - 140°C.

### Reference Example 101

A mixture of ethyl 4-(2,4-dichlorophenyl)-2-(isobutyrylamino)-3-oxobutanoate (8.90 g), Lawesson reagent (11.0 g) and toluene (125 ml) was stirred overnight with heating under reflux. The reaction mixture was concentrated, and the residue was subjected to silica gel column chromatography to give, from a fraction eluted with ethyl acetate-hexane (1:7, v/v), ethyl 5-(2,4-dichlorobenzyl)-2-isopropyl-1,3-thiazole-4-carboxylate (8.41 g, yield 95%) as a pale-yellow solid. ¹H-NMR (300 MHz, CDCl₃) δ:1.34 (6 H, d, J = 6.9 Hz), 1.40 (3 H, t, J = 7.2 Hz), 3.25 - 3.40 (1 H, m), 4.43 (2 H, q, J = 7.2 Hz), 4.63 (2 H, s), 7.17 - 7.24 (2 H, m), 7.41 - 7.43 (1 H, m).

### Reference Example 102

To a solution of ethyl 5-(2,4-dichlorobenzyl)-2-isopropyl-1,3-thiazole-4-carboxylate (8.31 g) in tetrahydrofuran (120 ml) was added dropwise at 0°C a 1.5M solution (70 ml) of diisobutylaluminum hydride in toluene, and the mixture was stirred at the same temperature for 1 hr. Diluted hydrochloric acid was added to the reaction mixture, and the mixture was extracted with ethyl acetate. The extract was washed with diluted hydrochloric acid and saturated brine, dried (MgSO₄) and concentrated. To a solution of the residue in methylene chloride (120 ml) was added Dess-Martin reagent (10.0 g) at 0°C, and the mixture was stirred at room temperature for 1 hr. To the reaction mixture were added a saturated aqueous sodium sulfite solution and a saturated aqueous sodium hydrogencarbonate solution, the resulting insoluble substance was removed by filtration, and the mixture was extracted with ethyl acetate. The extract was washed with a saturated aqueous sodium hydrogencarbonate solution and saturated brine, dried (MgSO₄) and concentrated. The residue was subjected to silica gel column chromatography, from a fraction eluted with ethyl acetate-hexane (3:37, v/v) to give 5-(2,4-dichlorobenzyl)-2-isopropyl-1,3-thiazole-4-carbaldehyde (6.44 g, yield 88%) as a yellow oil. ¹H-NMR (300 MHz, CDCl₃) δ:1.37 (6 H, d, J = 6.9 Hz), 3.18 - 3.33 (1 H, m), 4.65 (2 H, s), 7.20 (1 H, dd, J = 2.1, 8.4 Hz), 7.27 (1 H, d, J = 8.4 Hz), 7.41 (1 H, d, J = 2.1 Hz), 10.18 (1 H, s).

### Reference Example 103

To a solution of ethyl diethylphosphonoacetate (2.08 ml) in N,N-dimethylformamide (50 ml) was added sodium hydride (420 mg, 60% in oil) at 0°C, and the mixture was stirred at room temperature for 1 hr. Then, a solution of 5-(2,4-dichlorobenzyl)-2-isopropyl-1,3-thiazole-4-carbaldehyde (3.00 g) in N,N-dimethylformamide (10 ml) was added at -45°C, and the mixture was stirred at said temperature for 1 hr. A saturated aqueous ammonium chloride solution was added to the reaction solution, and the mixture was extracted with ethyl acetate. The extract was washed with a saturated aqueous ammonium chloride solution and saturated brine, dried (MgSO₄) and concentrated. The residue was subjected to silica gel column chromatography to give, from a fraction eluted with ethyl acetate-hexane (1:7, v/v), ethyl (2E)-3-[5-(2,4-dichlorobenzyl)-2-isopropyl-1,3-thiazol-4-yl]acrylate (2.93 g, yield 80%) as a yellow oil. ¹H-NMR (300 MHz, CDC1₃) δ:1.32 (3 H, t, J = 7.2 Hz), 1.35 (6 H, d, J = 6.9 Hz), 3.14 - 3.29 (1 H, m), 4.26 (2 H, q, J = 7.2 Hz), 4.27 (2 H, s), 6.82 (1 H, d, J = 15.6 Hz), 7.07 (1 H, d, J = 8.4 Hz), 7.19 (1 H, dd, J = 2.4, 8.4 Hz), 7.41 (1 H, d, J = 2.4 Hz), 7.67 (1 H, d, J = 15.6 Hz).

### Reference Example 104

A mixture of ethyl (2E)-3-[5-(2,4-dichlorobenzyl)-2-isopropyl-1,3-thiazol-4-yl]acrylate (3.13 g), 5% palladium-carbon (626 mg) and ethanol (75 ml) was stirred overnight at room temperature under a hydrogen atmosphere. Palladium-carbon was removed by filtration, and the filtrate was concentrated. The residue was subjected to silica gel column chromatography to give, from a fraction eluted with ethyl acetate-hexane (1:4, v/v), ethyl 3-[5-(2,4-dichlorobenzyl)-2-isopropyl-1,3-thiazol-4-yl]propionate (1.55 g, yield 49%) as a colorless oil. ¹H-NMR (300 MHz, CDCl₃) δ:1.24 (3 H, t, J = 7.2 Hz), 1.32 (6 H, d, J = 6.9 Hz), 2.68 - 2.76 (2 H, m), 2.94 - 3.01 (2 H, m), 3.10 - 3.25 (1 H, m), 4.11 (2 H, q, J = 7.2 Hz), 4.13 (2 H, s), 7.06 (1 H, d, J = 8.1 Hz), 7.17 (1 H, dd, J = 1.8, 8.1 Hz), 7.38 (1 H, d, J = 1.8 Hz).

### Reference Example 105

To a mixture of ethyl 3-[5-(2,4-dichlorobenzyl)-2-isopropyl-1,3-thiazol-4-yl]propionate (3.78 g), calcium chloride (2.18 g), ethanol (50 ml) and tetrahydrofuran (50 ml) was added sodium borohydride (1.44 g) at 0°C, and the mixture was stirred at room temperature for 3 hr. To the reaction mixture was added a saturated aqueous ammonium chloride solution, and the mixture was extracted with ethyl acetate. The extract was washed with saturated brine, dried (MgSO₄) and concentrated. The residue was subjected to silica gel column chromatography to give, from a fraction eluted with ethyl acetate-hexane (3:2, v/v), 3-[5-(2,4-dichlorobenzyl)-2-isopropyl-1,3-thiazol-4-yl]propan-1-ol (3.28 g, yield 97%) as a colorless oil. ¹H-NMR (300 MHz, CDCl₃) δ:1.34 (6 H, d, J = 6.9 Hz), 1.84 - 1.94 (2 H, m), 2.78 - 2.87 (2 H, m), 3.12 - 3.27 (1 H, m), 3.67 - 3.74 (2 H, m), 4.10 (2 H, s), 7.06 (1 H, d, J = 8.1 Hz), 7.19 (1 H, dd, J = 2.4, 8.1 Hz), 7.40 (1 H, d, J = 2.4 Hz).

### Reference Example 106

To a solution of methyl 3-propoxy-1H-pyrazole-5-carboxylate (25.3 g) in N,N-dimethylformamide (150 ml) were added benzyl bromide (18.0 ml) and potassium carbonate (21.3 g) at room temperature, and the mixture was stirred at room temperature for 3 days. Water was poured into the reaction mixture, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:10 to 1:5, v/v) to give methyl 1-benzyl-3-propoxy-1H-pyrazole-5-carboxylate as a colorless oil (29.5 g, yield 78%). ¹H-NMR (300 MHz, CDCl₃) δ:1.01 (3 H, t, J = 7.4 Hz), 1.69 - 1.92 (2 H, m), 3.82 (3 H, s), 4.08 (2 H, t, J = 6.7 Hz), 5.60 (2 H, s), 6.23 (1 H, s), 7.20 - 7.48 (5 H, m).

### Reference Example 107

To a solution (150 ml) of methyl 1-benzyl-3-propoxy-1H-pyrazole-5-carboxylate (29.5 g) in tetrahydrofuran was added lithium aluminum hydride (4.1 g) at room temperature, and the mixture was stirred for 30 min. To this reaction mixture was added sodium sulfate decahydrate (34.6 g), and the mixture was filtered through celite and concentrated. The residue was subjected to silica gel column chromatography and eluted with ethyl acetate-hexane (1:3, v/v) to give (1-benzyl-3-propoxy-1H-pyrazol-5-yl)methanol as a colorless oil (19.3 g, yield 73%). ¹H-NMR (300 MHz, CDCl₃) δ:0.99 (3 H, t, J = 7.4 Hz), 1.70 - 1.84 (2 H, m), 2.44 (1 H, brs), 4.03 (2 H, t, J = 7.0 Hz), 4.44 (2 H, s), 5.18 (2 H, s), 5.63 (1 H, s), 7.06 - 7.45 (5 H, m).

### Reference Example 108

To a solution (250 ml) of (1-benzyl-3-propoxy-1H-pyrazol-5-yl)methanol (19.3 g) in methylene chloride were added triethylamine (33.0 ml) and dimethyl sulfoxide (16.6 ml) at room temperature. To this reaction mixture was added sulfur trioxide-pyridine complex (37.4 g), and the mixture was stirred for 20 hr. Water was poured into the reaction mixture, and the mixture was extracted with methylene chloride. The methylene chloride layer was washed with water and saturated brine, dried (MgSO₄) and concentrated. The residue was subjected to silica gel column chromatography and eluted with ethyl acetate-hexane (1:10, v/v) to give 1-benzyl-3-propoxy-1H-pyrazole-5-carbaldehyde as a colorless oil (14.2 g, yield 74%). ¹H-NMR (300 MHz, CDCl₃) δ:1.02 (3 H, t, J = 7.4 Hz), 1.72 - 1.88 (2 H, m), 4.11 (2 H, t, J = 6.6 Hz), 5.56 (2 H, s), 6.26 (1 H, s), 7.20 - 7.48 (5 H, m), 9.71 (1 H, s).

### Reference Example 109

To a solution (200 ml) of ethyl diethylphosphonoacetate (18.8 ml) in tetrahydrofuran was added sodium hydride (3.0 g, 60% in oil) at room temperature, and the mixture was stirred for 10 min. To this reaction mixture was added dropwise a solution (50 ml) of 1-benzyl-3-propoxy-1H-pyrazole-5-carbaldehyde (14.2 g) in tetrahydrofuran, and the mixture was stirred at room temperature for 10 min. Saturated aqueous ammonium chloride was poured into the reaction mixture, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:10, v/v) to give ethyl (2E)-3-(1-benzyl-3-propoxy-1H-pyrazol-5-yl)acrylate as a colorless oil (17.2 g, yield 94%). Recrystallization from ethyl acetate-hexane gave colorless prism crystals. melting point 56 - 57°C.

### Reference Example 110

To a solution (200 ml) of ethyl (2E)-3-(1-benzyl-3-propoxy-1H-pyrazol-5-yl)acrylate (17.2 g) in a mixed solvent of tetrahydrofuran-ethanol (1:1, v/v) was added 10% palladium-carbon (1.6 g), and the mixture was stirred under a hydrogen atmosphere for 20 hr. The reaction mixture was filtered, and the filtrate was concentrated. The residue was subjected to silica gel column chromatography and eluted with ethyl acetate-hexane (1:3, v/v) to give ethyl 3-(1-benzyl-3-propoxy-1H-pyrazol-5-yl)propanoate as a colorless oil (12.3 g, yield 71%). ¹H-NMR (300 MHz, CDCl₃) δ:1.00 (3 H, t, J = 7.4 Hz), 1.23 (3 H, t, J = 7.2 Hz), 1.69 - 1.85 (2 H, m), 2.46 - 2.56 (2 H, m), 2.72 - 2.82 (2 H, m), 4.01 - 4.16 (4 H, m), 5.16 (2 H, s), 5.50 (1 H, s), 7.03 - 7.12 (2 H, m), 7.19 - 7.48 (3 H, m).

### Reference Example 111

To a solution (30 ml) of ethyl 3-(1-benzyl-3-propoxy-1H-pyrazol-5-yl)propanoate (1.0 g) in tetrahydrofuran was added lithium aluminum hydride (120 mg) at room temperature, and the mixture was stirred for 30 min. To this reaction mixture was added sodium sulfate decahydrate (1.0 g), and the mixture was filtered through celite and concentrated. The residue was subjected to silica gel column chromatography and eluted with ethyl acetate-hexane (1:3, v/v) to give 3-(1-benzyl-3-propoxy-1H-pyrazol-5-yl)propan-1-ol as a colorless oil (711 mg, yield 82%). ¹H-NMR (300 MHz, CDCl₃) δ:1.01 (3 H, t, J = 7.4 Hz), 1.29 (1 H, t, J = 4.7 Hz), 1.70 - 1.86 (4 H, m), 2.51 - 2.61 (2 H, m), 3.55 - 3.66 (2 H, m), 4.06 (2 H, t, J = 6.7 Hz), 5.15 (2 H, s), 5.52 (1 H, s), 7.03 - 7.13 (2 H, m), 7.18 - 7.39 (3 H, m).

### Reference Example 112

To a solution of ethyl 3-(1-benzyl-3-propoxy-1H-pyrazol-5-yl)propanoate (11.3 g) in ethanol (150 ml) was added 10% palladium-carbon (11.3 g) at room temperature. To this reaction mixture was added dropwise formic acid (75 ml), and the mixture was heated under reflux for 1 hr. The reaction mixture was filtrated, and concentrated. The residue was neutralized with a saturated aqueous ammonium carbonate solution, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:1, v/v) to give ethyl 3-(3-propoxy-1H-pyrazol-5-yl)propanoate as a colorless oil (8.1 g, yield 100%). ¹H-NMR (300 MHz, CDCl₃) δ:1.00 (3 H, t, J = 7.4 Hz), 1.25 (3 H, t, J = 7.2 Hz), 1.68 - 1.88 (2 H, m), 2.63 (2 H, t, J = 6.9 Hz), 2.88 (2 H, t, J = 6.9 Hz), 4.06 (2 H, t, J = 6.7 Hz), 4.15 (2 H, q, J = 7.2 Hz), 5.49 (1 H, s), 9.85 (1 H, brs).

### Reference Example 113

To a solution of ethyl 3-(3-propoxy-1H-pyrazol-5-yl)propanoate (1.5 g) in N,N-dimethylformamide (30 ml) were added 2,4-dichlorobenzyl chloride (1.4 ml) and potassium carbonate (1.0 g) at room temperature, and the mixture was stirred at 80°C for 12 hr. Water was poured into the reaction mixture, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:10, v/v) to give ethyl 3-[1-(2,4-dichlorobenzyl)-3-propoxy-1H-pyrazol-5-yl]propanoate as a yellow oil (1.1 g, yield 44%). ¹H-NMR (300 MHz, CDCl₃) δ:1.00 (3 H, t, J = 7.4 Hz), 1.24 (3 H, t, J = 7.2 Hz), 1.69 - 1.85 (2 H, m), 2.57 (2 H, t, J = 7.4 Hz), 2.77 (2 H, t, J = 7.4 Hz), 3.98 - 4.20 (4 H, m), 5.19 (2 H, s), 5.55 (1 H, s), 6.55 (1 H, d, J = 8.5 Hz), 7.15 (1 H, dd, J = 8.5, 2.1 Hz), 7.38 (1 H, d, J = 2.1 Hz).

### Reference Example 114

To a solution (30 ml) of ethyl 3-[1-(2,4-dichlorobenzyl)-3-propoxy-1H-pyrazol-5-yl]propanoate (1.1 g) in tetrahydrofuran was added a 1.5M solution (5.0 ml) of diisobutylaluminum hydride in toluene at 0°C, and the mixture was stirred for 15 min. To the reaction mixture was added a saturated aqueous ammonium chloride solution (1.3 ml), and the mixture was filtered through celite and concentrated. The residue was subjected to silica gel column chromatography and eluted with ethyl acetate-hexane (1:4, v/v) to give 3-[1-(2,4-dichlorobenzyl)-3-propoxy-1H-pyrazol-5-yl]propan-1-ol as a colorless oil (795 mg, yield 81%). ¹H-NMR (300 MHz, CDCl₃) δ:1.01 (3 H, t, J = 7.4 Hz), 1.38 (1 H, t, J = 5.0 Hz), 1.69 - 1.88 (4 H, m), 2.49 - 2.61 (2 H, m), 3.58 - 3.73 (2 H, m), 4.05 (2 H, t, J = 6.7 Hz), 5.18 (2 H, s), 5.57 (1 H, s), 6.54 (1 H, d, J = 8.5 Hz), 7.14 (1 H, dd, J = 8.5, 2.1 Hz), 7.37 (1 H, d, J = 2.1 Hz).

### Reference Example 115

To a mixture of ethyl 3-(3-propoxy-1H-pyrazol-5-yl)propionate (1.50 g) and N,N-dimethylformamide (10 ml) was added sodium hydride (0.29 g, 60% in oil) at room temperature, and the mixture was stirred for 15 min. To the reaction mixture was added 4-(trifluoromethyl)benzyl bromide (1.74 g), and the mixture was stirred at room temperature for 2.5 days. Water was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:9 to 1:4, v/v) to give ethyl 3-{3-propoxy-1-[4-(trifluoromethyl)benzyl]-1H-pyrazol-5-yl}propanoate (1.21 g, yield 48%) as a colorless oil. ¹H-NMR (300 MHz, CDCl₃) δ:1.01 (3 H, t, J = 7.2 Hz), 1.25 (3 H, t, J = 7.2 Hz), 1.71 - 1.83 (2 H, m), 2.55 (2 H, t, J = 7.4 Hz), 2.77 (2 H, t, J = 7.4 Hz), 4.05 (2 H, t, J = 7.2 Hz), 4.11 (2 H, q, J = 7.2 Hz), 5.22 (2 H, s), 5.53 (1 H, s), 7.16 - 7.19 (2 H, m), 7.54 - 7.58 (2 H, m).

### Reference Example 116

To a solution of ethyl 3-{3-propoxy-1-[4-(trifluoromethyl)benzyl]-1H-pyrazol-5-yl}propanoate (1.20 g) in tetrahydrofuran (30 ml) was added at 0°C a 1.5M solution (10.3 ml) of diisobutylaluminum hydride in toluene, and the mixture was stirred at room temperature for 2 hr. Saturated aqueous ammonium chloride (2.8 ml) was added to the reaction solution, and the mixture was stirred for 30 min. The resulting insoluble substance was removed by filtration. The filtrate was concentrated, and the obtained residue was subjected to silica gel column chromatography and eluted with ethyl acetate-hexane (1:1, v/v) to give 3-{3-propoxy-1-[4-(trifluoromethyl)benzyl]-1H-pyrazol-5-yl}propan-1-ol (1.00 g, yield 94%) as a colorless oil. ¹H-NMR (300 MHz, CDCl₃) δ:1.01 (3 H, t, J = 7.5 Hz), 1.26 - 1.31 (1 H, m), 1.72 - 1.86 (4 H, m), 2.56 (2 H, t, J = 5.2 Hz), 3.62 - 3.68 (2 H, m), 4.06 (2 H, t, J = 4.5 Hz), 5.20 (2 H, s), 5.55 (1 H, s), 7.15 - 7.16 (2 H, m), 7.54 - 7.61 (2 H, m).

### Reference Example 117

To a solution of ethyl 3-(3-isopropoxy-1H-pyrazol-5-yl)propionate (1.50 g) in N,N-dimethylformamide (20 ml) was added sodium hydride (290 mg, 60% in oil) at room temperature, and the mixture was stirred for 10 min. To the reaction mixture was added 2,4-difluorobenzyl bromide (1.65 g), and the mixture was stirred at room temperature for 4 hr. 1N Hydrochloric acid was added, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:49 to 1:3, v/v) to give ethyl 3-[1-(2,4-difluorobenzyl)-3-isopropoxy-1H-pyrazol-5-yl]propionate (660 mg, yield 28%) as a colorless oil. ¹H-NMR (300 MHz, CDCl₃) δ:1.24 (3 H, t, J = 7.2 Hz), 1.32 (6 H, d, J = 6.3 Hz), 2.52 - 2.62 (2 H, m), 2.77 - 2.87 (2 H, m), 4.12 (2 H, q, J = 7.2 Hz), 4.60 - 4.76 (1 H, m), 5.13 (2 H, s), 5.47 (1 H, s), 6.66 - 6.97 (3 H, m).

### Reference Example 118

To a solution of ethyl 3-[1-(2,4-difluorobenzyl)-3-isopropoxy-1H-pyrazol-5-yl]propionate (500 mg) in tetrahydrofuran (10 ml) was added at 0°C a 1.5M solution (2.4 ml) of diisobutylaluminum hydride in toluene solution. After stirring the mixture at room temperature for 1 hr, methanol was added to quench the reaction. A 10% aqueous Rochelle salt solution (100 ml) was added, and the mixture was stirred for 4 hr and extracted with diethyl ether. The extract was washed with saturated brine, dried (MgSO₄) and concentrated. The residue was subjected to silica gel column chromatography and eluted with ethyl acetate-hexane (1:19 to 1:1, v/v) to give 3-[1-(2,4-difluorobenzyl)-3-isopropoxy-1H-pyrazol-5-yl]propan-1-ol (340 mg, yield 77%) as a colorless oil. ¹H-NMR (200 MHz, CDCl₃) δ:1.33 (6 H, d, J = 6.2 Hz), 1.73 - 1.90 (2 H, m), 2.53 - 2.66 (2 H, m), 3.60 - 3.72 (2 H, m), 4.59 - 4.78 (1 H, m), 5.12 (2 H, s), 5.51 (1 H, s), 6.72 - 7.00 (3 H, m).

### Reference Example 119

To a solution of ethyl 3-(3-isopropoxy-1H-pyrazol-5-yl)propionate (1.50 g) in N,N-dimethylformamide (20 ml) was added sodium hydride (290 mg, 60% in oil) at room temperature, and the mixture was stirred for 10 min. To the reaction mixture was added 2-chlorobenzyl chloride (1.28 g), and the mixture was stirred at room temperature for 4 hr. 1N Hydrochloric acid was added, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:49 to 1:4, v/v) to give ethyl 3-[1-(2-chlorobenzyl)-3-isopropoxy-1H-pyrazol-5-yl]propionate (1.25 g, yield 54%) as a colorless oil. ¹H-NMR (300 MHz, CDCl₃) δ:1.24 (3 H, t, J = 6.9 Hz), 1.33 (6 H, d, J = 6.3 Hz), 2.52 - 2.62 (2 H, m), 2.72 - 2.82 (2 H, m), 4.11 (2 H, q, J = 6.9 Hz), 4.64 - 4.77 (1 H, m), 5.23 (2 H, s), 5.52 (1 H, s), 6.55-6.62 (1 H, m), 7.09 - 7.22 (2 H, m), 7.30 - 7.40 (1 H, m).

### Reference Example 120

To a solution of ethyl 3-[1-(2-chlorobenzyl)-3-isopropoxy-1H-pyrazol-5-yl]propionate (1.25 g) in tetrahydrofuran (30 ml) was added at 0°C a 1.5M solution (8.9 ml) of diisobutylaluminum hydride in toluene. After stirring the mixture at room temperature for 1 hr, methanol was added to quench the reaction. A 10% aqueous Rochelle salt solution (100 ml) was added, and the mixture was stirred for 4 hr and extracted with diethyl ether. The extract was washed with saturated brine, dried (MgSO₄) and concentrated. The residue was subjected to silica gel column chromatography and eluted with ethyl acetate-hexane (1:19 to 1:2, v/v) to give 3-[1-(2-chlorobenzyl)-3-isopropoxy-1H-pyrazol-5-yl]propan-1-ol (990 mg, yield 90%) as a colorless oil. ¹H-NMR (300 MHz, CDC1₃) δ:1.33 (6 H, d, J = 6.0 Hz), 1.74 - 1.86 (2 H, m), 2.51 - 2.58 (2 H, m), 3.60 - 3.68 (2 H, m), 4.65 - 4.78 (1 H, m), 5.22 (2 H, s), 5.54 (1 H, s), 6.57 - 6.63 (1 H, m), 7.10 - 7.21 (2 H, m), 7.31 - 7.36 (1 H, m).

### Reference Example 121

To a solution of ethyl 3-(3-isopropoxy-1H-pyrazol-5-yl)propionate (1.50 g) in N,N-dimethylformamide (20 ml) was added sodium hydride (290 mg, 60% in oil) at room temperature, and the mixture was stirred for 10 min. To the reaction mixture was added 2-methoxybenzyl chloride (1.25 g), and the mixture was stirred at room temperature for 4 hr. 1N Hydrochloric acid was added, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:49 to 1:4, v/v) to give ethyl 3-[1-(2-methoxybenzyl)-3-isopropoxy-1H-pyrazol-5-yl]propionate (870 mg, yield 37%) as a colorless oil. ¹H-NMR (300 MHz, CDCl₃) δ:1.24 (3 H, t, J = 6.9 Hz), 1.32 (6 H, d, J = 6.0 Hz), 2.51 - 2.58 (2 H, m), 2.76 - 2.84 (2 H, m), 3.85 (3 H, s), 4.11 (2 H, q, J = 6.9 Hz), 4.64 - 4.75 (1 H, m), 5.13 (2 H, s), 5.47 (1 H, s), 6.62-6.68 (1 H, m), 6.78 - 6.88 (2 H, m), 7.14 - 7.24 (1 H, m).

### Reference Example 122

To a solution of ethyl 3-[1-(2-methoxybenzyl)-3-isopropoxy-1H-pyrazol-5-yl]propionate (870 mg) in tetrahydrofuran (20 ml) was added at 0°C a 1.5M solution (4.2 ml) of diisobutylaluminum hydride in toluene. After stirring the mixture at room temperature for 1 hr, methanol was added to quench the reaction. A 10% aqueous Rochelle salt solution (100 ml) was added, and the mixture was stirred for 4 hr and extracted with diethyl ether. The extract was washed with saturated brine, dried (MgSO₄) and concentrated. The residue was subjected to silica gel column chromatography and eluted with ethyl acetate-hexane (1:19 to 1:2, v/v) to give 3-[1-(2-methoxybenzyl)-3-isopropoxy-1H-pyrazol-5-yl]propan-1-ol (650 mg, yield 85%) as a colorless oil. ¹H-NMR (300 MHz, CDCl₃) δ:1.33 (6 H, d, J = 6.0 Hz), 1.75 - 1.86 (2 H, m), 2.52 - 2.60 (2 H, m), 3.58 - 3.66 (2 H, m), 3.85 (3 H, s), 4.65 - 4.78 (1 H, m), 5.13 (2 H, s), 5.50 (1 H, s), 6.60 - 6.64 (1 H, m), 6.81 - 6.86 (2 H, m), 7.10 - 7.23 (1 H, m).

### Reference Example 123

To a solution of ethyl 3-(3-isopropoxy-1H-pyrazol-5-yl)propionate (1.50 g) in N,N-dimethylformamide (20 ml) was added sodium hydride (290 mg, 60% in oil) at room temperature, and the mixture was stirred for 10 min. To the reaction mixture was added 2,3-dichlorobenzyl chloride (1.55 g), and the mixture was stirred at room temperature for 4 hr. 1N Hydrochloric acid was added, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:49 to 1:4, v/v) to give ethyl 3-[1-(2,3-dichlorobenzyl)-3-isopropoxy-1H-pyrazol-5-yl]propionate (1.19 g, yield 72%) as a pale-yellow oil. ¹H-NMR (300 MHz, CDCl₃) δ:1.24 (3 H, t, J = 7.2 Hz), 1.32 (6 H, d, J = 6.0 Hz), 2.52 - 2.62 (2 H, m), 2.72 - 2.82 (2 H, m), 4.12 (2 H, q, J = 7.2 Hz), 4.64 - 4.76 (1 H, m), 5.23 (2 H, s), 5.53 (1 H, s), 6.41 - 6.45 (1 H, m), 7.12 (1 H, t, J = 8.0 Hz), 7.34 - 7.38 (1 H, m).

### Reference Example 124

To a solution of ethyl 3-[1-(2,3-dichlorobenzyl)-3-isopropoxy-1H-pyrazol-5-yl]propionate (1.19 g) in tetrahydrofuran (30 ml) was added at 0°C a 1.5M solution (5.2 ml) of diisobutylaluminum hydride in toluene. After stirring the mixture at room temperature for 1 hr, methanol was added to quench the reaction. A 10% aqueous Rochelle salt solution (100 ml) was added, and the mixture was stirred for 4 hr and extracted with diethyl ether. The extract was washed with saturated brine, dried (MgSO₄) and concentrated. The residue was subjected to silica gel column chromatography and eluted with ethyl acetate-hexane (1:19 to 1:4, v/v) to give 3-[1-(2,3-dichlorobenzyl)-3-isopropoxy-1H-pyrazol-5-yl]propan-1-ol (770 mg, yield 73%) as a colorless oil. ¹H-NMR (300 MHz, CDCl₃) δ:1.33 (6 H, d, J = 6.0 Hz), 1.75 - 1.90 (2 H, m), 2.49 - 2.60 (2 H, m), 3.60 - 3.75 (2 H, m), 4.64 - 4.78 (1 H, m), 5.22 (2 H, s), 5.55 (1 H, s), 6.44 (1 H, d J = 8.1 Hz), 7.09 (1 H, t, J = 8.1 Hz), 7.34 (1 H, d, J = 8.1 Hz).

### Reference Example 125

To a solution of ethyl 3-(3-isopropoxy-1H-pyrazol-5-yl)propionate (1.00 g) in N,N-dimethylformamide (20 ml) was added sodium hydride (190 mg, 60% in oil) at room temperature, and the mixture was stirred for 10 min. To the reaction mixture was added 2-chloro-4-fluorobenzyl bromide (1.55 g), and the mixture was stirred at room temperature for 4 hr. 1N Hydrochloric acid was added, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:49 to 1:4, v/v) to give ethyl 3-[1-(2-chloro-4-fluorobenzyl)-3-isopropoxy-1H-pyrazol-5-yl]propionate (790 mg, yield 48%) as a colorless oil. ¹H-NMR (300 MHz, CDCl₃) δ:1.24 (3 H, t, J = 7.2 Hz), 1.32 (6 H, d, J = 6.0 Hz), 2.53 - 2.59 (2 H, m), 2.74 - 2.80 (2 H, m), 4.12 (2 H, q, J = 7.2 Hz), 4.63 - 4.75 (1 H, m), 5.18 (2 H, s), 5.51 (1 H, s), 6.61 (1 H, dd, J = 6.0, 8.7 Hz), 6.85 - 6.91 (1 H, m), 7.10 (1 H, dd, J = 2.5, 8.3 Hz).

### Reference Example 126

To a solution of ethyl 3-[1-(2-chloro-4-fluorobenzyl)-3-isopropoxy-1H-pyrazol-5-yl]propionate (1.19 g) in tetrahydrofuran (20 ml) was added at 0°C a 1.5M solution (3.6 ml) of diisobutylaluminum hydride in toluene. After stirring the mixture at room temperature for 1 hr, methanol was added to quench the reaction. A 10% aqueous Rochelle salt solution (100 ml) was added, and the mixture was stirred for 4 hr and extracted with diethyl ether. The extract was washed with saturated brine, dried (MgSO₄) and concentrated. The residue was subjected to silica gel column chromatography and eluted with ethyl acetate-hexane (1:48 to 3:7, v/v) to give 3-[1-(2-chloro-4-fluorobenzyl)-3-isopropoxy-1H-pyrazol-5-yl]propan-1-ol (500 mg, yield 71%) as a colorless oil. ¹H-NMR (300 MHz, CDC1₃) δ:1.33 (6 H, d, J = 6.0 Hz), 1.75 - 1.87 (2 H, m), 2.52 - 2.59 (2 H, m), 3.61 - 3.70 (2 H, m), 4.64 - 4.76 (1 H, m), 5.16 (2 H, s), 5.54 (1 H, s), 6.61 (1 H, dd, J = 6. 0, 9.0 Hz), 6. 83 - 6. 91 (1 H, m), 7.10 (1 H, dd, J = 2.7, 8.4 Hz).

### Reference Example 127

To a solution of ethyl 3-(3-isopropoxy-1H-pyrazol-5-yl)propionate (1.00 g) in N,N-dimethylformamide (20 ml) was added sodium hydride (190 mg, 60% in oil) at room temperature, and the mixture was stirred for 10 min. To the reaction mixture was added 4-chloro-2-fluorobenzyl bromide (1.55 g), and the mixture was stirred at room temperature for 4 hr. 1N Hydrochloric acid was added, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:49 to 1:4, v/v) to give ethyl 3-[1-(4-chloro-2-fluorobenzyl)-3-isopropoxy-1H-pyrazol-5-yl]propionate (930 mg, yield 57%) as a colorless oil. ¹H-NMR (300 MHz, CDCl₃) δ:1.24 (3 H, t, J = 7.2 Hz), 1.31 (6 H, d, J = 6.0 Hz), 2.53 - 2.61 (2 H, m), 2.76 - 2.84 (2 H, m), 4.12 (2 H, q, J = 7.2 Hz), 4.60 - 4.73 (1 H, m), 5.14 (2 H, s), 5.48 (1 H, s), 6.80 - 6.87 (1 H, m), 7.00 - 7.10 (1 H, m).

### Reference Example 128

To a solution of ethyl 3-[1-(4-chloro-2-fluorobenzyl)-3-isopropoxy-1H-pyrazol-5-yl]propionate (930 mg) in tetrahydrofuran (20 ml) was added at 0°C a 1.5M solution (4.2 ml) of diisobutylaluminum hydride in toluene. After stirring the mixture at room temperature for 2 hr, methanol was added to quench the reaction. A 10% aqueous Rochelle salt solution (100 ml) was added, and the mixture was stirred for 4 hr and extracted with diethyl ether. The extract was washed with saturated brine, dried (MgSO₄) and concentrated. The residue was subjected to silica gel column chromatography and eluted with ethyl acetate-hexane (1:48 to 3:7, v/v) to give 3-[1-(4-chloro-2-fluorobenzyl)-3-isopropoxy-1H-pyrazol-5-yl]propan-1-ol (690 mg, yield 84%) as a colorless oil. ¹H-NMR (300 MHz, CDCl₃) δ:1.32 (6 H, d, J = 6.0 Hz), 1.76 - 1.87 (2 H, m), 2.55 - 2.61 (2 H, m), 3.63 - 3.70 (2 H, m), 4.61 - 4.73 (1 H, m), 5.12 (2 H, s), 5.50 (1 H, s), 6.80 - 6.86 (1 H, m), 7.01 - 7.09 (2 H, m).

### Reference Example 129

To a 1N solution (30 ml) of borane in tetrahydrofuran was added 2-chloro-4-(trifluoromethyl)benzoic acid (2.50 g), and the mixture was stirred at room temperature for 1 hr. The reaction mixture was poured into 1N hydrochloric acid, and the mixture was extracted with ethyl acetate. The extract was washed with aqueous sodium hydrogencarbonate and saturated brine, dried (MgSO₄) and concentrated. The residue was subjected to silica gel column chromatography and eluted with ethyl acetate-hexane (1:24 to 1:4, v/v) to give 2-chloro-4-(trifluoromethyl)benzyl alcohol (2.23 g, yield 96%) as a colorless oil. ¹H-NMR (300 MHz, CDCl₃) δ:4.85 (2 H, d, J = 9.0 Hz), 7.52 - 7.80 (3 H, m).

### Reference Example 130

To a solution of 2-chloro-4-(trifluoromethyl)benzyl alcohol (2.23 g) and triphenylphosphine (4.17 g) in tetrahydrofuran (25 ml) was added carbon tetrabromide (5.27 g), and the mixture was stirred at room temperature for 2 hr. The reaction solution was concentrated, hexane and diethyl ether were added to the residue, and the insoluble substance was removed by filtration. The filtrate was concentrated, and the obtained residue was subjected to silica gel column chromatography and eluted with ethyl acetate-hexane (0:1 to 1:4, v/v) to give 2-chloro-4-(trifluoromethyl)benzyl bromide (2.90 g, yield 99%) as a colorless oil. ¹H-NMR (300 MHz, CDCl₃) δ:4.59 (2 H, s), 7.46 - 7.60 (2 H, m), 7.65 (1 H, s).

### Reference Example 131

To a solution of ethyl 3-(3-isopropoxy-1H-pyrazol-5-yl)propionate (1.50 g) in N,N-dimethylformamide (20 ml) was added sodium hydride (290 mg, 60% in oil) at room temperature, and the mixture was stirred for 10 min. To the reaction mixture was added 2-chloro-4-(trifluoromethyl)benzyl bromide (2.17 g), and the mixture was stirred at room temperature for 4 hr. 1N Hydrochloric acid was added, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:49 to 1:4, v/v) to give ethyl 3-{1-[2-chloro-4-(trifluoromethyl)benzyl]-3-isopropoxy-1H-pyrazol-5-yl}propionate (610 mg, yield 22%) as a yellow oil. ¹H-NMR (300 MHz, CDC1₃) δ:1.23 (3 H, t, J = 7.2 Hz), 1.32 (6 H, d, J = 6.3 Hz), 2.53 - 2.62 (2 H, m), 2.73 - 2.80 (2 H, m), 4.11 (2 H, q, J = 7.2 Hz), 4.64 - 4.76 (1 H, m), 5.25 (2 H, s), 5.54 (1 H, s), 6.69 (1 H, d, J = 7.8 Hz), 7.41 (1 H, d, J = 7.8 Hz), 7.62 (1 H, s).

### Reference Example 132

To a solution of ethyl 3-{1-[2-chloro-4-(trifluoromethyl)benzyl]-3-isopropoxy-1H-pyrazol-5-yl}propionate (610 mg) in tetrahydrofuran (20 ml) was added at 0°C a 1.5M solution (3.6 ml) of diisobutylaluminum hydride in toluene. After stirring the mixture at room temperature for 2 hr, methanol was added to quench the reaction. A 10% aqueous Rochelle salt solution (100 ml) was added, and the mixture was stirred for 4 hr and extracted with diethyl ether. The extract was washed with saturated brine, dried (MgSO₄) and concentrated. The residue was subjected to silica gel column chromatography and eluted with ethyl acetate-hexane (1:48 to 1:4, v/v) to give 3-{1-[2-chloro-4-(trifluoromethyl)benzyl]-3-isopropoxy-1H-pyrazol-5-yl}propan-1-ol (390 mg, yield 71%) as a pale-yellow oil. ¹H-NMR (300 MHz, CDCl₃) δ:1.33 (6 H, d, J = 6.0 Hz), 1.78 - 1.87 (2 H, m), 2.51 - 2.59 (2 H, m), 3.63 - 3.70 (2 H, m), 4.65 - 4.77 (1 H, m), 5.24 (2 H, s), 5.56 (1 H, s), 6.69 (1 H, d, J = 8.2 Hz), 7.41 (1 H, d, J = 8.2 Hz), 7.62 (1 H, s).

### Reference Example 133

To a solution of ethyl 3-(3-isopropoxy-1H-pyrazol-5-yl)propionate (1.00 g) in N,N-dimethylformamide (20 ml) was added sodium hydride (190 mg, 60% in oil) at room temperature, and the mixture was stirred for 10 min. To the reaction mixture was added 2-chloro-6-fluorobenzyl chloride (1.19 g), and the mixture was stirred at room temperature for 4 hr. 1N Hydrochloric acid was added, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:49 to 1:4, v/v) to give ethyl 3-[1-(2-chloro-6-fluorobenzyl)-3-isopropoxy-1H-pyrazol-5-yl]propionate (730 mg, yield 45%) as a colorless oil. ¹H-NMR (300 MHz, CDCl₃) δ:1.25 (6 H, t, J = 6.2 Hz), 1.27 (3 H, d, J = 6.9 Hz), 2.59 - 2.66 (2 H, m), 2.94 - 3.01 (2 H, m), 4.15 (2 H, q, J = 6.9 Hz), 4.48 - 4.60 (1 H, m), 5.20 (2 H, d, J = 1.5 Hz), 5.40 (1 H, s), 6.95 - 7.04 (1 H, m), 7.15 - 7.30 (2 H, m).

### Reference Example 134

To a solution of ethyl 3-[1-(2-chloro-6-fluorobenzyl)-3-isopropoxy-1H-pyrazol-5-yl]propionate (730 mg) in tetrahydrofuran (20 ml) was added at 0°C a 1.5M solution (3.3 ml) of diisobutylaluminum hydride in toluene, and the mixture was stirred at room temperature for 2 hr. A 10% aqueous Rochelle salt solution (100 ml) was added to the reaction solution, and the mixture was stirred for 2 hr and extracted with diethyl ether. The extract was washed with saturated brine, dried (MgSO₄) and concentrated. The residue was subjected to silica gel column chromatography and eluted with ethyl acetate-hexane (1:48 to 1:2, v/v) to give 3-[1-(2-chloro-6-fluorobenzyl)-3-isopropoxy-1H-pyrazol-5-yl]propan-1-ol (580 mg, yield 90%) as a colorless oil. ¹H-NMR (300 MHz, CDCl₃) δ:1.26 (6 H, d, J = 6.2 Hz), 1.87 - 1.92 (2 H, m), 2.72 - 2.79 (2 H, m), 3.69 - 3.76 (2 H, m), 4.49 - 4.62 (1 H, m), 5.20 (2 H, d, J = 1.5 Hz), 5.43 (1 H, s), 6.97 - 7.03 (1 H, m), 7.18 - 7.27 (2 H, m).

### Reference Example 135

To a solution of ethyl 3-(3-isopropoxy-1H-pyrazol-5-yl)propionate (1.50 g) in N,N-dimethylformamide (20 ml) was added sodium hydride (290 mg, 60% in oil) at room temperature, and the mixture was stirred for 10 min. To the reaction mixture was added 4-chloro-2-methylbenzyl chloride (1.74 g), and the mixture was stirred at room temperature for 4 hr. 1N Hydrochloric acid was added, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:49 to 1:4, v/v) to give ethyl 3-[1-(4-chloro-2-methylbenzyl)-3-isopropoxy-1H-pyrazol-5-yl]propionate (1.30 g, yield 54%) as a pale-yellow oil. ¹H-NMR (300 MHz, CDCl₃) δ:1.24 (3 H, t, J = 7.2 Hz), 1.32 (6 H, d, J = 6.0 Hz), 2.31 (3 H, s), 2.50 - 2.60 (2 H, m), 2.70 - 2.74 (2 H, m), 4.11 (2 H, q, J = 7.2 Hz), 4.62 - 4.77 (1 H, m), 5.08 (2 H, s), 5.51 (1 H, s), 6.45 (1 H, d, J = 8.4 Hz), 7.03 - 7.19 (2 H, m) .

### Reference Example 136

To a solution of ethyl 3-[1-(4-chloro-2-methylbenzyl)-3-isopropoxy-1H-pyrazol-5-yl]propionate (1.30 g) in tetrahydrofuran (20 ml) was added at 0°C a 1.5M solution (5.9 ml) of diisobutylaluminum hydride in toluene. After stirring the mixture at room temperature for 2 hr, methanol was added to quench the reaction. A 10% aqueous Rochelle salt solution (100 ml) was added, and the mixture was stirred for 4 hr and extracted with diethyl ether. The extract was washed with saturated brine, dried (MgSO₄) and concentrated. The residue was subjected to silica gel column chromatography and eluted with ethyl acetate-hexane (1:49 to 1:2, v/v) to give 3-[1-(4-chloro-2-methylbenzyl)-3-isopropoxy-1H-pyrazol-5-yl]propan-1-ol (820 mg, yield 71%) as a colorless oil. ¹H-NMR (300 MHz, CDCl₃) δ:1.33 (6 H, d, J = 6.3 Hz), 1.77 - 1.84 (2 H, m), 2.30 (3 H, s), 2.49 - 2.55 (2 H, m), 3.61 - 3.68 (2 H, m), 4.64 - 4.77 (1 H, m), 5.05 (2 H, s), 5.53 (1 H, s), 6.44 (1 H, d, J = 8.4 Hz), 7.05 (1 H, dd, J = 2.1, 8.1 Hz), 7.13 (1 H, d, J = 2.1 Hz).

### Reference Example 137

To a solution of 4-chlorosalicylic acid (10.0 g) and ethyl iodide (22.6 g) in N,N-dimethylformamide (100 ml) was added potassium carbonate (28.0 g), and the mixture was stirred at room temperature for 20 hr. The reaction mixture was poured into water, and the mixture was extracted with ethyl acetate. The extract was washed with water and saturated brine, dried (MgSO₄) and concentrated. The residue was subjected to silica gel column chromatography and eluted with ethyl acetate-hexane (1:49 to 3:17, v/v) to give ethyl 4-chloro-2-ethoxybenzoate (8.80 g, yield 66%) as a colorless oil. ¹H-NMR (300 MHz, CDCl₃) δ:1.37 (3 H, t, J = 7.2 Hz), 1.47 (3 H, t, J = 7.0 Hz), 4.09 (2 H, q, J = 7.0 Hz), 4.34 (2 H, q, J = 7.2 Hz), 6.90 - 6.99 (2 H, m), 7.70 - 7.80 (1 H, m).

### Reference Example 138

To a solution of ethyl 4-chloro-2-ethoxybenzoate (8.80 g) in tetrahydrofuran (150 ml) was added at 0°C a 1.5M solution (77.0 ml) of diisobutylaluminum hydride in toluene. After stirring the mixture at room temperature for 2 hr, methanol was added to quench the reaction. A 10% aqueous Rochelle salt solution (300 ml) was added to the reaction solution, and the mixture was stirred for 4 hr and extracted with diethyl ether. The extract was washed with saturated brine, dried (MgSO₄) and concentrated. The residue was subjected to silica gel column chromatography and eluted with ethyl acetate-hexane (1:19 to 1:4, v/v) to give (4-chloro-2-ethoxyphenyl)methanol (6.90 g, yield 96%) as colorless solid. ¹H-NMR (300 MHz, CDCl₃) δ:1.45 (3 H, t, J = 7.1 Hz), 2.25 (1 H, t, J = 6.6 Hz), 4.06 (2 H, q, J = 7.2 Hz), 4.63 (2 H, d, J = 6.6 Hz), 6.84 (1 H, d, J = 2.0 Hz), 6.90 (1 H, dd, J = 2.0, 8.0 Hz), 7.19 (1 H, d, J = 8.0 Hz).

### Reference Example 139

To a mixture of (4-chloro-2-ethoxyphenyl)methanol (6.90 g), pyridine (0.6 ml), tetrahydrofuran (30 ml) and diethyl ether (120 ml) was added thionyl chloride (4.0 ml), and the mixture was stirred at room temperature for 15 hr. The reaction mixture was poured into water, and the mixture was extracted with ethyl acetate. The extract was washed with 1N hydrochloric acid, saturated aqueous sodium hydrogencarbonate and saturated brine, dried (MgSO₄) and concentrated. The residue was subjected to silica gel column chromatography and eluted with ethyl acetate-hexane (1:49 to 1:9, v/v) to give 4-chloro-(1-chloromethyl)-2-ethoxybenzene (7.05 g, yield 93%) as a colorless oil. ¹H-NMR (300 MHz, CDCl₃) δ:1.46 (3 H, t, J = 7.0 Hz), 4.07 (2 H, q, J = 7.0 Hz), 4. 61 (2 H, s), 6.86 (1 H, d, J = 2.0 Hz), 6.91 (1 H, dd, J = 2.0, 8.3 Hz), 7.27 (1 H, d, J = 8.3 Hz).

### Reference Example 140

To a solution of ethyl 3-(3-isopropoxy-1H-pyrazol-5-yl)propionate (1.50 g) in N,N-dimethylformamide (20 ml) was added sodium hydride (290 mg, 60% in oil) at room temperature, and the mixture was stirred for 10 min. To the reaction mixture was added 4-chloro-(1-chloromethyl)-2-ethoxybenzene (2.04 g), and the mixture was stirred at room temperature for 4 hr. 1N Hydrochloric acid was added, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:49 to 1:4, v/v) to give ethyl 3-[1-(4-chloro-2-ethoxybenzyl)-3-isopropoxy-1H-pyrazol-5-yl]propionate (1.23 g, yield 47%) as a yellow oil. ¹H-NMR (300 MHz, CDCl₃) δ:1.24 (3 H, t, J = 7.2 Hz), 1.31 (6 H, d, J = 6.3 Hz), 2.51 - 2.58 (2 H, m), 2.76 - 2.83 (2 H, m), 4.04 (2 H, q, J = 6.9 Hz), 4.11 (2 H, q, J = 7.2 Hz), 4.61 - 4.75 (1 H, m), 5.08 (2 H, s), 5.47 (1 H, s), 6.59 (1 H, d, J = 8.4 Hz), 6.75 - 6.85 (2 H, m).

### Reference Example 141

To a solution of ethyl 3-[1-(4-chloro-2-ethoxybenzyl)-3-isopropoxy-1H-pyrazol-5-yl]propionate (1.23 g) in tetrahydrofuran (20 ml) was added at 0°C a 1.5M solution (5.2 ml) of diisobutylaluminum hydride in toluene. After stirring the mixture at room temperature for 2 hr, methanol was added to quench the reaction. A 10% aqueous Rochelle salt solution (100 ml) was added, and the mixture was stirred for 4 hr and extracted with diethyl ether. The extract was washed with saturated brine, dried (MgSO₄) and concentrated. The residue was subjected to silica gel column chromatography and eluted with ethyl acetate-hexane (1:49 to 1:2, v/v) to give 3-[1-(4-chloro-2-ethoxybenzyl)-3-isopropoxy-1H-pyrazol-5-yl]propan-1-ol (880 mg, yield 80%) as a colorless oil. ¹H-NMR (300 MHz, CDCl₃) δ:1.32 (6 H, d, J = 6.3 Hz), 1.44 (3 H, t, J = 7.2 Hz), 1.76 - 1.90 (2 H, m), 2.50 - 2.60 (2 H, m), 3.61 - 3.70 (2 H, m), 4.04 (2 H, q, J = 7.2 Hz), 4.63 - 4.76 (1 H, m), 5.07 (2 H, s), 5.50 (1 H, s), 6.55 (1 H, d, J = 8.7 Hz), 6.78 - 6.83 (2 H, m).

### Reference Example 142

To a solution of 4-chlorosalicylic acid (10.0 g) and isopropyl iodide (24.6 g) in N,N-dimethylformamide (100 ml) was added potassium carbonate (28.0 g), and the mixture was stirred at room temperature for 15 hr. The reaction mixture was poured into water, and the mixture was extracted with ethyl acetate. The extract was washed with water and saturated brine, dried (MgSO₄) and concentrated. The residue was subjected to silica gel column chromatography and eluted with ethyl acetate-hexane (1:49 to 1:9, v/v) to give isopropyl 4-chloro-2-isopropoxybenzoate (10.6 g, yield 71%) as a colorless oil. ¹H-NMR (300 MHz, CDCl₃) δ:1.34 (6 H, t, J = 6. 3 Hz), 1.37 (6 H, d, J = 6.3 Hz), 4.51 - 4.64 (1 H, m), 5.16 - 5.29 (1 H, m), 6.90 - 6.95 (2 H, m), 7.66 (1 H, d, J = 8.4 Hz).

### Reference Example 143

To a solution of isopropyl 4-chloro-2-isopropoxybenzoate (10.6 g) in tetrahydrofuran (150 ml) was added at 0°C a 1.5M solution (82.0 ml) of diisobutylaluminum hydride in toluene. After stirring the mixture at room temperature for 4 hr, methanol was added to quench the reaction. A 10% aqueous Rochelle salt solution (300 ml) was added to the reaction solution, and the mixture was stirred for 4 hr and extracted with diethyl ether. The extract was washed with saturated brine, dried (MgSO₄) and concentrated. The residue was subjected to silica gel column chromatography and eluted with ethyl acetate-hexane (1:49 to 1:4, v/v) to give (4-chloro-2-isopropoxyphenyl)methanol (7.00 g, yield 85%) as a pale-yellow oil. ¹H-NMR (300 MHz, CDCl₃) δ:1.37 (6 H, d, J = 6.0 Hz), 2.29 (1 H, t, J = 6.6 Hz), 4.52 - 4.67 (3 H, m), 6. 84 - 6.93 (2 H, m), 7.20 (1 H, d, J = 8.1 Hz).

### Reference Example 144

To a mixture of (4-chloro-2-isopropoxyphenyl)methanol (7.00 g), pyridine (0.6 ml), tetrahydrofuran (30 ml) and diethyl ether (120 ml) was added thionyl chloride (3.8 ml), and the mixture was stirred at room temperature for 15 hr. The reaction mixture was poured into water, and the mixture was extracted with ethyl acetate. The extract was washed with 1N hydrochloric acid, saturated aqueous sodium hydrogencarbonate and saturated brine, dried (MgSO₄) and concentrated. The residue was subjected to silica gel column chromatography and eluted with ethyl acetate-hexane (1:49 to 1:9, v/v) to give 4-chloro-(1-chloromethyl)-2-isopropoxybenzene (6.40 g, yield 84%) as a colorless oil. ¹H-NMR (300 MHz, CDCl₃) δ:1.37 (6 H, d, J = 6.0 Hz), 4.53 - 4.63 (3 H, m), 6.86 - 6.93 (2 H, m), 7.26 - 7.28 (1 H, m).

### Reference Example 145

To a solution of ethyl 3-(3-isopropoxy-1H-pyrazol-5-yl)propionate (1.12 g) in N,N-dimethylformamide (20 ml) was added sodium hydride (290 mg, 60% in oil) at room temperature, and the mixture was stirred for 10 min. To the reaction mixture was added 4-chloro-(1-chloromethyl)-2-isopropoxybenzene (1.63 g), and the mixture was stirred at room temperature for 4 hr. 1N Hydrochloric acid was added, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:49 to 1:4, v/v) to give ethyl 3-[1-(4-chloro-2-isopropoxybenzyl)-3-isopropoxy-1H-pyrazol-5-yl]propionate (1.32 g, yield 65%) as a yellow oil. ¹H-NMR (300 MHz, CDCl₃) δ:1.24 (3 H, t, J = 7.1 Hz), 1.32 (6 H, d, J = 6.0 Hz), 1.36 (6 H, d, J = 6.1 Hz), 2.51 - 2.62 (2 H, m), 2.74 - 2.84 (2 H, m), 4.13 (2H, q, J = 7.1 Hz), 4.48 - 4.62 (1 H, m), 4.63 - 4.75 (1H, m), 5.06 (2 H, s), 5.47 (1 H, s), 6.60 (1 H, d, J = 8.1 Hz), 6.76 - 6.84 (2 H, m).

### Reference Example 146

To a solution of ethyl 3-[1-(4-chloro-2-isopropoxybenzyl)-3-isopropoxy-1H-pyrazol-5-yl]propionate (1.32 g) in tetrahydrofuran (20 ml) was added at 0°C a 1.5M solution (5.4 ml) of diisobutylaluminum hydride in toluene. After stirring the mixture at room temperature for 2 hr, methanol was added to quench the reaction. A 10% aqueous Rochelle salt solution (100 ml) was added, and the mixture was stirred for 4 hr and extracted with diethyl ether. The extract was washed with saturated brine, dried (MgSO₄) and concentrated. The residue was subjected to silica gel column chromatography and eluted with ethyl acetate-hexane (1:49 to 1:2, v/v) to give 3-[1-(4-chloro-2-isopropoxybenzyl)-3-isopropoxy-1H-pyrazol-5-yl]propan-1-ol (1.06 g, yield 89%) as a colorless oil. ¹H-NMR (300 MHz, CDCl₃) δ:1.33 (6 H, d, J = 6.0 Hz), 1.36 (6 H, d, J = 6.0 Hz), 1.75 - 1.86 (2 H, m), 2.52 - 2.60 (2 H, m), 3.61 - 3.69 (2 H, m), 4.49 - 4.61 (1 H, m), 4.63 - 4.76 (1 H, m), 5.05 (2 H, s), 5.50 (1 H, s), 6.57 (1 H, d, J = 8.1 Hz), 6.76 - 6.84 (2 H, m).

### Reference Example 147

To a solution of ethyl 3-isopropyl-1H-pyrazole-5-carboxylate (4.39 g) and 2-chloro-4-(trifluoromethyl)benzyl chloride (6.07 g) in N,N-dimethylformamide (70 ml) was added potassium carbonate (3.99 g), and the mixture was stirred at room temperature for 15 hr. The reaction mixture was poured into water, and the mixture was extracted with ethyl acetate. The extract was washed with water and saturated brine, dried (MgSO₄) and concentrated. The residue was subjected to silica gel column chromatography and eluted with ethyl acetate-hexane (1:49 to 1:4, v/v) to give ethyl 1-[2-chloro-4-(trifluoromethyl)benzyl]-3-isopropyl-1H-pyrazole-5-carboxylate (3.80 g, yield 42%) as a colorless oil. ¹H-NMR (300 MHz, CDCl₃) δ:1.29 (3 H, t, J = 7.1 Hz), 1.30 (6 H, d, J = 7.0 Hz), 2.91 - 3.10 (1 H, m), 4.26 (2 H, q, J = 7.1 Hz), 5.85 (2 H, s), 6.48 (1 H, d, J = 8.1 Hz), 6.80 (1 H, s), 7.38 (1 H, d, J = 8.1 Hz), 7.64 (1 H, s).

### Reference Example 148

To a solution of ethyl 1-[2-chloro-4-(trifluoromethyl)benzyl]-3-isopropyl-1H-pyrazole-5-carboxylate (3.80 g) in tetrahydrofuran (50 ml) was added at 0°C a 1.5M solution (17 ml) of diisobutylaluminum hydride in toluene. After stirring the mixture at room temperature 1 hr, methanol was added to quench the reaction. A 10% aqueous Rochelle salt solution (200 ml) was added to the reaction solution, and the mixture was stirred for 4 hr and extracted with diethyl ether. The extract was washed with saturated brine, dried (MgSO₄) and concentrated. The residue was subjected to silica gel column chromatography and eluted with ethyl acetate-hexane (1:19 to 2:3, v/v) to give {1-[2-chloro-4-(trifluoromethyl)benzyl]-3-isopropyl-1H-pyrazol-5-yl}methanol (3.37 g, yield 99%) as a pale-yellow oil. ¹H-NMR (300 MHz, CDCl₃) δ:1.29 (6 H, d, J = 6.9 Hz), 1.63 (1 H, t, J = 5.6 Hz), 2.92 - 3.06 (1 H, m), 4.57 (1 H, d, J = 5.6 Hz), 5.49 (2 H, s), 6.14 (1 H, s), 6.59 (1 H, d, J = 8.0 Hz), 7.39 (1 H, dd, J = 1.2, 8.0 Hz), 7.62 (1 H, d, J = 1.2 Hz).

### Reference Example 149

Under nitrogen atmosphere, to a solution of oxalyl chloride (2.56 g) in methylene chloride (50 ml) was added dimethyl sulfoxide (2.15 ml) at -78°C. The mixture was stirred at -78°C for 5 min, and a solution of {1-[2-chloro-4-(trifluoromethyl)benzyl]-3-isopropyl-1H-pyrazol-5-yl}methanol (3.37 g) in methylene chloride (35 ml) was added. After stirring at -78°C for 1 hr, triethylamine (5.11 g) was added. After stirring the mixture at room temperature for 1 hr, the reaction mixture was poured into water, and the mixture was extracted with ethyl acetate. The extract was washed with 1N hydrochloric acid, saturated aqueous sodium hydrogencarbonate and saturated brine, dried (MgSO₄) and concentrated. The residue was subjected to silica gel column chromatography and eluted with ethyl acetate-hexane (1:19 to 1:4, v/v) to give 1-[2-chloro-4-(trifluoromethyl)benzyl]-3-isopropyl-1H-pyrazole-5-carbaldehyde (3.32 g, yield 99%) as a pale-yellow oil. ¹H-NMR (300 MHz, CDCl₃) δ:1.31 (6 H, d, J = 7.2 Hz), 2.98 - 3.13 (1 H, m), 5.82 (2 H, s), 6.52 (1 H, d, J = 8.1 Hz), 6.83 (1 H, s), 7.36 (1 H, d, J = 1.2, 8.1 Hz), 7.65 (1 H, d, J = 1.2 Hz), 9.79 (1 H, s).

### Reference Example 150

Under ice-cooling, to a suspension of sodium hydride (560 mg, 60% in oil) in N,N-dimethylformamide (30 ml) was added a mixed solution of 1-[2-chloro-4-(trifluoromethyl)benzyl]-3-isopropyl-1H-pyrazole-5-carbaldehyde (3.32 g) and ethyl diethylphosphonoacetate (2.69 g) in tetrahydrofuran (15 ml). After stirring the mixture at room temperature for 1.5 hr, the reaction mixture was poured into water, and the mixture was extracted with ethyl acetate. The extract was washed with water and saturated brine, dried (MgSO₄) and concentrated. The residue was subjected to silica gel column chromatography and eluted with ethyl acetate-hexane (1:49 to 3:17, v/v) to give ethyl (2E)-3-{1-[2-chloro-4-(trifluoromethyl)benzyl]-3-isopropyl-1H-pyrazol-5-yl}acrylate (1.18 g, yield 29%) as a pale-yellow oil. ¹H-NMR (300 MHz, CDCl₃) δ:1.28 (6 H, d, J = 6.9 Hz), 1.30 (3 H, t, J = 7.2 Hz), 2.93 - 3.07 (1 H, m), 4.21 (2 H, q, J = 7.2 Hz), 5.51 (2 H, s), 6.31 (1 H, d, J = 15.8 Hz), 6.50 (1 H, s), 6.60 (1 H, d, J = 7.8 Hz), 7.38 (1 H, d, J = 15.8 Hz), 7.40 (1 H, d, J = 7.8 Hz), 7.65 (1 H, s).

### Reference Example 151

A mixture of ethyl (2E)-3-{1-[2-chloro-4-(trifluoromethyl)benzyl]-3-isopropyl-1H-pyrazol-5-yl}acrylate (1.18 g), 5% palladium-carbon (290 mg) and tetrahydrofuran (15 ml) was hydrogenated at room temperature at normal pressure. The reaction mixture was filtrated, and the filtrate was concentrated to give ethyl 3-{1-[2-chloro-4-(trifluoromethyl)benzyl]-3-isopropyl-1H-pyrazol-5-yl}propionate (1.00 g, yield 84%) as a pale-yellow oil. ¹H-NMR (300 MHz, CDCl₃) δ:1.23 (3 H, t, J = 7.2 Hz), 1.26 (6 H, d, J = 7.2 Hz), 2.57 - 2.64 (2 H, m), 2.74 - 2.81 (2 H, m), 2.89 - 3.02 (1 H, m), 4.11 (2 H, q, J = 7.2 Hz), 5.38 (2 H, s), 5.96 (1 H, s), 6.51 (1 H, d, J = 8.1 Hz), 7.40 (1 H, dd, J = 1. 2, 8.1 Hz), 7. 63 (1 H, d, J = 1.2 Hz).

### Reference Example 152

To a solution of ethyl 3-{1-[2-chloro-4-(trifluoromethyl)benzyl]-3-isopropyl-1H-pyrazol-5-yl}propionate (1.00 g) in tetrahydrofuran (15 ml) was added at 0°C a 1.5M solution (4.1 ml) of diisobutylaluminum hydride in toluene. After stirring the mixture at room temperature for 1 hr, methanol was added to quench the reaction. A 10% aqueous Rochelle salt solution (100 ml) was added, and the mixture was stirred for 4 hr and extracted with diethyl ether. The extract was washed with saturated brine, dried (MgSO₄) and concentrated. The residue was subjected to silica gel column chromatography and eluted with ethyl acetate-hexane (1:19 to 2:3, v/v) to give 3-{1-[2-chloro-4-(trifluoromethyl)benzyl]-3-isopropyl-1H-pyrazol-5-yl}propan-1-ol (500 mg, yield 56%) as a colorless oil. ¹H-NMR (300 MHz, CDCl₃) δ:1.27 (6 H, d, J = 7.2 Hz), 1.80 - 1.90 (2 H, m), 2.53 - 2.60 (2 H, m), 2.90 - 3.04 (1 H, m), 3.62 - 3.71 (2 H, m), 5.36 (2 H, s), 5.98 (1 H, s), 6.51 (1 H, d, J = 8.4 Hz), 7.39 (1 H, dd, J = 1.2, 8.4 Hz), 7.62 (1 H, d, J = 1.2 Hz).

### Reference Example 153

To a solution of N,N,N'-trimethylethylenediamine (3.23 g) in tetrahydrofuran (60 ml) was added at 0°C a 1.6M solution (19 ml) of butyllithium in hexane, and the mixture was stirred for 15 min. After cooling the reaction mixture to -45°C, 4-(trifluoromethyl)benzaldehyde (5.00 g) was added. After stirring the mixture for 30 min, a 1.6M solution (56 ml) of butyllithium in hexane was added. After stirring the mixture for 3 hr, methyl iodide (32.6 g) was added. The temperature of the reaction mixture was raised to room temperature, and the reaction mixture was stirred for 1 hr and poured into 1N hydrochloric acid. The mixture was extracted with ethyl acetate. The extract was washed with saturated brine, dried (MgSO₄) and concentrated. The residue was subjected to silica gel column chromatography and eluted with ethyl acetate-hexane (0:1 to 1:19, v/v) to give 2-methyl-4-(trifluoromethyl)benzaldehyde (2.90 g, yield 54%) as a yellow oil. ¹H-NMR (300 MHz, CDCl₃) δ:2.74 (3 H, s), 7.54 (1 H, s), 7.62 (1 H, d, J = 7.8 Hz), 7.92 (1 H, d, J = 7.8 Hz), 10.35 (1 H, s).

### Reference Example 154

To a solution of 2-methyl-4-(trifluoromethyl)benzaldehyde (2.90 g) in ethanol (40 ml) was added sodium borohydride (875 mg), and the mixture was stirred at room temperature for 1 hr. The reaction mixture was poured into 1N hydrochloric acid, and the mixture was extracted with ethyl acetate. The extract was washed with saturated brine, dried (MgSO₄) and concentrated. The residue was subjected to silica gel column chromatography and eluted with ethyl acetate-hexane (1:19 to 1:3, v/v) to give [2-methyl-4-(trifluoromethyl)phenyl]methanol (2.93 g, yield 99%) as a colorless oil. ¹H-NMR (300 MHz, CDCl₃) δ:1.88 (1 H, brs), 2.37 (3 H, s), 4.74 (2 H, s), 7.39 - 7.54 (1 H, m).

### Reference Example 155

To a mixture of [2-methyl-4-(trifluoromethyl)phenyl]methanol (2.93 g), pyridine (0.3 ml), tetrahydrofuran (5.0 ml) and diethyl ether (60 ml) was added thionyl chloride (1.7 ml), and the mixture was stirred at room temperature for 2 hr. The reaction mixture was concentrated, and partitioned between ethyl acetate and water. The organic layer was washed with 1N hydrochloric acid, saturated aqueous sodium hydrogencarbonate and saturated brine, dried (MgSO₄) and concentrated. The residue was subjected to silica gel column chromatography and eluted with ethyl acetate-hexane (1:49 to 1:9, v/v) to give 1-(chloromethyl)-2-methyl-4-(trifluoromethyl)benzene (2.52 g, yield 78%) as a colorless oil. ¹H-NMR (300 MHz, CDCl₃) δ:2.48 (3 H, s), 4.61 (2 H, s), 7.35 - 7.50 (3 H, m).

### Reference Example 156

To a solution of ethyl 3-(3-isopropoxy-1H-pyrazol-5-yl)propionate (1.38 g) in N,N-dimethylformamide (18 ml) was added sodium hydride (270 mg, 60% in oil) at room temperature, and the mixture was stirred for 10 min. To the reaction mixture was added 1-(chloromethyl)-2-methyl-4-(trifluoromethyl)benzene (1.91 g), and the mixture was stirred at room temperature for 4 hr. 1N Hydrochloric acid was added, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:49 to 3:17, v/v) to give ethyl 3-{3-isopropoxy-1-[2-methyl-4-(trifluoromethyl)benzyl]-1H-pyrazol-5-yl}propionate (1.19 g, yield 49%) as a colorless oil. ¹H-NMR (300 MHz, CDCl₃) δ:1.23 (3 H, t, J = 7.1 Hz), 1.32 (6 H, d, J = 6.3 Hz), 2.39 (3 H, s), 2.52 - 2.62 (2 H, m), 2.70 - 2.76 (2 H, m), 4.11 (2 H, q, J = 7.2 Hz), 4.64 - 4.77 (1 H, m), 5.15 (2 H, s), 5.54 (1 H, s), 6.59 (1 H, d, J = 8.1 Hz), 7.35 (1 H, d, J = 8.1 Hz), 7.41 (1 H, s).

### Reference Example 157

To a solution of ethyl 3-{3-isopropoxy-1-[2-methyl-4-(trifluoromethyl)benzyl]-1H-pyrazol-5-yl}propionate (1.19 g) in tetrahydrofuran (20 ml) was added at 0°C a 1.5M solution (5.0 ml) of diisobutylaluminum hydride in toluene. After stirring the mixture at room temperature for 1 hr, methanol was added to quench the reaction. A 10% aqueous Rochelle salt solution (100 ml) was added, and the mixture was stirred for 4 hr and extracted with diethyl ether. The extract was washed with saturated brine, dried (MgSO₄) and concentrated. The residue was subjected to silica gel column chromatography and eluted with ethyl acetate-hexane (1:49 to 1:2, v/v) to give 3-{3-isopropoxy-1-[2-methyl-4-(trifluoromethyl)benzyl]-1H-pyrazol-5-yl}propan-1-ol (900 mg, yield 84%) as a colorless oil. ¹H-NMR (300 MHz, CDCl₃) δ:1.33 (6 H, d, J = 6.2 Hz), 1.73 - 1.92 (2 H, m), 2.38 (3 H, s), 2.48 - 2.60 (2 H, m), 3.60 - 3.75 (2 H, m), 4.62 - 4.81 (1 H, m), 5.14 (2 H, s), 5.56 (1 H, s), 6.58 (1 H, d, J = 8.1 Hz), 7.35 (1 H, d, J = 8.1 Hz), 7.40 (1 H, s).

### Reference Example 158

To a suspension of sodium hydride (2.73 g, 60% in oil) in N,N-dimethylformamide (50 ml) was added ethyl 3-(3-propoxy-1H-pyrazol-5-yl)propionate (1.50 g) at room temperature, and the mixture was stirred for 15 min. To the reaction mixture was added 4-chlorobenzyl chloride (1.10 g), and the mixture was stirred at room temperature for 30 min. Water was added, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:20 to 1:2, v/v) to give ethyl 3-[1-(4-chlorobenzyl)-3-propoxy-1H-pyrazol-5-yl]propionate (1.50 g, yield 65%) as a colorless oil. ¹H-NMR (300 MHz, CDCl₃) δ:1.00 (3 H, t, J = 7.41 Hz), 1.24 (3 H, t, J = 7.14 Hz), 1.69 - 1.85 (2 H, m), 2.48 - 2.58 (2 H, m), 2.71 - 2.80 (2 H, m), 4.04 (2 H, t, J = 6.72 Hz), 4.11 (2 H, q, J = 7.14 Hz), 5.11 (2 H, s), 5.49 (1 H, d, J = 0.55 Hz), 7.00 (2 H, d, J = 8.51 Hz), 7.23 - 7.29 (2 H, m).

### Reference Example 159

To a solution of ethyl 3-[1-(4-chlorobenzyl)-3-propoxy-1H-pyrazol-5-yl]propionate (1.49 g) in tetrahydrofuran (40 ml) was added at 0°C a 1.5M solution (10 ml) of diisobutylaluminum hydride in toluene, and the mixture was stirred at room temperature for 16 hr. A 1.5M solution (5 ml) of diisobutylaluminum hydride in toluene was added to the reaction mixture at room temperature, and the mixture was stirred for 1 hr. Saturated aqueous ammonium chloride (4.3 ml) was added to the reaction solution, and the mixture was stirred for 30 min. The resulting insoluble substance was removed by filtration. The filtrate was concentrated, and the obtained residue was subjected to silica gel column chromatography and eluted with ethyl acetate-hexane (1:25 to 2:1, v/v) to give 3-[1-(4-chlorobenzyl)-3-propoxy-1H-pyrazol-5-yl]propan-1-ol (1.22 g, yield 92%) as a colorless oil. ¹H-NMR (300 MHz, CDC1₃) δ:1.01 (3 H, t, J = 7.44 Hz), 1.69 - 1.86 (4 H, m), 2.50 - 2.59 (2 H, m), 3.64 (2 H, q, J = 6.03 Hz), 4.05 (2 H, t, J = 6.69 Hz), 5.11 (2 H, s), 5.53 (1 H, s), 6.96 - 7.08 (2 H, m), 7.21 - 7.30 (2 H, m).

### Reference Example 160

To a suspension of sodium hydride (156 mg, 60% in oil) in N,N-dimethylformamide (50 ml) was added ethyl 3-(3-propoxy-1H-pyrazol-5-yl)propionate (800 mg) at room temperature, and the mixture was stirred for 15 min. To the reaction mixture was added 2,4-difluorobenzyl bromide (0.499 ml), and the mixture was stirred at room temperature for 1 hr. Water was added, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:20 to 1:2, v/v) to give ethyl 3-[1-(2,4-difluorobenzyl)-3-propoxy-1H-pyrazol-5-yl]propanoate (0.74 g, yield 60%) as a colorless oil. ¹H-NMR (300 MHz, CDCl₃) δ:1.00 (3 H, t, J = 7.4 Hz), 1.24 (3 H, t, J = 7.1 Hz), 1.69 - 1.85 (2 H, m), 2.48 - 2.58 (2 H, m), 2.71 - 2.80 (2 H, m), 4.04 (2 H, t, J = 6.7 Hz), 4.11 (2 H, q, J = 7.1 Hz), 5.11 (2 H, s), 5.49 (1 H, d, J = 0.6 Hz), 7.00 (2 H, d, J = 8.5 Hz), 7.23 - 7.29 (2 H, m).

### Reference Example 161

To a solution of ethyl 3-[1-(2,4-difluorobenzyl)-3-propoxy-1H-pyrazol-5-yl]propanoate (529 mg) in tetrahydrofuran (15 ml) was added at 0°C a 1.5M solution (3.5 ml) of diisobutylaluminum hydride in toluene, and the mixture was stirred at room temperature for 2 hr. Saturated aqueous ammonium chloride (1.0 ml) was added to the reaction solution, and the mixture was stirred for 1 hr. The resulting insoluble substance was removed by filtration. The filtrate was concentrated, and the obtained residue was subjected to silica gel column chromatography and eluted with ethyl acetate-hexane (1:25 to 2:1, v/v) to give 3-[1-(2,4-difluorobenzyl)-3-propoxy-1H-pyrazol-5-yl]propan-1-ol (0.38 g, yield 81%) as colorless crystals. melting point 66 - 67°C.

### Reference Example 162

To a suspension of sodium hydride (156 mg, 60% in oil) in N,N-dimethylformamide (50 ml) was added ethyl 3-(3-propoxy-1H-pyrazol-5-yl)propionate (800 mg) at room temperature, and the mixture was stirred for 15 min. To the reaction mixture was added 2-methoxybenzyl chloride (0.542 ml), and the mixture was stirred at room temperature for 1 hr. Water was added, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:20 to 1:2, v/v) to give ethyl 3-[1-(2-methoxybenzyl)-3-propoxy-1H-pyrazol-5-yl]propanoate (0.61 g, yield 50%) as a colorless oil. ¹H-NMR (300 MHz, CDCl₃) δ:1.00 (3 H, t, J = 7.4 Hz), 1.24 (3 H, t, J = 7.2 Hz), 1.67 - 1.86 (2 H, m), 2.46 - 2.60 (2 H, m), 2.71 - 2.87 (2 H, m), 3.86 (3 H, s), 4.05 (2 H, t, J = 6.8 Hz), 4.12 (2 H, q, J = 7.2 Hz), 5.15 (2 H, s), 5.50 (1 H, s), 6.60 - 6.70 (1 H, m), 6.79 - 6.90 (2 H, m), 7.15 - 7.26 (1 H, m).

### Reference Example 163

To a solution of ethyl 3-[1-(2-methoxybenzyl)-3-propoxy-1H-pyrazol-5-yl]propanoate (0.52 g) in tetrahydrofuran (15 ml) was added at 0°C a 1.5M solution (3.5 ml) of diisobutylaluminum hydride in toluene, and the mixture was stirred at room temperature for 2 hr. Saturated aqueous ammonium chloride (1.0 ml) was added to the reaction solution, and the mixture was stirred for 1 hr. The resulting insoluble substance was removed by filtration. The filtrate was concentrated, and the obtained residue was subjected to silica gel column chromatography and eluted with ethyl acetate-hexane (1:25 to 2:1, v/v) to give 3-[1-(2-methoxybenzyl)-3-propoxy-1H-pyrazol-5-yl]propan-1-ol (0.42 g, yield 92%) as a colorless oil. ¹H-NMR (300 MHz, CDCl₃) δ: 1.00 (3 H, t, J = 7.4 Hz), 1.70 - 1.89 (4 H, m), 2.50 - 2.65 (2 H, m), 3.63 (2 H, q, J = 6.2 Hz), 3.86 (3 H, s), 4.06 (2 H, t, J = 6.7 Hz), 5.14 (2 H, s), 5.53 (1 H, s), 6.58 - 6.67 (1 H, m), 6.79 - 6.89 (2 H, m), 7.17 - 7.25 (1 H, m).

### Reference Example 164

To a 1N solution (100 ml) of borane in tetrahydrofuran was added 4-chloro-2-methoxybenzoic acid (9.33 g), and the mixture was stirred at room temperature for 16 hr. The reaction mixture was poured into 1N hydrochloric acid, and the mixture was extracted with ethyl acetate. The extract was washed with saturated brine, dried (MgSO₄) and concentrated to give a colorless oil.

To a mixture of the obtained oil, pyridine (0.687 ml), tetrahydrofuran (50 ml) and diethyl ether (200 ml) was added thionyl chloride (6.50 ml), and the mixture was stirred at room temperature for 16 hr. The reaction mixture was poured into 1N hydrochloric acid, and the mixture was extracted with ethyl acetate. The extract was washed with saturated aqueous sodium hydrogencarbonate, dried (MgSO₄) and concentrated. The residue was passed through a silica gel layer, and the eluate was concentrated. The obtained residue was recrystallized from cold hexane to give 4-chloro-2-methoxybenzyl chloride (7.05 g, yield 93%) as colorless crystals. melting point 43 - 45°C.

### Reference Example 165

To a suspension of sodium hydride (156 mg, 60% in oil) in N,N-dimethylformamide (25 ml) was added ethyl 3-(3-isopropoxy-1H-pyrazol-5-yl)propanoate (800 mg) at room temperature, and the mixture was stirred for 15 min. To the reaction mixture was added 4-chloro-2-methoxybenzyl chloride (743 mg), and the mixture was stirred at room temperature for 1 hr. Water was added, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:20 to 1:2, v/v) to give ethyl 3-[1-(4-chloro-2-methoxybenzyl)-3-isopropoxy-1H-pyrazol-5-yl]propanoate (0.39 g, yield 32%) as a colorless oil. ¹H-NMR (300 MHz, CDCl₃) δ:1.24 (3 H, t, J = 7.2 Hz), 1.32 (6 H, d, J = 6.0 Hz), 2.49 - 2.62 (2 H, m), 2.72 - 2.84 (2 H, m), 3.85 (3 H, s), 4.12 (2 H, q, J = 7.0 Hz), 4.62 - 4.75 (1 H, m), 5.08 (2 H, s), 5.48 (1 H, s), 6.55 - 6.63 (1 H, m), 6.76 - 6.90 (2 H, m).

### Reference Example 166

To a solution of ethyl 3-[1-(4-chloro-2-methoxybenzyl)-3-isopropoxy-1H-pyrazol-5-yl]propanoate (0.39 g) in tetrahydrofuran (15 ml) was added at 0°C a 1.5M solution (3.5 ml) of diisobutylaluminum hydride in toluene, and the mixture was stirred at room temperature for 2 hr. Saturated aqueous ammonium chloride (1.0 ml) was added to the reaction solution, and the mixture was stirred for 1 hr. The resulting insoluble substance was removed by filtration. The filtrate was concentrated, and the obtained residue was subjected to silica gel column chromatography and eluted with ethyl acetate-hexane (1:10 to 1:2, v/v) to give 3-[1-(4-chloro-2-methoxybenzyl)-3-isopropoxy-1H-pyrazol-5-yl]propan-1-ol (0.34 g, yield 95%) as a colorless oil. ¹H-NMR (300 MHz, CDCl₃) δ:1.33 (6 H, d, J = 6.0 Hz), 1.70 - 1.87 (2 H, m), 2.46 - 2.61 (2 H, m), 3.58 - 3.72 (2 H, m), 3.84 (3 H, s), 4.61 - 4.76 (1 H, m), 5.06 (2 H, s), 5.50 (1 H, s), 6.49 - 6.58 (1 H, m), 6.77 - 6.86 (2 H, m).

### Reference Example 167

A mixture of 1-hydroxy-2-naphtoaldehyde (10.19 g), benzyl bromide (7.73 ml) and potassium carbonate (8.98 g) in N,N-dimethylformamide (150 ml) was stirred at 80°C for 2 hr. Water was added to the reaction mixture, and the mixture was extracted with ethyl acetate. The ethyl acetate layer was washed with water and saturated brine and dried (MgSO₄). The ethyl acetate layer was passed through a silica gel column and concentrated. The obtained residue was washed with diethyl ether-hexane to give 1-(benzyloxy)-2-naphtoaldehyde as white crystals (21.45 g, yield 98%). melting point 62 - 63°C.

### Reference Example 168

To a solution of potassium tert-butoxide (13.20 g) in dimethoxyethane (75 ml) was added at -78°C a solution of p-toluenesulfonylmethyl isocyanide (11.48 g) in dimethoxyethane (75 ml), and the mixture was stirred for 30 min. A solution of 1-(benzyloxy)-2-naphtoaldehyde (7.44 g) in dimethoxyethane (75 ml) was added, and the mixture was stirred for 10 min. The temperature was gradually raised to room temperature, and methanol (100 ml) was added to the reaction mixture. The reaction mixture was stirred with heating under reflux for 6 hr and concentrated. Water was added, and the mixture was extracted with ethyl acetate. The organic layer was washed with brine, dried (MgSO₄) and concentrated. The residue was subjected to silica gel column chromatography and eluted with ethyl acetate-hexane (1:10 to 1:4, v/v) to give [1-(benzyloxy)-2-naphthyl]acetonitrile as a colorless oil (4.81 g, yield 62%). ¹H-NMR (300 MHz, CDCl₃) δ:3.73 (2 H, s), 5.12 (2 H, s), 7.38 - 7.51 (6 H, m), 7.51 - 7.61 (2 H, m), 7.70 (1 H, d, J = 8.3 Hz), 7.83 - 7.94 (1 H, m), 8.08 - 8.18 (1 H, m).

### Reference Example 169

A mixture of [1-(benzyloxy)-2-naphthyl]acetonitrile (4.81 g), 4N aqueous sodium hydroxide (18 ml) and ethanol (75 ml) was stirred with heating under reflux for 12 hr. To the reaction mixture was added 5N hydrochloric acid (14.4 ml), and the mixture was extracted with ethyl acetate. The organic layer was dried (MgSO₄) and concentrated to give a yellow oil. A mixture of the obtained oil, methyl iodide (0.89 ml) and potassium carbonate (2.49 g) in N,N-dimethylformamide (50 ml) was stirred at 80°C for 2 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:10 to 1:3, v/v) to give methyl [1-(benzyloxy)-2-naphthyl]acetate as a pale-yellow oil (21.45 g, yield 98%). ¹H-NMR (300 MHz, CDCl₃) δ:3.68 (3 H, s), 3.84 (2 H, s), 5.06 (2 H, s), 7.34 - 7.57 (8 H, m), 7.63 (1 H, d, J = 8.5 Hz), 7.80 - 7.89 (1 H, m), 8.07 - 8.16 (1H, m).

### Reference Example 170

A mixture of methyl [1-(benzyloxy)-2-naphthyl]acetate (3.93 g), 10% palladium-carbon (700 mg) and ethanol (75 ml) was stirred at room temperature for 4 hr under a hydrogen atmosphere. The catalyst was filtered off, and the filtrate was applied to silica gel column chromatography. The obtained solution was concentrated to give methyl (1-hydroxy-2-naphthyl)acetate as a pale-orange oil (2.35 g, yield 85%). ¹H-NMR (300 MHz, CDCl₃) δ:3.78 (3 H, s), 3.84 (2 H, s), 7.17 (1 H, d, J = 8.3 Hz), 7.39 (1 H, d, J = 8.3 Hz), 7.43 - 7.55 (2 H, m), 7.69 - 7.84 (1 H, m), 8.26 - 8.37 (1 H, m), 8.43 (1 H, s).

### Reference Example 171

A mixture of [1-benzyl-3-(benzyloxy)-1H-pyrazol-4-yl]acetonitrile (12.47 g), 4N aqueous sodium hydroxide (100 ml) and ethanol (150 ml) was stirred with heating under reflux for 16 hr. To the reaction mixture was added 5N hydrochloric acid (80 ml), and the mixture was extracted with ethyl acetate. The organic layer was dried (MgSO₄) and passed through a silica gel layer. The organic layer was concentrated to give a pale-yellow oil (13.25 g). A mixture of the obtained oil, methyl iodide (6.39 g), potassium carbonate (6.22 g) and N,N-dimethylformamide (120 ml) was stirred at 80°C for 2 hr. The reaction mixture was poured into water, and the mixture was extracted with ethyl acetate. The organic layer was passed through a silica gel layer, and the organic layer was concentrated to give methyl [1-benzyl-3-(benzyloxy)-1H-pyrazol-4-yl]acetate (13.50 g, yield 98%) as a pale-yellow oil. ¹H-NMR (300 MHz, CDCl₃) δ:3.40 (2 H, s), 3.67 (3 H, s), 5.12 (2 H, s), 5.24 (2 H, m), 7.15 - 7.23 (3 H, m), 7.27 - 7.39 (6 H, m), 7.40 - 7.47 (2 H, m).

### Reference Example 172

A mixture of methyl [1-benzyl-3-(benzyloxy)-1H-pyrazol-4-yl]acetate (13.50 g), 10% palladium-carbon (1.35 g) and ethanol (150 ml) was stirred at room temperature for 3 hr under a hydrogen atmosphere. The catalyst was filtered off, and the filtrate was concentrated. The obtained solid was recrystallized from ethyl acetate-hexane to give methyl (1-benzyl-3-hydroxy-1H-pyrazol-4-yl)acetate as colorless crystals (7.35 g, yield 74%). melting point 118 - 119°C.

### Reference Example 173

A mixture of methyl (1-benzyl-3-{3-[1-(2,4-dichlorobenzyl)-3-(methoxymethoxy)-1H-pyrazol-5-yl]propoxy}-1H-pyrazol-4-yl)acetate (1.25 g), concentrated hydrochloric acid (3 drops) and methanol (30 ml) was heated under reflux for 14 hr. After cooling, the reaction solution was concentrated. The obtained residue was subjected to silica gel column chromatography and eluted with ethyl acetate-hexane (1:4 to 1:1, v/v). The eluate was concentrated, and the residue was crystallized from ethyl acetate-hexane to give methyl (1-benzyl-3-{3-[1-(2,4-dichlorobenzyl)-3-hydroxy-1H-pyrazol-5-yl]propoxy}-1H-pyrazol-4-yl)acetate (634 mg, yield 55%) as colorless crystals. melting point 105.5 - 107.0°C.

### Reference Example 174

To a solution of methyl 3-(2-ethoxy-4-hydroxyphenyl)propanoate (5.2 g) in N,N-dimethylformamide (50 ml) was added sodium hydride (1.1 g, 60% in oil) at room temperature, and the mixture was stirred for 15 min. To this reaction mixture was added 2-chloro-5-(trifluoromethyl)pyridine (3.0 ml), and the mixture was stirred for 1 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:5, v/v) to give methyl 3-(2-ethoxy-4-{[5-(trifluoromethyl)pyridin-2-yl]oxy}phenyl)propanoate as a colorless oil (5.6 g, yield 65%). ¹H-NMR (300 MHz, CDCl₃) δ:1.41 (3 H, t, J = 7.0 Hz), 2.64 (2 H, t, J = 7.7 Hz), 2.95 (2 H, t, J = 7.7 Hz), 3.68 (3 H, s), 4.00 (2 H, q, J = 7.0 Hz), 6.59 - 6.71 (2 H, m), 6.98 (1 H, d, J = 8.7 Hz), 7.18 (1 H, d, J = 7.7 Hz), 7.88 (1 H, dd, J = 8.7, 2.5 Hz), 8.46 (1 H, s).

### Reference Example 175

To a solution (100 ml) of methyl 3-(2-ethoxy-4-{[5-(trifluoromethyl)pyridin-2-yl]oxy}phenyl)propanoate (5.5 g) in tetrahydrofuran was added lithium aluminum hydride (0.57 g) at 0°C, and the mixture was stirred for 30 min. To this reaction mixture was added sodium sulfate decahydrate (4.8 g), and the mixture was filtered through celite and concentrated. The residue was subjected to silica gel column chromatography and eluted with ethyl acetate-hexane (1:4, v/v) to give 3-(2-ethoxy-4-{[5-(trifluoromethyl)pyridin-2-yl]oxy}phenyl)propan-1-ol (4.6 g, yield 90%) as crystals. Recrystallization from ethyl acetate-hexane gave colorless needle crystals. melting point 50 - 51°C.

### Reference Example 176

To a solution (30 ml) of methyl 3-(4-hydroxy-2-isopropoxyphenyl)propanoate (1.02 g) in tetrahydrofuran was added sodium hydride (0.21 g, 60% in oil) at room temperature, and the mixture was stirred for 30 min. To this reaction mixture was added 2-chloro-5-(trifluoromethyl)pyridine (0.78 g), and the mixture was heated under reflux for 3 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:10, v/v) to give methyl 3-(2-isopropoxy-4-{[5-(trifluoromethyl)pyridin-2-yl]oxy}phenyl)propanoate as a colorless oil (0.52 g, yield 31%). ¹H-NMR (300 MHz, CDCl₃) δ:1.34 (6 H, d, J = 6.0 Hz), 2.63 (2 H, t, J = 7.8 Hz), 2.92 (2 H, t, J = 7.8 Hz), 3.68 (3 H, s), 4.41 - 4.57 (1 H, m), 6.56 - 6.68 (2 H, m), 6.98 (1 H, d, J = 8.7 Hz), 7.13 - 7.22 (1 H, m), 7.88 (1 H, dd, J = 8.7, 2.5 Hz), 8.41 - 8.50 (1 H, m).

### Reference Example 177

To a solution (30 ml) of methyl 3-(2-isopropoxy-4-{[5-(trifluoromethyl)pyridin-2-yl]oxy}phenyl)propanoate (492 mg) in tetrahydrofuran was added lithium aluminum hydride (49 mg) at room temperature, and the mixture was stirred for 15 min. To this reaction mixture was added sodium sulfate decahydrate (419 mg), and the mixture was filtered through celite and concentrated. The residue was subjected to silica gel column chromatography and eluted with ethyl acetate-hexane (1:3, v/v) to give 3-(2-isopropoxy-4-{[5-(trifluoromethyl)pyridin-2-yl]oxy}phenyl)propan-1-ol as a colorless oil (433 mg, yield 95%). ¹H-NMR (300 MHz, CDCl₃) δ:1.35 (6 H, d, J = 6.0 Hz), 1.78 - 1.92 (3 H, m), 2.72 (2 H, t, J = 7.3 Hz), 3.62 (2 H, q, J = 6.2 Hz), 4.44 - 4.59 (1 H, m), 6.61 - 6.72 (2 H, m), 6.98 (1 H, d, J = 8.7 Hz), 7.13 - 7.22 (1 H, m), 7.88 (1 H, dd, J = 8.7, 2.5 Hz), 8.42 - 8.50 (1 H, m).

### Reference Example 178

To a solution (100 ml) of ethyl (2E)-3-{2-(benzyloxy)-4-{[5-(trifluoromethyl)pyridin-2-yl]oxy}phenyl}acrylate (4.65 g) in a mixed solvent of tetrahydrofuran-ethanol (1:1, v/v) was added 10% palladium-carbon, and the mixture was stirred under a hydrogen atmosphere for 2 hr. The reaction mixture was filtered and concentrated. The residue was subjected to silica gel column chromatography and eluted with ethyl acetate-hexane (1:5, v/v) to give ethyl 3-(2-hydroxy-4-{[5-(trifluoromethyl)pyridin-2-yl]oxy}phenyl)propanoate as a pale-yellow oil (3.49 g, yield 94%). ¹H-NMR (300 MHz, CDCl₃) δ:1.26 (3 H, t, J = 7.2 Hz), 2.65 - 2.76 (2 H, m), 2.83 - 2.95 (2 H, m), 4.16 (2 H, q, J = 7.2 Hz), 6.60 - 6.69 (2 H, m), 6.99 (1 H, d, J = 8.8 Hz), 7.11 (1 H, d, J = 8.1 Hz), 7.84 - 7.92 (2 H, m), 8.41 - 8.49 (1 H, m).

### Reference Example 179

To a solution of ethyl 3-(2-hydroxy-4-{[5-(trifluoromethyl)pyridin-2-yl]oxy}phenyl)propanoate (1.0 g) in N,N-dimethylformamide (30 ml) were added 1-iodopropane (0.35 ml) and potassium carbonate (0.43 g), and the mixture was stirred at 80°C for 2 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:5, v/v) to give ethyl 3-(2-propoxy-4-{[5-(trifluoromethyl)pyridin-2-yl]oxy}phenyl)propanoate as a yellow oil (1.1 g, yield 97%). ¹H-NMR (300 MHz, CDC1₃) δ:1.05 (3 H, t, J = 7.4 Hz), 1.25 (3 H, t, J = 7.2 Hz), 1.75 - 1.90 (2 H, m), 2.62 (2 H, t, J = 7.7 Hz), 2.95 (2 H, t, J = 7.7 Hz), 3.89 (2 H, t, J = 6.4 Hz), 4.14 (2 H, q, J = 7.2 Hz), 6.59 - 6.69 (2 H, m), 6.98 (1 H, d, J = 8.7 Hz), 7.18 (1 H, d, J = 8.5 Hz), 7.88 (1 H, dd, J = 8.7, 2.1 Hz), 8.43 - 8.49 (1 H, m).

### Reference Example 180

To a solution (30 ml) of ethyl 3-(2-propoxy-4-{[5-(trifluoromethyl)pyridin-2-yl]oxy}phenyl)propanoate (1.0 g) in tetrahydrofuran was added lithium aluminum hydride (95 mg) at room temperature, and the mixture was stirred for 10 min. To this reaction mixture was added sodium sulfate decahydrate (806 mg), and the mixture was filtered through celite and concentrated. The residue was subjected to silica gel column chromatography and eluted with ethyl acetate-hexane (1:4, v/v) to give 3-(2-propoxy-4-{[5-(trifluoromethyl)pyridin-2-yl]oxy}phenyl)propan-1-ol as a colorless oil (894 mg, yield 100%). ¹H-NMR (300 MHz, CDCl₃) δ:1.04 (3 H, t, J = 7.4 Hz), 1.70 - 1.95 (5 H, m), 2.74 (2 H, t, J = 7.4 Hz), 3.64 (2 H, t, J = 6.3 Hz), 3.90 (2 H, t, J = 6.4 Hz), 6.62 - 6.72 (2 H, m), 6.98 (1 H, d, J = 8.7 Hz), 7.17 (1 H, d, J = 7.9 Hz), 7.88 (1 H, dd, J = 8.7, 2.5 Hz), 8.43 - 8.50 (1 H, m).

### Reference Example 181

To a solution (30 ml) of ethyl 3-(2-hydroxy-4-{[5-(trifluoromethyl)pyridin-2-yl]oxy}phenyl)propanoate (1.0 g) in N,N-dimethylformamide were added 1-iodobutane (0.4 ml) and potassium carbonate (0.43 g), and the mixture was stirred at 80°C for 2 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:5, v/v) to give ethyl 3-(2-butoxy-4-{[5-(trifluoromethyl)pyridin-2-yl]oxy}phenyl)propanoate as a yellow oil (1.1 g, yield 95%). ¹H-NMR (300 MHz, CDCl₃) δ:0.97 (3 H, t, J = 7.4 Hz), 1.25 (3 H, t, J = 7.2 Hz), 1.42 - 1.58 (2 H, m), 1.71 - 1.86 (2 H, m), 2.56 - 2.67 (2 H, m), 2.94 (2 H, t, J = 7.7 Hz), 3.93 (2 H, t, J = 6.4 Hz), 4.14 (2 H, q, J = 7.2 Hz), 6.61 - 6.69 (2 H, m), 6.98 (1 H, d, J = 8.7 Hz), 7.14 - 7.22 (1 H, m), 7.88 (1 H, dd, J = 8.6, 2.4 Hz), 8.42 - 8.50 (1 H, m).

### Reference Example 182

To a solution (30 ml) of ethyl 3-(2-butoxy-4-{[5-(trifluoromethyl)pyridin-2-yl]oxy}phenyl)propanoate (1.0 g) in tetrahydrofuran was added lithium aluminum hydride (92 mg) at room temperature, and the mixture was stirred for 10 min. To this reaction mixture was added sodium sulfate decahydrate (783 mg), and the mixture was filtered through celite and concentrated. The residue was subjected to silica gel column chromatography and eluted with ethyl acetate-hexane (1:4, v/v) to give 3-(2-butoxy-4-{[5-(trifluoromethyl)pyridin-2-yl]oxy}phenyl)propan-1-ol as a colorless oil (845 mg, yield 94%). ¹H-NMR (300 MHz, CDCl₃) δ:0.97 (3 H, t, J = 7.4 Hz), 1.41 - 1.55 (2 H, m), 1.68 (1 H, t, J = 5.9 Hz), 1.72 - 1.94 (4 H, m), 2.73 (2 H, t, J = 7.4 Hz), 3.64 (2 H, t, J = 6.1 Hz), 3.94 (2 H, t, J = 6.5 Hz), 6.61 - 6.72 (2 H, m), 6.98 (1 H, d, J = 8.7 Hz), 7.17 (1 H, d, J = 7.7 Hz), 7.88 (1 H, dd, J = 8.7, 2.5 Hz), 8.42 - 8.50 (1 H, m).

### Reference Example 183

To a solution (30 ml) of ethyl 3-(2-hydroxy-4-{[5-(trifluoromethyl)pyridin-2-yl]oxy}phenyl)propanoate (1.1 g) in N,N-dimethylformamide were added 1-iodo-2-methylpropane (1.0 ml) and potassium carbonate (0.6 g), and the mixture was stirred at 90°C for 3 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:10, v/v) to give ethyl 3-(2-isobutoxy-4-{[5-(trifluoromethyl)pyridin-2-yl]oxy}phenyl)propanoate as a yellow oil (1.1 g, yield 87%). ¹H-NMR (300 MHz, CDCl₃) δ:1.04 (6 H, d, J = 6.8 Hz), 1.25 (3 H, t, J = 7.2 Hz), 2.01 - 2.21 (1 H, m), 2.63 (2 H, t, J = 7.8 Hz), 2.96 (2 H, t, J = 7.8 Hz), 3.69 (2 H, t, J = 6.4 Hz), 4.14 (2 H, q, J = 7.2 Hz), 6.59 - 6.68 (2 H, m), 6.98 (1 H, d, J = 8.7 Hz), 7.19 (1 H, d, J = 7.5 Hz), 7.88 (1 H, dd, J = 8.7, 2.5 Hz), 8.41 - 8.50 (1 H, m).

### Reference Example 184

To a solution (50 ml) of ethyl 3-(2-isobutoxy-4-{[5-(trifluoromethyl)pyridin-2-yl]oxy}phenyl)propanoate (1.0 g) in tetrahydrofuran was added lithium aluminum hydride (135 mg) at room temperature, and the mixture was stirred for 30 min. To this reaction mixture was added sodium sulfate decahydrate (1.2 g), and the mixture was filtered through celite and concentrated. The residue was subjected to silica gel column chromatography and eluted with ethyl acetate-hexane (1:5, v/v) to give 3-(2-isobutoxy-4-{[5-(trifluoromethyl)pyridin-2-yl]oxy}phenyl)propan-1-ol as a colorless oil (892 mg, yield 99%). ¹H-NMR (300 MHz, CDCl₃) δ:1.04 (6 H, d, J = 6.6 Hz), 1. 56 - 1.63 (1 H, m), 1.83 - 1.95 (2 H, m), 2.02 - 2.20 (1 H, m), 2.74 (2 H, t, J = 7.4 Hz), 3.60 - 3.75 (4 H, m), 6.61 - 6.70 (2 H, m), 6.98 (1 H, d, J = 8.7 Hz), 7.18 (1 H, d, J = 8.1 Hz), 7.88 (1 H, dd, J = 8.7, 2.5 Hz), 8.43 - 8.50 (1 H, m).

### Reference Example 185

To a solution (100 ml) of 2-hydroxy-4-(methoxymethoxy)benzaldehyde (4.4 g) in N,N-dimethylformamide was added sodium hydride (1.2 g, 60% in oil) at room temperature, and the mixture was stirred for 30 min. To this reaction mixture was added 2-chloro-5-(trifluoromethyl)pyridine (4.0 ml), and the mixture was stirred at room temperature for 17 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:10, v/v) to give 4-(methoxymethoxy)-2-{[5-(trifluoromethyl)pyridin-2-yl]oxy}benzaldehyde as a colorless oil (5.6 g, yield 71%). ¹H-NMR (300 MHz, CDCl₃) δ:3.49 (3 H, s), 5.22 (2 H, s), 6.84 (1 H, d, J = 2.3 Hz), 7.02 - 7.08 (1 H, m), 7.16 (1 H, d, J = 8.5 Hz), 7.88 - 8.02 (2 H, m), 8.36 - 8.45 (1 H, m), 10.03 (1 H, s).

### Reference Example 186

To a solution (100 ml) of ethyl diethylphosphonoacetate (4.4 ml) in tetrahydrofuran was added sodium hydride (1.0 g, 60% in oil) at room temperature, and the mixture was stirred for 30 min. To this reaction mixture was added a solution (20 ml) of 4-(methoxymethoxy)-2-{[5-(trifluoromethyl)pyridin-2-yl]oxy}benzaldehyde (5.6 g) in tetrahydrofuran, and the mixture was stirred for 30 min. Saturated aqueous ammonium chloride was poured into the reaction mixture, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:10, v/v) to give ethyl (2E)-3-(4-(methoxymethoxy)-2-{[5-(trifluoromethyl)pyridin-2-yl]oxy}phenyl)acrylate as a pale-yellow oil (6.3 g, yield 93%). Recrystallization from ethyl acetate-hexane gave colorless prism crystals. melting point 69 - 70°C.

### Reference Example 187

To a solution (200 ml) of ethyl (2E)-3-(4-(methoxymethoxy)-2-{[5-(trifluoromethyl)pyridin-2-yl]oxy}phenyl)acrylate (12.2 g) in a mixed solvent of tetrahydrofuran-ethanol (1:1, v/v) was added 10% palladium-carbon (1.00 g), and the mixture was stirred under a hydrogen atmosphere for 3 hr. The reaction mixture was filtered, and the filtrate was concentrated. The residue was subjected to silica gel column chromatography and eluted with ethyl acetate-hexane (1:8, v/v) to give ethyl 3-(4-(methoxymethoxy)-2-{[5-(trifluoromethyl)pyridin-2-yl]oxy}phenyl)propanoate as a colorless oil (10.5 g, yield 86%). ¹H-NMR (300 MHz, CDC1₃) δ:1.21 (3 H, t, J = 7.2 Hz), 2.54 (2 H, t, J = 7.8 Hz), 2.79 (2 H, t, J = 7.8 Hz), 3.46 (3 H, s), 4.08 (2 H, q, J = 7.2 Hz), 5.14 (2 H, s), 6.73 - 6.81 (1 H, m), 6.88 - 7.14 (2 H, m), 7.23 (1 H, d, J = 8.5 Hz), 7.90 (1 H, dd, J = 8.7, 2.5 Hz), 8.40 - 8.48 (1 H, m).

### Reference Example 188

To a solution (30 ml) of ethyl 3-(4-(methoxymethoxy)-2-{[5-(trifluoromethyl)pyridin-2-yl]oxy}phenyl)propanoate (1.2 g) in tetrahydrofuran was added lithium aluminum hydride (114 mg) at room temperature, and the mixture was stirred for 15 min. To this reaction mixture was added sodium sulfate decahydrate (967 mg), and the mixture was filtered through celite and concentrated. The residue was subjected to silica gel column chromatography and eluted with ethyl acetate-hexane (1:2, v/v) to give 3-(4-(methoxymethoxy)-2-{[5-(trifluoromethyl)pyridin-2-yl]oxy}phenyl)propan-1-ol as a colorless oil (950 mg, yield 89%). ¹H-NMR (300 MHz, CDCl₃) δ:1.48 (1 H, t, J = 5.6 Hz), 1.73 - 1.89 (2 H, m), 2.56 (2 H, t, J = 7.4 Hz), 3.47 (3 H, s), 3.54 - 3.64 (2 H, m), 5.14 (2 H, s), 6.72 - 6.81 (1 H, m), 6.88 - 7.05 (2 H, m), 7.20 - 7.28 (1 H, m), 7.90 (1 H, dd, J = 8.8, 1.9 Hz), 8.44 (1 H, s).

### Reference Example 189

To a solution (100 ml) of ethyl 3-(4-(methoxymethoxy)-2-{[5-(trifluoromethyl)pyridin-2-yl]oxy}phenyl)propanoate (9.1 g) in tetrahydrofuran was added concentrated hydrochloric acid (4.2 ml), and the mixture was stirred at 60°C for 3 hr. To the reaction mixture was added a 1N aqueous sodium hydroxide solution (50 ml), and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:4, v/v) to give ethyl 3-(4-hydroxy-2-{[5-(trifluoromethyl)pyridin-2-yl]oxy}phenyl)propanoate as a colorless oil (4.8 g, yield 59%). ¹H-NMR (300 MHz, CDCl₃) δ:1.20 (3 H, t, J = 7.2 Hz), 2.53 (2 H, t, J = 7.7 Hz), 2.74 (2 H, t, J = 7.7 Hz), 4.08 (2 H, q, J = 7.2 Hz), 6.47 (1 H, d, J = 2.5 Hz), 6.55 - 6.80 (2 H, m), 6.96 - 7.17 (2 H, m), 7.91 (1 H, dd, J = 8.8, 2.5 Hz), 8.37 - 8.48 (1 H, m).

### Reference Example 190

To a solution (20 ml) of ethyl 3-(4-hydroxy-2-{[5-(trifluoromethyl)pyridin-2-yl]oxy}phenyl)propanoate (1.1 g) in N,N-dimethylformamide were added 2-iodopropane (0.5 ml) and potassium carbonate (0.54 g), and the mixture was stirred at 80°C for 2 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:5, v/v) to give ethyl 3-(4-isopropoxy-2-{[5-(trifluoromethyl)pyridin-2-yl]oxy}phenyl)propanoate as a colorless oil (1.0 g, yield 81%). ¹H-NMR (300 MHz, CDCl₃) δ:1.20 (3 H, t, J = 7.2 Hz), 1.31 (6 H, d, J = 6.0 Hz), 2.54 (2 H, t, J = 7.9 Hz), 2.78 (2 H, t, J = 7.9 Hz), 4.08 (2 H, q, J = 7.2 Hz), 4.39 - 4.54 (1 H, m), 6.58 (1 H, d, J = 2.6 Hz), 6.75 (1 H, dd, J = 8.5, 2.6 Hz), 6.99 (1 H, d, J = 8.7 Hz), 7.20 (1 H, d, J = 8.5 Hz), 7.89 (1 H, dd, J = 8.7, 2.5 Hz), 8.42 - 8.47 (1 H, m).

### Reference Example 191

To a solution (30 ml) of ethyl 3-(4-isopropoxy-2-{[5-(trifluoromethyl)pyridin-2-yl]oxy}phenyl)propanoate (894 mg) in tetrahydrofuran was added lithium aluminum hydride (85 mg) at room temperature, and the mixture was stirred for 30 min. To this reaction mixture was added sodium sulfate decahydrate (725 mg), and the mixture was filtered through celite and concentrated. The residue was subjected to silica gel column chromatography and eluted with ethyl acetate-hexane (1:4, v/v) to give 3-(4-isopropoxy-2-{[5-(trifluoromethyl)pyridin-2-yl]oxy}phenyl)propan-1-ol as a colorless oil (731 mg, yield 91%). ¹H-NMR (300 MHz, CDCl₃) δ:1.32 (6 H, d, J = 6.0 Hz), 1.52 (1 H, t, J = 5.3 Hz), 1.75 - 1.88 (2 H, m), 2.50 - 2.60 (2 H, m), 3.55 - 3.65 (2 H, m), 4.39 - 4.54 (1 H, m), 6.57 (1 H, d, J = 2.5 Hz), 6.77 (1 H, dd, J = 8.5, 2.5 Hz), 6.97 (1 H, d, J = 8.7 Hz), 7.21 (1 H, d, J = 8.5 Hz), 7. 89 (1 H, dd, J = 8.7, 2.3 Hz), 8.41 - 8.49 (1 H, m).

### Reference Example 192

To a solution (30 ml) of ethyl 3-(4-hydroxy-2-{[5-(trifluoromethyl)pyridin-2-yl]oxy}phenyl)propanoate (1.1 g) in N,N-dimethylformamide were added 1-iodobutane (0.6 ml) and potassium carbonate (0.54 g), and the mixture was stirred at 80°C for 2 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:5, v/v) to give ethyl 3-(4-butoxy-2-{[5-(trifluoromethyl)pyridin-2-yl]oxy}phenyl)propanoate as a colorless oil (833 mg, yield 67%). ¹H-NMR (300 MHz, CDCl₃) δ:0.95 (3 H, t, J = 7.4 Hz), 1.20 (3 H, t, J = 7.1 Hz), 1.38 - 1.55 (2 H, m), 1.65 - 1.83 (2 H, m), 2.48 - 2.62 (2 H, m), 2.78 (2 H, t, J = 7.8 Hz), 3.91 (2 H, t, J = 6.5 Hz), 4.08 (2 H, q, J = 7.1 Hz), 6.59 (1 H, d, J = 2.5 Hz), 6.76 (1 H, dd, J = 8.5, 2.6 Hz), 7.00 (1 H, d, J = 8.7 Hz), 7.21 (1 H, d, J = 8.5 Hz), 7.90 (1 H, dd, J = 8.7, 2.5 Hz), 8.40 - 8.50 (1 H, m).

### Reference Example 193

To a solution (30 ml) of ethyl 3-(4-butoxy-2-{[5-(trifluoromethyl)pyridin-2-yl]oxy}phenyl)propanoate (770 mg) in tetrahydrofuran was added lithium aluminum hydride (71 mg) at room temperature, and the mixture was stirred for 30 min. To this reaction mixture was added sodium sulfate decahydrate (603 mg), and the mixture was filtered through celite and concentrated. The residue was subjected to silica gel column chromatography and eluted with ethyl acetate-hexane (1:3, v/v) to give 3-(4-butoxy-2-{[5-(trifluoromethyl)pyridin-2-yl]oxy}phenyl)propan-1-ol as a colorless oil (600 mg, yield 87%). ¹H-NMR (300 MHz, CDCl₃) δ:0.96 (3 H, t, J = 7.4 Hz), 1.38 - 1.57 (3 H, m), 1.67 - 1.88 (4 H, m), 2.55 (2 H, t, J = 7.4 Hz), 3.51 - 3.70 (2 H, m), 3.91 (2 H, t, J = 6.5 Hz), 6.59 (1 H, d, J = 2. 6 Hz), 6.78 (1 H, dd, J = 8.5, 2.6 Hz), 6.98 (1 H, d, J = 8.7 Hz), 7.21 (1 H, d, J = 8.5 Hz), 7.89 (1 H, dd, J = 8.7, 2.5 Hz), 8.41 - 8.49 (1 H, m).

### Reference Example 194

To a solution (100 ml) of 2-hydroxy-4-(methoxymethoxy)benzaldehyde (11.9 g) in N,N-dimethylformamide were added benzyl bromide (8.5 ml) and potassium carbonate (10.8 g) at room temperature, and the mixture was stirred at 80°C for 1 hr. Water was poured into the reaction mixture, and the mixture was extracted with ethyl acetate. The ethyl acetate layer was washed with water and saturated brine, dried (MgSO₄) and concentrated to give an oil. To a solution (150 ml) of ethyl diethylphosphonoacetate (15.0 ml) in tetrahydrofuran was added sodium hydride (3.3 g, 60% in oil) at room temperature, and the mixture was stirred for 30 min. To this reaction mixture was added dropwise to a solution (50 ml) of the oil obtained earlier in tetrahydrofuran, and the mixture was stirred at room temperature for 1 hr. Saturated aqueous ammonium chloride was poured into the reaction mixture, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:5, v/v) to give ethyl (2E)-3-[2-(benzyloxy)-4-(methoxymethoxy)phenyl]acrylate as a yellow oil (21.6 g, yield 96%). ¹H-NMR (300 MHz, CDCl₃) δ:1.32 (3 H, t, J = 7.2 Hz), 3.46 (3 H, s), 4.23 (2 H, q, J = 7.2 Hz), 5.14 (2 H, s), 5.16 (2 H, s), 6.44 (1 H, d, J = 16.2 Hz), 6.65 - 6.69 (2 H, m), 7.28 - 7.49 (6 H, m), 8.00 (1 H, d, J = 16.2 Hz).

### Reference Example 195

To a solution (100 ml) of ethyl (2E)-3-[2-(benzyloxy)-4-(methoxymethoxy)phenyl]acrylate (4.0 g) in a mixed solvent of tetrahydrofuran-ethanol (1:1, v/v) was added 10% palladium-carbon (0.5 g), and the mixture was stirred under a hydrogen atmosphere for 3 hr. The reaction mixture was filtered, and the filtrate was concentrated. The residue was subjected to silica gel column chromatography and eluted with ethyl acetate-hexane (1:5, v/v) to give ethyl 3-[2-hydroxy-4-(methoxymethoxy)phenyl]propanoate as a colorless oil (2.8 g, yield 95%). ¹H-NMR (300 MHz, CDC1₃) δ:1.24 (3 H, t, J = 7.2 Hz), 2.62 - 2.71 (2 H, m), 2.78 - 2.89 (2 H, m), 3.46 (3 H, s), 4.15 (2 H, q, J = 7.2 Hz), 5.12 (2 H, s), 6.52 - 6.64 (2 H, m), 6.97 (1 H, d, J = 8.3 Hz), 7.42 (1 H, s).

### Reference Example 196

To a solution (30 ml) of ethyl 3-[2-hydroxy-4-(methoxymethoxy)phenyl]propanoate (1.27 g) in N,N-dimethylformamide was added sodium hydride (240 mg, 60% in oil) at room temperature, and the mixture was stirred for 30 min. To this reaction mixture was added 2,3-dichloro-5-(trifluoromethyl)pyridine (0.80 ml), and the mixture was stirred at room temperature for 3 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:10, v/v) to give ethyl 3-[2-{[3-chloro-5-(trifluoromethyl)pyridin-2-yl]oxy}-4-(methoxymethoxy)phenyl]propanoate as a colorless oil (1.60 g, yield 74%). ¹H-NMR (300 MHz, CDCl₃) δ:1.21 (3 H, t, J = 7.2 Hz), 2.52 - 2.64 (2 H, m), 2.71 - 2.83 (2 H, m), 3.47 (3 H, s), 4.09 (2 H, q, J = 7.2 Hz), 5.15 (2 H, s), 6.82 (1 H, d, J = 2.6 Hz), 6.93 (1 H, dd, J = 8.5, 2.5 Hz), 7.24 (1 H, d, J = 8.5 Hz), 7.97 - 8.00 (1 H, m), 8.22 - 8.30 (1 H, m).

### Reference Example 197

To a solution (50 ml) of ethyl 3-[2-{[3-chloro-5-(trifluoromethyl)pyridin-2-yl]oxy}-4-(methoxymethoxy)phenyl]propanoate (1.4 g) in tetrahydrofuran was added lithium aluminum hydride (130 mg) at room temperature, and the mixture was stirred for 15 min. To this reaction mixture was added sodium sulfate decahydrate (1.1 g), and the mixture was filtered through celite and concentrated. The residue was subjected to silica gel column chromatography and eluted with ethyl acetate-hexane (1:4, v/v) to give 3-[2-{[3-chloro-5-(trifluoromethyl)pyridin-2-yl]oxy}-4-(methoxymethoxy)phenyl]propan-1-ol as a pale-yellow oil (1.1 g, yield 90%). Recrystallization from ethyl acetate-hexane gave pale-yellow prism crystals. melting point 92.5°C - 93.5°C.

### Reference Example 198

To a solution (100 ml) of ethyl (2E)-3-[2-(benzyloxy)-4-(methoxymethoxy)phenyl]acrylate (7.7 g) in tetrahydrofuran was added concentrated hydrochloric acid (2.7 ml), and the mixture was stirred at 60°C for 3 hr. To the reaction mixture was added a 1N aqueous sodium hydroxide solution (33 ml), and the mixture was extracted with ethyl acetate. The ethyl acetate layer was washed with saturated brine, dried (MgSO₄) and concentrated. The residue was recrystallized from ethyl acetate-hexane to give ethyl (2E)-3-[2-(benzyloxy)-4-hydroxyphenyl]acrylate as colorless prism crystals (5.1 g, yield 78%). melting point 131 - 133°C.

### Reference Example 199

To a solution (40 ml) of ethyl (2E)-3-[2-(benzyloxy)-4-hydroxyphenyl]acrylate (3.0 g) in N,N-dimethylformamide were added 1-iodopropane (1.4 ml) and potassium carbonate (1.9 g), and the mixture was stirred at 60°C for 1 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:9, v/v) to give ethyl (2E)-3-[2-(benzyloxy)-4-propoxyphenyl]acrylate as a colorless oil (3.0 g, yield 88%). ¹H-NMR (300 MHz, CDCl₃) δ:1.02 (3 H, t, J = 7.4 Hz), 1.31 (3 H, t, J = 7.2 Hz), 1.69-1.87 (2 H, m), 3.90 (2 H, t, J = 6.6 Hz), 4.23 (2 H, q, J = 7.2 Hz), 5.14 (2 H, s), 6.42 (1 H, d, J = 16.2 Hz), 6.46-6.56 (2 H, m), 7.27-7.52 (6 H, m), 8.00 (1 H, d, J = 16.2 Hz).

### Reference Example 200

To a solution (100 ml) of ethyl (2E)-3-[2-(benzyloxy)-4-propoxyphenyl]acrylate (2.9 g) in a mixed solvent of tetrahydrofuran-ethanol (1:1, v/v) was added 10% palladium-carbon (0.5 g), and the mixture was stirred under a hydrogen atmosphere for 3 hr. The reaction mixture was filtered, and the filtrate was concentrated. The residue was subjected to silica gel column chromatography and eluted with ethyl acetate-hexane (1:5, v/v) to give ethyl 3-(2-hydroxy-4-propoxyphenyl)propanoate as a pale-yellow oil (2.1 g, yield 97%). ¹H-NMR (300 MHz, CDCl₃) δ:1.01 (3 H, t, J = 7.4 Hz), 1.23 (3 H, t, J = 7.2 Hz), 1.68 - 1.86 (2 H, m), 2.60 - 2.74 (2 H, m), 2.77 - 2.89 (2 H, m), 3.86 (2 H, t, J = 6.6 Hz), 4.14 (2 H, q, J = 7.2 Hz), 6.38 - 6.51 (2 H, m), 6.95 (1 H, d, J = 8.3 Hz), 7.43 (1 H, s).

### Reference Example 201

To a solution (30 ml) of ethyl 3-(2-hydroxy-4-propoxyphenyl)propanoate (1.0 g) in N,N-dimethylformamide was added sodium hydride (192 mg, 60% in oil) at room temperature, and the mixture was stirred for 30 min. To this reaction mixture was added 2,3-dichloro-5-(trifluoromethyl)pyridine (0.6 ml), and the mixture was stirred at room temperature for 3 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:10, v/v) to give ethyl 3-(2-{[3-chloro-5-(trifluoromethyl)pyridin-2-yl]oxy}-4-propoxyphenyl)propanoate as a colorless oil (1.2 g, yield 68%). ¹H-NMR (300 MHz, CDCl₃) δ:1.01 (3 H, t, J = 7.4 Hz), 1.21 (3 H, t, J = 7.2 Hz), 1.71 - 1.86 (2 H, m), 2.51 - 2.62 (2 H, m), 2.70 - 2.81 (2 H, m), 3.88 (2 H, t, J = 6.5 Hz), 4.09 (2 H, q, J = 7.2 Hz), 6.64 (1 H, d, J = 2.5 Hz), 6.78 (1 H, dd, J = 8.5, 2.6 Hz), 7.22 (1 H, d, J = 8.5 Hz), 7.96 - 8.00 (1 H, m), 8.24 - 8.29 (1 H, m).

### Reference Example 202

To a solution (30 ml) of ethyl 3-(2-{[3-chloro-5-(trifluoromethyl)pyridin-2-yl]oxy}-4-propoxyphenyl)propanoate (1.1 g) in tetrahydrofuran was added lithium aluminum hydride (94 mg) at room temperature, and the mixture was stirred for 15 min. To this reaction mixture was added sodium sulfate decahydrate (796 mg), and the mixture was filtered through celite and concentrated. The residue was subjected to silica gel column chromatography and eluted with ethyl acetate-hexane (1:3, v/v) to give 3-(2-{[3-chloro-5-(trifluoromethyl)pyridin-2-yl]oxy}-4-propoxyphenyl)propan-1-ol as a colorless oil (810 mg, yield 84%). ¹H-NMR (300 MHz, CDCl₃) δ:1.02 (3 H, t, J = 7.4 Hz), 1.40 (1 H, t, J = 5.1 H,z), 1.72 - 1.88 (4 H, m), 2.46 - 2.60 (2 H, m), 3.60 (2 H, q, J = 6.0 Hz), 3.88 (2 H, t, J = 6.5 Hz), 6.63 (1 H, d, J = 2.6 Hz), 6.80 (1 H, dd, J = 8.5, 2.5 Hz), 7.22 (1 H, d, J = 8.5 Hz), 7.98 (1 H, d, J = 2.3 Hz), 8.27 (1 H, dd, J = 2.3, 1.0 Hz).

### Reference Example 203

To a solution (50 ml) of ethyl (2E)-3-[2-(benzyloxy)-4-hydroxyphenyl]acrylate (5.1 g) in N,N-dimethylformamide were added 2-iodopropane (2.0 ml) and potassium carbonate (3.1 g), and the mixture was stirred at 60°C for 1.5 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:5, v/v) to give ethyl (2E)-3-[2-(benzyloxy)-4-isopropoxyphenyl]acrylate as a colorless oil (5.0 g, yield 86%). ¹H-NMR (300 MHz, CDCl₃) δ:1.27 - 1.35 (9 H, m), 4.23 (2 H, q, J = 7.0 Hz), 4.46 - 4.61 (1 H, m), 5.13 (2 H, s), 6.38 - 6.54 (3 H, m), 7.28 - 7.51 (6 H, m), 8.00 (1 H, d, J = 16.2 Hz).

### Reference Example 204

To a solution (100 ml) of ethyl (2E)-3-[2-(benzyloxy)-4-isopropoxyphenyl]acrylate (5.0 g) in a mixed solvent of tetrahydrofuran-ethanol (1:1, v/v) was added 10% palladium-carbon (0.5 g), and the mixture was stirred under a hydrogen atmosphere for 2 hr. The reaction mixture was filtered, and the filtrate was concentrated. The residue was subjected to silica gel column chromatography and eluted with ethyl acetate-hexane (1:4, v/v) to give ethyl 3-(2-hydroxy-4-isopropoxyphenyl)propanoate as a pale-yellow oil (3.3 g, yield 89%). ¹H-NMR (300 MHz, CDCl₃) δ:1.24 (3 H, t, J = 7.2 Hz), 1.31 (6 H, d, J = 6.0 Hz), 2.63 - 2.72 (2 H, m), 2.76 - 2.86 (2 H, m), 4.15 (2 H, q, J = 7.2 Hz), 4.40 - 4.55 (1 H, m), 6.38 - 6.48 (2 H, m), 6.94 (1 H, d, J = 8.3 Hz), 7.43 (1 H, s).

### Reference Example 205

To a solution (60 ml) of ethyl 3-(2-hydroxy-4-isopropoxyphenyl)propanoate (1.51 g) in N,N-dimethylformamide was added sodium hydride (312 mg, 60% in oil) at room temperature, and the mixture was stirred for 30 min. To this reaction mixture was added 2,3-dichloro-5-(trifluoromethyl)pyridine (1.56 g), and the mixture was stirred at 60°C for 15 min. Saturated aqueous ammonium chloride was poured into the reaction mixture, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:15, v/v) to give ethyl 3-(2-{[3-chloro-5-(trifluoromethyl)pyridin-2-yl]oxy}-4-isopropoxyphenyl)propanoate as a colorless oil (2.02 g, yield 78%). ¹H-NMR (300 MHz, CDCl₃) δ:1.21 (3 H, t, J = 7.2 Hz), 1.32 (6 H, d, J = 6.0 Hz), 2.52 - 2.62 (2 H, m), 2.70 - 2.81 (2 H, m), 4.09 (2 H, q, J = 7.2 Hz), 4.41 - 4.56 (1 H, m), 6.63 (1 H, d, J = 2.6 Hz), 6.76 (1 H, dd, J = 8.5, 2.6 Hz), 7.21 (1 H, d, J = 8.5 Hz), 7.98 (1 H, d, J = 2.1 Hz), 8.27 (1 H, dd, J = 2.1, 0.9 Hz).

### Reference Example 206

To a solution (30 ml) of ethyl 3-(2-{[3-chloro-5-(trifluoromethyl)pyridin-2-yl]oxy}-4-isopropoxyphenyl)propanoate (1.9 g) in tetrahydrofuran was added lithium aluminum hydride (188 mg) at room temperature, and the mixture was stirred for 10 min. To this reaction mixture was added sodium sulfate decahydrate (1.6 g), and the mixture was filtered through celite and concentrated. The residue was subjected to silica gel column chromatography and eluted with ethyl acetate-hexane (1:4, v/v) to give 3-(2-{[3-chloro-5-(trifluoromethyl)pyridin-2-yl]oxy}-4-isopropoxyphenyl)propan-1-ol as a pale-yellow oil (1.3 g, yield 77%). Recrystallization from ethyl acetate-hexane gave pale-yellow prism crystals. melting point 72 - 73°C.

### Reference Example 207

To a solution (50 ml) of ethyl 3-(2-hydroxy-4-isopropoxyphenyl)propanoate (3.03 g) in N,N-dimethylformamide was added sodium hydride (528 mg, 60% in oil) at room temperature, and the mixture was stirred for 20 min. To this reaction mixture was added 2,3,5-trichloropyridine (3.28 g), and the mixture was stirred at 85°C for 4 hr. Saturated aqueous ammonium chloride was poured into the reaction mixture, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:10, v/v) to give ethyl 3-{2-[(3,5-dichloropyridin-2-yl)oxy]-4-isopropoxyphenyl}propanoate as a colorless oil (2.54 g, yield 53%). ¹H-NMR (300 MHz, CDCl₃) δ:1.22 (3 H, t, J = 7.1 H,z), 1.31 (6 H, d, J = 6.0 Hz), 2.51 - 2.63 (2 H, m), 2.72 - 2.83 (2 H, m), 4.09 (2 H, q, J = 7.1 Hz), 4.39 - 4.55 (1 H, m), 6.59 (1 H, d, J = 2.5 Hz), 6.72 (1 H, dd, J = 8.5, 2.5 Hz), 7.18 (1 H, d, J = 8.5 Hz), 7.77 (1 H, d, J = 2.5 Hz), 7.96 (1 H, d, J = 2.5 Hz).

### Reference Example 208

To a solution (30 ml) of ethyl 3-{2-[(3,5-dichloropyridin-2-yl)oxy]-4-isopropoxyphenyl}propanoate (957 mg) in tetrahydrofuran was added lithium aluminum hydride (91 mg) at room temperature, and the mixture was stirred for 10 min. To this reaction mixture was added sodium sulfate decahydrate (773 mg), and the mixture was filtered through celite and concentrated. The residue was subjected to silica gel column chromatography and eluted with ethyl acetate-hexane (1:4, v/v) to give 3-{2-[(3,5-dichloropyridin-2-yl)oxy]-4-isopropoxyphenyl}propan-1-ol as a colorless oil (799 mg, yield 93%). Recrystallization from ethyl acetate-hexane gave colorless prism crystals. melting point 57 - 59°C.

### Reference Example 209

To a mixture of ethyl (2E)-3-[2-(benzyloxy)-4-hydroxyphenyl]acrylate (2.5 g), 2-methoxyethanol (1.0 ml), tributylphosphine (3.5 ml) and tetrahydrofuran (100 ml) was added 1,1'-azodicarbonyldipiperidine (4.2 g) at room temperature, and the mixture was stirred for 2 hr. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:5, v/v) to give ethyl (2E)-3-[2-(benzyloxy)-4-(2-methoxyethoxy)phenyl]acrylate as a colorless oil (2.5 g, yield 83%). ¹H-NMR (300 MHz, CDCl₃) δ:1.31 (3 H, t, J = 7.2 Hz), 3.44 (3 H, s), 3.69 - 3.78 (2 H, m), 4.06 - 4.15 (2 H, m), 4.23 (2 H, q, J = 7.2 Hz), 5.13 (2 H, s), 6.37 - 6.58 (3 H, m), 7.28 - 7.51 (6 H, m), 7.99 (1 H, d, J = 16.2 Hz).

### Reference Example 210

To a solution (100 ml) of ethyl (2E)-3-[2-(benzyloxy)-4-(2-methoxyethoxy)phenyl]acrylate (2.5 g) in a mixed solvent of tetrahydrofuran-ethanol (1:1, v/v) was added 10% palladium-carbon (0.25 g), and the mixture was stirred under a hydrogen atmosphere for 3 hr. The reaction mixture was filtered, and the filtrate was concentrated. The residue was subjected to silica gel column chromatography and eluted with ethyl acetate-hexane (1:5 to 1:3, v/v) to give ethyl 3-[2-hydroxy-4-(2-methoxyethoxy)phenyl]propanoate as a colorless oil (2.8 g, yield 95%). ¹H-NMR (300 MHz, CDCl₃) δ:1.23 (3H, t, J = 7.2 Hz), 2.62 - 2.70 (2 H, m), 2.78 - 2.86 (2 H, m), 3.44 (3 H, s), 3.70 - 3.75 (2 H, m), 4.04 - 4.09 (2 H, m), 4.14 (2 H, q, J = 7.2 Hz), 6.42 - 6.52 (2 H, m), 6.95 (1 H, d, J = 8.1 Hz), 7.42 - 7.50 (1 H, m).

### Reference Example 211

To a solution (40 ml) of ethyl 3-[2-hydroxy-4-(2-methoxyethoxy)phenyl]propanoate (1.6 g) in N,N-dimethylformamide was added sodium hydride (285 mg, 60% in oil) at room temperature, and the mixture was stirred for 30 min. To this reaction mixture was added 2,3-dichloro-5-(trifluoromethyl)pyridine (0.9 ml), and the mixture was stirred at room temperature for 30 min. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:5, v/v) to give ethyl 3-[2-{[3-chloro-5-(trifluoromethyl)pyridin-2-yl]oxy}-4-(2-methoxyethoxy)phenyl]propanoate as a colorless oil (2.2 g, yield 84%). ¹H-NMR (300 MHz, CDCl₃) δ:1.21 (3 H, t, J = 7.2 Hz), 2.50 - 2.63 (2 H, m), 2.72 - 2.84 (2 H, m), 3.43 (3 H, s), 3.65 - 3.79 (2 H, m), 4.02 - 4.16 (4 H, m), 6.68 (1 H, d, J = 2.6 Hz), 6.82 (1 H, dd, J = 8.5, 2.6 Hz), 7.22 (1 H, d, J = 8.5 Hz), 7.98 (1 H, d, J = 1.9 Hz), 8.21 - 8.29 (1 H, m).

### Reference Example 212

To a solution (100 ml) of ethyl 3-[2-{[3-chloro-5-(trifluoromethyl)pyridin-2-yl]oxy}-4-(2-methoxyethoxy)phenyl]propanoate (2.2 g) in tetrahydrofuran was added lithium aluminum hydride (186 mg) at room temperature, and the mixture was stirred for 15 min. To this reaction mixture was added sodium sulfate decahydrate (1.6 g), and the mixture was filtered through celite and concentrated. The residue was subjected to silica gel column chromatography and eluted with ethyl acetate-hexane (1:2, v/v) to give 3-[2-{[3-chloro-5-(trifluoromethyl)pyridin-2-yl]oxy}-4-(2-methoxyethoxy)phenyl]propan-1-ol as a colorless oil (1.7 g, yield 83%). Recrystallization from ethyl acetate-hexane gave colorless prism crystals. melting point 70°C - 71°C.

### Reference Example 213

To a solution (100 ml) of 2-bromopyridine (3.1 ml) in tetrahydrofuran was added dropwise a 1.6M solution (22.5 ml) of n-butyllithium in hexane at -78°C, and the mixture was stirred for 30 min. To this reaction mixture was added a solution (20 ml) of 3-ethoxy-4-hydroxybenzaldehyde (2.5 g) in tetrahydrofuran, and the mixture was stirred at room temperature for 15 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:1, v/v) to give 2-ethoxy-4-[hydroxy(pyridin-2-yl)methyl]phenol as a colorless oil (1.6 g, yield 43%). ¹H-NMR (300 MHz, CDCl₃) δ:1.39 (3 H, t, J = 7.1 Hz), 3.96 - 4.15 (2 H, m), 5.18 - 5.27 (1 H, m), 5.63 - 5.69 (1 H, m), 5.73 (1 H, brs), 6.81 - 6.91 (3 H, m), 7.10 - 7.24 (2 H, m), 7.57 - 7.68 (1 H, m), 8.55 (1 H, d, J = 4.9 Hz).

### Reference Example 214

To a solution (30 ml) of 2-ethoxy-4-[hydroxy(pyridin-2-yl)methyl]phenol (1.6 g) in N,N-dimethylformamide were added ethyl bromoacetate (0.8 ml) and potassium carbonate (2.0 g) at room temperature, and the mixture was stirred at 80°C for 15 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:2, v/v) to give ethyl {2-ethoxy-4-[hydroxy(pyridin-2-yl)methyl]phenoxy}acetate as a brown oil (1.8 g, yield 84%). ¹H-NMR (300 MHz, CDCl₃) δ:1.28 (3 H, t, J = 7.2 Hz), 1.41 (3 H, t, J = 7.1 Hz), 3.97 - 4.15 (2 H, m), 4.24 (2 H, q, J = 7.2 Hz), 4.65 (2 H, s), 5.23 (1 H, d, J = 4.0 Hz), 5.64 - 5.70 (1 H, m), 6.77 - 6.95 (3 H, m), 7.08 - 7.25 (2 H, m), 7.57 - 7.68 (1 H, m), 8.52 - 8.61 (1 H, m).

### Reference Example 215

To a solution (100 ml) of ethyl {2-ethoxy-4-[hydroxy(pyridin-2-yl)methyl]phenoxy}acetate (1.7 g) in tetrahydrofuran was added lithium aluminum hydride (194 mg) at 0°C, and the mixture was stirred for 30 min. To this reaction mixture was added sodium sulfate decahydrate (1.6 g), and the mixture was filtered through celite and concentrated. The residue was subjected to silica gel column chromatography and eluted with ethyl acetate-hexane (5:1, v/v) to give 2-{2-ethoxy-4-[hydroxy(pyridin-2-yl)methyl]phenoxy}ethanol as a yellow oil (942 mg, yield 64%). ¹H-NMR (300 MHz, CDCl₃) δ:1.41 (3 H, t, J = 7.0 Hz), 2.76 (1 H, t, J = 5.4 Hz), 3.81 - 3.92 (2 H, m), 3.96 - 4.16 (4 H, m), 5.24 (1 H, d, J = 4.1 Hz), 5.68 (1 H, d, J = 4.1 Hz), 6.85 - 6.97 (3 H, m), 7.11 - 7.24 (2 H, m), 7.59 - 7.69 (1 H, m), 8.54 - 8.60 (1 H, m).

### Reference Example 216

To a solution (50 ml) of 2-{2-ethoxy-4-[hydroxy(pyridin-2-yl)methyl]phenoxy}ethanol (900 mg) in tetrahydrofuran was added manganese dioxide (4.0 g) at room temperature, and the mixture was stirred for 2.5 hr. The reaction mixture was filtered through celite and concentrated. The residue was recrystallized from ethyl acetate-hexane to give [3-ethoxy-4-(2-hydroxyethoxy)phenyl](pyridin-2-yl)methanone as colorless needle crystals (679 mg, yield 76%). melting point 140 - 141°C.

### Reference Example 217

To a solution (200 ml) of 2-bromopyridine (4.4 ml) in tetrahydrofuran was added dropwise a 1.6M solution (33 ml) of n-butyllithium in hexane at -78°C, and the mixture was stirred for 30 min. To this reaction mixture was added a solution (50 ml) of 4-(benzyloxy)-3-ethoxybenzaldehyde (9.1 g) in tetrahydrofuran, and the mixture was stirred at room temperature for 2 hr. Saturated aqueous ammonium chloride was poured into the reaction mixture, and the mixture was extracted with ethyl acetate. The ethyl acetate layer was washed with saturated brine, dried (MgSO₄) and concentrated. The residue was recrystallized from ethyl acetate-hexane to give [4-(benzyloxy)-3-ethoxyphenyl](pyridin-2-yl)methanol as pale-yellow prism crystals (8.6 g, yield 68%). melting point 109 - 110°C.

### Reference Example 218

To a solution (100 ml) of [4-(benzyloxy)-3-ethoxyphenyl](pyridin-2-yl)methanol (5.0 g) in trifluoroacetic acid was added dropwise triethylsilane (9.0 ml), and the mixture was stirred at room temperature for 20 hr. The reaction mixture was concentrated, the residue was neutralized with a saturated aqueous sodium hydrogencarbonate solution, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:2, v/v) to give 2-ethoxy-4-(pyridin-2-ylmethyl)phenol as a yellow oil (2.2 g, yield 68%). ¹H-NMR (300 MHz, CDCl₃) δ:1.40 (3 H, t, J = 7.0 Hz), 4.00 - 4.13 (2 H, m), 4.07 (2 H, s), 5.71 (1 H, brs), 6.69 - 6.92 (3 H, m), 7.06 - 7.18 (2 H, m), 7.52 - 7.74 (1 H, m), 8.49 - 8.61 (1 H, m).

### Reference Example 219

To a solution (100 ml) of 2-ethoxy-4-(pyridin-2-ylmethyl)phenol (2.2 g) in tetrahydrofuran was added sodium hydride (422 mg, 60% in oil) at room temperature, and the mixture was stirred for 30 min. To the reaction mixture was added N-phenylbis(trifluoromethanesulfonimide) (4.1 g), and the mixture was stirred at room temperature for 1 hr. Saturated aqueous ammonium chloride was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:2, v/v) to give 2-ethoxy-4-(pyridin-2-ylmethyl)phenyl trifluoromethanesulfonate as a yellow oil (3.3 g, yield 95%). ¹H-NMR (300 MHz, CDCl₃) δ:1.43 (3 H, t, J = 7.0 Hz), 4.07 (2 H, q, J = 7.0 Hz), 4.13 (2 H, s), 6.78 - 6.99 (2 H, m), 7.07 - 7.23 (3 H, m), 7.57 - 7.70 (1 H, m), 8.51 - 8.64 (1 H, m).

### Reference Example 220

To a mixture of 2-ethoxy-4-(pyridin-2-ylmethyl)phenyl trifluoromethanesulfonate (3.3 g), palladium(II) acetate (204 mg), triphenylphosphine (477 mg), benzyltriethylammonium chloride (2.1 g), methyl acrylate (3.3 ml) and 1-methyl-2-pyrrolidone (30 ml) was added sodium acetate (1.5 g) at room temperature, and the mixture was stirred at 80°C for 24 hr under an argon atmosphere. The reaction mixture was cooled to room temperature, water was poured thereinto, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:2, v/v) to give methyl (2E)-3-[2-ethoxy-4-(pyridin-2-ylmethyl)phenyl]acrylate as a brown oil (2.0 g, yield 74%). ¹H-NMR (300 MHz, CDCl₃) δ:1.45 (3 H, t, J = 7.0 Hz), 3.79 (3 H, s), 4.07 (2 H, q, J= 7.0 Hz), 4.14 (2 H, s), 6.52 (1 H, d, J = 16.2 Hz), 6.79 - 6.88 (2 H, m), 7.07 - 7.19 (2 H, m), 7.43 (1 H, d, J = 7.7 Hz), 7.53 - 7.63 (1 H, m), 7.96 (1 H, d, J = 16.2 Hz), 8.51 - 8.62 (1 H, m).

### Reference Example 221

To a solution (50 ml) of methyl (2E)-3-[2-ethoxy-4-(pyridin-2-ylmethyl)phenyl]acrylate (1.9 g) in ethanol was added 10% palladium-carbon (1.9 g) at room temperature. To this reaction mixture was added dropwise formic acid (50 ml), and the mixture was heated under reflux for 1.5 hr. The reaction mixture was filtrated and concentrated. The residue was neutralized with a saturated aqueous ammonium carbonate solution, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:2, v/v) to give methyl 3-[2-ethoxy-4-(pyridin-2-ylmethyl)phenyl]propanoate as a yellow oil (1.3 g, yield 68%). ¹H-NMR (300 MHz, CDCl₃) δ:1.38 (3 H, t, J = 7.0 Hz), 2.60 (2 H, t, J = 7.8 Hz), 2.86 - 2.96 (2 H, m), 3.66 (3 H, s), 3.99 (2 H, q, J = 7.0 Hz), 4.10 (2 H, s), 6.70 - 6.84 (2 H, m), 7.00 - 7.18 (3 H, m), 7.53 - 7.61 (1 H, m), 8.51 - 8.61 (1 H, m).

### Reference Example 222

To a solution (30 ml) of methyl 3-[2-ethoxy-4-(pyridin-2-ylmethyl)phenyl]propanoate (1.2 g) in tetrahydrofuran was added lithium aluminum hydride (152 mg) at room temperature, and the mixture was stirred for 30 min. To this reaction mixture was added sodium sulfate decahydrate (1.3 g), and the mixture was filtered through celite and concentrated. The residue was subjected to silica gel column chromatography and eluted with ethyl acetate-hexane (2:1, v/v) to give 3-[2-ethoxy-4-(pyridin-2-ylmethyl)phenyl]propan-1-ol as a colorless oil (686 mg, yield 63%). ¹H-NMR (300 MHz, CDCl₃) δ:1.40 (3 H, t, J = 7.0 Hz), 1.76 - 1.90 (2 H, m), 1.99 (1 H, t, J = 6.0 Hz), 2.70 (2 H, t, J = 7.2 Hz), 3.57 (2 H, q, J = 6.0 Hz), 4.01 (2 H, q, J = 7.0 Hz), 4.11 (2 H, s), 6.73 - 6.83 (2 H, m), 7.02 - 7.16 (3 H, m), 7.52 - 7.63 (1 H, m), 8.55 (1 H, dd, J = 5.3, 2.1 Hz).

### Reference Example 223

To a solution (250 ml) of 2-bromopyridine (5.7 ml) in tetrahydrofuran was added dropwise a 1.6M solution (43 ml) of n-butyllithium in hexane at -78°C, and the mixture was stirred for 30 min. To this reaction mixture was added a solution (50 ml) of 4-(benzyloxy)-3-isopropoxybenzaldehyde (12.4 g) in tetrahydrofuran, and the mixture was stirred at room temperature for 2 hr. Saturated aqueous ammonium chloride was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:2, v/v) to give [4-(benzyloxy)-3-isopropoxyphenyl](pyridin-2-yl)methanol as a brown oil (12.6 g, yield 79%). ¹H-NMR (300 MHz, CDCl₃) δ:1.30 (3 H, d, J = 6.0 Hz), 1.33 (3 H, d, J = 6.2 Hz), 4.43 - 4.58 (1 H, m), 5.10 (2 H, s), 5.22 (1 H, d, J = 4.3 Hz), 5.66 (1 H, d, J = 4.3 Hz), 6.84 - 6.97 (3 H, m), 7.09 - 7.22 (2 H, m), 7.27 - 7.47 (5 H, m), 7.56 - 7.66 (1 H, m), 8.52 - 8.60 (1 H, m).

### Reference Example 224

To a solution (100 ml) of [4-(benzyloxy)-3-isopropoxyphenyl](pyridin-2-yl)methanol (12.6 g) in trifluoroacetic acid was added dropwise triethylsilane (28.7 ml), and the mixture was stirred at room temperature for 20 hr and concentrated. The residue was neutralized with a saturated aqueous sodium hydrogencarbonate solution, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:2, v/v) to give 2-isopropoxy-4-(pyridin-2-ylmethyl)phenol as a yellow oil (4.64 g, yield 53%). ¹H-NMR (300 MHz, CDCl₃) δ:1.32 (6 H, d, J = 6.0 Hz), 4.06 (2 H, s), 4.46 - 4.61 (1 H, m), 5.66 (1 H, brs), 6.70 - 6.91 (3 H, m), 7.03 - 7.15 (2 H, m), 7.50 - 7.63 (1 H, m), 8.49 - 8.60 (1 H, m).

### Reference Example 225

To a solution (50 ml) of 2-isopropoxy-4-(pyridin-2-ylmethyl)phenol (1.5 g) in tetrahydrofuran was added sodium hydride (273 mg, 60% in oil) at room temperature, and the mixture was stirred for 30 min. To the reaction mixture was added N-phenylbis(trifluoromethanesulfonimide) (2.7 g), and the mixture was stirred for 1 hr. Saturated aqueous ammonium chloride was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:3, v/v) to give 2-isopropoxy-4-(pyridin-2-ylmethyl)phenyl trifluoromethanesulfonate as a yellow oil (2.1 g, yield 91%). ¹H-NMR (300 MHz, CDCl₃) δ:1.34 (6 H, d, J = 6.0 Hz), 4.13 (2H, s), 4.50 - 4.67 (1 H, m), 6.80 (1 H, dd, J = 8.3, 2.1 Hz), 6.89 - 6.97 (1 H, m), 7.07 - 7.21 (3 H, m), 7.57 - 7.68 (1 H, m), 8.52 - 8.62 (1 H, m).

### Reference Example 226

To a mixture of 2-isopropoxy-4-(pyridin-2-ylmethyl)phenyl trifluoromethanesulfonate (4.67 g), palladium(II) acetate (278 mg), triphenylphosphine (650 mg), benzyltriethylammonium chloride (2.80 g), methyl acrylate (6.7 ml) and 1-methyl-2-pyrrolidone (30 ml) was added sodium acetate (2.03 g) at room temperature, and the mixture was stirred at 80°C for 24 hr under an argon atmosphere. The reaction mixture was cooled to room temperature, water was poured thereinto, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:2, v/v) to give a brown oil. To a solution (50 ml) of the obtained oil in ethanol was added 10% palladium-carbon (3.0 g) at room temperature. To this reaction mixture was added dropwise formic acid (50 ml), and the mixture was heated under reflux for 1 hr. The reaction mixture was filtrated, and concentrated. The residue was neutralized with a saturated aqueous ammonium carbonate solution, and the mixture was extracted with ethyl acetate. The ethyl acetate layer was washed with water and saturated brine, dried (MgSO₄) and concentrated. To a solution (30 ml) of the obtained oil in tetrahydrofuran was added lithium aluminum hydride (182 mg) at room temperature, and the mixture was stirred for 30 min. To this reaction mixture was added sodium sulfate decahydrate (1.5 g), and the mixture was filtered through celite and concentrated. The residue was subjected to silica gel column chromatography and eluted with ethyl acetate-hexane (1:2, v/v) to give 3-[2-isopropoxy-4-(pyridin-2-ylmethyl)phenyl]propan-1-01 as a yellow oil (480 mg, yield 14%). ¹H-NMR (300 MHz, CDCl₃) δ:1.31 (6 H, d, J = 6.2 Hz), 1.73 - 1.89 (2 H, m), 2.07 (1 H, t, J = 6.2 Hz), 2.68 (2 H, t, J = 7.1 Hz), 3.55 (2 H, q, J = 6.0 Hz), 4.11 (2 H, s), 4.45 - 4.64 (1 H, m), 6.74 - 6.83 (2 H, m), 7.01 - 7.16 (3 H, m), 7.53 - 7.64 (1 H, m), 8.51 - 8.59 (1 H, m).

### Reference Example 227

To a mixture of ethyl 4-hydroxy-2-isopropylpyrimidine-5-carboxylate (14.3 g) and phosphorus oxychloride (41.9 g) was added dropwise N,N-dimethylformamide (7.0 ml) at 0°C. The mixture was stirred at room temperature for 1.5 hr. This reaction mixture was poured into a saturated aqueous sodium hydrogencarbonate solution cooled to 0°C, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:20, v/v) to give ethyl 4-chloro-2-isopropylpyrimidine-5-carboxylate as a colorless oil (3.56 g, yield 23%). ¹H-NMR (300 MHz, CDCl₃) δ: 1.36 (6 H, d, J = 6.8 Hz), 1.42 (3 H, t, J = 7.1 Hz), 3.18 - 3.33 (1 H, m), 4.44 (2 H, q, J = 7.1 Hz), 9.06 (1 H, s).

### Reference Example 228

To a solution (70 ml) of 2,4-dichlorophenol (2.5 g) in N,N-dimethylformamide was added sodium hydride (677 mg, 60% in oil) at 0°C, and the mixture was stirred for 30 min. To this reaction mixture was added ethyl 4-chloro-2-isopropylpyrimidine-5-carboxylate (3.2 g), and the mixture was stirred at room temperature for 30 min. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:10, v/v) to give ethyl 4-(2,4-dichlorophenoxy)-2-isopropylpyrimidine-5-carboxylate as a colorless oil (4.8 g, yield 96%). ¹H-NMR (300 MHz, CDCl₃) δ:1.14 (6 H, d, J = 6.0 Hz), 1.41 (3 H, t, J = 7.2 Hz), 2.93 - 3.08 (1 H, m), 4.44 (2 H, q, J = 7.2 Hz), 7.18 (1 H, d, J = 8.7 Hz), 7.28 - 7.34 (1 H, m), 7.48 (1 H, d, J = 2.5 Hz), 9.09 (1 H, s).

### Reference Example 229

Lithium aluminum hydride (31 mg) was added to a solution (30 ml) of ethyl 4-(2,4-dichlorophenoxy)-2-isopropylpyrimidine-5-carboxylate (290 mg) in tetrahydrofuran at 0°C, and the mixture was stirred for 30 min. To this reaction mixture was added sodium sulfate decahydrate (263 mg), and the mixture was filtered through celite and concentrated. The residue was subjected to silica gel column chromatography and eluted with ethyl acetate-hexane (2:3, v/v) to give [4-(2,4-dichlorophenoxy)-2-isopropylpyrimidin-5-yl]methanol as a colorless oil (158 mg, yield 62%). ¹H-NMR (300 MHz, CDCl₃) δ:1.15 (6 H, d, J = 7.0 Hz), 2.25 (1 H, t, J = 6.2 Hz), 2.89 - 3.07 (1 H, m), 4.84 (2 H, d, J = 6.2 Hz), 7.19 - 7.35 (2 H, m), 7.49 (1 H, d, J = 2.5 Hz), 8.57 (1 H, s).

### Reference Example 230

To a solution (100 ml) of [4-(2,4-dichlorophenoxy)-2-isopropylpyrimidin-5-yl]methanol (2.0 g) in tetrahydrofuran was added manganese dioxide (12.0 g) at room temperature, and the mixture was stirred for 3 hr. The reaction mixture was filtered through celite and concentrated. The residue was subjected to silica gel column chromatography and eluted with ethyl acetate-hexane (1:10, v/v) to give 4-(2,4-dichlorophenoxy)-2-isopropylpyrimidine-5-carbaldehyde as a colorless oil(1.9g,yield93%) . ¹H-NMR (300 MHz, CDCl₃) δ:1.18 (6 H, d, J = 7.0 Hz), 2.96 - 3.13 (1 H, m), 7.20 - 7.29 (1 H, m), 7.32 - 7.39 (1 H, m), 7.52 (1 H, d, J = 2.3 Hz), 9.04 (1 H, s), 10.51 (1 H, s).

### Reference Example 231

To a solution (60 ml) of ethyl diethylphosphonoacetate (1.4 ml) in tetrahydrofuran was added sodium hydride (307 mg, 60% in oil) at room temperature, and the mixture was stirred for 30 min. To this reaction mixture was added dropwise a solution (10 ml) of 4-(2,4-dichlorophenoxy)-2-isopropylpyrimidine-5-carbaldehyde (1.8 g) in tetrahydrofuran, and the mixture was stirred at room temperature for 30 min. Saturated aqueous ammonium chloride was poured into the reaction mixture, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:10, v/v) to give ethyl (2E)-3-[4-(2,4-dichlorophenoxy)-2-isopropylpyrimidin-5-yl]acrylate as a colorless oil (2.2 g, yield 100%). ¹H-NMR (300 MHz, CDCl₃) δ:1.15 (6 H, d, J = 6.8 Hz), 1.35 (3 H, t, J = 7.2 Hz), 2.90 - 3.06 (1 H, m), 4.29 (2 H, q, J = 7.2 Hz), 6.80 (1 H, d, J = 16.2 Hz), 7.18 (1 H, d, J = 8.7 Hz), 7.29 - 7.36 (1 H, m), 7.50 (1 H, d, J = 2.5 Hz), 7.78 (1 H, d, J = 16.2 Hz), 8.69 (1 H, s).

### Reference Example 232

To a solution (100 ml) of ethyl (2E)-3-[4-(2,4-dichlorophenoxy)-2-isopropylpyrimidin-5-yl]acrylate (2.2 g) in tetrahydrofuran was added 10% palladium-carbon (0.3 g), and the mixture was stirred under a hydrogen atmosphere for 1.5 hr. The reaction mixture was filtered, and the filtrate was concentrated. The residue was subjected to silica gel column chromatography and eluted with ethyl acetate-hexane (1:8 to 1:4, v/v) to give ethyl 3-[4-(2,4-dichlorophenoxy)-2-isopropylpyrimidin-5-yl]propanoate as a colorless oil (2.0 g, yield 90%). ¹H-NMR (300 MHz, CDCl₃) δ:1.13 (6 H, d, J = 7.0 Hz), 1.24 (3 H, t, J = 7.2 Hz), 2.76 (2 H, t, J = 7.5 Hz), 2.87 - 3.09 (3 H, m), 4.14 (2 H, q, J = 7.2 Hz), 7.15 - 7.21 (1 H, m), 7.27 - 7.33 (1 H, m), 7.48 (1 H, d, J = 2.5 Hz), 8.40 (1 H, s).

### Reference Example 233

To a solution (50 ml) of ethyl 3-[4-(2,4-dichlorophenoxy)-2-isopropylpyrimidin-5-yl]propanoate (1.9 g) in tetrahydrofuran was added lithium aluminum hydride (192 mg) at 0°C, and the mixture was stirred for 10 min. To this reaction mixture was added sodium sulfate decahydrate (1.6 g), and the mixture was filtered through celite and concentrated. The residue was subjected to silica gel column chromatography and eluted with ethyl acetate-hexane (2:1, v/v) to give 3-[4-(2,4-dichlorophenoxy)-2-isopropylpyrimidin-5-yl]propan-1-ol as a colorless oil (1.6 g, yield 95%). ¹H-NMR (300 MHz, CDCl₃) δ:1.14 (6 H, d, J = 6.8 Hz), 1.55 (1 H, t, J = 5.1 Hz), 1.90 - 2.08 (2 H, m), 2.72 - 2.87 (2 H, m), 2.89 - 3.02 (1 H, m), 3.75 (2 H, q, J = 6.0 Hz), 7.14 - 7.22 (1 H, m), 7.27 - 7.33 (1 H, m), 7.48 (1 H, d, J = 2.5 Hz), 8.38 (1 H, s).

### Reference Example 234

To a solution (100 ml) of 2,4-dichlorophenol (6.3 g) in N,N-dimethylformamide was added sodium hydride (1.6 g, 60% in oil) at room temperature, and the mixture was stirred for 30 min. To this reaction mixture was added a solution (20 ml) of methyl 2-chloro-6-isopropoxynicotinate (4.5 g) in N,N-dimethylformamide and the mixture was stirred at 80°C for 15 hr. Water was poured into the reaction mixture, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:30 to 1:10, v/v) to give methyl 2-(2,4-dichlorophenoxy)-6-isopropoxynicotinate as a pale-yellow oil (6.9 g, yield 100%). ¹H-NMR (300 MHz, CDCl₃) δ:1.11 (6 H, d, J = 6.2 Hz), 3.91 (3 H, s), 4.56 - 4.71 (1 H, m), 6.39 (1 H, d, J = 8.5 Hz), 7.15 (1 H, d, J = 8.7 Hz), 7.24 - 7.35 (1 H, m), 7.46 (1 H, d, J = 2.5 Hz), 8.21 (1 H, d, J = 8.5 Hz).

### Reference Example 235

To a solution (150 ml) of methyl 2-(2,4-dichlorophenoxy)-6-isopropoxynicotinate (6.8 g) in tetrahydrofuran was added lithium aluminum hydride (729 mg) at 0°C, and the mixture was stirred for 30 min. To this reaction mixture was added sodium sulfate decahydrate (6.2 g), and the mixture was filtered through celite and concentrated. The residue was subjected to silica gel column chromatography and eluted with ethyl acetate-hexane (1:5, v/v) to give [2-(2,4-dichlorophenoxy)-6-isopropoxypyridin-3-yl]methanol as a colorless oil (4.9 g, yield 78%). ¹H-NMR (300 MHz, CDCl₃) δ:1.13 (6 H, d, J = 6.2 Hz), 2.12 (1 H, brs), 4.60 - 4.80 (2 H, m), 6.37 (1 H, d, J = 8.1 Hz), 7.15 - 7.22 (1 H, m), 7.23 - 7.31 (1 H, m), 7.46 (1 H, d, J = 2.5 Hz), 7.61 (1 H, d, J = 8.1 Hz).

### Reference Example 236

To a solution (150 ml) of [2-(2,4-dichlorophenoxy)-6-isopropoxypyridin-3-yl]methanol (4.9 g) in tetrahydrofuran was added manganese dioxide (25.0 g), and the mixture was stirred at room temperature for 1.5 hr. The reaction mixture was filtered through celite and concentrated. The residue was subjected to silica gel column chromatography and eluted with ethyl acetate-hexane (1:20, v/v) to give 2-(2,4-dichlorophenoxy)-6-isopropoxynicotinaldehyde as a colorless oil (3.9 g, yield 80%). Recrystallization from ethyl acetate-hexane gave colorless prism crystals. melting point 73 - 74°C.

### Reference Example 237

To a solution (150 ml) of ethyl diethylphosphonoacetate (2.8 ml) in tetrahydrofuran was added sodium hydride (600 mg, 60% in oil) at room temperature, and the mixture was stirred for 30 min. To this reaction mixture was added dropwise a solution (20 ml) of 2-(2,4-dichlorophenoxy)-6-isopropoxynicotinaldehyde (3.8 g) in tetrahydrofuran, and the mixture was stirred at room temperature for 20 min. Saturated aqueous ammonium chloride was poured into the reaction mixture, and the mixture was extracted with ethyl acetate. The ethyl acetate layer was washed with water and saturated brine, dried (MgSO₄) and concentrated. The residue was recrystallized from ethyl acetate-hexane to give ethyl (2E)-3-[2-(2,4-dichlorophenoxy)-6-isopropoxypyridin-3-yl]acrylate as colorless prism crystals (3.9 g, yield 85%). melting point 92 - 93°C.

### Reference Example 238

To a solution (100 ml) of (2E)-3-[2-(2,4-dichlorophenoxy)-6-isopropoxypyridin-3-yl]ethyl acrylate (3.9 g) in tetrahydrofuran was added 10% palladium-carbon (0.4 g), and the mixture was stirred under a hydrogen atmosphere for 45 min. The reaction mixture was filtered, and the filtrate was concentrated. The residue was recrystallized from ethyl acetate-hexane to give ethyl 3-[2-(2,4-dichlorophenoxy)-6-isopropoxypyridin-3-yl]propanoate as colorless prism crystals (3.8 g, yield 98%). melting point 42.5 - 43.5°C.

### Reference Example 239

To a solution (100 ml) of ethyl 3-[2-(2,4-dichlorophenoxy)-6-isopropoxypyridin-3-yl]propanoate (3.8 g) in tetrahydrofuran was added lithium aluminum hydride (366 mg) at room temperature, and the mixture was stirred for 30 min. To this reaction mixture was added sodium sulfate decahydrate (3.1 g), and the mixture was filtered through celite and concentrated. The residue was subjected to silica gel column chromatography and eluted with ethyl acetate-hexane (1:4, v/v) to give 3-[2-(2,4-dichlorophenoxy)-6-isopropoxypyridin-3-yl]propan-1-ol as a colorless oil (3.3 g, yield 97%). ¹H-NMR (300 MHz, CDCl₃) δ:1.12 (6 H, d, J = 6.2 Hz), 1.46 (1 H, t, J = 5.5 Hz), 1.88 - 2.02 (2 H, m), 2.71 - 2.82 (2 H, m), 3.65 - 3.78 (2 H, m), 4.54 - 4.71 (1 H, m), 6.33 (1 H, d, J = 8.1 Hz), 7.07 - 7.15 (1 H, m), 7.20 - 7.29 (1 H, m), 7.39 - 7.48 (2 H, m).

### Reference Example 240

A mixture of 2,6-dichloronicotinic acid (5.05 g) and potassium tert-butoxide (8.98 g) in ethanol (250 ml) was heated under reflux for 16 hr. After cooling, the reaction mixture was concentrated. The residue was dissolved in water, 5N hydrochloric acid was added, and the mixture was adjusted to pH 1. The resulting crystals were collected by filtration and dried to give 2-chloro-6-ethoxynicotinic acid as pale-pink crystals (2.14 g, yield 40%). melting point 185 - 188°C.

### Reference Example 241

To a solution of 2-chloro-6-ethoxynicotinic acid (2.14 g) and N,N-dimethylformamide (0.085 ml) in tetrahydrofuran (30 ml) was added oxalyl chloride (0.960 ml) at 0°C, and the mixture was stirred at room temperature for 30 min. The reaction mixture was concentrated. Methanol (30 ml) was added to the residue, and the mixture was stirred at room temperature for 30 min. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 1:5, v/v) to give methyl 2-chloro-6-ethoxynicotinate as a pale-yellow oil (2.25 g, yield 98%). ¹H-NMR (300 MHz, CDCl₃) δ:1.40 (3 H, t, J = 7.1 Hz), 3.91 (3 H, s), 4.43 (2 H, q, J = 7.1 Hz), 6.67 (1 H, d, J = 8.5 Hz), 8.12 (1 H, d, J = 8.5 Hz). melting point 46 - 48°C.

### Reference Example 242

To a solution (50 ml) of 2,4-dichlorophenol (3.02 g) in N,N-dimethylformamide was added sodium hydride (0.74 g, 60% in oil) at room temperature, and the mixture was stirred for 30 min. To this reaction mixture was added methyl 2-chloro-6-ethoxynicotinate (2.0 g), and the mixture was stirred at 120°C for 6 hr. Water was poured into the reaction mixture, and the mixture was extracted with ethyl acetate. The ethyl acetate layer was washed with water and saturated brine, dried (MgSO₄) and passed through an aminopropyl silica gel layer. The filtrate was concentrated, and the obtained residue was subjected to silica gel column chromatography and eluted with ethyl acetate-hexane (1:20 to 1:5, v/v) to give methyl 2-(2,4-dichlorophenoxy)-6-ethoxynicotinate as a pale-yellow oil (2.49 g, yield 79%). ¹H-NMR (300 MHz, CDCl₃) 8:1.16 (3 H, t, J = 7.1 Hz) 3.91 (3 H, s) 3.96 (2 H, q, J = 7.2 Hz) 6.43 (1 H, d, J = 8.3 Hz) 7.15 (1 H, d, J = 8.7 Hz) 7.21 - 7.31 (1 H, m) 7.46 (1 H, d, J = 2.5 Hz) 8.23 (1 H, d, J = 8.3 Hz).

### Reference Example 243

To a solution of methyl 2-(2,4-dichlorophenoxy)-6-ethoxynicotinate (1.95 g) in tetrahydrofuran (75 ml) was added lithium aluminum hydride (216 mg) at 0°C, and the mixture was stirred at room temperature for 1 hr. Magnesium sulfate decahydrate (1.84 g) was added to the reaction solution, and the mixture was stirred for 30 min. The resulting insoluble substance was removed by filtration. The filtrate was concentrated, and the obtained residue was subjected to silica gel column chromatography and eluted with ethyl acetate-hexane (1:10 to 1:1, v/v). The eluate was concentrated, and the residue was crystallized from ethyl acetate-hexane to give [2-(2,4-dichlorophenoxy)-6-ethoxypyridin-3-yl]methanol as white crystals (0.90 g, yield 50%). melting point 106 - 107°C.

### Reference Example 244

A mixture of [2-(2,4-dichlorophenoxy)-6-ethoxypyridin-3-yl]methanol (0.90 g) and manganese dioxide (5.0 g) in tetrahydrofuran (15 ml)-toluene (15 ml) was stirred at room temperature for 16 hr. The insoluble substance was removed by filtration, and the filtrate was concentrated. The obtained residue was recrystallized from ethyl acetate-hexane to give 2-(2,4-dichlorophenoxy)-6-ethoxynicotinaldehyde as white crystals (0.80 g, yield 90%). melting point 95 - 96°C.

### Reference Example 245

To a solution (30 ml) of 2-(2,4-dichlorophenoxy)-6-ethoxynicotinaldehyde (1.50 g) and ethyl diethylphosphonoacetate (1.19 g) in tetrahydrofuran was added sodium hydride (0.212 g, 60% in oil) at room temperature, and the mixture was heated under reflux for 2 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 1:3, v/v). The eluate was concentrated, and the residue was crystallized from ethyl acetate-hexane to give ethyl (2E)-3-[2-(2,4-dichlorophenoxy)-6-ethoxypyridin-3-yl]acrylate as white crystals (1.63 g, yield 89%). melting point 90 - 92°C.

### Reference Example 246

A mixture of ethyl (2E)-3-[2-(2,4-dichlorophenoxy)-6-ethoxypyridin-3-yl]acrylate (1.60 g), 10% palladium-carbon (200 mg) and tetrahydrofuran (30 ml) was stirred at room temperature for 2 hr under a hydrogen atmosphere. The insoluble substance was removed by filtration, and the filtrate was concentrated. The obtained residue was subjected to silica gel column chromatography and eluted with ethyl acetate-hexane (1:20 to 1:2, v/v) to give ethyl 3-[2-(2,4-dichlorophenoxy)-6-ethoxypyridin-3-yl]propanoate (1.60 g, yield 98%) as a pale-yellow oil. ¹H-NMR (300 MHz, CDCl₃) δ:1.17 (3 H, t, J = 7.0 Hz) 1.24 (3 H, t, J = 7.1 Hz) 2.71 (2 H, t, J = 7.6 Hz) 2.98 (2 H, t, J = 7.4 Hz) 3.92 (2 H, q, J = 7.0 Hz) 4.12 (2 H, q, J = 7.1 Hz) 6.34 (1 H, d, J = 8.0 Hz) 7.13 (1 H, d, J = 8.5 Hz) 7.20 - 7.27 (1 H, m) 7.40 - 7.51 (2 H, m).

### Reference Example 247

To a solution of ethyl 3-[2-(2,4-dichlorophenoxy)-6-ethoxypyridin-3-yl]propanoate (1.60 g) in tetrahydrofuran (40 ml) was added lithium aluminum hydride (10 ml) at 0°C, and the mixture was stirred at room temperature for 1 hr. Magnesium sulfate decahydrate (4.3 ml) was added to the reaction solution, and the mixture was stirred for 30 min. The resulting insoluble substance was removed by filtration. The filtrate was concentrated, and the obtained residue was subjected to silica gel column chromatography and eluted with ethyl acetate-hexane (1:25 to 2:1, v/v) to give 3-[2-(2,4-dichlorophenoxy)-6-ethoxypyridin-3-yl]propan-1-ol (1.22 g, yield 92%) as a colorless oil. ¹H-NMR (300 MHz, CDCl₃) δ:1.18 (3 H, t, J = 7.1 Hz), 1.43 (1 H, t, J = 5.6 Hz), 1.85 - 1.99 (2 H, m), 2.70 - 2.82 (2 H, m), 3.71 (2 H, q, J = 6.0 Hz), 3.94 (2 H, q, J = 7.1 Hz), 6.37 (1 H, d, J = 7.9 Hz), 7.08 - 7.15 (1 H, m), 7.19 - 7.27 (1 H, m), 7.42 - 7.47 (2 H, m).

### Reference Example 248

To a solution of ethyl (2E)-3-[2-{[3-chloro-5-(trifluoromethyl)pyridin-2-yl]oxy}-4-(methoxymethoxy)phenyl]acrylate (10.0 g) in tetrahydrofuran (200 ml) was added 10% palladium-carbon (1.00 g), and the mixture was stirred under a hydrogen atmosphere for 1 hr. The reaction mixture was filtered, and the filtrate was concentrated. To a solution of the residue in tetrahydrofuran (200 ml) was added concentrated hydrochloric acid (3.0 ml), and the mixture was stirred at 60°C for 3 hr. A 1N aqueous sodium hydroxide solution (36.5 ml) was added to the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:9, v/v) to give ethyl 3-(2-{[3-chloro-5-(trifluoromethyl)pyridin-2-yl]oxy}-4-hydroxyphenyl)propanoate as a colorless oil (5.5 g, yield 61%). ¹H-NMR (300 MHz, CDCl₃) δ:1.21 (3 H, t, J = 7.2 Hz), 2.51 - 2.62 (2 H, m), 2.75 (2 H, t, J = 7.6 Hz), 4.09 (2 H, q, J = 7.2 Hz), 5.35 (1 H, s), 6.60 (1 H, d, J = 2.5 Hz), 6.69 (1 H, dd, J = 8.3, 2.5 Hz), 7.17 (1 H, d, J = 8.3 Hz), 7.99 (1 H, d, J = 2.3 Hz), 8.23 - 8.30 (1 H, m).

### Reference Example 249

To a mixture of ethyl 3-(2-{[3-chloro-5-(trifluoromethyl)pyridin-2-yl]oxy}-4-hydroxyphenyl)propanoate (2.5 g), 2-isopropoxyethanol (0.9 ml), tributylphosphine (3.2 ml) and tetrahydrofuran (100 ml) was added 1,1'-azodicarbonyldipiperidine (3.2 g) at room temperature, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:10, v/v) to give ethyl 3-[2-{[3-chloro-5-(trifluoromethyl)pyridin-2-yl]oxy}-4-(2-isopropoxyethoxy)phenyl]propanoate as a colorless oil (2.3 g, yield 75%). ¹H-NMR (300 MHz, CDCl₃) δ:1.13 - 1.25 (9 H, m), 2.51 - 2.62 (2 H, m), 2.76 (2 H, t, J = 7.7Hz), 3.61-3.79 (3 H, m), 4.02 - 4.12 (2 H, m), 6.68 (1 H, d, J = 2.5 Hz), 6.81 (1 H, dd, J = 8.7, 2.5 Hz), 7.22 (1 H, d, J = 8.7 Hz), 7.98 (1 H, d, J = 2.3 Hz), 8.23 - 8.29 (1 H, m).

### Reference Example 250

To a solution of ethyl 3-[2-{[3-chloro-5-(trifluoromethyl)pyridin-2-yl]oxy}-4-(2-isopropoxyethoxy)phenyl]propanoate (2.1 g) in tetrahydrofuran (100 ml) was added lithium aluminum hydride (169 mg) at room temperature, and the mixture was stirred for 30 min. To this reaction mixture was added sodium sulfate decahydrate (1.4 g), and the mixture was filtered through celite and concentrated. The residue was subjected to silica gel column chromatography and eluted with ethyl acetate-hexane (1:5, v/v) to give 3-[2-{[3-chloro-5-(trifluoromethyl)pyridin-2-yl]oxy}-4-(2-isopropoxyethoxy)phenyl]propan-1-ol as a colorless oil (1.2 g, yield 63%). ¹H-NMR (300 MHz, CDCl₃)δ:1.18 (6 H, d, J = 6.0 Hz), 1.44 (1 H, brs), 1.75 - 1.90 (2 H, m), 2.48 - 2.60 (2 H, m), 3.54 - 3.79 (5 H, m), 4.02 - 4.11 (2 H, m), 6.66 (1 H, d, J = 2.5 Hz), 6.83 (1 H, dd, J = 8.7, 2.6 Hz), 7.21 (1 H, d, J = 8.7 Hz), 7.98 (1 H, d, J = 1.9 Hz), 8.21 - 8.30 (1 H, m).

### Reference Example 251

To a solution of ethyl 3-(2-hydroxy-4-isopropoxyphenyl)propanoate (1.51 g) in N,N-dimethylformamide (30 ml) was added sodium hydride (288 mg, 60% in oil) at room temperature, and the mixture was stirred for 30 min. To this reaction mixture was added 2,4-dichlorobenzyl chloride (1.0 ml), and the mixture was stirred for 15 min. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:7, v/v) to give ethyl 3-{2-[(2,4-dichlorobenzyl)oxy]-4-isopropoxyphenyl}propanoate as a pale-yellow oil (2.2 g, yield 91%). ¹H-NMR (300 MHz, CDCl₃)δ:1.23 (3 H, t, J = 7.2 Hz), 1.32 (6 H, d, J = 6.0 Hz), 2.53 - 2.65 (2 H, m), 2.87 - 2.99 (2 H, m), 4.11 (2 H, q, J = 7.2 Hz), 4.43 - 4.57 (1 H, m), 5.09 (2 H, s), 6.39 - 6.49 (2 H, m), 7.06 (1 H, d, J = 8.3 Hz), 7.21 - 7.33 (1 H, m), 7.36 - 7.45 (1 H, m), 7.52 (1 H, d, J = 8.3 Hz).

### Reference Example 252

To a solution of ethyl 3-{2-[(2,4-dichlorobenzyl)oxy]-4-isopropoxyphenyl}propanoate (2.11 g) in tetrahydrofuran (100 ml) was added lithium aluminum hydride (195 mg) at room temperature, and the mixture was stirred for 20 min. To this reaction mixture was added sodium sulfate decahydrate (1.65 g), and the mixture was filtered through celite and concentrated. The residue was subjected to silica gel column chromatography and eluted with ethyl acetate-hexane (1:3, v/v) to give 3-{2-[(2,4-dichlorobenzyl)oxy]-4-isopropoxyphenyl}propan-1-ol as a colorless oil (1.42 g, yield 75%). Recrystallization from ethyl acetate-hexane gave colorless prism crystals. melting point 75.5 - 76.5°C.

### Reference Example 253

To a solution of ethyl 3-[2-hydroxy-4-(2-methoxyethoxy)phenyl]propanoate (3.0 g) in N,N-dimethylformamide (50 ml) was added sodium hydride (537 mg, 60% in oil) at room temperature, and the mixture was stirred for 30 min. To this reaction mixture was added 2,4-dichlorobenzyl chloride (1.9 ml), and the mixture was stirred for 2 hr. A saturated aqueous ammonium chloride solution was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:7, v/v) to give ethyl 3-[2-[(2,4-dichlorobenzyl)oxy]-4-(2-methoxyethoxy)phenyl]propanoate as a colorless oil (4.7 g, yield 99%). ¹H-NMR (300 MHz, CDCl₃)δ:1.23 (3 H, t, J = 7.2 Hz), 2.60 (2 H, t, J = 7.7 Hz), 2.95 (2 H, t, J = 7.7 Hz), 3.44 (3 H, s), 3.69 - 3.78 (2 H, m), 4.05 - 4.18 (4 H, m), 5.09 (2 H, s), 6.45 (1 H, dd, J = 8.2, 2.3 Hz), 6.54 (1 H, d, J = 2.3 Hz), 7.08 (1 H, d, J= 8.3 Hz), 7.24 - 7.33 (1 H, m), 7.42 (1 H, d, J = 2.1 Hz), 7.50 (1 H, d, J = 8.3 Hz).

### Reference Example 254

To a solution of ethyl 3-[2-[(2,4-dichlorobenzyl)oxy]-4-(2-methoxyethoxy)phenyl]propanoate (4.6 g) in tetrahydrofuran (100 ml) was added lithium aluminum hydride (0.41 g) at room temperature, and the mixture was stirred for 15 min. To this reaction mixture was added sodium sulfate decahydrate (3.5 g), and the mixture was filtered through celite and concentrated. The residue was recrystallized from ethyl acetate-hexane to give 3-[2-[(2,4-dichlorobenzyl)oxy]-4-(2-methoxyethoxy)phenyl]propan-1-ol as colorless prism crystals (3.8 g, yield 90%). ¹H-NMR (300 MHz, CDCl₃) δ:1.47 (1 H, t, J = 5.7 Hz), 1.77 - 1.92 (2 H, m), 2.72 (2 H, t, J = 7.4 Hz). 3.45 (3 H, s), 3.57 - 3.68 (2 H, m), 3.71 - 3.78 (2 H, m), 4.06 - 4.13 (2 H, m), 5.09 (2 H, s), 6.48 (1 H, dd, J = 8.3, 2.3 Hz), 6.56 (1 H, d, J = 2.3 Hz), 7.07 (1 H, d, J = 8.3 Hz), 7.24 - 7.33 (1 H, m), 7.42 (1 H, d, J = 2.1 Hz), 7.50 (1 H, d, J = 8.3 Hz).

### Reference Example 255

To a solution of ethyl 3-(2-hydroxy-4-isopropoxyphenyl)propanoate (5.1 g) in N,N-dimethylformamide (100 ml) was added sodium hydride (0.98 g, 60% in oil) at room temperature, and the mixture was stirred for 30 min. To this reaction mixture was added 4-(trifluoromethyl)benzyl chloride (4.7 g), and the mixture was stirred for 2 hr. A saturated aqueous ammonium chloride solution was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:30, v/v) to give ethyl 3-(4-isopropoxy-2-{[4-(trifluoromethyl)benzyl]oxy}phenyl)propanoate as a colorless oil (6.8 g, yield 81%). ¹H-NMR (300 MHz, CDCl₃) δ:1.22 (3 H, t, J = 7.2 Hz), 1.30 (6 H, d, J = 6.0 Hz), 2.54 - 2.64 (2 H, m), 2.94 (2 H, t, J = 7.7 Hz), 4.11 (2 H, q, J = 7.2 Hz), 4.41 - 4.55 (1 H, m), 5.11 (2 H, s), 6.40 - 6.48 (2 H, m), 7.02 - 7.11 (1 H, m), 7.51 - 7.58 (2 H, m), 7.61 - 7.70 (2 H, m).

### Reference Example 256

To a solution of ethyl 3-(4-isopropoxy-2-{[4-(trifluoromethyl)benzyl]oxy}phenyl)propanoate (6.7 g) in tetrahydrofuran (300 ml) was added lithium aluminum hydride (0.68 g) at 0°C, and the mixture was stirred for 30 min. To this reaction mixture was added sodium sulfate decahydrate (5.8 g), and the mixture was filtered through celite and concentrated. The residue was subjected to silica gel column chromatography and eluted with ethyl acetate-hexane (1:4, v/v) to give 3-(4-isopropoxy-2-{ [4-(trifluoromethyl)benzyl]oxy}phenyl)propan-1-ol as a colorless oil (6.0 g, yield 99%). ¹H-NMR (300 MHz, CDCl₃)δ:1.31 (6 H, d, J = 6.0 Hz), 1.52 (1 H, t, J = 5.8 Hz), 1.77 - 1.92 (2 H, m), 2.71 (2 H, t, J = 7.4 Hz), 3.55 - 3.68 (2 H, m), 4.42 - 4.56 (1 H, m), 5.10 (2 H, s), 6.43 - 6.51 (2 H, m), 7.02 - 7.10 (1 H, m), 7.50 - 7.60 (2 H, m), 7.61 - 7.70 (2 H, m).

### Reference Example 257

To a solution of ethyl 3-[2-hydroxy-4-(2-methoxyethoxy)phenyl]propanoate (3.0 g) in N,N-dimethylformamide (50 ml) was added sodium hydride (537 mg, 60% in oil) at room temperature, and the mixture was stirred for 30 min. To this reaction mixture was added 4-(trifluoromethyl)benzyl chloride (2.0 ml), and the mixture was stirred for 2 hr. A saturated aqueous ammonium chloride solution was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:7, v/v) to give ethyl 3-(4-(2-methoxyethoxy)-2-{[4-(trifluoromethyl)benzyl]oxy}phenyl)propanoate as a colorless oil (4.3 g, yield 90%). Recrystallization from ethyl acetate-hexane gave colorless prism crystals. ¹H-NMR (300 MHz, CDCl₃)δ:1.22 (3 H, t, J = 7.1 Hz), 2.50 - 2.69 (2 H, m), 2.94 (2 H, t, J = 7.7 Hz), 3.44 (3 H, s), 3.68 - 3.76 (2 H, m), 4.04 - 4.16 (4 H, m), 5.11 (2 H, s), 6.44 (1 H, dd, J = 8.3, 2.5 Hz), 6.52 (1 H, d, J = 2.5 Hz), 7.08 (1 H, d, J = 8.3 Hz), 7.50 - 7.58 (2 H, m), 7.61 - 7. 69 (2 H, m).

### Reference Example 258

To a solution of ethyl 3-(4-(2-methoxyethoxy)-2-{[4-(trifluoromethyl)benzyl]oxy}phenyl)propanoate (4.2 g) in tetrahydrofuran (100 ml) was added lithium aluminum hydride (0.37 g) at room temperature, and the mixture was stirred for 30 min. To this reaction mixture was added sodium sulfate decahydrate (3.2 g), and the mixture was filtered through celite and concentrated. The residue was subjected to silica gel column chromatography and eluted with ethyl acetate-hexane (1:1, v/v) to give 3-(4-(2-methoxyethoxy)-2-{[4-(trifluoromethyl)benzyl]oxy}phenyl)propan-1-ol as a colorless oil (3.7 g, yield 98%). ¹H-NMR (300 MHz, CDCl₃)δ:1.49 (1 H, t, J = 5.8 Hz), 1.78 - 1.92 (2 H, m), 2.72 (2 H, t, J = 7.4 Hz). 3.44 (3 H, s), 3.57 - 3.67 (2 H, m), 3.69 - 3.77 (2 H, m), 4.05 - 4.12 (2 H, m), 5.10 (2 H, s), 6.48 (1 H, dd, J = 8.2, 2.5 Hz), 6.54 (1 H, d, J = 2.5 Hz), 7.07 (1 H, d, J = 8.1 Hz), 7.50 - 7.58 (2 H, m), 7.61 - 7.69 (2 H, m).

### Reference Example 259

To a mixture of ethyl 3-(2-hydroxy-4-{[5-(trifluoromethyl)pyridin-2-yl]oxy}phenyl)propanoate (1.5 g), 2-methoxyethanol (0.7 ml), tributylphosphine (2.6 ml) and tetrahydrofuran (50 ml) was added 1,1*'*-azodicarbonyldipiperidine (2.1 g) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:8, v/v) to give ethyl 3-(2-(2-methoxyethoxy)-4-{[5-(trifluoromethyl)pyridin-2-yl]oxy}phenyl)propanoate as a colorless oil (1.3 g, yield 75%). ¹H-NMR (300 MHz, CDCl₃)δ:1.25 (3 H, t, J = 7.1 Hz), 2.64 (2 H, t, J = 7.7 Hz), 2.96 (2 H, t, J = 7.7 Hz), 3.44 (3 H, s), 3.70 - 3.83 (2 H, m), 4.03 - 4.21 (4 H, m), 6.60 - 6.71 (2 H, m), 6.98 (1 H, d, J = 8.7 Hz), 7.19 (1 H, d, J = 7.7 Hz), 7.88 (1 H, dd, J = 8.7, 2.5 Hz), 8.41 - 8.49 (1 H, m).

### Reference Example 260

To a solution of ethyl 3-(2-(2-methoxyethoxy)-4-{[5-(trifluoromethyl)pyridin-2-yl]oxy}phenyl)propanoate (1.2 g) in tetrahydrofuran (30 ml) was added lithium aluminum hydride (110 mg) at room temperature, and the mixture was stirred for 15 min. To this reaction mixture was added sodium sulfate decahydrate (936 mg), and the mixture was filtered through celite and concentrated. The residue was subjected to silica gel column chromatography and eluted with ethyl acetate-hexane (1:3, v/v) to give 3-(2-(2-methoxyethoxy)-4-{[5-(trifluoromethyl)pyridin-2-yl]oxy}phenyl)propan-1-ol as a colorless oil (1.08 g, yield 100%). ¹H-NMR (300 MHz, CDCl₃)δ:1.77 - 1.90 (2 H, m), 2.79 (2 H, t, J = 6.9 Hz), 3.45 (3 H, s), 3.50 - 3.62 (2 H, m), 3.73 - 3.80 (2 H, m), 4.04 - 4.11 (2 H, m), 6.61 - 6.76 (2 H, m), 6.98 (1 H, d, J = 8.7 Hz), 7.19 (1 H, d, J = 8.1 Hz), 7.89 (1 H, dd, J = 8.7, 2.1 Hz), 8.42 - 8.49 (1 H, m).

### Reference Example 261

To a mixture of ethyl 3-(2-{[3-chloro-5-(trifluoromethyl)pyridin-2-yl]oxy}-4-hydroxyphenyl)propanoate (2.76 g), 2-(pyrrolidin-1-yl)ethanol (1.0 ml), tributylphosphine (3.5 ml) and tetrahydrofuran (200 ml) was added 1,1'-azodicarbonyldipiperidine (3.58 mg) at 60°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (3:1 to 5:1, v/v) to give a colorless oil. To a solution of the obtained oil in tetrahydrofuran (50 ml) was added lithium aluminum hydride (152 mg) at room temperature, and the mixture was stirred for 30 min. To this reaction mixture was added sodium sulfate decahydrate (1.29 g), and the mixture was filtered through celite and concentrated. The residue was subjected to silica gel column chromatography and eluted with ethyl acetate-hexane (1:2, v/v) to give 3-(2-{[3-chloro-5-(trifluoromethyl)pyridin-2-yl]oxy}-4-[2-(pyrrolidin-1-yl)ethoxy]phenyl)propan-1-ol as a colorless oil (1.21 g, yield 38%). ¹H-NMR (300 MHz, CDCl₃)δ:1.66 - 1.95 (7 H, m), 2.45 - 2.70 (6 H, m), 2.88 (2 H, t, J = 6.0 Hz), 3.59 (2 H, t, J = 6.3 Hz), 4.07 (2 H, t, J = 5. 9 Hz), 6.66 (1 H, d, J = 2.6 Hz), 6.82 (1 H, dd, J = 8.5, 2.6 Hz), 7.22 (1 H, d, J = 8.5 Hz), 7.98 (1 H, d, J = 1.9 Hz), 8.20 - 8.32 (1 H, m).

### Reference Example 262

To a solution of ethyl 3-(2-{[3-chloro-5-(trifluoromethyl)pyridin-2-yl]oxy}-4-hydroxyphenyl)propanoate (6.7 g) in N,N-dimethylformamide (50 ml) were added chlorotriisopropylsilane (3.7 ml) and imidazole (1.4 g), and the mixture was stirred at room temperature for 72 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20, v/v) to give ethyl 3-{2-{[3-chloro-5-(trifluoromethyl)pyridin-2-yl]oxy}-4-[(triisopropylsilyl)oxy]phenyl}propanoate as a colorless oil (9.3 g, yield 100%). ¹H-NMR (300 MHz, CDCl₃)δ:1.01 - 1.14 (18 H, m), 1.15 - 1.31 (6 H, m), 2.53 - 2.61 (2 H, m), 2.70 - 2.80 (2 H, m), 4.08 (2 H, q, J = 7.2 Hz), 6.64 (1 H, d, J = 2.5 Hz), 6.75 (1 H, dd, J = 8.3, 2.5 Hz), 7.14 (1 H, d, J = 8.3 Hz), 7.97 (1 H, d, J = 2.3 Hz), 8.19 - 8.26 (1 H, m).

### Reference Example 263

To a solution of ethyl 3-{2-{[3-chloro-5-(trifluoromethyl)pyridin-2-yl]oxy}-4-[(triisopropylsilyl)oxy]phenyl}propanoate (9.3 g) in tetrahydrofuran (250 ml) was added lithium aluminum hydride (650 mg) at room temperature, and the mixture was stirred for 20 min. To this reaction mixture was added sodium sulfate decahydrate (5.5 g), and the mixture was filtered through celite and concentrated. The residue was subjected to silica gel column chromatography and eluted with ethyl acetate-hexane (1:7, v/v) to give 3-{2-{[3-chloro-5-(trifluoromethyl)pyridin-2-yl]oxy}-4-[(triisopropylsilyl)oxy]phenyl}propan-1-ol as a colorless oil (6.4 g, yield 74%). ¹H-NMR (300 MHz, CDCl₃)δ: 0.99 - 1.15 (18 H, m), 1.14 - 1.32 (3 H, m), 1.40 (1 H, t, J = 5.7 Hz), 1.73 - 1.93 (2 H, m), 2.53 (2 H, t, J = 7.4 Hz), 3.52 - 3.65 (2 H, m), 6.62 (1 H, d, J = 2.5 Hz), 6.73 - 6.81 (1 H, m), 7.15 (1 H, d, J = 8.3 Hz), 7.97 (1 H, d, J = 1.7 Hz), 8.18 - 8.28 (1 H, m).

### Reference Example 264

To a mixture of 3-{2-{[3-chloro-5-(trifluoromethyl)pyridin-2-yl]oxy}-4-[(triisopropylsilyl)oxy]phenyl}propan-1-ol (6.3 g), methyl (3-hydroxy-1-methyl-1H-pyrazol-4-yl)acetate (2.1 g), tributylphosphine (6.3 ml) and tetrahydrofuran (250 ml) was added 1,1'-azodicarbonyldipiperidine (6.3 g) at room temperature, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:5, v/v) to give a colorless oil. To a solution of the obtained oil in tetrahydrofuran (200 ml) was added tetrabutylammonium fluoride (1.0M tetrahydrofuran solution, 13.0 ml) at room temperature, and the mixture was stirred for 15 min. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (2:3, v/v) to give methyl {3-[3-(2-{[3-chloro-5-(trifluoromethyl)pyridin-2-yl]oxy}-4-hydroxyphenyl)propoxy]-1-methyl-1H-pyrazol-4-yl}acetate as a brown oil (3.3 g, yield 75%). ¹H-NMR (300 MHz, CDCl₃)δ:1.90 - 2.05 (2 H, m), 2.57 (2 H, t, J = 7.4 Hz), 3.32 (2 H, s), 3.66 (3 H, s), 3.69 (3 H, s), 4.08 (2 H, t, J = 6.3 Hz), 6.57 (1 H, d, J = 2.5 Hz), 6.64 (1 H, dd, J = 8.3, 2.5 Hz), 7.10 (1 H, d, J = 8.3 Hz), 7.14 (1 H, s), 7.92 - 7.98 (1 H, m), 8.18 - 8.25 (1 H, m).

### Reference Example 265

To a solution of 2-(benzyloxy)-4-isopropoxybenzaldehyde (5.0 g) and bromo[2-(1,3-dioxolan-2-yl)ethyl]triphenylphosphorane (13.1 g) in N,N-dimethylformamide (50 ml) was added sodium hydride (1.2 g, 60% in oil) at room temperature, and the mixture was stirred for 2 hr. A saturated aqueous ammonium chloride solution was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:10, v/v) to give 2-{3-[2-(benzyloxy)-4-isopropoxyphenyl]prop-2-ene-1-yl}-1,3-dioxolane as a pale-yellow oil (6.5 g). 2-{3-[2-(Benzyloxy)-4-isopropoxyphenyl]prop-2-ene-1-yl}-1,3-dioxolane (2.8 g) was obtained from 2-(benzyloxy)-4-isopropoxybenzaldehyde (8.1 g) by a similar method. To a solution (100 ml) of the oil (9.0 g) obtained above in a mixed solvent of tetrahydrofuran-ethanol (1:1, v/v) was added 10% palladium-carbon (1.00 g), and the mixture was stirred under a hydrogen atmosphere for 2 hr. The reaction mixture was filtered, and the filtrate was concentrated. The residue was subjected to silica gel column chromatography and eluted with ethyl acetate-hexane (1:5, v/v) to give 2-[3-(1,3-dioxolan-2-yl)propyl]-5-isopropoxyphenol as a colorless oil (6.5 g, yield 96%). ¹H-NMR (300 MHz, CDCl₃)δ:1.31 (6 H, d, J = 6.0 Hz), 1.65 - 1.82 (4 H, m), 2.61 (2 H, t, J = 6.9 Hz), 3.82 - 4.11 (4 H, m), 4.40 - 4.55 (1 H, m), 4.88 - 4.95 (1 H, m), 6.16 (1 H, s), 6.36 - 6.46 (2 H, m), 6. 95 (1 H, d, J = 8.3 Hz).

### Reference Example 266

To a solution of 2-[3-(1,3-dioxolan-2-yl)propyl]-5-isopropoxyphenol (6.5 g) in N,N-dimethylformamide (150 ml) was added sodium hydride (1.3 g, 60% in oil) at room temperature, and the mixture was stirred for 20 min. To this reaction mixture was added 2,3-dichloro-5-(trifluoromethyl)pyridine (6.3 ml), and the mixture was stirred for 2 hr. A saturated aqueous ammonium chloride solution was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:9, v/v) to give 3-chloro-2-{2-[3-(1,3-dioxolan-2-yl)propyl]-5-isopropoxyphenoxy}-5-(trifluoromethyl)pyridine as a colorless oil (9.8 g, yield 90%). ¹H-NMR (300 MHz, CDCl₃)δ:1.32 (6 H, d, J = 6.2 Hz), 1.56 - 1.75 (4 H, m), 2.46 (2 H, t, J = 7.3 Hz), 3.73 - 3.97 (4 H, m), 4.41 - 4.56 (1 H, m), 4.79 (1 H, t, J = 4.3 Hz), 6.63 (1 H, d, J = 2.5 Hz), 6.77 (1 H, dd, J = 8.5, 2.5 Hz), 7.18 (1 H, d, J = 8.5 Hz), 7.97 (1 H, d, J = 2.1 Hz), 8.22 - 8.31 (1 H, m).

### Reference Example 267

To a solution of 3-chloro-2-{2-[3-(1,3-dioxolan-2-yl)propyl]-5-isopropoxyphenoxy}-5-(trifluoromethyl)pyridine (8.2 g) in tetrahydrofuran (150 ml) was added 1N hydrochloric acid (50 ml), and the mixture was stirred at 60°C for 6 hr. The reaction mixture was neutralized with a 1N aqueous sodium hydroxide solution (50 ml), and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:9, v/v) to give 4-(2-{[3-chloro-5-(trifluoromethyl)pyridin-2-yl]oxy}-4-isopropoxyphenyl)butanal as a colorless oil (7.0 g, yield 94%). ¹H-NMR (300 MHz, CDCl₃)δ:1.33 (6 H, d, J = 6.0 Hz), 1.83 - 1.97 (2 H, m), 2.37 - 2.53 (4 H, m), 4.41 - 4.56 (1 H, m), 6.62 (1 H, d, J = 2.5 Hz), 6.78 (1 H, dd, J = 8.5, 2.5 Hz), 7.17 (1 H, d, J = 8.5 Hz), 7.99 (1 H, d, J = 1.7 Hz), 8.22 - 8.29 (1 H, m), 9.71 (1 H, t, J = 1.6 Hz).

### Reference Example 268

To a solution (100 ml) of 4-(2-{[3-chloro-5-(trifluoromethyl)pyridin-2-yl]oxy}-4-isopropoxyphenyl)butanal (7.0 g) in a mixed solvent of tetrahydrofuran-ethanol (1:1, v/v) was added sodium borohydride (791 mg) at room temperature, and the mixture was stirred for 3 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:5, v/v) to give 4-(2-{[3-chloro-5-(trifluoromethyl)pyridin-2-yl]oxy}-4-isopropoxyphenyl)butan-1-ol as a colorless oil (6.2 g, yield 88%). ¹H-NMR (300 MHz, CDCl₃)δ:1.19 (1 H, t, J = 5.5 Hz), 1.32 (6 H, d, J = 6.0 Hz), 1.47 - 1.71 (4 H, m), 2.45 (2 H, t, J = 7.3 Hz), 3.54 - 3.67 (2 H, m), 4.41 - 4.56 (1 H, m), 6.62 (1 H, d, J = 2.5 Hz), 6.77 (1 H, dd, J = 8.5, 2.5 Hz), 7.18 (1 H, d, J = 8.5 Hz), 7.98 (1 H, d, J = 2.3 Hz), 8.23 - 8.31 (1 H, m).

### Reference Example 269

To a solution of ethyl 3-(3-ethoxy-1H-pyrazol-4-yl)propanoate (15.0 g) in N,N-dimethylformamide (70 ml) was added sodium hydride (3.12 g, 60% in oil) at room temperature, and the mixture was stirred for 30 min. To this reaction mixture was added 2,4-dichlorobenzyl chloride (10.8 ml), and the mixture was stirred for 1 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:7, v/v) to give ethyl 3-[1-(2,4-dichlorobenzyl)-3-ethoxy-1H-pyrazol-4-yl]propanoate as a colorless oil (22.3 g, yield 85%). ¹H-NMR (300 MHz, CDCl₃)δ:1.23 (3 H, t, J = 7.2 Hz), 1.36 (3 H, t, J = 7.1 Hz), 2.50 - 2.60 (2 H, m), 2.62 - 2.73 (2 H, m), 4.11 (2 H, q, J = 7.2 Hz), 4.21 (2 H, q, J = 7.1 Hz), 5.13 (2 H, s), 6.77 (1 H, d, J = 8.3 Hz), 7.05 (1 H, s), 7.17 (1 H, dd, J = 8.3, 2.1 Hz), 7.38 (1 H, d, J = 2.1 Hz).

### Reference Example 270

To a solution of ethyl 3-[1-(2,4-dichlorobenzyl)-3-ethoxy-1H-pyrazol-4-yl]propanoate (19.5 g) in tetrahydrofuran (300 ml) was added lithium aluminum hydride (2.0 g) at 0°C, and the mixture was stirred for 30 min. To this reaction mixture was added sodium sulfate decahydrate (16.9 g), and the mixture was filtered through celite and concentrated. The residue was subjected to silica gel column chromatography and eluted with ethyl acetate-hexane (1:2, v/v) to give 3-[1-(2,4-dichlorobenzyl)-3-ethoxy-1H-pyrazol-4-yl]propan-1-ol as a colorless oil (14.3 g, yield 83%). Recrystallization from ethyl acetate-hexane gave colorless prism crystals. melting point 58 - 59°C.

### Reference Example 271

To a mixture of 1-tert-butyl 4-ethyl 3-hydroxy-1H-pyrazole-1,4-dicarboxylate (25.6 g), 2-propanol (9.2 ml), tributylphosphine (50 ml) and tetrahydrofuran (350 ml) was added a 40% solution (91 ml) of diethyl azodicarboxylate in toluene at room temperature, and the mixture was stirred for 2 hr. The reaction mixture was concentrated. The residue was subjected to silica gel column chromatography and eluted with ethyl acetate-hexane (1:3 to 1:10, v/v) to give a colorless oil. To a solution of the obtained oil in tetrahydrofuran (200 ml) was added a 40% aqueous methylamine solution (100 ml) at room temperature, and the mixture was stirred for 1 hr. The reaction mixture was concentrated, and the residue was extracted with ethyl acetate. The ethyl acetate layer was washed with saturated brine, dried (MgSO₄) and concentrated. The residue was subjected to silica gel column chromatography and eluted with ethyl acetate-hexane (1:3, v/v) to give ethyl 3-isopropoxy-1H-pyrazole-4-carboxylate as a colorless oil (10.2 g, yield 52%). ¹H-NMR (300 MHz, CDCl₃)δ:1.33 (3 H, t, J = 7.1 Hz), 1.40 (6 H, d, J = 6.0 Hz), 4.27 (2 H, q, J = 7.1 Hz), 4.86 - 5.02 (1 H, m), 7.87 (1 H, m), 9.70 (1 H, brs).

### Reference Example 272

To a solution of ethyl 3-isopropoxy-1H-pyrazole-4-carboxylate (5.5 g) and [(2-iodoethoxy)methyl]benzene (9.4 g) in N,N-dimethylformamide (50 ml) was added potassium carbonate (5.0 g), and the mixture was stirred at 60°C for 3 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:5, v/v) to give ethyl 1-[2-(benzyloxy)ethyl]-3-isopropoxy-1H-pyrazole-4-carboxylate as a colorless oil (6.9 g, yield 75%). ¹H-NMR (300 MHz, CDCl₃)δ:1.32 (3 H, t, J = 7.2 Hz), 1.38 (6 H, d, J = 6.0 Hz), 3.78 (2 H, t, J = 5.1 Hz), 4.10 (2 H, t, J = 5.1 Hz), 4.25 (2 H, q, J = 7.2 Hz), 4.48 (2 H, s), 4.81 - 4.96 (1 H, m), 7.18 - 7.39 (5 H, m), 7.76 (1H, s).

### Reference Example 273

To a solution of ethyl 1-[2-(benzyloxy)ethyl]-3-isopropoxy-1H-pyrazole-4-carboxylate (6.8 g) in tetrahydrofuran (200 ml) was added lithium aluminum hydride (0.78 g), and the mixture was stirred at 50°C for 5 hr. To this reaction mixture was added sodium sulfate decahydrate (6.4 g), and the mixture was filtered through celite and concentrated. To a solution of the obtained oil in tetrahydrofuran (200 ml) was added manganese dioxide (30.0 g), and the mixture was stirred at room temperature for 8 hr. The reaction mixture was filtered through celite and concentrated. The residue was subjected to silica gel column chromatography and eluted with ethyl acetate-hexane (1:4 to 1:2, v/v) to give 1-[2-(benzyloxy)ethyl]-3-isopropoxy-1H-pyrazole-4-carbaldehyde as a yellow oil (4.2 g, yield 71%). ¹H-NMR (300 MHz, CDCl₃)δ:1.38 (6 H, d, J = 6.2 Hz), 3.78 (2 H, t, J = 5.0 Hz), 4.12 (2 H, t, J = 5.0 Hz), 4.49 (2 H, s), 4.85 - 5.02 (1 H, m), 7.15 - 7.40 (5 H, m), 7.78 (1 H, s), 9.73 (1 H, s).

### Reference Example 274

To a solution of p-toluenesulfonylmethyl isocyanide (7.55 g) in 1,2-dimethoxyethane (80 ml) was added potassium tert-butoxide (7.85 g) at -78°C, and the mixture was stirred for 30 min. To this reaction mixture was added a solution of 1-[2-(benzyloxy)ethyl]-3-isopropoxy-1H-pyrazole-4-carbaldehyde (10.1 g) in 1,2-dimethoxyethane (45 ml), and the mixture was stirred at room temperature for 1 hr. Methanol (125 ml) was added, and the mixture was heated under reflux for 2 hr. The reaction mixture was concentrated, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:4, v/v) to give {1-[2-(benzyloxy)ethyl]-3-isopropoxy-1H-pyrazol-4-yl}acetonitrile as a pale-yellow oil (6.82 g, yield 65%). ¹H-NMR (300 MHz, CDCl₃) δ:1.32 (6 H, d, J = 6.0 Hz), 3.41 (2 H, s), 3.75 (2 H, t, J = 5.2 Hz), 4.09 (2 H, t, J = 5.2 Hz), 4.48 (2 H, s), 4.74 - 4.88 (1 H, m), 7.20 - 7.39 (6 H, m).

### Reference Example 275

To a solution of 1-[2-(benzyloxy)ethyl]-3-isopropoxy-1H-pyrazol-4-yl}acetonitrile (6.8 g) in ethanol (130 ml) was added a 8N aqueous sodium hydroxide solution (15 ml), and the mixture was heated under reflux for 15 hr. The reaction mixture was cooled to room temperature, neutralized with 6N hydrochloric acid (20 ml) and concentrated, and the residence was extracted with ethyl acetate. The ethyl acetate layer was washed with saturated brine, dried (MgSO₄) and concentrated. To a solution of the residue in N,N-dimethylformamide (70 ml) were added iodomethane (2.9 ml) and potassium carbonate (4.8 g), and the mixture was stirred at 80°C for 1.5 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:5 to 1:3, v/v) to give methyl {1-[2-(benzyloxy)ethyl]-3-isopropoxy-1H-pyrazol-4-yl}acetate as a brown oil (7.2 g, yield 95%). ¹H-NMR (300 MHz, CDCl₃) δ:1.30 (6 H, d, J = 6.2 Hz), 3.38 (2 H, s), 3.69 (3 H, s), 3.76 (2 H, t, J = 5.4 Hz), 4.10 (2 H, t, J = 5.4 Hz), 4.47 (2 H, s), 4.71 - 4.85 (1 H, m), 7.20 - 7.37 (6 H, m).

### Reference Example 276

To a solution of methyl {1-[2-(benzyloxy)ethyl]-3-isopropoxy-1H-pyrazol-4-yl}acetate (7.1 g) in tetrahydrofuran (250 ml) was added lithium aluminum hydride (0.81 g) at 0°C, and the mixture was stirred for 30 min. To this reaction mixture was added sodium sulfate decahydrate (6.9 g), and the mixture was filtered through celite and concentrated. The residue was subjected to silica gel column chromatography and eluted with ethyl acetate-hexane (1:2 to 1:1, v/v) to give 2-{1-[2-(benzyloxy)ethyl]-3-isopropoxy-1H-pyrazol-4-yl}ethanol as a colorless oil (6.2 g, yield 95%). ¹H-NMR (300 MHz, CDCl₃)δ:1.33 (6 H, d, J = 6.0 Hz), 2.12 (1 H, t, J = 5.9 Hz), 2.61 (2 H, t, J = 6.0 Hz), 3.69 - 3.81 (4 H, m), 4.08 (2 H, t, J = 5.3 Hz), 4.47 (2 H, s), 4.75 - 4.90 (1 H, m), 7.13 (1 H, s), 7.18 - 7.39 (5 H, m).

### Reference Example 277

To a mixture of 2-{1-[2-(benzyloxy)ethyl]-3-isopropoxy-1H-pyrazol-4-yl}ethanol (3.0 g), 2,4-dichlorophenol (1.7 g), tributylphosphine (3.7 ml) and tetrahydrofuran (100 ml) was added 1,1'-azodicarbonyldipiperidine (3.7 g) at room temperature, and the mixture was stirred for 4 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:5, v/v) to give 1-[2-(benzyloxy)ethyl]-4-[2-(2,4-dichlorophenoxy)ethyl]-3-isopropoxy-1H-pyrazole as a colorless oil (3.7 g, yield 82%). ¹H-NMR (300 MHz, CDCl₃) δ:1.32 (6 H, d, J = 6.0 Hz), 2.85 (2 H, t, J = 6.9 Hz), 3.76 (2 H, t, J = 5.4 Hz), 4.03 - 4.15 (4 H, m), 4.46 (2 H, s), 4.73 - 4.89 (1 H, m), 6.86 (1 H, d, J = 8.9 Hz), 7.11 - 7.38 (8 H, m).

### Reference Example 278

To a solution of 2-{1-[2-(benzyloxy)ethyl]-3-isopropoxy-1H-pyrazol-4-yl}ethanol (3.0 g) in N,N-dimethylformamide (50 ml) was added sodium hydride (0.43 g, 60% in oil) at 0°C, and the mixture was stirred for 30 min. To this reaction mixture was added 2,3-dichloro-5-(trifluoromethyl)pyridine (1.6 ml), and the mixture was stirred for 3 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:7, v/v) to give 2-(2-{1-[2-(benzyloxy)ethyl]-3-isopropoxy-1H-pyrazol-4-yl}ethoxy)-3-chloro-5-(trifluoromethyl)pyridine as a pale-yellow oil (3.5 g, yield 73%). ¹H-NMR (300 MHz, CDCl₃)δ:1.32 (6 H, d, J = 6.2 Hz), 2.87 (2 H, t, J = 6.9 Hz), 3.76 (2 H, t, J = 5.4 Hz), 4.09 (2 H, t, J = 5.4 Hz), 4.46 (2 H, s), 4.53 (2 H, t, J = 6.9 Hz), 4.72 - 4.87 (1 H, m), 7.17 - 7.35 (6 H, m), 7.81 (1 H, d, J = 2.3 Hz), 8.27 - 8.33 (1 H, m).

### Reference Example 279

To a solution (120 ml) of 1-[2-(benzyloxy)ethyl]-4-[2-(2,4-dichlorophenoxy)ethyl]-3-isopropoxy-1H-pyrazole (3.5 g) in a mixed solvent of tetrahydrofuran-ethanol (1:1, v/v) was added 10% palladium-carbon (0.3 g), and the mixture was stirred under a hydrogen atmosphere for 1.5 hr. The reaction mixture was filtered, and the filtrate was concentrated. The residue was subjected to silica gel column chromatography and eluted with ethyl acetate-hexane (1:2, v/v) to give 2-{4-[2-(2,4-dichlorophenoxy)ethyl]-3-isopropoxy-1H-pyrazol-1-yl}ethanol as a colorless oil (2.8 g, yield 99%). ¹H-NMR (300 MHz, CDCl₃)δ:1.33 (6 H, d, J = 6.0 Hz), 2.85 (2 H, t, J = 6.6 Hz), 3.41 (1 H, t, J = 5.9 Hz), 3.87 - 3.96 (2 H, m), 3.98 - 4.05 (2 H, m), 4.10 (2 H, t, J = 6.7 Hz), 4.74 - 4.91 (1 H, m), 6.86 (1 H, d, J = 8.9 Hz), 7.16 (1 H, dd, J = 8.9, 2.5 Hz), 7.22 (1 H, s), 7.36 (1 H, d, J = 2.5 Hz).

### Reference Example 280

To a solution (120 ml) of 2-(2-{1-[2-(benzyloxy)ethyl]-3-isopropoxy-1H-pyrazol-4-yl}ethoxy)-3-chloro-5-(trifluoromethyl)pyridine (3.4 g) in a mixed solvent of tetrahydrofuran-ethanol (1:1, v/v) was added 10% palladium-carbon (0.3 g), and the mixture was stirred under a hydrogen atmosphere for 1.5 hr. The reaction mixture was filtered, and the filtrate was concentrated. The residue was subjected to silica gel column chromatography and eluted with ethyl acetate-hexane (1:2, v/v) to give 2-[4-(2-{[3-chloro-5-(trifluoromethyl)pyridin-2-yl]oxy}ethyl)-3-isopropoxy-1H-pyrazol-1-yl]ethanol as a pale-yellow oil (2.7 g, yield 98%). ¹H-NMR (300 MHz, CDCl₃)δ:1.32 (6 H, d, J = 6.0 Hz), 2.86 (2 H, t, J = 6.8 Hz), 3.40 (1 H, t, J = 6.0 Hz), 3.84 - 4.07 (4 H, m), 4.52 (2 H, t, J = 6.8 Hz), 4.72 - 4.89 (1 H, m), 7.19 (1 H, s), 7.84 (1 H, d, J = 2.1 Hz), 8.25 - 8.36 (1 H, m).

### Reference Example 281

A mixture of ethyl 3-isopropoxy-1H-pyrazole-4-carboxylate (4.5 g) and 1-[4-(trifluoromethyl)phenyl]methanamine (6.0 ml) was stirred at 190°C for 16 hr. The reaction mixture was cooled, and the precipitated crystals were washed with hexane to give colorless crystals (5.2 g). To a solution of the obtained crystals (2.6 g) in N,N-dimethylformamide (50 ml) were added ethyl bromoacetate (1.2 ml) and potassium carbonate (1.7 g) at room temperature, and the mixture was stirred for 5 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:2, v/v) to give ethyl [3-isopropoxy-4-({[4-(trifluoromethyl)benzyl]amino}carbonyl)-1H-pyrazol-1-yl]acetate as colorless crystals (1.8 g, yield 54%). ¹H-NMR (300 MHz, CDCl₃) δ:1.28 (3 H, t, J = 7.1 Hz), 1.36 (6 H, d, J = 6.2 Hz), 4.24 (2 H, q, J = 7.1 Hz), 4.65 (2 H, d, J = 5.8 Hz), 4.68 - 4.73 (2 H, m), 4.92 - 5.06 (1 H, m), 7.17 - 7.27 (1 H, m), 7.44 (2 H, d, J = 8.1 Hz), 7.59 (2 H, d, J = 8.1 Hz), 7.84 (1 H, s).

### Reference Example 282

To a solution (30 ml) of ethyl [3-isopropoxy-4-({[4-(trifluoromethyl)benzyl]amino}carbonyl)-1H-pyrazol-1-yl]acetate (1.4 g) in a mixed solvent of tetrahydrofuran-ethanol (1:1, v/v) was added sodium borohydride (174 mg), and the mixture was stirred at 45°C for 2 hr. Water was poured into the reaction mixture, and the mixture was extracted with ethyl acetate. The ethyl acetate layer was washed with saturated brine, dried (MgSO₄) and concentrated. The residue was recrystallized from ethyl acetate-hexane to give 1-(2-hydroxyethyl)-3-isopropoxy-N-[4-(trifluoromethyl)benzyl]-1H-pyrazole-4-carboxamide as colorless prism crystals (0.97 g, yield 78%). melting point 107 - 108°C.

### Reference Example 283

A mixture of ethyl 3-isopropoxy-1H-pyrazole-4-carboxylate (5.7 g) and 1-[4-(trifluoromethyl)phenyl]methanamine (7.6 g) was stirred at 185°C for 20 hr. The reaction mixture was cooled, and the precipitated crystals were washed with hexane to give colorless crystals (6.6 g). To a solution of the obtained crystals (3.1 g) in N,N-dimethylformamide (50 ml) were added methyl 3-bromopropanoate (1.3 ml) and potassium carbonate (1.7 g) at 50°C, and the mixture was stirred for 3 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:1, v/v) to give a colorless oil. To a solution (80 ml) of the obtained oil in a mixed solvent of tetrahydrofuran-ethanol (1:1, v/v) was added sodium borohydride (395 mg), and the mixture was stirred at 50°C for 3 hr. Water was poured into the reaction mixture, and the mixture was extracted with ethyl acetate. The ethyl acetate layer was washed with saturated brine, dried (MgSO₄) and concentrated. The residue was recrystallized from ethyl acetate-hexane to give 1-(3-hydroxypropyl)-3-isopropoxy-N-[4-(trifluoromethyl)benzyl]-1H-pyrazole-4-carboxamide as colorless prism crystals (1.4 g, yield 38%). ¹H-NMR (300 MHz, CDCl₃)δ:1.36 (6 H, d, J = 6.2 Hz), 1.95 - 2.08 (2 H, m), 2.66 (1 H, t, J = 5.3 Hz), 3.58 - 3.70 (2 H, m), 4.12 (2 H, t, J = 6.3 Hz), 4.65 (2 H, d, J = 5.8 Hz), 4.90 - 5.04 (1 H, m), 7.20 (1 H, t, J = 5.8 Hz), 7.44 (2 H, d, J = 8.1 Hz), 7.59 (2 H, d, J = 8.1 Hz), 7.81 (1 H, s).

### Reference Example 284

To a solution of methyl 1-ethyl-3-hydroxy-1H-pyrazole-5-carboxylate (17.0 g) and benzyl bromide (13.0 ml) in N,N-dimethylformamide (100 ml) was added potassium carbonate (16.6 g) at room temperature, and the mixture was stirred at 60°C for 1 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:9, v/v) to give methyl 3-(benzyloxy)-1-ethyl-1H-pyrazole-5-carboxylate as a colorless oil (17.0 g, yield 65%). ¹H-NMR (300 MHz, CDCl₃)δ:1.40 (3 H, t, J = 7.2 Hz), 3.85 (3 H, s), 4.47 (2 H, q, J = 7.2 Hz), 5.19 (2 H, s), 6.20 (1 H, s), 7.28 - 7.48 (5 H, m).

### Reference Example 285

To a solution of methyl 3-(benzyloxy)-1-ethyl-1H-pyrazole-5-carboxylate (16.6 g) in tetrahydrofuran (400 ml) was added lithium aluminum hydride (2.54 g) at 0°C, and the mixture was stirred for 30 min. To this reaction mixture was added sodium sulfate decahydrate (21.5 g), and the mixture was filtered through celite and concentrated. To a solution of the obtained oil in tetrahydrofuran (400 ml) was added manganese dioxide (45.0 g), and the mixture was stirred at room temperature for 14 hr. The reaction mixture was filtered through celite and concentrated. The residue was subjected to silica gel column chromatography and eluted with ethyl acetate-hexane (1:10, v/v) to give 3-(benzyloxy)-1-ethyl-1H-pyrazole-5-carbaldehyde as a colorless oil (10.0 g, yield 68%). ¹H-NMR (300 MHz, CDCl₃)δ:1.39 (3 H, t, J = 7.2 Hz), 4.43 (2 H, q, J = 7.2 Hz), 5.22 (2 H, s), 6.25 (1 H, s), 7.28 - 7.50 (5 H, m), 9.72 (1 H, s).

### Reference Example 286

To a solution of ethyl diethylphosphonoacetate (11.2 ml) in tetrahydrofuran (250 ml) was added sodium hydride (2.08 g, 60% in oil) at room temperature, and the mixture was stirred for 20 min. To this reaction mixture was added a solution of 3-(benzyloxy)-1-ethyl-1H-pyrazole-5-carbaldehyde (10.0 g) in tetrahydrofuran (50 ml), and the mixture was stirred for 30 min. Saturated aqueous ammonium chloride was poured into the reaction mixture, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:10, v/v) to give ethyl (2E)-3-[3-(benzyloxy)-1-ethyl-1H-pyrazol-5-yl] acrylate as a pale-yellow oil (11.9 g, yield 91%). ¹H-NMR (300 MHz, CDCl₃) δ:1.33 (3 H, t, J = 7.1 Hz), 1.40 (3 H, t, J = 7.3 Hz), 4.13 (2 H, q, J = 7.3 Hz), 4.26 (2 H, q, J = 7.1 Hz), 5.19 (2 H, s), 5.94 (1 H, s), 6.27 (1 H, d, J = 15.6 Hz), 7.27 - 7.53 (6 H, m).

### Reference Example 287

To a solution (200 ml) of ethyl (2E)-3-[3-(benzyloxy)-1-ethyl-1H-pyrazol-5-yl]acrylate (10.7 g) in a mixed solvent of tetrahydrofuran-ethanol (1:1, v/v) was added 10% palladium-carbon (1.1 g), and the mixture was stirred under a hydrogen atmosphere for 4 hr. The reaction mixture was filtered, and the filtrate was concentrated. The residue was recrystallized from ethyl acetate-hexane to give ethyl 3-(1-ethyl-3-hydroxy-1H-pyrazol-5-yl)propanoate as colorless crystals (7.6 g, yield 100%). melting point 91 - 92°C.

### Reference Example 288

To a solution of methyl 3-(benzyloxy)-1H-pyrazole-5-carboxylate (9.0 g) and 1-iodo-2-methylpropane (5.5 ml) in N,N-dimethylformamide (100 ml) was added potassium carbonate (8.1 g) at room temperature, and the mixture was stirred at 50°C for 3 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:9, v/v) to give methyl 3-(benzyloxy)-1-isobutyl-1H-pyrazole-5-carboxylate as a colorless oil (8.2 g, yield 73%). ¹H-NMR (300 MHz, CDCl₃)δ:0.88 (6 H, d, J = 6.8 Hz), 2.12 - 2.27 (1 H, m), 3.84 (3 H, s), 4.25 (2 H, d, J = 7.5 Hz), 5.19 (2 H, s), 6.21 (1 H, s), 7.22 - 7.48 (5 H, m).

### Reference Example 289

To a solution of methyl 3-(benzyloxy)-1-isobutyl-1H-pyrazole-5-carboxylate (8.1 g) in tetrahydrofuran (200 ml) solution was added lithium aluminum hydride (1.1 g) at room temperature, and the mixture was stirred for 30 min. To this reaction mixture was added sodium sulfate decahydrate (10.9 g), and the mixture was filtered through celite and concentrated. To a solution of the obtained oil in tetrahydrofuran (200 ml) was added manganese dioxide (30.0 g), and the mixture was stirred at room temperature for 30 hr. The reaction mixture was filtered through celite and concentrated. The residue was subjected to silica gel column chromatography and eluted with ethyl acetate-hexane (1:10, v/v) to give 3-(benzyloxy)-1-isobutyl-1H-pyrazole-5-carbaldehyde as a colorless oil (5.3 g, yield 73%). ¹H-NMR (300 MHz, CDCl₃)δ:0.88 (6 H, d, J = 6.8 Hz), 2.08 - 2.28 (1 H, m), 4.21 (2 H, d, J = 7.5 Hz), 5.22 (2 H, s), 6.25 (1 H, s), 7.19 - 7.52 (5 H, m), 9.72 (1 H, s).

### Reference Example 290

To a solution of ethyl diethylphosphonoacetate (6.0 ml) in tetrahydrofuran (200 ml) was added sodium hydride (1.04 g, 60% in oil) at room temperature, and the mixture was stirred for 20 min. To this reaction mixture was added a solution of 3-(benzyloxy)-1-isobutyl-1H-pyrazole-5-carbaldehyde (5.2 g) in tetrahydrofuran (30 ml), and the mixture was stirred for 30 min. Saturated aqueous ammonium chloride was poured into the reaction mixture, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:9, v/v) to give ethyl (2E)-3-[3-(benzyloxy)-1-isobutyl-1H-pyrazol-5-yl]acrylate as a colorless oil (6.2 g, yield 94%). ¹H-NMR (300 MHz, CDCl₃)δ:0.88 (6 H, d, J = 6.6 Hz), 1.33 (3 H, t, J = 7.2 Hz), 2.10 - 2.17 (1 H, m), 3.86 (2 H, d, J = 7.5 Hz), 4.26 (2 H, q, J = 7.2 Hz), 5.19 (2 H, s), 5.95 (1 H, s), 6.27 (1 H, d, J = 15.6 Hz), 7.23 - 7.53 (6 H, m).

### Reference Example 291

To a solution (200 ml) of ethyl (2E)-3-[3-(benzyloxy)-1-isobutyl-1H-pyrazol-5-yl]acrylate (6.2 g) in a mixed solvent of tetrahydrofuran-ethanol (1:1, v/v) was added 10% palladium-carbon (0.6 g), and the mixture was stirred under a hydrogen atmosphere for 2 hr. The reaction mixture was filtered, and the filtrate was concentrated. The residue was recrystallized from ethyl acetate-hexane to give ethyl 3-(3-hydroxy-1-isobutyl-1H-pyrazol-5-yl)propanoate as colorless crystals (3.7 g, yield 82%). melting point 102 - 103°C.

### Reference Example 292

To a solution of methyl 3-hydroxy-1H-pyrazole-5-carboxylate (34.4 g) and benzyl bromide (62.8 ml) in N,N-dimethylformamide (200 ml) was added potassium carbonate (73.2 g) at room temperature, and the mixture was stirred for 20 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 1:3, v/v) to give methyl 1-benzyl-3-(benzyloxy)-1H-pyrazole-5-carboxylate as a colorless oil (60.2 g, yield 77%). As a by-product, methyl 1-benzyl-5-(benzyloxy)-1H-pyrazole-3-carboxylate was obtained as colorless crystals. (12.7 g, yield 16%). ¹H-NMR (300 MHz, CDCl₃)δ:3.81 (3 H, s), 5.22 (2 H, s), 5.61 (2 H, s), 6.26 (1 H, s), 7.14 - 7.49 (10 H, m).

### Reference Example 293

To a solution of methyl 1-benzyl-3-(benzyloxy)-1H-pyrazole-5-carboxylate (29.9 g) in tetrahydrofuran (400 ml) was added lithium aluminum hydride (3.51 g) at 0°C, and the mixture was stirred for 1 hr. To this reaction mixture was added sodium sulfate decahydrate (29.8 g), and the mixture was filtered through celite and concentrated. To a solution of the obtained oil in tetrahydrofuran (350 ml) was added manganese dioxide (64.0 g), and the mixture was stirred at room temperature for 4 hr. The reaction mixture was filtered through celite and concentrated. The residue was subjected to silica gel column chromatography and eluted with ethyl acetate-hexane (1:19 to 1:10, v/v) to give 1-benzyl-3-(benzyloxy)-1H-pyrazole-5-carbaldehyde as a pale-yellow oil (15.0 g, yield 55%). ¹H-NMR (300 MHz, CDCl₃) δ:5.24 (2 H, s), 5.57 (2 H, s), 6.29 (1 H, s), 7.18 - 7.51 (10 H, m), 9.71 (1 H, s).

### Reference Example 294

To a solution of ethyl diethylphosphonoacetate (7.8 ml) in tetrahydrofuran (250 ml) was added sodium hydride (2.44 g, 60% in oil) at 0°C, and the mixture was stirred for 30 min. To this reaction mixture was added a solution of 1-benzyl-3-(benzyloxy)-1H-pyrazole-5-carbaldehyde (14.8 g) in tetrahydrofuran (50 ml), and the mixture was stirred for 1 hr. Saturated aqueous ammonium chloride was poured into the reaction mixture, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:15, v/v) to give ethyl (2E)-3-[1-benzyl-3-(benzyloxy)-1H-pyrazol-5-yl]acrylate as a colorless oil (14.3 g, yield 78%). ¹H-NMR (300 MHz, CDCl₃)δ:1.30 (3 H, t, J = 7.1 Hz), 4.22 (2 H, q, J = 7.1 Hz), 5.21 (2 H, s), 5.30 (2 H, s), 6.01 (1 H, s), 6.25 (1 H, d, J = 15.6 Hz), 7.03 - 7.55 (11 H, m).

### Reference Example 295

To a solution (200 ml) of ethyl (2E)-3-[1-benzyl-3-(benzyloxy)-1H-pyrazol-5-yl]acrylate (15.1 g) in a mixed solvent of tetrahydrofuran-ethanol (1:1, v/v) was added 10% palladium-carbon (1.5 g), and the mixture was stirred under a hydrogen atmosphere for 4 hr. The reaction mixture was filtered, and the filtrate was concentrated. The residue was recrystallized from ethyl acetate-hexane to give ethyl 3-(1-benzyl-3-hydroxy-1H-pyrazol-5-yl)propanoate as colorless needle crystals (9.8 g, yield 85%). melting point 128 - 129°C.

### Reference Example 296

To a suspension of sodium hydride (2.64 g, 60% in oil) in N,N-dimethylformamide (150 ml) was added ethyl 3-(3-isopropyl-1H-pyrazol-4-yl)propanoate (12.62 g) at room temperature, and the mixture was stirred for 15 min. To the reaction mixture was added 2,4-dichlorobenzyl chloride (11.73 g), and the mixture was stirred at 80°C for 1 hr. After cooling the reaction mixture, water was added, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:10 to 1:5 to 1:2, v/v) to give ethyl 3-[1-(2,4-dichlorobenzyl)-3-isopropyl-1H-pyrazol-4-yl]propanoate (18.3 g, yield 83%) as a pale-yellow oil. ¹H-NMR (300 MHz, CDCl₃)δ:1.23 (3 H, t, J = 7.2 Hz), 1.28 (6 H, d, J = 7.0 Hz), 2.48 - 2.58 (2 H, m), 2.76 (2 H, t, J = 7.5 Hz), 2.91 - 3.05 (1 H, m), 4.11 (2 H, q, J = 7.2 Hz), 5.28 (2 H, s), 6.70 (1 H, d, J = 8.1 Hz), 7.13 (1 H, s), 7.16 (1 H, dd, J = 8.3, 2.1 Hz), 7.38 (1 H, d, J = 2.3 Hz).

### Reference Example 297

To a solution of ethyl 3-[1-(2,4-dichlorobenzyl)-3-isopropyl-1H-pyrazol-4-yl]propanoate (10.91 g) in tetrahydrofuran (150 ml) was added lithium aluminum hydride (1.12 g) at 0°C, and the mixture was stirred at room temperature for 2 hr. To this reaction mixture was added sodium sulfate decahydrate (9.50 g), and the mixture was filtered through celite and concentrated. The residue was subjected to silica gel column chromatography and eluted with ethyl acetate-hexane (1:20 to 1:2 to 4:1, v/v) to give 3-[1-(2,4-dichlorobenzyl)-3-isopropyl-1H-pyrazol-4-yl]propan-1-ol (8.33 g, yield 86%) as white crystals. melting point 68 - 69°C.

### Reference Example 298

A mixture of dimethyl 5-hydroxyisophthalate (21.02 g), 2-iodopropane (10.98 ml) and potassium carbonate (15.20 g) in N,N-dimethylformamide (150 ml) was stirred at room temperature for 16 hr. Water was added to the reaction mixture, and the mixture was extracted with ethyl acetate. The ethyl acetate layer was washed with water and saturated brine and dried (MgSO₄). The residue was subjected to silica gel column chromatography and eluted with ethyl acetate-hexane (1:20 to 1:5, v/v) to give a pale-yellow oil. To a solution of the obtained oil in tetrahydrofuran (150 ml) was added lithium aluminum hydride (5.69 g) at 0°C, and the mixture was stirred for 2 hr. To this reaction mixture was added sodium sulfate decahydrate (48.33 g), and the mixture was filtered through celite and concentrated to give (5-isopropoxy-1,3-phenylene)dimethanol (8.33 g, yield 86%) as a colorless oil. ¹H-NMR (300 MHz, CDCl₃)δ:1.34 (6 H, d, J = 6.0 Hz), 1.73 (2 H, t, J = 6.0 Hz), 4.49 - 4.63 (1 H, m), 4.66 (4 H, d, J = 5.7 Hz), 6.83 (2 H, s), 6.92 (1 H, s).

### Reference Example 299

To a mixture of (5-isopropoxy-1,3-phenylene)dimethanol (11.2 g), triethylamine (23.8 ml), 4-dimethylaminopyridine (0.698 g) and dichloromethane (350 ml) was added tert-butyl(chloro)diphenylsilane (15.69 g) at room temperature, and the mixture was stirred for 12 hr. Saturated aqueous sodium hydrogencarbonate was added to the reaction mixture, and the mixture was extracted with ethyl acetate. The ethyl acetate layer was washed with water and saturated brine and dried (MgSO₄). The residue was subjected to silica gel column chromatography and eluted with ethyl acetate-hexane (1:20 to 1:5 to 1:1, v/v) to give [3-({[tert-butyl(diphenyl)silyl]oxy}methyl)-5-isopropoxyphenyl]methanol (10.74 g, yield 43%) as a colorless oil. ¹H-NMR (300 MHz, CDCl₃)δ:1.10 (9 H, s), 1.33 (6 H, d, J = 6.0 Hz), 4.45 - 4.60 (1 H, m), 4.63 (2 H, d, J = 6.0 Hz), 4.74 (2 H, s), 6.71 - 6.92 (3 H, m), 7.31 - 7.48 (6 H, m), 7.69 (4 H, dd, J = 7.7, 1.5 Hz).

### Reference Example 300

To a mixture of [3-({[tert-butyl(diphenyl)silyl]oxy}methyl)-5-isopropoxyphenyl]methanol (5.01 g), 2,4-dichlorophenol (1.87 g), tributylphosphine (7.18 ml) and tetrahydrofuran (150 ml) was added 1,1'-azodicarbonyldipiperidine (5.82 g) at room temperature, and the mixture was stirred at 50°C for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:50 to 1:5, v/v) to give a pale-yellow oil. To a solution of the obtained oil in tetrahydrofuran (50 ml) was added tetrabutylammonium fluoride (1.0M tetrahydrofuran solution, 30 ml) at room temperature, and the mixture was stirred for 12 hr. Saturated aqueous sodium hydrogencarbonate was added to the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 1:1, v/v) to give {3-[(2,4-dichlorophenoxy)methyl]-5-isopropoxyphenyl}methanol (3.52 g, yield 90%) as a pale-yellow solid. ¹H-NMR (300 MHz, CDCl₃)δ:1.33 (6 H, d, J = 6.0 Hz), 4.51 - 4.62 (1 H, m), 4.66 (2 H, d, J = 4.1 Hz), 5.09 (2 H, s), 6.81 - 6.92 (4 H, m), 6.94 - 6.99 (1 H, m), 7.08 - 7.17 (1 H, m), 7.37 (1 H, d, J = 2.5 Hz).

### Reference Example 301

To a solution (300 ml) of [3-({[tert-butyl(diphenyl)silyl]oxy}methyl)-5-isopropoxyphenyl]methanol (5.73 g) in a mixed solvent of tetrahydrofuran-toluene (1:1, v/v) was added manganese dioxide (26 g) at room temperature, and the mixture was stirred for 16 hr. The reaction mixture was filtered through celite and concentrated to give a pale-yellow solid. To a solution of ethyl diethylphosphonoacetate (3.54 g) in N,N-dimethylformamide (30 ml) was added sodium hydride (0.632 g, 60% in oil) at 0°C, and the mixture was stirred for 30 min. To this reaction mixture was added a solution of the above-mentioned resultant product in tetrahydrofuran (30 ml), and the mixture was stirred at room temperature for 16 hr. Water was poured into the reaction mixture, and the mixture was extracted with ethyl acetate. The ethyl acetate layer was washed with water and saturated brine, dried (MgSO₄) and concentrated to give a pale-yellow oil. A mixture (60 ml) of the obtained oil, 5% palladium-carbon (500 mg) and tetrahydrofuran-ethanol (1:1, v/v) was stirred at room temperature for 12 hr under a hydrogen atmosphere. The catalyst was filtered off, and the filtrate was concentrated. The residue was subjected to silica gel column chromatography and eluted with ethyl acetate-hexane (1:50 to 1:2, v/v) and concentrated to give ethyl 3-[3-({[tert-butyl(diphenyl)silyl]oxy}methyl)-5-isopropoxyphenyl]propanoate (4.82 g, yield 72%) as a pale-yellow oil. ¹H-NMR (300 MHz, CDCl₃)δ:1.09 (9 H, s), 1.23 (3 H, t, J = 7.1 Hz), 1.32 (6 H, d, J = 6.0 Hz), 2.49 - 2.65 (2 H, m), 2.78 - 2.96 (2 H, m), 4.12 (2 H, q, J = 7.1 Hz), 4.40 - 4.57 (1 H, m), 4.70 (2 H, s), 6.56 - 6.65 (1 H, m), 6.67 (1 H, s), 6.78 (1 H, dd, J = 2.1, 1.0 Hz), 7.29 - 7.47 (6 H, m), 7.59 - 7.76 (4 H, m).

### Reference Example 302

To a solution of ethyl 3-[3-({[tert-butyl(diphenyl)silyl]oxy}methyl)-5-isopropoxyphenyl]propanoate (4.82 g) in tetrahydrofuran (50 ml) was added tetrabutylammonium fluoride (1.0M tetrahydrofuran solution, 30 ml) at room temperature, and the mixture was stirred for 12 hr. Saturated aqueous sodium hydrogencarbonate was added to the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:5 to 1:1, v/v) to give ethyl 3-[3-(hydroxymethyl)-5-isopropoxyphenyl]propanoate (2.54 g, yield 99%) as a pale-yellow oil. ¹H-NMR (300 MHz, CDCl₃)δ:1.24 (3 H, t, J = 7.2 Hz), 1.33 (6 H, d, J = 6.0 Hz), 1.61 (1 H, t, J = 6.2 Hz), 2.53 - 2.68 (2 H, m), 2.91 (2 H, t, J = 7.8 Hz), 4.13 (2 H, q, J = 7.2 Hz), 4.47 - 4.60 (1 H, m), 4.63 (2 H, d, J = 5.7 Hz), 6.67 (1 H, d, J = 1.5 Hz), 6.72 - 6.80 (2 H, m).

### Reference Example 303

To a mixture of ethyl 3-[3-(hydroxymethyl)-5-isopropoxyphenyl]propanoate (2.54 g), 2,4-dichlorophenol (1.71 g), tributylphosphine (5.95 ml) and tetrahydrofuran (120 ml) was added 1,1'-azodicarbonyldipiperidine (4.82 g) at room temperature, and the mixture was stirred at 50°C for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:100 to 1:2, v/v) to give a pale-yellow oil. To a solution of the obtained oil in tetrahydrofuran (40 ml) was added at 0°C a 1.5M solution (20 ml) of diisobutylaluminum hydride in toluene, and the mixture was stirred at room temperature for 3 hr. To this reaction mixture was added sodium sulfate decahydrate (6.44 g), and the mixture was filtered through celite and concentrated. The obtained residue was subjected to silica gel column chromatography and eluted with ethyl acetate-hexane (1:20 to 1:1, v/v) to give 3-{3-[(2,4-dichlorophenoxy)methyl]-5-isopropoxyphenyl}propan-1-ol (2.34 g, yield 68%) as a pale-yellow oil. ¹H-NMR (300 MHz, CDCl₃)δ:1.33 (6 H, d, J = 6.0 Hz), 1.78 - 1.97 (2 H, m), 2.57 - 2.78 (2 H, m), 3.67 (2 H, t, J = 6.3 Hz), 4.42 - 4.66 (1 H, m), 5.07 (2 H, s), 6.69 (1 H, t, J = 1.9 Hz), 6.81 (2 H, d, J = 1.5 Hz), 6.87 (1 H, d, J = 8.7 Hz), 7.14 (1 H, dd, J = 8.9, 2.5 Hz), 7.38 (1 H, d, J = 2.6 Hz).

### Reference Example 304

A mixture of dimethyl 5-hydroxyisophthalate (21.02 g), benzyl bromide (12.49 ml) and potassium carbonate (15.20 g) in N,N-dimethylformamide (150 ml) was stirred at room temperature for 16 hr. Water was added to the reaction mixture, and the mixture was stirred for 1 hr. The resulting precipitate was filtered and dried to give a white powder. To a solution of the obtained powder in tetrahydrofuran (250 ml) was added lithium aluminum hydride (5.39 g) at 0°C, and the mixture was stirred for 2 hr. To this reaction mixture was added sodium sulfate decahydrate (45.75 g), and the mixture was filtered through celite and concentrated to give [5-(benzyloxy)-1,3-phenylene]dimethanol (22.60 g, yield 98%) as a colorless oil. ¹H-NMR (300 MHz, CDCl₃)δ:1.66 (2 H, t, J = 5.9 Hz), 4.68 (4 H, d, J = 5.7 Hz), 5.09 (2 H, s), 6.88 - 7.04 (3 H, m), 7.30 - 7.49 (5 H, m).

### Reference Example 305

To a mixture of [5-(benzyloxy)-1,3-phenylene]dimethanol (21.86 g), triethylamine (37.35 ml), 4-dimethylaminopyridine (1.10 g) and dichloromethane (450 ml) was added tert-butyl(chloro)diphenylsilane (24.60 g) at room temperature, and the mixture was stirred for 12 hr. Saturated aqueous sodium hydrogencarbonate was added to the reaction mixture, and the mixture was extracted with ethyl acetate. The ethyl acetate layer was washed with water and saturated brine and dried (MgSO₄). The residue was subjected to silica gel column chromatography and eluted with ethyl acetate-hexane (1:20 to 1:5 to 1:1, v/v) to give [3-(benzyloxy)-5-({[tert-butyl(diphenyl)silyl]oxy}methyl)phenyl]methanol. (19.20 g, yield 44%) as a colorless oil. ¹H-NMR (300 MHz, CDCl₃) δ:1.09 (9 H, s), 1.57 (1 H, t, J = 6.0 Hz), 4.64 (2 H, d, J = 5.8 Hz), 4.74 (2 H, s), 5.06 (2 H, s), 6.78 - 7.03 (3 H, m), 7.29 - 7.49 (11 H, m), 7.68 (4 H, dd, J = 7.8, 1.6 Hz).

### Reference Example 306

To a mixture of [3-(benzyloxy)-5-({[tert-butyl(diphenyl)silyl]oxy}methyl)phenyl]methanol (19.20 g), 2,4-dichlorophenol (6.52 g), tributylphosphine (24.8 ml) and tetrahydrofuran (400 ml) was added 1,1'-azodicarbonyldipiperidine (20.08 g) at room temperature, and the mixture was stirred at 50°C for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:50 to 1:5, v/v) to give ({3-(benzyloxy)-5-[(2,4-dichlorophenoxy)methyl]benzyl}oxy)(tert-butyl)diphenylsilane (21.16 g, yield 85%) as a pale-yellow oil. ¹H-NMR (300 MHz, CDCl₃)δ:1.08 (9 H, s), 4.74 (2 H, s), 5.06 (2 H, s), 5.09 (2 H, s), 6.83 (1 H, d, J = 8.8 Hz), 6.95 (3 H, s), 7.11 (1 H, dd, J = 8.8, 2.5 Hz), 7.31 - 7.48 (12 H, m), 7.60 - 7.73 (4 H, m).

### Reference Example 307

To a solution (20 ml) of ({3-(benzyloxy)-5-[(2,4-dichlorophenoxy)methyl]benzyl}oxy)(tert-butyl)diphenylsilane (6.28 g) in tetrahydrofuran was added tetrabutylammonium fluoride (1.0M tetrahydrofuran solution, 20 ml) at room temperature, and the mixture was stirred for 12 hr. Saturated aqueous sodium hydrogencarbonate was added to the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 1:1, v/v), and the eluate was concentrated. The obtained residue was crystallized from ethyl acetate-hexane to give {3-(benzyloxy)-5-[(2,4-dichlorophenoxy)methyl]phenyl}methanol (3.06 g, yield 79%) as white crystals. melting point 85 - 88°C.

### Reference Example 308

To a solution of dimethyl 5-hydroxyisophthalate (21.02 g) in N,N-dimethylformamide (150 ml) was added sodium hydride (4.2 g, 60% in oil) at 0°C, and the mixture was stirred for 1 hr. To the reaction mixture was added 2,3-dichloro-5-(trifluoromethyl)pyridine (22.68 g), and the mixture was stirred at room temperature for 2 hr. Water was added to the reaction mixture, and the mixture was extracted with ethyl acetate. The ethyl acetate layer was washed with water and saturated brine, dried (MgSO₄) and concentrated. The obtained residue was washed with diethyl ether-hexane to give a white powder. To a solution of the obtained powder in tetrahydrofuran (200 ml) was added lithium aluminum hydride (3.25 g) at 0°C, and the mixture was stirred for 2 hr. To this reaction mixture was added sodium sulfate decahydrate (27.58 g), and the mixture was filtered through celite and concentrated. The obtained residue was subjected to silica gel column chromatography and eluted with ethyl acetate-hexane (1:10 to 1:1 to 4:1, v/v) to give (5-{[3-chloro-5-(trifluoromethyl)pyridin-2-yl]oxy}-1,3-phenylene)dimethanol (11.57 g, yield 40%) as a colorless oil. ¹H-NMR (300 MHz, CDCl₃)δ:1.94 (2 H, t, J = 5.8 Hz), 4.74 (4 H, d, J = 5.1 Hz), 7.07 - 7.15 (2 H, m), 7.27 - 7.32 (1 H, m), 7.99 (1 H, d, J = 1.7 Hz), 8.25 (1 H, dd, J = 2.2, 1.0 Hz).

### Reference Example 309

To a mixture of (5-{[3-chloro-5-(trifluoromethyl)pyridin-2-yl]oxy}-1,3-phenylene)dimethanol (9.55 g), triethylamine (11.96 ml), 4-dimethylaminopyridine (0.354 g) and dichloromethane (150 ml) was added tert-butyl(chloro)diphenylsilane (7.86 g) at room temperature, and the mixture was stirred for 12 hr. Saturated aqueous sodium hydrogencarbonate was added to the reaction mixture, and the mixture was extracted with ethyl acetate. The ethyl acetate layer was washed with water and saturated brine and dried (MgSO₄). The residue was subjected to silica gel column chromatography and eluted with ethyl acetate-hexane (1:20 to 1:5 to 1:1, v/v) to give (3-({[tert-butyl(diphenyl)silyl]oxy}methyl)-5-{[3-chloro-5-(trifluoromethyl)pyridin-2-yl]oxy}phenyl)methanol (6.75 g, yield 41%) as a colorless oil. ¹H-NMR (300 MHz, CDCl₃)δ:1.08 (9 H, s), 1.68 (1 H, t, J = 5.7 Hz), 4.71 (2 H, d, J = 4.3 Hz), 4.79 (2 H, s), 7.01 - 7.14 (2 H, m), 7.19 (1 H, s), 7.31 - 7.48 (6 H, m), 7.60 - 7.75 (4 H, m), 7.98 (1 H, d, J = 1.9 Hz), 8.26 (1 H, dd, J = 2.2, 1.0 Hz).

### Reference Example 310

To a solution of (3-({[tert-butyl(diphenyl)silyl]oxy}methyl)-5-{[3-chloro-5-(trifluoromethyl)pyridin-2-yl]oxy}phenyl)methanol (6.75 g) in tetrahydrofuran (100 ml) was added thionyl chloride (2 ml) at room temperature, and the mixture was stirred at 80°C for 2 hr. The reaction mixture was cooled and excess thionyl chloride was evaporated. Saturated aqueous sodium hydrogencarbonate was added to the residue, and the mixture was extracted with ethyl acetate. The ethyl acetate layer was washed with water and saturated brine and dried (MgSO₄). The residue was subjected to silica gel column chromatography and eluted with ethyl acetate-hexane (1:20 to 1:2, v/v) to give a pale-yellow oil. A mixture of the obtained oil, sodium ethoxide (0.068 g) and ethanol (50 ml) was heated under reflux for 12 hr. The reaction mixture was cooled, and the solvent was evaporated. Water was added to the residue, and the mixture was extracted with ethyl acetate. The ethyl acetate layer was washed with water and saturated brine, dried (MgSO₄). The residue was subjected to silica gel column chromatography and eluted with ethyl acetate-hexane (1:100 to 1:2, v/v) to give 3-({[tert-butyl(diphenyl)silyl]oxy}methyl)-5-(ethoxymethyl)phenol (1.74 g, yield 47%) as a colorless oil. ¹H-NMR (300 MHz, CDCl₃)δ:1.09 (9 H, s), 1.24 (3 H, t, J = 6.9 Hz), 3.53 (2 H, q, J = 7.1 Hz), 4.44 (2 H, s), 4.72 (2 H, s), 4.78 (1 H, s), 6.72 (1 H, s), 6.76 - 6.86 (2 H, m), 7.31 - 7.48 (6 H, m), 7.61 - 7.75 (4 H, m).

### Reference Example 311

To a solution of 3-({[tert-butyl(diphenyl)silyl]oxy}methyl)-5-(ethoxymethyl)phenol (1.74 g) in N,N-dimethylformamide (30 ml) was added sodium hydride (166 mg, 60% in oil) at 0°C, and the mixture was stirred for 1 hr. To the reaction mixture was added 2,3-dichloro-5-(trifluoromethyl)pyridine (907 mg), and the mixture was stirred at room temperature for 3 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:20 to 1:5, v/v) to give a pale-yellow oil. To a solution of the obtained oil in tetrahydrofuran (15 ml) was added tetrabutylammonium fluoride (1.0M tetrahydrofuran solution, 8 ml) at room temperature, and the mixture was stirred for 12 hr. Saturated aqueous sodium hydrogencarbonate was added to the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:10 to 1:2, v/v), and the eluate was concentrated to give [3-{[3-chloro-5-(trifluoromethyl)pyridin-2-yl]oxy}-5-(ethoxymethyl)phenyl]methanol (0.91 g, yield 61%) as a pale-yellow oil. ¹H-NMR (300 MHz, CDCl₃)δ:1.25 (3 H, t, J = 7.1 Hz), 1.73 (1 H, s), 3.57 (2 H, q, J = 7.0 Hz), 4.54 (2 H, s), 4.7 4 (2 H, s), 7.11 (2 H, d, J = 5 . 5 Hz), 7.98 (1 H, d, J = 2.3 Hz), 8.26 (1 H, d, J = 1.0 Hz).

### Reference Example 312

To a mixture of [3-({[tert-butyl(diphenyl)silyl]oxy}methyl)-5-isopropoxyphenyl]methanol (6.52 g), ethyl 3-(3-hydroxy-1-phenyl-1H-pyrazol-5-yl)propanoate (3.90 g), tributylphosphine (9.34 ml) and tetrahydrofuran (150 ml) was added 1,1'-azodicarbonyldipiperidine (7.57 g) at room temperature, and the mixture was stirred at 50°C for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:50 to 1:5, v/v) to give a pale-yellow oil. To a solution of the obtained oil in tetrahydrofuran (40 ml) was added tetrabutylammonium fluoride (1.0M tetrahydrofuran solution, 17 ml) at room temperature, and the mixture was stirred for 12 hr. Saturated aqueous sodium hydrogencarbonate was added to the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 1:1, v/v), and the eluate was concentrated to give ethyl 3-(3-{[3-(hydroxymethyl)-5-isopropoxybenzyl]oxy}-1-phenyl-1H-pyrazol-5-yl)propanoate (2.90 g, yield 44%) as a pale-yellow solid. ¹H-NMR (300 MHz, CDCl₃)δ:1.23 (3 H, t, J = 7.2 Hz), 1.33 (6 H, d, J = 6.0 Hz), 1.68 (1 H, t, J = 6.1 Hz), 2.49 - 2.69 (2 H, m), 2.84 - 3.01 (2 H, m), 4.11 (2 H, q, J = 7.2 Hz), 4.49 - 4.64 (1 H, m), 4.67 (2 H, d, J = 5.7 Hz), 5.20 (2 H, s), 5.70 (1 H, s), 6.90 (2 H, d, J = 16.0 Hz), 6.96 - 7.06 (1 H, m), 7.30 - 7.51 (5 H, m).

### Reference Example 313

To a mixture (60 ml) of ({3-(benzyloxy)-5-[(2,4-dichlorophenoxy)methyl]benzyl}oxy)(tert-butyl)diphenylsilane (12.25 g), 10% palladium-carbon (6.10 g) and ethyl acetate-ethanol (1:2, v/v) was added formic acid (7.9 ml) at room temperature, and the mixture was stirred for 6 hr under a hydrogen atmosphere. The catalyst was filtered off, and the filtrate was concentrated. Saturated aqueous sodium hydrogencarbonate was added to the residue, and the mixture was extracted with ethyl acetate. The obtained residue was subjected to silica gel column chromatography and eluted with ethyl acetate-hexane (1:10 to 5:1, v/v), and the eluate was concentrated to give 3-({[tert-butyl(diphenyl)silyl]oxy}methyl)-5-[(2,4-dichlorophenoxy)methyl]phenol (1.50 g, yield 14%) as a pale-yellow solid. ¹H-NMR (300 MHz, CDCl₃)δ:1.09 (9 H, s), 4.72 (2 H, s), 4.80 (1 H, s), 5.08 (2 H, s), 6.72 - 6.89 (3 H, m), 6.93 (1 H, s), 7.13 (1 H, dd, J = 8.9, 2.5 Hz), 7.31 - 7.50 (7 H, m), 7.68 (4 H, dd, J = 7.8, 1.4 Hz).

### Reference Example 314

To a mixture of 3-({[tert-butyl(diphenyl)silyl]oxy}methyl)-5-[(2,4-dichlorophenoxy)methyl]phenol (0.75 g), ethanol (0.107 ml), tributylphosphine (0.872 ml) and tetrahydrofuran (15 ml) was added 1,1'-azodicarbonyldipiperidine (706 mg) at room temperature, and the mixture was stirred at 50°C for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:50 to 1:5, v/v) to give a pale-yellow oil. To a solution of the obtained oil in tetrahydrofuran (40 ml) was added tetrabutylammonium fluoride (1.0M tetrahydrofuran solution, 17 ml) at room temperature, and the mixture was stirred for 12 hr. Saturated aqueous sodium hydrogencarbonate was added to the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 1:1, v/v), and the eluate was concentrated to give {3-[(2,4-dichlorophenoxy)methyl]-5-ethoxyphenyl}methanol (0.30 g, yield 65%) as a pale-yellow solid. ¹H-NMR (300 MHz, CDCl₃)δ:1.41 (3 H, t, J = 7.0 Hz), 1.64 (1 H, t, J = 5.8 Hz), 4.05 (2 H, q, J = 7.0 Hz), 4.68 (2 H, d, J = 5.5 Hz), 5.10 (2 H, s), 6.81 - 6.95 (3 H, m), 6.99 (1 H, s), 7.14 (1 H, dd, J = 8.9, 2.6 Hz), 7.38 (1 H, d, J = 2.5 Hz).

### Reference Example 315

To a solution of 2-phenylethanethioamide (101.3 g) in toluene (1.4 1) was added diethyl bromomalonate (125 ml), and the mixture was heated under reflux for 1.5 hr. After cooling the mixture to room temperature, the supernatant was transferred to a different container and concentrated to give a brown solid. The obtained solid was washed with water, petroleum ether and diisopropyl ether to give ethyl 2-benzyl-4-hydroxy-1, 3-thiazole-5-carboxylate (55.2 g, yield 31%) as a pale-brown solid. The solid was dissolved in a suspension of activated carbon in ethyl acetate, and the mixture was stirred at room temperature for 1 hr, filtrated and concentrated to give a pale-yellow solid. The solid was washed with diisopropyl ether. Recrystallization from ethyl acetate-hexane gave pale-flesh-colored crystals. melting point 97.0 - 98.0°C.

### Reference Example 316

To a mixture of 3-[1-(2,4-dichlorobenzyl)-3-isopropoxy-1H-pyrazol-5-yl]propan-1-ol (1.00 g), ethyl 2-benzyl-4-hydroxy-1,3-thiazole-5-carboxylate (919 mg) and tetrahydrofuran (50 ml) were added 1,1'-azodicarbonyldipiperidine (1.47 g) and tributylphosphine (1.45 ml) at room temperature. The reaction mixture was stirred overnight at 50°C and concentrated. Diisopropyl ether was added to the residue, and the insoluble substance was removed by filtration. The filtrate was concentrated, and the obtained residue was subjected to silica gel column chromatography and eluted with ethyl acetate-hexane (3:17, v/v) to give ethyl 2-benzyl-4-{3-[1-(2,4-dichlorobenzyl)-3-isopropoxy-1H-pyrazol-5-yl]propoxy}-1,3-thiazole-5-carboxylate (1.45 g, yield 85%) as a brown oil. ¹H-NMR (300 MHz, CDCl₃)δ:1.26 (3 H, t, J = 7.2 Hz), 1.33 (6 H, d, J = 6.0 Hz), 2.01 - 2.12 (2 H, m), 2.66 - 2.75 (2 H, m), 4.16 (2 H, s), 4.20 (2 H, q, J = 7.2 Hz), 4.39 - 4.45 (2 H, m), 4.63 - 4.77 (1 H, m), 5.16 (2 H, s), 5.58 (1 H, s), 6.50 (1 H, d, J = 8.4 Hz), 7.06 (1 H, dd, J = 8.4, 2.4 Hz), 7.25 - 7.40 (6 H, m).

### Reference Example 317

To a solution of ethyl 2-benzyl-4-{3-[1-(2,4-dichlorobenzyl)-3-isopropoxy-1H-pyrazol-5-yl]propoxy}-1,3-thiazole-5-carboxylate (1.45 g) in tetrahydrofuran (50 ml) was added dropwise at 0°C a 1.0M solution (12.3 ml) of diisobutylaluminum hydride in hexane, and the mixture was stirred at the same temperature for 1 hr. Diluted hydrochloric acid was added to the reaction mixture, and the mixture was extracted with ethyl acetate. The extract was washed with saturated brine, dried (MgSO₄) and concentrated. The residue was subjected to silica gel column chromatography and eluted with ethyl acetate-hexane (2:3, v/v) to give (2-benzyl-4-{3-[1-(2,4-dichlorobenzyl)-3-isopropoxy-1H-pyrazol-5-yl]propoxy}-1,3-thiazol-5-yl)methanol (960 mg, yield 71%) as a pale-brown oil. ¹H-NMR (300 MHz, CDCl₃)δ:1.33 (6 H, d, J = 6.3 Hz), 1.94 - 2.06 (2 H, m), 2.56 - 2.64 (2 H, m), 3.61 - 3.69 (1 H, m), 4.16 (2 H, s), 4.27 - 4.34 (2 H, m), 4.55 (2 H, d, J = 6.0 Hz), 4.65 - 4.76 (1 H, m), 5.14 (2 H, s), 5.56 (1 H, s), 6.55 (1 H, d, J = 8.4 Hz), 7.09 (1 H, dd, J = 8.4, 2.1 Hz), 7.23 - 7.37 (6 H, m).

### Reference Example 318

A mixture of 2,6-dichloronicotinic acid (20.33 g) and potassium tert-butoxide (35.68 g) in 2-propanol (500 ml) was heated under reflux for 16 hr. After cooling, the reaction mixture was concentrated. The residue was dissolved in water, 5N hydrochloric acid was added to adjust to pH 1, and the mixture was extracted with ethyl acetate. The ethyl acetate layer was dried (MgSO₄) and concentrated to give pale-orange crystals. The obtained crude crystals were eluted with ethyl acetate-hexane (3:2, v/v) and recrystallized to give 2-chloro-6-isopropoxynicotinic acid as white crystals (4.51 g, yield 20%). melting point 173 - 174.5°C.

### Reference Example 319

To a solution of 2-chloro-6-isopropoxynicotinic acid (4.51 g) and N,N-dimethylformamide (0.155 ml) in tetrahydrofuran (50 ml) was added oxalyl chloride (2.01 ml) at 0°C, and the mixture was stirred at room temperature for 1 hr. The reaction mixture was concentrated. Methanol (30 ml) was added to the residue, and the mixture was stirred at room temperature for 30 min. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 1:5, v/v) to give methyl 2-chloro-6-isopropoxynicotinate as a pale-yellow oil (2.14 g, yield 45%). ¹H-NMR (300 MHz, CDCl₃) δ:1.36 (6 H, d, J = 6.2 Hz), 3.91 (3 H, s), 5.22 - 5.51 (1 H, m), 6.62 (1 H, d, J = 8.7 Hz), 8.10 (1 H, d, J = 8.7 Hz).

### Reference Example 320

To a solution (500 ml) of ethyl diethylphosphonoacetate (8.26 ml) in tetrahydrofuran was added sodium hydride (2.11 g, 60% in oil) at room temperature, and the mixture was stirred for 30 min. To this reaction mixture was added 3-(benzyloxy)-1-phenyl-1H-pyrazole-4-carbaldehyde (26.06 g) at room temperature, and the mixture was heated under reflux for 1.5 hr. After cooling, water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:10 to 1:4, v/v) to give a pale-yellow solid (18.50 g). A mixture of the obtained solid (18.50 g), 10% palladium-carbon (2.00 g), tetrahydrofuran (100 ml) and ethanol (100 ml) was stirred at room temperature for 16 hr under a hydrogen atmosphere. The catalyst was filtered off, and the filtrate was concentrated. The obtained residue was subjected to silica gel column chromatography and eluted with ethyl acetate-hexane (1:2 to 100:0, v/v). The obtained solid was recrystallized from ethyl acetate-hexane to give ethyl 3-(3-hydroxy-1-phenyl-1H-pyrazol-4-yl)propanoate as colorless crystals (6.68 g, yield 54%). melting point 116.5 - 117.5°C.

### Reference Example 321

To a solution of ethyl diethylphosphonoacetate (19.2 g) in N,N-dimethylformamide (75 ml) was added sodium hydride (3.43 g, 60% in oil) at room temperature, and the mixture was stirred for 30 min. To this reaction mixture was added a solution (75 ml) of 3-(benzyloxy)-1-methyl-1H-pyrazole-4-carbaldehyde (15.45 g) in tetrahydrofuran, and the mixture was stirred for 16 hr. Water was poured into the reaction mixture, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:10 to 2:1, v/v) to give ethyl (2E)-3-[3-(benzyloxy)-1-methyl-1H-pyrazol-4-yl]acrylate as white crystals (16.44 g, yield 80%). ¹H-NMR (300 MHz, CDCl₃) δ:1.29 (3 H, t, J = 7.2 Hz), 3.75 (3 H, s), 4.20 (2 H, q, J = 7.2 Hz), 5.31 (2 H, s), 6.31 (1 H, d, J = 16.01 Hz), 7.29 - 7.44 (4 H, m), 7.44 - 7.54 (3 H, m).

### Reference Example 322

A mixture of ethyl (2E)-3-[3-(benzyloxy)-1-methyl-1H-pyrazol-4-yl]acrylate (16.44 g), 10% palladium-carbon (1.65 g), tetrahydrofuran (150 ml) and ethanol (150 ml) was stirred at room temperature for 6 hr under a hydrogen atmosphere. The catalyst was filtered off, and the filtrate was concentrated. The obtained solid was washed with diethyl ether-hexane to give ethyl 3-(3-hydroxy-1-methyl-1H-pyrazol-4-yl)propanoate as colorless crystals (10.86 g, yield 95%). melting point 89 - 91°C.

### Example 1

To a solution of 2-[4-propyl-3-(quinolin-2-ylmethoxy)-1H-pyrazol-1-yl]ethanol (0.30 g), methyl (2-hydroxyphenyl)acetate (0.18 g) and tributylphosphine (0.48 ml) in tetrahydrofuran (30 ml) was added 1,1'-azodicarbonyldipiperidine (0.49 g), and the mixture was stirred overnight at room temperature. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:1, v/v), and the eluate was concentrated to give a yellow oil.

To a solution of the obtained oil in tetrahydrofuran (4 ml) and methanol (4 ml) was added 1N aqueous sodium hydroxide (2 ml), and the mixture was stirred overnight at room temperature. To the reaction mixture were added 1N hydrochloric acid (2 ml) and brine (10 ml), and the mixture was extracted with ethyl acetate (15 mlx2). The organic layer was washed with brine, dried (MgSO₄), filtrated and concentrated to give a pale-yellow oil (0.38 g). To a solution of the obtained oil in 1N aqueous sodium hydroxide (1.0 ml) and water (20 ml) was slowly added a solution of calcium chloride (60 mg) in water (0.3 ml), and the precipitated solid was collected by filtration, washed with water and dried to give (2-{2-[4-propyl-3-(quinolin-2-ylmethoxy)-1H-pyrazol-1-yl]ethoxy}phenyl)acetic acid 1/2 calcium salt as a yellow amorphous form (0.26 g, 58%). ¹H-NMR (300 MHz, DMSO-d₆) δ:0.87 (3 H, t, J = 7.1 Hz), 1.51 (2 H, sextet, J = 7.4 Hz), 2.29 (2 H, t, J = 7.5 Hz), 3.28 (2 H, s), 4.00 - 4.26 (4 H, m), 5.41 (2 H, s), 6.70 - 6.85 (2 H, m), 7.05 (1 H, t, J = 7.7 Hz), 7.19 (1 H, d, J = 7.2 Hz), 7.48 (1 H, s), 7.52 - 7.65 (2 H, m), 7.76 (1 H, dd, J = 8.6, 7.1 Hz), 7.90 - 8.06 (2 H, m), 8.33 (1 H, d, J = 8.4 Hz).

### Example 2

To a solution of 2-[4-propyl-3-(quinolin-2-ylmethoxy)-1H-pyrazol-l-yl]ethanol (0.30 g), methyl (1-ethyl-3-hydroxy-1H-pyrazol-4-yl)acetate (0.21 g) and tributylphosphine (0.48 ml) in tetrahydrofuran (30 ml) was added 1,1'-azodicarbonyldipiperidine (0.49 g), and the mixture was stirred overnight at room temperature. The reaction mixture was concentrated, diisopropyl ether was added, and the precipitated crystals were filtered off. The filtrate was concentrated, and the obtained residue was subjected to silica gel column and eluted with ethyl acetone-hexane (2:3, v/v) to give a colorless oil.

To a solution of the obtained oil in tetrahydrofuran (4 ml) and methanol (4 ml) was added 1N aqueous sodium hydroxide (2 ml), and the mixture was stirred overnight at room temperature. To the reaction mixture were added 1N hydrochloric acid (2 ml) and brine (10 ml), and the mixture was extracted with ethyl acetate (15 mlx2). The organic layer was washed with brine, dried (MgSO₄), filtrated and concentrated to give a pale-yellow oil (0.55 g). To a solution of the obtained oil in 1N aqueous sodium hydroxide (1.0 ml) and water (20 ml) was slowly added a solution of calcium chloride (60 mg) in water (0.3 ml), and the precipitated solid was collected by filtration, washed with water and dried to give (1-ethyl-3-{2-[4-propyl-3-(quinolin-2-ylmethoxy)-1H-pyrazol-1-yl]ethoxy}-1H-pyrazol-4-yl)acetic acid 1/2 calcium salt as a yellow amorphous form (0.14 g, yield 30%). ¹H-NMR (300 MHz, DMSO-d₆) δ:0.87 (3 H, t, J = 7.1 Hz), 1.23 (3 H, t, J = 6.9 Hz), 1.50 (2 H, sextet, J = 7.3 Hz), 2.28 (2 H, t, J = 7.4 Hz), 2.96 (2 H, s), 3.81 (2 H, q, J = 6.9 Hz), 4.10 - 4.35 (4 H, m), 5.40 (2 H, s), 7.34 (1 H, s), 7.40 (1 H, s), 7.52 - 7.68 (2 H, m), 7.76 (1 H, t, J = 7.7 Hz), 7.98 (2 H, t, J = 8.6 Hz), 8.37 (1 H, d, J = 8.7 Hz).

### Example 3

To a solution of 2-[4-propyl-3-(quinolin-2-ylmethoxy)-1H-pyrazol-1-yl]ethanol (0.30 g), methyl (3-ethyl-2-hydroxyphenyl)acetate (0.21 g) and tributylphosphine (0.48 ml) in tetrahydrofuran (30 ml) was added 1,1'-azodicarbonyldipiperidine (0.49 g), and the mixture was stirred overnight at room temperature. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:1, v/v) to give a colorless oil.

To a solution of the obtained oil in tetrahydrofuran (4 ml) and methanol (4 ml) was added 1N aqueous sodium hydroxide (2 ml), and the mixture was stirred overnight at room temperature. To the reaction mixture were added 1N hydrochloric acid (2 ml) and brine (10 ml), and the mixture was extracted with ethyl acetate (15 mlx2). The organic layer was washed with brine, dried (MgSO₄), filtrated and concentrated to give a pale-yellow oil (0.55 g). To a solution of the obtained oil in 1N aqueous sodium hydroxide (1.0 ml) and water (20 ml) was slowly added a solution of calcium chloride (60 mg) in water (0.3 ml), and the precipitated solid was collected by filtration, washed with water and dried to give (3-ethyl-2-{2-[4-propyl-3-(quinolin-2-ylmethoxy)-1H-pyrazol-1-yl]ethoxy}phenyl)acetic acid 1/2 calcium salt as a yellow amorphous form (0.16 g, yield 33%). ¹H-NMR (300 MHz, DMSO-d₆) δ:0.89 (3 H, t, J = 7.2 Hz), 0.91 (3 H, t, J = 7.5 Hz), 1.53 (2 H, sextet, J = 7.3 Hz), 2.18 (2 H, t, J = 7.4 Hz), 2.32 (2 H,t, J = 7.4 Hz), 3.34 (2 H, s), 3.93 - 4.25 (2 H, m), 5.42 (2 H, s), 6.82 - 6.95 (2 H, m), 7.06 - 7.16 (1 H, m), 7.42 (1 H, s), 7.53 - 7.82 (3 H, m), 7.97 (2 H, t, J = 6.8 Hz), 8.37 (1 H, d, J = 8.7 Hz).

### Example 4

To a solution of 3-[4-propyl-3-(quinolin-2-ylmethoxy)-1H-pyrazol-1-yl]propan-1-ol (0.40 g), methyl (3-ethyl-2-hydroxyphenyl)acetate (0.29 g) and tributylphosphine (0.61 ml) in tetrahydrofuran (30 ml) was added 1,1'-azodicarbonyldipiperidine (0.62 g), and the mixture was stirred overnight at room temperature. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:3, v/v) to give a colorless oil.

To a solution of the obtained oil in tetrahydrofuran (4 ml) and methanol (4 ml) was added 1N aqueous sodium hydroxide (1.5 ml), and the mixture was stirred overnight at room temperature. To the reaction mixture were added 1N hydrochloric acid (1.5 ml) and brine (10 ml), and the mixture was extracted with ethyl acetate (15 mlx2). The organic layer was washed with brine, dried (MgSO₄), filtrated and concentrated to give a pale-yellow oil (0.27 g). To a solution of the obtained oil in 1N aqueous sodium hydroxide (0.7 ml) and water (30 ml) was slowly added a solution of calcium chloride (34 mg) in water (0.3 ml), and the precipitated solid was collected by filtration, washed with water and dried to give (3-ethyl-2-{3-[4-propyl-3-(quinolin-2-ylmethoxy)-1H-pyrazol-1-yl]propoxy}phenyl)acetic acid 1/2 calcium salt as a colorless amorphous form (0.15 g, yield 24%). ¹H-NMR (300 MHz, DMSO-d₆) δ:0.96 (3 H, t, J = 7.3 Hz), 1.20 (3 H, t, J = 7.5 Hz), 1.57 (2 H, sextet, J = 7.5 Hz), 2.25 (2 H, quintet, J = 6.4 Hz), 2.40 (2 H, t, J = 7.5 Hz), 2.60 (2 H, q, J = 7.5 Hz), 3.62 (2 H, s), 3.70 (2 H, t, J = 5.8 Hz), 4.14 (2 H, t, J = 6.8 Hz), 5.53 (2 H, s), 6.97 - 7.16 (3 H, m), 7.45 - 7.84 (3 H, m), 8.02 - 8.17 (2 H, m).

### Example 5

To a solution of [1-propyl-3-(quinolin-2-ylmethoxy)-1H-pyrazol-5-yl]methanol (0.50 g), methyl (3-ethyl-2-hydroxyphenyl)acetate (0.36 g) and tributylphosphine (0.84 ml) in tetrahydrofuran (30 ml) was added 1,1'-azodicarbonyldipiperidine (0.85 g), and the mixture was stirred overnight at room temperature. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:1, v/v) to give a colorless oil.

To a solution of the obtained oil in tetrahydrofuran (6 ml) and methanol (6 ml) was added 1N aqueous sodium hydroxide (2.5 ml), and the mixture was stirred overnight at room temperature. To the reaction mixture were added 1N hydrochloric acid (2.5 ml) and brine (10 ml), and the mixture was extracted with ethyl acetate (15 mlx2). The organic layer was washed with brine, dried (MgSO₄), filtered and concentrated. The residue was subjected to silica gel column chromatography and eluted with ethyl acetate-hexane (2:1, v/v). The obtained crystals were recrystallized from hexane-ethyl acetate to give (3-ethyl-2-{[1-propyl-3-(quinolin-2-ylmethoxy)-1H-pyrazol-5-yl]methoxy}phenyl)acetic acid as colorless crystals (0.49 g, yield 65%). melting point 118 - 119°C.

### Example 6

To a solution of [1-propyl-5-(quinolin-2-ylmethoxy)-1H-pyrazol-3-yl]methanol (0.50 g), methyl (3-ethyl-2-hydroxyphenyl)acetate (0.36 g) and tributylphosphine (0.84 ml) in tetrahydrofuran (30 ml) was added 1,1'-azodicarbonyldipiperidine (0.85 g), and the mixture was stirred overnight at room temperature. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:2, v/v) to give a colorless oil.

To a solution of the obtained oil in tetrahydrofuran (6 ml) and methanol (6 ml) was added 1N aqueous sodium hydroxide (2.5 ml), and the mixture was stirred overnight at room temperature. To the reaction mixture were added 1N hydrochloric acid (2.5 ml) and brine (10 ml), and the mixture was extracted with ethyl acetate (15 mlx2). The organic layer was washed with brine, dried (MgSO₄), filtrated and concentrated. The residue was subjected to silica gel column chromatography and eluted with ethyl acetate-hexane (2:1, v/v) to give a colorless oil (0.55 g). To a solution of the obtained oil in 1N aqueous sodium hydroxide (1.5 ml) and water (20 ml) was slowly added a solution of calcium chloride (73 mg) in water (1 ml), and the precipitated solid was collected by filtration, washed with water and dried to give (3-ethyl-2-{[1-propyl-5-(quinolin-2-ylmethoxy)-1H-pyrazol-3-yl]methoxy}phenyl)acetic acid 1/2 calcium salt as a colorless amorphous form (0.55 g, yield 65%). ¹H-NMR (300 MHz, DMSO-d₆) δ:0.84 (3 H, t, J = 7.5 Hz), 1.03 (3 H, t, J = 7.6 Hz), 1.73 (2 H, sextet, J = 7.1 Hz), 2.40 - 2.57 (2 H, m), 3.92 (2 H, t, J = 6.9 Hz), 4.64 (2 H, s), 5.42 (2 H, s), 5.86 (1 H, s), 6.84 - 7.16 (3 H, m), 7.57 - 7.86 (3 H, m), 7.96 - 8.07 (2 H, m), 8.44 (1 H, d, J = 8.4Hz).

### Example 7

To a solution of [3-propoxy-1-(quinolin-2-ylmethyl)-1H-pyrazol-5-yl]methanol (0.50 g), methyl (3-ethyl-2-hydroxyphenyl)acetate (0.35 g) and tributylphosphine (0.84 ml) in tetrahydrofuran (30 ml) was added 1,1'-azodicarbonyldipiperidine (0.85 g), and the mixture was stirred overnight at room temperature. The reaction mixture was concentrated, diisopropyl ether was added, and the precipitated crystals were filtered off. The filtrate was concentrated, and the residue was subjected to silica gel column chromatography and eluted with ethyl acetate-hexane (1:2, v/v) to give a yellow oil (0.63 g).

To a solution of the obtained oil in tetrahydrofuran (4 ml) and methanol (4 ml) was added 1N aqueous sodium hydroxide (2 ml), and the mixture was stirred at 50°C for 2 hr. To the reaction mixture were added 1N hydrochloric acid (2 ml) and brine (10 ml), and the mixture was extracted with ethyl acetate (15 mlx2). The organic layer was washed with brine, dried (MgSO₄), filtered and concentrated. The obtained crude crystals were recrystallized from ethyl acetate to give (3-ethyl-2-{[3-propoxy-1-(quinolin-2-ylmethyl)-1H-pyrazol-5-yl]methoxy}phenyl)acetic acid as colorless crystals (0.47 g, yield 59%). melting point 110 - 111°C.

### Example 8

To a solution of [3-isopropoxy-1-(quinolin-2-ylmethyl)-1H-pyrazol-5-yl]methanol (0.80 g), methyl (2-hydroxy-3-methoxyphenyl)acetate (0.58 g) and tributylphosphine (1.34 ml) in tetrahydrofuran (50 ml) was added 1,1'-azodicarbonyldipiperidine (1.36 g), and the mixture was stirred at room temperature for 2 hr. The reaction mixture was concentrated, diisopropyl ether was added, and the precipitated crystals were filtered off. The filtrate was concentrated, and the obtained residue was subjected to silica gel column chromatography and eluted with ethyl acetate-hexane (2:3, v/v) to give a yellow oil (1.25 g).

To a solution of the obtained oil in tetrahydrofuran (6 ml) and methanol (6 ml) was added 1N aqueous sodium hydroxide (4.0 ml), and the mixture was stirred at 50°C for 1 hr. To the reaction mixture were added 1N hydrochloric acid (4.0 ml) and brine (10 ml), and the mixture was extracted with ethyl acetate (20 mlx2). The organic layer was washed with brine, dried (MgSO₄), filtered and concentrated. The residue was subjected to silica gel column chromatography and eluted with ethyl acetate-hexane (1:2, v/v). Recrystallization from ethyl acetate-hexane gave (2-{[3-isopropoxy-1-(quinolin-2-ylmethyl)-1H-pyrazol-5-yl]methoxy}-3-methoxyphenyl)acetic acid as colorless crystals (0.98 g, yield 78%). melting point 129 - 130°C.

### Example 9

To a solution of [1-isopropyl-5-(quinolin-2-ylmethoxy)-1H-pyrazol-3-yl]methanol (0.80 g), methyl (2-hydroxy-3-methoxyphenyl)acetate (0.58 g) and tributylphosphine (1.34 ml) in tetrahydrofuran (40 ml) was added 1,1'-azodicarbonyldipiperidine (1.36 g), and the mixture was stirred overnight at room temperature. The reaction mixture was concentrated, diisopropyl ether was added, and the precipitated crystals were filtered off. The filtrate was concentrated, and the obtained residue was subjected to silica gel column chromatography and eluted with ethyl acetate-hexane (2:3, v/v) to give a yellow oil (1.33 g).

To a solution of the obtained oil in tetrahydrofuran (6 ml) and methanol (6 ml) was added 1N aqueous sodium hydroxide (4.0 ml), and the mixture was stirred at 50°C for 1 hr. To the reaction mixture were added 1N hydrochloric acid (4.0 ml) and brine (10 ml), and the mixture was extracted with ethyl acetate (20 mlx2). The organic layer was washed with brine, dried (MgSO₄), filtered and concentrated. The residue was subjected to silica gel column chromatography and eluted with ethyl acetate-hexane (6:1, v/v). The obtained crude crystals were recrystallized from ethyl acetate to give (2-{[1-isopropyl-5-(quinolin-2-ylmethoxy)-1H-pyrazol-3-yl]methoxy}-3-methoxyphenyl)acetic acid as colorless crystals (0.98 g, yield 78%). melting point 84 - 85°C.

### Example 10

To a solution of 3-[3-isopropoxy-1-(quinolin-2-ylmethyl)-1H-pyrazol-5-yl]propan-1-ol (0.64 g), methyl (2-hydroxy-3-methoxyphenyl)acetate (0.43 g) and tributylphosphine (1.00 ml) in tetrahydrofuran (30 ml) was added 1,1'-azodicarbonyldipiperidine (1.00 g), and the mixture was stirred at room temperature for 2 hr. The reaction mixture was concentrated, diisopropyl ether was added, and the precipitated crystals were filtered off. The filtrate was concentrated, and the obtained residue was subjected to silica gel column chromatography and eluted with ethyl acetate-hexane (2:3, v/v) to give a yellow oil (1.00 g).

To a solution of the obtained oil in tetrahydrofuran (6 ml) and methanol (6 ml) was added 1N aqueous sodium hydroxide (3.0 ml), and the mixture was stirred at 50°C for 2 hr. To the reaction mixture were added 1N hydrochloric acid (3.0 ml) and brine (10 ml), and the mixture was extracted with ethyl acetate (15 mlx2). The organic layer was washed with brine, dried (MgSO₄), filtered and concentrated. The residue was subjected to silica gel column chromatography and eluted with ethyl acetate-hexane (4:1, v/v). Recrystallization from ethyl acetate-hexane gave (2-{3-[3-isopropoxy-1-(quinolin-2-ylmethyl)-1H-pyrazol-5-yl]propoxy}-3-methoxyphenyl)acetic acid as colorless crystals (0.70 g, yield 70%). melting point 129 - 130°C.

### Example 11

To a solution of 3-[3-propoxy-1-(quinolin-2-ylmethyl)-1H-pyrazol-5-yl]propan-1-ol (0.63 g), methyl (2-hydroxy-3-methoxyphenyl)acetate (0.43 g) and tributylphosphine (1.00 ml) in tetrahydrofuran (30 ml) was added 1,1'-azodicarbonyldipiperidine (1.00 g), and the mixture was stirred at room temperature for 1 hr. The reaction mixture was concentrated, diisopropyl ether was added, and the precipitated crystals were filtered off. The filtrate was concentrated, and the obtained residue was subjected to silica gel column chromatography and eluted with ethyl acetate-hexane (2:3, v/v) to give a yellow oil (0.97 g).

To a solution of the obtained oil in tetrahydrofuran (6 ml) and methanol (6 ml) was added 1N aqueous sodium hydroxide (3.0 ml), and the mixture was stirred at 50°C for 2 hr. To the reaction mixture were added 1N hydrochloric acid (3.0 ml) and brine (10 ml), and the mixture was extracted with ethyl acetate (15 mlx2). The organic layer was washed with brine, dried (MgSO₄), filtered and concentrated. The residue was subjected to silica gel column chromatography and eluted with ethyl acetate-hexane (4:1, v/v). Recrystallization from ethyl acetate-hexane gave (3-methoxy-2-{3-[3-propoxy-1-(quinolin-2-ylmethyl)-1H-pyrazol-5-yl]propoxy}phenyl)acetic acid as colorless crystals (0.74 g, yield 79%). melting point 97 - 98°C.

### Example 12

To a solution of [1-isopropyl-3-(quinolin-2-ylmethoxy)-1H-pyrazol-5-yl]methanol (0.80 g), methyl (2-hydroxy-3-methoxyphenyl)acetate (0.58 g) and tributylphosphine (1.3 ml) in tetrahydrofuran (50 ml) was added 1,1'-azodicarbonyldipiperidine (1.36 g), and the mixture was stirred at room temperature for 2 hr. The reaction mixture was concentrated, diisopropyl ether was added, and the precipitated crystals were filtered off. The filtrate was concentrated, and the obtained residue was subjected to silica gel column chromatography and eluted with ethyl acetate-hexane (2:3, v/v) to give a yellow oil (1.29 g).

To a solution of the obtained oil in tetrahydrofuran (6 ml) and methanol (6 ml) was added 1N aqueous sodium hydroxide (4.5 ml), and the mixture was stirred at 50°C for 1 hr. To the reaction mixture were added 1N hydrochloric acid (4.5 ml) and brine (10 ml), and the mixture was extracted with ethyl acetate (20 mlx2). The organic layer was washed with brine, dried (MgSO₄), filtered and concentrated. The obtained crude crystals were recrystallized from ethyl acetate to give {2-[1-isopropyl-3-(quinolin-2-ylmethoxy)-1H-pyrazol-5-ylmethoxy]-3-methoxyphenyl}acetic acid as colorless crystals (1.01 g, yield 81%). melting point 163 - 164°C.

### Example 13

To a solution of 3-[1-isopropyl-5-(quinolin-2-ylmethoxy)-1H-pyrazol-3-yl]propan-1-ol (0.59 g), methyl (2-hydroxy-3-methoxyphenyl)acetate (0.39 g) and tributylphosphine (0.90 ml) in tetrahydrofuran (30 ml) was added 1,1'-azodicarbonyldipiperidine (0.91 g), and the mixture was stirred at room temperature for 2 hr. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:1, v/v) to give a yellow oil (0.89 g).

To a solution of the obtained oil in tetrahydrofuran (6 ml) and methanol (6 ml) was added 1N aqueous sodium hydroxide (3.0 ml), and the mixture was stirred at 50°C for 1 hr. To the reaction mixture were added 1N hydrochloric acid (3.0 ml) and brine (10 ml), and the mixture was extracted with ethyl acetate (20 mlx2). The organic layer was washed with brine, dried (MgSO₄), filtered and concentrated. The residue was subjected to silica gel column chromatography and eluted with ethyl acetate-hexane (1:8, v/v) to give (2-{3-[1-isopropyl-5-(quinolin-2-ylmethoxy)-1H-pyrazol-3-yl]propoxy}-3-methoxyphenyl)acetic acid as a yellow oil (0.70 g, yield 79%). ¹H-NMR (300 MHz, CDCl₃) δ:1.46 (6 H, d, J = 7.0 Hz), 2.04 (2 H, quintet, J = 6.4 Hz), 2.76 (2 H, t, J = 6.6 Hz), 3.67 (2 H, s), 3.81 (3 H, s), 3.92 (2 H, t, J = 6.3 Hz), 4.57 (1 H, septet, J = 6.8 Hz), 5.43 (2 H, s), 5.47 (1 H, s), 6.77 - 7.04 (3 H, m), 7.53 - 7.90 (4 H, m), 8.12 (1 H, d, J = 8.8 Hz), 8.24 (1 H, d, J = 8.4 Hz).

### Example 14

To a solution of 3-[1-isopropyl-3-(quinolin-2-ylmethoxy)-1H-pyrazol-5-yl]propan-1-ol (0.60 g), methyl (2-hydroxy-3-methoxyphenyl)acetate (0.40 g) and tributylphosphine (0.92 ml) in tetrahydrofuran (30 ml) was added 1,1'-azodicarbonyldipiperidine (0.93 g), and the mixture was stirred overnight at room temperature. The reaction mixture was concentrated, diisopropyl ether was added, and the precipitated crystals were filtered off. The filtrate was concentrated, and the obtained residue was subjected to silica gel column chromatography and eluted with ethyl acetate-hexane (2:3, v/v) to give a yellow oil (0.91 g).

To a solution of the obtained oil in tetrahydrofuran (6 ml) and methanol (6 ml) was added 1N aqueous sodium hydroxide (3.0 ml), and the mixture was stirred at 50°C for 2 hr. To the reaction mixture were added 1N hydrochloric acid (3.0 ml) and brine (10 ml), and the mixture was extracted with ethyl acetate (25 mlx2). The organic layer was washed with brine, dried (MgSO₄), filtered and concentrated. The residue was subjected to silica gel column chromatography and eluted with ethyl acetate-hexane (6:1, v/v) to give (2-{3-{1-isopropyl-3-(quinolin-2-ylmethoxy)-1H-pyrazol-5-yl]propoxy}-3-methoxyphenyl)acetic acid as a yellow oil (0.78 g, yield 87%). ¹H-NMR (300 MHz, CDCl₃) δ:1.37 (6 H, d, J = 6.6 Hz), 2.09 (2 H, quintet, J = 6.9 Hz), 2.70 (2 H, t, J = 7.0 Hz), 3.74 (2 H, s), 3.81 (3 H, s), 4.04 (2 H, t, J = 6.7 Hz), 4.28 (1 H, septet, J = 6.5 Hz), 5.46 (2 H, s), 5.64 (1 H, s), 6.80 - 7.08 (3 H, m), 7.48 - 7.85 (4 H, m), 8.12 - 8.24 (2 H, m).

### Example 15

To a solution of 3-[1-isopropyl-3-(quinolin-2-yloxy)-1H-pyrazol-5-yl]propan-1-ol (0.33 g), methyl (2-hydroxy-3-methoxyphenyl)acetate (0.24 g) and tributylphosphine (0.55 ml) in tetrahydrofuran (30 ml) was added 1,1'-azodicarbonyldipiperidine (0.56 g), and the mixture was stirred overnight at room temperature. The reaction mixture was concentrated, diisopropyl ether was added, and the precipitated crystals were filtered off. The filtrate was concentrated, and the obtained residue was subjected to silica gel column chromatography and eluted with ethyl acetate-hexane (2:3, v/v) to give a yellow oil (0.51 g).

To a solution of the obtained oil in tetrahydrofuran (4 ml) and methanol (4 ml) was added 1N aqueous sodium hydroxide (1.7 ml), and the mixture was stirred overnight at room temperature. To the reaction mixture were added 1N hydrochloric acid (2.0 ml) and brine (10 ml), and the mixture was extracted with ethyl acetate (20 mlx2). The organic layer was washed with brine, dried (MgSO₄), filtered and concentrated. The residue was subjected to silica gel column chromatography and eluted with ethyl acetate-hexane (6:1, v/v) to give (2-{3-[1-isopropyl-3-(quinolin-2-yloxy)-1H-pyrazol-5-yl]propoxy}-3-methoxyphenyl)acetic acid as a yellow oil (0.48 g, yield 91%). ¹H-NMR (300 MHz, CDCl₃) δ:1.51 (6 H, d, J = 6.6 Hz), 2.18 (2 H, quintet, J = 6.4 Hz), 2.93 (2 H, t, J = 6.7 Hz), 3.63 (2 H, s), 3.83 (3 H, s), 4.12 (2 H, t, J = 6.1 Hz), 4.48 (1 H, septet, J = 6.6 Hz), 6.01 (1 H, s), 6.78 - 6.90 (2 H, m), 7.01 (1 H, dd, J= 8.4, 7.4 Hz), 7.15 (1 H, d, J = 8.8 Hz), 7.36 - 7.47 (1 H, m), 7.54 - 7.78 (2 H, m), 7.91 (1 H, d, J = 8.4 Hz), 8.11 (1 H, d, J = 8.8 Hz).

### Example 16

To a solution of 3-[1-(2,4-dichlorobenzyl)-3-isopropoxy-1H-pyrazol-5-yl]propan-1-ol (0.50 g), methyl (2-hydroxy-3-methoxyphenyl)acetate (0.33 g) and tributylphosphine (0.75 ml) in tetrahydrofuran (40 ml) was added 1,1'-azodicarbonyldipiperidine (0.76 g), and the mixture was stirred overnight at room temperature. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:3, v/v) to give a yellow oil (0.72 g).

To a solution of the obtained oil in tetrahydrofuran (6 ml) and methanol (6 ml) was added 1N aqueous sodium hydroxide (2.3 ml), and the mixture was stirred overnight at room temperature. To the reaction mixture were added 1N hydrochloric acid (2.5 ml) and brine (10 ml), and the mixture was extracted with ethyl acetate (20 mlx2). The organic layer was washed with brine, dried (MgSO₄), filtered and concentrated. The residue was subjected to silica gel column chromatography and eluted with ethyl acetate-hexane (1:1, v/v) to give (2-{3-[1-(2,4-dichlorobenzyl)-3-isopropoxy-1H-pyrazol-5-yl]propoxy}-3-methoxyphenyl)acetic acid as a yellow oil (0.70 g, yield quant.). ¹H-NMR (300 MHz, CDCl₃) δ:1.33 (6 H, d, J = 6.0 Hz), 1.90 - 2.08 (2 H, m), 2.66 (2 H, t, J = 7.7 Hz), 3.61 (2 H, s), 3.78 (3 H, s), 3.99 (2 H, t, J = 6.3 Hz), 4.70 (1 H, septet, J = 6.1 Hz), 5. 18 (2 H, s), 5.58 (1 H, s), 6.57 (1 H, d, J = 8.4 Hz), 6.76 - 6.90 (2 H, m), 7.00 (1 H, dd, J = 8.2, 7.4 Hz), 7.11 (1 H, dd, J = 8.2, 2.0 Hz), 7.34 (1 H, d, J = 2.2 Hz).

### Example 17

To a solution of 3-{1-[1-(2,4-dichlorophenyl)ethyl]-3-isopropoxy-1H-pyrazol-5-yl}propan-1-ol (0.50 g), methyl (2-hydroxy-3-methoxyphenyl)acetate (0.34 g) and tributylphosphine (0.73 ml) in tetrahydrofuran (40 ml) was added 1,1'-azodicarbonyldipiperidine (0.74 g), and the mixture was stirred at room temperature for 2 hr. The reaction mixture was concentrated, diisopropyl ether was added, and the precipitated crystals were filtered off. The filtrate was concentrated, and the residue was subjected to silica gel column chromatography and eluted with ethyl acetate-hexane (1:6, v/v) to give a colorless oil (0.68 g).

To a solution of the obtained oil in tetrahydrofuran (6 ml) and methanol (6 ml) was added 1N aqueous sodium hydroxide (2.3 ml), and the mixture was stirred overnight at room temperature. To the reaction mixture were added 1N hydrochloric acid (2.3 ml) and brine (10 ml), and the mixture was extracted with ethyl acetate (20 mlx2). The organic layer was washed with brine, dried (MgSO₄), filtered and concentrated. The residue was subjected to silica gel column chromatography and eluted with ethyl acetate-hexane (1:1, v/v) to give [2-(3-{1-[1-(2,4-dichlorophenyl)ethyl]-3-isopropoxy-1H-pyrazol-5-yl}propoxy)-3-methoxyphenyl]acetic acid as a colorless oil (0.63 g, yield 80%). ¹H-NMR (300 MHz, CDCl₃) δ:1.37 (6 H, d, J = 6.0 Hz), 1.78 (3 H, d, J = 6.9 Hz), 1.82 - 2.00 (2 H, m), 2.44 - 2.80 (2 H, m), 3.64 (2 H, s), 3.80 (3 H, s), 3.90 - 4.03 (2 H, m), 4.66 (1 H, septet, J = 6.2 Hz), 5.53 (1 H, s), 5.68(1 H, q, J = 7.0 Hz), 6.78 - 6.90 (2 H, m), 6.97 - 7.36 (4 H, m).

### Example 18

To a solution of 3-[3-isopropoxy-1-(pyridin-2-ylmethyl)-1H-pyrazol-5-yl]propan-1-ol (0.45 g), methyl (2-hydroxy-3-methoxyphenyl)acetate (0.38 g) and tributylphosphine (0.80 ml) in tetrahydrofuran (40 ml) was added 1,1'-azodicarbonyldipiperidine (0.81 g), and the mixture was stirred at room temperature for 2 hr. The reaction mixture was concentrated, diisopropyl ether was added, and the precipitated crystals were filtered off. The filtrate was concentrated, and the residue was subjected to silica gel column chromatography and eluted with ethyl acetate-hexane (1:6, v/v) to give a yellow oil (0.57 g).

To a solution of the obtained oil in tetrahydrofuran (4 ml) and methanol (4 ml) was added 1N aqueous sodium hydroxide (2.0 ml), and the mixture was stirred at 50°C for 2 hr. To the reaction mixture were added 1N hydrochloric acid (2.0 ml) and brine (10 ml), and the mixture was extracted with ethyl acetate (20 mlx2). The organic layer was washed with brine, dried (MgSO₄), filtrated and concentrated to give (2-{3-[3-isopropoxy-1-(pyridin-2-ylmethyl)-1H-pyrazol-5-yl]propoxy}-3-methoxyphenyl)acetic acid as a colorless oil (0.63 g, yield 80%). ¹H-NMR (300 MHz, CDCl₃) δ:1.37 (6 H, d, J = 6.0 Hz), 1.78 (3 H, d, J = 6.9 Hz), 1.82 - 2.00 (2 H, m), 2.44 - 2.80 (2 H, m), 3.64 (2 H, s), 3.80 (3 H, s), 3.90 - 4.03 (2 H, m), 4.66 (1 H, septet, J = 6.2 Hz), 5.53 (1 H, s), 5.68 (1 H, q, J = 7.0 Hz), 6.78 - 6.90 (2 H, m), 6. 97 - 7.36 (4 H, m).

### Example 19

To a solution of 3-{1-[1-(2,4-dichlorophenyl)ethyl]-3-propoxy-1H-pyrazol-5-yl}propan-1-ol (0.44 g), methyl (2-hydroxy-3-methoxyphenyl)acetate (0.31 g) and tributylphosphine (0.65 ml) in tetrahydrofuran (30 ml) was added 1,1'-azodicarbonyldipiperidine (0.66 g), and the mixture was stirred at room temperature for 2 hr. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:5, v/v) to give a yellow oil (0.56 g).

To a solution of the obtained oil in tetrahydrofuran (4 ml) and methanol (4 ml) was added 1N aqueous sodium hydroxide (2.0 ml), and the mixture was stirred at 50°C for 2 hr. To the reaction mixture were added 1N hydrochloric acid (2.0 ml) and brine (10 ml), and the mixture was extracted with ethyl acetate (20 mlx2). The organic layer was washed with brine, dried (MgSO₄), filtrated and concentrated to give [2-(3-{1-[1-(2,4-dichlorophenyl)ethyl]-3-propoxy-1H-pyrazol-5-yl}propoxy)-3-methoxyphenyl]acetic acid as a colorless oil (0.50 g, yield 74%). ¹H-NMR (300 MHz, CDCl₃) δ:1.03 (3 H, t, J = 7.5 Hz), 1.70 - 2.00 (7 H, m), 2.42 - 2.85 (2 H, m), 3.63 (2 H, s), 3.79 (3 H, s), 3.90 - 4.10 (4 H, m), 5.55 (1 H, s), 5.68 (1 H, q, J = 6.9 Hz), 6.77 - 6.88 (2 H, m), 6.95 - 7.33 (5 H, m).

### Example 20

To a solution of 3-(1-benzyl-3-isopropoxy-1H-pyrazol-5-yl)propan-1-ol (0.77 g), methyl (2-hydroxy-3-methoxyphenyl)acetate (0.65 g) and tributylphosphine (1.42 ml) in tetrahydrofuran (40 ml) was added 1,1'-azodicarbonyldipiperidine (1.43 g), and the mixture was stirred at room temperature for 2 hr. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:5, v/v) to give a colorless oil (1.28 g).

To a solution of the obtained oil in tetrahydrofuran (6 ml) and methanol (6 ml) was added 1N aqueous sodium hydroxide (4.5 ml), and the mixture was stirred overnight at room temperature. To the reaction mixture were added 1N hydrochloric acid (4.5 ml) and brine (10 ml), and the mixture was extracted with ethyl acetate (25 mlx2). The organic layer was washed with brine, dried (MgSO₄), filtered and concentrated. The residue was subjected to silica gel column chromatography and eluted with ethyl acetate-hexane (2:1, v/v) to give {2-[3-(1-benzyl-3-isopropoxy-1H-pyrazol-5-yl)propoxy]-3-methoxyphenyl}acetic acid as a pale-yellow oil (1.09 g, yield 89%). ¹H-NMR (300 MHz, CDCl₃) δ:1.33 (6 H, d, J = 6.0 Hz), 1.90 - 2.00 (2 H, m), 2.65 (2 H, t, J = 7.8 Hz), 3.60 (2 H, s), 3.77 (3 H, s), 3.96 (2 H, t, J = 6.2 Hz), 4.69 (1 H, septet, J = 6.1 Hz), 5.14 (2 H, s), 5.52 (1 H, s), 6.76 - 6.86 (2 H, m), 6.95 - 7.10 (3 H, m), 7.15 - 7.30 (3 H, m) .

### Example 21

To a solution of 3-[3-isopropoxy-1-(2-methylbenzyl)-1H-pyrazol-5-yl]propan-1-ol (0.78 g), methyl (2-hydroxy-3-methoxyphenyl)acetate (0.58 g) and tributylphosphine (1.35 ml) in tetrahydrofuran (50 ml) was added 1,1'-azodicarbonyldipiperidine (1.36 g), and the mixture was stirred at room temperature for 2 hr. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:3, v/v) to give a colorless oil (1.02 g).

To a solution of the obtained oil in tetrahydrofuran (6 ml) and methanol (6 ml) was added 1N aqueous sodium hydroxide (4.1 ml), and the mixture was stirred at 50°C for 2 hr. To the reaction mixture were added 1N hydrochloric acid (4.5 ml) and brine (10 ml), and the mixture was extracted with ethyl acetate (20 mlx2). The organic layer was washed with brine, dried (MgSO₄), filtered and concentrated. The residue was subjected to silica gel column chromatography and eluted with ethyl acetate-hexane (2:1, v/v) to give (2-{3-[3-isopropoxy-1-(2-methylbenzyl)-1H-pyrazol-5-yl]propoxy}-3-methoxyphenyl)acetic acid as a colorless oil (0.94 g, yield 78%). ¹H-NMR (300 MHz, CDCl₃) δ:1.33 (6 H, d, J = 6.2 Hz), 1.90 - 2.06 (2 H, m), 2.29 (3 H, s), 2.62 (2 H, t, J = 7.7 Hz), 3.58 (2 H, s), 3.77 (3 H, s), 3.97 (2 H, t, J = 6.2 Hz), 4.71 (1 H, septet, J = 6.2 Hz), 5.13 (2 H, s), 5.56 (1 H, s), 6.53 (1 H, d, J = 6.6 Hz), 6.76 - 6.87 (2 H, m), 6.94 - 7.16 (4 H, m).

### Example 22

To a solution of 3-[1-(2,4-dichlorobenzyl)-3-isopropoxy-1H-pyrazol-5-yl]propan-1-ol (0.50 g), methyl (1-ethyl-3-hydroxy-1H-pyrazol-4-yl)acetate (0.30 g) and tributylphosphine (0.75 ml) in tetrahydrofuran (30 ml) was added 1,1'-azodicarbonyldipiperidine (0.76 g), and the mixture was stirred at room temperature for 2 hr. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:3, v/v) to give a colorless oil (0.77 g).

To a solution of the obtained oil in tetrahydrofuran (6 ml) and methanol (6 ml) was added 1N aqueous sodium hydroxide (2.5 ml), and the mixture was stirred at 50°C for 1 hr. To the reaction mixture were added 1N hydrochloric acid (2.5 ml) and brine (10 ml), and the mixture was extracted with ethyl acetate (20 mlx2). The organic layer was washed with brine, dried (MgSO₄), filtered and concentrated. The residue was subjected to silica gel column chromatography and eluted with ethyl acetate-hexane (1:5, v/v) to give (3-{3-[1-(2,4-dichlorobenzyl)-3-isopropoxy-1H-pyrazol-5-yl]propoxy}-1-ethyl-1H-pyrazol-4-yl)acetic acid as a colorless oil (0.56 g, yield 73%). ¹H-NMR (300 MHz, CDCl₃) δ:1.33 (6 H, d, J = 5.8 Hz), 1.39 (3 H, t, J = 7.3 Hz), 1.90 - 2.07 (2 H, m), 2.60 (2 H, t, J = 7.7 Hz), 3.34 (2 H, s), 3.94 (2 H, q, J = 7.3 Hz), 4.16 (2 H, t, J = 6.1 Hz), 4.69 (1 H, septet, J = 6.2 Hz), 5.15 (2 H, s), 5.57 (1 H, s), 6.55 (1 H, d, J = 8.4 Hz), 7.09 (1 H, dd, J = 8.4, 2.2 Hz), 7.34 (1 H, d, J = 2.2 Hz).

### Example 23

To a solution of 3-[1-(2,4-dichlorobenzyl)-3-isopropoxy-1H-pyrazol-5-yl]propan-1-ol (0.50 g), methyl (3-ethoxy-2-hydroxyphenyl)acetate (0.34 g) and tributylphosphine (0.73 ml) in tetrahydrofuran (40 ml) was added 1,1'-azodicarbonyldipiperidine (0.74 g), and the mixture was stirred overnight at room temperature. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:4, v/v) to give a yellow oil.

To a solution of the obtained oil in tetrahydrofuran (6 ml) and methanol (6 ml) was added 1N aqueous sodium hydroxide (2.0 ml), and the mixture was stirred at 50°C for 1 hr. To the reaction mixture were added 1N hydrochloric acid (2.0 ml) and brine (10 ml), and the mixture was extracted with ethyl acetate (15 mlx2). The organic layer was washed with brine, dried (MgSO₄), filtered and concentrated. The obtained crude crystals were recrystallized from ethyl acetate-hexane to give (2-{3-[1-(2,4-dichlorobenzyl)-3-isopropoxy-1H-pyrazol-5-yl]propoxy}-3-ethoxyphenyl)acetic acid as colorless crystals (0.61 g, yield 80%). melting point 132 - 133°C.

### Example 24

To a solution of 3-[1-(2,4-dichlorobenzyl)-3-isopropoxy-1H-pyrazol-5-yl]propan-1-ol (0.50 g), methyl (2-hydroxy-3-methylphenyl)acetate (0.29 g) and tributylphosphine (0.73 ml) in tetrahydrofuran (40 ml) was added 1,1'-azodicarbonyldipiperidine (0.74 g), and the mixture was stirred overnight at room temperature. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:4, v/v) to give a yellow oil.

To a solution of the obtained oil in tetrahydrofuran (6 ml) and methanol (6 ml) was added 1N aqueous sodium hydroxide (2.0 ml), and the mixture was stirred at 50°C for 1 hr. To the reaction mixture were added 1N hydrochloric acid (2.0 ml) and brine (10 ml), and the mixture was extracted with ethyl acetate (15 mlx2). The organic layer was washed with brine, dried (MgSO₄), filtered and concentrated. The residue was subjected to silica gel column chromatography and eluted with ethyl acetate-hexane (1:1, v/v). The obtained crystals were recrystallized from ethyl acetate-hexane to give (2-{3-[1-(2,4-dichlorobenzyl)-3-isopropoxy-1H-pyrazol-5-yl]propoxy}-3-methylphenyl)acetic acid as colorless crystals (0.12 g, yield 16%). melting point 70 - 71°C.

### Example 25

To a solution of 3-[1-(2,4-dichlorobenzyl)-3-(methoxymethoxy)-1H-pyrazol-5-yl]propan-1-ol (3.00 g), methyl (2-hydroxy-3-methoxyphenyl)acetate (1.88 g) and tributylphosphine (4.33 ml) in tetrahydrofuran (150 ml) was added 1,1*'*-azodicarbonyldipiperidine (4.39 g), and the mixture was stirred at room temperature for 2 hr. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:2, v/v) to give methyl (2-{3-[1-(2,4-dichlorobenzyl)-3-(methoxymethoxy)-1H-pyrazol-5-yl]propoxy}-3-methoxyphenyl)acetate as a yellow oil (4.34 g, yield 95%). ¹H-NMR (300 MHz, CDCl₃) δ:1.95 - 2.07 (2 H, m), 2.68 (2 H, t, J = 7.7 Hz), 3.52 (3 H, s), 3.60 (2 H, s), 3.63 (3 H, s), 3.79 (3 H, s), 3.98 (2 H, t, J = 6.2 Hz), 5.21 (2 H, s), 5.22 (2 H, s), 5.72 (1 H, s), 6.60 (1 H, d, J = 8.4 Hz), 6.77 - 6.85 (2 H, m), 6.99 (1 H, t, J = 7.8 Hz), 7.13 (1 H, dd, J = 8.4, 2.1 Hz), 7.35 (1 H, d, J = 2.4 Hz).

### Example 26

To a solution of methyl (2-{3-[1-(2,4-dichlorobenzyl)-3-hydroxy-1H-pyrazol-5-yl]propoxy}-3-methoxyphenyl)acetate (0.60 g), ethanol (0.12 g) and tributylphosphine (0.62 ml) in tetrahydrofuran (30 ml) was added 1,1'-azodicarbonyldipiperidine (0.63 g), and the mixture was stirred at room temperature for 2 nights. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:4, v/v) to give a colorless oil (0.59 g).

To a solution of the obtained oil in tetrahydrofuran (6 ml) and methanol (6 ml) was added 1N aqueous sodium hydroxide (2.5 ml), and the mixture was stirred overnight at room temperature. To the reaction mixture were added 1N hydrochloric acid (2.5 ml) and brine (10 ml), and the mixture was extracted with ethyl acetate (15 mlx2). The organic layer was washed with brine, dried (MgSO₄), filtered and concentrated. The obtained crude crystals were recrystallized from ethyl acetate-hexane to give (2-{3-[1-(2,4-dichlorobenzyl)-3-ethoxy-1H-pyrazol-5-yl]propoxy}-3-methoxyphenyl)acetic acid as colorless crystals (0.53 g, yield 85%). melting point 109 - 110°C.

### Example 27

To a solution of methyl (2-{3-[1-(2,4-dichlorobenzyl)-3-hydroxy-1H-pyrazol-5-yl]propoxy}-3-methoxyphenyl)acetate (0.60 g), butanol (0.14 g) and tributylphosphine (0.62 ml) in tetrahydrofuran (30 ml) was added 1,1'-azodicarbonyldipiperidine (0.63 g), and the mixture was stirred at room temperature for 2 nights. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:4, v/v) to give a colorless oil.

To a solution of the obtained oil in tetrahydrofuran (6 ml) and methanol (6 ml) was added 1N aqueous sodium hydroxide (2.5 ml), and the mixture was stirred overnight at room temperature. To the reaction mixture were added 1N hydrochloric acid (2.5 ml) and brine (10 ml), and the mixture was extracted with ethyl acetate (15 mlx2). The organic layer was washed with brine, dried (MgSO₄), filtered and concentrated. The residue was subjected to silica gel column chromatography and eluted with ethyl acetate-hexane (1:1, v/v) to give (2-{3-[3-butoxy-1-(2,4-dichlorobenzyl)-1H-pyrazol-5-yl]propoxy}-3-methoxyphenyl)acetic acid as a pale-yellow oil (0.57 g, yield 85%). ¹H-NMR (300 MHz, CDCl₃) δ:0.95 (3 H, t, J = 7.1 Hz), 1.35 - 1.57 (2 H, m), 1.64 - 1.82 (2 H, m), 1.90 - 2.10 (2 H, m), 2.65 (3 H, t, J = 7.7 Hz), 3.60 (2 H, s), 3.78 (3 H, s), 3.98 (2 H, t, J = 6.2 Hz), 4.09 (2 H, t, J = 6.6 Hz), 5.19 (2 H, s), 5.59 (1 H, s), 6.55 (1 H, d, J = 8.4 Hz), 6.76 - 6. 90 (2 H, m), 7.00 (1 H, dd, J = 8.5, 7.3 Hz), 7.11 (1 H, dd, J = 8.3, 1.9 Hz), 7.34 (1 H, d, J = 1.8 Hz).

### Example 28

To a solution of methyl (2-{3-[1-(2,4-dichlorobenzyl)-3-hydroxy-1H-pyrazol-5-yl]propoxy}-3-methoxyphenyl)acetate (0.60 g), methanol (0.08 g) and tributylphosphine (0.62 ml) in tetrahydrofuran (30 ml) was added 1,1'-azodicarbonyldipiperidine (0.63 g), and the mixture was stirred at room temperature for 2 nights. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:4, v/v) to give a colorless oil (0.59 g).

To a solution of the obtained oil in tetrahydrofuran (6 ml) and methanol (6 ml) was added 1N aqueous sodium hydroxide (2.5 ml), and the mixture was stirred overnight at room temperature. To the reaction mixture were added 1N hydrochloric acid (2.5 ml) and brine (10 ml), and the mixture was extracted with ethyl acetate (15 mlx2). The organic layer was washed with brine, dried (MgSO₄), filtered and concentrated. The obtained crude crystals were recrystallized from ethyl acetate-hexane to give (2-{3-[1-(2,4-dichlorobenzyl)-3-methoxy-1H-pyrazol-5-yl]propoxy}-3-methoxyphenyl)acetic acid as colorless crystals (0.47 g, yield 75%). melting point 111 - 112°C.

### Example 29

To a solution of 3-[1-(2,4-dichlorobenzyl)-3-isopropoxy-1H-pyrazol-5-yl]propan-1-ol (0.60 g), methyl (3-ethyl-2-hydroxyphenyl)acetate (0.37 g) and tributylphosphine (0.87 ml) in tetrahydrofuran (40 ml) was added 1,1'-azodicarbonyldipiperidine (0.88 g), and the mixture was stirred overnight at room temperature. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:4, v/v) to give a yellow oil.

To a solution of the obtained oil in tetrahydrofuran (6 ml) and methanol (6 ml) was added 1N aqueous sodium hydroxide (2.0 ml), and the mixture was stirred at 50°C for 1 hr. To the reaction mixture were added 1N hydrochloric acid (2.0 ml) and brine (10 ml), and the mixture was extracted with ethyl acetate (15 mlx2). The organic layer was washed with brine, dried (MgSO₄), filtered and concentrated. The obtained crude crystals were recrystallized from ethyl acetate-hexane to give (2-{3-[1-(2,4-dichlorobenzyl)-3-isopropoxy-1H-pyrazol-5-yl]propoxy}-3-ethylphenyl)acetic acid as colorless crystals (0.64 g, yield 72%). melting point 98 - 99°C.

### Example 30

To a solution of methyl (2-{3-[1-(2,4-dichlorobenzyl)-3-hydroxy-1H-pyrazol-5-yl]propoxy}-3-methoxyphenyl)acetate (0.60 g), 2-methylpropan-1-ol (0.14 g) and tributylphosphine (0.62 ml) in tetrahydrofuran (30 ml) was added 1,1'-azodicarbonyldipiperidine (0.63 g), and the mixture was stirred at room temperature for 2 nights. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:4, v/v) to give a colorless oil.

To a solution of the obtained oil in tetrahydrofuran (6 ml) and methanol (6 ml) was added 1N aqueous sodium hydroxide (2.5 ml), and the mixture was stirred overnight at room temperature. To the reaction mixture were added 1N hydrochloric acid (2.5 ml) and brine (10 ml), and the mixture was extracted with ethyl acetate (15 mlx2). The organic layer was washed with brine, dried (MgSO₄), filtered and concentrated. The residue was subjected to silica gel column chromatography and eluted with ethyl acetate-hexane (1:1, v/v) to give (2-{3-[1-(2,4-dichlorobenzyl)-3-isobutoxy-1H-pyrazol-5-yl]propoxy}-3-methoxyphenyl)acetic acid as a colorless oil (0.44 g, yield 65%). ¹H-NMR (300 MHz, CDCl₃) δ:1.00 (6 H, d, J = 6.8 Hz), 1.90 - 2.13 (3 H, m), 2.65 (3 H, t, J = 7.7 Hz), 3.61 (2 H, s), 3.78 (3 H, s), 3.86 (2 H, t, J = 6.6 Hz), 3.99 (2 H, t, J = 6.3 Hz), 5.19 (2 H, s), 5.60 (1 H, s), 6.55 (1 H, d, J = 8.4 Hz), 6.77 - 6.88 (2 H, m), 7.00 (1 H, dd, J = 8.0, 7.6 Hz), 7.11 (1 H, dd, J = 8.4, 2.2 Hz), 7.34 (1 H, d, J = 1.8 Hz).

### Example 31

To a solution of 3-[1-(2,4-dichlorobenzyl)-3-isopropoxy-1H-pyrazol-5-yl]propan-1-ol (1.00 g), methyl (2-oxo-1,2-dihydropyridin-3-yl)acetate (0.50 g) and tributylphosphine (2.20 ml) in tetrahydrofuran (80 ml) was added 1,1'-azodicarbonyldipiperidine (2.20 g), and the mixture was stirred overnight at room temperature. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:3, v/v) to give a colorless oil (0.86 g).

To a solution of the obtained oil in tetrahydrofuran (8 ml) and methanol (8 ml) was added 1N aqueous sodium hydroxide (4.0 ml), and the mixture was stirred at 50°C for 2 hr. To the reaction mixture were added 1N hydrochloric acid (4.0 ml) and brine (10 ml), and the mixture was extracted with ethyl acetate (20 mlx2). The organic layer was washed with brine, dried (MgSO₄), filtered and concentrated. The obtained crude crystals were recrystallized from ethyl acetate-hexane to give (2-{3-[1-(2,4-dichlorobenzyl)-3-isopropoxy-1H-pyrazol-5-yl]propoxy}pyridin-3-yl)acetic acid as colorless crystals (0.81 g, yield 58%). melting point 88 - 90°C.

### Example 32

To a solution of 3-[1-(2,4-dichlorobenzyl)-3-isopropoxy-1H-pyrazol-5-yl]propan-1-ol (0.50 g), methyl (3-hydroxy-1-methyl-1H-pyrazol-4-yl)acetate (0.27 g) and tributylphosphine (0.73 ml) in tetrahydrofuran (30 ml) was added 1,1'-azodicarbonyldipiperidine (0.74 g), and the mixture was stirred at 50°C for 1 hr. The reaction mixture was concentrated, diisopropyl ether was added, and the precipitated crystals were filtered off. The filtrate was concentrated, and the obtained residue was subjected to silica gel column chromatography and eluted with ethyl acetate-hexane (2:3, v/v) to give a yellow oil (0.63 g).

To a solution of the obtained oil in tetrahydrofuran (6 ml) and methanol (6 ml) was added 1N aqueous sodium hydroxide (20 ml), and the mixture was stirred at 50°C for 1 hr. To the reaction mixture were added 1N hydrochloric acid (20 ml) and brine (10 ml), and the mixture was extracted with ethyl acetate (20 mlx2). The organic layer was washed with brine, dried (MgSO₄), filtered and concentrated. The obtained crude crystals were recrystallized from ethyl acetate-hexane to give (3-{3-[1-(2,4-dichlorobenzyl)-3-isopropoxy-1H-pyrazol-5-yl]propoxy}-1-methyl-1H-pyrazol-4-yl)acetic acid as colorless crystals (0.54 g, yield 73%). melting point 96 - 97°C.

### Example 33

To a solution of 3-[1-(2,4-dichlorobenzyl)-3-isopropyl-1H-pyrazol-5-yl]propan-1-ol (0.50 g), methyl (3-hydroxy-1-methyl-1H-pyrazol-4-yl)acetate (0.29 g) and tributylphosphine (0.76 g) in tetrahydrofuran (30 ml) was added 1,1'-azodicarbonyldipiperidine (0.77 g), and the mixture was stirred overnight at room temperature. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:1, v/v) to give a yellow oil (0.6 g).

To a solution of the obtained oil in tetrahydrofuran (6 ml) and methanol (6 ml) was added 1N aqueous sodium hydroxide (30 ml), and the mixture was stirred at 60°C for 1 hr. To the reaction mixture were added 1N hydrochloric acid (30 ml) and brine (10 ml), and the mixture was extracted with ethyl acetate (20 mlx2). The organic layer was washed with brine, dried (MgSO₄), filtered and concentrated. The obtained crude crystals were recrystallized from ethyl acetate-hexane to give (3-{3-[1-(2,4-dichlorobenzyl)-3-isopropyl-1H-pyrazol-5-yl]propoxy}-1-methyl-1H-pyrazol-4-yl)acetic acid as colorless crystals (0.46 g, yield 66%). melting point 67 - 68°C.

### Example 34

To a solution of 3-[1-(2,4-dichlorobenzyl)-3-isopropyl-1H-pyrazol-5-yl]propan-1-ol (0.50 g), methyl (1-benzyl-3-hydroxy-1H-pyrazol-4-yl)acetate (0.41 g) and tributylphosphine (0.76 g) in tetrahydrofuran (30 ml) was added 1,1'-azodicarbonyldipiperidine (0.77 g), and the mixture was stirred overnight at room temperature. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:3, v/v) to give a yellow oil (0.78 g).

To a solution of the obtained oil in tetrahydrofuran (6 ml) and methanol (6 ml) was added 1N aqueous sodium hydroxide (30 ml), and the mixture was stirred at 60°C for 1 hr. To the reaction mixture were added 1N hydrochloric acid (30 ml) and brine (10 ml), and the mixture was extracted with ethyl acetate (20 mlx2). The organic layer was washed with brine, dried (MgSO₄), filtered and concentrated. The residue was subjected to silica gel column chromatography and eluted with ethyl acetate-hexane (1:1, v/v) to give a colorless oil (0.75 g).

To a solution of the obtained oil in methanol (2 ml) was added 1N aqueous sodium hydroxide (1.6 ml), and the mixture was stirred for 20 min. Water (20 ml) was added, and then a solution of calcium chloride (90 mg) in water (4 ml) was added slowly. The precipitated solid was collected by filtration, washed with water and dried to give (1-benzyl-3-{3-[1-(2,4-dichlorobenzyl)-3-isopropyl-1H-pyrazol-5-yl]propoxy}-1H-pyrazol-4-yl)acetic acid 1/2 calcium salt as colorless amorphous crystals (0.64 g, yield 73%). ¹H-NMR (300 MHz, DMSO-d₆) δ:1.14 (6 H, d, J = 7.0 Hz), 1.78 - 1.98 (2 H, m), 2.50 - 2.65 (2 H, m), 2.80 (1 H, septet, J = 7.0 Hz), 2.96 (2 H, brs), 4.00 (2 H, t, J = 6.1 Hz), 4.99 (2 H, brs), 5.22 (2 H, brs), 5.99 (1 H, s), 6.51 (1 H, d, J = 8.4 Hz), 7.06 - 7.36 (6 H, m), 7.49 (1 H, s), 7.60 (1 H, d, J = 2.2 Hz).

### Example 35

To a solution of 3-[1-(2,4-dichlorobenzyl)-3-isopropyl-1H-pyrazol-5-yl]propan-1-ol (0.50 g), methyl (2-hydroxy-3-methoxyphenyl)acetate (0.33 g) and tributylphosphine (0.76 ml) in tetrahydrofuran (30 ml) was added 1,1'-azodicarbonyldipiperidine (0.77 g), and the mixture was stirred at room temperature for 2 hr. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:3, v/v) to give a yellow oil (0.64 g).

To a solution of the obtained oil in tetrahydrofuran (6 ml) and methanol (6 ml) was added 1N aqueous sodium hydroxide (30 ml), and the mixture was stirred at 60°C for 1 hr. To the reaction mixture were added 1N hydrochloric acid (30 ml) and brine (10 ml), and the mixture was extracted with ethyl acetate (20 mlx2). The organic layer was washed with brine, dried (MgSO₄), filtered and concentrated. The obtained crude crystals were recrystallized from ethyl acetate-hexane to give (2-{3-[1-(2,4-dichlorobenzyl)-3-isopropyl-1H-pyrazol-5-yl]propoxy}-3-methoxyphenyl)acetic acid as colorless crystals (0.40 g, yield 54%). melting point 101 - 102°C.

### Example 36

A mixture of (4-{3-[1-(2,4-dichlorobenzyl)-3-isopropoxy-1H-pyrazol-5-yl]propoxy}-2-methyl-1,3-thiazol-5-yl)acetonitrile (0.72 g), 6N aqueous sodium hydroxide (2.4 ml) and ethanol (10 ml) was stirred overnight with heating under reflux. The reaction mixture was concentrated, 5N hydrochloric acid (3.5 ml) was added, and the mixture was extracted with ethyl acetate (20 mlx2). The organic layer was washed with brine, dried (MgSO₄), filtered and concentrated. The residue was subjected to silica gel column chromatography and eluted with ethyl acetate-hexane (1:3, v/v). Recrystallization from ethyl acetate-hexane gave (4-{3-[1-(2,4-dichlorobenzyl)-3-isopropoxy-1H-pyrazol-5-yl]propoxy}-2-methyl-1,3-thiazol-5-yl)acetic acid as yellow crystals (0.38 g, yield 51%). melting point 75 - 77°C.

### Example 37

To a solution of 3-[1-(2, 4-dichlorobenzyl)-3-isopropyl-1H-pyrazol-5-yl]propan-1-ol (0.90 g), methyl (2-oxo-1,2-dihydropyridin-3-yl)acetate (0.51 g) and tributylphosphine (2.06 ml) in tetrahydrofuran (40 ml) was added 1,1'-azodicarbonyldipiperidine (2.08 g), and the mixture was stirred at 50°C for 2 hr. The reaction mixture was concentrated, diisopropyl ether was added, and the precipitated crystals were filtered off. The filtrate was concentrated, and the obtained residue was subjected to silica gel column chromatography and eluted with ethyl acetate-hexane (1:4, v/v) to give a yellow oil (0.77 g).

To a solution of the obtained oil in tetrahydrofuran (6 ml) and methanol (6 ml) was added 1N aqueous sodium hydroxide (2.5 ml), and the mixture was stirred at 50°C for 1 hr. To the reaction mixture were added 1N hydrochloric acid (2.5 ml) and brine (10 ml), and the mixture was extracted with ethyl acetate (20 mlx2). The organic layer was washed with brine, dried (MgSO₄), filtered and concentrated. The obtained crude crystals were recrystallized from ethyl acetate-hexane to give (2-{3-[1-(2,4-dichlorobenzyl)-3-isopropyl-1H-pyrazol-5-yl]propoxy}pyridin-3-yl)acetic acid as colorless crystals (0.71 g, yield 54%). melting point 111 - 112°C.

### Example 38

To a mixture of N-(2,4-dichlorobenzyl)-3-ethoxy-1-(2-hydroxyethyl)-1H-pyrazole-4-carboxamide (480 mg), methyl (2-hydroxyphenyl)acetate (244 mg), tributylphosphine (668 µl) and tetrahydrofuran (50 ml) was added 1,1'-(azodicarbonyl)dipiperidine (676 mg) at room temperature, and the mixture was stirred for 3 days. The reaction mixture was concentrated, and the residue was washed with diisopropyl ether. The washing was concentrated. The residue was subjected to silica gel column chromatography to give, from a fraction eluted with ethyl acetate-hexane (2:3, v/v), a pale-yellow solid. A mixture of the obtained solid, a 1N aqueous sodium hydroxide solution (20 ml), tetrahydrofuran (20 ml) and ethanol (20 ml) was stirred at room temperature for 1 hr, 1N hydrochloric acid (20 ml) was added, and the mixture was extracted with ethyl acetate. The extract was washed with saturated brine, dried (MgSO₄) and concentrated. The obtained white solid was recrystallized from ethyl acetate to give {2-[2-(4-{[(2,4-dichlorobenzyl)amino]carbonyl}-3-ethoxy-1H-pyrazol-1-yl)ethoxy]phenyl}acetic acid (88.6 mg, yield 13%) as colorless crystals. melting point 167 - 169°C.

### Example 39

To a mixture of N-(2,4-dichlorobenzyl)-3-ethoxy-1-(2-hydroxyethyl)-1H-pyrazole-4-carboxamide (500 mg), methyl (3-ethyl-2-hydroxyphenyl)acetate (326 mg), tributylphosphine (698 µl) and tetrahydrofuran (50 ml) was added 1,1'-(azodicarbonyl)dipiperidine (707 mg), and the mixture was stirred overnight at 60°C. The reaction mixture was concentrated, and the residue was washed with diisopropyl ether. The washing was concentrated. The residue was subjected to silica gel column chromatography to give, from a fraction eluted with ethyl acetate-hexane (1:1, v/v), a yellow oil. A mixture of the obtained oil, a 1N aqueous sodium hydroxide solution (20 ml), tetrahydrofuran (20 ml) and ethanol (20 ml) was stirred at room temperature for 3 hr, 1N hydrochloric acid (20 ml) was added, and the mixture was extracted with ethyl acetate. The extract was washed with saturated brine, dried (MgSO₄) and concentrated. The obtained pale-yellow solid was recrystallized from ethyl acetate-hexane to give {2-[2-(4-{[(2,4-dichlorobenzyl)amino]carbonyl}-3-ethoxy-1H-pyrazol-1-yl)ethoxy]-3-ethylphenyl}acetic acid (223 mg, yield 31%) as colorless crystals. melting point 129 - 131°C.

### Example 40

To a mixture of N-(2,4-dichlorobenzyl)-3-ethoxy-1-(2-hydroxyethyl)-1H-pyrazole-4-carboxamide (510 mg), methyl (1-ethyl-3-hydroxy-1H-pyrazol-4-yl)acetate (315 mg), tributylphosphine (708 µl) and tetrahydrofuran (50 ml) was added 1,1'-(azodicarbonyl)dipiperidine (717 mg), and the mixture was stirred at room temperature for 5 hr. The reaction mixture was concentrated, and the residue was washed with diisopropyl ether. The washing was concentrated. The residue was subjected to silica gel column chromatography to give, from a fraction eluted with ethyl acetate-hexane (4:1, v/v), a white solid. A mixture of the obtained solid, a 1N aqueous sodium hydroxide solution (20 ml), tetrahydrofuran (20 ml) and ethanol (20 ml) was stirred at room temperature for 1 hr, 1N hydrochloric acid (20 ml) was added, and the mixture was extracted with ethyl acetate. The extract was washed with saturated brine, dried (MgSO₄) and concentrated. The obtained white solid was recrystallized from ethyl acetate-hexane to give {3-[2-(4-{[(2,4-dichlorobenzyl)amino]carbonyl}-3-ethoxy-1H-pyrazol-1-yl)ethoxy]-1-ethyl-1H-pyrazol-4-yl}acetic acid (182 mg, yield 25%) as colorless crystals. melting point 157 - 158°C.

### Example 41

To a mixture of N-(2,4-dichlorobenzyl)-3-ethoxy-1-(2-hydroxyethyl)-1H-pyrazole-4-carboxamide (530 mg), methyl (2-hydroxy-1-naphthyl)acetate (640 mg), tributylphosphine (738 µl) and tetrahydrofuran (50 ml) was added 1,1'-(azodicarbonyl)dipiperidine (747 mg), and the mixture was stirred overnight at room temperature. The reaction mixture was concentrated, and the residue was washed with diisopropyl ether. The washing was concentrated. The residue was subjected to silica gel column chromatography to give, from a fraction eluted with ethyl acetate-hexane (3:2, v/v), a pale-yellow solid. A mixture of the obtained solid, a 1N aqueous sodium hydroxide solution (20 ml), tetrahydrofuran (20 ml) and ethanol (20 ml) was stirred overnight at room temperature, 1N hydrochloric acid (20 ml) was added, and the mixture was extracted with ethyl acetate. The extract was washed with water and saturated brine, dried (MgSO₄) and concentrated. The obtained pale-yellow solid was recrystallized from ethyl acetate-hexane to give {2-[2-(4-{[(2,4-dichlorobenzyl)amino]carbonyl}-3-ethoxy-1H-pyrazol-1-yl)ethoxy]-1-naphthyl}acetic acid (379 mg, yield 47%) as colorless crystals. melting point 199 - 201°C.

### Example 42

To a mixture of N-(2,4-dichlorobenzyl)-3-ethoxy-1-(2-hydroxyethyl)-1H-pyrazole-4-carboxamide (500 mg), methyl (3-ethoxy-2-hydroxyphenyl)acetate (442 mg), tributylphosphine (698 µl) and tetrahydrofuran (50 ml) was added 1,1'-(azodicarbonyl)dipiperidine (707 mg), and the mixture was stirred overnight at room temperature. The reaction mixture was concentrated, and the residue was washed with diisopropyl ether. The washing was concentrated, and the residue was subjected to silica gel column chromatography to give, from a fraction eluted with ethyl acetate-hexane (3:2, v/v), a brown oil. A mixture of the obtained oil, a 1N aqueous Sodium hydroxide solution (20 ml), tetrahydrofuran (20 ml) and ethanol (20 ml) was stirred at room temperature for 5 hr, 1N hydrochloric acid (20 ml) was added, and the mixture was extracted with ethyl acetate. The extract was washed with water and saturated brine, dried (MgSO₄) and concentrated. The obtained white solid was recrystallized from ethyl acetate-hexane to give {2-[2-(4-{[(2,4-dichlorobenzyl)amino]carbonyl}-3-ethoxy-1H-pyrazol-1-yl)ethoxy]-3-ethoxyphenyl}acetic acid (578 mg, yield 77%) as colorless crystals. melting point 145 - 146°C.

### Example 43

To a mixture of N-(2,4-dichlorobenzyl)-1-(2-hydroxyethyl)-3-isopropoxy-1H-pyrazole-4-carboxamide (500 mg), methyl (3-ethyl-2-hydroxyphenyl)acetate (390 mg), tributylphosphine (668 µl) and tetrahydrofuran (50 ml) was added 1,1'-(azodicarbonyl)dipiperidine (676 mg), and the mixture was stirred overnight at room temperature. The reaction mixture was concentrated, and the residue was washed with diisopropyl ether. The washing was concentrated. The residue was subjected to silica gel column chromatography to give, from a fraction eluted with ethyl acetate-hexane (3:7, v/v), a brown oil. A mixture of the obtained oil, a 1N aqueous sodium hydroxide solution (20 ml), tetrahydrofuran (20 ml) and ethanol (20 ml) was stirred at room temperature for 5 hr, 1N hydrochloric acid (20 ml) was added, and the mixture was extracted with ethyl acetate. The extract was washed with water and saturated brine, dried (MgSO₄) and concentrated. The obtained pale-yellow solid was recrystallized from ethyl acetate-hexane to give {2-[2-(4-{[(2,4-dichlorobenzyl)amino]carbonyl}-3-isopropoxy-1H-pyrazol-1-yl)ethoxy]-3-ethylphenyl}acetic acid (342 mg, yield 48%) as colorless crystals. melting point 125 - 126°C.

### Example 44

To a mixture of N-(2,4-dichlorobenzyl)-1-(2-hydroxyethyl)-3-isopropoxy-1H-pyrazole-4-carboxamide (500 mg), methyl (3-ethoxy-2-hydroxyphenyl)acetate (423 mg), tributylphosphine (668 µl) and tetrahydrofuran (50 ml) was added 1,1'-(azodicarbonyl)dipiperidine (676 mg), and the mixture was stirred overnight at room temperature. The reaction mixture was concentrated, and the residue was washed with diisopropyl ether. The washing was concentrated, and the residue was subjected to silica gel column chromatography to give, from a fraction eluted with ethyl acetate-hexane (1:1, v/v), a yellow oil. A mixture of the obtained oil, a 1N aqueous sodium hydroxide solution (20 ml), tetrahydrofuran (20 ml) and ethanol (20 ml) was stirred at room temperature for 1 hr, 1N hydrochloric acid (20 ml) was added, and the mixture was extracted with ethyl acetate. The extract was washed with water and saturated brine, dried (MgSO₄) and concentrated. The obtained brown oil was crystallized from ethyl acetate-hexane to give {2-[2-(4-{[(2,4-dichlorobenzyl)amino]carbonyl}-3-isopropoxy-1H-pyrazol-1-yl)ethoxy]-3-ethoxyphenyl}acetic acid (520 mg, yield 70%) as colorless crystals. melting point 90 - 91°C.

### Example 45

To a mixture of N-(2,4-dichlorobenzyl)-1-(2-hydroxyethyl)-3-isopropoxy-1H-pyrazole-4-carboxamide (500 mg), methyl (2-hydroxy-3-methoxyphenyl)acetate (316 mg), tributylphosphine (668 µl) and tetrahydrofuran (50 ml) was added 1,1'-(azodicarbonyl)dipiperidine (676 mg), and the mixture was stirred overnight at room temperature. The reaction mixture was concentrated, and the residue was washed with diisopropyl ether. The washing was concentrated. The residue was subjected to silica gel column chromatography to give, from a fraction eluted with ethyl acetate-hexane (1:1, v/v), a yellow oil. A mixture of the obtained oil, a 1N aqueous sodium hydroxide solution (20 ml), tetrahydrofuran (20 ml) and ethanol (20 ml) was stirred overnight at room temperature, 1N hydrochloric acid (20 ml) was added, and the mixture was extracted with ethyl acetate. The extract was washed with water and saturated brine, dried (MgSO₄) and concentrated. The obtained white solid was recrystallized from ethyl acetate-hexane to give {2-[2-(4-{[(2,4-dichlorobenzyl)amino]carbonyl}-3-isopropoxy-1H-pyrazol-1-yl)ethoxy]-3-methoxyphenyl}acetic acid (565 mg, yield 79%) as colorless crystals. melting point 154 - 155°C.

### Example 46

To a mixture of N-(2,4-dichlorobenzyl)-1-(3-hydroxypropyl)-3-isopropoxy-1H-pyrazole-4-carboxamide (500 mg), methyl (2-hydroxy-3-methoxyphenyl)acetate (304 mg), tributylphosphine (643 µl) and tetrahydrofuran (50 ml) was added 1,1'-(azodicarbonyl)dipiperidine (651 mg), and the mixture was stirred overnight at room temperature. The reaction mixture was concentrated, and the residue was washed with diisopropyl ether. The washing was concentrated, and the residue was subjected to silica gel column chromatography to give, from a fraction eluted with ethyl acetate-hexane (1:1, v/v), a yellow oil. A mixture of the obtained oil, a 1N aqueous sodium hydroxide solution (20 ml), tetrahydrofuran (20 ml) and ethanol (20 ml) was stirred at room temperature for 3 hr, 1N hydrochloric acid (20 ml) was added, and the mixture was extracted with ethyl acetate. The extract was washed with water and saturated brine, dried (MgSO₄) and concentrated. The obtained pale-brown solid was recrystallized from ethyl acetate-hexane to give {2-[3-(4-{[(2,4-dichlorobenzyl)amino]carbonyl}-3-isopropoxy-1H-pyrazol-1-yl)propoxy]-3-methoxyphenyl}acetic acid (356 mg, yield 50%) as colorless crystals. melting point 134 - 135°C.

### Example 47

To a mixture of N-(2,4-dichlorobenzyl)-1-(3-hydroxypropyl)-3-isopropoxy-1H-pyrazole-4-carboxamide (500 mg), methyl (1-ethyl-3-hydroxy-1H-pyrazol-4-yl)acetate (286 mg), tributylphosphine (643 µl) and tetrahydrofuran (50 ml) was added 1,1'-(azodicarbonyl)dipiperidine (651 mg), and the mixture was stirred overnight at room temperature. The reaction mixture was concentrated, and the residue was washed with diisopropyl ether. The washing was concentrated, and the residue was subjected to silica gel column chromatography to give, from a fraction eluted with ethyl acetate-hexane (4:1, v/v), a white solid. A mixture of the obtained solid, a 1N aqueous sodium hydroxide solution (20 ml), tetrahydrofuran (20 ml) and ethanol (20 ml) was stirred at room temperature for 3 hr, 1N hydrochloric acid (20 ml) was added, and the mixture was extracted with ethyl acetate. The extract was washed with water and saturated brine, dried (MgSO₄) and concentrated. The obtained colorless oil was crystallized from ethyl acetate-hexane to give {3-[3-(4-{[(2,4-dichlorobenzyl)amino]carbonyl}-3-isopropoxy-1H-pyrazol-1-yl)propoxy]-1-ethyl-1H-pyrazol-4-yl}acetic acid (297 mg, yield 43%) as colorless crystals. melting point 138 - 139°C.

### Example 48

To a mixture of N-(2,4-dichlorobenzyl)-2-(hydroxymethyl)-4-isopropoxy-1,3-thiazole-5-carboxamide (500 mg), methyl (2-hydroxy-3-methoxyphenyl)acetate (392 mg), tributylphosphine (663 µl) and tetrahydrofuran (50 ml) was added 1,1'-(azodicarbonyl)dipiperidine (671 mg) at 0°C, and the mixture was stirred at 0°C for 30 min and at room temperature overnight. The reaction mixture was concentrated, and the residue was washed with diisopropyl ether. The washing was concentrated, and the residue was subjected to silica gel column chromatography to give, from a fraction eluted with ethyl acetate-hexane (2:3, v/v), a yellow oil. A mixture of the obtained oil, a 1N aqueous sodium hydroxide solution (10 ml), tetrahydrofuran (10 ml) and ethanol (10 ml) was stirred at room temperature for 1 hr, 1N hydrochloric acid (10 ml) was added, and the mixture was extracted with ethyl acetate. The extract was washed with water and saturated brine, dried (MgSO₄) and concentrated. The obtained yellow oil was crystallized from ethyl acetate-hexane to give {2-[(5-{[(2,4-dichlorobenzyl)amino]carbonyl}-4-isopropoxy-1,3-thiazol-2-yl)methoxy]-3-methoxyphenyl}acetic acid (188 mg, yield 26%) as colorless crystals. melting point 113 - 114°C.

### Example 49

To a mixture of N-(2,4-dichlorobenzyl)-2-(hydroxymethyl)-4-isopropoxy-1,3-thiazole-5-carboxamide (500 mg), thionyl chloride (485 µl) and toluene (50 ml) was added several drops of N,N-dimethylformamide at room temperature, and the mixture was stirred with heating under reflux for 1 hr. The reaction mixture was concentrated, and the residue was dissolved in ethyl acetate. The ethyl acetate solution was washed with a saturated aqueous sodium hydrogencarbonate solution and saturated brine, dried (MgSO₄) and concentrated to give a brown oil. To a mixture of the obtained oil, methyl (2-hydroxy-3-methylphenyl)acetate (288 mg) and N,N-dimethylformamide (50 ml) was added sodium hydride (64 mg, 60% in oil) at 0°C, and the mixture was stirred overnight at room temperature. Diluted hydrochloric acid was added to the reaction mixture, and the mixture was extracted with ethyl acetate. The extract was washed with diluted hydrochloric acid and saturated brine, dried (MgSO₄) and concentrated. The residue was subjected to silica gel column chromatography to give, from a fraction eluted with ethyl acetate-hexane (2:3, v/v), a brown oil. A mixture of the obtained oil, a 1N aqueous sodium hydroxide solution (10 ml), tetrahydrofuran (10 ml) and ethanol (10 ml) was stirred at room temperature for 2 hr, 1N hydrochloric acid (10 ml) was added, and the mixture was extracted with ethyl acetate. The extract was washed with water and saturated brine, dried (MgSO₄) and concentrated. The residue was subjected to silica gel column chromatography to give, from a fraction eluted with ethyl acetate-hexane (3:2, v/v), a yellow oil. The obtained oil was crystallized from ethyl acetate-hexane to give {2-[(5-{[(2,4-dichlorobenzyl)amino]carbonyl}-4-isopropoxy-1,3-thiazol-2-yl)methoxy]-3-methylphenyl}acetic acid (161 mg, yield 23%) as yellow crystals. melting point 125 - 126°C.

### Example 50

To a mixture of [1-(2,4-dichlorobenzyl)-3-isopropoxy-1H-pyrazol-5-yl]methanol (500 mg), methyl (2-hydroxy-3-methoxyphenyl)acetate (375 mg), tributylphosphine (792 µl) and tetrahydrofuran (50 ml) was added 1,1*'-*(azodicarbonyl)dipiperidine (802 mg), and the mixture was stirred overnight at room temperature. The reaction mixture was concentrated, and the residue was washed with diisopropyl ether. The washing was concentrated, and the residue was subjected to silica gel column chromatography to give, from a fraction eluted with ethyl acetate-hexane (1:5, v/v), a colorless oil. A mixture of the obtained oil, a 1N aqueous sodium hydroxide solution (20 ml), tetrahydrofuran (20 ml) and ethanol (20 ml) was stirred overnight at room temperature, 1N hydrochloric acid (20 ml) was added, and the mixture was extracted with ethyl acetate. The extract was washed with water and saturated brine, dried (MgSO₄) and concentrated. The obtained white solid was recrystallized from ethyl acetate-hexane to give (2-{[1-(2,4-dichlorobenzyl)-3-isopropoxy-1H-pyrazol-5-yl]methoxy}-3-methoxyphenyl)acetic acid (491 mg, yield 64%) as colorless crystals. melting point 127 - 128°C.

### Example 51

To a mixture of 3-{3-isopropoxy-1-[4-(trifluoromethyl)benzyl]-1H-pyrazol-5-yl}propan-1-ol (410 mg), methyl (2-hydroxy-3-methoxyphenyl)acetate (283 mg), tributylphosphine (600 µl) and tetrahydrofuran (50 ml) was added 1,1'-(azodicarbonyl)dipiperidine (606 mg), and the mixture was stirred at room temperature for 2 hr. The reaction mixture was concentrated, and the residue was washed with diisopropyl ether. The washing was concentrated, and the residue was subjected to silica gel column chromatography to give, from a fraction eluted with ethyl acetate-hexane (3:7, v/v) a yellow oil. A mixture of the obtained oil, a 1N aqueous sodium hydroxide solution (20 ml), tetrahydrofuran (20 ml) and ethanol (20 ml) was stirred at room temperature for 1 hr, 1N hydrochloric acid (20 ml) was added, and the mixture was extracted with ethyl acetate. The extract was washed with water and saturated brine, dried (MgSO₄) and concentrated. The obtained colorless oil was crystallized from ethyl acetate-hexane to give [2-(3-{3-isopropoxy-1-[4-(trifluoromethyl)benzyl]-1H-pyrazol-5-yl}propoxy)-3-methoxyphenyl]acetic acid (267 mg, yield 44%) as colorless crystals. melting point 116 - 117°C.

### Example 52

To a mixture of 3-[5-(2,4-dichlorobenzyl)-2-isopropyl-1,3-thiazol-4-yl]propan-1-ol (260 mg), methyl (1-benzyl-3-hydroxy-1H-pyrazol-4-yl)acetate (223 mg), tributylphosphine (376 µl) and tetrahydrofuran (50 ml) was added 1,1*'-*(azodicarbonyl)dipiperidine (381 mg), and the mixture was stirred overnight at room temperature. The reaction mixture was concentrated, and the residue was washed with diisopropyl ether. The washing was concentrated, and the residue was subjected to silica gel column chromatography to give, from a fraction eluted with ethyl acetate-hexane (1:4, v/v), a pale-yellow oil. A mixture of the obtained oil, a 1N aqueous sodium hydroxide solution (20 ml), tetrahydrofuran (20 ml) and ethanol (20 ml) was stirred at room temperature for 3 hr, 1N hydrochloric acid (20 ml) was added, and the mixture was extracted with ethyl acetate. The extract was washed with saturated brine, dried (MgSO₄) and concentrated. The residue was subjected to silica gel column chromatography to give, from a fraction eluted with ethyl acetate-hexane (9:1, v/v), a colorless oil. A mixture of the obtained oil, a 1N aqueous sodium hydroxide solution (483 µl), tetrahydrofuran (20 ml) and ethanol (20 ml) was stirred at room temperature for 30 min and concentrated. To a mixture of the residue and water (40 ml) was slowly added calcium chloride (53.6 mg) dissolved in a small amount of water, and the mixture was stirred at room temperature for 1 hr. The resulting white precipitate was collected by filtration to give (1-benzyl-3-{3-[5-(2,4-dichlorobenzyl)-2-isopropyl-1,3-thiazol-4-yl]propoxy}-1H-pyrazol-4-yl)acetic acid 1/2 calcium salt (270 mg, yield 62%) as an amorphous form. ¹H-NMR (300 MHz, DMSO-d₆) δ:1.22 (6 H, d, J = 6.6 Hz), 1.90 - 2.06 (2 H, m), 2.70 - 2.82 (2 H, m), 2.96 (2 H, s), 3.03 - 3.20 (1 H, m), 3.98 - 4.13 (2 H, m), 4.10 (2 H, s), 5.03 (2 H, s), 7.01 - 7.19 (2 H, m), 7.21 - 7.38 (5 H, m), 7.47 (1 H, s), 7.58 (1 H, d, J = 2.0 Hz).

### Example 53

To a mixture of 3-[5-(2,4-dichlorobenzyl)-2-isopropyl-1,3-thiazol-4-yl]propan-1-ol (500 mg), methyl (2-hydroxypyridin-3-yl)acetate (291 mg), tributylphosphine (723 µl) and tetrahydrofuran (50 ml) was added 1,1'-(azodicarbonyl)dipiperidine (732 mg), and the mixture was stirred overnight at room temperature. The reaction mixture was concentrated, and the residue was washed with diisopropyl ether. The washing was concentrated, and the residue was subjected to silica gel column chromatography to give, from a fraction eluted with ethyl acetate-hexane (1:4, v/v), a pale-yellow oil. A mixture of the obtained oil, a 1N aqueous sodium hydroxide solution (20 ml), tetrahydrofuran (20 ml) and ethanol (20 ml) was stirred at room temperature for 3 hr, 1N hydrochloric acid (20 ml) was added, and the mixture was extracted with ethyl acetate. The extract was washed with water and saturated brine, dried (MgSO₄) and concentrated. The obtained white solid was recrystallized from ethyl acetate-hexane to give (2-{3-[5-(2,4-dichlorobenzyl)-2-isopropyl-1,3-thiazol-4-yl]propoxy}pyridin-3-yl)acetic acid (211 mg, yield 30%) as colorless crystals. melting point 106 - 107°C.

### Example 54

To a mixture of 3-{3-isopropyl-1-[(5-methyl-2-phenyl-1,3-oxazol-4-yl)methyl]-1H-pyrazol-4-yl}propan-1-ol (210 mg), methyl (3-ethyl-2-hydroxyphenyl)acetate (120 mg), tributylphosphine (466 mg) and tetrahydrofuran (30 ml) was added 1,1'-azodicarbonyldipiperidine (451 mg) at 60°C, and the mixture was stirred for 1 hr. The reaction mixture was concentrated, diisopropyl ether was added, and the precipitated crystals were removed by filtration. The filtrate was concentrated, and the obtained residue was subjected to silica gel column chromatography and eluted from ethyl acetate-hexane (1:4, v/v) to give a pale-yellow oil (200 mg). To a solution (12 ml) of the obtained oil (178 mg) in a mixed solvent of tetrahydrofuran-methanol (3:1, v/v) was added a 1N aqueous sodium hydroxide solution (0.5 ml) at 60°C, and the mixture was stirred for 1 hr. 1N Hydrochloric acid (0.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:4, v/v) to give [3-ethyl-2-(3-{3-isopropyl-1-[(5-methyl-2-phenyl-1,3-oxazol-4-yl)methyl]-1H-pyrazol-4-yl}propoxy)phenyl]acetic acid as a colorless oil (140 mg, yield 51%). Recrystallization from ethyl acetate-hexane gave colorless prism crystals. melting point 94 - 95°C.

### Example 55

To a mixture of [1-isopropyl-5-(quinolin-2-ylmethoxy)-1H-pyrazol-3-yl]methanol (446 mg), methyl (2-hydroxyphenyl)acetate (249 mg), tributylphosphine (759 mg) and tetrahydrofuran (30 ml) was added 1,1'-azodicarbonyldipiperidine (758 mg) at 60°C, and the mixture was stirred for 1 hr. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:3, v/v) to give a yellow oil (472 mg). To a solution (12 ml) of the obtained oil (434 mg) in a mixed solvent of tetrahydrofuran-methanol (3:1, v/v) was added a 1N aqueous sodium hydroxide solution (1.5 ml) at 60°C, and the mixture was stirred for 30 min. 1N Hydrochloric acid (1.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:2, v/v) to give (2-{[1-isopropyl-5-(quinolin-2-ylmethoxy)-1H-pyrazol-3-yl]methoxy}phenyl)acetic acid as a colorless oil (417 mg, yield 71%). Recrystallization from ethyl acetate-hexane gave colorless prism crystals. melting point 116 - 117°C.

### Example 56

To a mixture of 3-(1-benzyl-3-propoxy-1H-pyrazol-5-yl)propan-1-ol (412 mg), methyl (2-hydroxy-3-methoxyphenyl)acetate (294 mg), tributylphosphine (758 mg) and tetrahydrofuran (30 ml) was added 1,1'-azodicarbonyldipiperidine (757 mg) at 60°C, and the mixture was stirred for 15 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:5, v/v) to give a colorless oil. To a solution (12 ml) of the obtained oil in a mixed solvent of tetrahydrofuran-methanol (3:1, v/v) was added a 1N aqueous sodium hydroxide solution (3.0 ml) at 60°C, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:3, v/v) to give {2-[3-(1-benzyl-3-propoxy-1H-pyrazol-5-yl)propoxy]-3-methoxyphenyl}acetic acid as a colorless oil (351 mg, yield 53%). ¹H-NMR (300 MHz, CDCl₃) δ:1.00 (3 H, t, J = 7.4 Hz), 1.69 - 1.86 (2 H, m), 1.90-2.03 (2 H, m), 2.60-2.71 (2 H, m), 3.61 (2 H, s), 3.78 (3 H, s), 3.98 (2 H, t, J = 6.2 Hz), 4.05 (2 H, t, J= 6.7 Hz), 5.15 (2 H, s), 5.55 (1 H, s), 6.76 - 6.89 (2 H, m), 6.95 - 7.12 (3 H, m), 7.16 - 7.32 (3 H, m).

### Example 57

To a mixture of 3-[1-(2,4-dichlorobenzyl)-3-propoxy-1H-pyrazol-5-yl]propan-1-ol (515 mg), methyl (2-hydroxy-3-methoxyphenyl)acetate (294 mg), tributylphosphine (758 mg) and tetrahydrofuran (30 ml) was added 1,1'-azodicarbonyldipiperidine (757 mg) at 60°C, and the mixture was stirred for 15 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:5, v/v) to give a colorless oil. To a solution (12 ml) of the obtained oil in a mixed solvent of tetrahydrofuran-methanol (3:1, v/v) was added a 1N aqueous sodium hydroxide solution (3.0 ml) at 60°C, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:3, v/v) to give (2-{3-[1-(2,4-dichlorobenzyl)-3-propoxy-1H-pyrazol-5-yl]propoxy}-3-methoxyphenyl)acetic acid as a colorless oil (513 mg, yield 67%). ¹H-NMR (300 MHz, CDCl₃) δ:1.00 (3 H, t, J = 7.4 Hz), 1.69 - 1.85 (2 H, m), 1.91 - 2.05 (2 H, m), 2.59 - 2.72 (2 H, m), 3.61 (2 H, s), 3.79 (3 H, s), 3.99 (2 H, t, J = 6.1 Hz), 4.05 (2 H, t, J = 6.7 Hz), 5.19 (2 H, s), 5.60 (1 H, s), 6.55 (1 H, d, J = 8.5 Hz), 6.77 - 6.88 (2 H, m), 6.94 - 7.04 (1 H, m), 7.11 (1 H, dd, J = 8.5, 2.1 Hz), 7.34 (1 H, d, J = 2.1 Hz).

### Example 58

To a mixture of [1-isopropyl-5-(quinolin-2-ylmethoxy)-1H-pyrazol-3-yl]methanol (0.47 g), methyl (2-hydroxy-3-methylphenyl)acetate (0.31 g), tributylphosphine (0.70 g) and tetrahydrofuran (30 ml) was added 1,1'-azodicarbonyldipiperidine (0.90 g) at room temperature, and the mixture was stirred overnight. The reaction solution was concentrated, diisopropyl ether was added to the residue, and the insoluble substance was removed by filtration. The filtrate was concentrated, and the obtained residue was subjected to silica gel column chromatography to give a colorless oil.

A mixture of the obtained oil, a 1N aqueous sodium hydroxide solution (3.0 ml), tetrahydrofuran (5 ml) and methanol (5 ml) was stirred at room temperature for 3 hr. The reaction mixture was neutralized with 1N hydrochloric acid, and the mixture was extracted with ethyl acetate. The ethyl acetate layer was washed with saturated brine, dried (MgSO₄) and concentrated to give (2-{[1-isopropyl-5-(quinolin-2-ylmethoxy)-1H-pyrazol-3-yl]methoxy}-3-methylphenyl)acetic acid as colorless crystals (0.21 g, yield 30%). Crystallization from ethyl acetate-hexane gave colorless prism crystals. ¹H-NMR (300 MHz, CDCl₃) δ:1.38 - 1.42 (6 H, m), 2.29 (3 H, s), 3.68 (2 H, s), 4.67 (2 H, s), 4.70 - 4.85 (1 H, m), 5.71 (2 H, s), 5.81 (1 H, s), 6.94 - 7.14 (3 H, m), 8.13 - 8.26 (2 H, m).

### Example 59

To a mixture of 3-(3-propoxy-1-[4-(trifluoromethyl)benzyl]-1H-pyrazol-5-yl)propan-1-ol (500 mg), methyl (2-hydroxy-3-methoxyphenyl)acetate (290 mg), tributylphosphine (610 mg) and tetrahydrofuran (50 ml) was added 1,1'-azodicarbonyldipiperidine (760 mg) at room temperature, and the mixture was stirred for 15 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:4, v/v) to give a colorless oil (0.60 g). A mixture of the obtained oil, a 1N aqueous sodium hydroxide solution (3.0 ml), tetrahydrofuran (3.0 ml) and methanol (3.0 ml) was stirred at 60°C for 30 min, 1N hydrochloric acid (3.0 ml) and water were added, and the mixture was extracted with ethyl acetate. The ethyl acetate layer was washed with saturated brine, dried (MgSO₄) and concentrated. The residue was washed with hexane to give [3-methoxy-2-(3-{3-propoxy-1-[4-(trifluoromethyl)benzyl]-1H-pyrazol-5-yl}propoxy)phenyl]acetic acid (490 mg, yield 63%) as colorless crystals. Recrystallization from ethyl acetate-hexane gave colorless prism crystals. melting point 122 - 123°C.

### Example 60

To a mixture of 3-[1-(2,4-difluorobenzyl)-3-isopropoxy-1H-pyrazol-5-yl]propan-1-ol (340 mg), methyl (2-hydroxy-3-methoxyphenyl)acetate (236 mg), tributylphosphine (445 mg) and tetrahydrofuran (30 ml) was added 1,1'-azodicarbonyldipiperidine (555 mg), and the mixture was stirred at room temperature for 15 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:49 to 1:4, v/v) to give a pale-yellow oil (450 mg). A mixture of the obtained oil, a 1N aqueous sodium hydroxide solution (1.9 ml), tetrahydrofuran (8.0 ml) and methanol (8.0 ml) was stirred at 50°C for 15 hr, and 1N hydrochloric acid (3.0 ml) was added. The reaction solution was concentrated, and the residue was partitioned between ethyl acetate and water. The ethyl acetate layer was washed with saturated brine, dried (MgSO₄) and concentrated. The residue was subjected to silica gel column chromatography and eluted with ethyl acetate-hexane (1:9 to 1:0, v/v) to give (2-{3-[1-(2,4-difluorobenzyl)-3-isopropoxy-1H-pyrazol-5-yl]propoxy}-3-methoxyphenyl)acetic acid (430 mg, yield 98%) as a pale-yellow oil. ¹H-NMR (300 MHz, CDCl₃) δ:1.32 (6 H, d, J = 6.0 Hz), 1.92 - 2.04 (2 H, m), 2.65 - 2.75 (2 H, m), 3.63 (2 H, s), 3.79 (3 H, s), 3.98 - 4.06 (2 H, m), 4.60 - 4.72 (1 H, m), 5.13 (2 H, s), 5.52 (1 H, s), 6.70-7.04 (6 H, m).

### Example 61

To a mixture of 3-[1-(2-chlorobenzyl)-3-isopropoxy-1H-pyrazol-5-yl]propan-1-ol (990 mg), methyl (2-hydroxy-3-methoxyphenyl)acetate (693 mg), tributylphosphine (1.30 g) and tetrahydrofuran (100 ml) was added 1,1'-azodicarbonyldipiperidine (1.62 g), and the mixture was stirred at room temperature for 15 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:49 to 1:4, v/v) to give a pale-yellow oil (950 mg). A mixture of the obtained oil, a 1N aqueous sodium hydroxide solution (4.0 ml), tetrahydrofuran (15 ml) and methanol (15 ml) was stirred at 50°C for 2 hr, and 1N hydrochloric acid (6.0 ml) was added. The reaction solution was concentrated, and the residue was partitioned between ethyl acetate and water. The ethyl acetate layer was washed with saturated brine, dried (MgSO₄) and concentrated. The residue was subjected to silica gel column chromatography and eluted with ethyl acetate-hexane (1:9 to 1:1, v/v) to give (2-{3-[1-(2-chlorobenzyl)-3-isopropoxy-1H-pyrazol-5-yl]propoxy}-3-methoxyphenyl)acetic acid (810 mg, yield 88%) as a pale-yellow oil. ¹H-NMR (300 MHz, CDCl₃) δ:1.33 (6 H, d, J = 6.0 Hz), 1.92 - 2.06 (2 H, m), 2.60 - 2.69 (2 H, m), 3.60 (2 H, s), 3.77 (3 H, s), 3.94 - 4.04 (2 H, m), 4. 64 - 4.77 (1 H, m), 5.23 (2 H, s), 5.57 (1 H, s), 6.59 - 6.64 (1 H, m), 6.74 - 6.87 (2 H, m), 6.95 - 7.03 (1 H, m), 7.08 - 7.20 (2 H, m), 7.28 - 7.35 (1 H, m).

### Example 62

To a mixture of 3-[3-isopropoxy-1-(2-methoxybenzyl)-1H-pyrazol-5-yl]propan-1-ol (650 mg), methyl (2-hydroxy-3-methoxyphenyl)acetate (461 mg), tributylphosphine (866 mg) and tetrahydrofuran (50 ml) was added 1,1'-azodicarbonyldipiperidine (1.08 g), and the mixture was stirred at room temperature for 15 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:49 to 1:4, v/v) to give a pale-yellow oil (840 mg). A mixture of the obtained oil, a 1N aqueous sodium hydroxide solution (4.0 ml), tetrahydrofuran (15 ml) and methanol (15 ml) was stirred at 50°C for 2 hr, and 1N hydrochloric acid (6.0 ml) was added. The reaction solution was concentrated, and the residue was partitioned between ethyl acetate and water. The ethyl acetate layer was washed with saturated brine, dried (MgSO₄) and concentrated. The residue was subjected to silica gel column chromatography and eluted with ethyl acetate-hexane (1:9 to 1:1, v/v) to give (2-{3-[3-isopropoxy-1-(2-methoxybenzyl)-1H-pyrazol-5-yl]propoxy}-3-methoxyphenyl)acetic acid (750 mg, yield 92%) as a pale-yellow oil. ¹H-NMR (300 MHz, CDCl₃) δ:1.33 (6 H, d, J = 6.3 Hz), 1.92 - 2.04 (2 H, m), 2.62 - 2.70 (2 H, m), 3.60 (2 H, s), 3.78 (3 H, s), 3.82 (3 H, s), 3.95 - 4.04 (2 H, m), 4.64 - 4.77 (1 H, m), 5.14 (2 H, s), 5.53 (1 H, s), 6.61 - 6.67 (1 H, m), 6.77 - 6.87 (4 H, m), 6.95 - 7.02 (1 H, m), 7.05 - 7.12 (1 H, m) .

### Example 63

To a mixture of 3-[1-(2,3-dichlorobenzyl)-3-isopropoxy-1H-pyrazol-5-yl]propan-1-ol (770 mg), methyl (2-hydroxy-3-methoxyphenyl)acetate (483 mg), tributylphosphine (906 mg) and tetrahydrofuran (50 ml) was added 1,1'-azodicarbonyldipiperidine (1.13 g), and the mixture was stirred at room temperature for 15 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:49 to 1:4, v/v) to give a pale-yellow oil (770 mg). A mixture of the obtained oil, a 1N aqueous sodium hydroxide solution (4.0 ml), tetrahydrofuran (15 ml) and methanol (15 ml) was stirred at 50°C for 2 hr, and 1N hydrochloric acid (6.0 ml) was added. The reaction solution was concentrated, and the residue was partitioned between ethyl acetate and water. The ethyl acetate layer was washed with saturated brine, dried (MgSO₄) and concentrated. The residue was subjected to silica gel column chromatography and eluted with ethyl acetate-hexane (1:9 to 1:1, v/v) to give (2-{3-[1-(2,3-dichlorobenzyl)-3-isopropoxy-1H-pyrazol-5-yl]propoxy}-3-methoxyphenyl)acetic acid (610 mg, yield 81%) as a colorless oil. ¹H-NMR (300 MHz, CDCl₃) δ:1.33 (6 H, d, J = 6.3 Hz), 1.93 - 2.06 (2 H, m), 2.61 - 2.68 (2 H, m), 3.60 (2 H, s), 3.78 (3 H, s), 3.96 - 4.01 (2 H, m), 4.63 - 4.77 (1 H, m), 5.23 (2 H, s), 5.58 (1 H, s), 6.43 - 6.48 (1 H, m), 6.80 - 6.85 (2 H, m), 6.96 - 7.10 (1 H, m), 7.30 - 7.35 (1 H, m).

### Example 64

To a mixture of 3-[1-(2-chloro-4-fluorobenzyl)-3-isopropoxy-1H-pyrazol-5-yl]propan-1-ol (500 mg), methyl (2-hydroxy-3-methoxyphenyl)acetate (330 mg), tributylphosphine (619 mg) and tetrahydrofuran (40 ml) was added 1,1'-azodicarbonyldipiperidine (772 mg), and the mixture was stirred at room temperature for 15 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:49 to 1:4, v/v) to give a colorless oil (490 mg). A mixture of the obtained oil, a 1N aqueous sodium hydroxide solution (4.0 ml), tetrahydrofuran (15 ml) and methanol (15 ml) was stirred at 50°C for 2 hr, and 1N hydrochloric acid (6.0 ml) was added. The reaction solution was concentrated, and the residue was partitioned between ethyl acetate and water. The ethyl acetate layer was washed with saturated brine, dried (MgSO₄) and concentrated. The residue was subjected to silica gel column chromatography and eluted with ethyl acetate-hexane (1:9 to 2:3, v/v) to give (2-{3-[1-(2-chloro-4-fluorobenzyl)-3-isopropoxy-1H-pyrazol-5-yl]propoxy}-3-methoxyphenyl)acetic acid (320 mg, yield 67%) as a colorless oil. ¹H-NMR (300 MHz, CDCl₃) δ:1.33 (6 H, d, J = 6.3 Hz), 1.93 - 2.03 (2 H, m), 2.62 - 2.69 (2 H, m), 3.61 (2 H, s), 3.78 (3 H, s), 3.95 - 4.01 (2 H, m), 4.63 - 4.75 (1 H, m), 5.17 (2 H, s), 5.57 (1 H, s), 6.61 (1 H, dd, J = 6.2, 8.9 Hz), 6.78 - 6.90 (3 H, m), 6.96 - 7.25 (2 H, m).

### Example 65

To a mixture of 3-[1-(4-chloro-2-fluorobenzyl)-3-isopropoxy-1H-pyrazol-5-yl]propan-1-ol (680 mg), methyl (2-hydroxy-3-methoxyphenyl)acetate (449 mg), tributylphosphine (842 mg) and tetrahydrofuran (50 ml) was added 1,1'-azodicarbonyldipiperidine (1.05 g), and the mixture was stirred at room temperature for 15 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:49 to 1:4, v/v) to give a colorless oil (630 mg). A mixture of the obtained oil, a 1N aqueous sodium hydroxide solution (4.0 ml), tetrahydrofuran (15 ml) and methanol (15 ml) was stirred at 50°C for 2 hr, and 1N hydrochloric acid (6.0 ml) was added. The reaction solution was concentrated, and the residue was partitioned between ethyl acetate and water. The ethyl acetate layer was washed with saturated brine, dried (MgSO₄) and concentrated. The residue was subjected to silica gel column chromatography and eluted with ethyl acetate-hexane (1:19 to 2:3, v/v) to give (2-{3-[1-(4-chloro-2-fluorobenzyl)-3-isopropoxy-1H-pyrazol-5-yl]propoxy}-3-methoxyphenyl)acetic acid (500 mg, yield 82%) as a colorless oil. ¹H-NMR (300 MHz, CDCl₃) δ:1.32 (6 H, d, J = 6.3 Hz), 1.96 - 2.07 (2 H, m), 2.66 - 2.73 (2 H, m), 3.63 (2 H, s), 3.79 (3 H, s), 3.97 - 4.01 (2 H, m), 4.61 - 4.73 (1 H, m), 5.14 (2 H, s), 5.54 (1 H, s), 6.78 - 6.87 (3 H, m), 6.96 - 7.04 (3 H, m) .

### Example 66

To a mixture of 3-{1-[2-chloro-4-(trifluoromethyl)benzyl]-3-isopropoxy-1H-pyrazol-5-yl}propan-1-ol (390 mg), methyl (2-hydroxy-3-methoxyphenyl)acetate (201 mg), tributylphosphine (376 mg) and tetrahydrofuran (30 ml) was added 1,1'-azodicarbonyldipiperidine (469 mg), and the mixture was stirred at room temperature for 10 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:19 to 1:4, v/v) to give a colorless oil (110 mg). A mixture of the obtained oil, a 1N aqueous sodium hydroxide solution (1.0 ml), tetrahydrofuran (5.0 ml) and methanol (5.0 ml) was stirred at 50°C for 2 hr, and 1N hydrochloric acid (2.0 ml) was added. The reaction solution was concentrated, and the residue was partitioned between ethyl acetate and water. The ethyl acetate layer was washed with saturated brine, dried (MgSO₄) and concentrated. The residue was subjected to silica gel column chromatography and eluted with ethyl acetate-hexane (1:19 to 1:1, v/v). The eluate was concentrated, and the residue was crystallized from ethyl acetate-hexane to give [2-(3-{1-[2-chloro-4-(trifluoromethyl)benzyl]-3-isopropoxy-1H-pyrazol-5-yl}propoxy)-3-methoxyphenyl]acetic acid (68 mg, yield 63%) as colorless crystals. melting point 98 - 99°C.

### Example 67

To a mixture of 3-[1-(2-chloro-6-fluorobenzyl)-3-isopropoxy-1H-pyrazol-5-yl]propan-1-ol (580 mg), methyl (2-hydroxy-3-methoxyphenyl)acetate (382 mg), tributylphosphine (716 mg) and tetrahydrofuran (40 ml) was added 1,1'-azodicarbonyldipiperidine (893 mg), and the mixture was stirred at room temperature for 15 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:49 to 1:4, v/v) to give a colorless oil (820 mg). A mixture of the obtained oil, a 1N aqueous sodium hydroxide solution (4.0 ml), tetrahydrofuran (15 ml) and methanol (15 ml) was stirred at 50°C for 2 hr, and 1N hydrochloric acid (6.0 ml) was added. The reaction solution was concentrated, and the residue was partitioned between ethyl acetate and water. The ethyl acetate layer was washed with saturated brine, dried (MgSO₄) and concentrated. The residue was subjected to silica gel column chromatography and eluted with ethyl acetate-hexane (1:19 to 1:1, v/v). The eluate was concentrated, and the residue was crystallized from ethyl acetate-hexane to give (2-{3-[1-(2-chloro-6-fluorobenzyl)-3-isopropoxy-1H-pyrazol-5-yl]propoxy}-3-methoxyphenyl)acetic acid (620 mg, yield 78%) as colorless crystals. melting point 97 - 98°C.

### Example 68

To a mixture of 3-[1-(4-chloro-2-methylbenzyl)-3-isopropoxy-1H-pyrazol-5-yl]propan-1-ol (820 mg), methyl (2-hydroxy-3-methoxyphenyl)acetate (548 mg), tributylphosphine (1.03 g) and tetrahydrofuran (60 ml) was added 1,1'-azodicarbonyldipiperidine (1.28 g), and the mixture was stirred at room temperature for 15 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:49 to 1:4, v/v) to give a colorless oil (1.07 g). A mixture of the obtained oil, a 1N aqueous sodium hydroxide solution (6.0 ml), tetrahydrofuran (25 ml) and methanol (25 ml) was stirred at 50°C for 2 hr, and 1N hydrochloric acid (8.0 ml) was added. The reaction solution was concentrated, and the residue was partitioned between ethyl acetate and water. The ethyl acetate layer was washed with saturated brine, dried (MgSO₄) and concentrated. The residue was subjected to silica gel column chromatography and eluted with ethyl acetate-hexane (1:19 to 1:1, v/v). The eluate was concentrated, and the residue was crystallized from ethyl acetate-hexane to give (2-{3-[1-(4-chloro-2-methylbenzyl)-3-isopropoxy-1H-pyrazol-5-yl]propoxy}-3-methoxyphenyl)acetic acid (850 mg, yield 82%) as colorless crystals. melting point 108 - 109°C.

### Example 69

To a mixture of 3-[1-(4-chloro-2-ethoxybenzyl)-3-isopropoxy-1H-pyrazol-5-yl]propan-1-ol (880 mg), methyl (2-hydroxy-3-methoxyphenyl)acetate (537 mg), tributylphosphine (1.01 g) and tetrahydrofuran (60 ml) was added 1,1'-azodicarbonyldipiperidine (1.26 g), and the mixture was stirred at room temperature for 15 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:24 to 1:4, v/v) to give a colorless oil (850 mg). A mixture of the obtained oil, a 1N aqueous sodium hydroxide solution (4.0 ml), tetrahydrofuran (15 ml) and ethanol (15 ml) was stirred at 50°C for 2 hr, and 1N hydrochloric acid (6.0 ml) was added. The reaction solution was concentrated, and the residue was partitioned between ethyl acetate and water. The ethyl acetate layer was washed with saturated brine, dried (MgSO₄) and concentrated. The residue was subjected to silica gel column chromatography and eluted with ethyl acetate-hexane (1:19 to 1:1, v/v). The eluate was concentrated, and the residue was crystallized from ethyl acetate-hexane to give (2-{3-[1-(4-chloro-2-ethoxybenzyl)-3-isopropoxy-1H-pyrazol-5-yl]propoxy}-3-methoxyphenyl)acetic acid (630 mg, yield 76%) as colorless crystals. melting point 98 - 99°C.

### Example 70

To a mixture of 3-[1-(4-chloro-2-isopropoxybenzyl)-3-isopropoxy-1H-pyrazol-5-yl]propan-1-ol (100 mg), methyl (2-hydroxy-3-methoxyphenyl)acetate (59 mg), tributylphosphine (110 mg) and tetrahydrofuran (5.0 ml) was added 1,1'-azodicarbonyldipiperidine (139 mg), and the mixture was stirred at room temperature for 15 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:49 to 1:4, v/v) to give a colorless oil (80 mg). A mixture of the obtained oil (50 mg), a 1N aqueous sodium hydroxide solution (0.5 ml), tetrahydrofuran (4.0 ml) and methanol (4.0 ml) was stirred at 50°C for 1 hr, and 1N hydrochloric acid (1.0 ml) was added. The reaction solution was concentrated, and the residue was partitioned between ethyl acetate and water. The ethyl acetate layer was washed with saturated brine, dried (MgSO₄) and concentrated. The residue was subjected to silica gel column chromatography and eluted with ethyl acetate-hexane (1:19 to 1:1, v/v) to give (2-{3-[1-(4-chloro-2-isopropoxybenzyl)-3-isopropoxy-1H-pyrazol-5-yl]propoxy}-3-methoxyphenyl)acetic acid (50 mg, yield 64%) as a colorless oil. ¹H-NMR (300 MHz, CDCl₃) δ:1.31 (12 H, d, J = 6.3 Hz), 1.94 - 2.04 (2 H, m), 2.61 - 2.68 (2 H, m), 3.60 (2 H, s), 3.77 (3 H, s), 3.94 - 4.01 (2 H, m), 4.45 - 4.57 (1 H, m), 4.61 - 4.73 (1 H, m), 5.06 (2 H, s), 5.52 (1 H, s), 6.54 (1 H, d, J = 7.8 Hz), 6.72 - 6.84 (4 H, m), 6.98 (1 H, dd, J = 7.8, 8.1 Hz).

### Example 71

To a mixture of 3-[1-(4-chloro-2-isopropoxybenzyl)-3-isopropoxy-1H-pyrazol-5-yl]propan-1-ol (480 mg), methyl (1-benzyl-3-hydroxy-1H-pyrazol-4-yl)acetate (354 mg), tributylphosphine (530 mg) and tetrahydrofuran (30 ml) was added 1,1'-azodicarbonyldipiperidine (661 mg), and the mixture was stirred at room temperature for 15 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:49 to 1:4, v/v) to give a yellow oil (600 mg). A mixture of the obtained oil, a 1N aqueous sodium hydroxide solution (4.0 ml), tetrahydrofuran (15 ml) and ethanol (15 ml) was stirred at 50°C for 1 hr, and 1N hydrochloric acid (6.0 ml) was added. The reaction solution was concentrated, and the residue was partitioned between ethyl acetate and water. The ethyl acetate layer was washed with saturated brine, dried (MgSO₄) and concentrated. The residue was subjected to silica gel column chromatography and eluted with ethyl acetate-hexane (1:19 to 1:1, v/v) to give (1-benzyl-3-{3-[1-(4-chloro-2-isopropoxybenzyl)-3-isopropoxy-1H-pyrazol-5-yl]propoxy}-1H-pyrazol-4-yl)acetic acid (560 mg, yield 95%) as a colorless oil. ¹H-NMR (300 MHz, CDCl₃) δ:1.31 (6 H, d, J = 6.0 Hz), 1.33 (6 H, d, J = 5.8 Hz), 1.94 - 2.08 (2 H, m), 2.56 - 2.64 (2 H, m), 3.33 (2 H, s), 4.16 - 4.22 (2 H, m), 4.47 - 4.60 (1 H, m), 4.61 - 4.73 (1 H, m), 5.04 (2 H, s), 5.07 (2 H, s), 5.52 (1 H, s), 6.54 (1 H, d, J = 8.1 Hz), 7.14 - 7.21 (4 H, m), 7.27 - 7.36 (3 H, m).

### Example 72

To a mixture of (1-benzyl-3-{3-[1-(4-chloro-2-isopropoxybenzyl)-3-isopropoxy-1H-pyrazol-5-yl]propoxy}-1H-pyrazol-4-yl)acetic acid (560 mg), tetrahydrofuran (10 ml) and ethanol (10 ml) was added a 1N aqueous sodium hydroxide solution (1.1 ml), and the mixture was stirred at room temperature for 1 hr. The reaction mixture was concentrated, and the residue was dissolved in water (20 ml). To the solution was added a solution of calcium chloride (213 mg) in water (3 ml), and the mixture was stirred at room temperature for 5 min. The resulting precipitate was collected by filtration and dried to give (1-benzyl-3-{3-[1-(4-chloro-2-isopropoxybenzyl)-3-isopropoxy-1H-pyrazol-5-yl]propoxy}-1H-pyrazol-4-yl)acetic acid 1/2 calcium salt (350 mg, yield 61%) as a colorless solid. elemental analysis C₃₁H₃₆N₄O₅Cl·0.5Ca·0.5H₂O : Calculated C, 61.12; H, 6.12; N, 9.20. Found C, 60.89; H, 6.15; N, 9.20.

### Example 73

To a mixture of 3-[1-(4-chloro-2-methylbenzyl)-3-isopropoxy-1H-pyrazol-5-yl]propan-1-ol (1.27 g), methyl (1-benzyl-3-hydroxy-1H-pyrazol-4-yl)acetate (1.06 g), tributylphosphine (1.59 g) and tetrahydrofuran (50 ml) was added 1,1'-azodicarbonyldipiperidine (1.98 g), and the mixture was stirred at room temperature for 2 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:49 to 1:4, v/v) to give a yellow oil (600 mg). A mixture of the obtained oil (1.32 g), a 1N aqueous sodium hydroxide solution (6.0 ml), tetrahydrofuran (20 ml) and methanol (20 ml) was stirred at 50°C for 1 hr, and 1N hydrochloric acid (8.0 ml) was added. The reaction solution was concentrated, and the residue was partitioned between ethyl acetate and water. The ethyl acetate layer was washed with saturated brine, dried (MgSO₄) and concentrated. The residue was subjected to silica gel column chromatography and eluted with ethyl acetate-hexane (1:19 to 1:1, v/v). The eluate was concentrated, and the residue was crystallized from ethyl acetate-hexane to give (1-benzyl-3-{3-[1-(4-chloro-2-methylbenzyl)-3-isopropoxy-1H-pyrazol-5-yl]propoxy}-1H-pyrazol-4-yl)acetic acid (1.02 g, yield 79%) as colorless crystals. melting point 81 - 82°C.

### Example 74

To a mixture of 3-[1-(4-chloro-2-methylbenzyl)-3-isopropoxy-1H-pyrazol-5-yl]propan-1-ol (570 mg), methyl (2-hydroxypyridin-3-yl)acetate (326 mg), tributylphosphine (716 mg) and tetrahydrofuran (25 ml) was added 1,1'-azodicarbonyldipiperidine (893 mg), and the mixture was stirred at room temperature for 15 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:14 to 1:3, v/v) to give a yellow oil (480 mg). A mixture of the obtained oil, a 1N aqueous sodium hydroxide solution (4.0 ml), tetrahydrofuran (15 ml) and methanol (15 ml) was stirred at 50°C for 1 hr, and 1N hydrochloric acid (8.0 ml) was added. The reaction solution was concentrated, and the residue was partitioned between ethyl acetate and water. The ethyl acetate layer was washed with saturated brine, dried (MgSO₄) and concentrated. The residue was subjected to silica gel column chromatography and eluted with ethyl acetate-hexane (1:19 to 1:1, v/v). The eluate was concentrated, and the residue was crystallized from ethyl acetate-hexane to give (2-{3-[1-(4-chloro-2-methylbenzyl)-3-isopropoxy-1H-pyrazol-5-yl]propoxy}pyridin-3-yl)acetic acid (290 mg, yield 62%) as colorless crystals. melting point 97 - 98°C.

### Example 75

To a mixture of 3-{1-[2-chloro-4-(trifluoromethyl)benzyl]-3-isopropyl-1H-pyrazol-5-yl}propan-1-ol (230 mg), methyl (1-benzyl-3-hydroxy-1H-pyrazol-4-yl)acetate (173 mg), tributylphosphine (259 mg) and tetrahydrofuran (15 ml) was added 1'-azodicarbonyldipiperidine (323 mg), and the mixture was stirred at room temperature for 15 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:49 to 1:4, v/v) to give a pale-yellow oil (290 mg). A mixture of the obtained oil, a 1N aqueous sodium hydroxide solution (3.0 ml), tetrahydrofuran (10 ml) and methanol (10 ml) was stirred at 50°C for 1 hr, and 1N hydrochloric acid (5.0 ml) was added. The reaction solution was concentrated, and the residue was partitioned between ethyl acetate and water. The ethyl acetate layer was washed with saturated brine, dried (MgSO₄) and concentrated. The residue was subjected to silica gel column chromatography and eluted with ethyl acetate-hexane (1:19 to 1:1, v/v) to give [1-benzyl-3-(3-{1-[2-chloro-4-(trifluoromethyl)benzyl]-3-isopropyl-1H-pyrazol-5-yl}propoxy)-1H-pyrazol-4-yl]acetic acid (280 mg, yield 99%) as a pale-yellow oil. ¹H-NMR (300 MHz, CDCl₃) δ:1.25 (6 H, d, J = 6.9 Hz), 1.94 - 2.07 (2 H, m), 2.53 - 2.64 (2 H, m), 2.90 - 3.04 (1 H, m), 3.28 (2 H, s), 4.13 - 4.22 (2 H, m), 5.06 (2 H, s), 5.36 (2 H, s), 6.00 (1 H, s), 6.52 (1 H, d, J = 8.1 Hz), 7.12 - 7.20 (3 H, m), 7.23 - 7.37 (4 H, m), 7.59 (1 H, d, J = 1.0 Hz).

### Example 76

To a mixture of [1-benzyl-3-(3-{1-[2-chloro-4-(trifluoromethyl)benzyl]-3-isopropyl-1H-pyrazol-5-yl}propoxy)-1H-pyrazol-4-yl]acetic acid (280 mg), tetrahydrofuran (5.0 ml) and ethanol (5.0 ml) was added a 1N aqueous sodium hydroxide solution (0.55 ml), and the mixture was stirred at room temperature for 1 hr. The reaction mixture was concentrated, and the residue was dissolved in water (10 ml). To the solution was added a solution of calcium chloride (108 mg) in water (3 ml), and the mixture was stirred at room temperature for 5 min. The resulting precipitate was collected by filtration and dried to give [1-benzyl-3-(3-{1-[2-chloro-4-(trifluoromethyl)benzyl]-3-isopropyl-1H-pyrazol-5-yl}propoxy)-1H-pyrazol-4-yl]acetic acid 1/2 calcium salt (150 mg, yield 52%) as a colorless solid. ¹H-NMR (300 MHz, DMSO-d₆) δ:1.15 (6 H, d, J = 6.9 Hz), 1.83 - 1.98 (2 H, m), 2.56 - 2.67 (2 H, m), 2.72 - 2.90 (1 H, m), 2.92 (2 H, s), 3.96 - 4.07 (2 H, m), 5.02 (2 H, s), 5.34 (2 H, s), 6.03 (1 H, s), 6.66 (1 H, d, J = 8.1 Hz), 7.10 - 7.33 (5 H, m), 7.46 (1 H, s), 7.62 (1 H, d, J = 8.1 Hz), 7.88 (1 H, s).

### Example 77

To a mixture of 3-{1-[2-chloro-4-(trifluoromethyl)benzyl]-3-isopropyl-1H-pyrazol-5-yl}propan-1-ol (250 mg), methyl (2-hydroxypyridin-3-yl)acetate (127 mg), tributylphosphine (280 mg) and tetrahydrofuran (15 ml) was added 1,1*'-*azodicarbonyldipiperidine (348 mg), and the mixture was stirred at room temperature for 15 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:24 to 1:3, v/v) to give a colorless oil (190 mg). A mixture of the obtained oil, a 1N aqueous sodium hydroxide solution (2.0 ml), tetrahydrofuran (8.0 ml) and methanol (8.0 ml) was stirred at 50°C for 1 hr, and 1N hydrochloric acid (2.5 ml) was added. The reaction solution was concentrated, and the residue was partitioned between ethyl acetate and water. The ethyl acetate layer was washed with saturated brine, dried (MgSO₄) and concentrated. The residue was subjected to silica gel column chromatography and eluted with ethyl acetate-hexane (1:19 to 1:1, v/v). The eluate was concentrated, and the residue was crystallized from ethyl acetate-hexane to give [2-(3-{1-[2-chloro-4-(trifluoromethyl)benzyl]-3-isopropyl-1H-pyrazol-5-yl}propoxy)pyridin-3-yl]acetic acid (130 mg, yield 70%) as colorless crystals. melting point 114 - 115°C.

### Example 78

To a mixture of 3-{3-isopropoxy-1-[2-methyl-4-(trifluoromethyl)benzyl]-1H-pyrazol-5-yl}propan-1-ol (400 mg), methyl (1-benzyl-3-hydroxy-1H-pyrazol-4-yl)acetate (303 mg), tributylphosphine (453 mg) and tetrahydrofuran (25 ml) was added 1,1'-azodicarbonyldipiperidine (565 mg), and the mixture was stirred at room temperature for 4 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:49 to 1:4, v/v) to give a pale-yellow oil (630 mg). A mixture of the obtained oil, a 1N aqueous sodium hydroxide solution (4.0 ml), tetrahydrofuran (15 ml) and ethanol (15 ml) was stirred at 50°C for 1 hr, and 1N hydrochloric acid (6.0 ml) was added. The reaction solution was concentrated, and the residue was partitioned between ethyl acetate and water. The ethyl acetate layer was washed with saturated brine, dried (MgSO₄) and concentrated. The residue was subjected to silica gel column chromatography and eluted with ethyl acetate-hexane (1:19 to 1:1, v/v) to give [1-benzyl-3-(3-{3-isopropoxy-1-[2-methyl-4-(trifluoromethyl)benzyl]-1H-pyrazol-5-yl}propoxy)-1H-pyrazol-4-yl]acetic acid (600 mg, yield 98%) as a colorless oil. ¹H-NMR (300 MHz, CDCl₃) δ:1.32 (6 H, d, J = 6.2 Hz), 1.94 - 2.08 (2 H, m), 2.33 (3 H, s), 2.52 - 2.61 (2 H, m), 3.30 (2 H, s), 4.15 - 4.23 (2 H, m), 4.62 - 4.75 (1 H, m), 5.06 (2 H, s), 5.10 (2 H, s), 5.58 (1 H, s), 6.57 (1 H, d, J = 8.1 Hz), 7.13 - 7.21 (3 H, m), 7.27 - 7.39 (5 H, m).

### Example 79

To a mixture of [1-benzyl-3-(3-{3-isopropoxy-1-[2-methyl-4-(trifluoromethyl)benzyl]-1H-pyrazol-5-yl}propoxy)-1H-pyrazol-4-yl]acetic acid (600 mg), tetrahydrofuran (10 ml) and ethanol (10 ml) was added a 1N aqueous sodium hydroxide solution (1.2 ml), and the mixture was stirred at room temperature for 1 hr. The reaction mixture was concentrated, and the residue was dissolved in water (20 ml). To the solution was added a solution of calcium chloride (233 mg) in water (5 ml), and the mixture was stirred at room temperature for 5 min. The resulting precipitate was collected by filtration and dried to give [1-benzyl-3-(3-{3-isopropoxy-1-[2-methyl-4-(trifluoromethyl)benzyl]-1H-pyrazol-5-yl}propoxy)-1H-pyrazol-4-yl]acetic acid 1/2 calcium salt (470 mg, yield 76%) as a pale-yellow solid. ¹H-NMR (300 MHz, DMSO-d₆) δ:1.21 (6 H, d, J = 6.0 Hz), 1.78 - 1.96 (2 H, m), 2.35 (3 H, s), 2.50 - 2.65 (2 H, m), 2.98 (2 H, s), 3.85 - 4.10 (2 H, m), 4.51 - 4.64 (1 H, m), 5.16 (2 H, s), 5.59 (1 H, s), 6.56 (1 H, d, J = 8.1 Hz), 7.05 - 7.33 (5 H, m), 7.37 - 7.60 (3 H, m).

### Example 80

To a mixture of 3-{3-isopropoxy-1-[2-methyl-4-(trifluoromethyl)benzyl]-1H-pyrazol-5-yl}propan-1-ol (500 mg), methyl (2-hydroxypyridin-3-yl)acetate (257 mg), tributylphosphine (566 mg) and tetrahydrofuran (30 ml) was added 1,1'-azodicarbonyldipiperidine (706 mg), and the mixture was stirred at room temperature for 4 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:49 to 1:2, v/v) to give a pale-yellow oil (420 mg). A mixture of the obtained oil, a 1N aqueous sodium hydroxide solution (4.0 ml), tetrahydrofuran (15 ml) and methanol (15 ml) was stirred at 50°C for 1 hr, and 1N hydrochloric acid (6.0 ml) was added. The reaction solution was concentrated, and the residue was partitioned between ethyl acetate and water. The ethyl acetate layer was washed with saturated brine, dried (MgSO₄) and concentrated. The residue was subjected to silica gel column chromatography and eluted with ethyl acetate-hexane (1:9 to 2:3, v/v). The eluate was concentrated, and the residue was crystallized from ethyl acetate-hexane to give [2-(3-{3-isopropoxy-1-[2-methyl-4-(trifluoromethyl)benzyl]-1H-pyrazol-5-yl}propoxy)pyridin-3-yl]acetic acid (290 mg, yield 71%) as colorless crystals. melting point 105 - 107°C.

### Example 81

To a mixture of 3-[1-(4-chlorobenzyl)-3-propoxy-1H-pyrazol-5-yl]propan-1-ol (500 mg), methyl (2-hydroxy-3-methoxyphenyl)acetate (318 mg), tributylphosphine (1.01 g) and tetrahydrofuran (30 ml) was added 1,1'-azodicarbonyldipiperidine (817 mg) at 50°C, and the mixture was stirred for 15 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:10 to 1:2, v/v) to give a colorless oil (0.58 g). A mixture of the obtained oil, a 1N aqueous sodium hydroxide solution (2.5 ml), tetrahydrofuran (10 ml) and methanol (10 ml) was stirred at 60°C for 30 min, 1N hydrochloric acid (2.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:25 to 2:1, v/v). The elute was concentrated, and the residue was crystallized from ethyl acetate-hexane to give (2-{3-[1-(4-chlorobenzyl)-3-propoxy-1H-pyrazol-5-yl]propoxy}-3-methoxyphenyl)acetic acid (449 mg, yield 80%) as colorless crystals. melting point 131 - 132°C.

### Example 82

To a mixture of 3-[1-(2,4-difluorobenzyl)-3-propoxy-1H-pyrazol-5-yl]propan-1-ol (310 mg), methyl (2-hydroxy-3-methoxyphenyl)acetate (196 mg), tributylphosphine (0.623 ml) and tetrahydrofuran (15 ml) was added 1,1'-azodicarbonyldipiperidine (505 mg) at 60°C, and the mixture was stirred for 15 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:10 to 1:2, v/v) to give a colorless oil (0.38 g). A mixture of the obtained oil, a 1N aqueous sodium hydroxide solution (1.5 ml), tetrahydrofuran (7 ml) and methanol (7 ml) was stirred at 60°C for 30 min, 1N hydrochloric acid (1.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:25 to 2:1, v/v). The elute was concentrated, and the residue was crystallized from ethyl acetate-hexane to give (2-{3-[1-(2,4-difluorobenzyl)-3-propoxy-1H-pyrazol-5-yl]propoxy}-3-methoxyphenyl)acetic acid (221 mg, yield 60%) as colorless crystals. melting point 77 - 80°C.

### Example 83

To a mixture of 3-[1-(2-methoxybenzyl)-3-propoxy-1H-pyrazol-5-yl]propan-l-ol (304 mg), methyl (2-hydroxy-3-methoxyphenyl)acetate (196 mg), tributylphosphine (0.623 ml) and tetrahydrofuran (15 ml) was added 1,1'-azodicarbonyldipiperidine (505 mg) at 60°C, and the mixture was stirred for 15 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:10 to 1:2, v/v) to give a colorless oil (0.32 g). A mixture of the obtained oil, a 1N aqueous sodium hydroxide solution (1.5 ml), tetrahydrofuran (7 ml) and methanol (7 ml) was stirred at 60°C for 30 min, 1N hydrochloric acid (1.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:25 to 2:1, v/v). The elute was concentrated, and the residue was crystallized from ethyl acetate-hexane to give (2-{3-[1-(2-methoxybenzyl)-3-propoxy-1H-pyrazol-5-yl]propoxy}-3-methoxyphenyl)acetic acid (254 mg, yield 82%) as colorless crystals. melting point 79 - 82°C.

### Example 84

To a mixture of 3-[1-(4-chloro-2-methoxybenzyl)-3-isopropoxy-1H-pyrazol-5-yl]propan-1-ol (310 mg), methyl (2-hydroxy-3-methoxyphenyl)acetate (196 mg), tributylphosphine (0.623 ml) and tetrahydrofuran (15 ml) was added 1,1'-azodicarbonyldipiperidine (505 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:10 to 1:4, v/v) to give a colorless oil (0.27 g). A mixture of the obtained oil, a 1N aqueous sodium hydroxide solution (1.2 ml), tetrahydrofuran (3 ml) and methanol (3 ml) was stirred at 60°C for 1 hr, 1N hydrochloric acid (1.2 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 2:1, v/v) to give (2-{3-[1-(4-chloro-2-methoxybenzyl)-3-isopropoxy-1H-pyrazol-5-yl]propoxy}-3-methoxyphenyl)acetic acid (0.22 g, yield 84%) as a colorless oil. ¹H-NMR (300 MHz, CDCl₃) δ:1.32 (6 H, d, J = 6.0 Hz), 1.92 - 2.04 (2 H, m), 2.58 - 2.74 (2 H, m), 3.62 (2 H, s), 3.79 (3 H, s), 3.81 (3 H, s), 4.00 (2 H, t, J = 6.2 Hz), 4.60 - 4.77 (1 H, m), 5.09 (2 H, s), 5.53 (1 H, s), 6.53 - 6.61 (1 H, m), 6.76 - 6.88 (4 H, m), 6.95 - 7.06 (1 H, m).

### Example 85

To a mixture of 3-[1-(2,4-dichlorobenzyl)-3-isopropoxy-1H-pyrazol-5-yl]propan-1-ol (172 mg), methyl (2-hydroxy-1-naphthyl)acetate (108 mg), tributylphosphine (0.312 ml) and tetrahydrofuran (15 ml) was added 1,1'-azodicarbonyldipiperidine (252 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:10 to 1:4, v/v) to give a colorless oil (0.15 g). A mixture of the obtained oil, a 1N aqueous sodium hydroxide solution (0.8 ml), tetrahydrofuran (1.5 ml) and methanol (1.5 ml) was stirred at 60°C for 1 hr, 1N hydrochloric acid (0.8 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 2:1, v/v). The elute was concentrated, and the residue was crystallized from ethyl acetate-hexane to give (2-{3-[1-(2,4-dichlorobenzyl)-3-isopropoxy-1H-pyrazol-5-yl]propoxy}-1-naphthyl)acetic acid (132 mg, yield 90%) as colorless crystals. melting point 120 - 122°C.

### Example 86

To a mixture of 3-[1-(2,4-dichlorobenzyl)-3-isopropoxy-1H-pyrazol-5-yl]propan-1-ol (172 mg), methyl (1-benzyl-3-hydroxy-1H-pyrazol-4-yl)acetate (123 mg), tributylphosphine (0.312 ml) and tetrahydrofuran (15 ml) was added 1,1'-azodicarbonyldipiperidine (252 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:10 to 1:4, v/v) to give a colorless oil (0.23 g). A mixture of the obtained oil, a 1N aqueous sodium hydroxide solution (1.0 ml), tetrahydrofuran (3.0 ml) and methanol (3.0 ml) was stirred at 60°C for 1 hr, 1N hydrochloric acid (1.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 2:1, v/v). The elute was concentrated, and the residue was crystallized from ethyl acetate-hexane to give (1-benzyl-3-{3-[1-(2,4-dichlorobenzyl)-3-isopropoxy-1H-pyrazol-5-yl]propoxy}-1H-pyrazol-4-yl)acetic acid (154 mg, yield 69%) as colorless crystals. melting point 108 - 110°C.

### Example 87

To a mixture of 3-[1-(2,4-dichlorobenzyl)-3-isopropoxy-1H-pyrazol-5-yl]propan-1-ol (172 mg), methyl (1-hydroxy-2-naphthyl)acetate (108 mg), tributylphosphine (0.312 ml) and tetrahydrofuran (15 ml) was added 1,1'-azodicarbonyldipiperidine (252 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:10 to 1:4, v/v) to give a colorless oil (0.13 g). A mixture of the obtained oil, a 1N aqueous sodium hydroxide solution (0.6 ml), tetrahydrofuran (1.5 ml) and methanol (1.5 ml) was stirred at 60°C for 1 hr, 1N hydrochloric acid (0.6 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 2:1, v/v). The elute was concentrated, and the residue was crystallized from ethyl acetate-hexane to give (1-{3-[1-(2,4-dichlorobenzyl)-3-isopropoxy-1H-pyrazol-5-yl]propoxy}-2-naphthyl)acetic acid (101 mg, yield 80%) as colorless crystals. melting point 96 - 98°C.

### Example 88

To a mixture of 3-[1-(2,4-dichlorobenzyl)-3-propoxy-1H-pyrazol-5-yl]propan-1-ol (343 mg), methyl (3-hydroxy-1-methyl-1H-pyrazol-4-yl)acetate (170 mg), tributylphosphine (0.623 ml) and tetrahydrofuran (15 ml) was added 1,1'-azodicarbonyldipiperidine (505 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:20 to 1:1, v/v) to give a colorless oil (0.44 g). A mixture of the obtained oil, a 1N aqueous sodium hydroxide solution (1.9 ml), tetrahydrofuran (5.0 ml) and methanol (5.0 ml) was stirred at 50°C for 1 hr, 1N hydrochloric acid (1.9 ml) was added, and the mixture was extracted with ethyl acetate. The ethyl acetate layer was washed with saturated brine, dried (MgSO₄) and concentrated. The residue was crystallized from ethyl acetate-hexane to give (3-{3-[1-(2,4-dichlorobenzyl)-3-propoxy-1H-pyrazol-5-yl]propoxy}-1-methyl-1H-pyrazol-4-yl)acetic acid (321 mg, yield 75%) as colorless crystals, melting point 77 - 81°C.

### Example 89

To a mixture of 3-[1-(2,4-dichlorobenzyl)-3-propoxy-1H-pyrazol-5-yl]propan-1-ol (343 mg), methyl (1-benzyl-3-hydroxy-1H-pyrazol-4-yl)acetate (246 mg), tributylphosphine (0.623 ml) and tetrahydrofuran (15 ml) was added 1,1'-azodicarbonyldipiperidine (505 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:20 to 1:3, v/v) to give a colorless oil (0.47 g). A mixture of the obtained oil, a 1N aqueous sodium hydroxide solution (1.9 ml), tetrahydrofuran (5.0 ml) and methanol (5.0 ml) was stirred at 50°C for 1 hr, 1N hydrochloric acid (1.9 ml) was added, and the mixture was extracted with ethyl acetate. The ethyl acetate layer was washed with saturated brine, dried (MgSO₄) and concentrated. The residue was crystallized from ethyl acetate-hexane to give (1-benzyl-3-{3-[1-(2,4-dichlorobenzyl)-3-propoxy-1H-pyrazol-5-yl]propoxy}-1H-pyrazol-4-yl)acetic acid (363 mg, yield 79%) as colorless crystals. melting point 132 - 134°C.

### Example 90

To a mixture of 3-[1-(2,4-difluorobenzyl)-3-propoxy-1H-pyrazol-5-yl]propan-1-ol (201 mg), methyl (1-benzyl-3-hydroxy-1H-pyrazol-4-yl)acetate (159 mg), tributylphosphine (0.404 ml) and tetrahydrofuran (15 ml) was added 1,1'-azodicarbonyldipiperidine (327 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:20 to 1:3, v/v) to give a colorless oil (0.31 g). A mixture of the obtained oil, a 1N aqueous sodium hydroxide solution (1.2 ml), tetrahydrofuran (8.0 ml) and methanol (8.0 ml) was stirred at 50°C for 1 hr, 1N hydrochloric acid (1.2 ml) was added, and the mixture was extracted with ethyl acetate. The ethyl acetate layer was washed with saturated brine, dried (MgSO₄) and concentrated. The residue was crystallized from ethyl acetate-hexane to give (1-benzyl-3-{3-[1-(2,4-difluorobenzyl)-3-propoxy-1H-pyrazol-5-yl]propoxy}-1H-pyrazol-4-yl)acetic acid (223 mg, yield 74%) as colorless crystals. melting point 94 - 95°C.

### Example 91

To a mixture of 3-{3-propoxy-1-[4-(trifluoromethyl)benzyl]-1H-pyrazol-5-yl}propan-1-ol (192 mg), methyl (1-benzyl-3-hydroxy-1H-pyrazol-4-yl)acetate (138 mg), tributylphosphine (0.349 ml) and tetrahydrofuran (15 ml) was added 1,1'-azodicarbonyldipiperidine (283 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:20 to 1:4, v/v) to give a colorless oil (0.31 g). A mixture of the obtained oil, a 1N aqueous sodium hydroxide solution (1.2 ml), tetrahydrofuran (8.0 ml) and methanol (8.0 ml) was stirred at 50°C for 1 hr, 1N hydrochloric acid (1.2 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 2:1, v/v) to give [1-benzyl-3-(3-{3-propoxy-1-[4-(trifluoromethyl)benzyl]-1H-pyrazol-5-yl}propoxy)-1H-pyrazol-4-yl]acetic acid (0.25 g, yield 83%) as a colorless oil. ¹H-NMR (300 MHz, CDCl₃) δ:1.00 (3 H, t, J = 7.4 Hz), 1.67 - 1.85 (2 H, m), 1.89 - 2.07 (2 H, m), 2.51 - 2.66 (2 H, m), 3.33 (2 H, s), 4.04 (2 H, t, J = 6.7 Hz), 4.19 (2 H, t, J = 5.9 Hz), 5.07 (2 H, s), 5.16 (2 H, s), 5.56 (1 H, s), 7.08 - 7.22 (5 H, m), 7.27 - 7.37 (3 H, m), 7.51 (2 H, d, J = 8.1 Hz).

### Example 92

To a mixture of 3-[1-(2-chloro-4-fluorobenzyl)-3-isopropoxy-1H-pyrazol-5-yl]propan-1-ol (500 mg), methyl (1-benzyl-3-hydroxy-1H-pyrazol-4-yl)acetate (377 mg), tributylphosphine (0.954 ml) and tetrahydrofuran (30 ml) was added 1,1'-azodicarbonyldipiperidine (772 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:20 to 1:3, v/v) to give a colorless oil (0.71 g). A mixture of the obtained oil, a 1N aqueous sodium hydroxide solution (2.6 ml), tetrahydrofuran (8.0 ml) and methanol (8.0 ml) was stirred at 50°C for 1 hr, 1N hydrochloric acid (2.6 ml) was added, and the mixture was extracted with ethyl acetate. The ethyl acetate layer was washed with saturated brine, dried (MgSO₄) and concentrated. The residue was crystallized from ethyl acetate-hexane to give (1-benzyl-3-{3-[1-(2-chloro-4-fluorobenzyl)-3-isopropoxy-1H-pyrazol-5-yl]propoxy}-1H-pyrazol-4-yl)acetic acid (618 mg, yield 89%) as colorless crystals. melting point 121 - 123°C.

### Example 93

To a mixture of 3-[1-(2-chloro-4-fluorobenzyl)-3-isopropoxy-1H-pyrazol-5-yl]propan-1-ol (252 mg), methyl (2-hydroxypyridin-3-yl)acetate (76 mg), tributylphosphine (0.284 ml) and tetrahydrofuran (15 ml) was added 1,1'-azodicarbonyldipiperidine (230 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:20 to 1:3, v/v) to give methyl (2-{3-[1-(2-chloro-4-fluorobenzyl)-3-isopropoxy-1H-pyrazol-5-yl]propoxy}pyridin-3-yl)acetate (0.09 g, yield 42%) as a colorless oil. A mixture of the obtained oil, a 1N aqueous sodium hydroxide solution (0.4 ml), tetrahydrofuran (1.0 ml) and methanol (1.0 ml) was stirred at 50°C for 1 hr, 1N hydrochloric acid (0.4 ml) was added, and the mixture was extracted with ethyl acetate. The ethyl acetate layer was washed with saturated brine, dried (MgSO₄) and concentrated. The residue was crystallized from ethyl acetate-hexane to give (2-{3-[1-(2-chloro-4-fluorobenzyl)-3-isopropoxy-1H-pyrazol-5-yl]propoxy}pyridin-3-yl)acetic acid (79 mg, yield 91%) as colorless crystals. melting point 90 - 91°C.

### Example 94

To a mixture of 3-[1-(4-chloro-2-isopropoxybenzyl)-3-isopropoxy-1H-pyrazol-5-yl]propan-1-ol (500 mg), methyl (2-hydroxypyridin-3-yl)acetate (227 mg), tributylphosphine (0.847 ml) and tetrahydrofuran (20 ml) was added 1,1'-azodicarbonyldipiperidine (686 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:10 to 1:3, v/v) to give a colorless oil (0.36 g). A mixture of the obtained oil, a 1N aqueous sodium hydroxide solution (1.4 ml), tetrahydrofuran (4.0 ml) and methanol (4.0 ml) was stirred at 50°C for 1 hr, 1N hydrochloric acid (1.4 ml) was added, and the mixture was extracted with ethyl acetate. The ethyl acetate layer was washed with saturated brine, dried (MgSO₄) and concentrated. The residue was crystallized from ethyl acetate-hexane to give (2-{3-[1-(4-chloro-2-isopropoxybenzyl)-3-isopropoxy-1H-pyrazol-5-yl]propoxy}pyridin-3-yl)acetic acid (303 mg, yield 86%) as colorless crystals. melting point 82 - 84°C.

### Example 95

To a mixture of 3-[1-(2,4-dichlorobenzyl)-3-(methoxymethoxy)-1H-pyrazol-5-yl]propan-1-ol (1.19 g), methyl (1-benzyl-3-hydroxy-1H-pyrazol-4-yl)acetate (0.93 g), tributylphosphine (2.13 g) and tetrahydrofuran (50 ml) was added 1,1'-azodicarbonyldipiperidine (2.64 g) at room temperature, and the mixture was stirred for 3 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (3:7 to 2:3, v/v) to give a pale-yellow oil (1.45 g). A mixture of the obtained oil (0.26 g), a 1N aqueous sodium hydroxide solution (1.0 ml), tetrahydrofuran (2.5 ml) and methanol (2.5 ml) was stirred at room temperature for 19 hr, 1N hydrochloric acid (1.1 ml) was added, and the mixture was extracted with ethyl acetate. The ethyl acetate layer was washed with saturated brine, dried (Na₂SO₄) and concentrated. The residue was subjected to silica gel column chromatography and eluted with ethyl acetate-hexane (1:1 to 4:1, v/v). The elute was concentrated, and the residue was crystallized from ethyl acetate-hexane to give (1-benzyl-3-{3-[1-(2,4-dichlorobenzyl)-3-(methoxymethoxy)-1H-pyrazol-5-yl]propoxy}-1H-pyrazol-4-yl)acetic acid (151 mg, yield 66%) as colorless crystals. melting point 89.5 - 91.5°C.

### Example 96

To a mixture of methyl (1-benzyl-3-{3-[1-(2,4-dichlorobenzyl)-3-hydroxy-1H-pyrazol-5-yl]propoxy}-1H-pyrazol-4-yl)acetate (207 mg), 2-(dimethylamino)ethanol (70 mg), tributylphosphine (158 mg) and tetrahydrofuran (5 ml) was added 1,1*'*-azodicarbonyldipiperidine (198 mg) at room temperature, and the mixture was stirred for 4.5 hr. The reaction solution was concentrated, diisopropyl ether was added to the residue, and the insoluble substance was removed by filtration. The filtrate was concentrated, the obtained residue was subjected to aminopropyl silica gel column chromatography and eluted with ethyl acetate-hexane (1:1 to 1:0, v/v) to give a colorless oil (409 mg). A mixture of the obtained oil, a 1N aqueous sodium hydroxide solution (1.0 ml), tetrahydrofuran (2.5 ml) and ethanol (2.5 ml) was stirred at room temperature for 16 hr, 1N hydrochloric acid (1.0 ml) was added, and the mixture was extracted with ethyl acetate. The ethyl acetate layer was washed with saturated brine, dried (Na₂SO₄) and concentrated. The residue was subjected to silica gel column chromatography and eluted with methanol-ethyl acetate (1:1 to 1:0, v/v). The eluate was concentrated, and the residue was crystallized from ethyl acetate-hexane to give [1-benzyl-3-(3-{1-(2,4-dichlorobenzyl)-3-[2-(dimethylamino)ethoxy]-1H-pyrazol-5-yl}propoxy)-1H-pyrazol-4-yl]acetic acid (82 mg, yield 35%) as colorless crystals. melting point 78.3 - 80.8°C.

### Example 97

To a mixture of methyl (1-benzyl-3-{3-[1-(2,4-dichlorobenzyl)-3-hydroxy-1H-pyrazol-5-yl]propoxy}-1H-pyrazol-4-yl)acetate (218 mg), 2-(ethylthio)ethanol (88 mg), tributylphosphine (166 mg) and tetrahydrofuran (5 ml) was added 1,1'-azodicarbonyldipiperidine (208 mg) at room temperature, and the mixture was stirred for 17 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:9 to 2:3, v/v) to give methyl [1-benzyl-3-(3-{1-(2,4-dichlorobenzyl)-3-[2-(ethylthio)ethoxy]-1H-pyrazol-5-yl}propoxy)-1H-pyrazol-4-yl]acetate (253 mg, yield 99%) as a colorless oil. ¹H-NMR (300 MHz, CDCl₃) δ:1.27 (3 H, t, J = 7.4 Hz), 1.95 - 2.05 (2 H, m), 2.56 - 2.67 (4 H, m), 2.88 (2 H, t, J = 6.8 Hz), 3.30 (2 H, s), 3.65 (3 H, s), 4.17 (2 H, t, J = 6.0 Hz), 4.26 (2 H, t, J = 6.9 Hz), 5.07 (2 H, s), 5.15 (2 H, s), 5.59 (1 H, s), 6.52 (1 H, d, J = 8.1 Hz), 7.09 (1 H, dd, J = 2.1, 8.1 Hz), 7.13 - 7.20 (3 H, m), 7.23 - 7.37 (4 H, m).

### Example 98

A mixture of methyl [1-benzyl-3-(3-{1-(2,4-dichlorobenzyl)-3-[2-(ethylthio)ethoxy]-1H-pyrazol-5-yl}propoxy)-1H-pyrazol-4-yl]acetate (250 mg), 3-chloroperbenzoic acid (363 mg) and dichloromethane (20 ml) was stirred at room temperature for 4 hr. Sodium thiosulfate and water were added to the reaction solution, and the mixture was vigorously stirred at room temperature for 30 min, and the mixture was extracted with dichloromethane. The dichloromethane layer was washed with saturated aqueous sodium hydrogencarbonate and saturated brine, dried (Na₂SO₄) and concentrated. The residue was subjected to silica gel column chromatography and eluted with ethyl acetate-hexane (3:7 to 1:1, v/v) to give a colorless oil (189 mg). A mixture of the obtained oil, conc. sulfuric acid (0.65 ml), acetic acid (4.5 ml) and water (4.0 ml) was stirred at 90°C for 25 min. After cooling, the reaction solution was concentrated. Water (20 ml) was added to the residue, and the mixture was extracted with ethyl acetate. The ethyl acetate layer was washed with saturated brine, dried (Na₂SO₄) and concentrated. The residue was subjected to silica gel column chromatography and eluted with ethyl acetate-hexane (1:1 to 1:0, v/v). The eluate was concentrated, and the residue was crystallized from ethyl acetate-hexane to give [1-benzyl-3-(3-{1-(2,4-dichlorobenzyl)-3-[2-(ethylsulfonyl)ethoxy]-1H-pyrazol-5-yl}propoxy)-1H-pyrazol-4-yl]acetic acid (137 mg, yield 74%) as colorless crystals. melting point 100.4 - 101.4°C.

### Example 99

To a mixture of 3-(2-ethoxy-4-{[5-(trifluoromethyl)pyridin-2-yl]oxy}phenyl)propan-1-ol (512 mg), methyl (3-ethyl-2-hydroxyphenyl)acetate (291 mg), tributylphosphine (759 mg) and tetrahydrofuran (35 ml) was added 1,1*'*-azodicarbonyldipiperidine (758 mg) at 60°C, and the mixture was stirred for 30 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:5, v/v) to give a colorless oil (643 mg). To a solution (12 ml) of the obtained oil (541 mg) in a mixed solvent of tetrahydrofuran-methanol (3:1, v/v) was added a 1N aqueous sodium hydroxide solution (1.5 ml) at 60°C, and the mixture was stirred for 30 min. 1N Hydrochloric acid (1.5 ml) was added, and the mixture was stirred and extracted with ethyl acetate. The ethyl acetate layer was washed with saturated brine, dried (MgSO₄) and concentrated. The residue was subjected to silica gel column chromatography and eluted with ethyl acetate-hexane (1:4, v/v) to give {2-[3-(2-ethoxy-4-{[5-(trifluoromethyl)pyridin-2-yl]oxy}phenyl)propoxy]-3-ethylphenyl}acetic acid as a colorless oil (370 mg, yield 58%). ¹H-NMR (300 MHz, CDCl₃) δ:1.24 (3 H, t, J = 7.6 Hz), 1.36 (3 H, t, J = 7.0 Hz), 2.17 - 2.31 (2 H, m), 2.64 - 2.82 (4 H, m), 3.50 (2 H, s), 3.67 - 3.76 (2 H, m), 3.96 (2 H, q, J = 7.0 Hz), 6.52 - 6.60 (2 H, m), 6.97 - 7.18 (5 H, m), 7.96 (1 H, dd, J = 8.8, 2.2 Hz), 8.50 - 8.57 (1 H, m).

### Example 100

To a mixture of 3-(2-ethoxy-4-{[5-(trifluoromethyl)pyridin-2-yl]oxy}phenyl)propan-1-ol (444 mg), methyl (1-ethyl-3-hydroxy-1H-pyrazol-4-yl)acetate (239 mg), tributylphosphine (658 mg) and tetrahydrofuran (30 ml) was added 1,1'-azodicarbonyldipiperidine (656 mg) at 60°C, and the mixture was stirred for 30 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:4, v/v) to give a pale-yellow oil (557 mg). To a solution (12 ml) of the obtained oil (482 mg) in a mixed solvent of tetrahydrofuran-methanol (3:1, v/v) was added a 1N aqueous sodium hydroxide solution (2.0 ml) at 60°C, and the mixture was stirred for 30 min. 1N Hydrochloric acid (2.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:2, v/v) to give {3-[3-(2-ethoxy-4-{[5-(trifluoromethyl)pyridin-2-yl]oxy}phenyl)propoxy]-1-ethyl-1H-pyrazol-4-yl}acetic acid as a colorless oil (370 mg, yield 59%). Recrystallization from ethyl acetate-hexane gave colorless prism crystals. melting point 82 - 83°C.

### Example 101

To a mixture of 3-(2-isopropoxy-4-{[5-(trifluoromethyl)pyridin-2-yl]oxy}phenyl)propan-1-ol (200 mg), methyl (2-hydroxy-3-methoxyphenyl)acetate (110 mg), tributylphosphine (282 mg) and tetrahydrofuran (20 ml) was added 1,1'-azodicarbonyldipiperidine (283 mg) at 60°C, and the mixture was stirred for 30 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:5, v/v) to give a colorless oil (284 mg). To a solution (12 ml) of the obtained oil (238 mg) in a mixed solvent of tetrahydrofuran-methanol (3:1, v/v) was added a 1N aqueous sodium hydroxide solution (1.0 ml) at 60°C, and the mixture was stirred for 30 min. 1N Hydrochloric acid (1.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:4, v/v) to give {2-[3-(2-isopropoxy-4-{[5-(trifluoromethyl)pyridin-2-yl]oxy}phenyl)propoxy]-3-methoxyphenyl}acetic acid as a colorless oil (186 mg, yield 76%). ¹H-NMR (300 MHz, CDCl₃) δ:1.30 (6 H, d, J = 6.0 Hz), 2.11 - 2.28 (2 H, m), 2.68 - 2.79 (2 H, m), 3.55 (2 H, s), 3.81 - 3.95 (5 H, m), 4.40 - 4.55 (1 H, m), 6.49 - 6.63 (2 H, m), 6.77 - 6.93 (2 H, m), 6.95 - 7.14 (3 H, m), 7.88 (1 H, dd, J = 8.8, 2.5 Hz), 8.51 - 8.59 (1 H, m).

### Example 102

To a mixture of 3-(2-propoxy-4-{[5-(trifluoromethyl)pyridin-2-yl]oxy}phenyl)propan-1-ol (355 mg), methyl (2-hydroxy-3-methoxyphenyl)acetate (196 mg), tributylphosphine (506 mg) and tetrahydrofuran (30 ml) was added 1,1*'*-azodicarbonyldipiperidine (505 mg) at 60°C, and the mixture was stirred for 10 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:5, v/v) to give a colorless oil (455 mg). To a solution (12 ml) of the obtained oil (400 mg) in a mixed solvent of tetrahydrofuran-methanol (3:1, v/v) was added a 1N aqueous sodium hydroxide solution (1.5 ml) at 60°C, and the mixture was stirred for 30 min. 1N Hydrochloric acid (1.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:5, v/v) to give {2-[3-(2-propoxy-4-{[5-(trifluoromethyl)pyridin-2-yl]oxy}phenyl)propoxy]-3-methoxyphenyl}acetic acid as crystals (372 mg, yield 82%). Recrystallization from ethyl acetate-hexane gave colorless prism crystals. melting point 78 - 79°C.

### Example 103

To a mixture of 3-(2-butoxy-4-{[5-(trifluoromethyl)pyridin-2-yl]oxy}phenyl)propan-1-ol (369 mg), methyl (2-hydroxy-3-methoxyphenyl)acetate (196 mg), tributylphosphine (506 mg) and tetrahydrofuran (30 ml) was added 1,1'-azodicarbonyldipiperidine (505 mg) at 60°C, and the mixture was stirred for 15 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:5, v/v) to give a colorless oil (484 mg). To a solution (12 ml) of the obtained oil (437 mg) in a mixed solvent of tetrahydrofuran-methanol (3:1, v/v) was added a 1N aqueous sodium hydroxide solution (1.5 ml) at 60°C, and the mixture was stirred for 30 min. 1N Hydrochloric acid (1.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:5, v/v) to give {2-[3-(2-butoxy-4-{[5-(trifluoromethyl)pyridin-2-yl]oxy}phenyl)propoxy]-3-methoxyphenyl}acetic acid as a colorless oil (359 mg, yield 74%). Recrystallization from ethyl acetate-hexane gave colorless prism crystals. melting point 64 - 65°C.

### Example 104

To a mixture of 3-(2-isobutoxy-4-{[5-(trifluoromethyl)pyridin-2-yl]oxy}phenyl)propan-1-ol (443 mg), methyl (2-hydroxy-3-methoxyphenyl)acetate (235 mg), tributylphosphine (607 mg) and tetrahydrofuran (30 ml) was added 1,1'-azodicarbonyldipiperidine (606 mg) at 60°C, and the mixture was stirred for 30 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (5:1, v/v) to give a colorless oil. To a solution (12 ml) of the obtained oil in a mixed solvent of tetrahydrofuran-methanol (3:1, v/v) was added a 1N aqueous sodium hydroxide solution (2.5 ml) at 60°C, and the mixture was stirred for 1 hr. 1N Hydrochloric acid (2.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:3, v/v) to give {2-[3-(2-isobutoxy-4-{[5-(trifluoromethyl)pyridin-2-yl]oxy}phenyl)propoxy]-3-methoxyphenyl}acetic acid as a colorless oil (415 mg, yield 65%). ¹H-NMR (300 MHz, CDCl₃) δ:1.02 (6 H, d, J = 6.8 Hz), 1.99 - 2.28 (3 H, m), 2.71 - 2.85 (2 H, m), 3.56 (2 H, s), 3.66 (2 H, d, J = 6.4 Hz), 3.84 (3 H, s), 3.87 - 3.95 (2 H, m), 6.52 - 6.63 (2 H, m), 6.76 - 6.92 (2 H, m), 6.93 - 7.13 (3 H, m), 7.93 (1 H, dd, J = 8.7, 2.5 Hz), 8.48 - 8.55 (1 H, m).

### Example 105

To a mixture of 3-(4-(methoxymethoxy)-2-{[5-(trifluoromethyl)pyridin-2-yl]oxy}phenyl)propan-1-ol (429 mg), methyl (2-hydroxy-3-methoxyphenyl)acetate (235 mg), tributylphosphine (607 mg) and tetrahydrofuran (30 ml) was added 1,1'-azodicarbonyldipiperidine (606 mg) at 60°C, and the mixture was stirred for 15 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:5, v/v) to give a colorless oil. To a solution (12 ml) of the obtained oil in a mixed solvent of tetrahydrofuran-methanol (3:1, v/v) was added a 1N aqueous sodium hydroxide solution (2.0 ml) at 60°C, and the mixture was stirred for 1 hr. 1N Hydrochloric acid (2.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:2, v/v) to give {3-methoxy-2-[3-(4-(methoxymethoxy)-2-{[5-(trifluoromethyl)pyridin-2-yl]oxy}phenyl)propoxy]phenyl}acetic acid as a colorless oil (470 mg, yield 75%). Recrystallization from ethyl acetate-hexane gave colorless prism crystals. melting point 70 - 72°C.

### Example 106

To a mixture of 3-(4-(methoxymethoxy)-2-{[5-(trifluoromethyl)pyridin-2-yl]oxy}phenyl)propan-1-ol (440 mg), methyl (1-ethyl-3-hydroxy-1H-pyrazol-4-yl)acetate (227 mg), tributylphosphine (622 mg) and tetrahydrofuran (30 ml) was added 1,1'-azodicarbonyldipiperidine (621 mg) at 60°C, and the mixture was stirred for 30 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:2, v/v) to give a colorless oil. To a solution (12 ml) of the obtained oil in a mixed solvent of tetrahydrofuran-methanol (3:1, v/v) was added a 1N aqueous sodium hydroxide solution (2.0 ml) at 60°C, and the mixture was stirred for 1 hr. 1N Hydrochloric acid (2.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:2, v/v) to give {1-ethyl-3-[3-(4-(methoxymethoxy)-2-{[5-(trifluoromethyl)pyridin-2-yl]oxy}phenyl)propoxy]-1H-pyrazol-4-yl}acetic acid as a colorless oil (392 mg, yield 62%). Recrystallization from ethyl acetate-hexane gave colorless prism crystals. melting point 82 - 83°C.

### Example 107

To a mixture of 3-(4-isopropoxy-2-{[5-(trifluoromethyl)pyridin-2-yl]oxy}phenyl)propan-1-ol (355 mg), methyl (2-hydroxy-3-methoxyphenyl)acetate (196 mg), tributylphosphine (506 mg) and tetrahydrofuran (30 ml) was added 1,1*'*-azodicarbonyldipiperidine (505 mg) at 60°C, and the mixture was stirred for 30 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:5, v/v) to give a colorless oil. To a solution (12 ml) of the obtained oil in a mixed solvent of tetrahydrofuran-methanol (3:1, v/v) was added a 1N aqueous sodium hydroxide solution (1.5 ml) at 60°C, and the mixture was stirred for 1 hr. 1N Hydrochloric acid (1.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:4, v/v) to give {2-[3-(4-isopropoxy-2-{[5-(trifluoromethyl)pyridin-2-yl]oxy}phenyl)propoxy]-3-methoxyphenyl}acetic acid as a colorless oil (334 mg, yield 64%). ¹H-NMR (300 MHz, CDCl₃) δ:1.32 (6 H, d, J = 6.0 Hz), 1.96 - 2.12 (2 H, m), 2.48 - 2.63 (2 H, m), 3.64 (2 H, s), 3.79 (3 H, s), 3.99 (2 H, t, J = 6.8 Hz), 4.39 - 4.58 (1 H, m), 6.61 (1 H, d, J = 2.6 Hz), 6.73 - 6.91 (4 H, m), 6.94 - 7.03 (1 H, m), 7.22 (1 H, d, J = 8.5 Hz), 7.85 (1 H, dd, J = 9.0, 2.4 Hz), 8.46 - 8.54 (1 H, m).

### Example 108

To a mixture of 3-(4-isopropoxy-2-{[5-(trifluoromethyl)pyridin-2-yl]oxy}phenyl)propan-1-ol (288 mg), methyl (1-ethyl-3-hydroxy-1H-pyrazol-4-yl)acetate (149 mg), tributylphosphine (410 mg) and tetrahydrofuran (30 ml) was added 1,1'-azodicarbonyldipiperidine (409 mg) at 60°C, and the mixture was stirred for 30 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:2, v/v) to give a colorless oil. To a solution (12 ml) of the obtained oil in a mixed solvent of tetrahydrofuran-methanol (3:1, v/v) was added a 1N aqueous sodium hydroxide solution (2.0 ml) at 60°C, and the mixture was stirred for 1 hr. 1N Hydrochloric acid (2.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:4, v/v) to give {1-ethyl-3-[3-(4-isopropoxy-2-{[5-(trifluoromethyl)pyridin-2-yl]oxy}phenyl)propoxy]-1H-pyrazol-4-yl}acetic acid as a colorless oil (218 mg, yield 53%). ¹H-NMR (300 MHz, CDCl₃) δ:1.31 (6 H, d, J = 6.0 Hz), 1.38 (3 H, t, J = 7.3 Hz), 1.89 - 2.05 (2 H, m), 2.56 - 2.66 (2 H, m), 3.36 (2 H, s), 3.93 (2 H, q, J = 7.3 Hz), 4.13 (2 H, t, J = 5.9 Hz), 4.40 - 4.55 (1 H, m), 6.59 (1 H, d, J = 2.6 Hz), 6.77 (1 H, dd, J = 8.6, 2.5 Hz), 6.88 (1 H, d, J = 8.7 Hz), 7.14 - 7.25 (2 H, m), 7.86 (1 H, dd, J = 9.0, 2.4 Hz), 8.44 - 8.52 (1 H, m).

### Example 109

To a mixture of 3-(4-butoxy-2-{[5-(trifluoromethyl)pyridin-2-yl]oxy}phenyl)propan-1-ol (235 mg), methyl (2-hydroxy-3-methoxyphenyl)acetate (126 mg), tributylphosphine (324 mg) and tetrahydrofuran (25 ml) was added 1,1'-azodicarbonyldipiperidine (323 mg) at 60°C, and the mixture was stirred for 30 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:5, v/v) to give a colorless oil. To a solution (12 ml) of the obtained oil in a mixed solvent of tetrahydrofuran-methanol (3:1, v/v) was added a 1N aqueous sodium hydroxide solution (1.5 ml) at 60°C, and the mixture was stirred for 1 hr. 1N Hydrochloric acid (1.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:4, v/v) to give {2-[3-(4-butoxy-2-{[5-(trifluoromethyl)pyridin-2-yl]oxy}phenyl)propoxy]-3-methoxyphenyl}acetic acid as a colorless oil (334 mg, yield 64%). Recrystallization from ethyl acetate-hexane gave colorless prism crystals. melting point 69 - 70°C.

### Example 110

To a mixture of 3-(4-butoxy-2-{[5-(trifluoromethyl)pyridin-2-yl]oxy}phenyl)propan-1-ol (300 mg), methyl (1-ethyl-3-hydroxy-1H-pyrazol-4-yl)acetate (149 mg), tributylphosphine (410 mg) and tetrahydrofuran (30 ml) was added 1,1'-azodicarbonyldipiperidine (409 mg) at 60°C, and the mixture was stirred for 30 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:2, v/v) to give a colorless oil. To a solution (12 ml) of the obtained oil in a mixed solvent of tetrahydrofuran-methanol (3:1, v/v) was added a 1N aqueous sodium hydroxide solution (2.0 ml) at 60°C, and the mixture was stirred for 1 hr. 1N Hydrochloric acid (2.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:4, v/v) to give {1-ethyl-3-[3-(4-butoxy-2-{[5-(trifluoromethyl)pyridin-2-yl]oxy}phenyl)propoxy]-1H-pyrazol-4-yl}acetic acid as a colorless oil (216 mg, yield 51%). Recrystallization from ethyl acetate-hexane gave colorless prism crystals. melting point 81 - 82°C.

### Example 111

To a mixture of 3-[2-{[3-chloro-5-(trifluoromethyl)pyridin-2-yl]oxy}-4-(methoxymethoxy)phenyl]propan-1-ol (470 mg), methyl (1-methyl-3-hydroxy-1H-pyrazol-4-yl)acetate (204 mg), tributylphosphine (607 mg) and tetrahydrofuran (30 ml) was added 1,1'-azodicarbonyldipiperidine (606 mg) at 60°C, and the mixture was stirred for 20 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:3, v/v) to give a colorless oil. To a solution (12 ml) of the obtained oil in a mixed solvent of tetrahydrofuran-methanol (3:1, v/v) was added a 1N aqueous sodium hydroxide solution (2.5 ml) at 60°C, and the mixture was stirred for 1 hr. 1N Hydrochloric acid (2.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:2, v/v) to give (3-{3-[2-{[3-chloro-5-(trifluoromethyl)pyridin-2-yl]oxy}-4-(methoxymethoxy)phenyl]propoxy}-1-methyl-1H-pyrazol-4-yl)acetic acid as a colorless oil (500 mg, yield 79%). Recrystallization from ethyl acetate-hexane gave colorless prism crystals. melting point 122 - 123°C.

### Example 112

To a mixture of 3-[2-{[3-chloro-5-(trifluoromethyl)pyridin-2-yl]oxy}-4-(methoxymethoxy)phenyl]propan-1-ol (470 mg), methyl (1-benzyl-3-hydroxy-1H-pyrazol-4-yl)acetate (296 mg), tributylphosphine (607 mg) and tetrahydrofuran (30 ml) was added 1,1'-azodicarbonyldipiperidine (606 mg) at 60°C, and the mixture was stirred for 10 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:5, v/v) to give a colorless oil. To a solution (12 ml) of the obtained oil in a mixed solvent of tetrahydrofuran-methanol (3:1, v/v) was added a 1N aqueous sodium hydroxide solution (2.5 ml) at 60°C, and the mixture was stirred for 1 hr. 1N Hydrochloric acid (2.5 ml) was added, and the mixture was extracted with ethyl acetate. The ethyl acetate layer was washed with saturated brine, dried (MgSO₄) and concentrated. Recrystallization from ethyl acetate-hexane gave (3-{3-[2-{[3-chloro-5-(trifluoromethyl)pyridin-2-yl]oxy}-4-(methoxymethoxy)phenyl]propoxy}-1-benzyl-1H-pyrazol-4-yl)acetic acid as colorless prism crystals (434 mg, yield 72%). melting point 108 - 109°C.

### Example 113

To a mixture of 3-(2-{[3-chloro-5-(trifluoromethyl)pyridin-2-yl]oxy}-4-propoxyphenyl)propan-1-ol (390 mg), methyl (1-methyl-3-hydroxy-1H-pyrazol-4-yl)acetate (170 mg), tributylphosphine (506 mg) and tetrahydrofuran (30 ml) was added 1,1*'*-azodicarbonyldipiperidine (505 mg) at 60°C, and the mixture was stirred for 20 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:3, v/v) to give a colorless oil. To a solution (12 ml) of the obtained oil in a mixed solvent of tetrahydrofuran-methanol (3:1, v/v) was added a 1N aqueous sodium hydroxide solution (2.0 ml) at 60°C, and the mixture was stirred for 30 min. 1N Hydrochloric acid (2.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:2, v/v) to give {3-[3-(2-{[3-chloro-5-(trifluoromethyl)pyridin-2-yl]oxy}-4-propoxyphenyl)propoxy]-1-methyl-1H-pyrazol-4-yl}acetic acid as a colorless oil. Recrystallization from ethyl acetate-hexane gave colorless prism crystals (297 mg, yield 56%). melting point 95 - 96°C.

### Example 114

To a mixture of 3-(2-{[3-chloro-5-(trifluoromethyl)pyridin-2-yl]oxy}-4-propoxyphenyl)propan-1-ol (390 mg), methyl (1-benzyl-3-hydroxy-1H-pyrazol-4-yl)acetate (246 mg), tributylphosphine (506 mg) and tetrahydrofuran (30 ml) was added 1,1'-azodicarbonyldipiperidine (505 mg) at 60°C, and the mixture was stirred for 10 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:5, v/v) to give a colorless oil. To a solution (12 ml) of the obtained oil in a mixed solvent of tetrahydrofuran-methanol (3:1, v/v) was added a 1N aqueous sodium hydroxide solution (2.0 ml) at 60°C, and the mixture was stirred for 30 min. 1N Hydrochloric acid (2.0 ml) was added, and the mixture was extracted with ethyl acetate. The ethyl acetate layer was washed with saturated brine, dried (MgSO₄) and concentrated. Recrystallization from ethyl acetate-hexane gave {3-[3-(2-{[3-chloro-5-(trifluoromethyl)pyridin-2-yl]oxy}-4-propoxyphenyl)propoxy]-1-benzyl-1H-pyrazol-4-yl}acetic acid as colorless prism crystals (434 mg, yield 72%). melting point 96.5 - 97.5°C.

### Example 115

To a mixture of 3-(2-{[3-chloro-5-(trifluoromethyl)pyridin-2-yl]oxy}-4-isopropoxyphenyl)propan-1-ol (585 mg), methyl (2-hydroxy-3-methoxyphenyl)acetate (294 mg), tributylphosphine (759 mg) and tetrahydrofuran (30 ml) was added 1,1'-azodicarbonyldipiperidine (758 mg) at 60°C, and the mixture was stirred for 30 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:10, v/v) to give a colorless oil. To a solution (12 ml) of the obtained oil in a mixed solvent of tetrahydrofuran-methanol (3:1, v/v) was added a 1N aqueous sodium hydroxide solution (3.0 ml) at 60°C, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:3, v/v) to give {2-[3-(2-{[3-chloro-5-(trifluoromethyl)pyridin-2-yl]oxy}-4-isopropoxyphenyl)propoxy]-3-methoxyphenyl}acetic acid as a colorless oil (517 mg, yield 62%). Recrystallization from ethyl acetate-hexane gave colorless prism crystals. melting point 100 - 101°C.

### Example 116

To a mixture of 3-(2-{[3-chloro-5-(trifluoromethyl)pyridin-2-yl]oxy}-4-isopropoxyphenyl)propan-1-ol (467 mg), methyl (1-methyl-3-hydroxy-1H-pyrazol-4-yl)acetate (204 mg), tributylphosphine (607 mg) and tetrahydrofuran (30 ml) was added 1,1'-azodicarbonyldipiperidine (606 mg) at 60°C, and the mixture was stirred for 20 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:4, v/v) to give a colorless oil. To a solution (12 ml) of the obtained oil in a mixed solvent of tetrahydrofuran-methanol (3:1, v/v) was added a 1N aqueous sodium hydroxide solution (2.0 ml) at 60°C, and the mixture was stirred for 30 min. 1N Hydrochloric acid (2.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:2, v/v) to give {3-[3-(2-{[3-chloro-5-(trifluoromethyl)pyridin-2-yl]oxy}-4-isopropoxyphenyl)propoxy]-1-methyl-1H-pyrazol-4-yl}acetic acid as a colorless oil (395 mg, yield 62%). Recrystallization from ethyl acetate-hexane gave colorless prism crystals. melting point 92 - 94°C.

### Example 117

To a mixture of 3-(2-{[3-chloro-5-(trifluoromethyl)pyridin-2-yl]oxy}-4-isopropoxyphenyl)propan-1-ol (585 mg), methyl (1-ethyl-3-hydroxy-1H-pyrazol-4-yl)acetate (276 mg), tributylphosphine (759 mg) and tetrahydrofuran (30 ml) was added 1,1'-azodicarbonyldipiperidine (758 mg) at 60°C, and the mixture was stirred for 30 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:4, v/v) to give a colorless oil. To a solution (12 ml) of the obtained oil in a mixed solvent of tetrahydrofuran-methanol (3:1, v/v) was added a 1N aqueous sodium hydroxide solution (3.0 ml) at 60°C, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:4, v/v) to give {3-[3-(2-{[3-chloro-5-(trifluoromethyl)pyridin-2-yl]oxy}-4-isopropoxyphenyl)propoxy]-1-ethyl-1H-pyrazol-4-yl}acetic acid as a colorless oil (443 mg, yield 54%). ¹H-NMR (300 MHz, CDCl₃) δ:1.32 (6 H, d, J = 6.0 Hz), 1.38 (3 H, t, J = 7.3 Hz), 1.93 - 2.06 (2 H, m), 2.52 - 2.64 (2 H, m), 3.36 (2 H, s), 3.93 (2 H, q, J = 7.3 Hz), 4.13 (2 H, t, J = 6.2 Hz), 4.42 - 4.55 (1 H, m), 6.62 (1 H, d, J = 2.6 Hz), 6.76 (1 H, dd, J = 8.5, 2.6 Hz), 7.14
- 7.24 (2 H, m), 7.96 (1 H, d, J = 2.0 Hz), 8.24 (1 H, dd, J = 2.0, 0.9 Hz).

### Example 118

To a mixture of 3-(2-{[3-chloro-5-(trifluoromethyl)pyridin-2-yl]oxy}-4-isopropoxyphenyl)propan-1-ol (467 mg), methyl (1-benzyl-3-hydroxy-1H-pyrazol-4-yl)acetate (296 mg), tributylphosphine (607 mg) and tetrahydrofuran (30 ml) was added 1,1'-azodicarbonyldipiperidine (606 mg) at 60°C, and the mixture was stirred for 20 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:5, v/v) to give a colorless oil. To a solution (12 ml) of the obtained oil in a mixed solvent of tetrahydrofuran-methanol (3:1, v/v) was added a 1N aqueous sodium hydroxide solution (3.0 ml) at 60°C, and the mixture was stirred for 30 min. 1N Hydrochloric acid (3.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:3, v/v) to give {3-[3-(2-{[3-chloro-5-(trifluoromethyl)pyridin-2-yl]oxy}-4-isopropoxyphenyl)propoxy]-1-benzyl-1H-pyrazol-4-yl}acetic acid. Recrystallization from ethyl acetate-hexane gave colorless prism crystals (494 mg, yield 68%). melting point 115 - 116°C.

### Example 119

To a mixture of 3-{2-[(3,5-dichloropyridin-2-yl)oxy]-4-isopropoxyphenyl}propan-1-ol (356 mg), methyl (2-hydroxy-3-methoxyphenyl)acetate (196 mg), tributylphosphine (506 mg) and tetrahydrofuran (20 ml) was added 1,1'-azodicarbonyldipiperidine (505 mg) at 60°C, and the mixture was stirred for 30 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:5, v/v) to give a colorless oil. To a solution (12 ml) of the obtained oil in a mixed solvent of tetrahydrofuran-methanol (3:1, v/v) was added a 1N aqueous sodium hydroxide solution (2.0 ml) at 60°C, and the mixture was stirred for 1 hr. 1N Hydrochloric acid (2.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:4, v/v) to give [2-(3-{2-[(3,5-dichloropyridin-2-yl)oxy]-4-isopropoxyphenyl}propoxy)-3-methoxyphenyl]acetic acid as a colorless oil (435 mg, yield 84%). Recrystallization from ethyl acetate-hexane gave colorless prism crystals. melting point 92 - 93°C.

### Example 120

To a mixture of 3-{2-[(3,5-dichloropyridin-2-yl)oxy]-4-isopropoxyphenyl}propan-1-ol (356 mg), methyl (1-ethyl-3-hydroxy-1H-pyrazol-4-yl)acetate (184 mg), tributylphosphine (506 mg) and tetrahydrofuran (20 ml) was added 1,1'-azodicarbonyldipiperidine (505 mg) at 60°C, and the mixture was stirred for 30 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:4, v/v) to give a colorless oil. To a solution (12 ml) of the obtained oil in a mixed solvent of tetrahydrofuran-methanol (3:1, v/v) was added a 1N aqueous sodium hydroxide solution (2.0 ml) at 60°C, and the mixture was stirred for 1 hr. 1N Hydrochloric acid (2.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:4, v/v) to give [3-(3-{2-[(3,5-dichloropyridin-2-yl)oxy]-4-isopropoxyphenyl}propoxy)-1-ethyl-1H-pyrazol-4-yl]acetic acid as a colorless oil (303 mg, yield 60%). ¹H-NMR (300 MHz, CDCl₃) δ:1.31 (6 H, d, J = 6.0 Hz), 1.39 (3 H, t, J = 7.3 Hz), 1.91 - 2.07 (2 H, m), 2.54 - 2.65 (2 H, m), 3.38 (2 H, s), 3.94 (2 H, q, J = 7.2 Hz), 4.13 (2 H, t, J = 6.2 Hz), 4.40 - 4.55 (1 H, m), 6.59 (1 H, d, J = 2.6 Hz), 6.73 (1 H, dd, J = 8.5, 2.5 Hz), 7.15 - 7.22 (2 H, m), 7.74 (1 H, d, J = 2.5 Hz), 7.92 (1 H, d, J = 2.3 Hz).

### Example 121

To a mixture of 3-{2-[(3,5-dichloropyridin-2-yl)oxy]-4-isopropoxyphenyl}propan-1-01 (371 mg), methyl (2-hydroxypyridin-3-yl)acetate (174 mg), tributylphosphine (526 mg) and tetrahydrofuran (30 ml) was added 1,1'-azodicarbonyldipiperidine (525 mg) at 60°C, and the mixture was stirred for 15 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:5, v/v) to give a colorless oil. To a solution (12 ml) of the obtained oil in a mixed solvent of tetrahydrofuran-methanol (3:1, v/v) was added a 1N aqueous sodium hydroxide solution (2.0 ml) at 60°C, and the mixture was stirred for 1 hr. 1N Hydrochloric acid (2.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:2, v/v) to give [2-(3-{2-[(3,5-dichloropyridin-2-yl)oxy]-4-isopropoxyphenyl}propoxy)pyridin-3-yl]acetic acid as a colorless oil (220 mg, yield 43%). Recrystallization from ethyl acetate-hexane gave colorless prism crystals. melting point 84 - 85°C.

### Example 122

To a mixture of 3-[2-{[3-chloro-5-(trifluoromethyl)pyridin-2-yl]oxy}-4-(2-methoxyethoxy)phenyl]propan-1-ol (609 mg), methyl (1-methyl-3-hydroxy-1H-pyrazol-4-yl)acetate (255 mg), tributylphosphine (759 mg) and tetrahydrofuran (30 ml) was added 1,1'-azodicarbonyldipiperidine (758 mg) at 60°C, and the mixture was stirred for 30 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:2, v/v) to give a colorless oil. To a solution (12 ml) of the obtained oil in a mixed solvent of tetrahydrofuran-methanol (3:1, v/v) was added a 1N aqueous sodium hydroxide solution (3.0 ml) at 60°C, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give (3-{3-[2-{[3-chloro-5-(trifluoromethyl)pyridin-2-yl]oxy}-4-(2-methoxyethoxy)phenyl]propoxy}-1-methyl-1H-pyrazol-4-yl)acetic acid as colorless prism crystals (467 mg, yield 57%). melting point 127 - 129°C.

### Example 123

To a mixture of [3-ethoxy-4-(2-hydroxyethoxy)phenyl](pyridin-2-yl)methanone (345 mg), methyl (2-hydroxy-3-methoxyphenyl)acetate (235 mg), tributylphosphine (607 mg) and tetrahydrofuran (30 ml) was added 1,1'-azodicarbonyldipiperidine (606 mg) at 60°C, and the mixture was stirred for 30 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:2, v/v) to give a yellow oil (435 mg). To a solution (12 ml) of the obtained oil (390 mg) in a mixed solvent of tetrahydrofuran-methanol (3:1, v/v) was added a 1N aqueous sodium hydroxide solution (2.0 ml) at 60°C, and the mixture was stirred for 30 min. 1N Hydrochloric acid (2.0 ml) was added, and the mixture was extracted with ethyl acetate. The ethyl acetate layer was washed with saturated brine, dried (MgSO₄) and concentrated. The residue was recrystallized from tetrahydrofuran-hexane to give (2-{2-[2-ethoxy-4-(pyridin-2-ylcarbonyl)phenoxy]ethoxy}-3-methoxyphenyl)acetic acid as colorless prism crystals (315 mg, yield 65%). melting point 184 - 186°C.

### Example 124

To a mixture of 3-[2-ethoxy-4-(pyridin-2-ylmethyl)phenyl]propan-1-ol (250 mg), methyl (1-ethyl-3-hydroxy-1H-pyrazol-4-yl)acetate (170 mg), tributylphosphine (465 mg) and tetrahydrofuran (30 ml) was added 1,1'-azodicarbonyldipiperidine (464 mg) at 60°C, and the mixture was stirred for 15 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:2, v/v) to give a colorless oil. To a solution (12 ml) of the obtained oil in a mixed solvent of tetrahydrofuran-methanol (3:1, v/v) was added a 1N aqueous sodium hydroxide solution (2.0 ml) at 60°C, and the mixture was stirred for 30 min. 1N Hydrochloric acid (2.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (2:1, v/v) to give (3-{3-[2-ethoxy-4-(pyridin-2-ylmethyl)phenyl]propoxy}-1-ethyl-1H-pyrazol-4-yl)acetic acid as a colorless oil (204 mg, yield 52%). ¹H-NMR (300 MHz, CDCl₃) δ:1.26 - 1.44 (6 H, m), 1.97 - 2.12 (2 H, m), 2.74 (2 H, t, J = 7.0 Hz), 3.23 (2 H, s), 3.86 - 4.00 (4 H, m), 4.13 (2 H, s), 4.18 (2 H, t, J = 6.0 Hz), 6.55 - 6.68 (2 H, m), 7.04 (1 H, d, J = 7.4 Hz), 7.10 - 7.25 (3 H, m), 7.60 - 7.72 (1 H, m), 8.50 - 8.60 (1 H, m).

### Example 125

To a mixture of 3-[2-ethoxy-4-(pyridin-2-ylmethyl)phenyl]propan-1-ol (326 mg), methyl (2-hydroxy-3-methoxyphenyl)acetate (235 mg), tributylphosphine (607 mg) and tetrahydrofuran (30 ml) was added 1,1'-azodicarbonyldipiperidine (606 mg) at 60°C, and the mixture was stirred for 15 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:4, v/v) to give a colorless oil. To a solution (12 ml) of the obtained oil in a mixed solvent of tetrahydrofuran-methanol (3:1, v/v) was added a 1N aqueous sodium hydroxide solution (2.0 ml) at 60°C, and the mixture was stirred for 30 min. 1N Hydrochloric acid (2.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (2:1, v/v) to give (2-{3-[2-ethoxy-4-(pyridin-2-ylmethyl)phenyl]propoxy}-3-methoxyphenyl)acetic acid as a colorless oil (324 mg, yield 62%). Recrystallization from ethyl acetate-hexane gave colorless prism crystals. melting point 97.5 - 98.5°C.

### Example 126

To a mixture of 3-[2-isopropoxy-4-(pyridin-2-ylmethyl)phenyl]propan-1-ol (230 mg), methyl (1-ethyl-3-hydroxy-1H-pyrazol-4-yl)acetate (149 mg), tributylphosphine (410 mg) and tetrahydrofuran (30 ml) was added 1,1'-azodicarbonyldipiperidine (409 mg) at 60°C, and the mixture was stirred for 30 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:2, v/v) to give a colorless oil. To a solution (12 ml) of the obtained oil in a mixed solvent of tetrahydrofuran-methanol (3:1, v/v) was added a 1N aqueous sodium hydroxide solution (1.5 ml) at 60°C, and the mixture was stirred for 30 min. 1N Hydrochloric acid (1.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (2:1, v/v) to give (3-{3-[2-isopropoxy-4-(pyridin-2-ylmethyl)phenyl]propoxy}-1-ethyl-1H-pyrazol-4-yl)acetic acid as a colorless oil (164 mg, yield 47%). Recrystallization from ethyl acetate-hexane gave colorless prism crystals. melting point 97 - 98°C.

### Example 127

To a mixture of 3-[2-isopropoxy-4-(pyridin-2-ylmethyl)phenyl]propan-1-ol (230 mg), methyl (2-hydroxy-3-methoxyphenyl)acetate (158 mg), tributylphosphine (410 mg) and tetrahydrofuran (30 ml) was added 1,1'-azodicarbonyldipiperidine (409 mg) at 60°C, and the mixture was stirred for 15 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:4, v/v) to give a colorless oil. To a solution (12 ml) of the obtained oil in a mixed solvent of tetrahydrofuran-methanol (3:1, v/v) was added a 1N aqueous sodium hydroxide solution (1.5 ml) at 60°C, and the mixture was stirred for 30 min. 1N Hydrochloric acid (1.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:1, v/v) to give (2-{3-[2-isopropoxy-4-(pyridin-2-ylmethyl)phenyl]propoxy}-3-methoxyphenyl)acetic acid as a colorless oil (207 mg, yield 57%). ¹H-NMR (300 MHz, CDCl₃) δ:1.27 (6 H, d, J = 6.0 Hz), 2.10 - 2.27 (2 H, m), 2.68 - 2.80 (2 H, m), 3.52 (2 H, s), 3.74 - 3.87 (5 H, m), 4.14 (2 H, s), 4.41 - 4.57 (1 H, m), 6.54 - 6.68 (2 H, m), 6.74 - 6.90 (2 H, m), 6.92 - 7.04 (2 H, m), 7.12 - 7.22 (1 H, m), 7.23 - 7.30 (1 H, m), 7.61 - 7.73 (1 H, m), 8.52 - 8.62 (1 H, m).

### Example 128

To a mixture of 3-[4-(2,4-dichlorophenoxy)-2-isopropylpyrimidin-5-yl]propan-1-ol (409 mg), methyl (1-methyl-3-hydroxy-1H-pyrazol-4-yl)acetate (204 mg), tributylphosphine (607 mg) and tetrahydrofuran (30 ml) was added 1,1'-azodicarbonyldipiperidine (606 mg) at 60°C, and the mixture was stirred for 20 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (2:1, v/v) to give a colorless oil. To a solution (12 ml) of the obtained oil in a mixed solvent of tetrahydrofuran-methanol (3:1, v/v) was added a 1N aqueous sodium hydroxide solution (2.0 ml) at 60°C, and the mixture was stirred for 1 hr. 1N Hydrochloric acid (2.0 ml) was added, and the mixture was extracted with ethyl acetate. The ethyl acetate layer was washed with saturated brine, dried (MgSO₄) and concentrated. The residue was recrystallized from ethyl acetate-hexane to give (3-{3-[4-(2,4-dichlorophenoxy)-2-isopropylpyrimidin-5-yl]propoxy}-1-methyl-1H-pyrazol-4-yl)acetic acid as colorless prism crystals (422 mg, yield 73%). melting point 107 - 108°C.

### Example 129

To a mixture of 3-[4-(2,4-dichlorophenoxy)-2-isopropylpyrimidin-5-yl]propan-1-ol (409 mg), methyl (1-benzyl-3-hydroxy-1H-pyrazol-4-yl)acetate (296 mg), tributylphosphine (607 mg) and tetrahydrofuran (30 ml) was added 1,1'-azodicarbonyldipiperidine (606 mg) at 60°C, and the mixture was stirred for 10 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:2, v/v) to give a colorless oil. To a solution (12 ml) of the obtained oil in a mixed solvent of tetrahydrofuran-methanol (3:1, v/v) was added a 1N aqueous sodium hydroxide solution (2.0 ml) at 60°C, and the mixture was stirred for 1 hr. 1N Hydrochloric acid (2.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:2, v/v) to give (3-{3-[4-(2,4-dichlorophenoxy)-2-isopropylpyrimidin-5-yl]propoxy}-1-benzyl-1H-pyrazol-4-yl)acetic acid (453 mg, yield 68%). Recrystallization from ethyl acetate-hexane gave colorless prism crystals. melting point 98 - 99°C.

### Example 130

To a mixture of 3-[2-(2,4-dichlorophenoxy)-6-isopropoxypyridin-3-yl]propan-1-ol (428 mg), methyl (1-methyl-3-hydroxy-1H-pyrazol-4-yl)acetate (204 mg), tributylphosphine (607 mg) and tetrahydrofuran (30 ml) was added 1,1'-azodicarbonyldipiperidine (606 mg) at 60°C, and the mixture was stirred for 20 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:4, v/v) to give a colorless oil. To a solution (12 ml) of the obtained oil in a mixed solvent of tetrahydrofuran-methanol (3:1, v/v) was added a 1N aqueous sodium hydroxide solution (2.5 ml) at 60°C, and the mixture was stirred for 30 min. 1N Hydrochloric acid (2.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:2, v/v) to give (3-{3-[2-(2,4-dichlorophenoxy)-6-isopropoxypyridin-3-yl]propoxy}-1-methyl-1H-pyrazol-4-yl)acetic acid as a pale-brown oil (422 mg, yield 71%). ¹H-NMR (300 MHz, CDCl₃) δ:1.11 (6 H, d, J = 6.2 Hz), 2.05 - 2.21 (2 H, m), 2.80 (2 H, t, J = 7.4 Hz), 3.42 (2 H, s), 3.69 (3 H, s), 4.21 (2 H, t, J = 6.3 Hz), 4.55 - 4.70 (1 H, m), 6.30 (1 H, d, J = 7.9 Hz), 7.07 - 7.18 (2 H, m), 7.19 - 7.25 (1 H, m), 7.38 - 7.50 (2 H, m).

### Example 131

To a mixture of 3-[2-(2,4-dichlorophenoxy)-6-isopropoxypyridin-3-yl]propan-1-ol (428 mg), methyl (1-benzyl-3-hydroxy-1H-pyrazol-4-yl)acetate (296 mg), tributylphosphine (607 mg) and tetrahydrofuran (30 ml) was added 1,1'-azodicarbonyldipiperidine (606 mg) at 60°C, and the mixture was stirred for 20 min. The reaction mixture was concentrated, diisopropyl ether was added, and the precipitated crystals were removed by filtration. The filtrate was concentrated, and the obtained residue was subjected to silica gel column chromatography and eluted with ethyl acetate-hexane (1:3, v/v) to give a colorless oil. To a solution (12 ml) of the obtained oil in a mixed solvent of tetrahydrofuran-methanol (3:1, v/v) was added a 1N aqueous sodium hydroxide solution (2.5 ml) at 60°C, and the mixture was stirred for 30 min. 1N Hydrochloric acid (2.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:3, v/v) to give (1-benzyl-3-{3-[2-(2,4-dichlorophenoxy)-6-isopropoxypyridin-3-yl]propoxy}-1H-pyrazol-4-yl)acetic acid as a colorless oil (489 mg, yield 71%). ¹H-NMR (300 MHz, CDCl₃) δ:1.11 (6 H, d, J = 6.2 Hz), 2.06 - 2.20 (2 H, m), 2.80 (2 H, t, J = 7.4 Hz), 3.41 (2 H, s), 4.24 (2 H, t, J = 6.3 Hz), 4.57 - 4.68 (1 H, m), 5.08 (2 H, s), 6.30 (1 H, d, J = 8.1 Hz), 7.05 - 7.12 (1 H, m), 7.13 - 7.23 (4 H, m), 7.27 - 7.46 (5 H, m).

### Example 132

To a mixture of 3-{2-[(3,5-dichloropyridin-2-yl)oxy]-4-isopropoxyphenyl}propan-1-ol (0.14 g), methyl (3-hydroxy-1-methyl-1H-pyrazol-4-yl)acetate (67 mg), tributylphosphine (0.245 ml) and tetrahydrofuran (10 ml) was added 1,1'-azodicarbonyldipiperidine (198 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:10 to 1:1, v/v) to give a colorless oil (0.11 g, yield 57%). A mixture of the obtained oil, a 1N aqueous sodium hydroxide solution (0.5 ml), tetrahydrofuran (2 ml) and methanol (2 ml) was stirred at 50°C for 1 hr, 1N hydrochloric acid (0.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:25 to 2:1, v/v). The elute was concentrated, and the residue was crystallized from ethyl acetate-hexane to give [3-(3-{2-[(3,5-dichloropyridin-2-yl)oxy]-4-isopropoxyphenyl}propoxy)-1-methyl-1H-pyrazol-4-yl]acetic acid (110 mg, yield quant.) as a colorless amorphous form. ¹H-NMR (300 MHz, CDCl₃) δ:1.31 (6 H, d, J = 6.2 Hz), 1.86 - 2.08 (2 H, m), 2.50 - 2.67 (2 H, m), 3.37 (2 H, s), 3.68 (3 H, s), 4.12 (2 H, t, J = 6.1 Hz), 4.35 - 4.58 (1 H, m), 6.58 (1 H, d, J = 2.5 Hz), 6.73 (1 H, dd, J = 8.5, 2.6 Hz), 7.13 (1 H, s), 7.18 (1 H, d, J = 8.5 Hz), 7.74 (1 H, d, J = 2.3 Hz), 7.92 (1 H, d, J = 2.3 Hz).

### Example 133

To a mixture of 3-{2-[(3,5-dichloropyridin-2-yl)oxy]-4-isopropoxyphenyl}propan-1-ol (0.25 g), methyl (1-benzyl-3-hydroxy-1H-pyrazol-4-yl)acetate (173 mg), tributylphosphine (0.444 ml) and tetrahydrofuran (15 ml) was added 1,1'-azodicarbonyldipiperidine (354 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:20 to 1:3, v/v) to give a colorless oil (0.25 g, yield 61%). A mixture of the obtained oil, a 1N aqueous sodium hydroxide solution (0.6 ml), tetrahydrofuran (2 ml) and methanol (2 ml) was stirred at 50°C for 1 hr, 1N hydrochloric acid (0.6 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:25 to 2:1, v/v). The elute was concentrated, and the residue was crystallized from ethyl acetate-hexane to give [1-benzyl-3-(3-{2-[(3,5-dichloropyridin-2-yl)oxy]-4-isopropoxyphenyl}propoxy)-1H-pyrazol-4-yl]acetic acid (110 mg, yield quant.) as colorless crystals. melting point 111 - 113°C.

### Example 134

To a mixture of 3-[2-(2,4-dichlorophenoxy)-6-ethoxypyridin-3-yl]propan-1-ol (342 mg), methyl (2-hydroxy-3-methoxyphenyl)acetate (196 mg), tributylphosphine (0.623 ml) and tetrahydrofuran (15 ml) was added 1,1'-azodicarbonyldipiperidine (505 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:10 to 1:3, v/v) to give a colorless oil (0.19 g). A mixture of the obtained oil, a 1N aqueous sodium hydroxide solution (0.7 ml), tetrahydrofuran (2 ml) and methanol (2 ml) was stirred at 50°C for 1 hr, 1N hydrochloric acid (0.7 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 2:1, v/v). The elute was concentrated, and the residue was crystallized from ethyl acetate-hexane to give (2-{3-[2-(2,4-dichlorophenoxy)-6-ethoxypyridin-3-yl]propoxy}-3-methoxyphenyl)acetic acid (104 mg, yield 20%) as colorless crystals. melting point 89 - 91°C.

### Example 135

To a mixture of 3-[2-(2,4-dichlorophenoxy)-6-ethoxypyridin-3-yl]propan-1-ol (342 mg), methyl (3-hydroxy-1-methyl-1H-pyrazol-4-yl)acetate (170 mg), tributylphosphine (0.623 ml) and tetrahydrofuran (15 ml) was added 1,1'-azodicarbonyldipiperidine (505 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:10 to 1:3, v/v) to give a colorless oil (0.24 g). A mixture of the obtained oil (0.25 g), a 1N aqueous sodium hydroxide solution (0.8 ml), tetrahydrofuran (2 ml) and methanol (2 ml) was stirred at 60°C for 1 hr, 1N hydrochloric acid (0.8 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 2:1, v/v). The elute was concentrated, and the residue was crystallized from ethyl acetate-hexane to give (3-{3-[2-(2,4-dichlorophenoxy)-6-ethoxypyridin-3-yl]propoxy}-1-methyl-1H-pyrazol-4-yl)acetic acid (187 mg, yield 39%) as colorless crystals. melting point 81 - 83°C.

### Example 136

To a mixture of 3-(2-{[3-chloro-5-(trifluoromethyl)pyridin-2-yl]oxy}-4-isopropoxyphenyl)propan-1-ol (585 mg), ethyl 3-(3-hydroxy-1-phenyl-1H-pyrazol-5-yl)propanoate (390 mg), tributylphosphine (759 mg) and tetrahydrofuran (30 ml) was added 1,1'-azodicarbonyldipiperidine (758 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:5, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at 50°C, and the mixture was stirred for 30 min. 1N Hydrochloric acid (3.0 ml) was added, and the mixture was stirred at and extracted with ethyl acetate. The ethyl acetate layer was washed with saturated brine, dried (MgSO₄) and concentrated. The residue was recrystallized from ethyl acetate-hexane to give 3-{3-[3-(2-{[3-chloro-5-(trifluoromethyl)pyridin-2-yl]oxy}-4-isopropoxyphenyl)propoxy]-1-phenyl-1H-pyrazol-5-yl}propanoic acid as colorless prism crystals (681 mg, yield 75%). melting point 86 - 88°C.

### Example 137

To a mixture of 3-(2-{[3-chloro-5-(trifluoromethyl)pyridin-2-yl]oxy}-4-isopropoxyphenyl)propan-1-ol (585 mg), ethyl 3-(1-benzyl-3-hydroxy-1H-pyrazol-5-yl)propanoate (411 mg), tributylphosphine (759 mg) and tetrahydrofuran (30 ml) was added 1,1'-azodicarbonyldipiperidine (758 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:8, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at 50°C, and the mixture was stirred for 30 min. 1N Hydrochloric acid (3.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:4, v/v) to give 3-{1-benzyl-3-[3-(2-{[3-chloro-5-(trifluoromethyl)pyridin-2-yl]oxy}-4-isopropoxyphenyl)propoxy]-1H-pyrazol-5-yl}propanoic acid as a colorless oil. Recrystallization from ethyl acetate-hexane gave colorless prism crystals (272 mg, yield 29%). melting point 99 - 100°C.

### Example 138

To a mixture of 3-{2-[(3,5-dichloropyridin-2-yl)oxy]-4-isopropoxyphenyl}propan-1-ol (534 mg), ethyl 3-(3-hydroxy-1-phenyl-1H-pyrazol-5-yl)propanoate (390 mg), tributylphosphine (759 mg) and tetrahydrofuran (30 ml) was added 1,1'-azodicarbonyldipiperidine (758 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:8, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at 50°C, and the mixture was stirred for 30 min. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-[3-(3-{2-[(3,5-dichloropyridin-2-yl)oxy]-4-isopropoxyphenyl}propoxy)-1-phenyl-1H-pyrazol-5-yl]propanoic acid as colorless prism crystals (604 mg, yield 71%). ¹H-NMR (300 MHz, CDCl₃)δ:1.31 (6 H, d, J = 6.0 Hz), 1.94 - 2.08 (2 H, m), 2.55 - 2.67 (4 H, m), 2.92 (2 H, t, J = 7.6 Hz), 4.12 (2 H, t, J = 6.2 Hz), 4.40 - 4.53 (1 H, m), 5.62 (1 H, s), 6.59 (1 H, d, J = 2.5 Hz), 6.73 (1 H, dd, J = 8.5, 2.5 Hz), 7.20 (1 H, d, J = 8.5 Hz), 7.29 - 7.48 (5 H, m), 7.73 (1 H, d, J = 2.3 Hz), 7.93 (1 H, d, J = 2.3 Hz).

### Example 139

To a mixture of 3-(2-{[3-chloro-5-(trifluoromethyl)pyridin-2-yl]oxy}-4-isopropoxyphenyl)propan-1-ol (468 mg), ethyl 3-(3-hydroxy-1-methyl-1H-pyrazol-5-yl)propanoate (238 mg), tributylphosphine (607 mg) and tetrahydrofuran (30 ml) was added 1,1'-azodicarbonyldipiperidine (606 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:3, v/v) to give a colorless oil. To a solution (20 ml) of the obtained oil in a mixed solvent of tetrahydrofuran-methanol (3:1, v/v) was added a 1N aqueous sodium hydroxide solution (2.5 ml) at room temperature, and the mixture was stirred for 1 hr. 1N Hydrochloric acid (2.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:2, v/v) to give 3-{3-[3-(2-{[3-chloro-5-(trifluoromethyl)pyridin-2-yl]oxy}-4-isopropoxyphenyl)propoxy]-1-methyl-1H-pyrazol-5-yl}propanoic acid as a colorless oil. Recrystallization from ethyl acetate-hexane gave colorless prism crystals (362 mg, yield 56%). melting point 88 - 89°C.

### Example 140

To a mixture of 3-{2-[(3,5-dichloropyridin-2-yl)oxy]-4-isopropoxyphenyl}propan-1-ol (428 mg), ethyl 3-(3-hydroxy-1-methyl-1H-pyrazol-5-yl)propanoate (238 mg), tributylphosphine (607 mg) and tetrahydrofuran (30 ml) was added 1,1'-azodicarbonyldipiperidine (606 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:3 to 1:2, v/v) to give a colorless oil. To a solution (20 ml) of the obtained oil in a mixed solvent of tetrahydrofuran-methanol (3:1, v/v) was added a 1N aqueous sodium hydroxide solution (2.5 ml) at 50°C, and the mixture was stirred for 1 hr. 1N Hydrochloric acid (2.5 ml) was added, and the mixture was extracted with ethyl acetate. The ethyl acetate layer was washed with saturated brine, dried (MgSO₄) and concentrated. The residue was recrystallized from ethyl acetate-hexane to give 3-[3-(3-{2-[(3,5-dichloropyridin-2-yl)oxy]-4-isopropoxyphenyl}propoxy)-1-methyl-1H-pyrazol-5-yl]propanoic acid as colorless prism crystals (317 mg, yield 52%). melting point 77 - 78°C.

### Example 141

To a mixture of 3-(2-{[3-chloro-5-(trifluoromethyl)pyridin-2-yl]oxy}-4-isopropoxyphenyl)propan-1-ol (468 mg), ethyl 3-(1-ethyl-3-hydroxy-1H-pyrazol-5-yl)propanoate (255 mg), tributylphosphine (607 mg) and tetrahydrofuran (30 ml) was added 1,1'-azodicarbonyldipiperidine (606 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:4, v/v) to give a pale-yellow oil. To a solution (20 ml) of the obtained oil in a mixed solvent of tetrahydrofuran-methanol (3:1, v/v) was added a 1N aqueous sodium hydroxide solution (3.0 ml) at room temperature, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-{3-[3-(2-{[3-chloro-5-(trifluoromethyl)pyridin-2-yl]oxy}-4-isopropoxyphenyl)propoxy]-1-ethyl-1H-pyrazol-5-yl}propanoic acid as colorless prism crystals (255 mg, yield 38%). melting point 73 - 74°C.

### Example 142

To a mixture of 3-(2-{[3-chloro-5-(trifluoromethyl)pyridin-2-yl]oxy}-4-isopropoxyphenyl)propan-1-ol (585 mg), ethyl 3-(3-hydroxy-1-isopropyl-1H-pyrazol-5-yl)propanoate (339 mg), tributylphosphine (607 mg) and tetrahydrofuran (30 ml) was added 1,1'-azodicarbonyldipiperidine (606 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:4, v/v) to give a pale-yellow oil. To a solution (20 ml) of the obtained oil in a mixed solvent of tetrahydrofuran-methanol (3:1, v/v) was added a 1N aqueous sodium hydroxide solution (3.0 ml) at room temperature, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-{3-[3-(2-{[3-chloro-5-(trifluoromethyl)pyridin-2-yl]oxy}-4-isopropoxyphenyl)propoxy]-1-isopropyl-1H-pyrazol-5-yl}propanoic acid as colorless prism crystals (406 mg, yield 59%). melting point 127.5 - 128.5°C.

### Example 143

To a mixture of 3-(2-{[3-chloro-5-(trifluoromethyl)pyridin-2-yl]oxy}-4-isopropoxyphenyl)propan-1-ol (585 mg), ethyl 3-(3-hydroxy-1-isobutyl-1H-pyrazol-5-yl)propanoate (360 mg), tributylphosphine (758 mg) and tetrahydrofuran (30 ml) was added 1,1'-azodicarbonyldipiperidine (757 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:5, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at room temperature, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-{3-[3-(2-{[3-chloro-5-(trifluoromethyl)pyridin-2-yl]oxy}-4-isopropoxyphenyl)propoxy]-1-isobutyl-1H-pyrazol-5-yl}propanoic acid as colorless prism crystals (384 mg, yield 44%). melting point 71 - 73°C.

### Example 144

To a mixture of 3-(2-{[3-chloro-5-(trifluoromethyl)pyridin-2-yl]oxy}-4-isopropoxyphenyl)propan-1-ol (585 mg), ethyl 3-(1-cyclohexyl-3-hydroxy-1H-pyrazol-5-yl)propanoate (400 mg), tributylphosphine (758 mg) and tetrahydrofuran (30 ml) was added 1,1'-azodicarbonyldipiperidine (757 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:9, v/v) to give colorless crystals. To a solution (20 ml) of the obtained crystal in a mixed solvent of tetrahydrofuran-methanol (3:1, v/v) was added a 1N aqueous sodium hydroxide solution (3.0 ml) at room temperature, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-{3-[3-(2-{[3-chloro-5-(trifluoromethyl)pyridin-2-yl]oxy}-4-isopropoxyphenyl)propoxy]-1-cyclohexyl-1H-pyrazol-5-yl}propanoic acid as colorless prism crystals (452 mg, yield 49%). melting point 114 - 115°C .

### Example 145

To a mixture of 3-(2-{[3-chloro-5-(trifluoromethyl)pyridin-2-yl]oxy}-4-isopropoxyphenyl)propan-1-ol (468 mg), ethyl 3-(3-hydroxy-1-methyl-1H-pyrazol-4-yl)propanoate (238 mg), tributylphosphine (607 mg) and tetrahydrofuran (30 ml) was added 1,1'-azodicarbonyldipiperidine (606 mg) at 60°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:4, v/v) to give a colorless oil. To a solution (20 ml) of the obtained oil in a mixed solvent of tetrahydrofuran-methanol (3:1, v/v) was added a 1N aqueous sodium hydroxide solution (2.5 ml) at room temperature, and the mixture was stirred for 1 hr. 1N Hydrochloric acid (2.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:4, v/v) to give 3-{3-[3-(2-{[3-chloro-5-(trifluoromethyl)pyridin-2-yl]oxy}-4-isopropoxyphenyl)propoxy]-1-methyl-1H-pyrazol-4-yl}propanoic acid as a colorless oil (193 mg, yield 30%). ¹H-NMR (300 MHz, CDCl₃) δ:1.32 (6 H, d, J = 6.0 Hz), 1.93 - 2.06 (2 H, m), 2.48 - 2.66 (6 H, m), 3.64 (3 H, s), 4.10 (2 H, t, J = 6.2 Hz), 4.41 - 4.56 (1 H, m), 6.62 (1 H, d, J = 2.5 Hz), 6.77 (1 H, dd, J = 8.5, 2.5 Hz), 6.94 (1 H, s), 7.22 (1 H, d, J = 8.5 Hz), 7.96 (1 H, d, J = 2.3 Hz), 8.20 - 8.29 (1 H, m).

### Example 146

To a mixture of 3-(2-{[3-chloro-5-(trifluoromethyl)pyridin-2-yl]oxy}-4-isopropoxyphenyl)propan-1-ol (468 mg), ethyl 3-(3-hydroxy-1-phenyl-1H-pyrazol-4-yl)propanoate (312 mg), tributylphosphine (607 mg) and tetrahydrofuran (30 ml) was added 1,1'-azodicarbonyldipiperidine (606 mg) at 60°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:8, v/v) to give a colorless oil. To a solution (20 ml) of the obtained oil in a mixed solvent of tetrahydrofuran-methanol (3:1, v/v) was added a 1N aqueous sodium hydroxide solution (2.5 ml) at room temperature, and the mixture was stirred for 1 hr. 1N Hydrochloric acid (2.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:4, v/v) to give 3-{3-[3-(2-{[3-chloro-5-(trifluoromethyl)pyridin-2-yl]oxy}-4-isopropoxyphenyl)propoxy]-1-phenyl-1H-pyrazol-4-yl}propanoic acid as a colorless oil (461 mg, yield 64%). ¹H-NMR (300 MHz, CDCl₃)δ:1.32 (6 H, d, J = 6.0 Hz), 1.98 - 2.13 (2 H, m), 2.55 - 2.75 (6 H, m), 4.26 (2 H, t, J = 6.2 Hz), 4.41 - 4.55 (1 H, m), 6.63 (1 H, d, J = 2.5 Hz), 6.78 (1 H, dd, J = 8.5, 2.5 Hz), 7.09 - 7.26 (2 H, m), 7.30 - 7.42 (2 H, m), 7.46 - 7.58 (3 H, m), 7.94 (1 H, d, J = 2.1 Hz), 8.21 - 8.27 (1 H, m).

### Example 147

To a mixture of 3-[2-{[3-chloro-5-(trifluoromethyl)pyridin-2-yl]oxy}-4-(2-methoxyethoxy)phenyl]propan-1-ol (609 mg), ethyl 3-(3-hydroxy-1-phenyl-1H-pyrazol-5-yl)propanoate (390 mg), tributylphosphine (759 mg) and tetrahydrofuran (30 ml) was added 1,1'-azodicarbonyldipiperidine (758 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:5, v/v) to give colorless crystals. To a solution (20 ml) of the obtained crystal in a mixed solvent of tetrahydrofuran-methanol (3:1, v/v) was added a 1N aqueous sodium hydroxide solution (3.0 ml) at 50°C, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-(3-{3-[2-{[3-chloro-5-(trifluoromethyl)pyridin-2-yl]oxy}-4-(2-methoxyethoxy)phenyl]propoxy}-1-phenyl-1H-pyrazol-5-yl)propanoic acid as colorless prism crystals (711 mg, yield 76%). melting point 128 - 129°C.

### Example 148

To a mixture of 3-[2-{[3-chloro-5-(trifluoromethyl)pyridin-2-yl]oxy}-4-(2-methoxyethoxy)phenyl]propan-1-ol (263 mg), ethyl 3-(3-hydroxy-1-methyl-1H-pyrazol-5-yl)propanoate (129 mg), tributylphosphine (329 mg) and tetrahydrofuran (30 ml) was added 1,1'-azodicarbonyldipiperidine (328 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:2, v/v) to give colorless crystals. To a solution (12 ml) of the obtained crystals in a mixed solvent of tetrahydrofuran-methanol (3:1, v/v) was added a 1N aqueous sodium hydroxide solution (1.5 ml) at room temperature, and the mixture was stirred for 30 min. 1N Hydrochloric acid (1.5 ml) was added, and the mixture was extracted with ethyl acetate. The ethyl acetate layer was washed with saturated brine, dried (MgSO₄) and concentrated. The residue was recrystallized from ethyl acetate-hexane to give 3-(3-{3-[2-{[3-chloro-5-(trifluoromethyl)pyridin-2-yl]oxy}-4-(2-methoxyethoxy)phenyl]propoxy}-1-methyl-1H-pyrazol-5-yl)propanoic acid as colorless prism crystals (233 mg, yield 64%). melting point 114 - 115°C.

### Example 149

To a mixture of 3-[2-{[3-chloro-5-(trifluoromethyl)pyridin-2-yl]oxy}-4-(2-methoxyethoxy)phenyl]propan-1-ol (609 mg), ethyl 3-(1-ethyl-3-hydroxy-1H-pyrazol-5-yl)propanoate (318 mg), tributylphosphine (758 mg) and tetrahydrofuran (30 ml) was added 1,1'-azodicarbonyldipiperidine (757 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:3, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at room temperature, and the mixture was stirred for 30 min. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-(3-{3-[2-{[3-chloro-5-(trifluoromethyl)pyridin-2-yl]oxy}-4-(2-methoxyethoxy)phenyl]propoxy}-1-ethyl-1H-pyrazol-5-yl)propanoic acid as colorless prism crystals (180 mg, yield 21%). melting point 101 - 103°C.

### Example 150

To a mixture of 3-[2-{[3-chloro-5-(trifluoromethyl)pyridin-2-yl]oxy}-4-(2-isopropoxyethoxy)phenyl]propan-1-ol (341 mg), ethyl 3-(1-ethyl-3-hydroxy-1H-pyrazol-5-yl)propanoate (167 mg), tributylphosphine (398 mg) and tetrahydrofuran (30 ml) was added 1,1'-azodicarbonyldipiperidine (397 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:3, v/v) to give a colorless oil. To a solution (12 ml) of the obtained oil in a mixed solvent of tetrahydrofuran-methanol (3:1, v/v) was added a 1N aqueous sodium hydroxide solution (1.5 ml) at room temperature, and the mixture was stirred for 30 min. 1N Hydrochloric acid (1.5 ml) was added, and the mixture was extracted with ethyl acetate. The ethyl acetate layer was washed with saturated brine, dried (MgSO₄) and concentrated. The residue was recrystallized from ethyl acetate-hexane to give 3-(3-{3-[2-{[3-chloro-5-(trifluoromethyl)pyridin-2-yl]oxy}-4-(2-isopropoxyethoxy)phenyl]propoxy}-1-ethyl-1H-pyrazol-5-yl)propanoic acid as colorless prism crystals (270 mg, yield 57%). melting point 130.5 - 131.5°C.

### Example 151

To a mixture of 3-(2-isopropoxy-4-{[5-(trifluoromethyl)pyridin-2-yl]oxy}phenyl)propan-1-ol (426 mg), ethyl 3-(3-hydroxy-1-phenyl-1H-pyrazol-5-yl)propanoate (312 mg), tributylphosphine (607 mg) and tetrahydrofuran (30 ml) was added 1,1'-azodicarbonyldipiperidine (606 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:8, v/v) to give a colorless oil. To a solution (20 ml) of the obtained oil in a mixed solvent of tetrahydrofuran-methanol (3:1, v/v) was added a 1N aqueous sodium hydroxide solution (2.5 ml) at 50°C, and the mixture was stirred for 1 hr. 1N Hydrochloric acid (2.5 ml) was added, and the mixture was extracted with ethyl acetate. The ethyl acetate layer was washed with saturated brine, dried (MgSO₄) and concentrated. The residue was recrystallized from ethyl acetate-hexane to give 3-{3-[3-(2-isopropoxy-4-{[5-(trifluoromethyl)pyridin-2-yl]oxy}phenyl)propoxy]-1-phenyl-1H-pyrazol-5-yl}propanoic acid as colorless prism crystals (497 mg, yield 73%). melting point 129.5 - 130.5°C.

### Example 152

To a mixture of 3-(2-isopropoxy-4-{[5-(trifluoromethyl)pyridin-2-yl]oxy}phenyl)propan-1-ol (426 mg), ethyl 3-(3-hydroxy-1-methyl-1H-pyrazol-5-yl)propanoate (238 mg), tributylphosphine (607 mg) and tetrahydrofuran (30 ml) was added 1,1'-azodicarbonyldipiperidine (606 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:3, v/v) to give a colorless oil. To a solution (20 ml) of the obtained oil in a mixed solvent of tetrahydrofuran-methanol (3:1, v/v) was added a 1N aqueous sodium hydroxide solution (2.5 ml) at 50°C, and the mixture was stirred for 1 hr. 1N Hydrochloric acid (2.5 ml) was added, and the mixture was extracted with ethyl acetate. The ethyl acetate layer was washed with saturated brine, dried (MgSO₄) and concentrated. The residue was recrystallized from ethyl acetate-hexane to give 3-{3-[3-(2-isopropoxy-4-{[5-(trifluoromethyl)pyridin-2-yl]oxy}phenyl)propoxy]-1-methyl-1H-pyrazol-5-yl}propanoic acid as colorless prism crystals (292 mg, yield 48%). melting point 128 - 130°C.

### Example 153

To a mixture of 3-(2-(2-methoxyethoxy)-4-{[5-(trifluoromethyl)pyridin-2-yl]oxy}phenyl)propan-1-ol (446 mg), ethyl 3-(3-hydroxy-1-phenyl-1H-pyrazol-5-yl)propanoate (312 mg), tributylphosphine (607 mg) and tetrahydrofuran (30 ml) was added 1,1'-azodicarbonyldipiperidine (606 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:4, v/v) to give a colorless oil. To a solution (20 ml) of the obtained oil in a mixed solvent of tetrahydrofuran-methanol (3:1, v/v) was added a 1N aqueous sodium hydroxide solution (2.5 ml) at 50°C, and the mixture was stirred for 1 hr. 1N Hydrochloric acid (2.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:3, v/v) to give 3-{3-[3-(2-(2-methoxyethoxy)-4-{[5-(trifluoromethyl)pyridin-2-yl]oxy}phenyl)propoxy]-1-phenyl-1H-pyrazol-5-yl}propanoic acid as a colorless oil (392 mg, yield 56%). Recrystallization from ethyl acetate-hexane gave colorless prism crystals. melting point 83 - 85°C.

### Example 154

To a mixture of 3-(2-(2-methoxyethoxy)-4-{[5-(trifluoromethyl)pyridin-2-yl]oxy}phenyl)propan-1-ol (426 mg), ethyl 3-(3-hydroxy-1-methyl-1H-pyrazol-5-yl)propanoate (238 mg), tributylphosphine (607 mg) and tetrahydrofuran (30 ml) was added 1,1'-azodicarbonyldipiperidine (606 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:2, v/v) to give a colorless oil. To a solution (20 ml) of the obtained oil in a mixed solvent of tetrahydrofuran-methanol (3:1, v/v) was added a 1N aqueous sodium hydroxide solution (2.5 ml) at room temperature, and the mixture was stirred for 1 hr. 1N Hydrochloric acid (2.5 ml) was added, and the mixture was extracted with ethyl acetate. The ethyl acetate layer was washed with saturated brine, dried (MgSO₄) and concentrated. The residue was recrystallized from ethyl acetate-hexane to give 3-{3-[3-(2-(2-methoxyethoxy)-4-{[5-(trifluoromethyl)pyridin-2-yl]oxy}phenyl)propoxy]-1-methyl-1H-pyrazol-5-yl}propanoic acid as colorless prism crystals (241 mg, yield 38%). melting point 109.5 - 110.5°C.

### Example 155

To a mixture of 4-(2-{[3-chloro-5-(trifluoromethyl)pyridin-2-yl]oxy}-4-isopropoxyphenyl)butan-1-ol (606 mg), ethyl 3-(3-hydroxy-1-methyl-1H-pyrazol-5-yl)propanoate (297 mg), tributylphosphine (758 mg) and tetrahydrofuran (30 ml) was added 1,1'-azodicarbonyldipiperidine (757 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:4, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at room temperature, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-{3-[4-(2-{[3-chloro-5-(trifluoromethyl)pyridin-2-yl]oxy}-4-isopropoxyphenyl)butoxy]-1-methyl-1H-pyrazol-5-yl}propanoic acid as colorless prism crystals (299 mg, yield 36%). melting point 156 - 157°C.

### Example 156

To a mixture of 4-(2-{[3-chloro-5-(trifluoromethyl)pyridin-2-yl]oxy}-4-isopropoxyphenyl)butan-1-ol (606 mg), ethyl 3-(3-hydroxy-1-phenyl-1H-pyrazol-5-yl)propanoate (390 mg), tributylphosphine (758 mg) and tetrahydrofuran (30 ml) was added 1,1'-azodicarbonyldipiperidine (757 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:7, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at room temperature, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-{3-[4-(2-{[3-chloro-5-(trifluoromethyl)pyridin-2-yl]oxy}-4-isopropoxyphenyl)butoxy]-1-phenyl-1H-pyrazol-5-yl}propanoic acid as colorless prism crystals (648 mg, yield 70%). melting point 75 - 77°C.

### Example 157

To a mixture of 4-(2-{[3-chloro-5-(trifluoromethyl)pyridin-2-yl]oxy}-4-isopropoxyphenyl)butan-1-ol (606 mg), ethyl 3-(3-hydroxy-1-isobutyl-1H-pyrazol-5-yl)propanoate (360 mg), tributylphosphine (758 mg) and tetrahydrofuran (30 ml) was added 1,1'-azodicarbonyldipiperidine (757 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:5, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at room temperature, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-{3-[4-(2-{[3-chloro-5-(trifluoromethyl)pyridin-2-yl]oxy}-4-isopropoxyphenyl)butoxy]-1-isobutyl-1H-pyrazol-5-yl}propanoic acid as colorless prism crystals (630 mg, yield 70%). melting point 122 - 123°C.

### Example 158

To a mixture of 4-(2-{[3-chloro-5-(trifluoromethyl)pyridin-2-yl]oxy}-4-isopropoxyphenyl)butan-1-ol (606 mg), ethyl 3-(1-ethyl-3-hydroxy-1H-pyrazol-5-yl)propanoate (318 mg), tributylphosphine (607 mg) and tetrahydrofuran (30 ml) was added 1,1'-azodicarbonyldipiperidine (606 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:5, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at room temperature, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-{3-[4-(2-{[3-chloro-5-(trifluoromethyl)pyridin-2-yl]oxy}-4-isopropoxyphenyl)butoxy]-1-ethyl-1H-pyrazol-5-yl}propanoic acid as colorless prism crystals (532 mg, yield 62%). melting point 129 - 130°C.

### Example 159

To a mixture of 4-(2-{[3-chloro-5-(trifluoromethyl)pyridin-2-yl]oxy}-4-isopropoxyphenyl)butan-1-ol (606 mg), ethyl 3-(3-hydroxy-1-isopropyl-1H-pyrazol-5-yl)propanoate (339 mg), tributylphosphine (607 mg) and tetrahydrofuran (30 ml) was added 1,1'-azodicarbonyldipiperidine (606 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:10, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at room temperature, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-{3-[4-(2-{[3-chloro-5-(trifluoromethyl)pyridin-2-yl]oxy}-4-isopropoxyphenyl)butoxy]-1-isopropyl-1H-pyrazol-5-yl}propanoic acid as colorless prism crystals (456 mg, yield 52%). melting point 99.5 - 100.5°C.

### Example 160

To a mixture of 3-{2-[(2,4-dichlorobenzyl)oxy]-4-isopropoxyphenyl}propan-1-ol (554 mg), ethyl 3-(3-hydroxy-1-phenyl-1H-pyrazol-5-yl)propanoate (390 mg), tributylphosphine (758 mg) and tetrahydrofuran (30 ml) was added 1,1'-azodicarbonyldipiperidine (757 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:8, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at room temperature, and the mixture was stirred for 30 min. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-[3-(3-{2-[(2,4-dichlorobenzyl)oxy]-4-isopropoxyphenyl}propoxy)-1-phenyl-1H-pyrazol-5-yl]propanoic acid as colorless prism crystals (665 mg, yield 76%). melting point 139 - 141°C.

### Example 161

To a mixture of 3-{2-[(2,4-dichlorobenzyl)oxy]-4-isopropoxyphenyl}propan-1-ol (554 mg), ethyl 3-(3-hydroxy-1-methyl-1H-pyrazol-5-yl)propanoate (297 mg), tributylphosphine (758 mg) and tetrahydrofuran (30 ml) was added 1,1'-azodicarbonyldipiperidine (757 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:2, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at room temperature, and the mixture was stirred for 30 min. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-[3-(3-{2-[(2,4-dichlorobenzyl)oxy]-4-isopropoxyphenyl}propoxy)-1-methyl-1H-pyrazol-5-yl]propanoic acid as colorless prism crystals (516 mg, yield 66%). melting point 125 - 127°C.

### Example 162

To a mixture of 3-{2-[(2,4-dichlorobenzyl)oxy]-4-isopropoxyphenyl}propan-1-ol (554 mg), ethyl 3-(3-hydroxy-1-isobutyl-1H-pyrazol-5-yl)propanoate (360 mg), tributylphosphine (607 mg) and tetrahydrofuran (30 ml) was added 1,1'-azodicarbonyldipiperidine (606 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:4, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at room temperature, and the mixture was stirred for 30 min. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-[3-(3-{2-[(2,4-dichlorobenzyl)oxy]-4-isopropoxyphenyl}propoxy)-1-isobutyl-1H-pyrazol-5-yl]propanoic acid as colorless prism crystals (560 mg, yield 66%). melting point 133 - 134°C.

### Example 163

To a mixture of 3-{2-[(2,4-dichlorobenzyl)oxy]-4-isopropoxyphenyl}propan-1-ol (554 mg), ethyl 3-(1-ethyl-3-hydroxy-1H-pyrazol-5-yl)propanoate (318 mg), tributylphosphine (758 mg) and tetrahydrofuran (30 ml) was added 1,1'-azodicarbonyldipiperidine (757 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:4, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at room temperature, and the mixture was stirred for 30 min. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-[3-(3-{2-[(2,4-dichlorobenzyl)oxy]-4-isopropoxyphenyl}propoxy)-1-ethyl-1H-pyrazol-5-yl]propanoic acid as colorless prism crystals (413 mg, yield 51%). melting point 110 - 111°C.

### Example 164

To a mixture of 3-[2-[(2,4-dichlorobenzyl)oxy]-4-(2-methoxyethoxy)phenyl]propan-1-ol (578 mg), ethyl 3-(3-hydroxy-1-methyl-1H-pyrazol-5-yl)propanoate (297 mg), tributylphosphine (758 mg) and tetrahydrofuran (30 ml) was added 1,1'-azodicarbonyldipiperidine (757 mg) at 60°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:2, v/v) to give colorless crystals. To a solution (20 ml) of the obtained crystal in a mixed solvent of tetrahydrofuran-methanol (3:1, v/v) was added a 1N aqueous sodium hydroxide solution (3.0 ml) at room temperature, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-(3-{3-[2-[(2,4-dichlorobenzyl)oxy]-4-(2-methoxyethoxy)phenyl]propoxy}-1-methyl-1H-pyrazol-5-yl)propanoic acid as colorless prism crystals (411 mg, yield 51%). melting point 128.5 - 129.5°C.

### Example 165

To a mixture of 3-[2-[(2,4-dichlorobenzyl)oxy]-4-(2-methoxyethoxy)phenyl]propan-1-ol (578 mg), ethyl 3-(1-ethyl-3-hydroxy-1H-pyrazol-5-yl)propanoate (318 mg), tributylphosphine (758 mg) and tetrahydrofuran (30 ml) was added 1,1'-azodicarbonyldipiperidine (757 mg) at 60°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:2, v/v) to give colorless crystals. To a solution (20 ml) of the obtained crystal in a mixed solvent of tetrahydrofuran-methanol (3:1, v/v) was added a 1N aqueous sodium hydroxide solution (3.0 ml) at room temperature, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-(3-{3-{2-[(2,4-dichlorobenzyl)oxy]-4-(2-methoxyethoxy)phenyl}propoxy}-1-ethyl-1H-pyrazol-5-yl)propanoic acid as colorless prism crystals (450 mg, yield 54%). melting point 114 - 116°C.

### Example 166

To a mixture of 3-(4-isopropoxy-2-{[4-(trifluoromethyl)benzyl]oxy}phenyl)propan-1-ol (616 mg), ethyl 3-(3-hydroxy-1-phenyl-1H-pyrazol-5-yl)propanoate (390 mg), tributylphosphine (607 mg) and tetrahydrofuran (30 ml) was added 1,1'-azodicarbonyldipiperidine (606 mg) at 60°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:5, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at room temperature, and the mixture was stirred for 30 min. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-{3-[3-(4-isopropoxy-2-{[4-(trifluoromethyl)benzyl]oxy}phenyl)propoxy]-1-phenyl-1H-pyrazol-5-yl}propanoic acid as colorless prism crystals (734 mg, yield 84%). melting point 118 - 119°C.

### Example 167

To a mixture of 3-(4-isopropoxy-2-{[4-(trifluoromethyl)benzyl]oxy}phenyl)propan-1-ol (616 mg), ethyl 3-(3-hydroxy-1-methyl-1H-pyrazol-5-yl)propanoate (297 mg), tributylphosphine (607 mg) and tetrahydrofuran (30 ml) was added 1,1'-azodicarbonyldipiperidine (606 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:3, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at room temperature, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-{3-[3-(4-isopropoxy-2-{[4-(trifluoromethyl)benzyl]oxy}phenyl)propoxy]-1-methyl-1H-pyrazol-5-yl}propanoic acid as colorless prism crystals (426 mg, yield 55%). melting point 88 - 89°C.

### Example 168

To a mixture of 3-(4-isopropoxy-2-{[4-(trifluoromethyl)benzyl]oxy}phenyl)propan-1-ol (616 mg), ethyl 3-(1-ethyl-3-hydroxy-1H-pyrazol-5-yl)propanoate (318 mg), tributylphosphine (758 mg) and tetrahydrofuran (30 ml) was added 1,1'-azodicarbonyldipiperidine (757 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:4, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at room temperature, and the mixture was stirred for 30 min. 1N Hydrochloric acid (3.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:3, v/v) to give 3-{1-ethyl-3-[3-(4-isopropoxy-2-{[4-(trifluoromethyl)benzyl]oxy}phenyl)propoxy]-1H-pyrazol-5-yl}propanoic acid as a colorless oil. Recrystallization from ethyl acetate-hexane gave colorless prism crystals (224 mg, yield 28%). melting point 75 - 76°C.

### Example 169

To a mixture of 3-(4-(2-methoxyethoxy)-2-{[4-(trifluoromethyl)benzyl]oxy}phenyl)propan-1-ol (577 mg), ethyl 3-(3-hydroxy-1-methyl-1H-pyrazol-5-yl)propanoate (297 mg), tributylphosphine (758 mg) and tetrahydrofuran (30 ml) was added 1,1*'*-azodicarbonyldipiperidine (757 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:2, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at room temperature, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-{3-[3-(4-(2-methoxyethoxy)-2-{[4-(trifluoromethyl)benzyl]oxy}phenyl)propoxy]-1-methyl-1H-pyrazol-5-yl}propanoic acid as colorless prism crystals (461 mg, yield 57%). melting point 110 - 111°C.

### Example 170

To a mixture of 3-(4-(2-methoxyethoxy)-2-{[4-(trifluoromethyl)benzyl]oxy}phenyl)propan-1-ol (577 mg), ethyl 3-(1-ethyl-3-hydroxy-1H-pyrazol-5-yl)propanoate (318 mg), tributylphosphine (758 mg) and tetrahydrofuran (30 ml) was added 1,1'-azodicarbonyldipiperidine (757 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:3, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at room temperature, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-{1-ethyl-3-[3-(4-(2-methoxyethoxy)-2-{[4-(trifluoromethyl)benzyl]oxy}phenyl)propoxy]-1H-pyrazol-5-yl}propanoic acid as colorless prism crystals (555 mg, yield 67%). melting point 112 - 113°C.

### Example 171

To a mixture of 3-[2-(2,4-dichlorophenoxy)-6-isopropoxypyridin-3-yl]propan-1-ol (534 mg), ethyl 3-(3-hydroxy-1-phenyl-1H-pyrazol-5-yl)propanoate (390 mg), tributylphosphine (758 mg) and tetrahydrofuran (30 ml) was added 1,1'-azodicarbonyldipiperidine (757 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:8, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at room temperature, and the mixture was stirred for 30 min. 1N Hydrochloric acid (3.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:4, v/v) to give 3-(3-{3-[2-(2,4-dichlorophenoxy)-6-isopropoxypyridin-3-yl]propoxy}-1-phenyl-1H-pyrazol-5-yl)propanoic acid as a colorless oil. Recrystallization from ethyl acetate-hexane gave colorless prism crystals (650 mg, yield 76%). melting point 60 - 62°C.

### Example 172

To a mixture of 3-(4-isopropoxy-2-{[5-(trifluoromethyl)pyridin-2-yl]oxy}phenyl)propan-1-ol (533 mg), ethyl 3-(3-hydroxy-1-phenyl-1H-pyrazol-5-yl)propanoate (390 mg), tributylphosphine (758 mg) and tetrahydrofuran (30 ml) was added 1,1'-azodicarbonyldipiperidine (757 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:8, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at room temperature, and the mixture was stirred for 30 min. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-{3-[3-(4-isopropoxy-2-{[5-(trifluoromethyl)pyridin-2-yl]oxy}phenyl)propoxy]-1-phenyl-1H-pyrazol-5-yl}propanoic acid as colorless prism crystals (629 mg, yield 74%). melting point 80 - 82°C.

### Example 173

To a mixture of 3-(4-isopropoxy-2-{[5-(trifluoromethyl)pyridin-2-yl]oxy}phenyl)propan-1-ol (426 mg), ethyl 3-(3-hydroxy-1-methyl-1H-pyrazol-5-yl)propanoate (238 mg), tributylphosphine (607 mg) and tetrahydrofuran (30 ml) was added 1,1'-azodicarbonyldipiperidine (606 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:2, v/v) to give a colorless oil. To a solution (20 ml) of the obtained oil in a mixed solvent of tetrahydrofuran-methanol (3:1, v/v) was added a 1N aqueous sodium hydroxide solution (2.5 ml) at room temperature, and the mixture was stirred for 30 min. 1N Hydrochloric acid (2.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:2, v/v) to give 3-{3-[3-(4-isopropoxy-2-{[5-(trifluoromethyl)pyridin-2-yl]oxy}phenyl)propoxy]-1-methyl-1H-pyrazol-5-yl}propanoic acid as a colorless oil (302 mg, yield 50%). ¹H-NMR (300 MHz, CDCl₃)δ:1.31 (6 H, d, J = 6.0 Hz), 1.88 - 2.04 (2 H, m), 2.52 - 2.73 (4 H, m), 2.84 (2 H, t, J = 7.4 Hz), 3.62 (3 H, s), 4.00 (2 H, t, J = 6.3 Hz), 4.38 - 4.56 (1 H, m), 5.39 (1 H, s), 6.58 (1 H, d, J = 2.6 Hz), 6.75 (1 H, dd, J = 8.5, 2.6 Hz), 6.93 (1 H, d, J = 8.7 Hz), 7.20 (1 H, d, J = 8.5 Hz), 7.86 (1 H, dd, J = 8.7, 2.5 Hz), 8.39 - 8.48 (1 H, m).

### Example 174

To a mixture of 3-{2-[(2,4-dichlorobenzyl)oxy]-4-isopropoxyphenyl}propan-1-ol (443 mg), methyl (3-hydroxy-1-methyl-1H-pyrazol-4-yl)acetate (204 mg), tributylphosphine (607 mg) and tetrahydrofuran (30 ml) was added 1,1'-azodicarbonyldipiperidine (606 mg) at 60°C, and the mixture was stirred for 10 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:3, v/v) to give a colorless oil. To a solution (20 ml) of the obtained oil in a mixed solvent of tetrahydrofuran-methanol (3:1, v/v) was added a 1N aqueous sodium hydroxide solution (2.5 ml) at 60°C, and the mixture was stirred for 1 hr. 1N Hydrochloric acid (2.5 ml) was added, and the mixture was extracted with ethyl acetate. The ethyl acetate layer was washed with saturated brine, dried (MgSO₄) and concentrated. The residue was recrystallized from ethyl acetate-hexane to give [3-(3-{2-[(2,4-dichlorobenzyl)oxy]-4-isopropoxyphenyl}propoxy)-1-methyl-1H-pyrazol-4-yl]acetic acid as colorless prism crystals (438 mg, yield 72%). melting point 102 - 103°C.

### Example 175

To a mixture of 3-{2-[(2,4-dichlorobenzyl)oxy]-4-isopropoxyphenyl}propan-1-ol (443 mg), methyl (1-benzyl-3-hydroxy-1H-pyrazol-4-yl)acetate (296 mg), tributylphosphine (607 mg) and tetrahydrofuran (30 ml) was added 1,1'-azodicarbonyldipiperidine (606 mg) at 60°C, and the mixture was stirred for 10 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:4, v/v) to give a colorless oil. To a solution (20 ml) of the obtained oil in a mixed solvent of tetrahydrofuran-methanol (3:1, v/v) was added a 1N aqueous sodium hydroxide solution (2.5 ml) at 60°C, and the mixture was stirred for 1 hr. 1N Hydrochloric acid (2.5 ml) was added, and the mixture was extracted with ethyl acetate. The ethyl acetate layer was washed with saturated brine, dried (MgSO₄) and concentrated. The residue was recrystallized from ethyl acetate-hexane to give [1-benzyl-3-(3-{2-[(2,4-dichlorobenzyl)oxy]-4-isopropoxyphenyl}propoxy)-1H-pyrazol-4-yl]acetic acid as colorless prism crystals (452 mg, yield 65%). melting point 103 - 104°C.

### Example 176

To a mixture of 3-{2-[(3,5-dichloropyridin-2-yl)oxy]-4-isopropoxyphenyl}propan-1-ol (8.2 g), methyl (1-ethyl-3-hydroxy-1H-pyrazol-4-yl)acetate (4.2 g), tributylphosphine (14.3 ml) and tetrahydrofuran (100 ml) was added 1,1'-azodicarbonyldipiperidine (11.6 g) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:2, v/v) to give a colorless oil. To a solution (160 ml) of the obtained oil in a mixed solvent of tetrahydrofuran-methanol (3:1, v/v) was added a 1N aqueous sodium hydroxide solution (50 ml) at 50°C, and the mixture was stirred for 30 min. 1N Hydrochloric acid (50 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:2, v/v) to give a pale-yellow oil. To a solution (30 ml) of the obtained oil in a mixed solvent of tetrahydrofuran-methanol (1:1, v/v) was added a 1N aqueous sodium hydroxide solution (15 ml) at room temperature, and the mixture was stirred for 20 min. The reaction solution was concentrated, and the residue was dissolved in water (50 ml), an aqueous solution (30 ml) of calcium chloride (991 mg) was added, and the mixture was stirred for 15 hr. The reaction mixture was filtered, and the precipitated crystals were washed with water to give [3-(3-{2-[(3,5-dichloropyridin-2-yl)oxy]-4-isopropoxyphenyl}propoxy)-1-ethyl-1H-pyrazol-4-yl]acetic acid 1/2 calcium salt1hydrate as pale-yellow crystals (7.2 g, yield 57%). melting point 225.5 - 226.5°C.

### Example 177

To a mixture of 3-[2-{[3-chloro-5-(trifluoromethyl)pyridin-2-yl]oxy}-4-(2-isopropoxyethoxy)phenyl]propan-1-ol (651 mg), methyl (3-hydroxy-1-methyl-1H-pyrazol-4-yl)acetate (255 mg), tributylphosphine (759 mg) and tetrahydrofuran (30 ml) was added 1,1'-azodicarbonyldipiperidine (758 mg) at 60°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:4, v/v) to give a colorless oil. To a solution (12 ml) of the obtained oil in a mixed solvent of tetrahydrofuran-methanol (3:1, v/v) was added a 1N aqueous sodium hydroxide solution (3.0 ml) at 60°C, and the mixture was stirred for 30 min. 1N Hydrochloric acid (3.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:2, v/v) to give (3-{3-[2-{[3-chloro-5-(trifluoromethyl)pyridin-2-yl]oxy}-4-(2-isopropoxyethoxy)phenyl]propoxy}-1-methyl-1H-pyrazol-4-yl)acetic acid as a colorless oil. Recrystallization from ethyl acetate-hexane gave colorless prism crystals (477 mg, yield 56%). melting point 103.5 - 104.5°C.

### Example 178

To a mixture of 3-(2-{[3-chloro-5-(trifluoromethyl)pyridin-2-yl]oxy}-4-[2-(pyrrolidin-1-yl)ethoxy]phenyl)propan-1-ol (1.2 g), methyl (3-hydroxy-1-methyl-1H-pyrazol-4-yl)acetate (444 mg), tributylphosphine (1.3 ml) and tetrahydrofuran (100 ml) was added 1,1'-azodicarbonyldipiperidine (1.3 g) at room temperature, and the mixture was stirred for 30 min. The reaction solution was concentrated, diisopropyl ether was added to the residue, and the insoluble substance was removed by filtration. The filtrate was concentrated, the obtained residue was subjected to NH-silica gel column chromatography and eluted with ethyl acetate-hexane (1:2, v/v) to give a colorless oil. To a solution (20 ml) of the obtained oil in a mixed solvent of tetrahydrofuran-methanol (3:1, v/v) was added a 1N aqueous sodium hydroxide solution (5.0 ml) at 60°C, and the mixture was stirred for 30 min. 1N Hydrochloric acid (5.0 ml) was added, and the mixture was extracted with ethyl acetate. The ethyl acetate layer was washed with saturated brine, dried (MgSO₄) and concentrated. To a solution (10 ml) of the obtained oil in a mixed solvent of tetrahydrofuran-methanol (1:1, v/v) was added a 1N aqueous sodium hydroxide solution (0.6 ml) at room temperature, and the mixture was stirred for 30 min. The reaction solution was concentrated, and the residue was dissolved in water (10 ml). An aqueous solution (5 ml) of calcium chloride (39 mg) was added, and the mixture was stirred for 2 hr. The reaction solution was filtrated, and the precipitated crystals were washed with water to give {3-[3-(2-{[3-chloro-5-(trifluoromethyl)pyridin-2-yl]oxy}-4-[2-(pyrrolidin-1-yl)ethoxy]phenyl)propoxy]-1-methyl-1H-pyrazol-4-yl}acetic acid 1/2 calcium salt 7 hydrate as colorless crystals (192 mg, yield 10%). ¹H-NMR (300 MHz, CDCl₃)δ:1.66 - 1.81 (4 H, m), 1.81 - 1.98 (2 H, m), 2.40 - 2.66 (6 H, m), 2.82 (2 H, t, J = 5.8 Hz), 3.12 (2 H, s), 3.40 (3 H, s), 3, 92 - 4.08 (4 H, m), 6.59 (1 H, d, J = 2.5 Hz), 6.72 (1 H, dd, J = 8.5, 2.5 Hz), 6.99 (1 H, s), 7.12 (1 H, d, J = 8.5 Hz), 7.91 (1 H, d, J = 2.1 Hz), 8.13 - 8.22 (1 H, m).

### Example 179

To a mixture of methyl {3-[3-(2-{[3-chloro-5-(trifluoromethyl)pyridin-2-yl]oxy}-4-hydroxyphenyl)propoxy]-1-methyl-1H-pyrazol-4-yl}acetate (1.0 g), 1-(2-hydroxyethyl)pyrrolidin-2-one (0.3 ml), tributylphosphine (1.0 ml) and tetrahydrofuran (30 ml) was added 1,1'-azodicarbonyldipiperidine (1.0 g) at room temperature, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:1, v/v) to give an oil. To a solution (12 ml) of the obtained oil in a mixed solvent of tetrahydrofuran-methanol (3:1, v/v) was added a 1N aqueous sodium hydroxide solution (2.5 ml) at 60°C, and the mixture was stirred for 1 hr. The reaction solution was concentrated, and the residue was dissolved in water (20 ml). An aqueous solution (3 ml) of calcium chloride (174 mg) was added, and the mixture was stirred for 30 min. The reaction solution was filtrated, and the precipitated crystals were washed with water to give [3-(3-{2-{[3-chloro-5-(trifluoromethyl)pyridin-2-yl]oxy}-4-[2-(2-oxopyrrolidin-1-yl)ethoxy]phenyl}propoxy)-1-methyl-1H-pyrazol-4-yl]acetic acid 1/2 calcium salt 4 hydrate as colorless crystals (547 mg, yield 42%). melting point 113 - 115°C.

### Example 180

To a mixture of methyl {3-[3-(2-{[3-chloro-5-(trifluoromethyl)pyridin-2-yl]oxy}-4-hydroxyphenyl)propoxy]-1-methyl-1H-pyrazol-4-yl}acetate (1.0 g), 2-(ethylamino)ethanol (0.25 ml), tributylphosphine (1.0 ml) and tetrahydrofuran (40 ml) was added 1,1'-azodicarbonyldipiperidine (1.0 g) at room temperature, and the mixture was stirred for 30 min. The reaction solution was concentrated, diisopropyl ether was added to the residue, and the insoluble substance was removed by filtration. The filtrate was concentrated, and the obtained residue was subjected to NH-silica gel column chromatography and eluted with ethyl acetate-hexane (1:1, v/v) to give an oil. To a solution of the obtained oil in tetrahydrofuran (40 ml) were added acetic anhydride (0.3 ml) and triethylamine (0.5 ml) at room temperature, and the mixture was stirred for 20 min. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (2:1, v/v) to give a colorless oil. To a solution (12 ml) of the obtained oil in a mixed solvent of tetrahydrofuran-methanol (3:1, v/v) was added a 1N aqueous sodium hydroxide solution (1.5 ml) at 60°C, and the mixture was stirred for 15 min. 1N Hydrochloric acid (1.5 ml) was added, and the mixture was extracted with ethyl acetate. The ethyl acetate layer was washed with saturated brine, dried (MgSO₄) and concentrated. To a solution (12 ml) of the residue in a mixed solvent of tetrahydrofuran-methanol (5:1, v/v) was added a 1N aqueous sodium hydroxide solution (0.8 ml), and the mixture was stirred at room temperature for 20 min. Then, an aqueous solution (1 ml) of calcium chloride (52 mg) was added. The reaction solution was filtrated, and the precipitated crystals were washed with water to give {3-[3-(4-{2-[acetyl(ethyl)amino]ethoxy}-2-{[3-chloro-5-(trifluoromethyl)pyridin-2-yl]oxy}phenyl)propoxy]-1-methyl-1H-pyrazol-4-yl}acetic acid 1/2 calcium salt 7.5 hydrate as colorless crystals (373 mg, yield 65%). melting point 103 - 105°C.

### Example 181

To a solution (20 ml) of methyl {3-[3-(2-{[3-chloro-5-(trifluoromethyl)pyridin-2-yl]oxy}-4-hydroxyphenyl)propoxy]-1-methyl-1H-pyrazol-4-yl}acetate (1.0 g) and 2-chloro-N,N-diethylacetamide (0.4 ml) in N,N-dimethylformamide was added potassium carbonate (456 mg), and the mixture was stirred at 60°C for 1.5 hr. Water was poured into the reaction mixture, and the mixture was extracted with ethyl acetate. The ethyl acetate layer was washed with saturated brine, dried (MgSO₄) and concentrated. The residue was subjected to silica gel column chromatography and eluted with ethyl acetate-hexane (2:1, v/v) to give a colorless oil. To a solution (12 ml) of the obtained oil in a mixed solvent of tetrahydrofuran-methanol (3:1, v/v) was added a 1N aqueous sodium hydroxide solution (3.0 ml) at 60°C over 1.5 hr, 1N hydrochloric acid (3.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:1, v/v) to give [3-(3-{2-{[3-chloro-5-(trifluoromethyl)pyridin-2-yl]oxy}-4-[2-(diethylamino)-2-oxoethoxy]phenyl}propoxy)-1-methyl-1H-pyrazol-4-yl]acetic acid as a colorless oil (740 mg, yield 69%). ¹H-NMR (300 MHz, CDCl₃)δ:1.13 (3 H, t, J = 7.2 Hz), 1.21 (3 H, t, J = 7.2 Hz), 1.93 - 2.06 (2 H, m), 2.59 (2 H, t, J = 7.4 Hz), 3.28 (2 H, s), 3.32 - 3.46 (4 H, m), 3.66 (3 H, s), 4.06 - 4.15 (2 H, m), 4.65 (2 H, s), 6.71 (1 H, d, J = 2.5 Hz), 6.81 (1 H, dd, J = 8.5, 2.5 Hz), 7.11 (1 H, s), 7.23 (1 H, d, J = 8.5 Hz), 7.95 (1 H, d, J = 2.3 Hz), 8.16 - 8.24 (1 H, m).

### Example 182

To a mixture of 3-[1-(2,4-dichlorobenzyl)-3-ethoxy-1H-pyrazol-4-yl]propan-1-ol (1.0 g), ethyl 3-(1-benzyl-3-hydroxy-1H-pyrazol-5-yl)propanoate (833 mg), tributylphosphine (1.5 ml) and tetrahydrofuran (50 ml) was added 1,1'-azodicarbonyldipiperidine (1.5 g) at room temperature, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:5, v/v) to give a colorless oil. To a solution (20 ml) of the obtained oil in a mixed solvent of tetrahydrofuran-methanol (3:1, v/v) was added a 1N aqueous sodium hydroxide solution (4.0 ml) at 50°C, and the mixture was stirred for 30 min. 1N Hydrochloric acid (4.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:2, v/v) to give 3-(1-benzyl-3-{3-[1-(2,4-dichlorobenzyl)-3-ethoxy-1H-pyrazol-4-yl]propoxy}-1H-pyrazol-5-yl)propanoic acid as a colorless oil (939 mg, yield 76%). Recrystallization from ethyl acetate-hexane gave colorless prism crystals. melting point 84 - 85°C.

### Example 183

To a mixture of 3-[1-(2,4-dichlorobenzyl)-3-ethoxy-1H-pyrazol-4-yl]propan-1-ol (500 mg), ethyl 3-(3-hydroxy-1-phenyl-1H-pyrazol-5-yl)propanoate (395 mg), tributylphosphine (0.8 ml) and tetrahydrofuran (30 ml) was added 1,1'-azodicarbonyldipiperidine (0.8 g) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:5, v/v) to give a colorless oil. To a solution (20 ml) of the obtained oil in a mixed solvent of tetrahydrofuran-methanol (3:1, v/v) was added a 1N aqueous sodium hydroxide solution (3.0 ml) at 50°C, and the mixture was stirred for 30 min. 1N Hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give 3-(3-{3-[1-(2,4-dichlorobenzyl)-3-ethoxy-1H-pyrazol-4-yl]propoxy}-1-phenyl-1H-pyrazol-5-yl)propanoic acid as colorless prism crystals (710 mg, yield 86%). melting point 132 - 133°C.

### Example 184

To a mixture of 1-(2-hydroxyethyl)-3-isopropoxy-N-[4-(trifluoromethyl)benzyl]-1H-pyrazole-4-carboxamide (483 mg), methyl (3-ethyl-2-hydroxyphenyl)acetate (214 mg), tributylphosphine (658 mg) and tetrahydrofuran (20 ml) was added 1,1'-azodicarbonyldipiperidine (656 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:2, v/v) to give a brown oil. To a solution (20 ml) of the obtained oil in a mixed solvent of tetrahydrofuran-methanol (3:1, v/v) was added a 1N aqueous sodium hydroxide solution (2.5 ml) at 50°C, and the mixture was stirred for 1 hr. 1N Hydrochloric acid (2.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:2, v/v) to give (3-ethyl-2-{2-[3-isopropoxy-4-({[4-(trifluoromethyl)benzyl]amino}carbonyl)-1H-pyrazol-1-yl]ethoxy}phenyl)acetic acid as a pale-yellow oil (109 mg, yield 16%). Recrystallization from ethyl acetate-hexane gave colorless prism crystals. melting point 110 - 111°C.

### Example 185

To a mixture of 1-(2-hydroxyethyl)-3-isopropoxy-N-[4-(trifluoromethyl)benzyl]-1H-pyrazole-4-carboxamide (477 mg), ethyl (4-ethyl-5-hydroxy-1H-pyrazol-1-yl)acetate (255 mg), tributylphosphine (658 mg) and tetrahydrofuran (25 ml) was added 1,1'-azodicarbonyldipiperidine (656 mg) at room temperature, and the mixture was stirred for 15 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (3:2, v/v) to give a brown oil. To a solution (20 ml) of the obtained oil in a mixed solvent of tetrahydrofuran-methanol (3:1, v/v) was added a 1N aqueous sodium hydroxide solution (3.0 ml) at room temperature. After stirring the mixture for 30 min, 1N hydrochloric acid (3.0 ml) was added, and the mixture was extracted with ethyl acetate. The ethyl acetate layer was washed with saturated brine, dried (MgSO₄) and concentrated. The residue was recrystallized from ethyl acetate-hexane to give (4-ethyl-5-{2-[3-isopropoxy-4-({[4-(trifluoromethyl)benzyl]amino}carbonyl)-1H-pyrazol-1-yl]ethoxy}-1H-pyrazol-1-yl)acetic acid as colorless prism crystals (292 mg, yield 43%). melting point 183 - 184°C.

### Example 186

To a mixture of 1-(3-hydroxypropyl)-3-isopropoxy-N-[4-(trifluoromethyl)benzyl]-1H-pyrazole-4-carboxamide (578 mg), methyl (3-ethyl-2-hydroxyphenyl)acetate (350 mg), tributylphosphine (758 mg) and tetrahydrofuran (30 ml) was added 1,1'-azodicarbonyldipiperidine (757 mg) at 50°C, and the mixture was stirred for 30 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:2, v/v) to give an oil. To a solution (20 ml) of the obtained oil in a mixed solvent of tetrahydrofuran-methanol (3:1, v/v) was added a 1N aqueous sodium hydroxide solution (3.0 ml) at 50°C, and the mixture was stirred for 1 hr. 1N Hydrochloric acid (3.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:3, v/v) to give (3-ethyl-2-{3-[3-isopropoxy-4-({[4-(trifluoromethyl)benzyl]amino}carbonyl)-1H-pyrazol-1-yl]propoxy}phenyl)acetic acid as a colorless oil (230 mg, yield 28%). Recrystallization from ethyl acetate-hexane gave colorless prism crystals. melting point 107.5 - 108.5°C.

### Example 187

To a mixture of 2-{4-[2-(2,4-dichlorophenoxy)ethyl]-3-isopropoxy-1H-pyrazol-1-yl}ethanol (431 mg), methyl (3-ethyl-2-hydroxyphenyl)acetate (291 mg), tributylphosphine (1.2 g) and tetrahydrofuran (30 ml) was added 1,1*'*-azodicarbonyldipiperidine (1.2 g) at 60°C, and the mixture was stirred at room temperature for 60 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:7, v/v) to give an oil. To a solution (12 ml) of the obtained oil in a mixed solvent of tetrahydrofuran-methanol (3:1, v/v) was added a 1N aqueous sodium hydroxide solution (2.0 ml) at room temperature, and the mixture was stirred for 1 hr. 1N Hydrochloric acid (2.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:3, v/v) to give [2-(2-{4-[2-(2,4-dichlorophenoxy)ethyl]-3-isopropoxy-1H-pyrazol-1-yl}ethoxy)-3-ethylphenyl]acetic acid as a colorless oil (140 mg, yield 22%). ¹H-NMR (300 MHz, CDCl₃)δ:1.12 (3 H, t, J = 7.5 Hz), 1.33 (6 H, d, J = 6.2 Hz), 2.45 (2 H, q, J = 7.5 Hz), 2.86 (2 H, t, J = 6.9 Hz), 3.50 (2 H, s), 4.02 - 4.17 (4 H, m), 4.26 (2 H, t, J = 4.9 Hz), 4.75 - 4.89 (1 H, m), 6.87 (1 H, d, J = 8.9 Hz), 6.98 - 7.18 (4 H, m), 7.29 - 7.38 (2 H, m).

### Example 188

To a mixture of 2-[4-(2-{[3-chloro-5-(trifluoromethyl)pyridin-2-yl]oxy}ethyl)-3-isopropoxy-1H-pyrazol-1-yl]ethanol (473 mg), methyl (3-ethyl-2-hydroxyphenyl)acetate (291 mg), tributylphosphine (1.2 g) and tetrahydrofuran (30 ml) was added 1,1'-azodicarbonyldipiperidine (1.2 g) at 60°C, and the mixture was stirred at room temperature for 60 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:3, v/v) to give an oil. To a solution (12 ml) of the obtained oil in a mixed solvent of tetrahydrofuran-methanol (3:1, v/v) was added a 1N aqueous sodium hydroxide solution (2.0 ml) at room temperature, and the mixture was stirred for 1 hr. 1N Hydrochloric acid (2.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:4, v/v) to give (2-{2-[4-(2-{[3-chloro-5-(trifluoromethyl)pyridin-2-yl]oxy}ethyl)-3-isopropoxy-1H-pyrazol-1-yl]ethoxy}-3-ethylphenyl)acetic acid as a pale-yellow oil (176 mg, yield 26%). Recrystallization from ethyl acetate-hexane gave colorless prism crystals. melting point 76.5 - 77.5°C.

### Example 189

To a mixture of 2-[4-(2-{[3-chloro-5-(trifluoromethyl)pyridin-2-yl]oxy}ethyl)-3-isopropoxy-1H-pyrazol-1-yl]ethanol (473 mg), ethyl 3-(2-hydroxy-3-methoxyphenyl)propanoate (350 mg), tributylphosphine (607 mg) and tetrahydrofuran (20 ml) was added 1,1'-azodicarbonyldipiperidine (606 mg) at 50°C, and the mixture was stirred for 20 min. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:5, v/v) to give an oil. To a solution (12 ml) of the obtained oil in a mixed solvent of tetrahydrofuran-methanol (3:1, v/v) was added a 1N aqueous sodium hydroxide solution (2.0 ml) at room temperature, and the mixture was stirred for 30 min. 1N Hydrochloric acid (2.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:3, v/v) to give 3-(2-{2-[4-(2-{[3-chloro-5-(trifluoromethyl)pyridin-2-yl]oxy}ethyl)-3-isopropoxy-1H-pyrazol-1-yl]ethoxy}-3-methoxyphenyl)propanoic acid as a colorless oil (320 mg, yield 47%). ¹H-NMR (300 MHz, CDCl₃)δ:1.32 (6 H, d, J = 6.0 Hz), 2.44 - 2.55 (2 H, m), 2.65 - 2.78 (2 H, m), 2.88 (2 H, t, J = 7.6 Hz), 3.82 (3 H, s), 4.22 - 4.35 (4 H, m), 4.55 (2 H, t, J = 7.6 Hz), 4.72 - 4.89 (1 H, m), 6.71 - 6.84 (2 H, m), 6.92 - 7.03 (1 H, m), 7.33 (1 H, s), 7.86 (1 H, d, J = 2.1 Hz), 8.32 - 8.40 (1 H, m).

### Example 190

To a mixture of 3-[1-(2,4-dichlorobenzyl)-3-isopropyl-1H-pyrazol-4-yl]propan-1-ol (1.11 g), ethyl 3-(3-hydroxy-1-phenyl-1H-pyrazol-5-yl)propanoate (0.78 g), tributylphosphine (1.87 ml) and tetrahydrofuran (30 ml) was added 1,1'-azodicarbonyldipiperidine (1.51 g) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:20 to 1:3, v/v) to give a pale-yellow oil. To a solution (30 ml) of the obtained oil in a mixed solvent of tetrahydrofuran-ethanol (1:1, v/v) was added a 1N aqueous sodium hydroxide solution (5 ml) at 60°C, and the mixture was stirred for 30 min. 1N Hydrochloric acid (5 ml) was added, and the mixture was extracted with ethyl acetate. The ethyl acetate layer was washed with saturated brine, dried (MgSO₄) and concentrated. The obtained residue was crystallized from ethyl acetate-hexane to give 3-(3-{3-[1-(2,4-dichlorobenzyl)-3-isopropyl-1H-pyrazol-4-yl]propoxy}-1-phenyl-1H-pyrazol-5-yl)propanoic acid (1.20 g, yield 88%) as colorless crystals. melting point 105 - 107°C.

### Example 191

To a mixture of 3-[1-(2,4-dichlorobenzyl)-3-isopropoxy-1H-pyrazol-5-yl]propan-1-ol (343 mg), ethyl 3-(1-benzyl-3-hydroxy-1H-pyrazol-5-yl)propanoate (274 mg), tributylphosphine (0.623 ml) and tetrahydrofuran (10 ml) was added 1,1'-azodicarbonyldipiperidine (505 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:20 to 1:3, v/v) to give a pale-yellow oil. To a solution (10 ml) of the obtained oil in a mixed solvent of tetrahydrofuran-ethanol (1:1, v/v) was added a 1N aqueous sodium hydroxide solution (2 ml) at room temperature, and the mixture was stirred for 16 hr. 1N Hydrochloric acid (2 ml) was added, and the mixture was extracted with ethyl acetate. The ethyl acetate layer was washed with saturated brine, dried (MgSO₄) and concentrated. The obtained residue was crystallized from ethyl acetate-hexane to give 3-(1-benzyl-3-{3-[1-(2,4-dichlorobenzyl)-3-isopropoxy-1H-pyrazol-5-yl]propoxy}-1H-pyrazol-5-yl)propanoic acid (411 mg, yield 72%) as white crystals. melting point 123 - 126°C.

### Example 192

To a mixture of 3-[1-(2,4-dichlorobenzyl)-3-isopropoxy-1H-pyrazol-5-yl]propan-1-ol (343 mg), ethyl 3-(3-hydroxy-1-phenyl-1H-pyrazol-5-yl)propanoate (260 mg), tributylphosphine (0.623 ml) and tetrahydrofuran (10 ml) was added 1,1'-azodicarbonyldipiperidine (505 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:20 to 1:2, v/v) to give a pale-yellow oil. To a solution (10 ml) of the obtained oil in a mixed solvent of tetrahydrofuran-ethanol (1:1, v/v) was added a 1N aqueous sodium hydroxide solution (2 ml) at room temperature, and the mixture was stirred for 16 hr. 1N Hydrochloric acid (2 ml) was added, and the mixture was extracted with ethyl acetate. The ethyl acetate layer was washed with saturated brine, dried (MgSO₄) and concentrated. The obtained residue was crystallized from ethyl acetate-hexane to give 3-(3-{3-[1-(2,4-dichlorobenzyl)-3-isopropoxy-1H-pyrazol-5-yl]propoxy}-1-phenyl-1H-pyrazol-5-yl)propanoic acid (408 mg, yield 88%) as white crystals. melting point 145.5 - 147.5°C.

### Example 193

To a mixture of 3-[1-(2,4-dichlorobenzyl)-3-ethoxy-1H-pyrazol-4-yl]propan-1-ol (329 mg), ethyl 3-(3-hydroxy-1-phenyl-1H-pyrazol-4-yl)propanoate (260 mg), tributylphosphine (0.623 ml) and tetrahydrofuran (10 ml) was added 1,1'-azodicarbonyldipiperidine (505 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:20 to 1:2, v/v) to give a pale-yellow oil. To a solution (20 ml) of the obtained oil in a mixed solvent of tetrahydrofuran-ethanol (1:1, v/v) was added a 1N aqueous sodium hydroxide solution (1 ml) at room temperature, and the mixture was stirred for 16 hr. 1N Hydrochloric acid (1 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 1:2, v/v). The eluate was concentrated. The obtained residue was crystallized from ethyl acetate-hexane to give 3-(3-{3-[1-(2,4-dichlorobenzyl)-3-ethoxy-1H-pyrazol-4-yl]propoxy}-1-phenyl-1H-pyrazol-4-yl)propanoic acid (371 mg, yield 85%) as white crystals. melting point 114 - 118°C.

### Example 194

To a mixture of {3-[(2,4-dichlorophenoxy)methyl]-5-isopropoxyphenyl}methanol (342 mg), ethyl 3-(3-hydroxy-1-methyl-1H-pyrazol-5-yl)propanoate (198 mg), tributylphosphine (0.623 ml) and tetrahydrofuran (10 ml) was added 1,1'-azodicarbonyldipiperidine (505 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:20 to 1:2, v/v) to give a pale-yellow oil. To a solution (14 ml) of the obtained oil in a mixed solvent of tetrahydrofuran-ethanol (1:1, v/v) was added a 1N aqueous sodium hydroxide solution (1.2 ml) at room temperature, and the mixture was stirred for 16 hr. 1N Hydrochloric acid (1.2 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 4:1, v/v). The elute was concentrated, and the obtained residue was crystallized from ethyl acetate-hexane to give 3-[3-({3-[(2,4-dichlorophenoxy)methyl]-5-isopropoxybenzyl}oxy)-1-methyl-1H-pyrazol-5-yl]propanoic acid (175 mg, yield 36%) as white crystals. melting point 105 - 106°C.

### Example 195

To a mixture of {3-[(2,4-dichlorophenoxy)methyl]-5-isopropoxyphenyl}methanol (342 mg), ethyl 3-(3-hydroxy-1-phenyl-1H-pyrazol-5-yl)propanoate (260 mg), tributylphosphine (0.623 ml) and tetrahydrofuran (10 ml) was added 1,1'-azodicarbonyldipiperidine (505 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:20 to 1:2, v/v) to give a pale-yellow oil. To a solution (14 ml) of the obtained oil in a mixed solvent of tetrahydrofuran-ethanol (1:1, v/v) was added a 1N aqueous sodium hydroxide solution (1.2 ml) at room temperature, and the mixture was stirred for 16 hr. 1N Hydrochloric acid (1.2 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 4:1, v/v). The elute was concentrated, and the obtained residue was crystallized from ethyl acetate-hexane to give 3-[3-({3-[(2,4-dichlorophenoxy)methyl]-5-isopropoxybenzyl}oxy)-1-phenyl-1H-pyrazol-5-yl]propanoic acid (238 mg, yield 43%) as white crystals. melting point 108 - 110°C.

### Example 196

To a mixture of 3-{3-[(2,4-dichlorophenoxy)methyl]-5-isopropoxyphenyl}propan-1-ol (369 mg), ethyl 3-(3-hydroxy-1-phenyl-1H-pyrazol-5-yl)propanoate (260 mg), tributylphosphine (0.623 ml) and tetrahydrofuran (10 ml) was added 1,1'-azodicarbonyldipiperidine (505 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:100 to 1:2, v/v) to give a pale-yellow oil. To a solution (14 ml) of the obtained oil in a mixed solvent of tetrahydrofuran-ethanol (1:1, v/v) was added a 1N aqueous sodium hydroxide solution (1.2 ml) at room temperature, and the mixture was stirred for 16 hr. 1N Hydrochloric acid (1.2 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 4:1, v/v). The elute was concentrated, and the obtained residue was crystallized from ethyl acetate-hexane to give 3-[3-(3-{3-[(2,4-dichlorophenoxy)methyl]-5-isopropoxyphenyl}propoxy)-1-phenyl-1H-pyrazol-5-yl]propanoic acid (346 mg, yield 59%) as white crystals. melting point 104 - 106°C.

### Example 197

To a mixture of 3-{3-[(2,4-dichlorophenoxy)methyl]-5-isopropoxyphenyl}propan-1-ol (369 mg), ethyl 3-(3-hydroxy-1-methyl-1H-pyrazol-5-yl)propanoate (198 mg), tributylphosphine (0.623 ml) and tetrahydrofuran (10 ml) was added 1,1'-azodicarbonyldipiperidine (505 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:100 to 1:2, v/v) to give a pale-yellow oil. To a solution (14 ml) of the obtained oil in a mixed solvent of tetrahydrofuran-ethanol (1:1, v/v) was added a 1N aqueous sodium hydroxide solution (1.5 ml) at room temperature, and the mixture was stirred for 16 hr. 1N Hydrochloric acid (1.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 4:1, v/v). The eluate was concentrated. The obtained residue was crystallized from ethyl acetate-hexane to give 3-[3-(3-{3-[(2,4-dichlorophenoxy)methyl]-5-isopropoxyphenyl}propoxy)-1-methyl-1H-pyrazol-5-yl]propanoic acid (111 mg, yield 21%) as white crystals, melting point 116 - 120°C.

### Example 198

To a mixture of {3-(benzyloxy)-5-[(2,4-dichlorophenoxy)methyl]phenyl}methanol (3.00 g), ethyl 3-(3-hydroxy-1-phenyl-1H-pyrazol-5-yl)propanoate (2.01 g), tributylphosphine (4.81 ml) and tetrahydrofuran (100 ml) was added 1,1'-azodicarbonyldipiperidine (3.89 g) at room temperature, and the mixture was stirred at 50°C for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:100 to 1:2, v/v) to give a pale-yellow oil. To a solution (20 ml) of the obtained oil (0.55 g) in a mixed solvent of tetrahydrofuran-ethanol (1:1, v/v) was added a 1N aqueous sodium hydroxide solution (1.6 ml) at room temperature, and the mixture was stirred for 16 hr. 1N Hydrochloric acid (1.6 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 4:1, v/v). The eluate was concentrated. The obtained residue was recrystallized from ethyl acetate-hexane to give 3-[3-({3-(benzyloxy)-5-[(2,4-dichlorophenoxy)methyl]benzyl}oxy)-1-phenyl-1H-pyrazol-5-yl]propanoic acid (461 mg, yield 88%) as white crystals. melting point 126 - 130°C.

### Example 199

To a mixture of [3-{[3-chloro-5-(trifluoromethyl)pyridin-2-yl]oxy}-5-(ethoxymethyl)phenyl]methanol (361 mg), ethyl 3-(3-hydroxy-1-phenyl-1H-pyrazol-5-yl)propanoate (260 mg), tributylphosphine (0.623 ml) and tetrahydrofuran (10 ml) was added 1,1'-azodicarbonyldipiperidine (505 mg) at room temperature, and the mixture was stirred at 50°C for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:100 to 1:4, v/v) to give a pale-yellow oil. To a solution (14 ml) of the obtained oil in a mixed solvent of tetrahydrofuran-ethanol (1:1, v/v) was added a 1N aqueous sodium hydroxide solution (1.2 ml) at room temperature, and the mixture was stirred for 8 hr. 1N Hydrochloric acid (1.2 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 4:1, v/v). The eluate was concentrated. The obtained residue was recrystallized from diisopropyl ether-hexane to give 3-(3-{[3-{[3-chloro-5-(trifluoromethyl)pyridin-2-yl]oxy}-5-(ethoxymethyl)benzyl]oxy}-1-phenyl-1H-pyrazol-5-yl)propanoic acid (461 mg, yield 88%) as white crystals. melting point 124 - 128°C.

### Example 200

To a mixture of {3-[(2,4-dichlorophenoxy)methyl]-5-isopropoxyphenyl}methanol (341 mg), ethyl 3-(3-hydroxy-1-phenyl-1H-pyrazol-4-yl)propanoate (260 mg), tributylphosphine (0.623 ml) and tetrahydrofuran (10 ml) was added 1,1'-azodicarbonyldipiperidine (505 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:100 to 1:4, v/v) to give a pale-yellow oil. To a solution (12 ml) of the obtained oil in a mixed solvent of tetrahydrofuran-ethanol (1:1, v/v) was added a 1N aqueous sodium hydroxide solution (0.8 ml) at room temperature, and the mixture was stirred for 8 hr. 1N Hydrochloric acid (0.8 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 4:1, v/v). The eluate was concentrated to give 3-[3-({3-[(2,4-dichlorophenoxy)methyl]-5-isopropoxybenzyl}oxy)-1-phenyl-1H-pyrazol-4-yl]propanoic acid (0.21 g, yield 38%) as a colorless oil. ¹H-NMR (300 MHz, CDCl₃)δ:1.34 (6 H, d, J = 6.0 Hz), 2.57 - 2.72 (2 H, m), 2.72 - 2.85 (2 H, m), 4.46 - 4.71 (1 H, m), 5.12 (2 H, s), 5.31 (2 H, s), 6.87 (1 H, d, J = 8.9 Hz), 6.95 (2 H, d, J = 8.7 Hz), 7.04 - 7.22 (3 H, m), 7.31 - 7.45 (3 H, m), 7.50 - 7.59 (2 H, m), 7.60 (1 H, s).

### Example 201

To a mixture of {3-[(2,4-dichlorophenoxy)methyl]-5-isopropoxyphenyl}methanol (341 mg), ethyl 3-(3-hydroxy-1-methyl-1H-pyrazol-4-yl)propanoate (198 mg), tributylphosphine (0.623 ml) and tetrahydrofuran (10 ml) was added 1,1'-azodicarbonyldipiperidine (505 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:100 to 1:4, v/v) to give a pale-yellow oil. To a solution (14 ml) of the obtained oil in a mixed solvent of tetrahydrofuran-ethanol (1:1, v/v) was added a 1N aqueous sodium hydroxide solution (1.2 ml) at room temperature, and the mixture was stirred for 8 hr. 1N Hydrochloric acid (1.2 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 4:1, v/v). The eluate was concentrated to give 3-[3-({3-[(2,4-dichlorophenoxy)methyl]-5-isopropoxybenzyl}oxy)-1-methyl-1H-pyrazol-4-yl]propanoic acid (0.33 g, yield 55%) as a colorless oil. ¹H-NMR (300 MHz, CDCl₃)δ:1.33 (6 H, d, J = 6.2 Hz), 2.49 - 2.64 (2 H, m), 2.64 - 2.76 (2 H, m), 3.69 (3 H, s), 4.44 - 4.66 (1 H, m), 5.11 (2 H, s), 5.17 (2 H, s), 6.78 - 6.97 (3 H, m), 6.97 - 7.07 (2 H, m), 7.13 (1 H, dd, J = 8.9, 2.6 Hz), 7.37 (1 H, d, J = 2.5 Hz).

### Example 202

To a mixture of ethyl [3-(3-{[3-(hydroxymethyl)-5-isopropoxybenzyl]oxy}-1-phenyl-1H-pyrazol-5-yl)propanoate (0.37 g), 4-(trifluoromethyl)phenol (137 mg), tributylphosphine (0.526 ml) and tetrahydrofuran (10 ml) was added 1,1'-azodicarbonyldipiperidine (426 mg) at room temperature, and the mixture was stirred at 50°C for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:100 to 1:4, v/v) to give a pale-yellow oil. To a solution (14 ml) of the obtained oil in a mixed solvent of tetrahydrofuran-ethanol (1:1, v/v) was added a 1N aqueous sodium hydroxide solution (1.5 ml) at room temperature, and the mixture was stirred for 8 hr. 1N Hydrochloric acid (1.5 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 4:1, v/v). The eluate was concentrated. The obtained residue was recrystallized from diethyl ether-hexane to give 3-{3-[(3-isopropoxy-5-{[4-(trifluoromethyl)phenoxy]methyl}benzyl)oxy]-1-phenyl-1H-pyrazol-5-yl}propanoic acid (300 mg, yield 64%) as white crystals. melting point 55 - 58°C.

### Example 203

To a solution of ethyl [3-(3-{[3-(hydroxymethyl)-5-isopropoxybenzyl]oxy}-1-phenyl-1H-pyrazol-5-yl)propanoate (0.44 g) in N,N-dimethylformamide (10 ml) was added potassium tert-butoxide (135 mg) at 0°C, and the mixture was stirred for 1 hr. To the reaction mixture were added 2,3-dichloro-5-(trifluoromethyl)pyridine (216 mg), and the mixture was stirred at room temperature for 16 hr. Water was added to the reaction mixture, and the mixture was extracted with ethyl acetate. The ethyl acetate layer was washed with water and saturated brine, dried (MgSO₄) and concentrated. The residue was subjected to silica gel column chromatography and eluted with ethyl acetate-hexane (1:100 to 1:2, v/v) to give a pale-yellow oil. To a solution (12 ml) of the obtained oil in a mixed solvent of tetrahydrofuran-ethanol (1:1, v/v) was added a 1N aqueous sodium hydroxide solution (0.7 ml) at room temperature, and the mixture was stirred for 8 hr. 1N Hydrochloric acid (0.7 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 4:1, v/v) and concentrated. The obtained residue was recrystallized from ethyl acetate-hexane to give 3-(3-{[3-({[3-chloro-5-(trifluoromethyl)pyridin-2-yl]oxy}methyl)-5-isopropoxybenzyl]oxy}-1-phenyl-1H-pyrazol-5-yl)propanoic acid (187 mg, yield 32%) as white crystals. melting point 140 - 142°C.

### Example 204

To a mixture of {3-[(2,4-dichlorophenoxy)methyl]-5-ethoxyphenyl}methanol (0.30 g), ethyl 3-(3-hydroxy-1-phenyl-1H-pyrazol-5-yl)propanoate (239 mg), tributylphosphine (0.571 ml) and tetrahydrofuran (10 ml) was added 1,1'-azodicarbonyldipiperidine (463 mg) at room temperature, and the mixture was stirred at 50°C for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:100 to 1:4, v/v) to give a pale-yellow oil. To a solution (10 ml) of the obtained oil in a mixed solvent of tetrahydrofuran-ethanol (1:1, v/v) was added a 1N aqueous sodium hydroxide solution (0.6 ml) at room temperature, and the mixture was stirred for 8 hr. 1N Hydrochloric acid (0.6 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 4:1, v/v). The eluate was concentrated. The obtained residue was recrystallized from ethyl acetate-hexane to give 3-[3-({3-[(2,4-dichlorophenoxy)methyl]-5-ethoxybenzyl}oxy)-1-phenyl-1H-pyrazol-5-yl]propanoic acid (263 mg, yield 47%) as white crystals. melting point 151.5 - 152°C.

### Example 205

To a mixture of {3-[(2,4-dichlorophenoxy)methyl]-5-isopropoxyphenyl}methanol (0.28 g), ethyl 3-(1-ethyl-3-hydroxy-1H-pyrazol-5-yl)propanoate (173 mg), tributylphosphine (0.511 ml) and tetrahydrofuran (10 ml) was added 1,1'-azodicarbonyldipiperidine (414 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:100 to 1:4, v/v) to give a pale-yellow oil. To a solution (10 ml) of the obtained oil in a mixed solvent of tetrahydrofuran-ethanol (1:1, v/v) was added a 1N aqueous sodium hydroxide solution (1.0 ml) at room temperature, and the mixture was stirred for 8 hr. 1N Hydrochloric acid (1.0 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 4:1, v/v). The eluate was concentrated to give 3-[3-({3-[(2,4-dichlorophenoxy)methyl]-5-isopropoxybenzyl}oxy)-1-ethyl-1H-pyrazol-5-yl]propanoic acid (0.20 g, yield 48%) as a pale-yellow oil. ¹H-NMR (300 MHz, CDCl₃)δ:1.32 (6 H, d, J = 6.0 Hz), 1.37 (3 H, t, J = 7.2 Hz), 2.63 - 2.75 (2 H, m), 2.87 (2 H, t, J = 7.4 Hz), 3.97 (2 H, q, J = 7.3 Hz), 4.44 - 4.66 (1 H, m), 5.09 (2 H, s), 5.11 (2 H, s), 5.48 (1 H, s), 6.86 (1 H, d, J = 8.7 Hz), 6. 92 (2 H, s), 7.03 (1 H, s), 7.13 (1 H, dd, J = 8.8, 2.5 Hz), 7.37 (1 H, d, J = 2.6 Hz).

### Example 206

To a mixture of 3-{3-[(2,4-dichlorophenoxy)methyl]-5-isopropoxyphenyl}propan-1-ol (0.37 g), ethyl 3-(1-ethyl-3-hydroxy-1H-pyrazol-5-yl)propanoate (212 mg), tributylphosphine (0.623 ml) and tetrahydrofuran (10 ml) was added 1,1'-azodicarbonyldipiperidine (505 mg) at 50°C, and the mixture was stirred for 1 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:100 to 1:4, v/v) to give a pale-yellow oil. To a solution (10 ml) of the obtained oil in a mixed solvent of tetrahydrofuran-ethanol (1:1, v/v) was added a 1N aqueous sodium hydroxide solution (1.1 ml) at room temperature, and the mixture was stirred for 8 hr. 1N Hydrochloric acid (1.1 ml) was added, and the mixture was extracted with ethyl acetate. The ethyl acetate layer was washed with saturated brine, dried (MgSO₄) and concentrated. The residue was subjected to silica gel column chromatography and eluted with ethyl acetate-hexane (1:20 to 4:1, v/v). The eluate was concentrated to give 3-[3-(3-{3-[(2,4-dichlorophenoxy)methyl]-5-isopropoxyphenyl}propoxy)-1-ethyl-1H-pyrazol-5-yl]propanoic acid (0.24 g, yield 45%) as a pale-yellow oil. ¹H-NMR (300 MHz, CDCl₃)δ:1.31 (6 H, d, J = 6.0 Hz), 1.33 - 1.39 (3 H, m), 1.94 - 2.14 (2 H, m), 2.61 - 2.80 (4 H, m), 2.87 (2 H, t, J = 7.4 Hz), 3.95 (2 H, q, J = 7.2 Hz), 4.07 (2 H, t, J = 6.3 Hz), 4.41 - 4.64 (1 H, m), 5.06 (2 H, s), 5.44 (1 H, s), 6.70 (1 H, d, J = 1.5 Hz), 6.82 (2 H, s), 6.86 (1 H, d, J = 8.9 Hz), 7.13 (1 H, dd, J = 8.8, 2.5 Hz), 7.37 (1 H, d, J = 2.5 Hz).

### Example 207

To a mixture of 3-[1-(2,4-dichlorobenzyl)-3-(methoxymethoxy)-1H-pyrazol-5-yl]propan-1-ol (0.57 g), methyl (2-hydroxypyridin-3-yl)acetate (0.31 g), tributylphosphine (0.67 g) and tetrahydrofuran (20 ml) was added 1,1'-azodicarbonyldipiperidine (0.83 g) at room temperature, and the mixture was stirred for 4.5 hr. The reaction solution was concentrated, diisopropyl ether was added to the residue, and the insoluble substance was removed by filtration. The filtrate was concentrated, and the obtained residue was subjected to silica gel column chromatography and eluted with ethyl acetate-hexane (1:4 to 2:3, v/v) to give a pale-yellow oil. To a solution (20 ml) of the obtained oil (0.46 g) in a mixed solvent of tetrahydrofuran-methanol (1:1, v/v) was added a 1N aqueous sodium hydroxide solution (1.9 ml) at room temperature, and the mixture was stirred for 15.5 hr. 1N Hydrochloric acid (1.9 ml) was added, and the mixture was extracted with ethyl acetate. The ethyl acetate layer was washed with saturated brine, dried (Na₂SO₄) and concentrated. The residue was crystallized from ethyl acetate-hexane to give (2-{3-[1-(2,4-dichlorobenzyl)-3-(methoxymethoxy)-1H-pyrazol-5-yl]propoxy}pyridin-3-yl)acetic acid (388 mg, yield 87%) as colorless crystals. melting point 101 - 102°C.

### Example 208

To a mixture of (2-benzyl-4-{3-[1-(2,4-dichlorobenzyl)-3-isopropoxy-1H-pyrazol-5-yl]propoxy}-1,3-thiazol-5-yl)methanol (960 mg), acetonecyanohydrin (320 µl) and tetrahydrofuran (100 ml) were added tributylphosphine (872 µl) and 1,1'-azodicarbonyldipiperidine (883 mg), and the mixture was stirred at room temperature for 1 hr. The reaction mixture was concentrated, diisopropyl ether was added to the residue, and the insoluble substance was removed by filtration. The filtrate was concentrated, and the obtained residue was subjected to silica gel column chromatography to give, from a fraction eluted with ethyl acetate-hexane (3:17, v/v), a brown oil. To a solution of the obtained oil in ethanol (50 ml) was added a 8N aqueous sodium hydroxide solution (10.4 ml), and the mixture was heated until the solvent is refluxed. After stirring at said temperature for 1 hr, the reaction mixture was neutralized with 1N hydrochloric acid, and the mixture was extracted with ethyl acetate. The extract was washed with saturated brine, dried (MgSO₄) and concentrated. The obtained residue was subjected to reversed-phase high performance liquid chromatography and eluted with acetonitrile-water (1:9 to acetonitrile alone, v/v). Then the eluate was subjected to silica gel column chromatography and eluted with ethyl acetate-hexane (3:7 to 7:3, v/v). The obtained colorless oil was crystallized from ethyl acetate-hexane to give {(2-benzyl-4-{3-[1-(2,4-dichlorobenzyl)-3-isopropoxy-1H-pyrazol-5-yl]propoxy}-1,3-thiazol-5-yl)acetic acid (30.4 mg, yield 3%) as colorless crystals. melting point 106 - 108°C.

| **Formulation Example** 1 (production of capsule) | |
|---|---|
| 1) compound of Example 1 | 30 mg |
| 2) finely divided powder cellulose | 10 mg |
| 3) lactose | 19 mg |
| 4) magnesium stearate | 1 mg |
| total | 60 mg |

1), 2), 3) and 4) are mixed and filled in a gelatin capsule.

| **Formulation Example 2** (production of tablet) | |
|---|---|
| 1) compound of Example 1 | 30 g |
| 2) lactose | 50 g |
| 3) cornstarch | 15 g |
| 4) calcium carboxymethylcellulose | 44 g |
| 5) magnesium stearate | 1 g |
| 1000 tablets total | 140 g |

The total amount of 1), 2), 3) and 30 g of 4) are kneaded with water, vacuum dried and sized.

The sized powder is mixed with 14 g of 4) and 1 g of 5) and the mixture is punched out with a tableting machine. In this way, 1000 tablets containing 30 mg of the compound of Example 1 per tablet are obtained.

### Industrial Applicability

The compound of the present invention has superior hypoglycemic action and hypoglycemic action and is useful as an agent for the prophylaxis or treatment of diabetes, hyperlipidemia, impaired glucose tolerance and the like.

This application is based on a patent application No. 2004-342635 filed in Japan, the contents of which are incorporated in full herein by this reference.

## Claims

1. A compound represented by the formula (I): 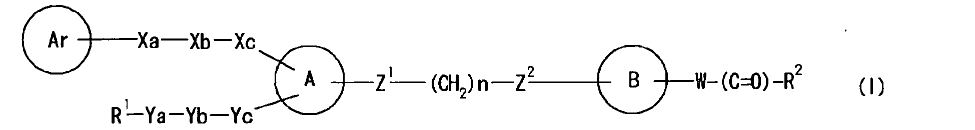 wherein
Ar is an optionally substituted aromatic ring;
Xa, Xc, Ya, Yc, Z¹ and Z² are the same or different and each is a bond, an oxygen atom, a sulfur atom, -CO-, -CS-, -CR³(OR⁴)-, - NR⁵-, -SO-, -SO₂-, -CONR⁶- or -NR⁶CO- (wherein R³ is a hydrogen atom or an optionally substituted hydrocarbon group, R⁴ is a hydrogen atom or a hydroxyl-protecting group, R⁵ is a hydrogen atom, an optionally substituted hydrocarbon group or an amino-protecting group, and R⁶ is a hydrogen atom or an optionally substituted hydrocarbon group);
Xb and Yb are the same or different and each is a bond or a divalent hydrocarbon group having 1 to 20 carbon atoms;
R¹ is an optionally substituted hydrocarbon group;
ring A is an optionally further substituted aromatic ring, provided that the ring should not be benzimidazole;
n is an integer of 1 to 8;
ring B is an optionally further substituted aromatic ring, provided that the ring should not be oxazole;
W is a divalent saturated hydrocarbon group having 1 to 20 carbon atoms; and
R² is -OR⁸ (wherein R⁸ is a hydrogen atom or an optionally substituted hydrocarbon group) or -NR⁹R¹⁰ (wherein R⁹ and R¹⁰ are the same or different and each is a hydrogen atom, an optionally substituted hydrocarbon group, an optionally substituted heterocyclic group or an acyl group, or R⁹ and R¹⁰ are bonded to each other to form, together with the adjacent nitrogen atom, an optionally substituted ring):
with the proviso that
a compound wherein Xa, Xb and Xc are each a bond is excluded;
a compound wherein Z¹ and Z² are each a bond or each an oxygen atom is excluded; and
when ring A is a benzene ring, then the carbon atom thereon to which Xc is bonded and the carbon atom thereon to which Yc is bonded should not be adjacent to each other, and Z² should not be a sulfur atom,
or a salt thereof.

2. The compound of claim 1, wherein the aromatic ring for Ar is a benzene ring, a pyridine ring, an oxazole ring or a quinoline ring.

3. The compound of claim 1, wherein Xa is a bond or an oxygen atom, Xb is a bond or a divalent hydrocarbon group having 1 to 6 carbon atoms, and Xc is a bond, an oxygen atom, -CO- or -NR⁶CO-(wherein R⁶ is a hydrogen atom or an alkyl group having 1 to 4 carbon atoms).

4. The compound of claim 3, wherein Xa is a bond, Xb is a bond or a divalent hydrocarbon group having 1 to 6 carbon atoms, and Xc is an oxygen atom.

5. The compound of claim 3, wherein Xa is a bond, Xb is a divalent hydrocarbon group having 1 to 6 carbon atoms, and Xc is a bond.

6. The compound of claim 1, wherein R¹ is an optionally substituted alkyl group having 1 to 10 carbon atoms.

7. The compound of claim 1, wherein Ya is a bond, an oxygen atom, a sulfur atom, -NR⁵- (wherein R⁵ is an alkyl group having 1 to 4 carbon atoms), -SO₂-, -CONR⁶- or -NR⁶CO- (wherein R⁶ is a hydrogen atom or an alkyl group having 1 to 4 carbon atoms), Yb is a bond or a divalent hydrocarbon group having 1 to 6 carbon atoms, and Yc is a bond or an oxygen atom.

8. The compound of claim 7, wherein Ya and Yb are each a bond.

9. The compound of claim 1, wherein the aromatic ring for ring A is a benzene ring, a pyridine ring, a pyrimidine ring, a pyrazole ring or a thiazole ring.

10. The compound of claim 9, wherein the aromatic ring for ring A is a benzene ring.

11. The compound of claim 1, wherein Z¹ is a bond, n is an integer of 1 to 4, and Z² is an oxygen atom.

12. The compound of claim 1, wherein the aromatic ring for ring B is a benzene ring, a naphthalene ring, a pyridine ring, a pyrazole ring or a thiazole ring.

13. The compound of claim 8, wherein the aromatic ring for ring B is a pyrazole ring.

14. The compound of claim 1, wherein R² is -OR⁸, and R⁸ is a hydrogen atom or an alkyl group having 1 to 4 carbon atoms.

15. The compound of claim 1, which is
3-{3-[3-(2-{[3-chloro-5-(trifluoromethyl)pyridin-2-yl]oxy}-4-isopropoxyphenyl)propoxy]-1-ethyl-1H-pyrazol-5-yl}propanoic acid,
3-(3-{3-[2-{[3-chloro-5-(trifluoromethyl)pyridin-2-yl]oxy}-4-(2-methoxyethoxy)phenyl]propoxy}-1-ethyl-1H-pyrazol-5-yl)propanoic acid,
{2-[3-(2-{[3-chloro-5-(trifluoromethyl)pyridin-2-yl]oxy}-4-isopropoxyphenyl)propoxy]-3-methoxyphenyl}acetic acid,
(1-benzyl-3-{3-[1-(2,4-dichlorobenzyl)-3-isopropoxy-1H-pyrazol-5-yl]propoxy}-1H-pyrazol-4-yl)acetic acid,
(2-{3-[1-(2,4-dichlorobenzyl)-3-isopropoxy-1H-pyrazol-5-yl]propoxy}-3-methoxyphenyl)acetic acid,
(4-{3-[1-(2,4-dichlorobenzyl)-3-isopropoxy-1H-pyrazol-5-yl]propoxy}-2-methyl-1,3-thiazol-5-yl)acetic acid,
[2-(3-{1-[2-chloro-4-(trifluoromethyl)benzyl]-3-isopropyl-1H-pyrazol-5-yl}propoxy)pyridin-3-yl]acetic acid,
(3-{3-[2-(2,4-dichlorophenoxy)-6-isopropoxypyridin-3-yl]propoxy}-1-methyl-1H-pyrazol-4-yl)acetic acid, or
(1-{3-[1-(2,4-dichlorobenzyl)-3-isopropoxy-1H-pyrazol-5-yl]propoxy}-2-naphthyl)acetic acid.

16. A prodrug of the compound of claim 1.

17. A pharmaceutical agent comprising the compound of claim 1 or a prodrug thereof.

18. The pharmaceutical agent of claim 17, which is an agent for the prophylaxis or treatment of diabetes.

19. The pharmaceutical agent of claim 17, which is an agent for the prophylaxis or treatment of hyperlipidemia.

20. The pharmaceutical agent of claim 17, which is an agent for the prophylaxis or treatment of impaired glucose tolerance.

21. A retinoid-related receptor function regulator comprising the compound of claim 1 or a prodrug thereof.

22. The retinoid-related receptor function regulator of claim 21, which is a peroxisome proliferator-activated receptor ligand.

23. The retinoid-related receptor function regulator of claim 21, which is a retinoid X receptor ligand.

24. An insulin sensitizer comprising the compound of claim 1 or a prodrug thereof.

25. A method for the prophylaxis or treatment of diabetes in a mammal, which comprises administering the compound of claim 1 or a prodrug thereof to the mammal.

26. A method for the prophylaxis or treatment of hyperlipidemia in a mammal, which comprises administering the compound of claim 1 or a prodrug thereof to the mammal.

27. A method for the prophylaxis or treatment of impaired glucose tolerance in a mammal, which comprises administering the compound of claim 1 or a prodrug thereof to the mammal.

28. Use of the compound of claim 1 or a prodrug thereof for the production of an agent for the prophylaxis or treatment of diabetes.

29. Use of the compound of claim 1 or a prodrug thereof for the production of an agent for the prophylaxis or treatment of hyperlipidemia.

30. Use of the compound of claim 1 or a prodrug thereof for the production of an agent for the prophylaxis or treatment of impaired glucose tolerance.

31. A production method of a compound represented by the formula (I-1) : 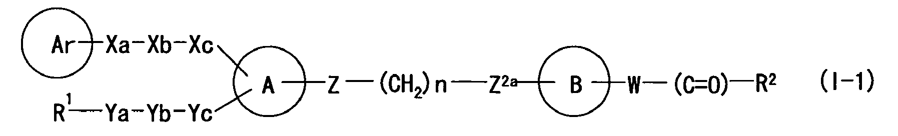 wherein each symbol is as defined in claim 1,
which comprises reacting a compound represented by the formula (II): 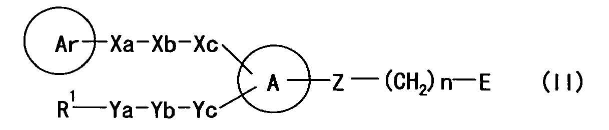 wherein E is a leaving group, and the other symbols are as defined in claim 1,
or a salt thereof with a compound represented by the formula (III) : 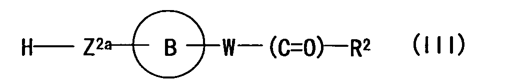 wherein Z^{2a} is an oxygen atom, a sulfur atom or -NR⁵- (wherein R⁵ is as defined in claim 1), and the other symbols are as defined in claim 1,
or a salt thereof.
